# EUROPEAN PATENT APPLICATION

(11) **EP 3 712 143 A1**
(43) Date of publication of application: **23.09.2020**
(21) Application number: 18876913.7
(22) Date of filing: 12.11.2018
(51) Int. Cl.: C07D 401/04, C07D 401/14, C07D 417/14, C07D 409/14, C07D 405/14, A61K 31/517, A61P 35/00

(54) **QUINAZOLINONE COMPOUND AND APPLICATION THEREOF**

(30) Priority: 13.11.2017 CN 201711116253
(71) Applicant: Luoxin Pharmaceutical (Shanghai) Co., Ltd., China (Shanghai) Pilot Free Trade Zone Pudong New Area Shanghai 201210 (CN); Shandong Luoxin Pharmaceutical Group Stock Co., Ltd., Shandong 276017 (CN)
(72) Inventor: WU, Chengde, Shanghai 200131 (CN); HUANG, Jingjie, Shanghai 200131 (CN); YU, Tao, Shanghai 200131 (CN); DONG, Jiaqiang, Shanghai 201210 (CN); WANG, Tie-Lin, Shanghai 201210 (CN); LI, Jie, Shanghai 200131 (CN); LI, Jian, Shanghai 200131 (CN); CHEN, Shuhui, Shanghai 200131 (CN)
(74) Representative: Gerauer, Marc Philippé
(86) International application number: PCT/CN2018/115044
(87) International publication number: WO 2019/091476

(57) **Abstract**

The present invention relates to a series of quinazolinone compounds and applications thereof as PI3Kα inhibitors. In particular, the present invention relates to a compound shown in formula (I) and a tautomer or pharmaceutically acceptable salt thereof.

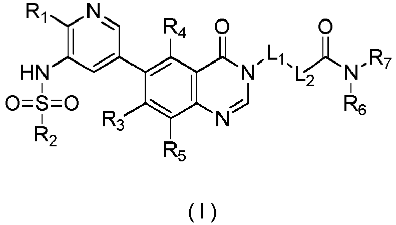

## Description

### Reference to related application

The present application claims the following priority:
CN201711116253.5 filed on November 13, 2017.

### Field of the invention

The present disclosure relates to a class of quinazolinone compounds and a use thereof as PI3Kα inhibitors. In particular, the present disclosure relates to a compound as represented by formula (I), a tautomer thereof or a pharmaceutically acceptable salt thereof.

### Prior arts

Phosphatidylinositol-3-kinase (PI3K) is a lipid kinase composed of a regulatory subunit p85 or p101, and a catalytic subunit p110 (further divided into four subtypes: p110α, p110β, p110δ, p110γ), which activates downstream Akt etc by catalyzing the phosphorylation of the inositol ring 3'-OH group in phosphatidylinositol 4,5-bisphosphate (PIP2) to phosphatidylinositol 3,4,5-triphosphate (PIP3), so it plays a key role in cell proliferation, survival and metabolism. PI3K is overexpressed in tumor cells, resulting in rapid proliferation and growth of tumor cells.

The tumor suppressor gene PTEN (Phosphatase and TENsin homolog deleted on chromosome 10) dephosphorylates PIP3 to generate PIP2, resulting in negative feedback regulation of the PI3K signaling pathway, inhibiting cell proliferation and promoting apoptosis. Frequent occurrence of PI3K gene mutations and amplifications in cancer and PTEN gene deletion in cancer suggest that PI3K overexpression is closely related to tumorigenesis.

Zhang hao et al. (Bioorganic Medicinal Chemistry, 2015 (23): 7765-7776*.*) found that compounds A2 and A10 (control examples R011 and R012) have a good inhibitory effect on PI3K.

### Content of the present invention

The present disclosure provides a compound of formula (I), an isomer thereof, or a pharmaceutically acceptable salt thereof, 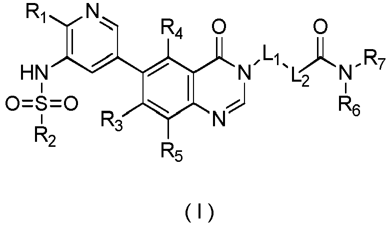 wherein,
R₁ is selected from H, F, Cl, Br, I, OH, NH₂, CN, NH₂, C₁₋₆ alkyl, C₁₋₆ heteroalkyl or C₃₋₆ cycloalkyl-O-, each of the NH₂, C₁₋₆ alkyl, C₁₋₆ heteroalkyl and C₃₋₆ cycloalkyl-O- is optionally substituted by one, two or three R;
R₂ is selected from phenyl or 5-6 membered heteroaryl, each of the phenyl and 5-6 membered heteroaryl is optionally substituted by one, two or three R;
R₂ is selected from phenyl and 5-6 membered heteroaryl, wherein the phenyl and 5-6 membered heteroaryl are optionally substituted by one, two or three R;
each of R₃, R₄ and R₅ is independently selected from H, F, Cl, Br, I, OH and NH₂;
R₆ is H, C₁₋₆ alkyl, C₁₋₆ heteroalkyl, C₃₋₇ cycloalkyl or 3-6 membered heterocycloalkyl, each of the C₁₋₆ alkyl, C₁₋₆ heteroalkyl, C₃₋₇ cycloalkyl and 3-6 membered heterocycloalkyl is optionally substituted by one, two or three R;
R₇ is H or C₁₋₆ alkyl which is optionally substituted by one, two or three R;
or, R₆ and R₇ are connected to form a 3-7-membered ring, which is optionally substituted by one, two or three R;
L₁ is a single bond or -C₁₋₆ alkyl- which is optionally substituted by one, two or three R;
L₂ is a single bond or -C₃₋₇ cycloalkyl- which is optionally substituted by one, two or three R;
each R is independently selected from H, F, Cl, Br, I, OH, NH₂, CN, C₁₋₆ alkyl or C₁₋₆ heteroalkyl, each of the C₁₋₆ alkyl and C₁₋₆ heteroalkyl is optionally substituted by one, two or three R';
each R' is independently selected from F, Cl, Br, I, OH, NH₂, CN, Me or Et;
the 3-6 membered heterocycloalkyl and the 5-6 membered heteroaryl contain 1-4 heteroatoms independently selected from N, O or S;
each heteroatom or heteroatomic group in the C₁₋₆ heteroalkyl is independently selected from N, -O-, -S-, -NH-, -C(=O)NH-, -C(=O)- or -C(=O)O-, and the number of the heteroatom or the heteroatomic group is one, two, three or four.

In some embodiments of the present disclosure, R is selected from H, F, Cl, Br, I, OH, NH₂, CN, C₁₋₃ alkyl or C₁₋₃ alkoxy, each of the C₁₋₃ alkyl and C₁₋₃ alkoxy is optionally substituted by one, two or three R', and other variables are as defined in the present disclosure.

In some embodiments of the present disclosure, R is selected from H, F, Cl, Br, I, OH, NH₂, CN, Me, Et or 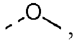 each of the Me, Et and 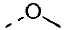 is optionally substituted by one, two or three R', and other variables are as defined in the present disclosure.

In some embodiments of the present disclosure, R is selected from H, F, Cl, Br, I, OH, NH₂, CN, Me, CF₃, Et, 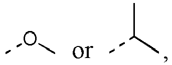 and other variables are as defined in the present disclosure.

In some embodiments of the present disclosure, R₁ is selected from H, F, Cl, Br, I, OH, NH₂, CN, C₁₋₃ alkyl, C₁₋₃ alkoxy, C₁₋₃ alkylamino or cyclopropyl-O-, each of the C₁₋₃ alkyl, C₁₋₃ alkoxy, C₁₋₃ alkylamino and cyclopropyl-O- is optionally substituted by one, two or three R, and other variables are as defined in the present disclosure.

In some embodiments of the present disclosure, R₁ is selected from H, F, Cl, Br, I, OH, NH₂, CN, Me, Et, 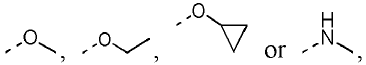 each of the Me, Et, 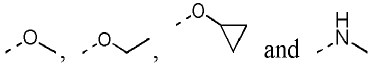 is optionally substituted by one, two or three R, and other variables are as defined in the present disclosure.

In some embodiments of the present disclosure, R₁ is selected from H, F, Cl, Br, I, OH, NH₂, CN, Me, Et, CF₃, 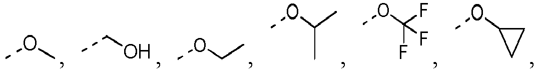 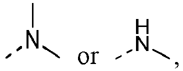 and other variables are as defined in the present disclosure.

In some embodiments of the present disclosure, R₂ is selected from phenyl, thiazolyl, furyl, oxazolyl, isoxazolyl, pyrrolyl orthienyl, each of the phenyl, thiazolyl, furyl, oxazolyl, isoxazolyl, pyrrolyl and thienyl is optionally substituted by one, two or three R, and other variables are as defined in the present disclosure.

In some embodiments of the present disclosure, R₂ is selected from 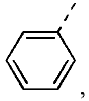 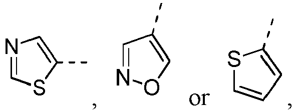 each of the 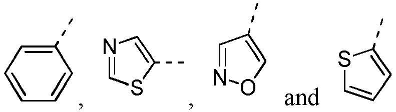 is optionally substituted by one, two or three R.

In some embodiments of the present disclosure, R₂ is selected from 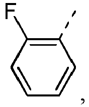 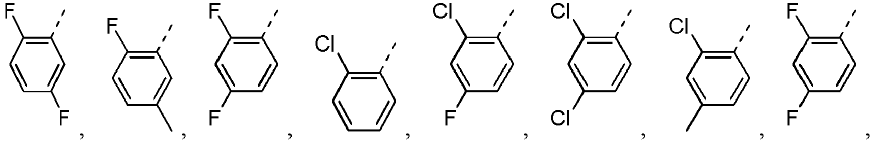 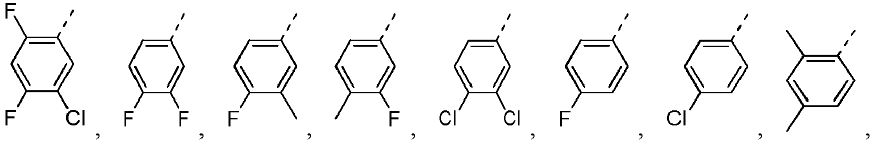 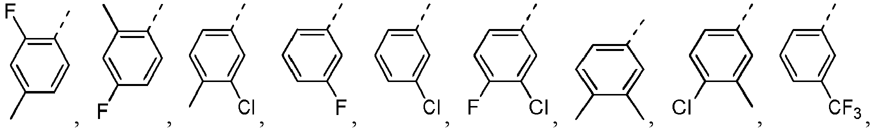 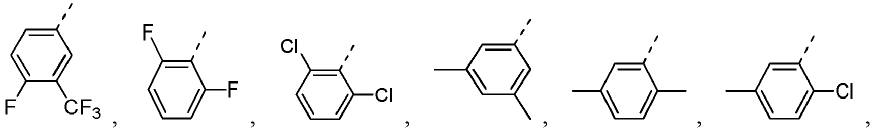 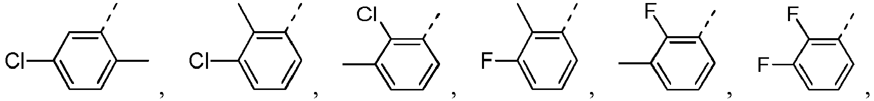 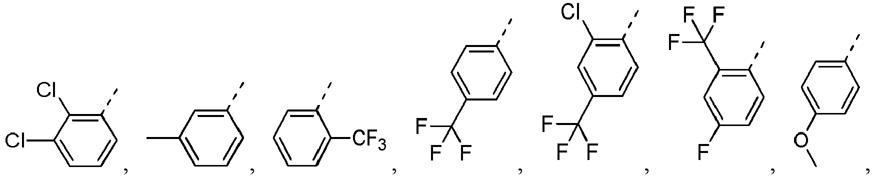 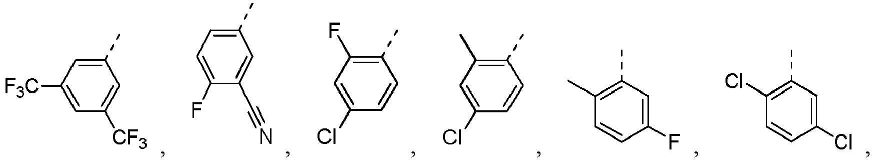 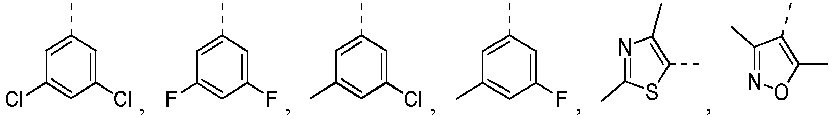 or 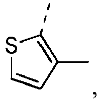 and other variables are as defined in the present disclosure.

In some embodiments of the present disclosure, R₆ is H, C₁₋₃ alkyl, C₁₋₃ heteroalkyl, cyclopropyl, cyclobutyl, oxetanyl or tetrahydrofuranyl, each of the C₁₋₃ alkyl, C₁₋₃ heteroalkyl, cyclopropyl, cyclobutyl, oxetanyl and tetrahydrofuranyl is optionally substituted by one, two or three R, and other variables are as defined in the present disclosure.

In some embodiments of the present disclosure, R₆ is H, Me, Et, 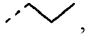 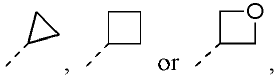 each of the Me, Et, 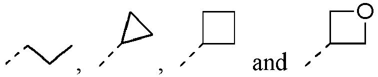 is optionally substituted by one, two or three R, and other variables are as defined in the present disclosure.

In some embodiments of the present disclosure, R₆ is selected from H, Me, Et, CF₃, 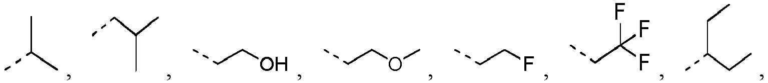 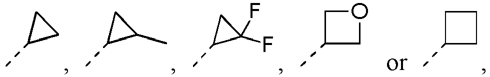 and other variables are as defined in the present disclosure.

In some embodiments of the present disclosure, R₇ is selected from H, Me or Et, and other variables are as defined in the present disclosure.

In some embodiments of the present disclosure, the structural unit 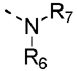 is selected from 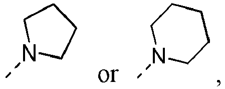 each of the 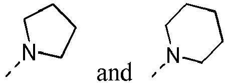 is optionally substituted by one, two or three R, and other variables are as defined in the present disclosure.

In some embodiments of the present disclosure, the structural unit 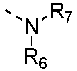 is 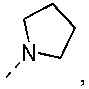 and other variables are as defined in the present disclosure.

In some embodiments of the present disclosure, L₁ is a single bond, -CH₂-,-CH₂-CH₂-, 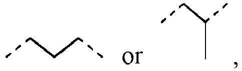 each of the -CH₂-, -CH₂-CH₂-, 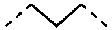 and 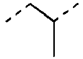 is optionally substituted by one, two or three R, and other variables are as defined in the present disclosure.

In some embodiments of the present disclosure, L₁ is selected from a single bond, -CH₂-, -CH₂-CH₂-, 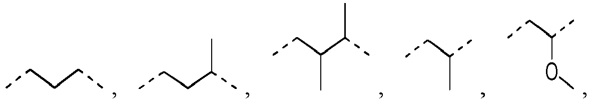 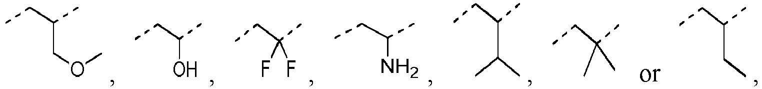 and other variables are as defined in the present disclosure.

In some embodiments of the present disclosure, L₂ is a single bond,-cyclopropyl-, -cyclobutyl- or -cyclopentyl-, each of the -cyclopropyl-, -cyclobutyl- and -cyclopentyl- is optionally substituted by one, two or three R, and other variables are as defined in the present disclosure.

In some embodiments of the present disclosure, L₂ is selected from a single bond, 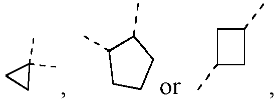 and other variables are as defined in the present disclosure.

In some embodiments of the present disclosure, the structural unit 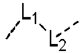 is selected from -CH₂-, -CH₂-CH₂-, 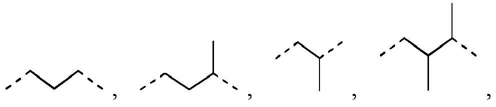 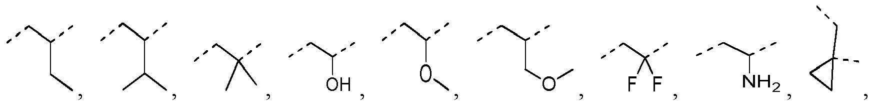 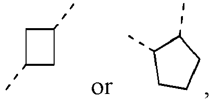 and other variables are as defined in the present disclosure.

Other embodiments of the present disclosure can be obtained by the arbitrary combination of the above variables.

In some embodiments of the present disclosure, the compound, the isomer thereof or the pharmaceutically acceptable salt thereof is selected from: 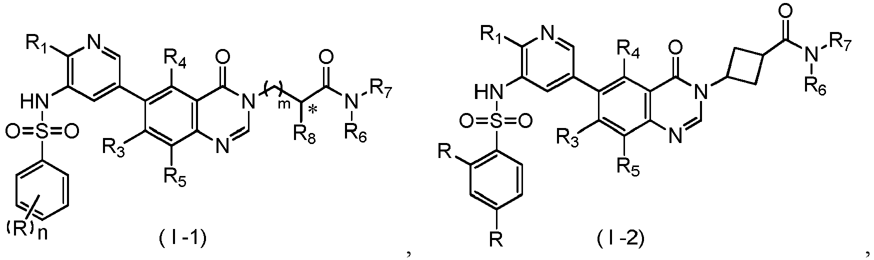 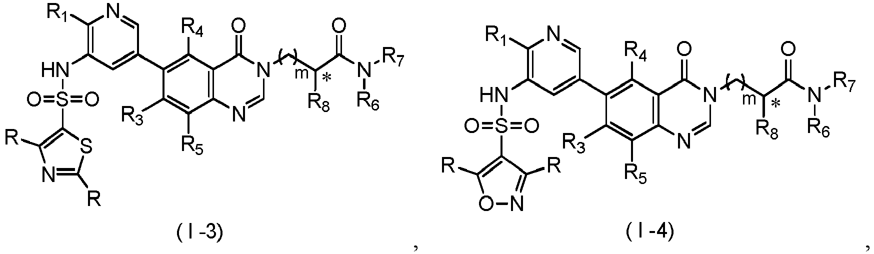 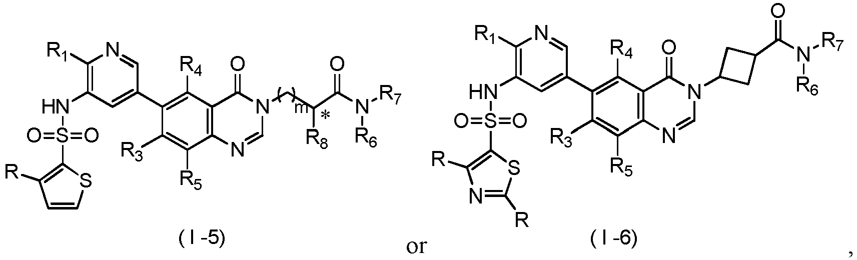 wherein,
n is 1, 2 or 3;
each m is independently 1, 2 or 3;
each Rs is independently selected from H, F, Cl, Br, I, OH, NH₂, C₁₋₃ alkyl or C₁₋₃ alkoxy, each of the C₁₋₃ alkyl and C₁₋₃ alkoxy is optionally substituted by one, two or three R;
R, R₁ and R₃ to R₇ are as defined in the present disclosure;
R₃, R₄, R₅, R₆ and R₇ are as defined in the present disclosure; and
when R₈ is not H, then the carbon atom with "*" is a chiral carbon atom, which exists in the form of a single enantiomer or enrich in one enantiomer of (*R*) or (*S*).

In some embodiments of the present disclosure, each R₈ is independently selected from H, F, Cl, Br, I, OH, NH₂, Me, Et, 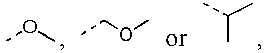 and other variables are as defined in the present disclosure.

In some embodiments of the present disclosure, the compound, the isomer thereof or the pharmaceutically acceptable salt thereof is 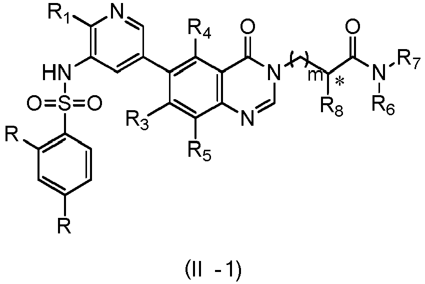 wherein,
m, R, R₁ and R₃ to R₈ are as defined in the present disclosure;
R₃, R₄, R₅, R₆, R₇ and R₈ are as defined in the present disclosure.

The present disclosure also provides a compound, an isomer thereof or a pharmaceutically acceptable salt thereof, which is selected from

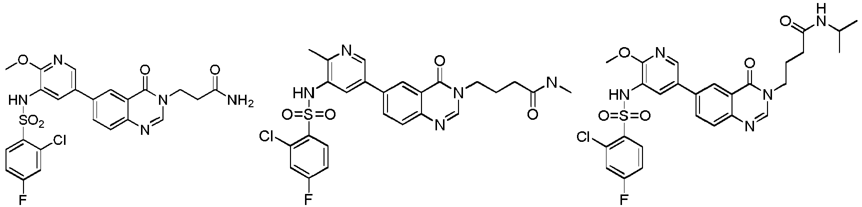

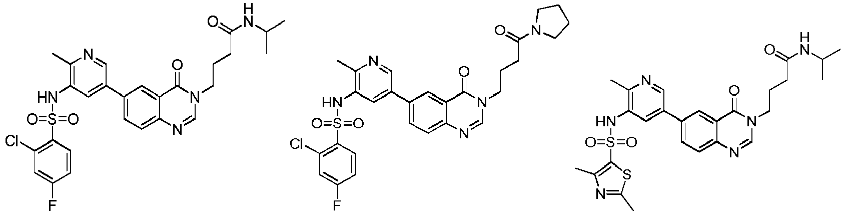

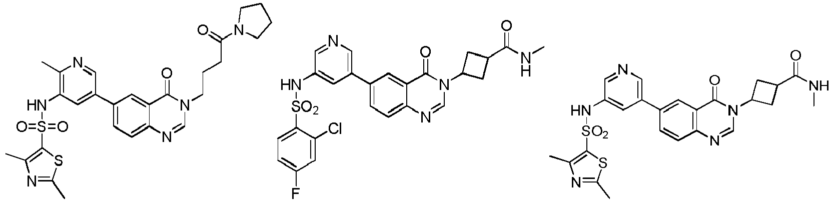

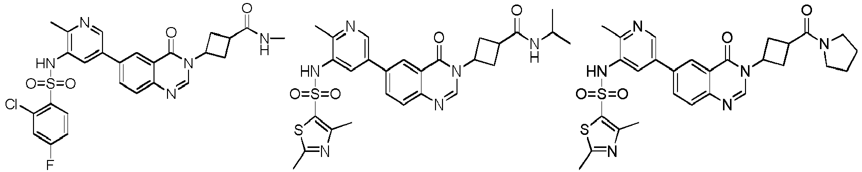

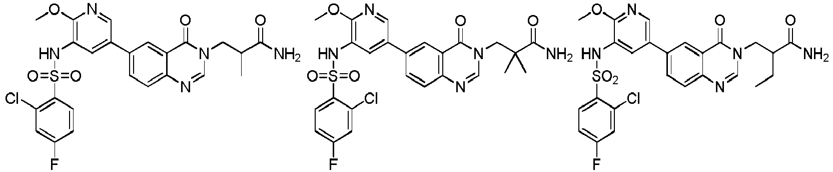

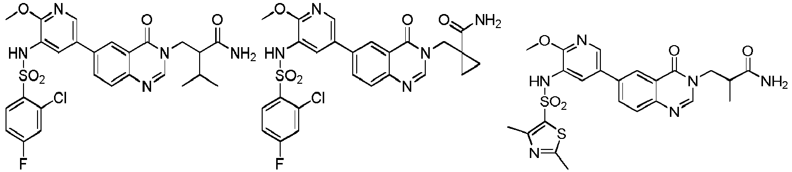

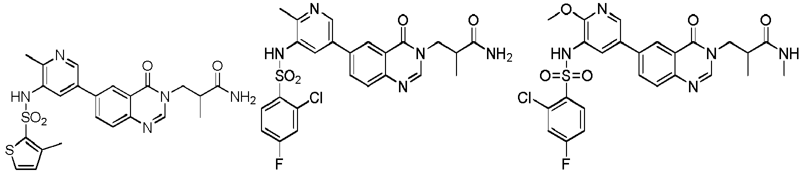

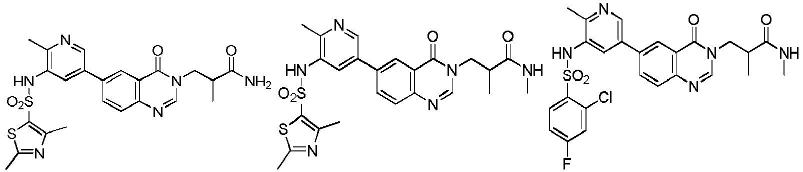

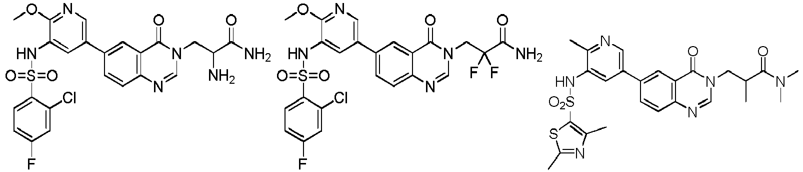

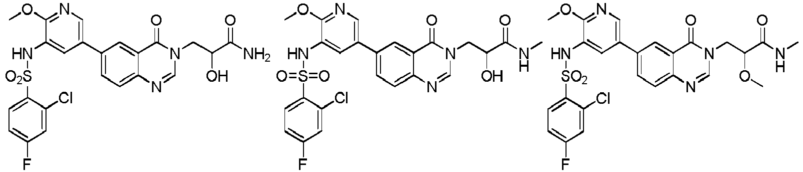

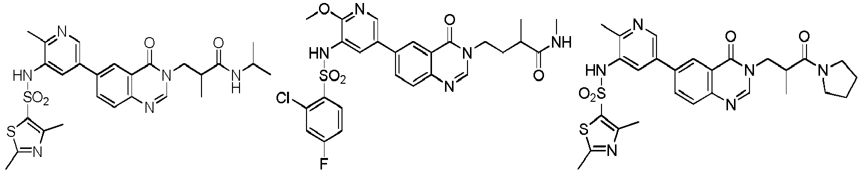

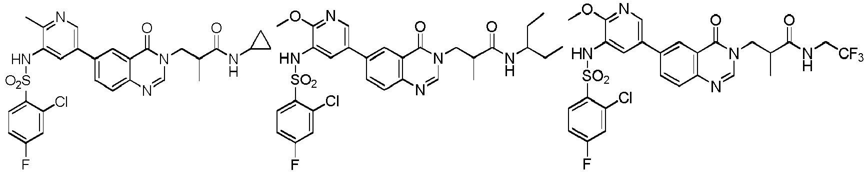

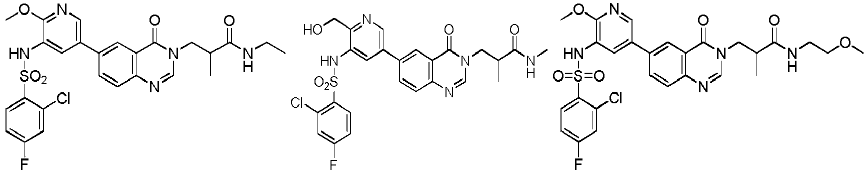

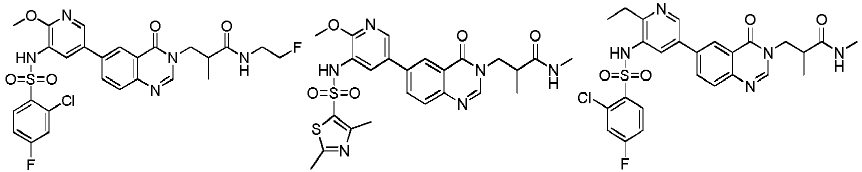

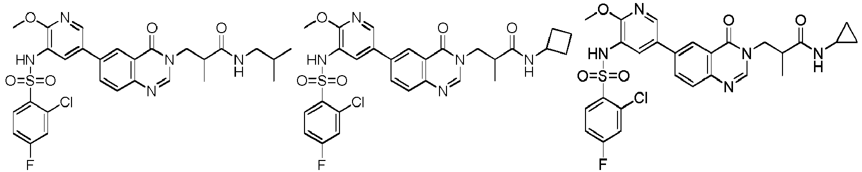

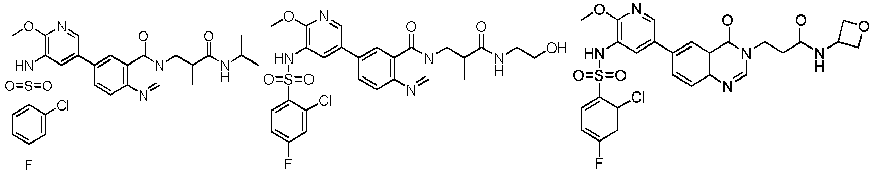

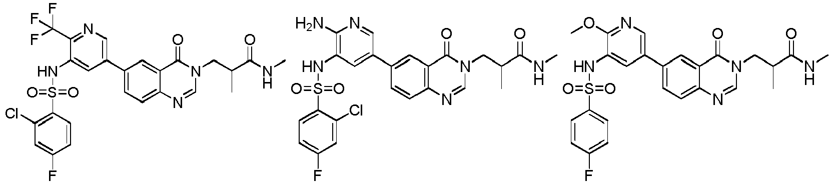

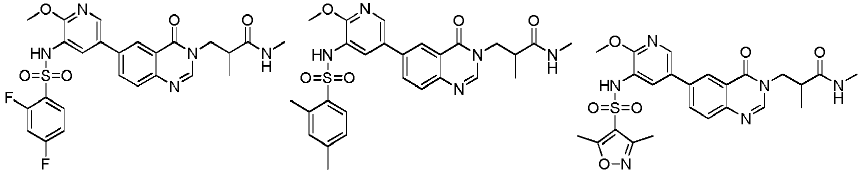

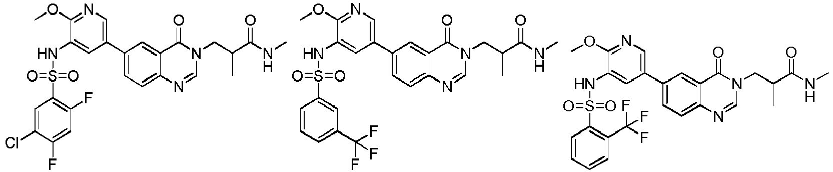

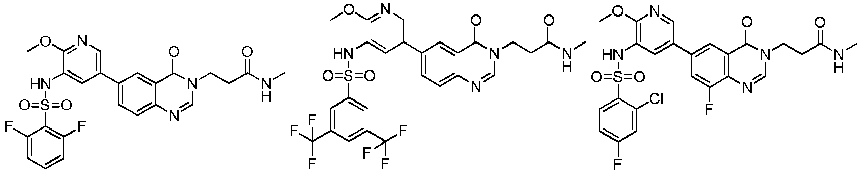

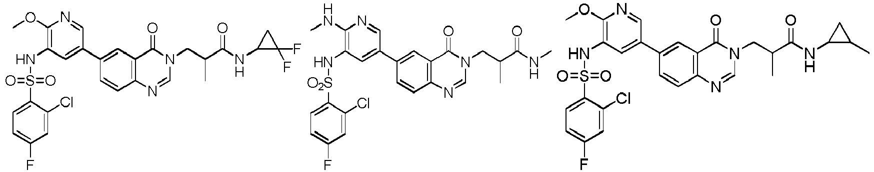

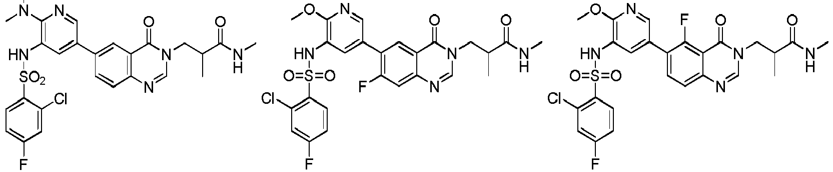

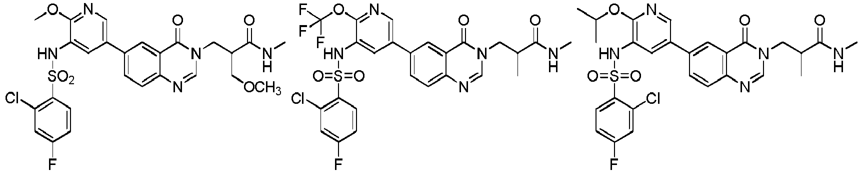

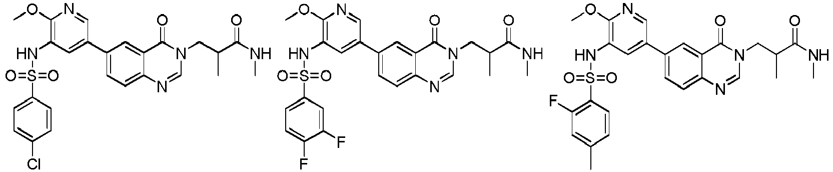

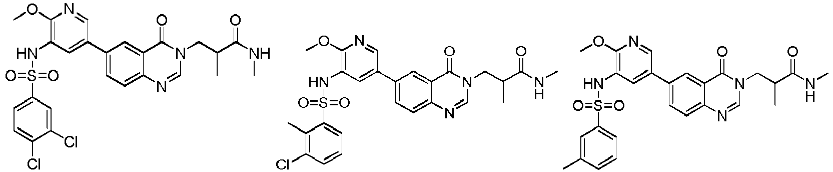

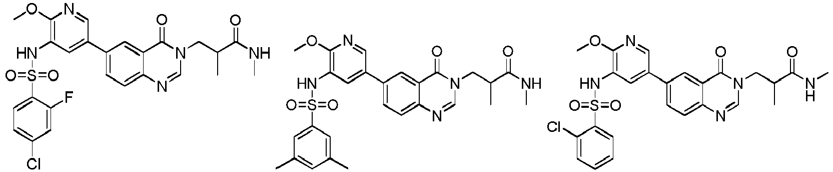

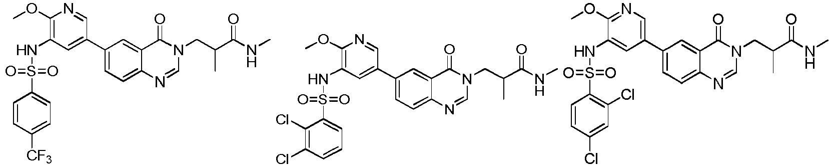

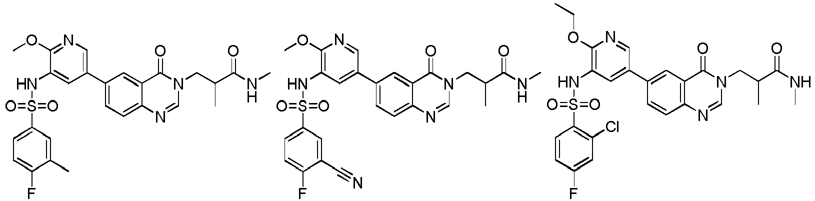

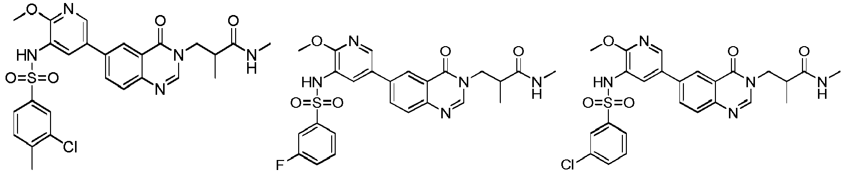

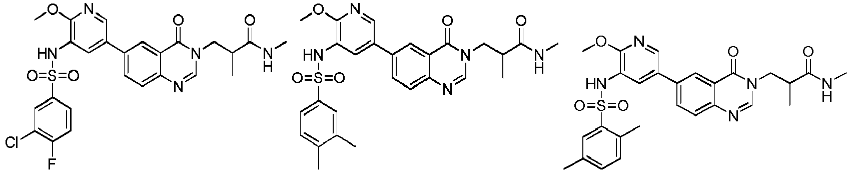

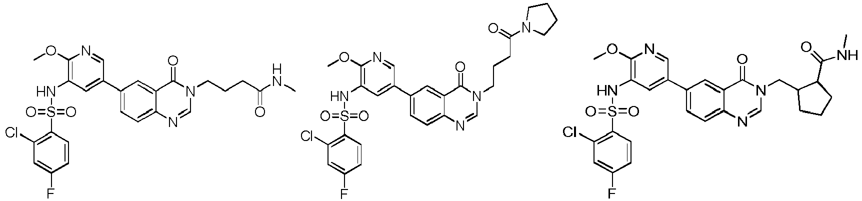

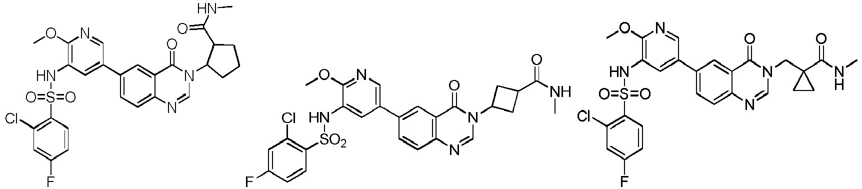

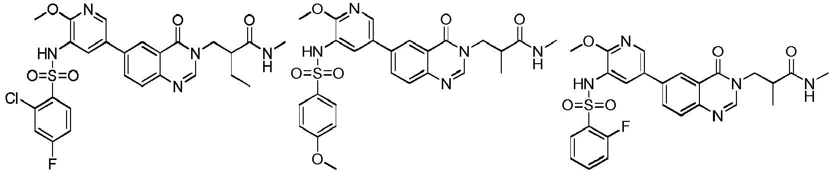

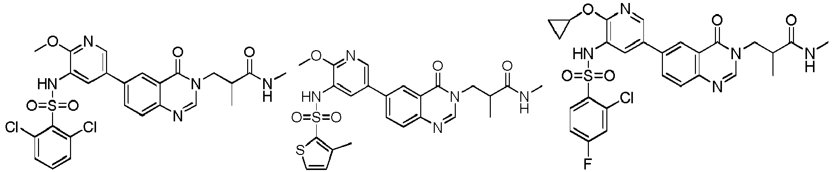

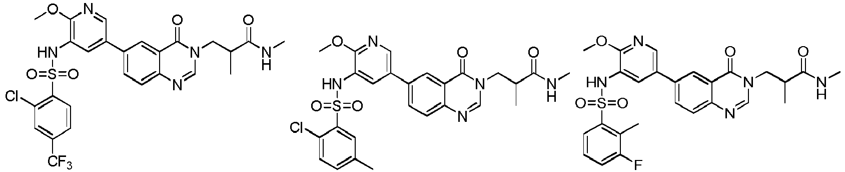

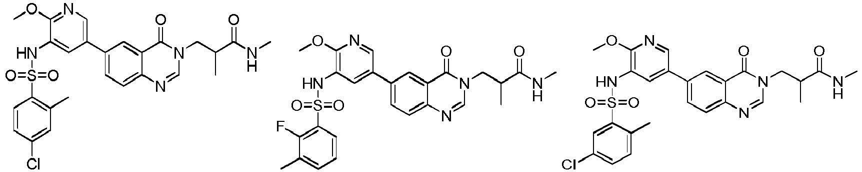

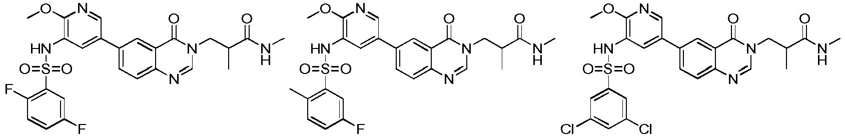

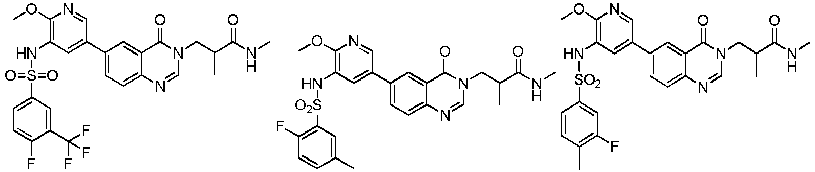

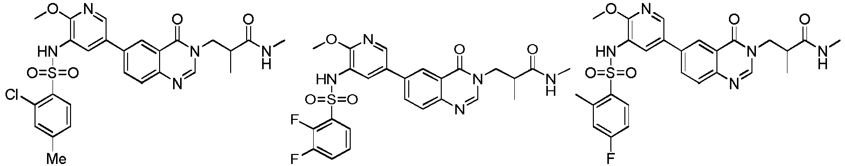

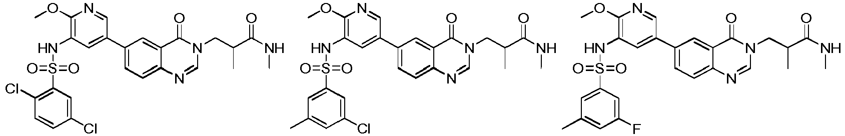

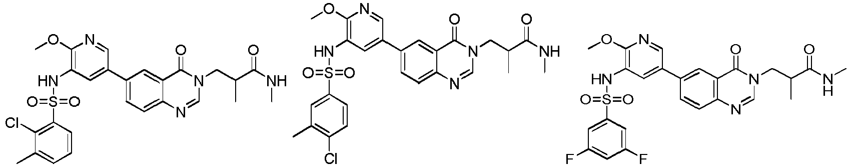

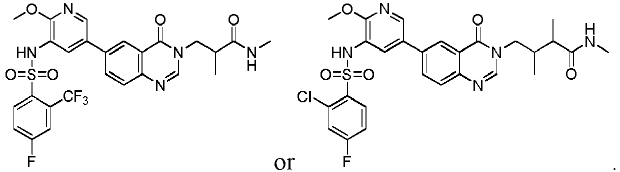

In some embodiments of the present disclosure, the compound, the isomer thereof or the pharmaceutically acceptable salt thereof is selected from 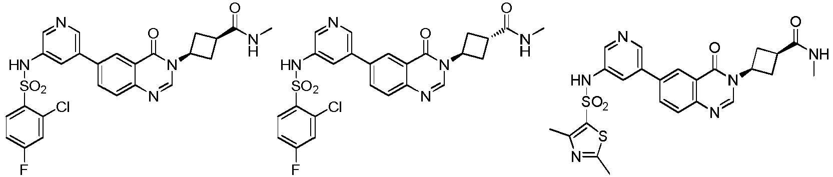 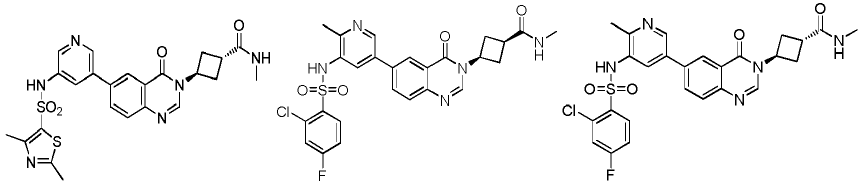 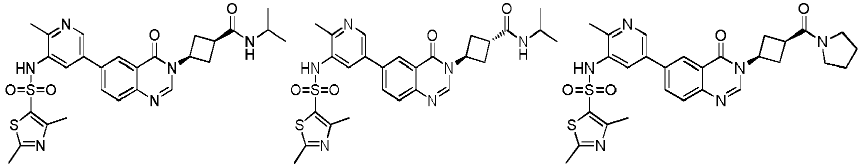 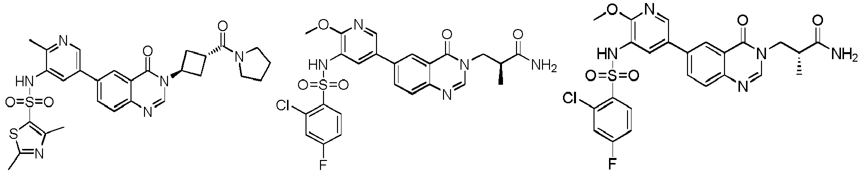 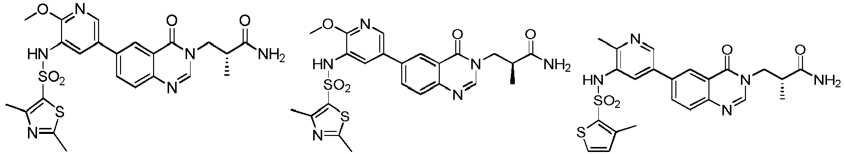 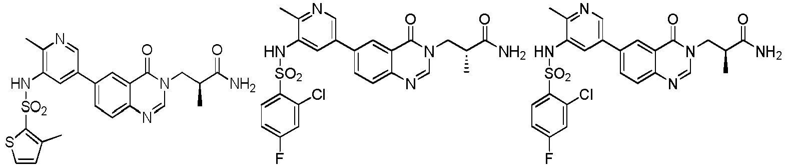 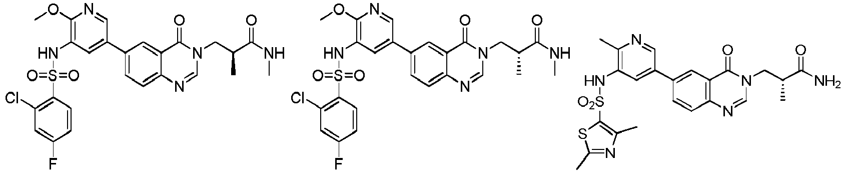 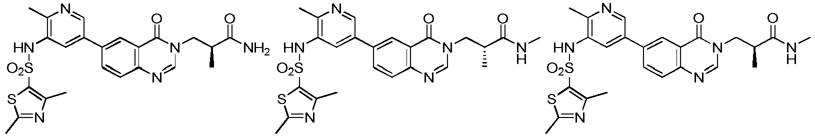 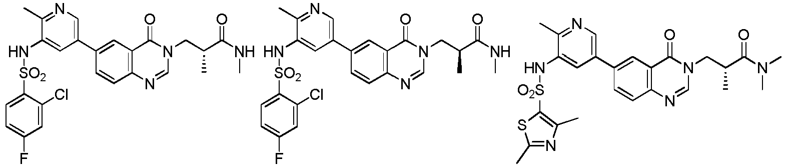 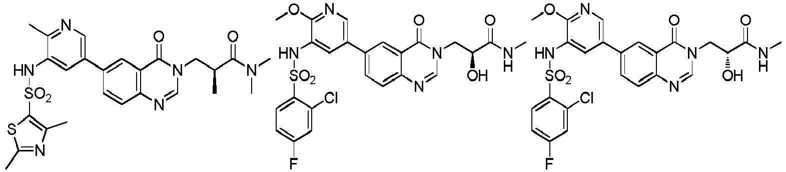 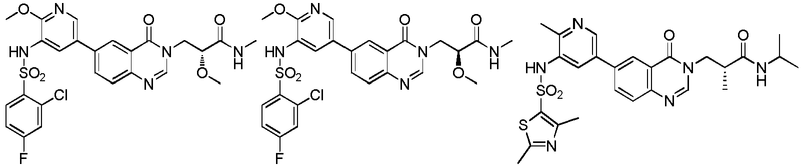 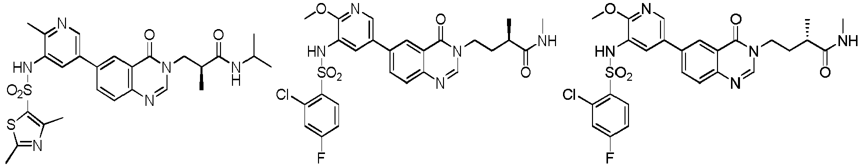 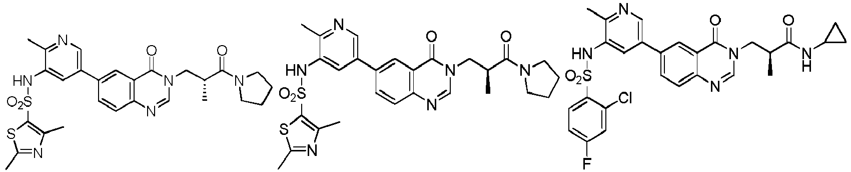 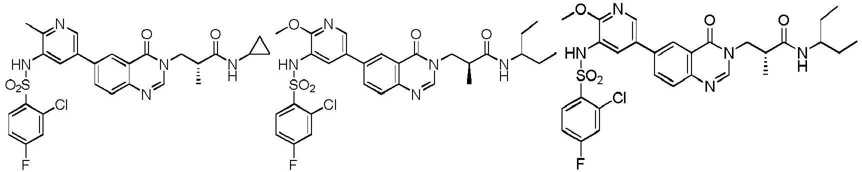 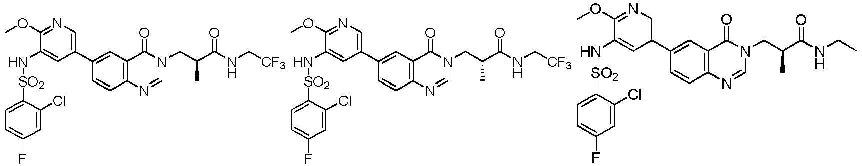 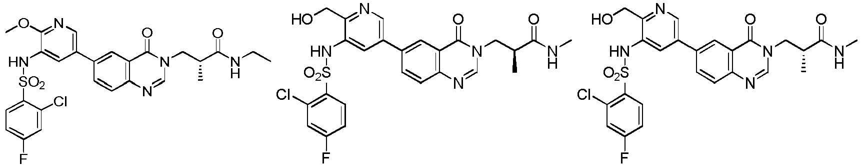 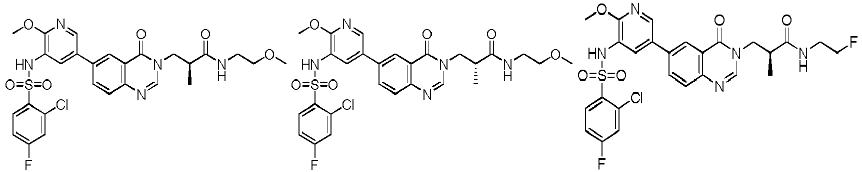 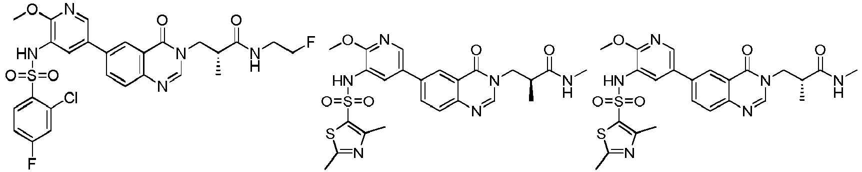 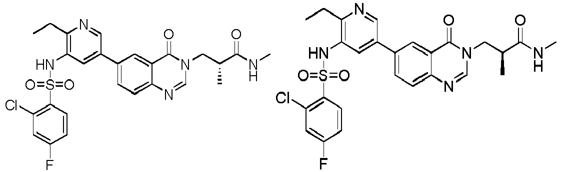 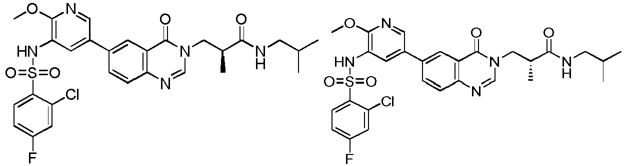 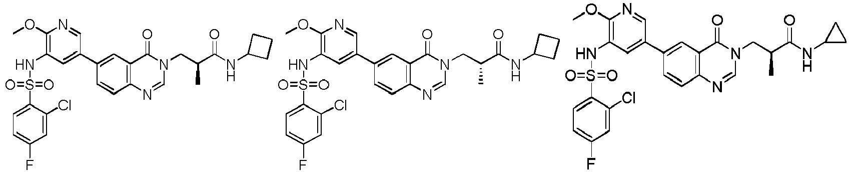 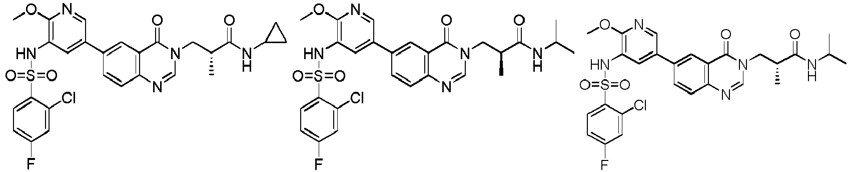 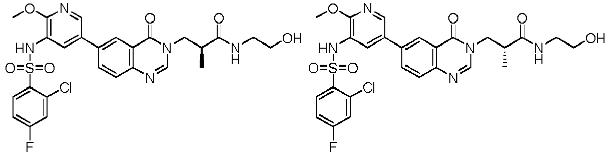 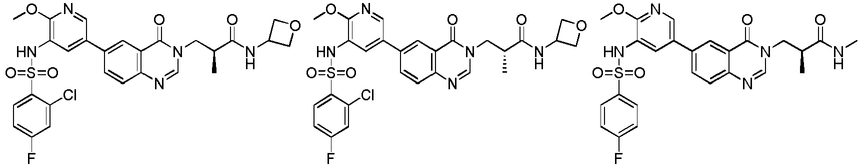 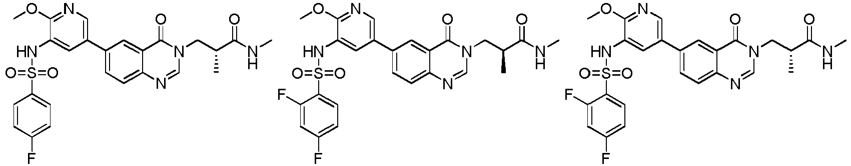 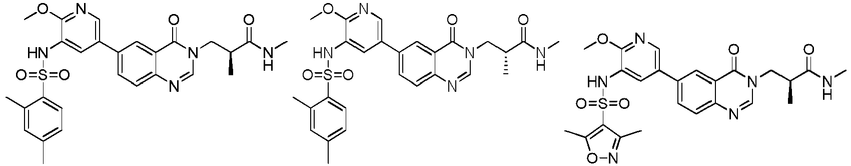 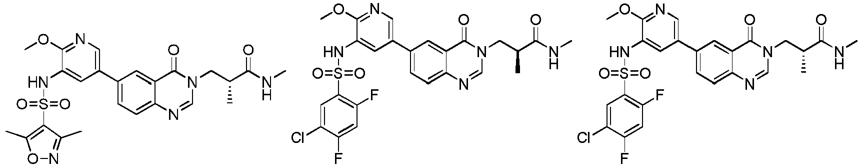 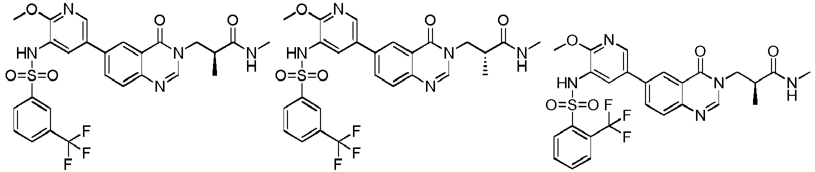 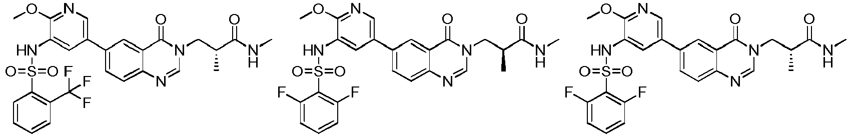 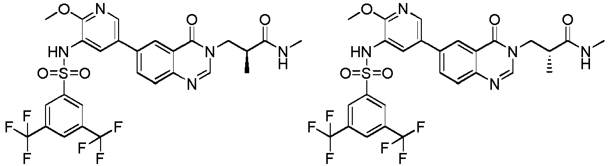 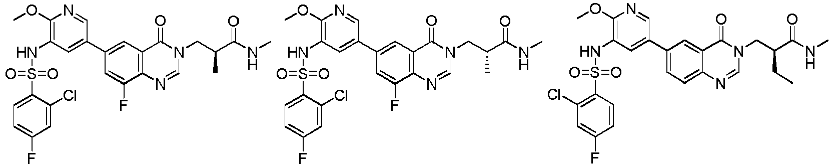 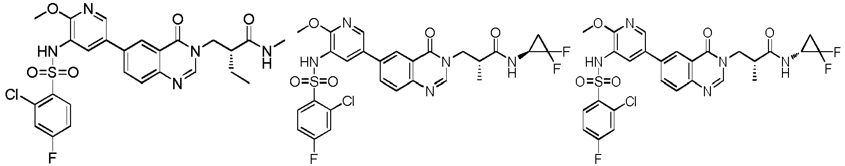 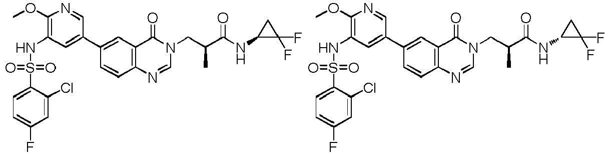 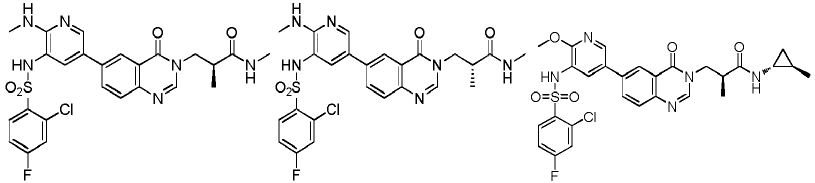 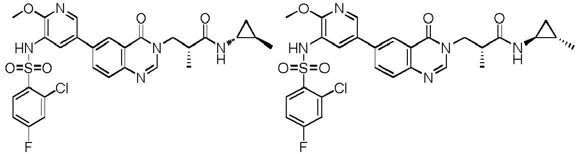 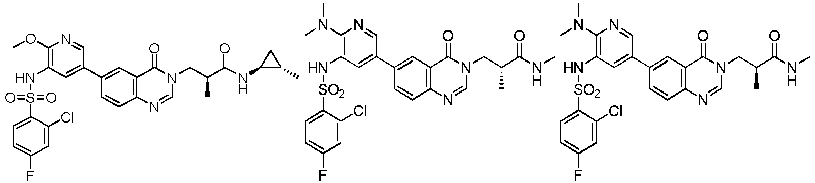 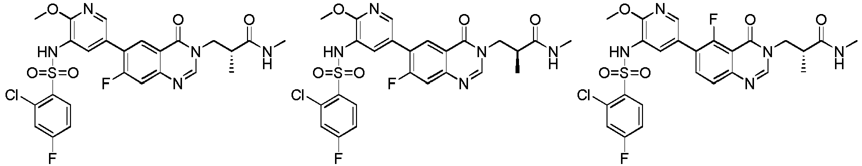 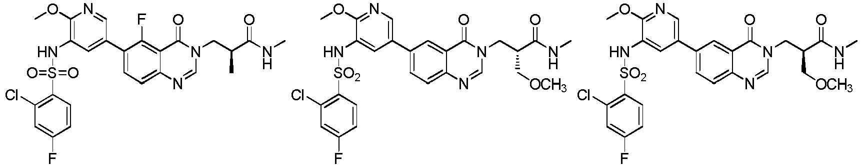 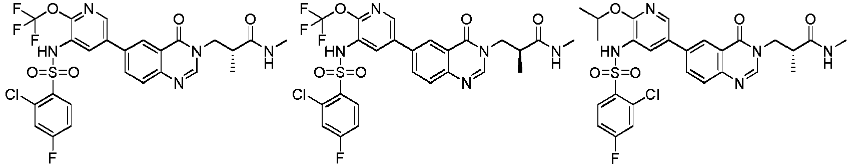 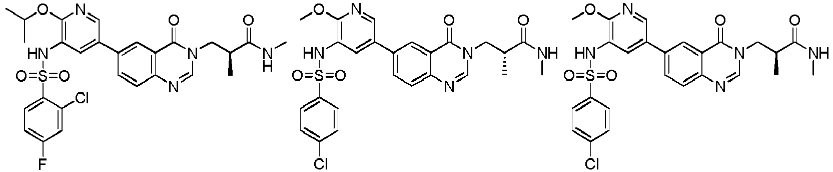 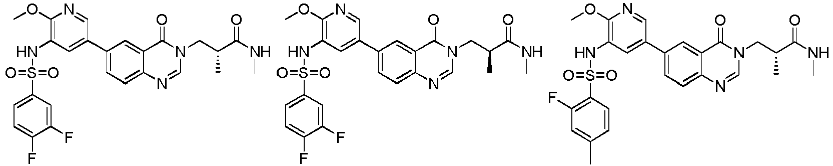 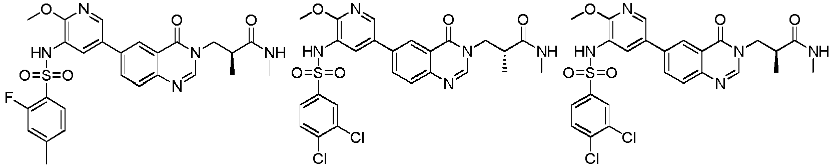 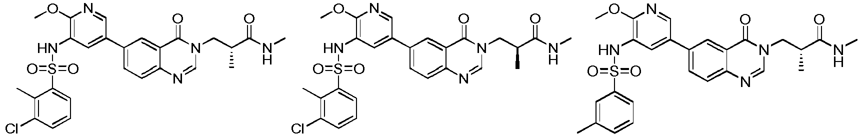 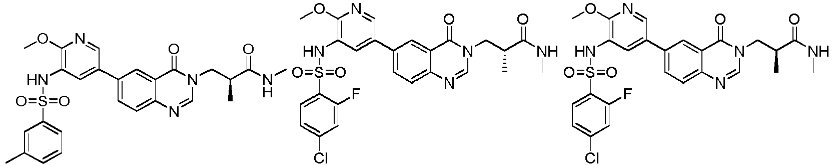 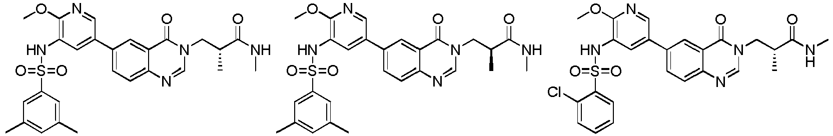 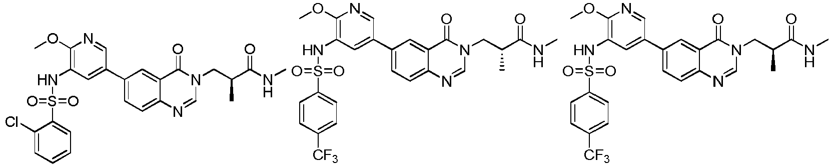 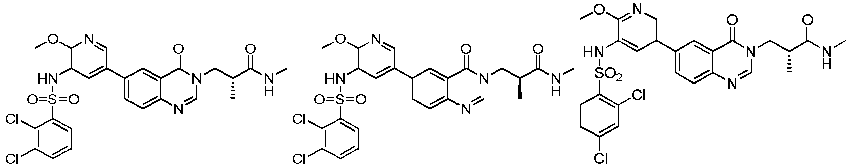 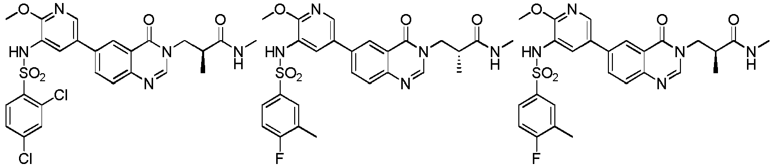 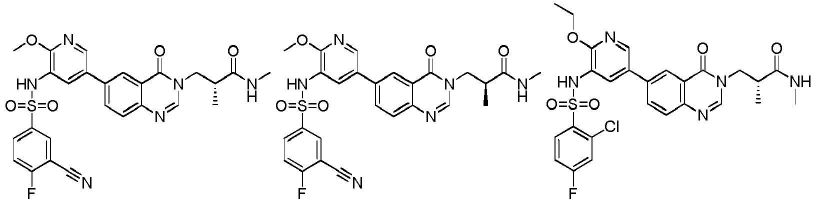 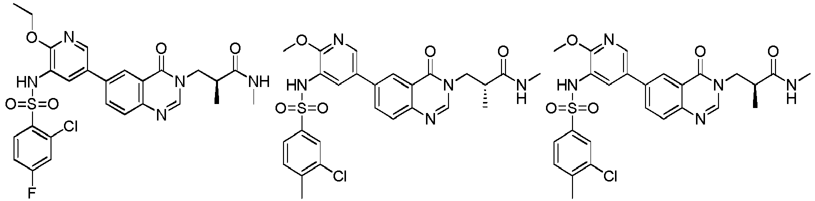 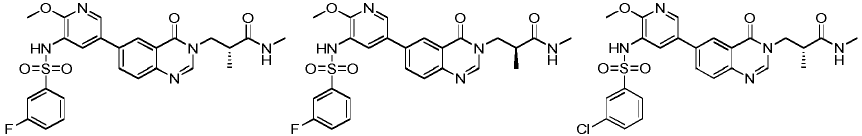 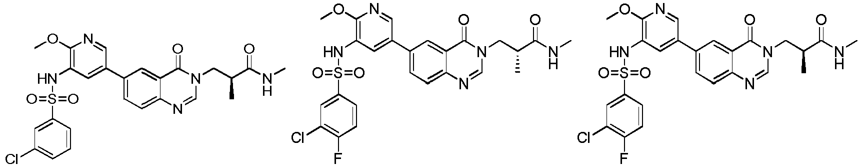 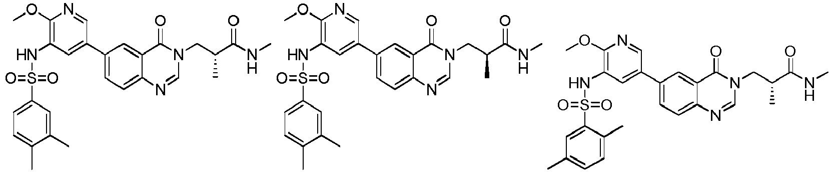 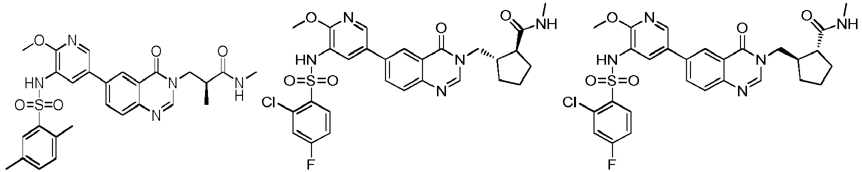 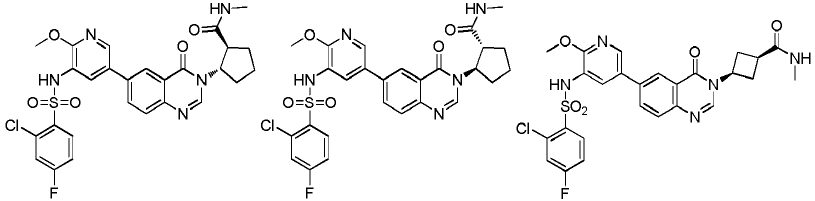 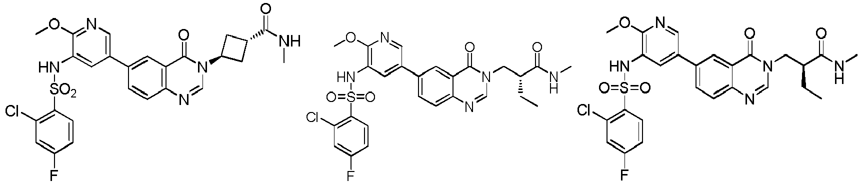 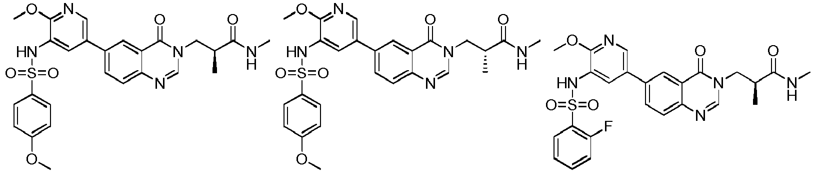 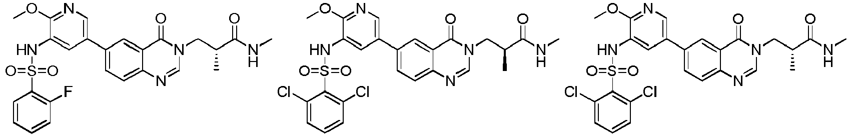 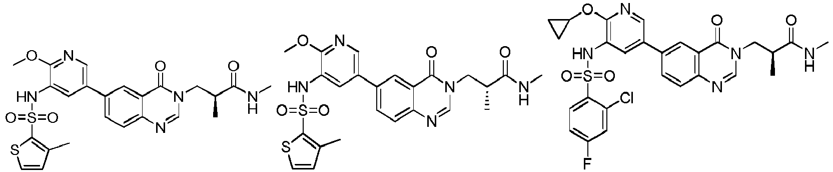 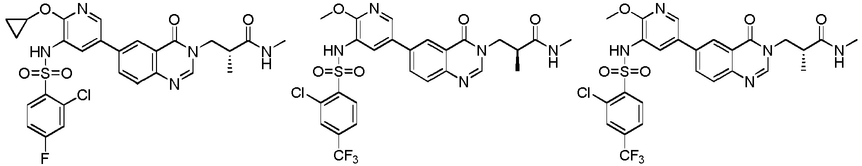 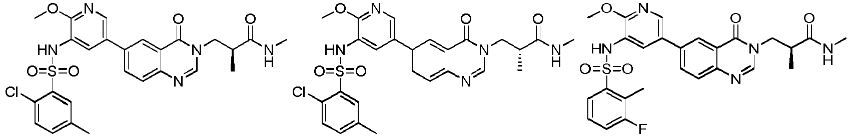 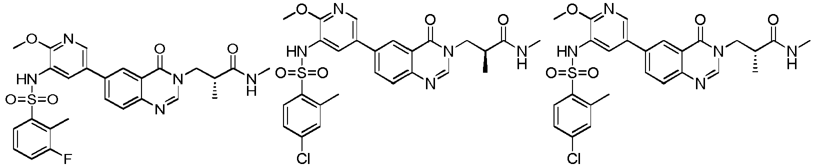 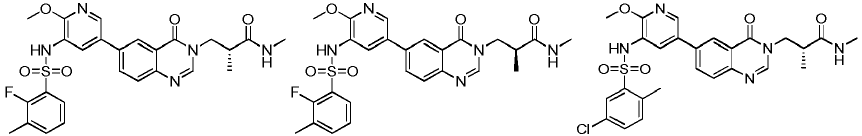 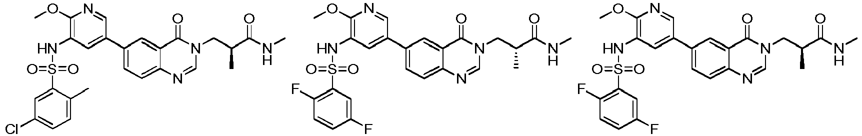 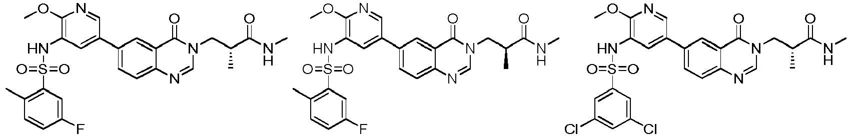 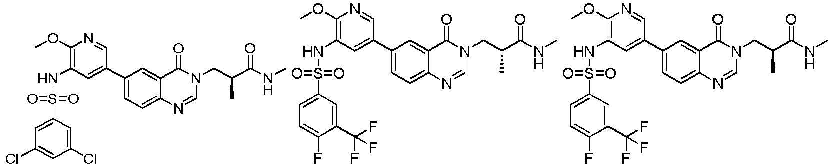 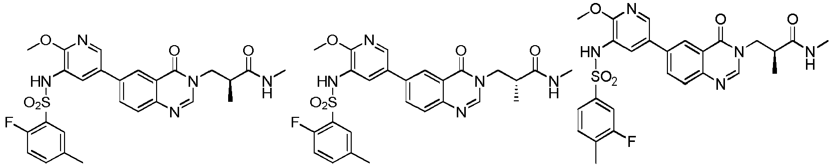 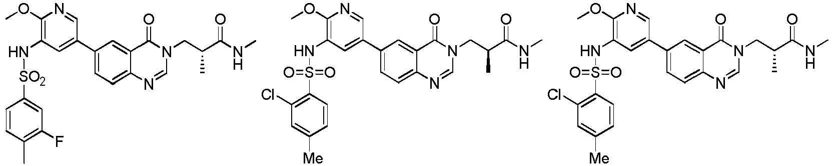 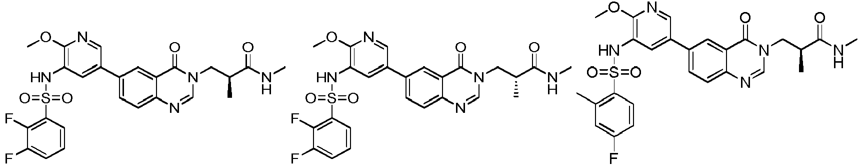 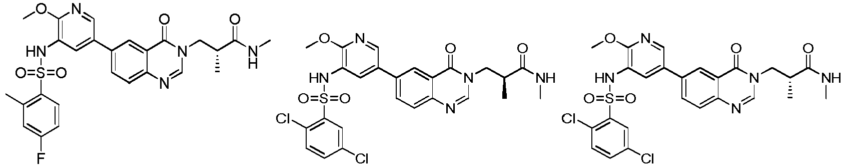 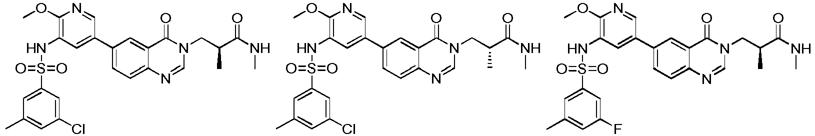 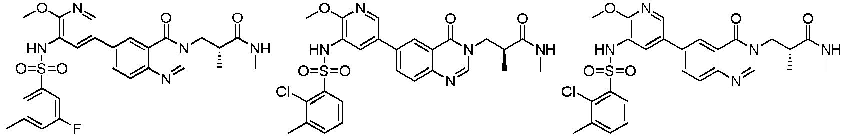 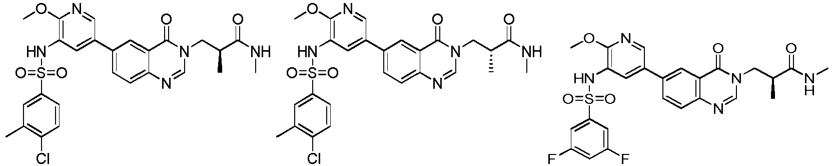 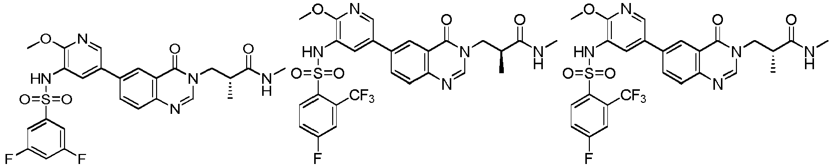 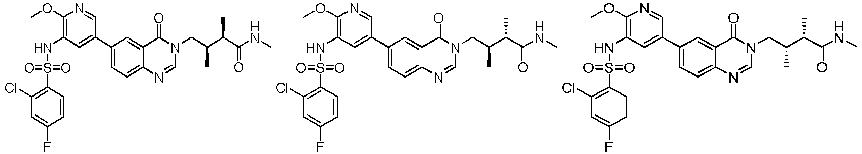 or

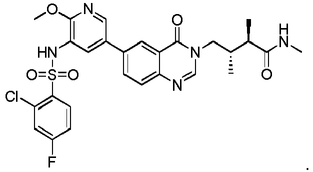

The present disclosure also provides a pharmaceutical composition, which comprises a therapeutically effective amount of the compound, the isomer thereof or the pharmaceutically acceptable salt thereof as an active ingredient, and a pharmaceutically acceptable carrier.

The present disclosure also provides a use of the compound, the isomer thereof or the pharmaceutically acceptable salt thereof or the pharmaceutical composition in manufacturing a PI3Kα inhibitor related medicament.

In some embodiments of the present disclosure, the PI3Kα inhibitor related medicament is a medicament for use in treating pain and pain-related disorders or a medicament for use in treating solid tumors.

### Definition and description

Unless otherwise indicated, the following terms and phrases used herein are intended to have the following meanings. A specific term or phrase should not be considered indefinite or unclear in the absence of a particular definition, but should be understood in the ordinary sense. When a trade name appears herein, it is intended to refer to its corresponding commodity or active ingredient thereof. The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, allergic reaction or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by bringing the neutral form of the compound into contact with a sufficient amount of base in a pure solution or a suitable inert solvent. The pharmaceutically acceptable base addition salt includes a salt of sodium, potassium, calcium, ammonium, organic amine or magnesium or similar salts. When the compound of the present disclosure contains a relatively basic functional group, an acid addition salt can be obtained by bringing the neutral form of the compound into contact with a sufficient amount of acid in a pure solution or a suitable inert solvent. Examples of the pharmaceutically acceptable acid addition salt include an inorganic acid salt, wherein the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid, and the like; and an organic acid salt, wherein the organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, *p*-toluenesulfonic acid, citric acid, tartaric acid, and methanesulfonic acid, and the like; and a salt of amino acid (such as arginine and the like), and a salt of an organic acid such as glucuronic acid and the like. Certain specific compounds of the present disclosure that contain both basic and acidic functional groups can be converted to any base or acid addition salt.

The pharmaceutically acceptable salt of the present disclosure can be prepared from the parent compound that contains an acidic or basic moiety by conventional chemical method. Generally, such salt can be prepared by reacting the free acid or base form of the compound with a stoichiometric amount of an appropriate base or acid in water or an organic solvent or a mixture thereof.

The compound of the present disclosure may exit specific geometric or stereoisomeric forms. The present disclosure contemplates all such compounds, including *cis-* and *trans*-isomers, (-)- and (+)-enantiomers, (*R*)- and (*S*)-enantiomers, diastereoisomers, (*D*)-isomers, (*L*)-isomers, and racemic mixture and other mixtures, for example, an enantiomer or diastereoisomer enriched mixtures, all of which are encompassed within the scope of the present disclosure. The substituents such as alkyl may have additional asymmetric carbon atoms. All these isomers and mixtures thereof are encompassed within the scope of the present disclosure.

Unless otherwise specified, the term "enantiomer" or "optical isomer" refers to stereoisomers that are mirror images of each other.

Unless otherwise specified, the term *"cis-trans* isomer" or "geometric isomer" is caused by the inability of a double bond or a single bond of carbon atoms on the ring to freely rotate.

Unless otherwise specified, the term "diastereomer" refers to stereoisomers in which the molecules have two or more chiral centers and are not mirror images of each other.

Unless otherwise specified, *"*(*D*)" or "(+)" stands for dextrorotation, "(*L*)" or "(-)" stands for levorotation, "(*DL*)" or "(±)" stands for racemization.

Unless otherwise specified, the absolute configuration of a stereogenic center is represented by a wedged solid bond ( 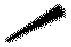 ) and a wedged dashed bond ( 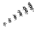 ), and the relative configuration of a stereogenic center is represented by a straight solid bond ( 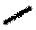 ) and a straight dashed bond ( 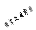 ). A wave line ( 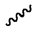 ) represents a wedged solid bond ( 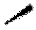 ) or a wedged dashed bond ( 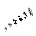 ), or represents a straight solid bond ( 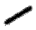 ) or a straight dashed bond ( 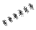 ).

The compounds of the disclosure may be present in particular. Unless otherwise indicated, the terms "tautomer" or "tautomeric form" refer to the fact that the isomers of different functional groups are in dynamic equilibrium at room temperature and can be rapidly converted into each other. If tautomers are possible (as in solution), the chemical equilibrium of the tautomers can be achieved. For example, proton tautomers (also known as prototropic tautomers) include interconversions by proton transfer, such as keto-enol isomerization and imine-enamine isomerization. The valence tautomer includes the mutual transformation of some bonding electrons. A specific example of keto-enol tautomerization is the interconversion between two tautomers of pentane-2,4-dione and 4-hydroxypent-3-en-2-one.

Unless otherwise specified, the terms "enriched in one isomer", "isomer enriched", "enriched in one enantiomer" or "enantiomer enriched" refer to the content of one of the isomers or enantiomers is less than 100%, and the content of the isomer or enantiomer is 60% or more, or 70% or more, or 80% or more, or 90% or more, or 95% or more, or 96% or more, or 97% or more, or 98% or more, or 99% or more, or 99.5% or more, or 99.6% or more, or 99.7% or more, or 99.8% or more, or 99.9% or more.

Unless otherwise specified, the terms "excess of isomer" or "excess of enantiomer" refers to the difference between the relative percentages of the two isomers or enantiomers. For example, wherein, the content of one of the isomers or enantiomers is 90%, and the other one is 10%, then the excess of isomer or enantiomer (ee value) is 80%.

Optically active (*R*)- and (*S*)-isomer, or *D* and *L* isomer can be prepared using chiral synthesis or chiral reagents or other conventional techniques. If one kind of enantiomer of certain compound of the present disclosure is to be obtained. The pure desired enantiomer can be obtained by asymmetric synthesis or derivative action of chiral auxiliary followed by separating the resulting diastereomeric mixture and cleaving the auxiliary group. Alternatively, when the molecule contains a basic functional group (such as amino) or an acidic functional group (such as carboxyl). The compound reacts with an appropriate optically active acid or base to form a salt of the diastereomeric isomer which is then subjected to diastereomeric resolution through the conventional method in the art to give the pure enantiomer. In addition, the enantiomer and the diastereoisomer are generally isolated through chromatography which uses a chiral stationary phase and optionally combines with a chemical derivative method (such as carbamate generated from amine). The compound of the present disclosure may contain an unnatural proportion of atomic isotope at one or more than one atom(s) that constitute the compound. For example, the compound can be radiolabeled with a radioactive isotope, such as tritium (³H), iodine-125 (¹²⁵I) or C-14 (¹⁴C). For another example, hydrogen can be replaced by heavy hydrogen to form a deuterated drug, and the bond composed of barium and carbon is stronger than the bond composed of common hydrogen and carbon. Compared with undeuterated drugs, deuterated drugs have reduced side effects and increased drug stability, enhanced the efficacy and prolonged the biological half-life of the drug. All isotopic variations of the compounds of the present disclosure, whether radioactive or not, are encompassed within the scope of the present disclosure. "Optional" or "optionally" means that the subsequent event or condition may occur but not requisite, that the term includes the instance in which the event or condition occurs and the instance in which the event or condition does not occur.

The term "substituted" means one or more than one hydrogen atom(s) on a specific atom are substituted with the substituent, including deuterium and hydrogen variants, as long as the valence of the specific atom is normal and the substituted compound is stable. When the substituent is an oxygen (i.e., =O), it means two hydrogen atoms are substituted. Positions on an aromatic ring cannot be substituted with a ketone. The term "optionally substituted" means an atom can be substituted by a substituent or not, unless otherwise specified. The type and number of the substituent may be arbitrary as long as being chemically achievable.

When any variable (such as R) occurs in the constitution or structure of the compound more than once, the definition of the variable at each occurrence is independent. Thus, for example, if a group is substituted with 0-2 R, the group can be optionally substituted with up to two R, wherein the definition of R at each occurrence is independent. Moreover, a combination of the substituent and/or the variant thereof is allowed only when the combination results in a stable compound.

When the number of a linking group is 0, such as -(CRR)₀-, it means that the linking group is a single bond.

When one of the variables is selected from a single bond, it means that the two groups linked by the single bond are connected directly. For example, when L in A-L-Z represents a single bond, the structure of A-L-Z is actually A-Z.

When a substituent is vacant, it means that the substituent does not exist. For example, when X is vacant in A-X, the structure of A-X is actually A. When the listed substituents are not indicated by which atom is attached to the substituted group, such a substituent may be bonded through any of its atoms, for example, the pyridyl group as a substituent may be bonded to the substituted group through any one of the carbon atoms on the pyridine ring. When the enumerative linking group does not indicate the direction for linking, the direction for linking is arbitrary, for example, the linking group L contained in 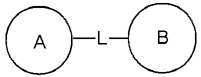 is -M-W-, then -M-W- can link ring A and ring B to form 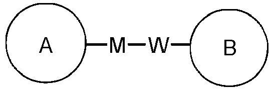 in the direction same as left-to-right reading order, and form 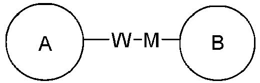 in the direction contrary to left-to-right reading order. Combinations of the linking groups, substituents and/or variants thereof are permissible only if such combinations result in stable compounds.

Unless otherwise specified, the term "hetero" represents heteroatoms or heteroatomic groups (e.g., atomic groups containing heteroatoms), including the atoms except carbon (C) and hydrogen (H) and the atomic groups containing the above heteroatoms, for example, including oxygen (O), nitrogen (N), sulfur (S), silicon (Si), germanium (Ge), aluminum (Al), boron (B), -O-, -S-, = O, = S, -C(=O)O-, -C(=O)-,-C(= S)-, -S(= O), -S(= O)₂-, and -C(=O)N(H)-, -N(H)-, -C(=NH)-, -S(=O)₂N(H)- and-S(=O)N(H)-, each of which is optionally substituted.

Unless otherwise specified, the term "ring" refers to a substituted or unsubstituted cycloalkyl, heterocycloalkyl, cycloalkenyl, heterocycloalkenyl, cycloalkynyl, heterocycloalkynyl, aryl or heteroaryl. The so called ring includes a single ring, a double ring, a spiral ring, a fused ring or a bridged ring. The number of the atom on the ring is usually defined as the member number of the ring, for example, a "5-7 membered ring" means that 5 to 7 atoms are arranged on a ring. Unless otherwise specified, the ring optionally contains 1 to 3 heteroatoms. Therefore, a "5-7 membered ring" includes, for example, phenyl, pyridinyl and piperidinyl; on the other hand. The term "5-7 membered heterocycloalkyl ring" includes pyridyl and piperidinyl, but excluding phenyl. The term "ring" also includes a ring system containing at least one ring, wherein each ring independently meets the above definition.

Unless otherwise specified, the term "heterocycle" or "heterocyclo" refers to a stable monocyclic, bicyclic or tricyclic ring containing a heteroatom or a heteroatom group, which can be saturated, partially unsaturated or unsaturated (aromatic) and can contain carbon atoms and 1, 2, 3 or 4 ring heteroatoms independently selected from N, O and S, wherein any of the above heterocycle can be fused to a benzene ring to form a bicyclic ring. Nitrogen and sulfur heteroatoms can optionally be oxidized (i.e., NO and S(O)ₚ, p is 1 or 2). Nitrogen atom can be substituted or unsubstituted (i.e., N or NR, wherein R is H or other substituents already defined herein). The heterocycle can be attached to the pendant group of any heteroatom or carbon atom to form a stable structure. If the resulting compound is stable, the heterocycle described herein may have a substitution at a carbon or nitrogen position. Nitrogen atom on the heterocycle is optionally quaternized. In a preferred embodiment, when the total number of S and O atom of the heterocycle is more than 1, the heteroatom is not adjacent to each other. In another preferred embodiment. The total number of S and O atom of the heterocycle is not more than 1. As used herein, the term "aromatic heterocyclic group" or "heteroaryl" refers to a stable 5-, 6- or 7-membered monocyclic or bicyclic or 7-, 8-, 9- or 10-membered bicyclic heterocyclic aromatic ring which contains carbon atoms and 1, 2, 3 or 4 ring heteroatoms independently selected from N, O and S. Nitrogen atom can be substituted or unsubstituted (i.e., N or NR, wherein R is H or other substituents already defined herein). Nitrogen and sulfur heteroatoms may optionally be oxidized (i.e., NO and S(O)ₚ, p is 1 or 2). It is worth noting that the total number of S and O atom of an aromatic heterocycle is not more than one. The bridged ring is also included in the definition of the heterocycle. A bridged ring is formed when one or more than one atom (i.e, C, O, N or S) link two non-adjacent carbon or nitrogen atoms. A preferred bridged ring includes, but not limited to one carbon atom. Two carbon atoms, one nitrogen atom, two nitrogen atoms and one carbon-nitrogen group. It is worth noting that a bridge always converts a monocyclic ring to a tricyclic ring. In a bridged ring, the substituent on the ring may also be present on the bridge.

Examples of the heterocyclic compound include, but are not limited to: acridinyl, azocinyl, benzimidazolyl, benzofuranyl, benzomercaptofuranyl, benzomercaptophenyl, benzoxazolyl, benzoxazolinyl, benzothiazolyl, benzotriazolyl, benzotetrazolyl, benzoisoxazolyl, benzoisothiazolyl, benzoimidazolinyl, carbazolyl, 4a*H*-carbazolyl, carbolinyl, chromanyl, chromene, cinnolinyl decahydroquinolinyl, 2*H*,6*H*-1,5,2-dithiazinyl, dihydrofuro[2,3-*b*]tetrahydrofuranyl, furanyl, furazanyl, imidazolidinyl, imidazolinyl, imidazolyl, 1*H*-indazolyl, indolenyl, indolinyl, indolizinyl, indolyl, 3*H*-indolyl, isobenzofuranyl, isoindolyl, isoindolinyl, isoquinolinyl, isothiazolyl, isoxazolyl, methylenedioxyphenyl, morpholinyl, naphthyridinyl, octahydro-isoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, hydroxindolyl, pyrimidinyl, phenanthridinyl, phenanthrolinyl, phenazine, phenothiazine, benzoxanthinyl, phenoloxazinyl, phthalazinyl, piperazinyl, piperidinyl, piperidonyl, 4-piperidonyl, piperonyl, pteridinyl, purinyl, pyranyl, pyrazinyl, pyrazolidinyl, pyrazolinyl, pyrazolyl, pyridazinyl, pyrido-oxazolyl, pyrido-imidazolyl, pyrido-thiazolyl, pyridinyl, pyrrolidinyl, pyrrolinyl, 2*H*-pyrrolyl, pyrrolyl, quinazolinyl, quinolinyl, 4*H*-quinolizinyl, quinoxalinyl, quinuclidinyl, tetrahydrofuranyl, tetrahydroisoquinolinyl, tetrahydroquinolinyl, tetrazolyl, 6*H*-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, isothiazolylthienyl, thieno-oxazolyl, thieno-thiazolyl, thieno-imidazolyl, thienyl, triazinyl, 1*H*-1,2,3-triazolyl, 2*H*-1,2,3-triazolyl, 1*H*-1,2,4-triazolyl, 4*H*-1,2,4-triazolyl and xanthenyl. Also included are fused-ring compounds and spiro compounds.

Unless otherwise specified, the term "hydrocarbyl" or its hyponyms (e.g., alkyl, alkenyl, alkynyl, and aryl, etc.), by itself or as part of another substituent, refers to a linear, branched chain or cyclic hydrocarbon radical or any combination thereof, they can be fully saturated (e.g., alkyl), mono- or polyunsaturated (e.g., alkenyl, alkynyl, and aryl), can be mono-, di- or poly-substituted, can be monovalent (e.g., methyl), divalent (e.g., methylene) or multivalent (e.g., methenyl), can also include a divalent or multivalent group, have a specified number of carbon atom (for example, C₁-C₁₂ indicates 1 to 12 carbon atoms, C₁₋₁₂ is selected from C₁, C₂, C₃, C₄, C₅, C₆, C₇, C₈, C₉, C₁₀, C₁₁ and C₁₂; C₃₋₁₂ is selected from C₃, C₄, C₅, C₆, C₇, C₈, C₉, C₁₀, C₁₁ and C₁₂). The term "hydrocarbyl" includes, but is not limited to aliphatic hydrocarbyl and aromatic hydrocarbyl, the aliphatic hydrocarbyl includes linear and cyclic hydrocarbyl, specifically includes but not limited to alkyl, alkenyl, and alkynyl. The aromatic hydrocarbyl includes but is not limited to 6-12 membered aromatic hydrocarbyl such as phenyl, naphthyl and the like. In some embodiments, the term "hydrocarbyl" refers to a linear or branched group or a combination thereof which can be fully saturated, mono- or polyunsaturated, and can include a divalent or multivalent group. Examples of the saturated hydrocarbyl group include, but are not limited to, methyl, ethyl, *n-*propyl, isopropyl, *n*-butyl, *tert*-butyl, isobutyl, *sec*-butyl, cyclohexyl, (cyclohexyl)methyl, cyclopropylmethyl, and the homolog or isomer of *n*-amyl, *n*-hexyl, *n*-heptyl, *n*-octyl and other atom groups. The unsaturated hydrocarbyl has one or more than one double or triple bonds. Examples of the unsaturated alkyl include but are not limited to, vinyl, 2-propenyl, butenyl, crotyl, 2-isopentenyl, 2-(butadienyl), 2,4-pentadienyl, 3-(1,4-pentadienyl), ethynyl, 1- and 3-propynyl, 3-butynyl, and more higher homologs and isomers.

Unless otherwise specified, the term "heterohydrocarbyl" or its hyponyms (such as heteroalkyl, heteroalkenyl, heteroalkynyl, and heteroaryl, etc.), by itself or as part of another substituent, refers to a stable linear, branched or cyclic hydrocarbon group or any combination thereof, which has a specified number of carbon atoms and at least one heteroatom. In some embodiments, the term "heteroalkyl" by itself or in combination with another term refers to a stable linear chain, branched hydrocarbon radical or a combination thereof which has a specified number of carbon atoms and at least one heteroatom. In a specific embodiment, a heteroatom is selected from B, O, N and S, wherein nitrogen and sulfur atoms are optionally oxidized and the nitrogen atom is optionally quaternized. The heteroatom or heteroatom group can be located at any interior position of a heterohydrocarbyl, including the position where the hydrocarbyl attaches to the rest part of the molecule. But the terms "alkoxy", "alkylamino" and "alkylthio" (or thioalkyl) are used by the conventional meaning and refer to an alkyl group connected to the rest part of the molecule via an oxygen atom, an amino or a sulfur atom respectively. Examples include, but are not limited to,-CH₂-CH₂-O-CH₃, -CH₂-CH₂-NH-CH₃, -CH₂-CH₂-N(CH₃)-CH₃, -CH₂-S-CH₂-CH₃,-CH₂-CH₂, -S(O)-CH₃, -CH₂-CH₂-S(O)₂-CH₃, -CH=CH-O-CH₃, -CH₂-CH=N-OCH₃ and -CH=CH-N(CH₃)-CH₃. Up to two consecutive heteroatoms can be present, such as, -CH₂-NH-OCH₃.

Unless otherwise specified, the term "cyclohydrocarbyl", "heterocyclohydrocarbyl" or its hyponyms (such as aryl, heteroaryl, cycloalkyl, heterocycloalkyl, cycloalkenyl, heterocycloalkenyl, cycloalkynyl, heterocycloalkynyl, etc.) by itself or in combination with another term refers to cyclized "hydrocarbyl" or "heterohydrocarbyl". Furthermore, for heterohydrocarbyl or heterocyclohydrocarbyl (e.g., heteroalkyl, and heterocycloalkyl), one heteroatom can occupy the position where the heterocycle attaches to the remainder position of the molecule. Examples of the cycloalkyl include, but are not limited to, cyclopentyl, cyclohexyl, 1-cyclohexenyl, 3-cyclohexenyl, cycloheptyl and the like. Non-limiting examples of heterocycloalkyl include 1-(1,2,5,6-tetrahydropyridyl), 1-piperidinyl, 2-piperidinyl, 3-piperidinyl, 4-morpholinyl, 3-morpholinyl, tetrahydrofuran-2-yl, tetrahydrofuran-3-yl, tetrahydro-thiophen-2-yl, tetrahydro-thiophen-3-yl, 1-piperazinyl and 2-piperazinyl.

Unless otherwise specified, the term "heterocycloalkyl" by itself or in combination with another term refers to cyclized "heteroalkyl". Furthermore, for heterocycloalkyl, one heteroatom can occupy the position where the heterocycle attaches to the remainder position of the molecule. In some embodiments, the heterocycloalkyl is 4-6 membered heterocycloalkyl; in other embodiments, the heterocycloalkyl is 5-6 membered heterocycloalkane. Examples of heterocycloalkyl include, but are not limited to, azetidinyl, oxetanyl, thiatanyl, pyrrolidinyl, pyrazolidinyl, imidazolidinyl, tetrahydrothienyl, tetrahydrofuryl, tetrahydropyranyl, piperidinyl, piperazinyl, morpholinyl, dioxanyl, dithianyl, isoxazolidinyl, isothiazolidinyl, 1,2-oxazinyl, 1,2-thiazinyl, hexahydropyridazinyl, homopiperazinyl, homopiperidinyl or oxetanyl.

Unless otherwise specified, the term "alkyl" refers to a linear chain or branched saturated hydrocarbon group, can be mono-substituted (e.g., -CH₂F) or poly-substituted (e.g., -CF₃), can be monovalent (e.g., methyl), divalent (e.g., methylene) or multivalent (e.g., methenyl). Examples of alkyl include methyl (Me), ethyl (Et), propyl (such as *n*-propyl and isopropyl), butyl (such as *n*-butyl, isobutyl, *s*-butyl, *t*-butyl), pentyl (such as *n*-pentyl, isopentyl, neopentyl) and the like.

Unless otherwise specified, cycloalkyl includes any stable cyclic or polycyclic hydrocarbyl, and any carbon atom is saturated, can be mono-substituted or poly-substituted, and can be monovalent, divalent or multivalent. Examples of cycloalkyl include, but are not limited to, cyclopropyl, norbornanyl, [2.2.2]bicyclooctane, [4.4.0]bicyclodecanyl and the like.

Unless otherwise specified, the term "halo" or "halogen" by itself or as part of another substituent refers to fluorine, chlorine, bromine or iodine atom. Furthermore, the term "haloalkyl" is meant to include monohaloalkyl and polyhaloalkyl. For example, the term "halo(C₁-C₄) alkyl" is meant to include, but not limited to, trifluoromethyl, 2,2,2-trifluoroethyl, 4-chlorobutyl, 3-bromopropyl and the like. Unless otherwise specified, examples of haloalkyl include, but not limited to trifluoromethyl, trichloromethyl, pentafluoroethyl and pentachloroethyl.

The term "alkoxy" represents any alkyl defined above having a specified number of carbon atoms attached by an oxygen bridge. Unless otherwise specified, C₁₋₆ alkoxy includes C₁, C₂, C₃, C₄, C₅ and C₆ alkoxy. Examples of alkoxy include, but not limited to methoxy, ethoxy, *n*-propoxy, isopropoxy, *n*-butoxy, *sec*-butoxy, *tert-*butoxy, *n*-pentyloxy and *S*-pentoxy.

Unless otherwise specified, the term "aryl" refers to a polyunsaturated aromatic substituent, can be mono-, di- or poly-substituted, can be a monovalent, divalent or multivalent, can be a single ring or a multiple ring (e.g., one to three rings; wherein at least one ring is aromatic), which are fused together or connected covalently. The term "heteroaryl" refers to an aryl (or ring) containing one to four heteroatoms. In an illustrative example, the heteroatom is selected from B, O, N and S, wherein nitrogen and sulfur atoms are optionally oxidized and nitrogen atom is optionally quaternized. A heteroaryl may attach to the rest part of a molecule via a heteroatom. Non-limiting examples of aryl or heteroaryl include phenyl, naphthyl, biphenyl, pyrrolyl, pyrazolyl, imidazolyl, pyrazinyl, oxazolyl, phenyl-oxazolyl, isoxazolyl, thiazolyl, furanyl, thienyl, pyridyl, pyrimidinyl benzothiazolyl, purinyl, benzimidazolyl, indolyl, isoquinolyl, quinoxalinyl, quinolyl, 1-naphthyl, 2-naphthyl, 4-biphenyl, 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, 4-pyridyl, 2-pyrimidyl, 4-pyrimidyl, 5-benzothiazolyl, purinyl, 2-benzimidazolyl, 5-indolyl, 1-isoquinolyl, 5-isoquinolyl, 2-quinoxalinyl, 5-quinoxalinyl, 3-quinolyl and 6-quinolyl. The substituent of any of the above aryl and heteroaryl ring system is selected from the acceptable substituent described below.

Unless otherwise specified, when aryl combines with other terms (such as aryloxy, arylthio, arylalkyl), the aryl includes the aryl and heteroaryl ring as defined above. Thus, the term "aralkyl" is meant to include the group (e.g., benzyl, phenethyl, pyridylmethyl, etc.) where an aryl is attached to an alkyl, including an alkyl where the carbon atom (e.g, methylene) has been replaced by an atom such as oxygen, for example, phenoxymethyl, 2-pyridyloxy, 3-(1-naphthyloxy)propyl, and the like.

The term "leaving group" refers to a functional group or atom which can be replaced by another functional group or atom through a substitution reaction (such as affinity substitution reaction). For example, representative leaving groups include triflate; chlorine, bromine and iodine; sulfonate group, such as mesylate, tosylate, p-bromobenzenesulfonate, p-toluenesulfonates and the like; acyloxy, such as acetoxy, trifluoroacetoxy and the like.

The term "protecting group" includes, but is not limited to "amino protecting group", "hydroxy protecting group" or "thio protecting group". The term "amino protecting group" refers to a protecting group suitable for blocking the side reaction on the nitrogen of an amino. Representative amino protecting groups include, but are not limited to: formyl; acyl, such as alkanoyl (e.g, acetyl, trichloroacetyl or trifluoroacetyl); alkoxycarbonyl, such as *tert*-butoxycarbonyl (Boc); arylmethoxycarbonyl such as benzyloxycarbonyl (Cbz) and 9-fluorenylmethoxycarbonyl (Fmoc); arylmethyl such as benzyl (Bn), trityl (Tr), 1,1-bis-(4'-methoxyphenyl)methyl; silyl such as trimethylsilyl (TMS) and tert-butyldimethylsilyl (TBS) and the like. The term "hydroxy protecting group" refers to a protecting group suitable for blocking the side reaction on hydroxy. Representative hydroxy protecting groups include, but are not limited to: alkyl such as methyl, ethyl and *tert*-butyl; acyl such as alkanoyl (e.g, acetyl); arylmethyl such as benzyl (Bn), *p*-methoxybenzyl (PMB), 9-fluorenylmethyl (Fm), and diphenylmethyl (benzhydryl, DPM); silyl such as trimethylsilyl (TMS) and *tert*-butyl dimethyl silyl (TBS) and the like.

The compounds of the present disclosure can be prepared by a variety of synthetic methods well known to the skilled in the art, including the following enumerative embodiments, the embodiments formed by the following enumerative embodiment in combination with other chemical synthesis methods and the equivalent replacement well known to the skilled in the art. The preferred embodiments include, but are not limited to the embodiments of the present disclosure.

The compounds of the present disclosure may have multiple uses or indications, including but not limited to the specific uses or indications listed in this application.

The solvent used in the present disclosure is commercially available. The present disclosure employs the following abbreviations: aq stands for water; HATU stands for *O*-(7-azabenzotriazol-1-yl)-*N,N,N'N*'-tetramethyluronium hexafluorophosphate ; EDC stands for *N*-(3-dimethylaminopropyl)-*N'-*ethylcarbodiimide hydrochloride; *m*-CPBA stands for 3-chloroperoxybenzoic acid; eq stands for equivalent; CDI stands for carbonyldiimidazole; DCM stands for dichloromethane; PE stands for petroleum ether; DIAD stands for diisopropyl azodicarboxylate; DMF stands for *N,N*-dimethylformamide; DMSO stands for dimethyl sulfoxide; EtOAc stands for ethyl acetate; EtOH stands for ethanol; MeOH for methanol; CBz stands for benzyloxycarbonyl, which is an amine protecting group; BOC stands for *tert*-butoxycarbonyl, which is an amine protecting group; HOAc stands for acetic acid; NaCNBH3 stands for sodium cyanoborohydride; r.t stands for room temperature; O/N stands for overnight; THF stands for tetrahydrofuran; BoC₂O stands for di-*tert*-butyldicarbonate; TFA stands for trifluoroacetic acid; DIPEA stands for diisopropylethylamine; SOCl2 stands for thionyl chloride; CS₂ stands for carbon disulfide; TsOH stands for *p*-toluenesulfonic acid; NFSI stands for *N*-fluoro-*N-*(phenylsulfonyl)benzenesulfonamide; NCS stands for *N*-chlorosuccinimide; *n*-Bu₄NF stands for tetrabutylammonium; iPrOH stands for 2-propanol; mp stands for melting point; LDA stands for diisopropylamino lithium;. EDCI stands for 1-(3-dimethylaminopropyl)-3-ethylcarbodiimide hydrochloride; TEA stands for triethylamine; DIEA stands for *N,N-*diisopropylethylamine; ACN stands for acetonitrile; IPA stands for isopropanol; Pd(dppf)Cl₂ stands for 1,1'-bisdiphenylphosphinoferrocene palladium dichloride.

Compounds are named manually or by ChemDraw® software, the commercially available compounds use their vendor directory names.

The compound of the present disclosure has a good inhibitory activity on PI3K kinase, and at the same time, it has a high subtype selectivity for PI3Kβ/γ/δ; it can also well inhibit the phosphorylation level of Akt which is the downstream of PI3K in cells, and also exhibits high subtype selectivity. The compound of the present disclosure can obviously inhibit the growth of tumors *in vivo,* and also shows an obvious time-dependent and dose-dependent inhibitory effect on the phosphorylation level of Akt which is the downstream of PI3K in animals. The compound of the present disclosure has no significant inhibitory effect on hERG and CYP enzymes, and is metabolically stable in liver cells of humans, rats, mice, dogs and monkeys.

### Detailed description of the preferred embodiment

The following examples further illustrate the present disclosure, but the present disclosure is not limited thereto. The present disclosure has been described in detail in the text, and its specific embodiments have also been disclosed, for one skilled in the art, it is obvious to modify and improve the embodiments of the present disclosure within the spirit and scope of the present disclosure.

### Reference example 1: fragment BB-1

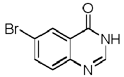

### Synthetic route:

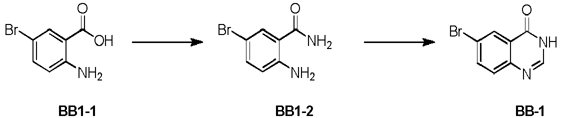

### Step 1: synthesis of compound BB1-2

Compound **BB1-1** (20.00 g, 92.58 mmol) was added to dichlorosulfoxide (110.14 g, 925.79 mmol, 67.16 mL), and stirred at 80 °C for 2 hours. The dichlorosulfoxide was then removed under reduced pressure, the residue was dissolved in tetrahydrofuran (550 mL), and the ammonia gas was introduced to the above system at 0 °C for 30 minutes. After the reaction was completed, the solvent was removed under reduced pressure to obtain compound **BB1-2**, which was directly used in the next reaction.

### Step 2: synthesis of compound BB-1

Compound **BB1-2** (20 g) and formamidine acetate (19.36 g, 186.01 mmol) were added to ethanol (800 mL), and the system was stirred at 80 °C for 16 hours. After the reaction was completed, ethanol was removed under reduced pressure, diluted with water (500 mL), and filtered to obtain compound **BB-1.** ¹H NMR (400 MHz, DMSO-*d*₆) δ : 8.20 (d, *J* = 2.5 Hz, 1H), 8.14 (s, 1H), 7.96 (dd, *J* = 8.8, 2.3 Hz, 1H), 7.63 (d, *J* = 8.5 Hz, 1H).

### Reference example 2: fragment BB-2

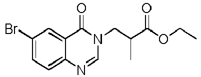

### Synthetic route:

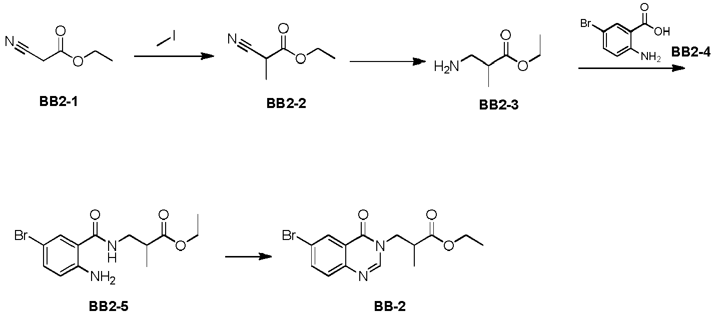

### Step 1: synthesis of compound BB2-2

Compound **BB2-1** (20.00 g, 176.82 mmol, 18.87 mL), methyl iodide (37.65 g, 265.23 mmol, 16.51 mL) and potassium carbonate (48.88 g, 353.64 mmol) were added to DMF (100 mL), and the system was stirred at 25 °C for 48 hours. After the reaction was completed, the solvent was removed under reduced pressure, diluted with water (200 mL), and extracted with dichloromethane (200 mL). The organic phase was concentrated under reduced pressure, and the residue was separated by chromatography column (ethyl acetate: petroleum ether = 0%-15%) to obtain compound **BB2-2.** ¹H NMR (400 MHz, CDCl₃) δ : 4.23-4.34 (m, 2 H), 3.56 (q, *J* = 7.4 Hz, 1H), 1.61 (dd, *J* = 7.5, 1.5 Hz, 3H), 1.31-1.37 (m, 3H).

### Step 2: synthesis of compound BB2-3

Compound **BB2-2** (2.30 g, 18.09 mmol) was dissolved in ethanol (20.00mL), and then Raney nickel (1.55 g, 18.09 mmol) was added under nitrogen atmosphere. The system was stirred under hydrogen atmosphere at 50 Pa and 25 °C for 24 hours. After the reaction was completed, the system was filtered, the filtrate was concentrated under reduced pressure, and the residue was separated by a chromatography column (methanol: dichloromethane = 0%-6%) to obtain compound **BB2-3.** ¹H NMR (400 MHz, DMSO-*d*₆) δ : 4.01-4.09 (m, 2 H), 2.72 (dd, *J* = 12.5, 7.0 Hz, 1H), 2.55-2.62 (m, 1H), 2.35-2.45 (m, 1H), 1.18 (t, *J* = 7.3 Hz, 3H), 1.04 (d, *J* = 7.0 Hz, 3H).

### Step 3: synthesis of compound BB2-5

Compound **BB2-4** (1.20 g, 5.55 mmol), compound **BB2-3** (800 mg, 6.11 mmol), EDCI (1.09 g, 5.66 mmol), 2-hydroxypyridine-*N*-oxide (722 mg, 6.49 mmol) and triethylamine (2.25g, 22.20mmol, 3.08mL) were added to dichloromethane (120 mL), and the system was stirred at 50 °C for 16 hours. After the reaction was completed, the reaction solution was diluted with water (200 mL) and extracted with dichloromethane (200 mL). The organic phase was concentrated under reduced pressure, and the residue was separated by chromatography column (methanol: dichloromethane = 0%-2%) to obtain the compound **BB2-5.** ¹H NMR (400 MHz, DMSO-*d*₆) δ : 8.46 (t, *J* = 5.6 Hz, 1 H), 7.61 (d, *J* = 2.3 Hz, 1 H), 7.26 (dd, *J* = 8.8, 2.3 Hz, 1 H), 6.67 (d, *J* = 8.8 Hz, 1 H), 6.52 (br s, 2 H), 4.06 (q, *J* = 7.1 Hz, 2 H), 3.37-3.45 (m, 1 H), 3.21-3.29 (m, 1 H), 2.67-2.80 (m, 1 H), 1.17 (t, *J* = 7.2 Hz, 3 H), 1.08 (d, *J* = 7.0 Hz, 3 H).

### Step 4: synthesis of compound BB-2

Compound **BB2-5** (1.00 g, 2.86 mmol) was added to formic acid (24.40 g, 530.09 mmol, 20.00 mL), and the system was stirred at 100 °C for 16 hours. After the reaction was completed, the reaction solution was concentrated under reduced pressure, and the residue was separated by chromatography column (ethyl acetate: petroleum ether = 0%-40%) to obtain compound **BB-2.** MS-ESI *m*/*z:* 340.8 [M+H]⁺.

### Reference example 3: fragment BB-3

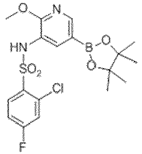

### Synthetic route:

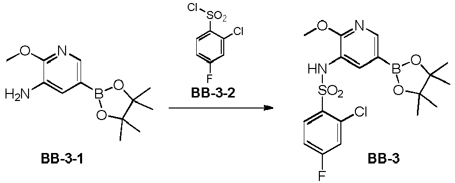

### Step 1: synthesis of compound BB-3

Compound **BB-3-1** (10.00 g, 39.98 mmol) was dissolved in pyridine (20 mL), and compound **BB-3-2** (9.16 g, 39.98 mmol, 5.83 mL) was added thereto, and stirred at 25 °C for 16 hours. The reaction solution was rotary-evaporated, diluted with water (200 mL), and then extracted with ethyl acetate (200 mL), and the organic phase was rotary-evaporated to obtain target compound **BB-3.**

### Reference example 4: fragment BB-4

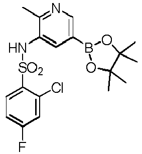

### Synthetic route:

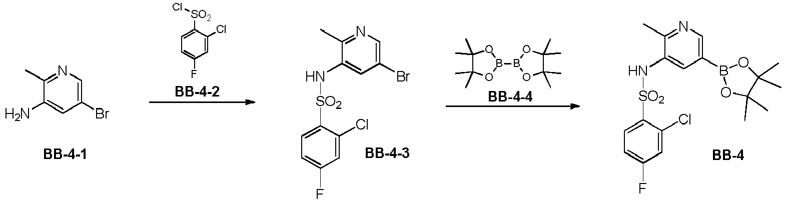

### Step 1: synthesis of compound BB-4-3

Compound **BB-4-1** (200 mg, 1.07 mmol) was dissolved in pyridine (5.00 mL), and compound **BB-4-2** (245 mg, 1.07 mmol, 156.00 µL) was added dropwise thereto, and stirred at 25 °C for 16 hours. After the reaction was completed, the reaction solution was rotary-evaporated to obtain target compound **BB-4-3.**

### Step 2: synthesis of compound BB-4

Compound **BB-4-3** (400 mg, 1.05 mmol), compound **BB-4-4** (400 mg, 1.58 mmol), potassium acetate (207 mg, 2.11 mmol) and ferrocene palladium chloride (77 mg, 105.23 µmol) were dissolved in dioxane (12 mL), and the reaction solution was stirred at 100 °C for 16 hours under nitrogen atmosphere. The reaction solution was diluted with water (100 mL), extracted with dichloromethane (100 mL × 2), and rotary-evaporated to obtain target compound **BB-4.**

### Reference example 5: fragment BB-5

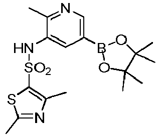

### Synthetic route:

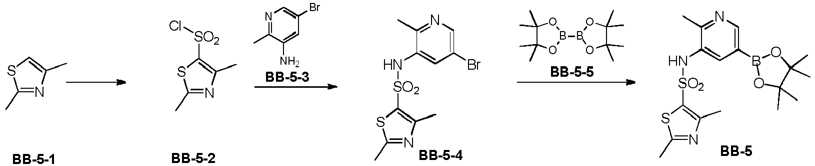

### Step 1: synthesis of compound BB-5-2

Compound **BB-5-1** (10.00 g, 88.35 mmol) was dissolved in chlorosulfonic acid (35.00 g, 300.39 mmol, 20.00 mL) at room temperature, and stirred at 140 °C for 18 hours. The reaction solution was cooled to 30 °C, then phosphorus pentachloride (36.80 g, 176.70 mmol) was added to the reaction solution in portions, and the reaction solution was stirred at 110 °C for 1 hour. The reaction solution was cooled to 30 °C, then ice water (100 mL) was added dropwise to the reaction solution, and extracted with dichloromethane (100 mL × 5). The organic phase was washed with water (100 mL × 3), and the organic phase was collected, dried over anhydrous sodium sulfate, and rotary-evaporated to obtain target compound **BB-5-2.** 1H NMR (400 MHz, CDCl₃) δ : 2.75 (s, 3H), 2.73 (s, 3H), 2.72-2.74 (m, 1H).

### Step 2: synthesis of compound BB-5-4

Compound **BB-5-2** (265 mg, 1.42 mmol) was dissolved in pyridine (1.00 mL) at 25 °C, and compound **BB-5-3** (300 mg, 1.42 mmol) was added thereto, and stirred at 25 °C for 16 hours. The reaction solution was rotary-evaporated, and TLC (petroleum ether: ethyl acetate = 2:1) showed that a new spot was formed, and then purified by column chromatography (petroleum ether: ethyl acetate = 1:0-1:1) to obtain target compound **BB-5-4.** ¹H NMR (400 MHz, CDCl₃) δ : 8.40 (d, *J* = 2.0 Hz, 1H), 7.89 (d, *J* = 2.0 Hz, 1H), 2.65 (s, 3H), 2.45 (s, 3H), 2.30-2.34 (m, 3H).

### Step 3: synthesis of compound BB-5

Compound **BB-5-4** (300 mg, 828.11 µµmol), compound **BB-5-5** (315 mg, 1.24 mmol), potassium acetate (243 mg, 2.48 mmol) and ferrocene palladium chloride (61 mg, 83.37 µmol) were dissolved in dioxane (12 mL), and the reaction solution was stirred at 100 °C for 1 hour under nitrogen atmosphere. The reaction solution was diluted with water (100 mL), then extracted with dichloromethane (100 mL), and rotary-evaporated to obtain target compound **BB-5.**

### Reference example 6: fragment BB-6

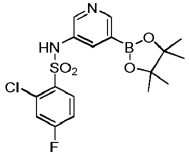

### Synthetic route:

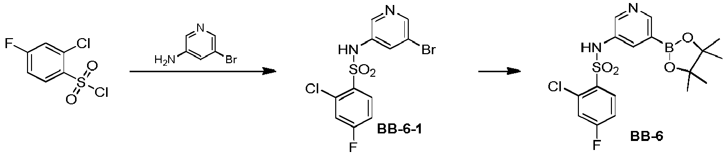

### Step 1: synthesis of compound BB-6-1

A solution of 3-amino-5-bromopyridine (200 mg, 1.16 mmol) and 2-chloro-4-fluorobenzenesulfonyl chloride (265 mg, 1.16 mmol) in pyridine (1 mL) was stirred at 15 °C for 16 hours. After the reaction was complete, the mixture was rotary-evaporated directly. Water (50 mL) was added to the reaction, and extracted with dichloromethane (50 mL). The organic phase was concentrated under reduced pressure and purified by column chromatography (MeOH: DCM = 0%-10%) to obtain **BB-6-1.** MS-ESI *m*/*z:* 364.7 [M+H]⁺, 366.7 [M+H+2]⁺.

### Step 2: synthesis of compound BB-6

The suspension of **BB-6-1** (320 mg, 875.25 µmol), bis(pinacolato)diboron (333 mg, 1.31 mmol), potassium acetate (258 mg, 2.63 mmol) and [1,1'-bis(diphenyl phosphinyl) ferrocene] palladium dichloride (64 mg, 87.47 µmol) in dioxane (20 mL) was replaced with nitrogen three times, and then the reaction solution was heated to 100 °C and stirred for 16 hours under nitrogen atmosphere. After the reaction was completed, the reaction solution was concentrated and rotary-evaporated. The crude product was slurried with water (100 mL), and then extracted with dichloromethane (100 mL). The organic phase was concentrated under reduced pressure to obtain **BB-6.** MS-ESI *m*/*z:* 330.8.

### Reference example 7: fragment BB-7

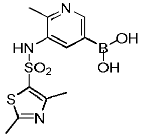

### Synthetic route:

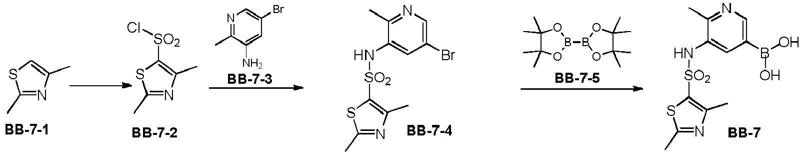

### Step 1: synthesis of compound BB-7-2

Compound **BB-7-1** (10.00 g, 88.35 mmol) was dissolved in chlorosulfonic acid (35.00 g, 300.39 mmol, 20.00 mL) at room temperature, and stirred at 140 °C for 18 hours. The reaction solution was cooled to 30 °C, then phosphorus pentachloride (36.80 g, 176.70 mmol) was added to the reaction solution in portions, and the reaction solution was stirred at 110 °C for 1 hour. The reaction solution was cooled to 30 °C, then ice water (100 mL) was added dropwise to the reaction solution, followed by extraction with dichloromethane (100 mL × 5). The organic phase was washed with water (100 mL × 3), dried over anhydrous sodium sulfate and rotary-evaporated to obtain the target compound **BB-7-2.** 1H NMR (400 MHz, CDCl₃) δ: 2.75 (s, 3H), 2.73 (s, 3H), 2.72-2.74 (m, 1H).

### Step 2: synthesis of compound BB-7-4

Compound **BB-7-2** (1.2 g, 5.47 mmol) and compound **BB-7-3** (1.02 g, 5.47 mmol) were dissolved in pyridine (5 mL), and stirred at 15 °C for 16 hours. The reaction solution was diluted with water (30 mL), and then extracted with dichloromethane (30 mL × 2), and the organic phase was rotary-evaporated to obtain target compound **BB-7-4.**

### Step 3: synthesis of compound BB-7

Compound **BB-7-4** (2.10 g, 5.43 mmol), compound **BB-7-5** (2.07 g, 8.14 mmol), potassium acetate (1.6 g, 16.30 mmol) and ferrocene palladium chloride (0.4 g, 546.67 µmol) were dissolved in dioxane (80 mL), and the reaction solution was stirred at 105 °C for 16 hours under nitrogen atmosphere. The reaction solution was diluted with water (30 mL), and then extracted with dichloromethane (30 mL × 2), and the organic phase was rotary-evaporated to obtain target compound **BB-7.**

### Reference example 8: fragment BB-8

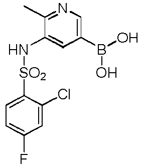

### Synthetic route:

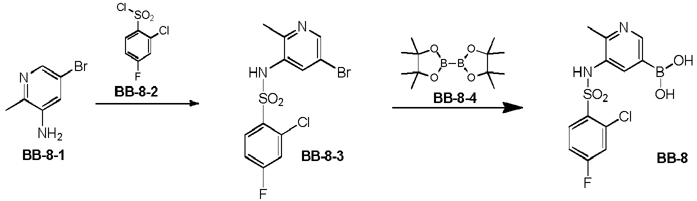

### Step 1: synthesis of compound BB-8-3

Compound **BB-8-1** (3.00 g, 16.04 mmol) was dissolved in pyridine (10 mL) and compound **BB-8-2** (3.67 g, 16.04 mmol, 2.34 mL) was slowly added thereto, and stirred at 25 °C for 16 hour. The reaction solution was rotary-evaporated, and TLC (petroleum ether: ethyl acetate = 2:1) showed that a new spot was formed. The target compound **BB-8-3** was obtained by purification by column chromatography (petroleum ether: ethyl acetate = 1:0-10:3).

### Step 2: synthesis of compound BB-8

Compound **BB-8-3** (5.50 g, 12.87 mmol), compound **BB-8-4** (4.90 g, 19.30 mmol), potassium acetate (3.76 g, 38.35 mmol) and ferrocene palladium chloride (941.00 mg, 1.29 mmol) were dissolved in dioxane (200 mL), and the reaction solution was stirred at 100 °C for 16 hours under nitrogen atmosphere. The reaction solution was diluted with water (200 mL), and then extracted with dichloromethane (200 mL). The organic phase was rotary-evaporated to obtain target compound **BB-8.**

### Reference example 9: fragment BB-9

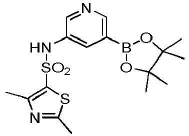

### Synthetic route:

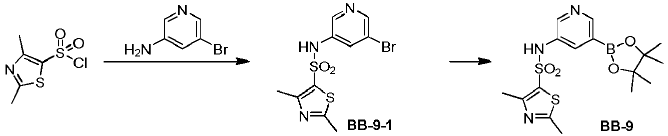

### Step 1: synthesis of compound BB-9-1

A solution of 3-amino-5-bromopyridine (200 mg, 1.16 mmol) and 2,4-dimethylthiazole-5-sulfonyl chloride (366 mg, 1.73 mmol) in pyridine (1 mL) was stirred at 15 °C for 16 hours. After the reaction was complete, the reaction solution was rotary-evaporated directly, followed by addition of water (50 mL) and extraction with dichloromethane (50 mL). The organic phase was concentrated under reduced pressure to obtain **BB-9-1.** MS-ESI *m*/*z:* 347.8 [M+H]⁺, 349.8 [M+H+2]⁺.

### Step 2: synthesis of compound BB-9

A suspension of **BB-9-1** (0.3 g, 838.13 µmol, purity: 97.29%), bis(pinacolato)diboron (0.319 g, 1.26 mmol), potassium acetate (0.247 g, 2.52 mmol) and [1,1'- bis(diphenylphosphino) ferrocene] palladium dichloride (0.061 g, 83.37 µmol) in dioxane (20 mL) was replaced with nitrogen three times. The reaction solution was heated to 100 °C and stirred for 16 hours under nitrogen atmosphere. After the reaction was completed, the reaction solution was concentrated and rotary-evaporated. The crude product was slurried with water (50 mL), and then extracted with dichloromethane (50 mL). The organic phase was concentrated under reduced pressure to obtain **BB-9.** MS-ESI *m*/*z:* 313.9.

### Reference example 10: fragment BB-10

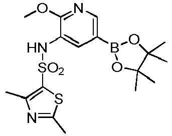

### Synthetic route:

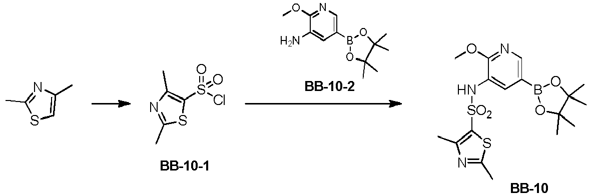

### Step 1: synthesis of compound BB-10-1

At 15 °C, 2,4-dimethylthiazole (2.00 g, 17.67 mmol) was added to chlorosulfonic acid (7.00 g, 60.08 mmol). The reaction was carried out at 140 °C for 18 hours with stirring. The reaction solution was then cooled to 30 °C, phosphorus pentachloride (7.36 g, 35.34 mmol) was added thereto in portions, and the reaction was stirred at 110 °C for 1 hour. After the reaction was completed, the reaction solution was cooled to 30 °C, and then added dropwise to the ice-water mixture (50 mL) with stirring. The reaction solution was then extracted with dichloromethane (50 mL) three times. The organic phase was evaporated under reduced pressure, separated and purified by column chromatography (ethyl acetate: petroleum ether = 0%, 5%, 10%) to obtain the target compound **BB-10-1.** MS-ESI *m*/*z:* 211.7 [M+H]⁺.

### Step 2: synthesis of compound BB-10

At 5 °C, **BB-10-1** (1.80 g, 8.50 mmol) was added dropwise to a solution of **BB-10-2** (2.13 g, 8.50 mmol) in pyridine (4 mL) within 10 minutes. The reaction solution was stirred at 5 °C for 16 hours. After the reaction is completed, the reaction solution was concentrated under reduced pressure to obtain **BB-10,** which is directly used in the next step. MS-ESI *m*/*z:* 426.0 [M+H]⁺.

### Comparative example 1: R001

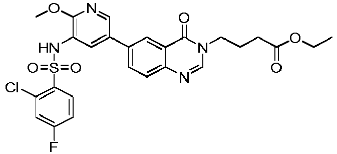

### Synthetic route:

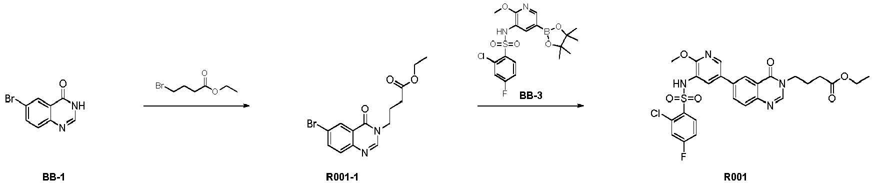

### Step 1: synthesis of compound R001-1

Compound **BB-1** (1.00 g, 4.44 mmol), ethyl 4-bromobutyrate (952 mg, 4.88 mmol) and cesium carbonate (2.17 g, 6.66 mmol) were dissolved in *N,N-*dimethylformamide methylal (15 mL), heated to 60 °C and stirred for 3 hours. After the reaction was completed, the reaction solution was cooled to room temperature, the organic solvent was rotary-evaporated, poured into water (10 mL), and extracted with dichloromethane (10 mL × 3). The organic phases obtained were combined and dried over anhydrous sodium sulfate. The desiccant was removed by filtration, and the solvent was removed under reduced pressure to obtain the target compound **R001-1.** MS-ESI *m*/*z:* 340.80[M+H]⁺, 342.8 [M+H+2]⁺.

### Step 2: synthesis of compound R001

Compound **R001-1** (1.00 g, 2.95 mmol), **BB-3** (1.31 g, 2.95 mmol) and potassium acetate (1.16 g, 11.80 mmol) were dissolved in dioxane (10 mL) and water (1 mL), followed by addition of ferrocene palladium dichloride (43.15 mg, 59.00 µmol). The reaction solution was heated to 95 °C and stirred for 2 hours under nitrogen atmosphere. After the reaction was completed, the reaction solution was cooled to room temperature, the organic solvent was rotary-evaporated, poured into water (100 mL), and extracted with dichloromethane (100 mL × 3). The organic phases obtained were combined and dried over anhydrous sodium sulfate. After the desiccant was removed by filtration, the solvent was removed under reduced pressure, and the target compound **R001** was obtained by separation through a preparative high-performance liquid phase column. ¹H NMR (400MHz, CDCl₃) δ : 8.34 (d, *J* = 1.8 Hz, 1H), 8.21-8.10 (m, 2H), 8.07 (s, 1H), 7.97 (d, *J* = 2.3 Hz, 1H), 7.91-7.82 (m, 1H), 7.82-7.74 (m, 1H), 7.25 (br d, *J* = 2.5 Hz, 1H), 7.18-7.05 (m, 1H), 4.21-4.07 (m, 4H), 3.98 (s, 3H), 2.53-2.35 (m, 2H), 2.16 (quin, *J* = 7.0 Hz, 2H), 1.27 (t, *J* = 7.2 Hz, 3H). MS-ESI *m*/*z:* 574.9.0[M+H]⁺, 376.9.[M+H+2]⁺.

### Comparative example 2: R002, R003

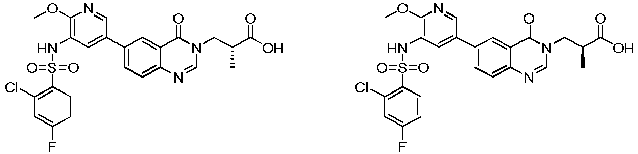

### Synthetic route:

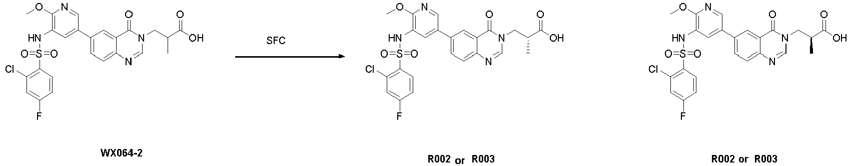

### Step 1: synthesis of compound R002 and compound R003

Compound **WX064-2** was resolved by supercritical fluid chromatography (separation conditions: chromatographic column: AD (250mm * 30mm, 10µm); mobile phase: [0.1% NH₄HCO₃ EtOH]; B%: 40%-40%) to obtain the enantiomers **R002** and **R003.** The retention time is 2.802min and 2.259min respectively, and the ratio is 1:1. **R002:** 1H NMR (400MHz, DMSO-*d*₆) δ: 12.55 (br s, 1H), 10.26 (br s, 1H), 8.46-8.32 (m, 2H), 8.25 (d, J = 2.3 Hz, 1H), 8.09 (dd, J = 2.3, 8.5 Hz, 1H), 8.01-7.87 (m, 1H), 8.03-7.87 (m, 1H), 8.02-7.63 (m, 2H), 7.36 (dt, J = 2.5, 8.5 Hz, 1H), 4.27-4.13 (m, 1H), 4.11-4.02 (m, 1H), 3.70 (s, 3H), 3.04-2.92 (m, 1H), 1.14 (d, J = 7.3 Hz, 3H). MS-ESI m/z: 547.0[M+H]⁺,549.0[M+H+2]⁺. **R003:** 1H NMR (400MHz, DMSO-*d*₆) δ: 8.42-8.33 (m, 2H), 8.25 (d, *J* = 2.3 Hz, 1H), 8.09 (dd, *J* = 2.3, 8.5 Hz, 1H), 7.96 (dd, *J* = 5.9, 8.9 Hz, 1H), 7.91 (d, *J* = 2.3 Hz, 1H), 7.82-7.69 (m, 2H), 7.36 (dt, *J* = 2.5, 8.5 Hz, 1H), 4.22-4.01 (m, 2H), 3.70 (s, 3H), 3.04-2.90 (m, 1H), 1.14 (d, *J* = 7.3 Hz, 3H). MS-ESI m/z: 547.0[M+H]⁺, 549.0[M+H+2]⁺.

### Comparative example 3: R004, R005

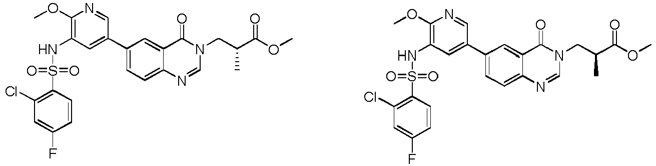

### Synthetic route:

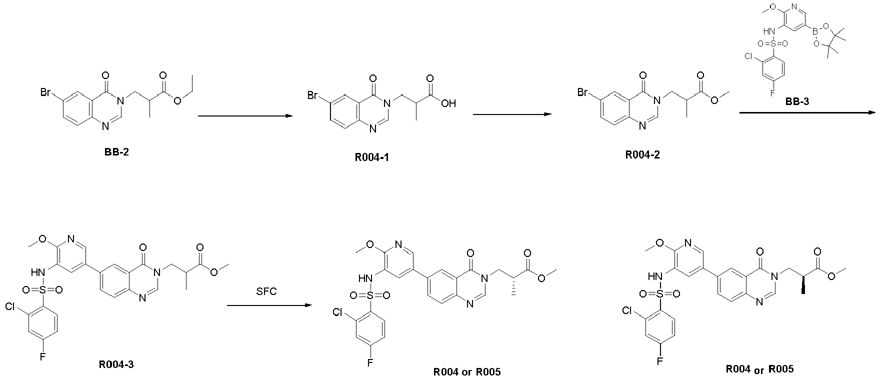

### Step 1: synthesis of compound R004-1

Compound **BB-2** (10.00 g, 29.48 mmol) and lithium hydroxide monohydrate (12.37 g, 294.80 mmol) were dissolved in ethanol (50 mL) and water (50 mL), and stirred at 10 °C for 16 hours. TLC (dichloromethane: methanol = 10:1) showed the formation of the new spot. The reaction solution was adjusted to pH = 3 to 4 with hydrochloric acid (4 mol/L), and then the mixture was diluted with water (200 mL) and extracted with ethyl acetate (200 mL × 2). The organic phase was rotary-evaporated to obtain the target compound **R004-1.**

### Step 2: synthesis of compound R004-2

Compound **R004-1** (1.00 g, 3.21 mmol) was dissolved in dichlorosulfoxide (1.64 g, 13.78 mmol, 1.00 mL) and methanol (20 mL), and stirred at 60 °C for 6 hours under nitrogen atmosphere. TLC (petroleum ether: ethyl acetate = 10:1) showed the formation of the new spot. The reaction solution was rotary-evaporated, and then the mixture was diluted with water (50 mL) and extracted with ethyl acetate (50 mL × 3). The organic phase was dried over anhydrous sodium sulfate, rotary-evaporated and purified by silica gel column (petroleum ether: ethyl acetate = 5:1-5:3) to obtain the target compound **R004-2.** ¹H NMR (400 MHz, CHLOROFORM-d) δ : 1.30 (d, *J* = 7.53 Hz, 3 H) 3.17 (dqd, *J* = 9.32, 7.27, 7.27, 7.27, 5.02 Hz, 1 H) 3.66 (s, 3 H) 4.02 (dd, *J* = 13.55, 9.03 Hz, 1 H) 4.18 (dd, *J* = 13.55, 5.02 Hz, 1 H) 7.58 (d, *J* = 8.53 Hz, 1 H) 7.83 (dd, *J* = 8.78, 2.26 Hz, 1 H) 8.12 (s, 1 H) 8.42 (d, *J* = 2.51 Hz, 1 H).

### Step 3: synthesis of compound R004-3

Compound **R004-2** (150 mg, 461.32 µmol), compound **BB-3** (200 mg, 451.77 (µmol), potassium acetate (180 mg, 1.83 mmol) and ferrocene palladium chloride (33 mg, 45.10 µmol) were dissolved in dioxane (8 mL) and water (1.5 mL). The reaction solution was stirred at 100 °C for 30 minutes under nitrogen atmosphere. TLC (dichloromethane: methanol = 10: 1) showed the formation of the new spot. The reaction solution was rotary-evaporated, then diluted with water (20 mL), and then extracted with ethyl acetate (10 mL × 2). The organic phase was collected and washed with saturated brine. The organic phase was collected, dried over anhydrous sodium sulfate, rotary-evaporated, and then separated and purified by preparative TLC (dichloromethane: methanol = 10: 1) to obtain the target compound **R004-3.**

### Step 4: synthesis of compound R004 and R005

Compound **R004-3** was resolved by supercritical fluid chromatography (separation conditions column: column: OJ (250mm * 30mm, 5µm); mobile phase: [0.1% NH₄HCO₃ EtOH]; B%: 40%-40%) to obtain the enantiomers **R004** and **R005.** ¹H NMR (400 MHz, DMSO-*d*₆) δ: 1.15 (d, *J* = 7.03 Hz, 3 H) 3.07 (sxt, *J* = 7.18 Hz, 1 H) 3.57 (s, 3 H) 3.69 (s, 3 H) 4.04-4.24 (m, 2 H) 7.32-7.40 (m, 1 H) 7.70-7.79 (m, 2 H) 7.91 (d, *J* = 2.26 Hz, 1 H) 7.96 (dd, *J* = 8.91, 5.90 Hz, 1 H) 8.08 (dd, *J* = 8.41, 1.63 Hz, 1 H) 8.24 (d, *J* = 2.01 Hz, 1 H) 8.37 (s, 1 H) 8.39 (d, *J* = 2.01 Hz, 1 H). **R005:** ¹H NMR (400 MHz, DMSO-d6) δ:1.15 (d, *J* = 7.03 Hz, 3 H) 3.07 (sxt, *J* = 7.13 Hz, 1 H) 3.57 (s, 3 H) 3.69 (s, 3 H) 4.04-4.25 (m, 2 H) 7.36 (td, *J* = 8.41, 2.51 Hz, 1 H) 7.69-7.80 (m, 2 H) 7.91 (d, *J* = 2.26 Hz, 1 H) 7.96 (dd, *J* = 8.78, 6.02 Hz, 1 H) 8.08 (dd, *J* = 8.53, 2.01 Hz, 1 H) 8.24 (d, *J* = 2.01 Hz, 1 H) 8.37 (s, 1 H) 8.40 (d, *J* = 2.26 Hz, 1 H). The retention time is 1.548 min and 1.782 min respectively, and the ratio is 1:1.

### Comparative example 4: R005, R006

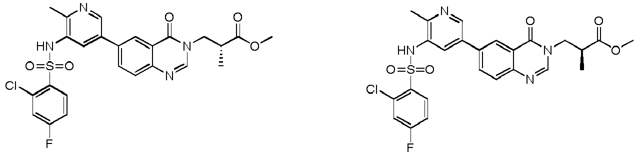

### Synthetic route:

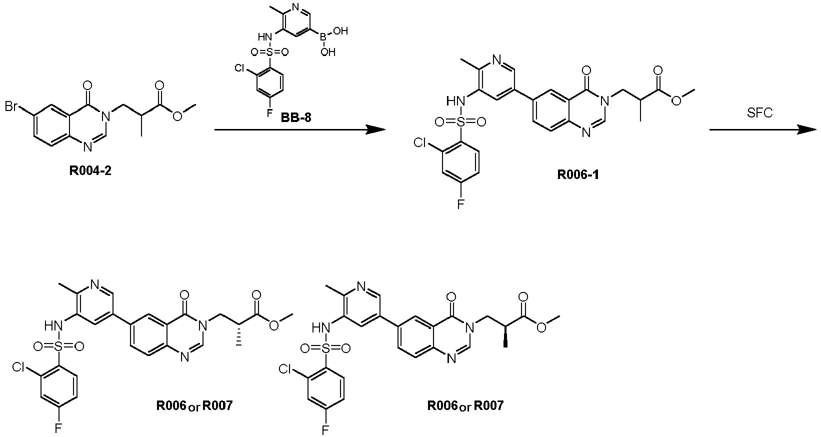

### Step 1: synthesis of compound R006-1

Compound **R004-2** (350 mg, 1.08 mmol), compound **BB-8** (400 mg, 1.16 mmol), potassium acetate (460 mg, 4.69 mmol) and ferrocene palladium chloride (85 mg, 116.17 µmol) were dissolved in dioxane (15 mL) and water (3 mL), and the reaction solution was stirred at 100 °C for 30 minutes under nitrogen atmosphere. TLC (dichloromethane: methanol = 10:1) showed the formation of new spots. The reaction solution was rotary-evaporated, diluted with water (20 mL), and extracted with ethyl acetate (10 mL × 2). The organic phase was collected and washed with saturated brine (20 mL). The organic phase was collected, dried over anhydrous sodium sulfate and rotary-evaporated, and then separated and purified by preparative TLC (dichloromethane: methanol = 10: 1) to obtain the target compound **R006-1.**

### Step 2: synthesis of compound R006 and R007

Compound **R006-1** was resolved by supercritical fluid chromatography (separation condition column: OD (250mm * 30mm, 10µm); mobile phase: [0.1% NH₄HCO₃ EtOH]; B%: 35% -35%) to obtain the enantiomers **R006** and **R007. R006:** ¹H NMR (400 MHz, DMSO-*d*₆) δ ppm 1.15 (d, *J* = 7.28 Hz, 3 H) 2.37 (s, 3 H) 3.01-3.13 (m, 1 H) 3.57 (s, 3 H) 4.05-4.24 (m, 2 H) 7.37 (td, *J* = 8.47, 2.38 Hz, 1 H) 7.71-7.81 (m, 3 H) 7.98 (dd, *J* = 8.78, 6.02 Hz, 1 H) 8.06 (dd, *J* = 8.53, 2.01 Hz, 1 H) 8.21 (d, *J* = 2.01 Hz, 1H) 8.38(s, 1 H) 8.64 (brs, 1 H). **R007:** ¹H NMR (400MHz, DMSO-*d₆*) δ ppm 1.15 (br d, *J* = 7.03 Hz, 3 H) 2.37 (br s, 3 H) 3.07 (br d, *J* = 7.53 Hz, 1 H) 3.57 (s, 3 H) 4.05-4.25 (m, 2 H) 7.32-7.42 (m, 1 H) 7.69-7.84 (m, 3 H) 7.93-8.02 (m, 1 H) 8.06 (br d, *J* = 8.53 Hz, 1 H) 8.21 (br s, 1 H) 8.38 (s, 1 H) 8.64 (br s, 1 H). The retention time is 1.413 min and 1.561min respectively, and the ratio is 1:1.

### Comparative example 5: R008

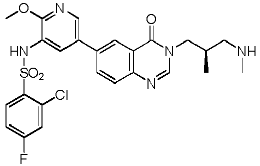

### Synthetic route:

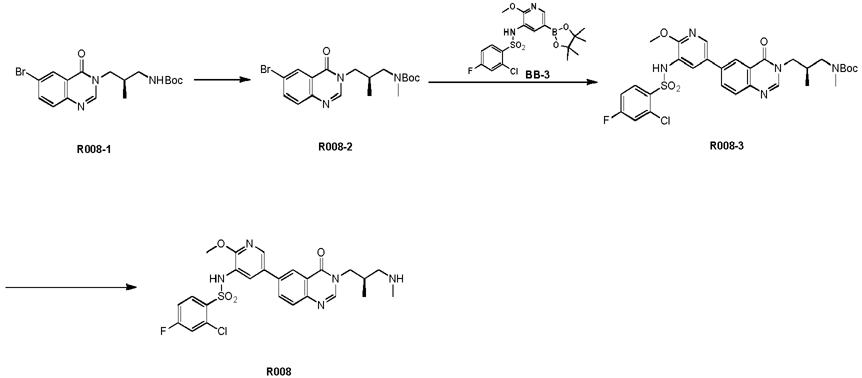

### Step 1: synthesis of compound R008-2

Compound **R008-1** (0.65 g, 1.64 mmol), methyl iodide (465 mg, 3.28 mmol, 204.22 µL) and tetrahydrofuran (6 mL) were added sequentially to a pre-dried reaction flask (8mL), and then lithium hexamethyldisilazide (1 M, 4.10 mL) was added slowly. The reaction solution was replaced with nitrogen, and stirred at 15 °C for 10 hours. After the reaction was completed, the reaction solution was quenched with methanol (10 mL), and the solvent was evaporated under reduced pressure. The mixture was separated and purified by flash column chromatography (petroleum ether: ethyl acetate = 5:1 to 1:1), and then further purified by preparative HPLC to obtain compound **R008-2.** Purification method: chromatographic column: Agela Durashell C18 150 * 25mm 5µm; mobile phase: [water (10mMNH₄HCO₃) -ACN]; B%: 42% -62%, 10.5min. ¹H NMR (400MHz, CHLOROFORM-d) δ = 8.41 (d, *J* = 2.0 Hz, 1H), 7.98 (br s, 1H), 7.81 (dd, *J* = 2.2, 8.8 Hz, 1H), 7.57 (d, *J* = 8.8 Hz, 1H), 4.08 (br s, 1H), 3.59 (br s, 1H), 3.49-3.19 (m, 1H), 3.08 (br dd, *J* = 6.5, 14.0 Hz, 1H), 2.87 (s, 3H), 2.41 (br s, 1H), 1.43 (s, 10H), 0.91 (br d, *J* = 6.6 Hz, 3H).

### Step 2: synthesis of compound R008-3

Compound **R008-2** (0.14 g, 341.21 µmol), compound **BB-3** (151 mg, 341.21 (µmol), potassium acetate (100 mg, 1.02 mmol), 1,4-dioxane (3 mL) and water (0.3 mL) as the solvent were added sequentially to a pre-dried reaction flask (10mL). The reaction solution was replaced with the nitrogen, followed by addition of 1,1-bis (diphenylphosphine) ferrocene palladium chloride (24.97 mg, 34.12 µmol). The reaction solution was replaced with nitrogen again, and heated to 90 °C and stirred for 5 hours. After the reaction was completed, the reaction solution was cooled and filtered, and washed with methanol (20 mL × 2). The filtrate was evaporated under reduced pressure, and purified by preparative HPLC to obtain compound **R008-3.** Purification method: chromatographic column: Agela Durashell C18 150 * 25mm 5µm; mobile phase: [water (10mMNH₄HCO₃) -ACN]; B%: 35% -65%, 10.5min. 1H NMR (400MHz, METHANOL-d4) δ = 8.34 (s, 2H), 8.23 (d, *J* = 2.1 Hz, 1H), 8.11 (dd, *J* = 6.0, 8.8 Hz, 1H), 8.05-7.97 (m, 2H), 7.80 (d, *J* = 8.5 Hz, 1H), 7.47 (dd, *J* = 2.4, 8.3 Hz, 1H), 7.27-7.20 (m, 1H), 4.58 (s, 2H), 4.13 (br s, 1H), 3.87 (s, 3H), 3.21-3.10 (m, 1H), 2.91 (s, 3H), 2.49 (br s, 1H), 1.44 (br s, 9H), 0.97 (br s, 3H).

### Step 3: synthesis of compound R008

Compound **R008-3** (0.14 g, 216.68 µmol) and dichloromethane (3 mL) were added sequentially to a pre-dried reaction flask (8mL), followed by addition 2,6-lutidine (92.87 mg, 866.70 µmol, 100.95 µL) and trimethylsilyl triflate (144.47 mg, 650.03 µmol, 117.46 µL). The reaction solution was replaced with nitrogen, and stirred at 20 °C for 10 hours. After the reaction was completed, the reaction solution was rotary-evaporated under reduced pressure and purified by preparative HPLC to obtain compound **R008.** Purification method: Column: Agela Durashell C18 150 * 25mm 5µm; mobile phase: [Water (10mM NH₄HCO₃) -ACN]; B%: 15% -45 %, 10.5min. ¹H NMR (400MHz, DMSO-*d*₆) Shift = 8.35 (s, 1H), 8.14-8.06 (m, 2H), 7.92 (dd, *J* = 2.1, 8.5 Hz, 1H), 7.77 (br s, 1H), 7.69 (d, *J* = 8.5 Hz, 1H), 7.52 (d, *J* = 1.9 Hz, 1H), 7.45 (dd, *J* = 2.4, 8.8 Hz, 1H), 7.30 (dt, *J* = 2.6, 8.4 Hz, 1H), 4.07-3.86 (m, 2H), 3.78 (s, 3H), 2.94-2.82 (m, 1H), 2.80-2.66 (m, 1H), 2.55 (s, 3H), 2.47-2.31 (m, 2H), 1.01 (d, *J* = 6.8 Hz, 3H).

### Comparative example 6: R009

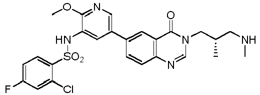

### Synthetic route:

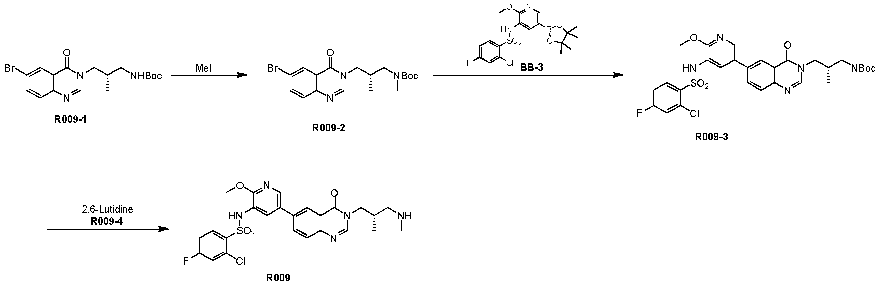

### Step 1: synthesis of compound R009-2

The raw material **R009-1** (893 mg, 2.25 mmol) and the solvent *N,N-*dimethylformamide (10 mL) were added to a pre-dried 40mL reaction flask, and iodomethane (1.18 g, 8.34 mmol, 519.06 µL) was added. The reaction system was placed at 0 °C, followed by addition of sodium hydrogen (135.20 mg, 5.63 mmol, 2.5 eq), and stirred at 25 °C for 2 hours. After the reaction was completed, water (10 mL) was added to the reaction solution, and extracted with dichloromethane (10 mL × 3) to collect the organic phase. The resulting organic phase was dried over anhydrous sodium sulfate and then dried under reduced pressure, which was then separated and purified by flash column chromatography (petroleum ether: ethyl acetate = 1: 0-3: 1) to obtain the target compound **R009-2.** ¹H NMR (400MHz, CHLOROFORM-d) δ = 8.41 (d, *J* = 2.2 Hz, 1H), 8.00 (s, 6H), 7.81 (dd, *J* = 2.2, 8.8 Hz, 1H), 7.57 (d, *J* = 8.6 Hz, 1H), 4.10 (q, *J* = 7.1 Hz, 2H), 3.69-3.24 (m, 2H), 3.12-3.02 (m, 1H), 2.41 (br s, 1H), 2.02 (s, 2H), 1.43 (s, 9H), 1.24 (t, *J* = 7.2 Hz, 2H), 0.91 (br d, *J* = 6.6 Hz, 3H).

### Step 2: synthesis of compound R009-3

Raw material **R009-2** (860.00 mg, 2.10 mmol), raw material **BB-3** (1.02 g, 2.31 mmol), solvent water (1 mL) and 1,4-dioxane (10 mL) were added to a pre-dried reaction flask, followed by addition of potassium acetate (411.40 mg, 4.19 mmol). The reaction system was replaced with nitrogen, followed by addition of 1,1-bis (diphenylphosphine) ferrocene palladium chloride (153.37 mg, 209.60 µmol). The reaction system was replaced with nitrogen, and stirred at 90 °C for 12 hours. After the reaction was completed, water (10 mL) and was added to the reaction solution and extracted with dichloromethane (10 mL × 3). The resulting organic phase was dried over anhydrous sodium sulfate and then rotary-evaporated under reduced pressure, which was separated and purified by flash column chromatography (petroleum ether: Ethyl acetate = 1: 0 to 1: 1) to obtain the target compound **R009-3.** ¹H NMR (400MHz, CHLOROFORM-d) δ = 8.35 (d, *J* = 2.2 Hz, 1H), 8.15 (d, *J* = 2.2 Hz, 1H), 8.14-8.10 (m, 1H), 8.03 (br s, 1H), 7.99 (d, *J* = 2.2 Hz, 1H), 7.87-7.83 (m, 1H), 7.81-7.77 (m, 1H), 7.54 (s, 1H), 7.26 (d, *J* = 2.4 Hz, 1H), 7.15-7.08 (m, 1H), 4.23-4.04 (m, 1H), 3.98 (s, 3H), 3.65 (br s, 1H), 3.55-3.27 (m, 1H), 3.13 (br d, *J* = 14.3 Hz, 1H), 2.93 (s, 3H), 2.49 (br s, 1H), 2.56-2.40 (m, 1H), 2.56-2.40 (m, 1H), 1.47 (s, 10H), 0.96 (br d, *J* = 6.0 Hz, 3H).

### Step 3: synthesis of compound R009

The raw material **R009-3** (470 mg, 727.41 µmol) and the solvent dichloromethane (5 mL) were added to a pre-dried reaction flask, followed by addition of **R009-4** (311.78 mg, 2.91 mmol, 338.89 µL) and trimethylsilyl trifluoromethanesulfonate (485.02 mg, 2.18 mmol, 394.32 µL), and stirred at 25 °C for 12 hours. After the reaction was completed, the reaction solution was directly rotary-evaporated under reduced pressure and separated by preparative HPLC (Column: Agela Durashell C18 150 * 25mm 5µm; Mobile phase: [Water (10mM NH₄HCO₃) -ACN]; B%: 15%-45%, 10.5 min) to obtain the product **R009.** ¹H NMR and MS showed that the structure was correct, HPLC showed that the retention time is 1.698 minutes, and SFC showed that the product s in a single configuration, ee% = 100%. ¹H NMR (400MHz, CHLOROFORM-d) δ = 8.34 (d, *J* = 2.0 Hz, 1H), 8.17-8.13 (m, 1H), 8.13-8.10 (m, 2H), 7.94 (d, *J* = 2.2 Hz, 1H), 7.86-7.82 (m, 1H), 7.79-7.75 (m, 1H), 7.26-7.23 (m, 1H), 7.12 (ddd, *J* = 2.4, 7.5, 8.8 Hz, 1H), 4.13-4.05 (m, 1H), 4.02-3.95 (m, 4H), 2.88 (br s, 5H), 2.60-2.55 (m, 2H), 2.49 (s, 3H), 2.35 (qd, *J* = 6.5, 13.0 Hz, 1H), 1.04 (d, J = 6.8 Hz, 3H), 1.07-1.00 (m, 1H), 1.07-1.00 (m, 1H), 1.07-1.00 (m, 1H).

### Comparative example 7: R010

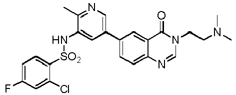

### Synthetic route:

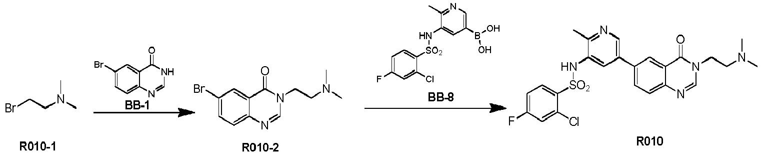

### Step 1: synthesis of compound R010-2

Compound **R010-1** (0.301 g, 1.29 mmol), compound **BB-1** (0.2 g, 647.26 µmol) and cesium carbonate (0.843 g, 2.59 mmol) were dissolved in *N,N-*dimethylformamide (5.00 mL), and stirred at 60 °C for 2 hours. After the reaction was completed, the reaction solution was rotary-evaporated, diluted with water (50 mL), and then extracted with dichloromethane (50 mL). The organic phase was rotary-evaporated to obtain the target compound **R010-2.**

### Step 2: synthesis of compound R010

Compound **R010-2** (0.2 g, 514.04 µmol), compound **BB-8** (0.177 g, 513.71 µmol), potassium acetate (0.202 g, 2.06 mmol) and ferrocene palladium chloride (0.038 g, 51.93 µmol) were dissolved in dioxane (5.00 mL) and water (1 mL), and the reaction solution was stirred at 100 °C for 1 hour under nitrogen atmosphere. After the reaction was completed, the reaction solution was rotary-evaporated, diluted with water (50 mL), and then extracted with dichloromethane (50 mL), and the organic phase was rotary-evaporated. The obtained material was separated by preparative HPLC (resolution method: chromatographic column: Phenomenex Gemini C18 250 * 50mm 10µm; mobile phase: [water (0.05% ammonium hydroxide v / v) -ACN]; B%: 23% -33%, 8min) to obtain the target compound **R010.** ¹H NMR (400 MHz, METHANOL-*d*₄) δ ppm 8.57 (d, *J* = 2.5 Hz, 1 H), 8.31-8.38 (m, 2 H), 8.09 (dd, *J* = 8.8, 5.8 Hz, 1H), 8.02 (dd, *J* = 8.5, 2.0 Hz, 1 H), 7.88 (d, *J* = 2.0 Hz, 1 H), 7.80 (d, *J* = 8.5 Hz, 1 H), 7.53 (dd, *J* = 8.5, 2.5 Hz, 1 H), 7.22-7.30 (m, 1 H), 4.26 (t, *J* = 6.3 Hz, 2 H), 2.85 (t, *J* = 6.5 Hz, 2 H), 2.51 (s, 3 H), 2.43 (s, 6 H).

### Comparative example 8: R011

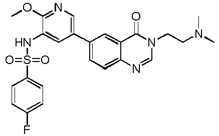

### Synthetic route:

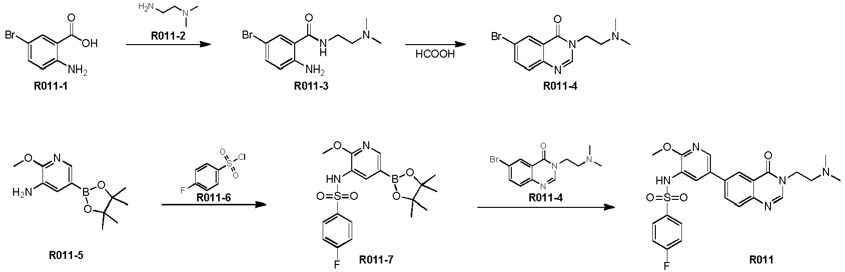

### Step 1: synthesis of compound R011-3

Compound **R011-1** (1 g, 4.63 mmol) was dissolved in dichloromethane (10 mL), followed by addition of compound **R011-2** (428.45 mg, 4.86 mmol, 530.92 µL), EDCI (905.12 mg, 4.72 mmol, 1.02 eq), HOPO (601.70 mg, 5.42 mmol, 1.17 eq) and triethylamine (1.87 g, 18.52 mmol, 2.58 mL, 4 eq), and the reaction solution was stirred at 40 °C for 16 hours. After the reaction was completed, the reaction solution was washed with 10 mL of water, and the organic phase was rotary-evaporated to obtain the target compound **R011-3**. ¹HNMR (400 MHz, CHLOROFORM-*d*) δ = 7.40 (d, *J* = 2.0 Hz, 1 H), 7.15-7.24 (m, 1 H), 6.71 (br s, 1 H), 6.49 (d, *J* = 8.5 Hz, 1 H), 5.49 (br s, 2 H), 3.39 (q, *J* = 5.4 Hz, 2 H), 2.43 (t, *J* = 5.9 Hz, 2 H), 2.19 (s, 6 H).

### Step 2: synthesis of compound R011-4

Compound **R011-3** (1.1 g, 3.84 mmol) was dissolved in formic acid (22.67 g, 492.48 mmol, 18.58 mL), and the reaction solution was stirred at 100 °C for 16 hours. After the reaction was completed, the reaction solution was rotary-evaporated to obtain the target compound **R011-4**. MS-ESI *m*/*z:* 297.9 [M+H]⁺.

### Step 3: synthesis of compound R011-7

Compound **R011-5** (0.2 g, 799.67 µmol) was dissolved in pyridine (3 mL). Compound **R011-6** (158.74 mg, 815.67 µmol) was added thereto, and the reaction solution was stirred at 25 °C for 10 hours. After the reaction was completed, the reaction solution was rotary-evaporated to obtain the target compound **R011-7**. ¹HNMR (400 MHz, CHLOROFORM-*d*) δ = 8.25 (br t, *J* = 7.8 Hz, 2 H), 8.17 (d, *J* = 1.5 Hz, 1 H), 8.01 (d, *J* = 1.3 Hz, 1 H), 7.67-7.75 (m, 2 H), 6.83 (br s, 1 H), 3.71 (s, 3 H), 1.26 (s, 12 H).

### Step 4: synthesis of compound R011

Compound **R011-7** (0.3 g, 734.84 µmol) was dissolved in dioxane (5 mL) and water (1 mL). Potassium acetate (288.48 mg, 2.94 mmol), compound **R011-4** (217.63 mg, 734.84 µmol) and Pd(dppf)Cl₂ (120.02 mg, 146.97 µmol) were added, and the reaction solution was stirred at 100 °C for 3 hours under nitrogen atmosphere. After the reaction was completed, the reaction solution was rotary-evaporated and separated by preparative thin-layer chromatographic plate (ethyl acetate: methanol = 10: 1) to obtain the target compound **R011.** ¹HNMR (400 MHz, DMSO-*d*₆) δ = 8.37 (s, 1 H), 8.35 (s, 1 H), 8.27 (d, *J* = 2.3 Hz, 1 H), 8.15 (s, 1 H), 8.08 (dd, *J* = 8.5, 2.3 Hz, 1 H), 7.92 (d, *J* = 2.3 Hz, 1 H), 7.83 (dd, *J* = 8.8, 5.3 Hz, 2 H), 7.78 (d, *J* = 8.5 Hz, 1 H), 7.42 (t, *J* = 8.8 Hz, 2 H), 4.12 (br t, *J* = 5.9 Hz, 2 H), 3.69 (s, 3 H), 2.63 (br t, *J* = 6.0 Hz, 2 H), 2.24 (s, 6 H). MS-ESI *m*/*z:* 498.3 [M+H]⁺.

### Comparative example 9: R012

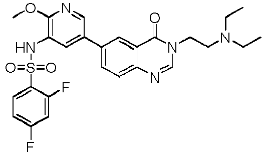

### Synthetic route:

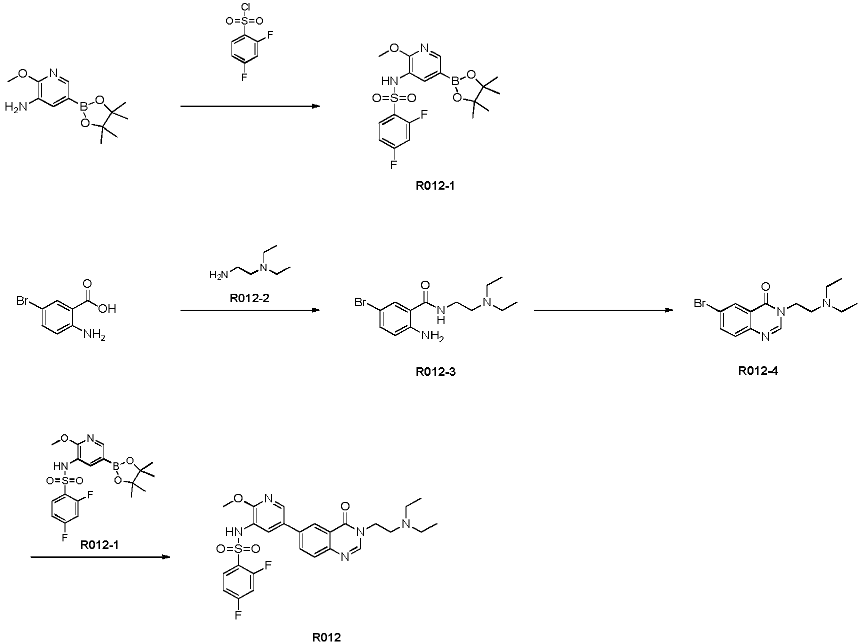

### Step 1: synthesis of compound R012-1

2-Methoxy-3-amino-5-pyridine borate (0.5 g, 2.00 mmol) was dissolved in pyridine (2.0 mL), and 2,4-difluorobenzenesulfonyl chloride (446.28 mg, 2.10 mmol) was added dropwise at 25 °C, and the reaction solution was stirred at 28 °C for 16 hours. After the reaction was completed, the organic solvent was rotary-evaporated, water (200 mL) was added and washed three times with dichloromethane (100 mL). The organic phases were combined, dried over anhydrous sodium sulfate and rotary-evaporated to obtain the target compound **R012-1.** ¹H NMR (400MHz, Methanol-d4) δ: 8.64 (d, *J* = 4.5 Hz, 1H), 8.22 (d, J = 1.8 Hz, 1H), 7.94 (d, *J* = 1.8 Hz, 1H), 7.84-7.70 (m, 1H), 7.62 (dd, *J* = 6.0, 7.8 Hz, 1H), 7.11-6.96 (m, 1H), 3.77 (s, 3H), 1.36 (s, 12H).

### Step 2: synthesis of compound R012-3

The compound 2-amino-5-bromobenzoic acid (2.0 g, 9.26 mmol) was dissolved in DMF (20.0 mL), and triethylamine (1.87 g, 18.52 mmol), HATU (3.52 g, 9.26 mmol) and **R012-2** (1.08 g, 9.26 mmol) were added and stirred at 28 °C for 2 hours. After the reaction was completed, the solvent was removed under reduced pressure, poured into water (200 mL), and extracted three times with dichloromethane (100 mL). The organic phases obtained were combined and dried over anhydrous sodium sulfate. After removing the desiccant by filtration, the solvent was removed under reduced pressure to obtain the target compound **R012-3.** ¹H NMR (400MHz, Methanol-d4) δ: 7.60 (d, *J =* 2.3 Hz, 1H), 7.27 (dd, *J* = 2.4, 8.7 Hz, 1H), 6.69 (d, *J* = 8.8 Hz, 1H), 3.47 (t, *J* = 7.0 Hz, 2H), 2.80-2.62 (m, 6H), 1.13 (t, *J* = 7.3 Hz, 6H).

### Step 3: synthesis of compound R012-4

Compound **R012-3** (1.0 g, 3.18 mmol) was dissolved in ethanol (40 mL), and methylphenidate acetate (662.64 mg, 6.36 mmol) was added, and stirred at 80 °C for 2 hours. After the reaction was completed, the solvent was removed under reduced pressure, poured into water (10 mL), and extracted three times with dichloromethane (10 mL). The organic phases obtained were combined and dried over anhydrous sodium sulfate. After removing the desiccant by filtration, the solvent was removed under reduced pressure to obtain the target compound **R012-4.** ¹H NMR (400MHz, Methanol-d4) δ: 8.35 (d, *J =* 2.3 Hz, 1H), 8.30 (s, 1H), 7.94 (dd, *J* = 2.3, 8.8 Hz, 1H), 7.62 (d, *J* = 8.5 Hz, 1H), 4.15 (t, *J* = 6.3 Hz, 2H), 2.88 (t, *J* = 6.4 Hz, 2H), 2.68 (q, *J* = 7.0 Hz, 4H), 1.01 (t, *J* = 7.2 Hz, 6H).

### Step 4: synthesis of compound R012

**R012-4** (0.2 g, 616.87 µmol) and **R012-1** (262.94 mg, 616.87 µmol) were dissolved in dioxane (5.00 mL) and water (1.00 mL), and [1,1'-bis (diphenylphosphino) ferrocene] palladium dichloride (100.75 mg, 123.37 µmol) and potassium acetate (242.17 mg, 2.47 mmol) were added, and the reaction solution was stirred at 95 °C for 2 hours under nitrogen atmosphere. After the reaction was completed, water (50 mL) was added thereto, and extracted three times with dichloromethane (10 mL), and dried over anhydrous sodium sulfate. The organic phase was rotary-evaporated to obtain an oily residue, which was separated by a thick preparation chromatographic plate (eluent: dichloromethane / methanol = 15: 1), and then separated by preparative high performance liquid column to obtain the target compound **R012.** ¹H NMR (400MHz, CDCl₃) δ : 8.31 (br s, 1H), 8.14-7.90 (m, 3H), 7.89-7.62 (m, 3H), 7.01-6.78 (m, 2H), 3.97 (br s, 2H), 3.89 (s, 3H), 2.71 (br s, 2H), 2.48 (q*, J* = 6.5 Hz, 4H), 0.87 (br t, *J* = 6.9 Hz, 6H). MS-ESI m/z: 544.2[M+H]⁺,546.2[M+H+2]⁺.

### Example 1: WX001

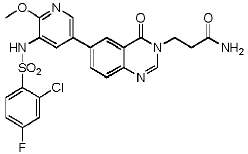

### Synthetic route:

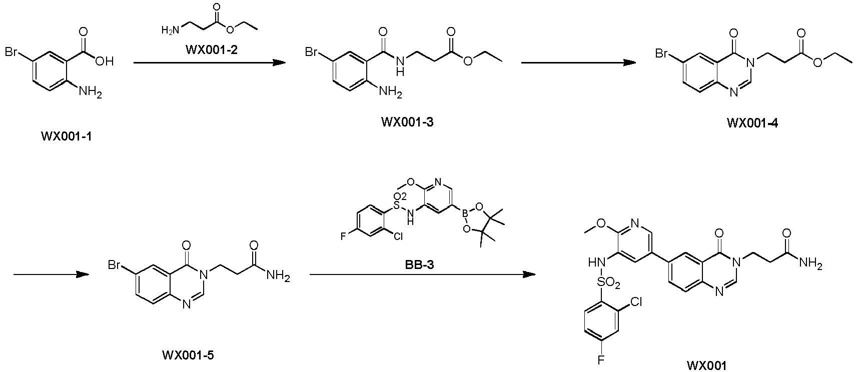

### Step 1: synthesis of compound WX001-3

**WX001-1** (500.00 mg, 2.31 mmol), **WX001-2** (350.00 mg, 2.28 mmol, 1.0 HCl), triethylamine (730.00 mg, 7.21 mmol, 1.00 mL), 2-hydroxypyridine *N*-oxide (260.00 mg, 2.34 mmol) and 1-(3-dimethylaminopropyl)-3-acetaldehyde hydrochloride (450.00 mg, 2.35 mmol) were dissolved in dichloromethane (50.00 mL) and stirred at 50 °C for 16 hours. After the reaction was completed, the mixture was washed with water (50 mL), and the organic phase was concentrated to obtain the target compound **WX001-3,** which was directly used in the next step. MS-ESI *m*/*z*: 315.0 [M+H]⁺, 317.0 [M+H+2]⁺.

### Step 2: synthesis of compound WX001-4

Compound **WX001-3** (900.00 mg, 2.52 mmol) was dissolved in formic acid solution (20 mL) and stirred at 100 °C for 16 hours. After the reaction was completed, the reaction solution was concentrated under reduced pressure to obtain the target compound **WX001-4**, which was directly used in the next step. ¹H NMR (400 MHz, CHLOROFORM-*d*) δ: 8.36 (d, *J* = 2.26 Hz, 1 H) 8.13-8.24 (m, 1 H) 7.73-7.82 (m, 1 H) 7.49-7.58 (m, 1 H) 4.19 (t, *J* = 6.02 Hz, 2 H) 4.07 (q, *J* = 7.03 Hz, 2 H) 2.82 (t, *J*= 6.02 Hz, 2 H) 1.09-1.20 (m, 3 H). MS-ESI *m*/*z:* 324.9 [M+H]⁺, 326.9 [M+H+2]⁺.

### Step 3: synthesis of compound WX001-5

At 0 °C, ammonia gas was introduced into a solution of **WX001-4** (950.00 mg, 2.92 mmol) in methanol (30 mL) for half an hour. Then, the reaction solution was stirred at 60 °C for 16 hours in a muffle tank. After the reaction was completed, the mixture was cooled to room temperature and concentrated under reduced pressure to obtain **WX001-5**, which was directly used in the next step. MS-ESI *m*/*z:* 296.0 [M+H]⁺, 298.0 [M+H+2]⁺.

### Step 4: synthesis of compound WX001

**WX001-5** (200.00 mg, 675.40 µmol), **BB-3** (300.00 mg, 648.38 µmol), [1,1-bis(diphenylphosphino) ferrocene] palladium dichloride (55.00 mg, 75.17 µmol) and potassium acetate (300.00 mg, 3.06 mmol) were dissolved in a mixture of dioxane (10 mL) and water (2 mL) under nitrogen atmosphere, and stirred at 100 °C for two hours. After the reaction was completed, the mixture was washed with water (30 mL) and extracted with dichloromethane (30 mL × 2). The organic phase was evaporated under reduced pressure, and the resulting residue was separated by a chromatography column (eluent: dichloromethane / methanol = 0%-5%) to obtain the target compound **WX001.** ¹H NMR (400 MHz, CHLOROFORM-d) δ: 8.34 (d, *J =* 2.26 Hz, 1 H) 8.28 (s, 1 H) 8.11-8.21 (m, 2 H) 7.99 (d, *J =* 2.26 Hz, 1 H) 7.77-7.91 (m, 2 H) 7.56 (s, 1 H) 7.27 (d, *J=* 2.51 Hz, 1 H) 7.09-7.18 (m, 1 H) 5.33-5.63 (m, 2 H) 4.35 (t, *J=* 6.02 Hz, 2 H) 4.00 (s, 3 H) 2.86 (t, *J=* 6.02 Hz, 2 H). MS-ESI *m*/*z:* 532.1 [M+H]⁺.

### Example 2: WX002

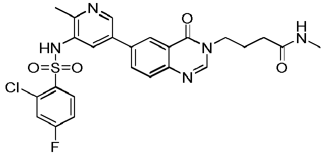

### Synthetic route:

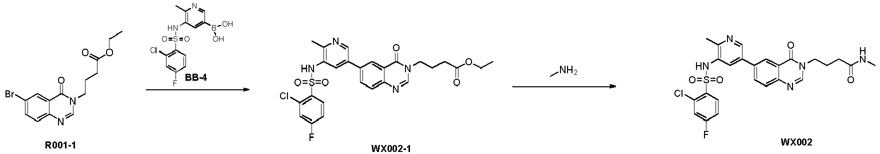

### Step 1: synthesis of compound WX002-1

Compound **R001-1** (200.00 mg, 589.66 µmol), **BB-4** (203.17 mg, 589.66 µmol) and potassium acetate (231.48 mg, 2.36 mmol) were dissolved in dioxane (1 mL) and water (1 mL), followed by the addition of ferrocene palladium dichloride (8.63 mg, 11.79 µmol). The reaction solution was heated to 100 °C and stirred for 2 hours under nitrogen atmosphere. After the reaction was completed, the mixture was cooled to room temperature. The organic solvent was rotary-evaporated, poured into water (10 mL), and extracted with dichloromethane (10 mL × 3). The organic phases obtained were combined and dried over anhydrous sodium sulfate. After the desiccant was removed by filtration, the solvent was removed under reduced pressure, separated by a preparative chromatographic plate (eluent: methanol/dichloromethane = 1:10), and further separated by a preparative high-performance liquid column to obtain the target compound **WX002-1.** ¹H NMR (400MHz, Methanol-*d₄*) δ : 8.61 (d, *J* = 2.3 Hz, 1H), 8.41-8.30 (m, 2H), 8.09 (dd, *J* = 5.8, 9.0 Hz, 1H), 8.02 (dd, *J* = 2.0, 8.5 Hz, 1H), 7.89 (d, *J=* 2.3 Hz, 1H), 7.85-7.76 (m, 1H), 7.80 (d, *J=* 8.5 Hz, 1H), 7.55 (dd, *J=* 2.5, 8.5 Hz, 1H), 7.30-7.17 (m, 1H), 4.20-4.14 (m, 2H), 4.22-4.00 (m, 2H), 2.58-2.41 (m, 5H), 2.30-2.03 (m, 2H), 1.23 (t, *J* = 7.0 Hz, 3H). MS-ESI m/z: 558.09[M+H]⁺, 560.9[M+H+2]⁺.

### Step 2: synthesis of compound WX002

The compound **WX002-1** (200.00 mg, 357.78 µmol) was dissolved in a methylamine alcohol solution (20 mL), and heated to 80 °C and stirred for 2 hours. After the reaction was completed, the mixture was cooled to room temperature. The organic solvent was rotary-evaporated, separated by a preparative chromatographic plate (eluent: methanol/dichloromethane/triethylamine = 1: 15: 0.15), and further separated by a preparative high-performance liquid phase column to obtain the target compound **WX002.** ¹H NMR (400MHz, Methanol-*d₄*) δ : 8.59 (s, 1H), 8.42-8.30 (m, 2H), 8.09 (dd, *J=* 5.8, 8.8 Hz, 1H), 8.03 (br d, *J=* 8.5 Hz, 1H), 7.89 (d, *J=* 2.0 Hz, 1H), 7.81 (br d, *J=* 8.8 Hz, 1H), 7.54 (dd, *J=* 2.5, 8.5 Hz, 1H), 7.36-7.16 (m, 1H), 4.15 (t, *J* = 6.9 Hz, 2H), 2.68 (s, 3H), 2.50 (s, 3H), 2.39-2.24 (m, 2H), 2.14 (quin, *J =* 7.0 Hz, 2H). MS-ESI m/z: 543.9[M+H]+, 545.9[M+H+2]+.

### Example 3: WX003

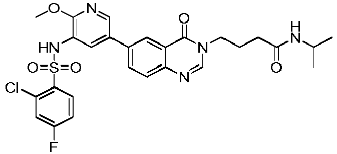

### Synthetic route:

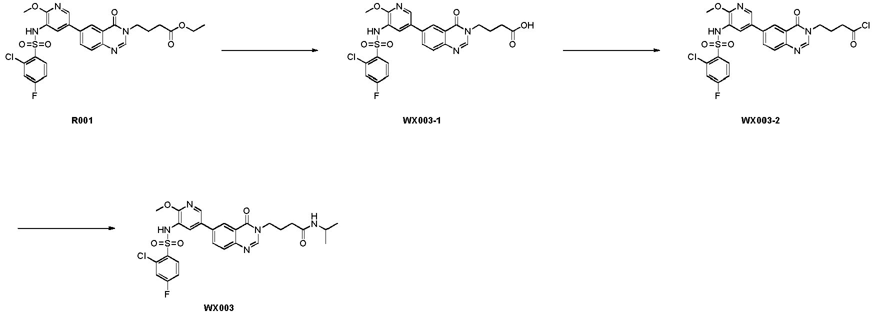

### Step 1: synthesis of compound WX003-1

Compound **R001** (800.00 mg, 1.39 mmol) was dissolved in tetrahydrofuran (10.0 mL) and water (10.0 mL), then lithium hydroxide (233.51 mg, 5.57 mmol) was added, and the reaction solution was stirred at 10 °C for 1 hour. After the reaction was completed, the reaction solution was extracted with dichloromethane (10 mL × 3). The aqueous phase was adjusted to pH=2 with dilute hydrochloric acid (1 M) at 0 °C, and extracted with dichloromethane (100 mL × 3). The organic phases were combined and dried over anhydrous sodium sulfate. After the desiccant was removed by filtration, the solvent was removed under reduced pressure to obtain the target compound **WX003-1.** MS-ESI m/z: 547.0[M+H]⁺, 549.0[M+H+2]⁺.

### Step 2: synthesis of compound WX003-2

Compound **WX003-1** (200.00 mg, 365.66 µmol) was dissolved in sulfoxide chloride (5.0 mL), heated to 80 °C and refluxed for 1 hour. After the reaction was completed, the solvent was removed under reduced pressure to obtain the target compound **WX003-2,** which was directly used in the next step.

### Step 3: synthesis of compound WX003

The compound isopropylamine (12.55 mg, 212.24 µmol) and triethylamine (35.79 mg, 353.74 µmol) were dissolved in anhydrous dichloromethane (5 mL), and the solution of **WX003-2** (100.00 mg, 176.87 µmol) in dichloromethane (1 mL) was added dropwise with stirring at 0 °C. The reaction solution was stirred at 0 °C for 1 hour. After the reaction was completed, the solvent was removed under reduced pressure. The residue was separated by a chromatographic plate (eluent: methanol / dichloromethane/triethylamine = 1:15:0.15), and further separated by a high performance liquid preparation column (Phenomenex Gemini C18 250 * 50mm * 10 µm; mobile phase: [water and water (0.04% NH₄HCO₃) -ACN]; B%: 10% -40%, 40min) to obtain the target compound **WX003.** ¹H NMR (400MHz, CDCl₃) δ : 8.37 (d, *J* = 1.8 Hz, 1H), 8.25-8.07 (m, 3H), 8.00 (d, *J* = 2.3 Hz, 1H), 7.92-7.73 (m, 2H), 7.55 (s, 1H), 7.30 (br s, 1H), 7.18-7.01 (m, 1H), 5.73-5.59 (m, 1H), 4.19-4.05 (m, 3H), 4.00 (s, 3H), 2.34-2.13 (m, 4H), 1.19 (d, *J* = 6.5 Hz, 6H). MS-ESI m/z: 588.1[M+H]+, 560.1[M+H+2]+.

### Example 4: WX004

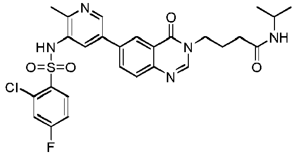

### Synthetic route:

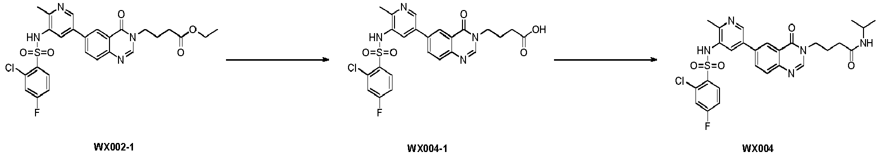

### Step 1: synthesis of compound WX004-1

Compound **WX002-1** (0.3 g, 536.66 µmol) was dissolved in tetrahydrofuran (10 mL) and water (10 mL), then lithium hydroxide (90.08 mg, 2.15 mmol) was added, and the reaction solution was stirred at 15 °C for 2 hours. After the reaction was completed, the reaction solution was extracted with dichloromethane (10 mL × 3). The aqueous phase was adjusted to pH=2 with dilute hydrochloric acid (1 M) at 0 °C, and extracted with dichloromethane (10 mL × 3). The organic phases were combined and dried over anhydrous sodium sulfate. After the desiccant was removed by filtration, the solvent was removed under reduced pressure to obtain the target compound **WX004-1,** which was directly used in the next step. MS-ESI m/z: 531.0[M+H]⁺, 532.0[M+H+2]⁺.

### Step 2: synthesis of compound WX004

Compound **WX004-1** (50 mg, 94.17 µmol) was dissolved in dichloromethane (3 mL), followed by addition of TEA(19.06 mg, 188.34 µmol), HATU (35.81 mg, 94.17 µmol) and isopropylamine (6.12 mg, 103.59 µmol), and stirred at 10 °C for 2 hours. After the reaction was completed, the mixture was poured into water (10 mL) and extracted with dichloromethane (10 mL × 3). The organic phases obtained were combined and dried over anhydrous sodium sulfate. After the desiccant was removed by filtration, the solvent was removed under reduced pressure. The residue was separated by a chromatographic plate (eluent: methanol / dichloromethane/triethylamine = 1:15:0.15), and further separated by a preparative high-performance liquid column to obtain the target compound **WX004.** ¹H NMR (400MHz, Methanol-*d₄*) δ : 8.57 (d, *J* = 2.0 Hz, 1H), 8.44-8.30 (m, 2H), 8.09 (dd, *J* = 5.8, 8.8 Hz, 1H), 8.02 (dd, *J* = 2.3, 8.5 Hz, 1H), 7.89 (d, *J* = 2.0 Hz, 1H), 7.81 (d, *J* = 8.5 Hz, 1H), 7.53 (dd, *J* = 2.5, 8.3 Hz, 1H), 7.41-7.07 (m, 1H), 4.15 (t, *J* = 7.0 Hz, 1H), 4.23-4.09 (m, 1H), 3.97-3.83 (m, 1H), 2.51 (s, 3H), 2.34-2.23 (m, 2H), 2.17-2.03 (m, 2H), 1.12 (d, *J* = 6.5 Hz, 6H). MS-ESI m/z: 572.0[M+H]⁺, 574.0[M+H+2]⁺.

### Example 5: WX005

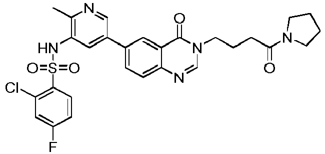

### Synthetic route:

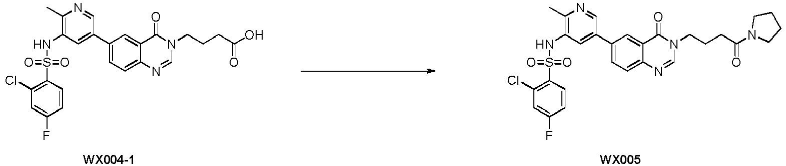

### Step 1: synthesis of compound WX005

Compound **WX004-1** (50 mg, 94.17 µmol) was dissolved in dichloromethane (3 mL), followed by addition of TEA(19.06 mg, 188.34 µmol), HATU (35.81 mg, 94.17 µmol) and tetrahydropyrrole (7.37 mg, 103.59 µmol), and stirred at 10 °C for 2 hours. After the reaction was completed, the mixture was poured into water (10 mL) and extracted with dichloromethane (10 mL × 3). The organic phases obtained were combined and dried over anhydrous sodium sulfate. After the desiccant was removed by filtration, the solvent was removed under reduced pressure. The residue was separated by a chromatographic plate (eluent: methanol/dichloromethane/triethylamine = 1:15:0.15), and further separated by a preparative high-performance liquid column to obtain the target compound **WX005.** ¹H NMR (400MHz, 400MHz, Methanol-*d₄*) δ : 8.58 (s, 1H), 8.40-8.26 (m, 2H), 8.09 (dd, *J* = 5.9, 8.9 Hz, 1H), 8.02 (br d, *J* = 8.5 Hz, 1H), 7.89 (s, 1H), 7.79 (dd, *J* = 3.5, 8.3 Hz, 1H), 7.54 (dd, *J* = 2.5, 8.5 Hz, 1H), 7.41-7.18 (m, 1H), 4.18 (br t, *J* = 6.8 Hz, 2H), 3.49 (t, *J* = 6.8 Hz, 2H), 3.30-3.23 (m, 2H), 3.30-3.22 (m, 2H), 2.50 (m, 5H), 2.17 (quin, *J* = 6.8 Hz, 2H), 1.96 (quin, *J* = 6.8 Hz, 2H), 1.82 (quin, *J* = 6.8 Hz, 2H). MS-ESI m/z: 583.9[M+H]⁺, 586.0[M+H+2]⁺.

### Example 6: WX006

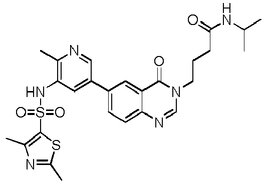

### Synthetic route:

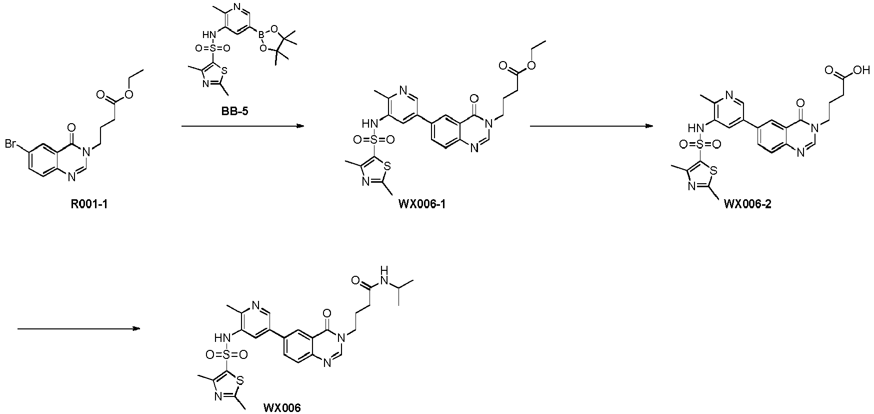

### Step 1: synthesis of compound WX006-1

Compound **R001-1** (500 mg, 1.53 mmol), **BB-5** (518.33 mg, 1.53 mmol) and potassium acetate (599.90 mg, 6.11 mmol) were dissolved in dioxane (5 mL) and water (1 mL), followed by addition of ferrocene palladium dichloride (249.59 mg, 305.63 µmol), and heated to 100 °C and stirred for 2 hours under nitrogen atmosphere. After the reaction was completed, the mixture was cooled to room temperature. The organic solvent was rotary-evaporated, poured into water (100 mL), and extracted with dichloromethane (100 mL × 3). The organic phases obtained were combined and dried over anhydrous sodium sulfate. After the desiccant was removed by filtration, the solvent was removed under reduced pressure. The residue was separated by a preparative chromatographic plate (eluent: methanol/dichloromethane = 1:10), and further separated by a preparative high-performance liquid column to obtain the target compound **WX006-1.** MS-ESI m/z: 542.1[M+H]⁺, 544.1[M+H+2]⁺.

### Step 2: synthesis of compound WX006-2

Compound **WX006-1** (0.3 g, 553.87 µmol) was dissolved in tetrahydrofuran (10.0 mL) and water (10.0 mL), followed by addition of lithium hydroxide (92.97 mg, 2.22 mmol), and the reaction solution was stirred at 15 °C for 2 hours. After the reaction was completed, the reaction solution was extracted with dichloromethane (10 mL × 3). The aqueous phase was adjusted to pH=2 with dilute hydrochloric acid (1 M) at 0 °C, and extracted with dichloromethane (10 mL × 3). The aqueous phase was collected and the solvent was removed under reduced pressure, followed by addition of methanol (60 mL) and filtration. The mother liquor was concentrated under reduced pressure to obtain the target compound **WX006-2,** which was directly used in the next step. MS-ESI m/z: 515.0[M+H]⁺, 515.8[M+H+2]⁺.

### Step 3: synthesis of compound WX006

Compound **WX006-2** (0.12 g, 233.65 µmol) was dissolved in DMF (3 mL), then DIEA (60.40 mg, 467.30 µmol), HATU (88.84 mg, 233.65 µmol) and isopropylamine (13.81 mg, 233.65 µmol) were added and stirred at 10 °C for 2 hours. After the reaction was completed, the mixture was poured into water (10 mL) and extracted with dichloromethane (10 mL × 3). The organic phases obtained were combined and dried over anhydrous sodium sulfate. After the desiccant was removed by filtration, the solvent was removed under reduced pressure. The residue was separated by a preparative chromatographic plate (eluent: methanol/dichloromethane = 1:15), and further separated by a preparative high-performance liquid column to obtain the target compound **WX006.** ¹H NMR (400MHz, CDCl₃) δ : 8.62 (d, *J* = 2.0 Hz, 1H), 8.39 (d, *J* = 2.0 Hz, 1H), 8.06 (s, 1H), 7.98-7.83 (m, 2H), 7.81-7.69 (m, 1H), 5.54 (br s, 1H), 5.35-5.13 (m, 1H), 4.16-4.03 (m, 2H), 2.60 (s, 3H), 2.37 (d, *J* = 7.0 Hz, 6H), 2.22-2.00 (m, 1H), 2.22-2.00 (m, 4H), 1.10 (d, *J* = 6.5 Hz, 6H). MS-ESI m/z: 555.2[M+H]⁺, 557.0[M+H+2]⁺.

### Example 7: WX007

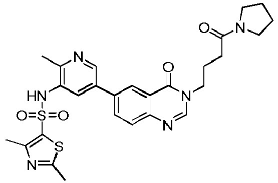

### Synthetic route:

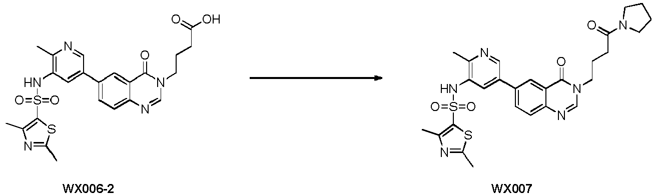

### Step 1: synthesis of compound WX007

Compound **WX006-2** (50 mg, 94.17 µm) was dissolved in dichloromethane (3 mL), followed by addition ofDIEA(60.39 mg, 467.30 µmol), HATU (88.84 mg, 233.65 µmol) and tetrahydropyrrole (16.62 mg, 233.65 µmol), and stirred at 10 °C for 2 hours. After the reaction was completed, the mixture was poured into water (10 mL) and extracted with dichloromethane (10 mL × 3). The organic phases times were combined and dried over anhydrous sodium sulfate. After the desiccant was removed by filtration, the solvent was removed under reduced pressure. The residue was separated by a preparative chromatographic plate (eluent: methanol/dichloromethane = 1:15), and further separated by a preparative high-performance liquid column to obtain the target compound **WX007.** ¹H NMR (400MHz, CDCl₃) δ : 8.62 (d, *J* = 2.0 Hz, 1H), 8.38 (d, *J* = 2.0 Hz, 1H), 8.09 (s, 1H), 7.91 (d, *J* = 2.0 Hz, 1H), 7.85 (dd, *J* = 2.1, 8.4 Hz, 1H), 7.76 (d, *J* = 8.3 Hz, 1H), 5.28 (br s, 1H), 4.11 (t, *J* = 7.2 Hz, 2H), 3.46-3.27 (m, 4H), 2.61 (s, 3H), 2.45-2.27 (m, 8H), 2.19-2.05 (m, 2H), 1.89 (td, *J* = 7.1, 13.7 Hz, 2H), 1.82-1.74 (m, 2H). MS-ESI m/z: 567.2[M+H]⁺, 568.1[M+H+2]⁺.

### Example 8: WX008, WX009

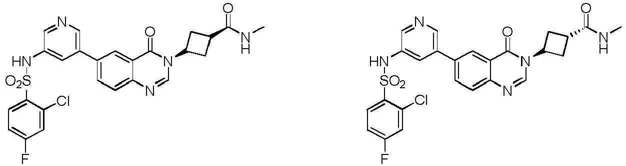

### Synthetic route:

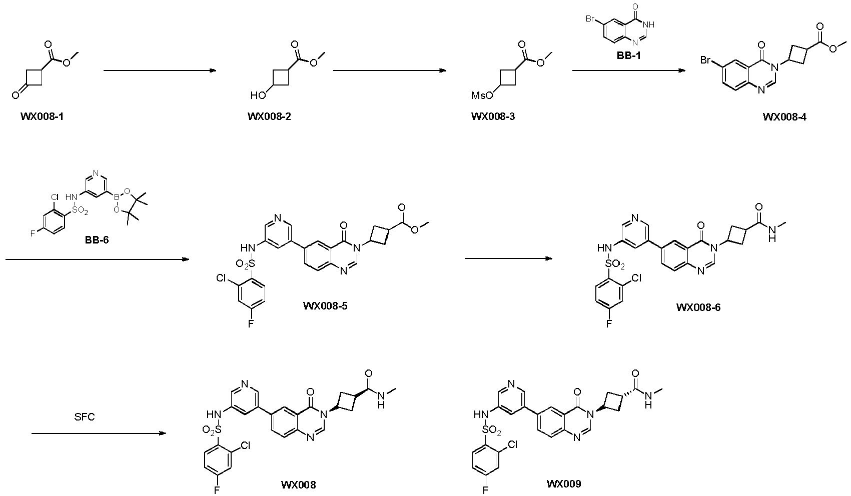

### Step 1: synthesis of compound WX008-2

Compound **WX008-1** (2.00 g, 15.61 mmol) was dissolved in methanol (5 mL), and then sodium borohydride (590.49 mg, 15.61 mmol) was added at 0 °C, and the reaction solution was stirred at 0 °C for 2 hours. After the reaction was completed, the mixture was cooled to room temperature, quench with a saturated NH₄Cl (5 mL) solution and extracted with ethyl acetate (15 mL × 3). The organic phases obtained were combined and dried over anhydrous sodium sulfate. After the desiccant was removed by filtration, the solvent was removed under reduced pressure to obtain the target compound **WX008-2**. ¹H NMR (400MHz, Methanol-*d₄*) δ: 4.14-4.11 (m, 1H), 3.69-3.65 (s, 3H), 2.70-2.57 (m, 1H), 2.55-2.43 (m, 2H), 2.16-2.07 (m, 2H).

### Step 2: synthesis of compound WX008-3

Compound **WX008-2** (500.00 mg, 3.84 mmol) and triethylamine (777.14 mg, 7.68 mmol) were dissolved in dichloromethane (5 mL), and then methylsulfonyl chloride (1.72 g, 14.98 mmol) was added at 0 °C, and the reaction solution was stirred at 0 °C for 2 hours. After the reaction was completed, the mixture was cooled to room temperature, quenched with water (5 mL), and extracted with ethyl acetate (15 mL × 3). The organic phases obtained were combined and dried over anhydrous sodium sulfate. After the desiccant was removed by filtration, the solvent was removed under reduced pressure to obtain the target compound **WX008-3**. ¹H NMR (400MHz, METHANOL-*d₄*) δ : 5.03-4.90 (m, 1H), 4.85 (br s, 2H), 3.72-3.69 (s, 3H), 3.08 (s, 3H), 2.91-2.79 (m, 1H), 2.76-2.66 (m, 2H), 2.55-2.42 (m, 2H).

### Step 3: synthesis of compound WX008-4

Compound **BB-1** (600.00 mg, 2.67 mmol), **WX008-3** (555.18 mg, 2.67 mmol) and cesium carbonate (1.74 g, 5.33 mmol) were dissolved in *N*,*N*-dimethylformamide formaldehyde (5 mL), then heated to 60 °C and stirred for 6 hours. After the reaction was completed, the mixture was cooled to room temperature. After the reaction was completed, the mixture was cooled to room temperature, the organic solvent was rotary-evaporated, poured into water (100 mL), and extracted with ethyl acetate (50 mL × 3). The organic phases obtained were combined and dried over anhydrous sodium sulfate. After the desiccant was removed by filtration, the solvent was removed under reduced pressure and separated by a chromatographic plate (eluent: petroleum ether/ethyl acetate = 3:1) to obtain the target compound **WX008-4.** MS-ESI m/z: 337.0[M+H]⁺,339.0[M+H+2]⁺.

### Step 4: synthesis of compound WX008-5

The suspension of **BB-6** (0.2 g, 290.06 µmol, purity: 59.85%), **WX008-4** (0.177 g, 289.99 µmol), potassium acetate (0.114 g, 1.16 mmol) and [1,1'-bis (diphenylphosphino) ferrocene] palladium dichloride (0.021 g, 28.70 µmol) in dioxane (8 mL) and water (1.6 mL) was replaced with nitrogen three times, and the reaction solution was then heated to 100 °C and stirred for 60 minutes. After the reaction was completed, the reaction solution was concentrated and rotary-evaporated. The crude product was slurried with water (50 mL), and then extracted with dichloromethane (50 mL). The organic phase was rotary-evaporated and separated by column chromatography (MeOH: DCM = 0%-10%) to obtain **WX008-5.** MS-ESI *m*/*z*: 542.9 [M+H]⁺.

### Step 5: synthesis of compound WX008-6

The suspension of **WX008-5** (0.25 g) in methylamine (10 mL) was stirred at 80 °C for 16 hours. After the reaction was completed, the reaction solution was concentrated, rotary-evaporated and separated by preparative thin layer plate (DCM: MeOH = 10: 1) to obtain the target compound **WX008-6.**

### Step 6: synthesis of compound WX008 and WX009

WX008-6 was resolved by SFC (column: OD (250mm * 30mm, 5µm); mobile phase: [0.1% NH₄HCO₃ EtOH]; B%: 40%-40%) to obtain cis-trans isomer **WX008** (Rt = 4.034 min) and **WX009** (Rt = 4.829 min). By NOX, **WX008** was determined as a cis isomer, ¹H NMR (400 MHz, METHANOL-*d*₄) δ ppm 8.42 (d, *J* = 1.25 Hz, 1 H) 8.34 (s, 1 H) 8.23 (d, *J* = 2.01 Hz, 1 H) 8.10-8.18 (m, 2 H) 7.86 (dd, *J* = 8.53, 2.26 Hz, 1 H) 7.75 (t, *J* = 2.26 Hz, 1 H) 7.63 (d, *J* = 8.53 Hz, 1 H) 7.33 (dd, *J* = 8.53, 2.51 Hz, 1 H) 7.18 (ddd, *J* = 8.91, 7.91, 2.51 Hz, 1 H) 4.82-4.98 (m, 1 H) 2.82-2.95 (m, 1 H) 2.63-2.74 (m, 5 H) 2.48-2.60 (m, 2 H), MS-ESI *m*/*z:* 542.0 [M+H]⁺. **WX009** as a trans isomer, ¹H NMR (400 MHz, METHANOL-*d*₄) δ ppm 8.44 (br s, 1 H) 8.29 (s, 1 H) 8.18-8.26 (m, 2 H) 8.15 (dd, *J* = 8.91, 5.90 Hz, 1 H) 7.90 (br d, *J* = 8.53 Hz, 1 H) 7.77 (s, 1 H) 7.66 (d, *J* = 8.53 Hz, 1 H) 7.28-7.41 (m, 1 H) 7.19 (br t, *J* = 8.28 Hz, 1 H) 5.22 (quin, *J* = 8.60 Hz, 1 H) 3.00-3.11 (m, 1 H) 2.73-2.86 (m, 2 H) 2.57-2.70 (m, 5 H), MS-ESI *m*/*z:* 542.0 [M+H]⁺.

### Example 9: WX010, WX011

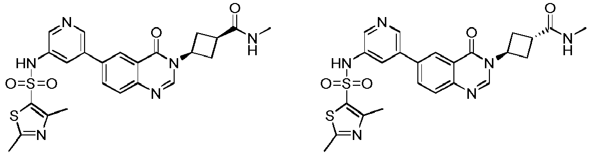

### Synthetic route:

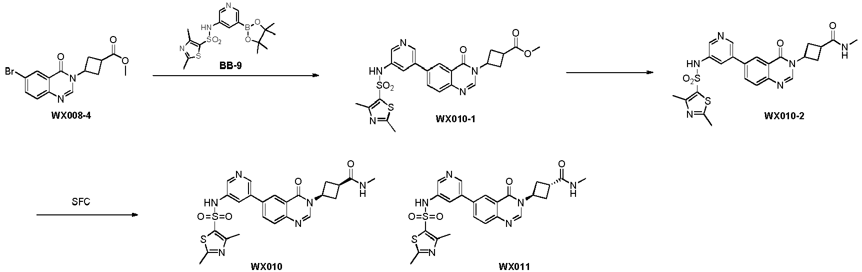

### Step 1: synthesis of compound WX010-1

The suspension **of BB-9** (0.28 g, 381.85 µmol, purity: 53.91%), **WX008-4** (0.233 g, 381.74 µmol, purity: 55.24%), potassium acetate (0.15 g, 1.53 mmol) and [1,1'-bis (diphenylphosphino) ferrocene] palladium dichloride (0.028 g, 38.27 µmol) in dioxane (8 mL) and water (1.6 mL) was replaced with nitrogen three times, and the reaction solution was then heated to 100 °C and stirred for 60 minutes under nitrogen atmosphere. After the reaction was completed, the reaction solution was concentrated and rotary-evaporated. The crude product was slurried with water (100 mL), and then extracted with dichloromethane (100 mL). The organic phase was rotary-evaporated and separated by column chromatography (MeOH: DCM = 0% ∼ 10%) to obtain **WX010-1.** MS-ESI *m*/*z:* 526.0 [M+H]⁺.

### Step 2: synthesis of compound WX010-2

The suspension of **WX010-1** (0.15 g, 158.31 µmol) in methylamine (10 mL) was stirred at 80 °C for 16 hours. After the reaction was completed, the reaction solution was concentrated, rotary-evaporated and separated by preparative thin layer plate (DCM: MeOH = 10:1) to obtain the target compound **WX010-2.**

### Step 3: synthesis of compound WX010 and WX011

**WX010-2** was resolved by SFC (chromatographic column: OD (250mm * 30mm, 5µm); mobile phase: [0.1% NH₄HCO₃ EtOH]; B%: 40% -40%) to obtain the target compound **WX010** (Rt = 3.703 min) and **WX011** (Rt = 4.458 min). By NOX, **WX010** was determined as a cis isomer, ¹H NMR (400 MHz, METHANOL-*d*4) δ : 8.33 (s, 1 H) 8.20-8.29 (m, 2H) 8.14 (d, *J* = 1.76 Hz, 1H) 7.92 (dd, *J* = 8.41, 2.13 Hz, 1 H) 7.71 (t, *J* = 2.13 Hz, 1 H) 7.67 (d, *J* = 8.53 Hz, 1 H) 4.83-4.97 (m, 1 H) 2.82-2.96 (m, 1 H) 2.62-2.72 (m, 5H) 2.53-2.61 (m, 2 H) 2.51 (s, 3 H) 2.40 (s, 3 H), MS-ESI *m*/*z:* 525.1 [M+H]⁺. **WX011** as a trans isomer, ¹H NMR (400 MHz, METHANOL-*d*₄) δ : 8.52 (s, 1 H) 8.19-8.35 (m, 3 H) 7.93 (dd, *J* = 8.53, 2.26 Hz, 1 H) 7.90-7.99 (m, 1 H) 7.79 (t, *J* = 2.01 Hz, 1 H) 7.66 (d, *J* = 8.53 Hz, 1 H) 5.21 (quin, *J* = 8.60 Hz, 1 H) 3.00-3.12 (m, 1 H) 2.74-2.86 (m, 2 H) 2.59-2.72 (m, 4 H) 2.59-2.72 (m, 1 H) 2.52 (s, 3 H) 2.37 (s, 3 H), MS-ESI *m*/*z:* 525.1 [M+H]⁺.

### Example 10: WX012, WX013

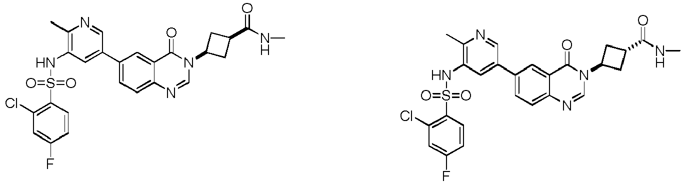

### Synthetic route:

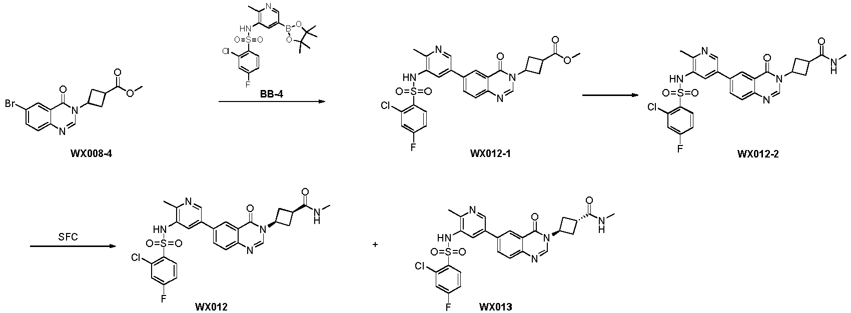

### Step 1: synthesis of compound WX012-1

Compound **WX008-4** (150.00 mg, 444.88 µmol), **BB-4** (189.83 mg, 444.88 µmol) and potassium acetate (174.64 mg, 1.78 mmol) were dissolved in dioxane (5 mL) and water (1 mL), and then Pd(dppf)Cl₂ (65.10 mg, 88.98 µmol) was added, heated to 95 °C and stirred for 3 hours under nitrogen atmosphere. After the reaction was completed, the mixture was cooled to room temperature. The organic solvent was rotary-evaporated, poured into water (10 mL), and extracted with dichloromethane (10 mL × 3). The organic phases obtained were combined and dried over anhydrous sodium sulfate. After the desiccant was removed by filtration, the solvent was removed under reduced pressure and separated by a chromatographic plate (eluent: methanol/dichloromethane/triethylamine = 1:10:0.01) to obtain the target compound **WX012-1.** MS-ESI m/z: 556.9[M+H]⁺,558.9[M+H+2]⁺.

### Step 2: synthesis of compound WX012-2

**WX012-1** (200.00 mg, 359.07 µmol) was dissolved in methylamine alcohol solution (20 mL), heated to 80 °C and stirred overnight. After the reaction was completed, the mixture was cooled to room temperature. The organic solvent was rotary-evaporated. The residue was separated by preparative chromatographic plate (eluent: methanol/dichloromethane/triethylamine = 1:15:0.15), and further separated by a preparative high-performance liquid column (Water s Xbridge 150 * 25mm 5µµm; mobile phase: [water water (10mM NH₄HCO₃) -ACN]; B%: 5% -35%, 10min) to obtain compound **WX012-2.**

### Step 3: synthesis of compound WX012 and WX013

**WX012-2** was resolved by supercritical fluid chromatography (separation conditions chromatographic column: OJ (250mm * 30mm, 5µm); mobile phase: [0.1% NH₄HCO₃ EtOH]; B%: 35% -35%) to obtain cis-trans isomers **WX012** and **WX013,** the retention time of which is 1.447 min and 1.686 min respectively, and the ratio is 1:1. By NOE, **WX012** was determined as a cis isomer. ¹H NMR (400MHz, CDCl₃)) δ : 8.50 (d, *J* = 2.0 Hz, 1H), 8.36-8.17 (m, 2H), 8.03 (dd, *J* = 5.8, 8.8 Hz, 1H), 7.87-7.66 (m, 3H), 7.24 (dd, *J* = 2.4, 7.9 Hz, 1H), 7.12-6.96 (m, 1H), 5.73 (br s, 1H), 5.15-4.93 (m, 1H), 2.83-2.62 (m, 8H), 2.47 (s, 3H). MS-ESI m/z: 555.9[M+H]⁺,557.9[M+H+2]⁺. **WX013** as a trans isomer, ¹H NMR (400MHz, CDCl₃) δ : 8.51 (d, *J* = 1.8 Hz, 1H), 8.26 (d, *J* = 2.0 Hz, 1H), 8.10-7.88 (m, 2H), 7.91-7.52 (m, 3H), 7.37-7.23 (m, 1H), 7.11-6.88 (m, 1H), 5.48 (br s, 1H), 5.14 (quin, *J* = 8.4 Hz, 1H), 3.11-2.95 (m, 1H), 2.93-2.67 (m, 7H), 2.47 (s, 3H). MS-ESI m/z: 555.9[M+H]⁺, 557.9[M+H+2]⁺.

### Example 11: WX014, WX015

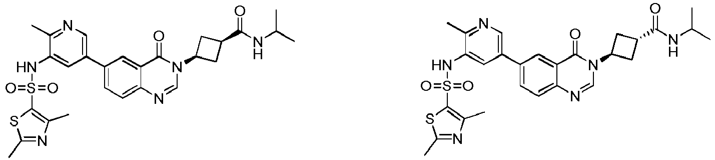

### Synthetic route:

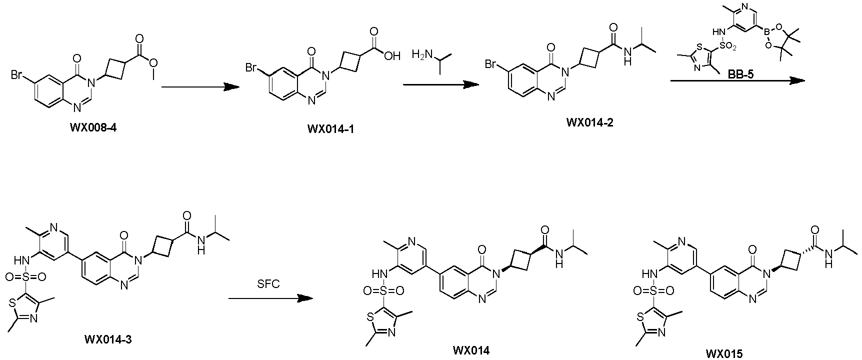

### Step 1: synthesis of compound WX014-1

Compound **WX008-4** (0.5 g, 1.48 mmol) and lithium hydroxide monohydrate (124.45 mg, 2.97 mmol) were dissolved in methanol (4.00 mL), tetrahydrofuran (4 ml) and water (4 mL), and stirred at 20 °C for 12 hours. The reaction solution was rotary-evaporated, diluted with water (20 mL), adjusted to pH = 4 with hydrochloric acid (2 mol/L), and extracted with ethyl acetate (20 mL × 3). The organic phase was rotary-evaporated to obtain the target compound **WX014-1.**

### Step 2: synthesis of compound WX014-2

Compound **WX014-1** (0.22 g, 680.82 µmol) was dissolved in *N,N-*dimethylformamide (5 mL), and *N*,*N*-diisopropylethylamine (351.95 mg, 2.72 mmol), isopropylamine (120.73 mg, 2.04 mmol, 175.48 µL) and 2-(7-benzobenzotriazole)-*N*,*N*,*N*,*N*-tetramethylurea hexafluorophosphate (388.30 mg, 1.02 mmol) were added at 0 °C under nitrogen atmosphere, and the mixture was stirred at 20 °C for 12 hours. Water (20 mL) was added to the reaction solution. The reaction solution was extracted with ethyl acetate (15 mL × 3), and the organic phase was collected and washed with water (10 mL × 2) and saturated sodium chloride solution (10 mL), respectively. After the organic phase was rotary-evaporated, the target compound **WX014-2** was obtained.

### Step 3: synthesis of compound WX014-3

Compound **WX014-2** (0.33 g, 906.01 µmol), compound **BB-5** (296.43 mg, 906.01 µmol), potassium acetate (355.66 mg, 3.62 mmol) and ferrocene palladium chloride (132.59 mg, 181.20 µmol) were dissolved in dioxane (10 mL) and water (1.5 mL), and the reaction solution was stirred at 100 °C for 1.5 hours under nitrogen atmosphere. The reaction solution was concentrated, followed by addition of *N,N-*dimethylformamide (5 mL) and waste water treatment agent (8 mL). The mixture was allowed to stand overnight, followed by filtration. The filtrate was rotary-evaporated. The target compound **WX014-3** was isolated by preparative HPLC.

### Step 4: synthesis of compound WX014 and WX015

Compound **WX014-3** was resolved by supercritical fluid chromatography (separation condition column: OD (250mm * 30mm, 5µm); mobile phase: [0.1% NH₄HCO₃ EtOH]; B%: 40% -40%; flow rate: 50mL/min) to obtain the cis-trans isomers WX014 and WX015 with a retention time of 3.228min and 3.513min, respectively. By NOE, **WX014** was determined a cis isomer, ¹H NMR (400MHz, DMSO-*d₆*) δ : 8.45(s, 1H), 8.39(s, 1H), 8.20(s, 1H), 8.02-8.04(d, *J* = 8.8Hz, 1H), 7.77-7.81(m, 2H), 7.70(s, 1H), 4.88-4.92(t, *J* = 8.8Hz , 1H), 3.82-3.87(m, 1H), 2.81-2.88(m, 1H), 2.61-2.63(m, 4H), 2.58(s, 3H), 2.33(s, 3H), 2.30(s, 3H), 1.05-1.06(d, *J* = 6.8Hz, 6H), MS-ESI m/z: 567.1[M+H]⁺. **WX015** as a trans isomer,¹H NMR (400MHz, DMSO-*d₆*) δ : 8.51(s, 1H), 8.27(s, 1H), 8.20(s, 1H), 7.99-8.01(d, *J* = 8.4Hz, 1H), 7.75-7.81(m, 2H), 7.69(s, 1H), 5.27-5.31(t, *J* = 9.2Hz , 1H), 3.88-3.93(m, 1H), 2.99(s, 1H), 2.74-2.79(m, 3H), 2.67(s, 1H), 2.56(s, 3H), 2.33(s, 6H), 1.07-1.08(d, *J* = 6.8Hz, 6H), MS-ESI m/z: 567.1[M+H]⁺.

### Example 12: WX016, WX017

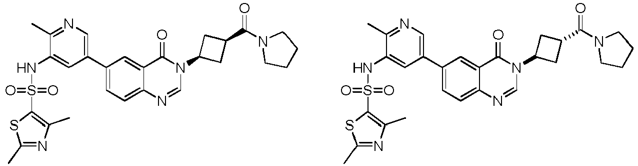

### Synthetic route:

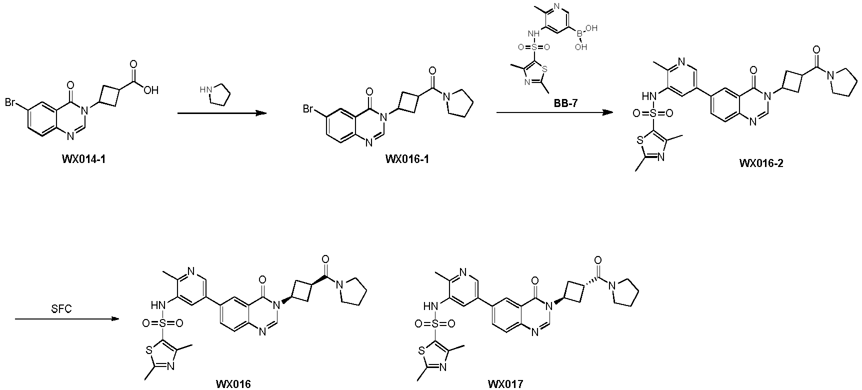

### Step 1: synthesis of compound WX016-1

**WX014-1** (0.5 g, 1.55 mmol) was dissolved in *N*,*N'*-dimethylformamide (10.00 mL), then tetrahydropyrrole (121.05 mg, 1.70 mmol, 142.08 µL), tetramethylurea hexafluorophosphate (882.50 mg, 2.32 mmol) and diisopropylethylamine (399.96 mg, 3.09 mmol, 539.03 µL) were added. The mixed solution was stirred at 25 °C for 5 hours under nitrogen atmosphere. After the reaction was completed, water was added to the reaction solution (10.00 mL), and extracted three times with ethyl acetate (10.00 mL). The organic phase was washed with water (10.00 mL × 3) and saturated brine (10.00 mL), and dried over anhydrous sodium sulfate. The organic phase was rotary-evaporated to obtain the target compound **WX016-1.**

### Step 2: synthesis of compound WX016-2

**WX016-1** (0.55 g, 1.41 mmol) was dissolved in dioxane (5 mL), and **BB-7** (573.94 mg, 1.75 mmol), potassium acetate ((573.84 mg, 5.85 mmol), water (1.00 mL) and [1,1'-bis (diphenylphosphino) ferrocene] palladium dichloride (213.92 mg, 292.36 µmol) were added. The reaction solution was heated to 100 °C and stirred for 16 hours. After the reaction was completed, the reaction solution was concentrated, rotary-evaporated, and separated by preparative HPLC to obtain the target compound **WX016-2.**

### Step 3: synthesis of compound WX016 and WX017

**WX016-2** was resolved by SFC (chromatographic column: OD (250mm * 30mm, 10µm), elution condition: [0.1% NH₄HCO₃ EtOH]; B%: 40% -40%; flow rate: 80mL/min) to obtain cis-trans isomers **WX016** (Rt = 0.736 min) and **WX017** (Rt = 0.946 min). By NOE, WX016 was determined as a cis isomer, ¹H NMR (400MHz, CDCl₃)δ : 8.67(s, 1H), 8.39 (s, 1H), 8.35 (s, 1H), 7.94 (s, 1H), 7.89-7.92(m, 1H), 7.79 (d, *J* = 8.4Hz, 2H), 5.09-5.18 (m, 1H), 3.44-3.52 (m, 4H), 3.12-3.20 (m, 1H), 2.73-2.83 (m, 2H), 2.66-2.70 (m, 2H), 2.66 (s, 3H), 2.46 (s, 3H), 2.42 (s, 3H), 1.98-2.01 (m, 2H), 1.87-1.91 (m, 2H). **WX017** as a trans isomer, ¹H NMR (400MHz, CDCl₃)δ : 8.70(s, 1H), 8.44 (s, 1H), 8.12 (s, 1H), 7.98 (s, 1H), 7.95-7.97(m, 1H), 7.80-7.83 (m, 1H), 5.11-5.15 (m, 1H), 3.53-3.57 (m, 2H), 3.37-3.39 (m, 3H), 2.91-2.95 (s, 4H), 2.69 (s, 3H), 2.46 (s, 3H), 2.44 (s, 3H), 1.89-1.92 (m, 4H).

### Example 13: WX018, WX019

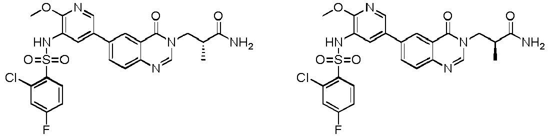

### Synthetic route:

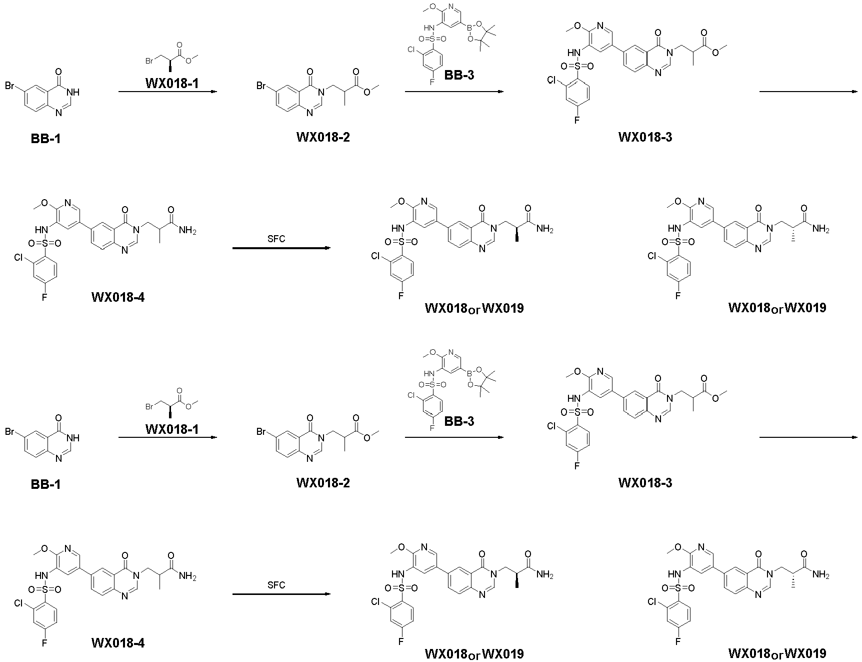

### Step 1: synthesis of compound WX018-2

Compound **BB-1** (50.00 mg, 222.18 µmol), **WX018-1** (80.44 mg, 444.36 µmol) and cesium carbonate (144.78 mg, 444.36 µmol) were dissolved in *N,N-*dimethylformamide formaldehyde (5 mL), heated to 100 °C by microwave and stirred for 2 hours. After the reaction was completed, the mixture was cooled to room temperature. The organic solvent was rotary-evaporated, and the residue was separated by a chromatographic plate (eluent: petroleum ether/ethyl acetate = 3:1) to obtain the target compound **WX018-2.** MS-ESI m/z: 324.8[M+H]⁺, 326.8[M+H+2]⁺.

### Step 2: synthesis of compound WX018-3

Compound **WX018-2** (60.02 mg, 141.47 µmol), **BB-3** (65.44 mg, 141.47 µmol) and potassium acetate (55.53 mg, 565.88 µmol) were dissolved in dioxane (2 mL) and water (0.2 mL), followed by addition of Pd (dppf)Cl₂ (2.07 mg, 2.83 µmol), heated to 95 °C and stirred for 2 hours under nitrogen atmosphere. After the reaction was completed, the mixture was cooled to room temperature. The organic solvent was rotary-evaporated, and the residue was separated by preparative chromatographic plate (eluent: methanol/dichloromethane/triethylamine = 1:20:0.02) to obtain the target compound **WX018-3.** MS-ESI m/z: 561.0[M+H]⁺,563.0[M+H+2]⁺.

### Step 3: synthesis of compound WX018-4

Ammonia gas was introduced into methanol (30 mL) at 0 °C for about 30 minutes. **WX018-3** (190.00 mg, 284.06 µmol) was dissolved in the above ammonia methanol solution, heated to 80 °C and stirred for 16 hours. After the reaction was completed, the mixture was cooled to room temperature. The organic solvent was rotary-evaporated, and the residue was separated by a chromatographic plate (eluent: methanol/dichloromethane/triethylamine = 1: 20: 0.2), and further separated by preparative high performance liquid phase column (AD (250mm * 30mm, 10µm); mobile phase: [0.1% NH₄HCO₃ EtOH]; B%: 40%-40%, min) to obtain target compound **WX018-4.**

### Step 4: synthesis of compound WX018, WX019

Compound **WX018-4** was resolved by supercritical fluid chromatography (separation conditions chromatographic column: AD (250mm * 30mm, 10µm); mobile phase: [0.1% NH₄HCO₃ EtOH]; B%: 40%-40%) to obtain the enantiomers **WX018** (retention time 4.861 min) and **WX019** (retention time 5.517 min respectively). **WX018,** ¹H NMR (400MHz, CDCl₃) δ : 8.31 (d, *J* = 2.0 Hz, 1H), 8.15-8.05 (m, 3H), 7.98 (d, *J* = 2.3 Hz, 1H), 7.90-7.69 (m, 2H), 7.28 (br s, 1H), 7.19-7.00 (m, 1H), 5.58 (br s, 1H), 5.40-5.23 (m, 1H), 4.28-4.12 (m, 1H), 4.12-4.01 (m, 1H), 3.98 (s, 3H), 3.10 (br dd, *J* = 7.4, 14.4 Hz, 1H), 1.33 (d, *J* = 7.0 Hz, 3H). MS-ESI m/z: 546.1[M+H]⁺,548.1[M+H+2]⁺. **WX019,** ¹H NMR (400MHz, CDCl₃) δ : 8.31 (d, *J* = 2.0 Hz, 1H), 8.16-8.08 (m, 3H), 7.97 (d, *J* = 2.0 Hz, 1H), 7.88-7.81 (m, 1H), 7.80-7.71 (m, 1H), 7.27 (d, *J* = 2.5 Hz, 1H), 7.16-7.05 (m, 1H), 5.63 (br s, 1H), 5.35 (br s, 1H), 4.25-4.13 (m, 1H), 4.12-4.02 (m, 1H), 3.98 (s, 3H), 3.10 (br dd, *J* = 6.8, 13.8 Hz, 1H), 1.33 (d, *J* = 7.0 Hz, 3H). MS-ESI m/z: 546.1[M+H]⁺,548.1[M+H+2]⁺. The ratio is 1:1.

### Example 14: WX020

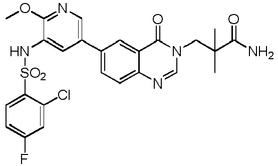

### Synthetic route:

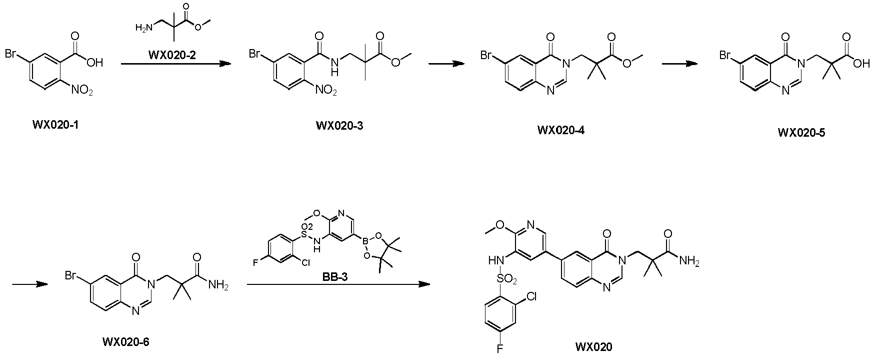

### Step 1: synthesis of compound WX020-3

**WX020-1** (500.00 mg, 2.03 mmol), **WX020-2** (350.00 mg, 2.09 mmol, 1.0HCl), triethylamine (1.00 g, 9.89 mmol), 2-hydroxypyridine *N*-oxide (250.00 mg, 2.25 mmol) and 1-(3-dimethylaminopropyl)-3-acetaldehyde hydrochloride (500.00 mg, 2.61 mmol) were dissolved in dichloromethane (30.00 mL), and the reaction solution was stirred for 16 hours under reflux. After the reaction was completed, the mixture was washed with water (20 mL). The aqueous phase was extracted with dichloromethane (20 mL). The organic phases were combined and concentrated, and the resulting residue was separated by a chromatographic column (eluent: petroleum ether / ethyl acetate = 0%-25%) to obtain the target compound **WX020-3.** MS-ESI *m*/*z:* 358.9 [M+H]⁺, 360.9 [M+H+2]⁺.

### Step 2: synthesis of compound WX020-4

The solution of iron powder (300.00 mg, 5.37 mmol) and compound **WX020-**3 (200.00 mg, 507.39 µmol) in formic acid (10.00 mL) was stirred at 100 °C for 16 hours. After the reaction was completed, the mixture was concentrated under reduced pressure to obtain the target compound **WX020-4,** which was used directly in the next step. MS-ESI *m*/*z:* 339.0 [M+H]⁺, 341.0 [M+H+2]⁺.

### Step 3: synthesis of compound WX020-5

A mixed solution of **WX020-4** (400.00 mg, 1.18 mmol) and sodium hydroxide (600.00 mg, 15.00 mmol) in methanol (15 mL) and water (15 mL) was stirred at 25 °C for 16 hours. The mixture was then heated to 70 °C and further stirred for 16 hours. After the reaction was completed, the mixture was concentrated under reduced pressure to remove the solvent methanol. The aqueous phase was neutralized with a 1.0M aqueous hydrochloric acid solution to pH of 5-6, and extracted twice with ethyl acetate (20 mL). The organic phases were combined, dried over anhydrous sodium sulfate, and filtered. The filtrate was distilled under reduced pressure to obtain the target compound **WX020-5,** which was directly used in the next step. MS-ESI *m*/*z:* 325.0 [M+H]⁺, 327.0 [M+H+2]⁺.

### Step 4: synthesis of compound WX020-6

Oxalyl chloride (139.83 mg, 1.10 mmol) was added to the solution of **WX020-5** (180.00 mg) in dichloromethane (30 mL). The reaction was stirred at 20 °C for one hour. Then ammonia gas was introduced into the solution for 10 minutes. The reaction solution was stirred for 15 hours. After the reaction was completed, the reaction solution was washed with water (15 mL), and the organic phase was concentrated under reduced pressure to obtain **WX020-6,** which was directly used in the next step. MS-ESI *m*/*z:* 323.9 [M+H]⁺, 325.9 [M+H+2]⁺.

### Step 5: synthesis of compound WX020

A mixed solution of **WX020-6** (100.00 mg, 181.17 µmol), **BB-3** (80.00 mg, 172.97 µmol), [1,1-bis(diphenylphosphino) ferrocene] palladium dichloride (15.00 mg, 20.50 µmol) and potassium acetate (15.00 mg, 20.50 µmol) in dioxane (5 mL) and water (1 mL) was stirred at 100 °C for two hours under nitrogen atmosphere. After the reaction was completed, the mixture was washed with water (10 mL) and extracted with dichloromethane (10 mL × 2). The organic phase was evaporated under reduced pressure, and the resulting residue was separated by high performance liquid chromatography (Kromasil 150 * 25mm * 10µµm; mobile phase: [water water (0.05% ammonium hydroxide ammonium hydroxide v / v) -ACN]; B%: 13% -43%, 8min) to obtain the target compound **WX020.** ¹H NMR (400 MHz, DMSO-d6) δ : 8.16 (s, 1 H) 8.02-8.10 (m, 2 H) 7.88 (dd, *J* = 8.53, 2.26 Hz, 1 H) 7.65-7.73 (m, 2 H) 7.47 (d, *J* = 2.26 Hz, 1 H) 7.37 (dd, *J* = 8.78, 2.51 Hz, 1 H) 7.21-7.29 (m, 2 H) 7.12 (s, 1 H) 6.07 (br s, 1 H) 4.16 (s, 2 H) 3.81 (s, 3 H) 1.15 (s, 6 H). MS-ESI *m*/*z*: 560.0 [M+H]⁺.

### Example 15: WX021

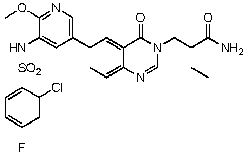

### Synthetic route:

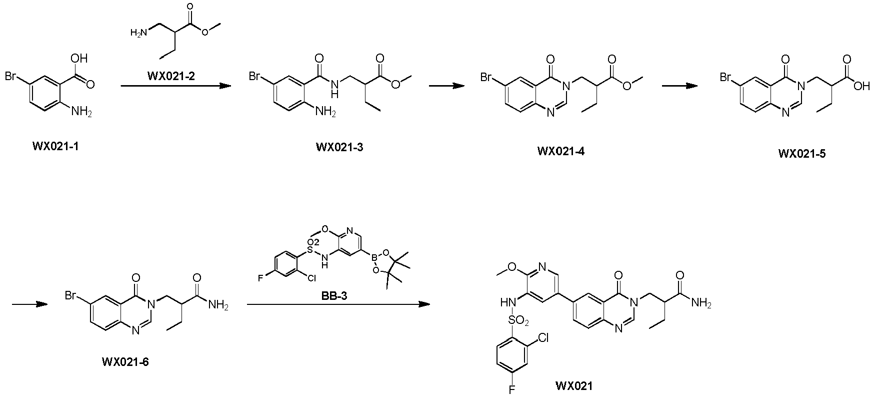

### Step 1: synthesis of compound WX021-3

**WX021-1** (400.00 mg, 1.85 mmol), **WX021-2** (314.00 mg, 1.87 mmol, 1.0 HCl), triethylamine (600.00 mg, 5.93 mmol), 2-hydroxypyridine *N*-oxide (210.00 mg, 1.89 mmol) and 1-(3-dimethylaminopropyl)-3-acetaldehyde hydrochloride (375.00 mg, 1.96 mmol) were dissolved in dichloromethane (50 mL), and stirred at 50 °C for 16 hours. After the reaction was completed, the mixture was washed with water (50 mL), and the organic phase was concentrated to obtain the target compound **WX021-3,** which was directly used in the next step.

### Step 2: synthesis of compound WX021-4

A solution of **WX021-3** (1000.00 mg, 3.04 mmol) in formic acid (20 mL) was stirred at 100 °C for 16 hours. After the reaction was completed, the mixture was concentrated under reduced pressure to obtain the target compound **WX021-4,** which was used directly in the next step. MS-ESI *m*/*z*: 339.0 [M+H]⁺, 341.0 [M+H+2]⁺.

### Step 3: synthesis of compound WX021-5

A mixed solution of **WX021-4** (1.0 g, 3.10 mmol) and lithium hydroxide monohydrate (1.30 g, 31.00 mmol) in methanol (30 mL) and water (40 mL) was stirred and reacted at 20 °C for 16 hours. After the reaction was completed, a stream of nitrogen was introduced to remove the solvent methanol. The aqueous phase was neutralized with a 1.0 M hydrochloric acid aqueous solution to pH=3-4, followed by filtration. The white solid was collected and dried to obtain the target compound **WX021-5,** which was used directly in the next step. MS-ESI *m*/*z:* 325.0 [M+H]⁺, 327.0 [M+H+2]⁺.

### Step 4: synthesis of compound WX021-6

Oxalyl chloride (362.50 mg, 2.86 mmol) was added to a solution of **WX021-**5 (450.00 mg, 1.38 mmol) in dichloromethane (20 mL). The reaction solution was stirred at 20 °C for two hours. Ammonia gas was then bubbled into the solution at - 30 °C for 30 minutes. The reaction was stirred at 20 °C for 15 hours. After the reaction was completed, the reaction solution was washed once with water (30 mL) and the aqueous phase was extracted with dichloromethane (30 mL × 2). The organic phase was concentrated under reduced pressure to obtain **WX021-6,** which was directly used in the next step. MS-ESI *m*/*z:* 323.9 [M+H]⁺, 325.9 [M+H+2]⁺.

### Step 5: synthesis of compound WX021

A mixed solution of **WX021-6** (500.00 mg, 1.54 mmol), **BB-3** (430.00 mg, 1.00 mmol), [1,1-bis(diphenylphosphino) ferrocene] palladium dichloride (135.22 mg, 184.80 µmol) and potassium acetate (700.00 mg, 7.13 mmol) in dioxane (20 mL) and water (4 mL) was stirred at 100 °C for two hours under nitrogen atmosphere. After the reaction was completed, the mixture was washed with water (30 mL) and extracted with dichloromethane (30 mL × 2). The organic phase was evaporated under reduced pressure, and the resulting residue was separated by high-performance liquid chromatography (Xtimate C18 150 * 25mm * 5µµm; mobile phase: [water and water (0.225% FA) -ACN]; B%: 46%-46%, 12min) to obtain the target compound **WX021.** ¹H NMR (400 MHz, DMSO-*d*₆) δ : 10.27 (s, 1 H) 8.40 (s, 1 H) 8.15-8.30 (m, 2 H) 8.04-8.13 (m, 1 H) 7.88-7.99 (m, 2 H) 7.76 (d, *J* = 8.53 Hz, 2 H) 7.30-7.52 (m, 2 H) 6.95 (br s, 1 H) 4.15 (dd, *J* = 12.92, 4.14 Hz, 1 H) 3.85-4.00 (m, 1 H) 3.70 (s, 3 H) 2.74 (br d, *J* = 3.76 Hz, 1 H) 1.37-1.68 (m, 1 H) 1.35-1.64 (m, 1 H) 0.91 (t, *J* = 7.40 Hz, 3 H). MS-ESI *m*/*z:* 560.2 [M+H]⁺.

### Example 16: WX022

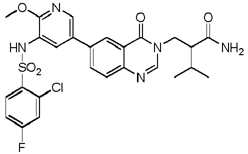

### Synthetic route:

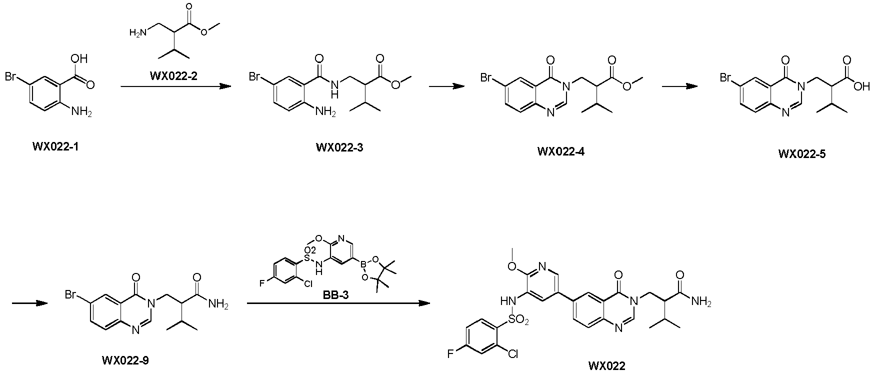

### Step 1: synthesis of compound WX022-3

**WX022-1** (400.00 mg, 1.85 mmol), **WX022-2** (340.00 mg, 1.87 mmol, 1.0 HCl), triethylamine (600.00 mg, 5.93 mmol), 2-hydroxypyridine *N*-oxide (210.00 mg, 1.89 mmol) and 1-(3-dimethylaminopropyl)-3-acetaldehyde hydrochloride (370.00 mg, 1.93 mmol) were dissolved in dichloromethane (50 mL), and the reaction solution was stirred at 50 °C for 16 hours. After the reaction was completed, the mixture was washed with water (50 mL), and the organic phase was concentrated to obtain the target compound **WX022-3,** which was directly used in the next step.

### Step 2: synthesis of compound WX022-4

A formic acid solution (24.38 mL) of **WX022-3** (1000.00 mg, 2.91 mmol) was stirred at 100 °C for 16 hours. After the reaction was completed, the mixture was concentrated under reduced pressure to obtain the target compound **WX022-4,** which was used directly in the next step. MS-ESI *m*/*z*: 353.0 [M+H]⁺, 355.0 [M+H+2]⁺.

### Step 3: synthesis of compound WX022-5

A mixed solution of **WX022-4** (1.0 g, 2.83 mmol) and lithium hydroxide monohydrate (1.19 g, 28.30 mmol) in methanol (30.00 mL) and water (30.00 mL) was stirred at 20 °C for 16 hours. After the reaction was completed, a stream of nitrogen was bubbled to remove the solvent methanol. The aqueous phase was neutralized with 1.0 M hydrochloric acid aqueous solution to pH 3-4. The aqueous phase was extracted with ethyl acetate (30 mL × 3). The organic phases were combined and rotary-evaporated to obtain the target compound **WX022-5,** which was directly used in the next step. MS-ESI *m*/*z:* 339.0 [M+H]⁺, 341.0 [M+H+2]⁺.

### Step 4: synthesis of compound WX022-6

Oxalyl chloride (580 mg, 4.57 mmol) was added to a solution of **WX022-5** (1000.00 mg, 2.95 mmol) in dichloromethane (30 mL). The reaction solution was stirred at 20 °C for two hours. Ammonia gas was then bubbled into the solution at - 30 °C for 30 minutes. The reaction solution was stirred at 20 °C for 15.5 hours. After the reaction was completed, the reaction was washed once with water (30 mL) and the aqueous phase was extracted with dichloromethane (30 mL × 2). The organic phase was concentrated under reduced pressure to obtain **WX022-6,** which was directly used in the next step. MS-ESI *m*/*z*: 338.1 [M+H]⁺, 340.0 [M+H+2]⁺.

### Step 5: synthesis of compound WX022

A mixed solution of **WX022-6** (400.00 mg, 1.18 mmol), **BB-3** (400.00 mg, 893.18 µmol), [1,1-bis(diphenylphosphino) ferrocene] palladium dichloride (100.00 mg, 136.67 µmol) and potassium acetate (500.00 mg, 5.09 mmol) in dioxane (20 mL) and water (4 mL) was stirred at 100 °C for two hours under nitrogen atmosphere. After the reaction was completed, the mixture was washed with water (30 mL) and extracted with dichloromethane (30 mL × 2). The organic phase was evaporated under reduced pressure, and the resulting residue was separated by high performance liquid chromatography (Xtimate C18 150 * 25mm * 5µm; mobile phase: [water (0.225% FA) -ACN]; B%: 44% -54%, 12min) to obtain the target compound **WX022.** ¹H NMR (400 MHz, CHLOROFORM-*d*) δ ppm 8.09-8.19 (m, 3 H) 8.06 (d, *J=* 2.20 Hz, 1 H) 7.90 (d, *J* = 2.20 Hz, 1 H) 7.70-7.78 (m, 1 H) 7.60-7.68 (m, 2 H) 7.21-7.35 (m, 1 H) 7.11 (ddd, *J* = 8.89, 7.51, 2.42 Hz, 1 H) 5.81-6.24 (m, 2 H) 4.49 (dd, *J* = 12.98, 3.52 Hz, 1 H) 3.81-4.05 (m, 4 H) 2.85 (ddd, *J* = 10.78, 7.43, 3.58 Hz, 1 H) 1.91-2.17 (m, 1 H) 1.04-1.23 (m, 6 H). MS-ESI *m*/*z:* 574.1 [M+H]⁺.

### Example 17: WX023

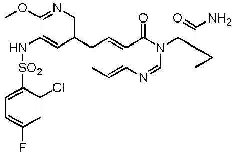

### Synthetic route:

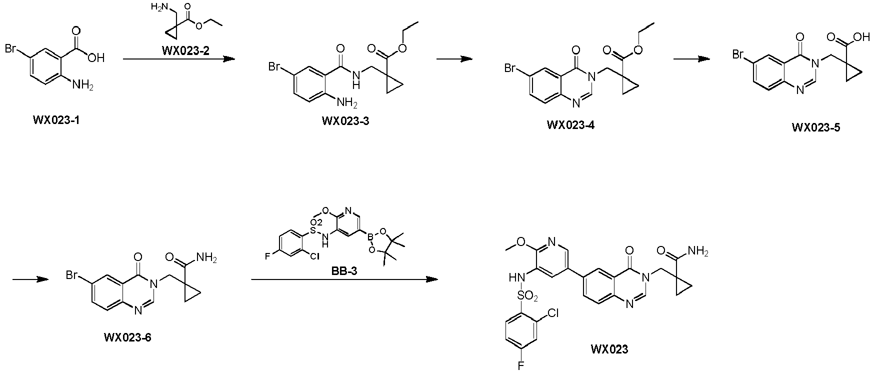

### Step 1: synthesis of compound WX023-3

**WX023-1** (500.00 mg, 2.31 mmol), **WX023-2** (375.00 mg, 2.62 mmol, 1.0HCl), triethylamine (800.00 mg, 7.90 mmol), 2-hydroxypyridine N-oxide (300.00 mg, 2.70 mmol) and 1-(3-dimethylaminopropyl)-3-acetaldehyde hydrochloride (450.00 mg, 2.35 mmol) were dissolved in dichloromethane (50.00 mL) and stirred at 50 °C for 16 hours. After the reaction was completed, the mixture was washed with water (50 mL) and the organic phase was concentrated to obtain the target compound **WX023-3,** which was directly used in the next step. MS-ESI *m*/*z:* 341.0 [M+H]⁺, 343.0 [M+H+2]⁺.

### Step 2: synthesis of compound WX023-4

A solution of **WX023-3** (1000.00 mg) in formic acid (20 mL) was stirred at 100 °C for 16 hours. After the reaction was completed, the mixture was concentrated under reduced pressure to obtain the target compound **WX023-4,** which was used directly in the next step. MS-ESI *m*/*z*: 351.0 [M+H]⁺, 353.0 [M+H+2]⁺.

### Step 3: synthesis of compound WX023-5

A mixed solution of **WX023-4** (0.5 g, 276.92 µmol) and lithium hydroxide monohydrate (120 mg, 2.86 mmol) in methanol (20.00 mL) and water (25.00 mL) was stirred and reacted at 20 °C for 16 hours. After the reaction was completed, a stream of nitrogen was bubbled to remove the solvent methanol. The aqueous phase was neutralized with 1.0 M hydrochloric acid aqueous solution to pH=3-4, followed by filtration. The filter cake was collected and rotary-evaporated to obtain the target compound **WX023-5,** which was directly used in the next step. MS-ESI *m*/*z:* 323.0 [M+H]⁺, 325.0 [M+H+2]⁺.

### Step 4: synthesis of compound WX023-6

Oxalyl chloride (362.50 mg, 2.86 mmol) was added to a solution of **WX023-5** (450.00 mg, 1.39 mmol) in dichloromethane (20 mL). The reaction solution was stirred at 20 °C for two hours. Ammonia gas was bubbled into the solution at -30 °C for 30 minutes. The reaction solution was stirred at 20 °C for 15.5 hours. After the reaction was completed, the reaction solution was washed once with water (30 mL) and the aqueous phase was extracted with dichloromethane (30 mL * 2). The organic phase was concentrated under reduced pressure to obtain **WX023-6,** which was directly used in the next step. MS-ESI *m*/*z:* 322.0 [M+H]⁺, 324.0 [M+H+2]⁺.

### Step 5: synthesis of compound WX023

A mixed solution of **WX023-6** (450.00 mg, 1.40 mmol), **BB-3** (430.00 mg, 1000 µmol), [1,1-bis(diphenylphosphino)ferrocene] palladium dichloride (125.00 mg, 170.83 µmol) and potassium acetate (600.00 mg, 6.11 mmol) in dioxane (20 mL) and water (4 mL) was stirred at 100 °C for two hours under nitrogen atmosphere. After the reaction was completed, the mixture was washed with water (30 mL) and extracted with dichloromethane (30 mL × 2). The organic phase was evaporated under reduced pressure, and the resulting residue was separated by high-performance liquid chromatography (Xtimate C18 150 * 25mm * 5µm; mobile phase: [water (0.225% FA) -ACN]; B%: 40% -50%, 12min) to obtain the target compound **WX023.** ¹H NMR (400 MHz, DMSO-*d*₆) δ : 9.52-10.62 (m, 1 H) 8.52 (s, 1 H) 8.40 (d, *J* = 2.26 Hz, 1 H) 8.25 (d, *J* = 2.26 Hz, 1 H) 8.04-8.12 (m, 1 H) 7.88-7.99 (m, 2 H) 7.71-7.79 (m, 2 H) 7.36 (td, *J=* 8.53, 2.51 Hz, 1 H) 6.89-7.09 (m, 2 H) 4.24 (s, 2 H) 3.70 (s, 3 H) 1.14 (s, 4 H). MS-ESI *m*/*z:* 558.1 [M+H]⁺.

### Example 18: WX024, WX025

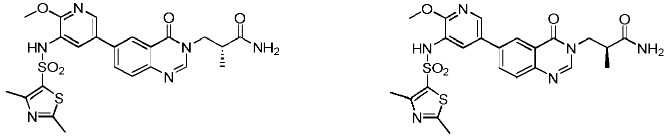

### Synthetic route:

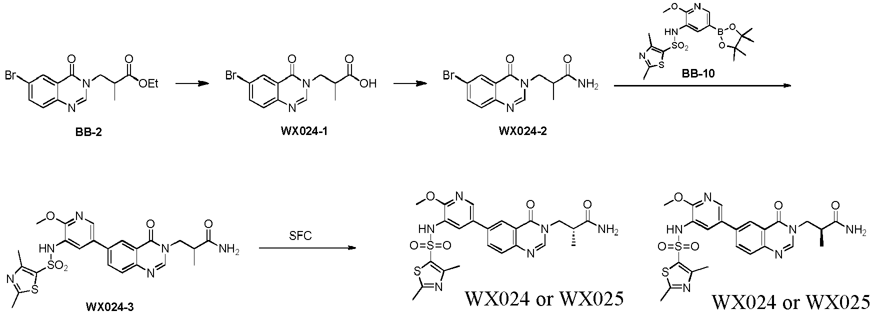

### Step 1: synthesis of compound WX024-1

A solution of **BB-2** (300.00 mg, 863.44 µmol, purity: 97.62%) and lithium hydroxide monohydrate (362.00 mg, 8.63 mmol) in ethanol (5.00 mL) and water (5.00 mL) was stirred at 25 °C for 16 hours. After the reaction was completed, the reaction solution was adjusted to pH=3-4 with 1.0 M hydrochloric acid aqueous solution, washed with water (50 mL) and extracted once with ethyl acetate (50 mL). The organic phase was evaporated under reduced pressure to obtain the target compound **WX024-1.** MS-ESI *m*/*z:* 310.8 [M+H]⁺, 312.8 [M+H+2]⁺.

### Step 2: synthesis of compound WX024-2

Oxalyl chloride (156.60 mg, 1.23 mmol) was added to a solution of **WX024-1** (150.00 mg, 411.19 µmol) in dichloromethane (20.00 mL). The reaction solution was stirred at 20 °C for one hour. Ammonia gas was then bubbled into the solution for 20 minutes. The reaction solution was stirred at 20 °C for 16 hours. After the reaction was completed, water (50 mL) was added to the reaction solution and then extracted with dichloromethane (50 mL). The organic phases were combined and concentrated under reduced pressure to obtain **WX024-2,** which was used directly in the next step. MS-ESI *m*/*z:* 309.9 [M+H]⁺, 311.9[M+H+2]⁺.

### Step 3: synthesis of compound WX024-3

A suspension of **WX024-2** (100.00 mg, 322.42 µmol), **BB-10** (205.00 mg, 481.98 µmol), potassium acetate (126.00 mg, 1.28 mmol) and [1,1'-bis(diphenylphosphino) ferrocene] palladium dichloride (24.00 mg, 32.80 µmol) in dioxane (4.0 mL) and water (0.8 mL) was replaced with nitrogen three times, then the reaction solution was heated to 100 °C and stirred for 40 minutes under nitrogen atmosphere. After the reaction was completed, the reaction solution was concentrated and rotary-evaporated. The crude product was slurried with water (50 mL), and then extracted with dichloromethane (50 mL). The organic phase was rotary-evaporated and separated by high-performance liquid chromatography (column: Xtimate C18 150 * 25mm * 5µm; mobile phase: [water (0.05% ammonium hydroxide v / v) -ACN]; B%: 8% -38%, 10min) to obtain compound **WX024-3.**

### Step 4: synthesis of compound WX024 and WX025

WX024-3 was resolved by SFC (chromatographic column: AD (250mm * 30mm, 5µm); mobile phase: [0.1% NH₄HCO₃ EtOH]; B%: 40%-40%) to obtain enantiomers **WX024** (Rt = 0.981 min) and **WX025** (Rt = 1.359 min). **WX024** ¹H NMR (400 MHz, CHLOROFORM-*d*) δ ppm 8.21 (d, *J* = 2.01 Hz, 1 H) 8.07-8.12 (m, 2 H) 7.95 (d, *J* = 2.26 Hz, 1 H) 7.77 (dd, *J* = 8.53, 2.01 Hz, 1 H) 7.66 (d, *J* = 8.53 Hz, 1 H) 6.14 (br s, 1 H) 5.74 (br s, 1 H) 4.06-4.19 (m, 1 H) 3.97 (dd, *J* = 13.30, 9.54 Hz, 1 H) 3.87 (s, 3 H) 2.99-3.15 (m, 1 H) 2.51-2.64 (m, 3 H) 2.47 (s, 3 H) 1.25 (d, *J* = 7.03 Hz, 3 H), MS-ESI *m*/*z*: 529.1 [M+H]⁺, 551.1 [M+Na]⁺. **WX025** ¹H NMR (400 MHz, CHLOROFORM-*d*) δ ppm 8.14-8.25 (m, 1 H) 8.09 (s, 2 H) 7.93 (d, *J* = 1.51 Hz, 1 H) 7.76 (dd, *J* = 8.41, 1.63 Hz, 1 H) 7.65 (d, *J* = 8.53 Hz, 1 H) 6.24 (br s, 1 H) 5.83 (br s, 1 H) 4.08-4.17 (m, 1 H) 3.97 (br dd, *J* = 13.18, 9.66 Hz, 1 H) 3.77 (br s, 1 H) 3.87 (s, 2 H) 2.98-3.17 (m, 1 H) 2.37-2.62 (m, 6 H) 1.25 (br d, *J* = 7.03 Hz, 3 H), MS-ESI *m*/*z:* 529.1 [M+H]⁺, 551.1 [M+Na]⁺.

### Example 19: WX026, WX027

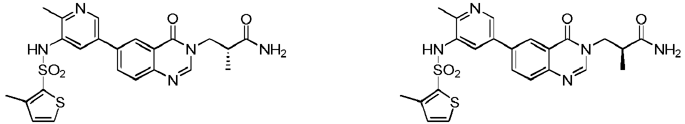

### Synthetic route:

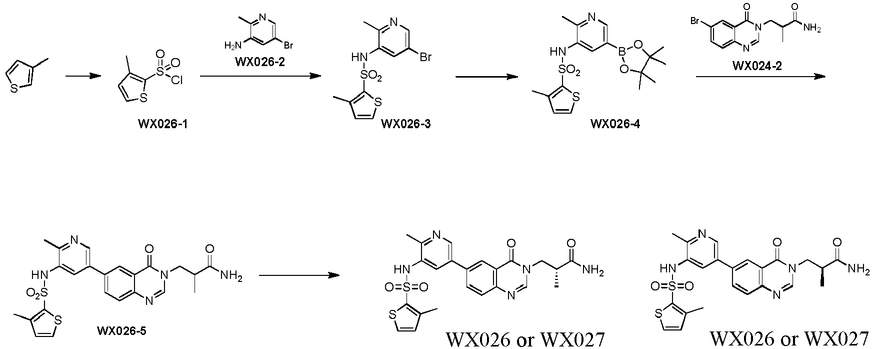

### Step 1: synthesis of compound WX026-1

At 0 °C, a solution of 3-methylthiophene (300.00 mg, 3.06 mmol) in chloroform (3 mL) was added to a solution of chlorosulfonic acid (1.07 g, 9.18 mmol) in chloroform (7 mL). The reaction was stirred at 0 °C for one hour. After the reaction was completed, the mixture was added dropwise to the ice-water mixture (50 mL) with stirring, and extracted twice with chloroform (50 mL). The organic phase was evaporated under reduced pressure to obtain the target compound **WX026-1.** ¹H NMR (400 MHz, CHLOROFORM-*d*) δ : 7.67 (d, *J* = 5.27 Hz, 1 H) 7.02 (d, *J* = 5.02 Hz, 1 H) 2.55-2.72 (m, 3 H).

### Step 2: synthesis of compound WX026-3

At 25 °C, **WX026-1** (230.00 mg, 1.17 mmol) was added dropwise to a solution of **WX026-2** (218.00 mg, 1.17 mmol) in pyridine (1 mL) within 10 minutes. The reaction was stirred at 25 °C for 16 hours. After the reaction was completed, water (50 mL) was added to the reaction, and extracted with dichloromethane (50 mL). The organic phase was concentrated under reduced pressure to obtain **WX026-3.** MS-ESI *m*/*z:* 346.8 [M+H]⁺, 348.8 [M+H+2]⁺; ¹H NMR (400 MHz, CHLOROFORM-*d*) δ ppm 8.54 (br d, *J* = 4.27 Hz, 1 H) 8.31 (d, *J* = 2.01 Hz, 1 H) 7.80 (d, *J* = 2.01 Hz, 1 H) 7.47-7.48 (m, 1 H) 7.40 (d, *J* = 5.02 Hz, 1 H) 7.20-7.26 (m, 1 H) 6.82 (d, *J* = 5.02 Hz, 1 H) 2.21 (d, *J* = 3.26 Hz, 6 H).

### Step 3: synthesis of compound WX026-4

A suspension of **WX026-3** (350.00 mg, 758.66 µmol, purity: 75.27%), bis(pinacolato)diboron (289.00 mg, 1.14 mmol), potassium acetate (223.00 mg, 2.27 mmol) and [1,1'-bis(diphenylphosphino) ferrocene] palladium dichloride (55.00 mg, 75.17 µmol) in dioxane (12 mL) was replaced with nitrogen three times, then the reaction solution was heated to 100 °C and stirred for 60 minutes under nitrogen atmosphere. After the reaction was completed, the reaction solution was combined with the previous batch. The reaction solution was oncentrated and rotary-evaporated. The crude product was slurried with water (100 mL), and then extracted with dichloromethane (100 mL). The organic phase was concentrated under reduced pressure to obtain **WX026-4.** MS-ESI *m*/*z:* 312.9 [M+H]⁺.

### Step 4: synthesis of compound WX026-5

A suspension of **WX024-2** (80.00 mg, 247.44 µmol), **WX026-4** (201.00 mg, 246.87 µmol), potassium acetate (97.00 mg, 988.38 µmol) and [1,1'-bis(diphenylphosphino) ferrocene] palladium dichloride (18.00 mg, 24.60 µmol) in dioxane (4.0 mL) and water (0.8 mL) was replaced with nitrogen three times, then the reaction solution was heated to 100 °C and stirred for 60 minutes under nitrogen atmosphere. After the reaction was completed, the reaction solution was concentrated and rotary-evaporated. The crude product was slurried with water (50 mL), and then extracted with dichloromethane (50 mL). The organic phase was rotary-evaporated and separated by column chromatography (MeOH: DCM = 0% ∼ 10%) to obtain the compound **WX026-5.**

### Step 5: synthesis of compound WX026 and WX027

**WX026-5** was further resolved by SFC (chromatographic column: AD (250mm * 30mm, 10µm); mobile phase: [0.1% NH₄HCO₃ MeOH]; B%: 55% -55%) to obtain the target compound **WX026** (Rt = 1.438 min) and **WX027** (Rt = 2.086 min). **WX026,** ¹H NMR (400 MHz, CHLOROFORM-*d*) δ : 8.43 (s, 1 H) 8.10 (br d, *J* = 2.76 Hz, 2 H) 7.62-7.88 (m, 3 H) 7.33-7.56 (m, 2 H) 6.84 (d, *J* = 5.02 Hz, 1 H) 6.00 (br s, 1 H) 5.37-5.58 (m, 1 H) 5.48 (br s, 1 H) 4.10-4.24 (m, 1 H) 4.15 (br dd, *J=* 13.18, 4.39 Hz, 1 H) 3.86-4.00 (m, 1 H) 4.00 (s, 1 H) 3.19 (br s, 1 H) 2.07-2.42 (m, 6 H) 1.28 (br d, *J* = 7.03 Hz, 3 H), MS-ESI *m*/*z:* 498.1 [M+H]⁺. **WX027,** ¹H NMR (400 MHz, CHLOROFORM-*d*) δ ppm 8.52 (br s, 1 H) 8.19 (br s, 2 H) 7.72-8.00 (m, 3 H) 7.51 (br d, *J* = 4.02 Hz, 1 H) 6.93 (br d, *J* = 3.76 Hz, 1 H) 6.16 (br s, 1 H) 5.62 (br s, 1 H) 4.24 (br d, *J* = 10.29 Hz, 1 H) 4.07 (br t, *J* = 11.04 Hz, 1 H) 3.14-3.40 (m, 1 H) 3.27 (br s, 1 H) 2.15-2.48 (m, 1 H) 2.15-2.48 (m, 5 H) 1.37 (br d, *J* = 6.27 Hz, 3 H), MS-ESI *m*/*z:* 498.1 [M+H]⁺.

### Example 20: WX028, WX029

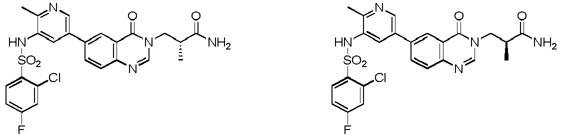

### Synthetic route:

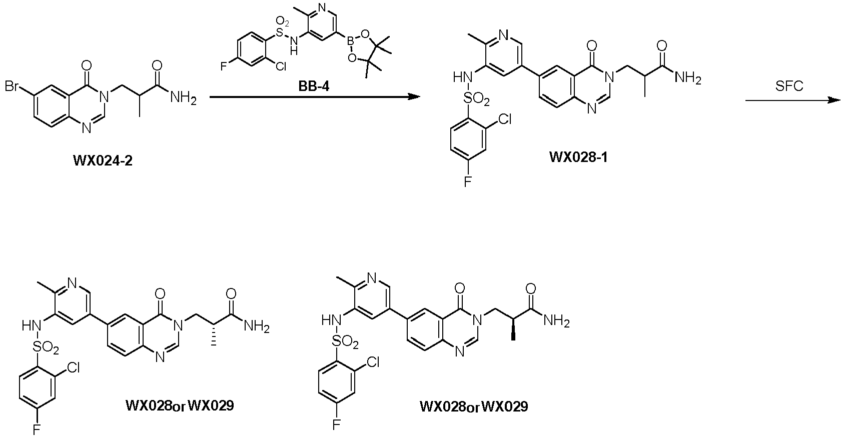

### Step 1: synthesis of compound WX028-1

A suspension of **WX024-2** (100.00 mg, 309.30 µmol), **BB-4** (186.00 mg, 309.32 µmol), potassium acetate (121.00 mg, 1.23 mmol) and [1,1'-bis(diphenylphosphino) ferrocene] palladium dichloride (23.00 mg, 31.43 µmol) in dioxane (4.0 mL) and water (0.8 mL) was replaced with nitrogen three times, then the reaction solution was heated to 100 °C and stirred for 1 hour under nitrogen atmosphere. After the reaction was completed, the reaction solution was concentrated and rotary-evaporated. The crude product was slurried with water (50 mL), and then extracted with dichloromethane (50 mL). The organic phase was rotary-evaporated, and separated by high performance liquid chromatography (chromatographic column: Kromasil 150 * 25mm * 10µm; mobile phase: [water (0.05% ammonium hydroxide v/v) -ACN]; B%: 16%-26%, 8min) to obtain compound **WX028-1.**

### Step 2: synthesis of compound WX028 and WX029

**WX028-1** was resolved by SFC (chromatographic column: AD (250mm * 30mm, 10µm); mobile phase: [0.1% NH₄HCO₃ EtOH]; B%: 50%-50%) to obtain the enantiomers **WX028** (Rt = 3.739 min) and **WX029** (Rt = 3.45 min). **WX028:** ¹H NMR (400 MHz, CHLOROFORM-*d*) δ ppm 8.46 (s, 1 H) 8.18 (s, 1 H) 8.09 (s, 1 H) 8.03 (dd, *J* = 8.66, 5.90 Hz, 1 H) 7.65-7.86 (m, 3 H) 7.22-7.33 (m, 1 H) 7.03-7.14 (m, 1 H) 5.64 (br s, 1 H) 5.22-5.42 (m, 1 H) 4.06-4.25 (m, 1 H) 3.86-4.02 (m, 1 H) 3.07 (br d, *J=* 6.78 Hz, 1 H) 2.37-2.62 (m, 3 H) 1.27 (d, *J=* 7.03 Hz, 3 H), MS-ESI *m*/*z:* 530.1 [M+H]⁺. **WX029:** ¹H NMR (400 MHz, CHLOROFORM-*d*) δ : 8.44 (s, 1 H) 8.15 (s, 1 H) 8.10 (s, 1 H) 8.02 (dd, *J* = 9.03, 5.77 Hz, 1 H) 7.68-7.84 (m, 3 H) 7.26 (dd, *J* = 8.03, 2.51 Hz, 1 H) 6.99-7.12 (m, 1 H) 5.73 (br s, 1 H) 5.63-5.84 (m, 1 H) 5.36 (br s, 1 H) 4.07-4.18 (m, 1 H) 4.07-4.18 (m, 1 H) 3.99 (dd, *J* = 13.30, 9.54 Hz, 1 H) 3.11 (br s, 1 H) 2.49 (s, 3 H) 1.27 (d, *J* = 7.03 Hz, 3 H), MS-ESI *m*/*z:* 530.1 [M+H]⁺.

### Example 21: WX030, WX031

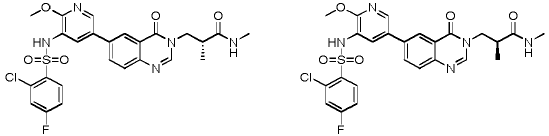

### Synthetic route:

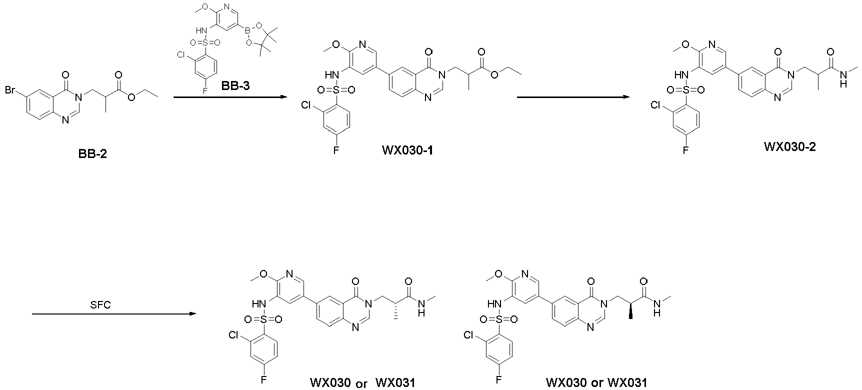

### Step 1: synthesis of compound WX030-1

Compounds **BB-2** (3.00 g, 9.23 mmol), **BB-3** (4.08 g, 9.23 mmol) and potassium acetate (3.62 g, 36.90 mmol) were dissolved in dioxane (2 mL) and water (0.2 mL), followed by addition ofPd (dppf)Cl₂ (1.35 g, 1.85 mmol), and heated to 95 °C and stirred for 2 hours under nitrogen atmosphere. After the reaction was completed, the mixture was cooled to room temperature, and the organic solvent was rotary-evaporated, then it was poured into water (50 mL), and extracted with dichloromethane (50 mL × 3). The organic phases obtained were combined and dried over anhydrous sodium sulfate. After the desiccant was removed by filtration, the solvent was removed under reduced pressure and separated by a preparative chromatographic plate (eluent: methanol/dichloromethane/triethylamine = 1:15:0.15) to obtain the target compound **WX030-1.** ¹H NMR (400MHz, CDCl₃) δ : 8.35 (d, *J* = 2.0 Hz, 1H), 8.25-8.10 (m, 2H), 7.99 (d, *J* = 2.3 Hz, 1H), 7.90-7.71 (m, 2H), 7.71-7.52 (m, 2H), 7.30-7.28 (m, 1H), 7.20-7.06 (m, 1H), 4.21-4.10 (m, 4H), 4.03 (S, 3H), 3.26-3.12 (m, 1H), 1.34 (d, *J* = 7.0 Hz, 3H), 1.24-1.18 (m, 3H). MS-ESI m/z: 575.1[M+H]⁺,577.1[M+H+2]⁺.

### Step 2: synthesis of compound WX030-2

**WX030-1** (300.00 mg, 534.78 µmol) was dissolved in methylamine alcohol solution (20 mL), heated to 80 °C and stirred overnight. After the reaction was completed, the mixture was cooled to room temperature, poured into water (50 mL), and extracted with dichloromethane (50 mL × 3). The organic phases obtained were combined and dried over anhydrous sodium sulfate. After the desiccant was removed by filtration, the solvent was removed under reduced pressure. The residue was separated by a chromatographic plate (eluent: methanol/dichloromethane/triethylamine = 1:20:0.2), and further separated by a preparative high-performance liquid column (AS (250mm * 30mm, 5µm); mobile phase: [0.1% NH₄HCO₃ ETOH]; B%: 30% -30%) to obtain the target compound **WX030-2.**

### Step 3: synthesis of compound WX030 and WX031

Compound **WX030-2** was resolved by supercritical fluid chromatography (separation conditions: chromatographic column: AS (250mm * 30mm, 5µm); mobile phase: [0.1% NH₄HCO₃ EtOH]; B%: 30%-30%) to obtain the enantiomers **WX030** and **WX031,** the retention time of which is 4.092min and 4.723min, respectively, and the ratio is 1:1. **WX030:** ¹H NMR (400MHz, CDCl₃) δ : 8.24 (d, J = 2.0 Hz, 1H), 8.14-8.04 (m, 3H), 7.91 (d, J = 2.3 Hz, 1H), 7.83-7.65 (m, 2H), 7.20 (d, J = 2.5 Hz, 1H), 7.11-6.93 (m, 1H), 5.49 (br d, J = 4.5 Hz, 1H), 4.17-4.05 (m, 1H), 4.04-3.93 (m, 1H), 3.91 (s, 3H), 2.93-2.81 (m, 1H), 2.67 (d, J = 5.0 Hz, 3H), 1.22 (d, J = 7.0 Hz, 3H). MS-ESI m/z: 560.2[M+H]⁺,562.0[M+H+2]⁺. **WX031:** ¹HNMR (400MHz, CDCl₃) δ : 8.24 (d, J = 2.0 Hz, 1H), 8.11-7.99 (m, 3H), 7.91 (d, J = 2.3 Hz, 1H), 7.83-7.73 (m, 1H), 7.73-7.65 (m, 1H), 7.46 (br s, 1H), 7.21 (s, 1H), 6.90-6.90 (m, 1H), 5.47 (br d, J = 5.5 Hz, 1H), 4.15-4.06 (m, 1H), 4.04-3.93 (m, 1H), 3.91 (s, 3H), 2.95-2.83 (m, 1H), 2.67 (d, J = 4.8 Hz, 3H), 1.22 (d, J = 7.0 Hz, 3H). MS-ESI m/z: 560.2[M+H]⁺,562.0[M+H+2]⁺.

### Example 22: WX032, WX033

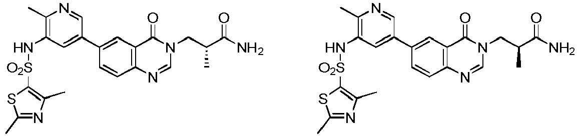

### Synthetic route:

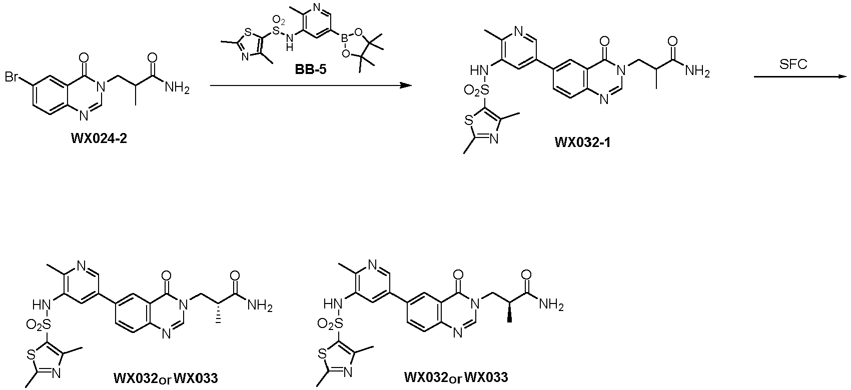

### Step 1: synthesis of compound WX032-1

A suspension of **WX024-2** (80.00 mg, 247.44 µmol), **BB-5** (190.00 mg, 246.24 µmol), potassium acetate (97.00 mg, 988.38 µmol) and [1,1'-bis(diphenylphosphino) ferrocene] palladium dichloride (18.00 mg, 24.60 µmol) in dioxane (4.0 mL) and water (0.8 mL) was replaced with nitrogen three times, then the reaction solution was heated to 100 °C and stirred for 60 minutes under nitrogen atmosphere. After the reaction was completed, the reaction solution was concentrated and rotary-evaporated. Water (50 mL) was added to the reaction solution, and extracted with dichloromethane (50 mL). The organic phase was evaporated under reduced pressure and purified by column chromatography (MeOH: DCM = 0% ∼ 10%) to obtain compound **WX032-1.**

### Step 2: synthesis of compound WX032 and WX033

**WX032-1** was resolved by SFC (chromatographic column: OD (250mm * 50mm, 10µm); mobile phase: [0.1% NH₄HCO₃ MeOH]; B%: 40%-40%) to obtain the enantiomers **WX032** (Rt = 4.037 min) and **WX033** (Rt = 4.298 min). **WX032:** ¹H NMR (400 MHz, CHLOROFORM-*d*) δ ppm 8.43 (br s, 1 H) 7.98-8.17 (m, 2 H) 7.69-7.83 (m, 2 H) 7.63 (br d, *J* = 8.53 Hz, 1 H) 7.58-7.66 (m, 1 H) 6.43 (br s, 1 H) 5.88 (br s, 1 H) 4.15 (br dd, *J* = 12.92, 3.89 Hz, 1 H) 3.89-4.02 (m, 1 H) 3.19 (br s, 1 H) 2.58 (s, 3 H) 2.28-2.45 (m, 6 H) 1.27 (br d, *J* = 6.78 Hz, 3 H), MS-ESI *m*/*z*:513.1 [M+H]⁺. **WX033:** ¹H NMR (400 MHz, CHLOROFORM-*d*) δ ppm 8.43 (br s, 1 H) 7.96-8.20 (m, 2 H) 7.69-7.81 (m, 2 H) 7.62 (br d, *J* = 8.03 Hz, 1 H) 6.46 (br s, 1 H) 5.92 (br s, 1 H) 4.91 (s, 1 H) 4.15 (br d, *J* = 9.79 Hz, 1 H) 3.87-4.03 (m, 1 H) 3.11-3.29 (m, 1 H) 3.19 (br s, 1 H) 2.58 (s, 3 H) 2.23-2.45 (m, 6 H) 1.21-1.32 (m, 1 H) 1.21-1.32 (m, 2 H), MS-ESI *m*/*z*:513.1 [M+H]⁺.

### Example 23: WX034, WX035

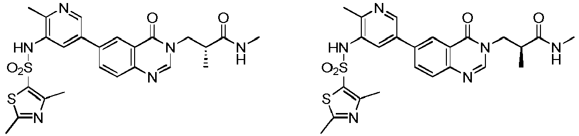

### Synthetic route:

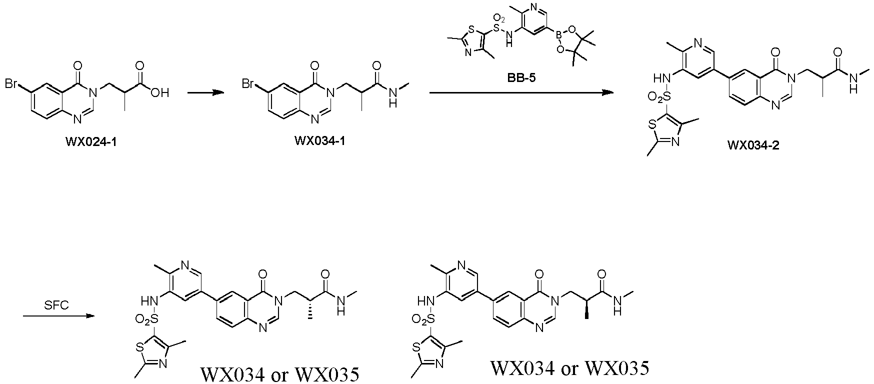

### Step 1: synthesis of compound WX034-1

The crude **WX024-1** (100.00 mg), methylamine (80.00 mg, 643.92 µmol), triethylamine (129.94 mg, 1.28 mmol), 2-hydroxypyridine N-oxide (42.00 mg, 378.04 µmol) and 1-(3-dimethylaminopropyl)-3-acetaldehyde hydrochloride (62.00 mg, 323.42 µmol) were dissolved in dichloromethane (5 mL), and the reaction solution was stirred at 50 °C for 16 hours. After the reaction was completed, the mixture was washed with water (50 mL) and extracted with dichloromethane (50 mL). The organic phase was concentrated to obtain the target compound **WX034-1,** which was directly used in the next step. MS-ESI *m*/*z:* 323.8 [M+H]⁺, 325.8 [M+H+2]⁺.

### Step 2: synthesis of compound WX034-2

A suspension of **WX034-1** (100.00 mg, 289.76 µmol), **BB-5** (142.00 mg, 290.17 µmol), potassium acetate (114.00 mg, 1.16 mmol) and [1,1'-bis(diphenylphosphino) ferrocene] palladium dichloride (21.00 mg, 28.70 µmol) in dioxane (4.0 mL) and water (0.8 mL) was replaced with nitrogen three times, then the reaction solution was heated to 100 °C and stirred for 60 minutes under nitrogen atmosphere. After the reaction was completed, the reaction solution was concentrated and rotary-evaporated, which was separated and purified by column chromatography (MeOH: DCM = 0% ∼ 8%) to obtain the target compound **WX034-2.**

### Step 3: synthesis of compound WX034 and WX035

**WX034-2** was resolved by SFC (chromatographic column: OD (250mm * 30mm, 5µm); mobile phase: [0.1% NH₄HCO₃ EtOH]; B%: 35%-35%) to obtain the enantiomers **WX034** (Rt = 3.665min) and **WX035** (Rt = 3.986 min). **WX034:** ¹H NMR (400 MHz, CHLOROFORM-*d*) δ ppm 8.45 (s, 1 H) 8.09 (br d, *J=* 8.53 Hz, 2 H) 7.69-7.87 (m, 3 H) 5.97 (br d, *J* = 4.27 Hz, 1 H) 4.14 (br dd, *J* = 12.92, 4.39 Hz, 1 H) 3.92-4.04 (m, 1 H) 3.04 (br s, 1 H) 2.98-3.12 (m, 1 H) 2.55-2.71 (m, 6 H) 2.29-2.49 (m, 6 H) 1.24 (br d, *J* = 7.03 Hz, 3 H), MS-ESI *m*/*z*:527.0 [M+H]⁺. **WX035:** ¹H NMR (400 MHz, CHLOROFORM-*d*) δ ppm 8.44 (s, 1 H) 8.08 (br d, *J* = 15.06 Hz, 2 H) 7.70-7.87 (m, 3 H) 6.03 (br s, 1 H) 4.15 (br dd, *J* = 13.18, 4.39 Hz, 1 H) 3.92-4.06 (m, 1 H) 2.97-3.14 (m, 1 H) 2.53-2.71 (m, 5 H) 2.53-2.71 (m, 1 H) 2.29-2.48 (m, 5 H) 2.39 (br s, 1 H) 1.24 (br d, *J* = 7.03 Hz, 3 H), MS-ESI *m*/*z*:527.0 [M+H]⁺.

### Example 24: WX036, WX037

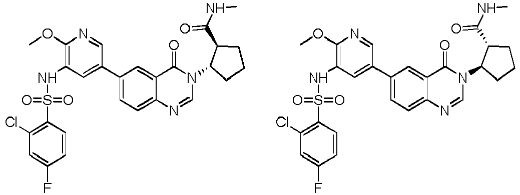

### Synthetic route:

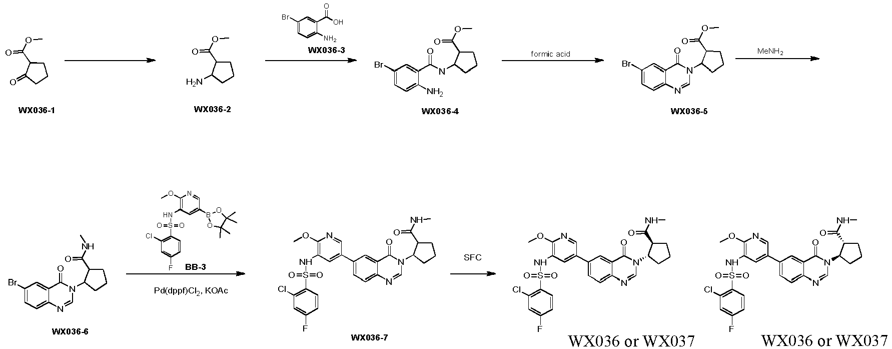

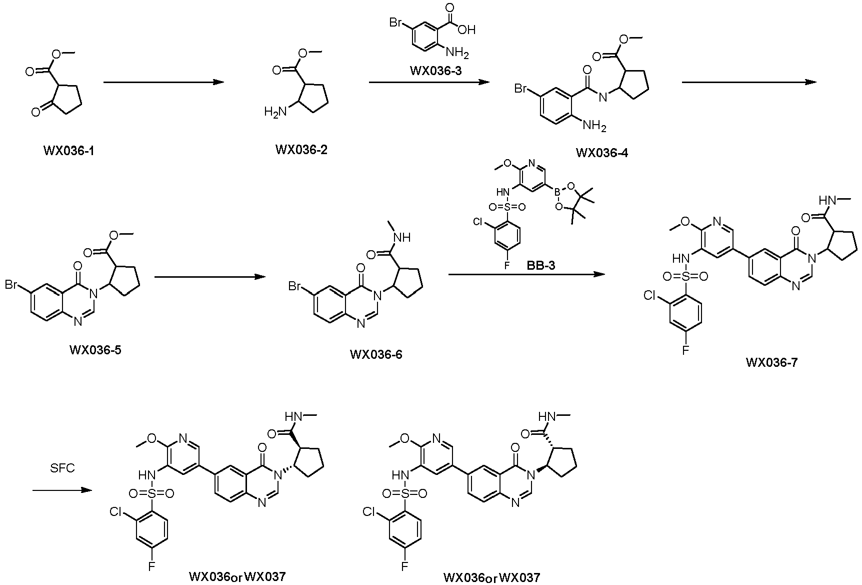

### Step 1: synthesis of compound WX036-2

**WX036-1** (5 g, 35.17 mmol, 4.35 mL) was added to a pre-dried 100mL reaction flask, and dissolved in MeOH (150 mL), then ammonium acetate (27.11 g, 351.73 mmol) and sodium cyanoborohydride (22.10 g, 351.73 mmol) were added. The reaction was stirred at 70 °C for 2 hours. After the reaction was completed, IN HCl was added to the reaction system to adjust the pH to 3, and diluted with 100 mL of ethyl acetate. The aqueous phase was collected after separation, and the organic phase was discarded. The aqueous phase was adjusted to pH 11 with saturated Na₂CO₃ and extracted with ethyl acetate (50 mL × 5). The organic phases were combined, washed with saturated brine (100 mL), dried over anhydrous sodium sulfate, and concentrated under reduced pressure to obtain **WX036-2.**

### Step 2: synthesis of compound WX036-4

**WX036-2** (4 g, 19.56 mmol) and **WX036-3** (4.65 g, 21.51 mmol) were added to a pre-dried 100 mL reaction flask, and dissolved in DCM (50 mL), then HATU (11.15 g, 29.33 mmol) was added, and finally DIEA(5.05 g, 39.11 mmol, 6.81 mL) was added at 0 °C, and the reaction was stirred at 20 °C for 16 hours. After the reaction was completed, 100 mL of water/50 mL of dichloromethane was added to the reaction system for dilution, the organic phase was collected after liquid separation, and the aqueous phase was extracted with dichloromethane (50 mL × 3). The organic phases were combined, washed with saturated brine (200 mL), dried over anhydrous sodium sulfate, concentrated under reduced pressure, slurried with dichloromethane (80 ml)/methanol (10 mL)/methyl tert-butyl ether (50 mL), and the filter cake was collected by filtration to obtain the target compound **WX036-4.** ¹H NMR (400MHz, CHLOROFORM-d) δ = 8.75 (dd, *J* = 1.4, 4.5 Hz, 1H), 8.48 (dd, J = 1.4, 8.4 Hz, 1H), 8.35 (d, *J* = 2.4 Hz, 1H), 7.56-7.45 (m, 2H), 7.39 (d, *J* = 2.3 Hz, 2H), 7.28 (br d, *J* = 2.3 Hz, 1H), 6.68 (d, *J* = 8.9 Hz, 1H), 6.57 (d, *J* = 8.7 Hz, 2H), 6.03 (br d, *J* = 6.1 Hz, 2H), 5.77 (br s, 2H), 5.50 (br s, 3H), 4.51 (quin, *J* = 7.6 Hz, 2H), 3.72 (s, 6H), 2.81 (s, 2H), 2.72 (q, *J* = 8.1 Hz, 2H), 2.33-2.20 (m, 2H), 2.14-1.92 (m, 4H), 1.90-1.75 (m, 5H), 1.70-1.54 (m, 9H), 1.52-1.39 (m, 3H).

### Step 3: synthesis of compound WX036-5

**WX036-4** (3.6 g, 10.55 mmol) was added to a pre-dried 250 mL single-necked flask and dissolved in formic acid (33.99 g, 738.57 mmol, 27.86 mL). The reaction solution was stirred at 100 °C for 16 hours. After the reaction was completed, the formic acid was rotary-evaporated, diluted with 100 mL of water/100 mL of ethyl acetate. The organic phase was collected after liquid separation, and the aqueous phase was extracted with ethyl acetate (50 mL × 3). The organic phases were combined, washed with saturated brine (20 mL), dried over anhydrous sodium sulfate, concentrated under reduced pressure, and purified by column chromatography (dichloromethane: methanol = 1: 0 to 100: 1) to obtain the target compound **WX036-5.** ¹H NMR (400MHz, CHLOROFORM-d) δ = 9.43 (br s, 1H), 8.45 (d, *J* = 2.0 Hz, 1H), 8.10 (d, *J* = 8.8 Hz, 1H), 7.97 (dd, *J* = 2.2, 8.6 Hz, 1H), 5.18 (q, *J* = 8.8 Hz, 1H), 3.68 (s, 3H), 3.57 (q, *J* = 8.8 Hz, 1H), 2.45-2.27 (m, 3H), 2.23-2.11 (m, 1H), 2.07-1.95 (m, 1H), 1.95-1.84 (m, 1H).

### Step 4: synthesis of compound WX036-6

**WX036-5** (800 mg, 2.28 mmol) was added to the pre-dried 40 mL reaction flask and dissolved in the solution of methylamine in methanol (30 mL). The reaction solution was stirred at 20 °C for 16 hours. After the reaction was completed, 10 mL of water and 10 mL of ethyl acetate were added to the reaction system for dilution, the organic phase was collected after liquid separation, and the aqueous phase was extracted with ethyl acetate (5 mL × 3). The organic phases were combined, washed with saturated brine (20 mL), dried over anhydrous sodium sulfate, concentrated under reduced pressure, and purified by column chromatography (dichloromethane: methanol = 1:0 to 20:1) to obtain the target compound **WX036-6.** ¹H NMR (400MHz, CHLOROFORM-d) δ = 8.39 (d, J = 2.3 Hz, 1H), 8.11 (s, 1H), 7.84 (dd, J = 2.3, 8.7 Hz, 1H), 7.59 (d, J = 8.7 Hz, 1H), 5.90 (br s, 1H), 4.95 (q, J = 8.2 Hz, 1H), 3.38 (q, J = 8.3 Hz, 1H), 2.80 (d, J = 4.9 Hz, 3H), 2.41-2.28 (m, 1H), 2.28-2.04 (m, 4H), 2.03-1.88 (m, 3H).

### Step 5: synthesis of compound WX036-7

**WX036-6** (270 mg, 770.97 µmol), **BB-3** (409.56 mg, 925.16 µmol) and methyl acetate (226.99 mg, 2.31 mmol) were added to a pre-dried 40mL reaction flask, followed by addition of dioxane (5 mL) and water (0.5 mL), and replaced with nitrogen. Pd (dppf)Cl₂ (56.41 mg, 77.10 µmol) was then added thereto and replaced with nitrogen again. The reaction solution was stirred at 90 °C for 12 hours. After the reaction was completed, 10 mL of water and 10 mL of ethyl acetate were added to the reaction system for dilution, the organic phase was collected after liquid separation, and the aqueous phase was extracted with ethyl acetate (5 mL × 3). The organic phases were combined, washed with saturated brine (20 mL), dried over anhydrous sodium sulfate, concentrated under reduced pressure, and purified by preparative high performance liquid chromatography (method: column: Nano-micro Kromasil C18 100 * 30mm 5µm; mobile phase : [Water (0.1% TFA) -ACN]; B%: 35% -55%, 10min) to obtain **WX036-7.**

### Step 6: synthesis of compound WX036 and WX037

**WX036-7** (0.5 g, 853.19 µmol) was subjected to SFC resolution (method: chromatographic column: AS (250mm * 30mm, 5µm); mobile phase: [MeOH]; B%: 34%-34%, 4min) to obtain the enantiomers **WX036** (retention time 1.10 min) and **WX037** (retention time 2.36 min). **WX036:** ¹H NMR (400MHz, CHLOROFORM-d) δ = 8.31 (d, *J* = 1.8 Hz, 1H), 8.16-8.07 (m, 3H), 7.98 (d, *J* = 2.2 Hz, 1H), 7.89-7.81 (m, 1H), 7.81-7.74 (m, 1H), 7.55 (s, 1H), 7.28-7.24 (m, 1H), 7.18-7.05 (m, 1H), 5.91 (br s, 1H), 4.99 (q, *J=* 8.0 Hz, 1H), 3.98 (s, 3H), 3.40 (br d, *J=* 7.7 Hz, 1H), 2.80 (d, *J* = 4.2 Hz, 3H), 2.39-2.30 (m, 1H), 2.24 (br s, 2H), 2.16-2.05 (m, 1H), 2.03-1.92 (m, 2H). **ESI,** m/z = 586.1 [M+1]. **WX037:** 1H NMR (400MHz, CHLOROFORM-d) δ = 8.31 (d, *J* = 2.0 Hz, 1H), 8.21-8.06 (m, 3H), 7.98 (d, *J* = 2.2 Hz, 1H), 7.89-7.80 (m, 1H), 7.80-7.73 (m, 1H), 7.55 (s, 1H), 7.28-7.23 (m, 1H), 7.17-7.04 (m, 1H), 5.91 (br d, *J* = 4.0 Hz, 1H), 4.98 (q, *J* = 8.2 Hz, 1H), 3.98 (s, 3H), 3.39 (q, *J* = 8.3 Hz, 1H), 2.80 (d, *J* = 4.9 Hz, 3H), 2.40-2.30 (m, 1H), 2.29-2.18 (m, 2H), 2.16-2.05 (m, 1H), 2.02-1.91 (m, 2H). **ESI,** m/z = 586.1 [M+1].

### Example 25: WX038, WX039

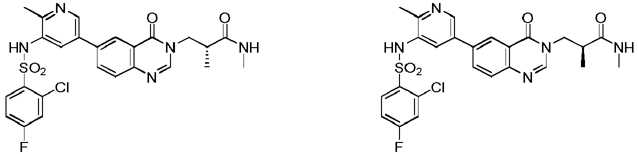

### Synthetic route:

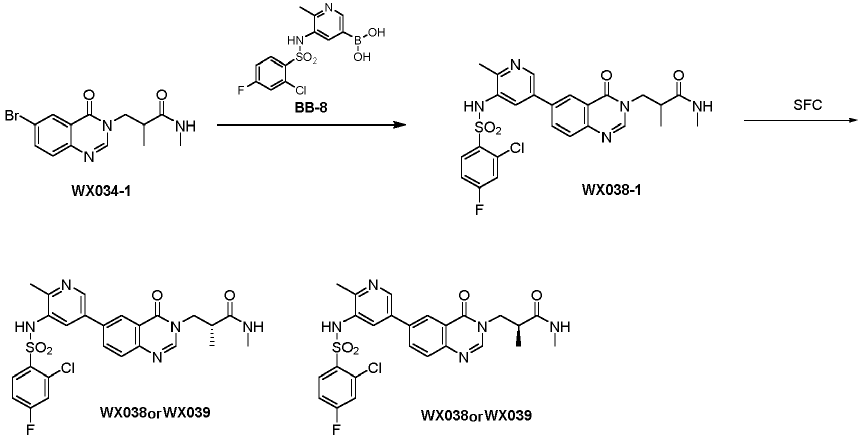

### Step 1: synthesis of compound WX038-1

A suspension of **WX034-2** (260.00 mg, 525.42 µmol), **BB-8** (300.00 mg, 446.06 µmol), potassium acetate (206.00 mg, 2.10 mmol) and [1,1'-bis(diphenylphosphino)ferrocene] palladium dichloride (38.00 mg, 51.93 µmol) in dioxane (10 mL) and water (2 mL) was replaced with nitrogen three times, then the reaction solution was heated to 100 °C and stirred for 1 hour under nitrogen atmosphere. After the reaction was completed, the reaction solution was concentrated and rotary-evaporated. The crude product was slurried with water (100 mL), and then extracted with dichloromethane (100 mL). The organic phase was rotary-evaporated and separated by high-performance liquid chromatography (column: Xtimate C18 150 * 25mm * 5µm; mobile phase: [water (10mM NH₄HCO₃) -ACN]; B%: 19% -39%, 8min) to obtain the target compound **WX038-1.**

### Step 2: synthesis of compound WX038 and WX039

**WX038-1** was resolved by SFC (column: OD (250mm * 30mm, 5µm); mobile phase: [0.1% NH₄HCO₃ EtOH]; B%: 35%-35%) to obtain the enantiomers **WX038** (Rt = 4.003 min) and **WX039** (Rt = 4.411 min). **WX038:** ¹H NMR (400 MHz, DMSO-*d*₆) δ ppm 8.74 (s, 1 H) 8.20-8.39 (m, 2 H) 8.11 (dd, *J* = 8.53, 2.01 Hz, 1 H) 8.03 (dd, *J* = 8.91, 5.90 Hz, 1 H) 7.95 (br d, *J* = 4.77 Hz, 1 H) 7.73-7.88 (m, 3 H) 7.35-7.50 (m, 1 H) 3.94-4.19 (m, 2 H) 2.84-2.96 (m, 1 H) 2.54 (br s, 3 H) 2.43 (s, 3 H) 1.14 (d, *J* = 7.03 Hz, 3 H), MS-ESI *m*/*z:* 544.0 [M+H]⁺. **WX039:** ¹H NMR (400 MHz, DMSO-*d*₆) δ ppm 8.74 (s, 1 H) 8.22-8.36 (m, 2 H) 8.11 (dd, *J* = 8.41, 1.88 Hz, 1 H) 8.03 (dd, *J* = 8.78, 6.02 Hz, 1 H) 7.94 (br d, *J* = 4.52 Hz, 1 H) 7.72-7.87 (m, 3 H) 7.43 (td, *J* = 8.34, 2.38 Hz, 1 H) 3.90-4.22 (m, 2 H) 2.83-3.01 (m, 1 H) 2.54 (br s, 3 H) 2.43 (s, 3 H) 1.11-1.17 (m, 3 H), MS-ESI *m*/*z:* 544.0 [M+H]⁺.

### Example 26: WX040

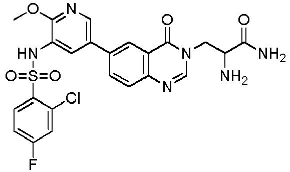

### Synthetic route:

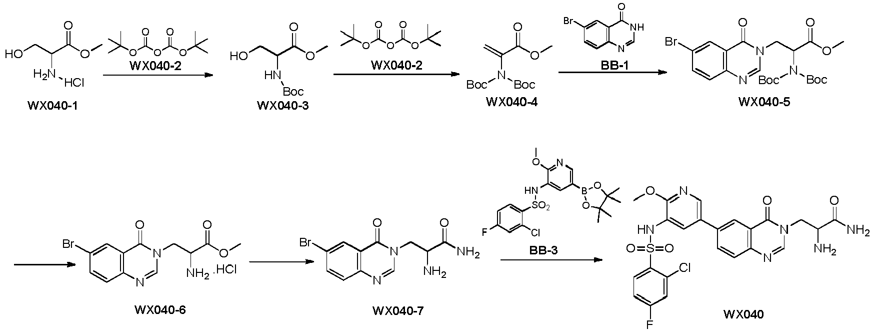

### Step 1: synthesis of compound WX040-3

Compound **WX040-1** (5.00 g, 32.14 mmol) and sodium bicarbonate (172.80 g, 80.00 mL) were dissolved in dioxane (120.00 mL), and **WX040-2** (21.04 g, 96.42 mmol, 22.15 mL) was added under nitrogen atmosphere and stirred at 25 °C for 14 hours. The reaction solution was filtered. The filtrate was concentrated by rotary evaporation and extracted with ethyl acetate (20 mL × 3). The organic phase was concentrated by rotary evaporation to obtain **WX040-3.** 1H NMR (400 MHz, CHLOROFORM-d) δ = 5.49(s, 1H), 4.35(s, 1H), 3.85-3.94(m, 2H), 3.75(s, 3H), 2.72-2.73(m, 1H), 1.42(s, 9H).

### Step 2: synthesis of compound WX040-4

Compound **WX040-3** (7.00 g, 31.93 mmol) was dissolved in acetonitrile (120.00 mL), followed by addition of 4-dimethylaminopyridine (780.18 mg, 6.39 mmol) and **WX040-2** (34.84 g, 159.65 mmol, 36.68 mL) under nitrogen atmosphere at 0 °C, and stirred at 25 °C for 15 minutes. The reaction was carried out at 60 °C for 12 hours. The reaction solution was concentrated, and separated by silica gel plate chromatography (petroleum ether: ethyl acetate = 7: 1) to obtain **WX040-4.** ¹H NMR (400 MHz, CHLOROFORM-d) δ = 6.32(s, 1H), 5.62(s, 1H), 3.77(s, 3H), 1.44(s, 18H).

### Step 3: synthesis of compound WX040-5

Compound **WX040-4** (6.55 g, 21.74 mmol), **BB-1** (4.89 g, 21.74 mmol) and cesium carbonate (2.12 g, 6.52 mmol) were dissolved in acetonitrile (100.00 mL) and stirred at 90 °C for 12 hours. The reaction solution was filtered. The filtrate was collected and concentrated by rotary evaporation, which was separated and purified by silica gel column (petroleum ether: ethyl acetate = 3:1) to obtain **WX040-5.** ¹H NMR (400MHz, DMSO-*d*₆) δ = 8.28(s, 1H), 8.21-8.22(d, J = 2.4Hz, 1H), 7.97-8.00(dd, J = 8.8, 1H), 7.63-7.65(d, J = 8.8Hz, 1H), 5.35-5.39(m, 1H), 4.51-4.60(m, 1H), 3.73 (s, 3H), 1.26 (s, 18H).

### Step 4: synthesis of compound WX040-6

Compound **WX040-5** (2.00 g, 3.80 mmol) was dissolved in hydrochloric acid ethanol (4 M, 30.00 mL), and stirred at 20 °C for 4 hours. During the stirring process, the precipitation was precipitated out. The reaction solution was filtered, and the filter cake was collected to obtain **WX040-6.**

### Step 5: synthesis of compound WX040-7

Ammonia gas was introduced to methanol (5.00 mL) at 0 °C to saturation to prepare an ammonia/methanol solution and placed in a 0 °C muffler. Compound **WX040-6** (100.00 mg, 275.78 µmol) was dissolved in methanol (1.00 mL), added to ammonia/methanol solution at 0 °C under nitrogen atmosphere, sealed and reacted at 60 °C for 12 hours. The reaction solution was allowed to stand overnight, and colorless crystals were precipitated out. The reaction solution was filtered, and the filter cake was collected to obtain **WX040-7.** ¹H NMR (400MHz, DMSO- *d₆*) δ = 8.31(s, 1H), 8.23-8.24(d, J = 2Hz, 1H), 7.97-7.99(m, 1H), 7.63-7.65(m, 1H), 7.52-7.54(m, 1H), 7.17(s, 1H), 4.26-4.31(m, 1H), 3.77-3.83(m, 1H), 3.17(s, 2H).

### Step 6: synthesis of compound WX040

Compound **WX040-7** (80.00 mg, 257.13 µmol), compound **BB-3** (113.83 mg, 257.13 µmol), potassium acetate (100.94 mg, 1.03 mmol) and ferrocene palladium chloride (37.63 mg, 51.43 µmol) were dissolved in dioxane (8.00 mL) and water (1 mL), the reaction solution was stirred at 80 °C for 12 hours under nitrogen atmosphere. The reaction solution was concentrated, slurried with water (20 mL). The filter cake was collected, followed by addition of *N*,*N*-dimethylformamide (7 mL) and waste water treatment agent (5 mL), and allowed to stand overnight. The mixture was filtered and the filtrate was rotary-evaporated, which was separated by preparative HPLC to obtain the target compound **WX040.** ¹H NMR (400MHz, DMSO- *d₆*) δ = 8.31(s, 1H), 8.26(s, 1H), 8.24-8.25(d, J = 2Hz, 1H), 8.06-8.08(m, 1H), 7.95-7.99(m, 1H), 7.87(s, 1H), 7.75-7.77(d, J = 8.4Hz, 1H), 7.69-7.71(m, 2H), 7.36-7.37(m, 2H), 4.31-4.35(m, 1H), 3.97-4.01(m, 1H), 3.73-3.76(m, 1H), 3.71(s,3H).

### Example 27: WX041

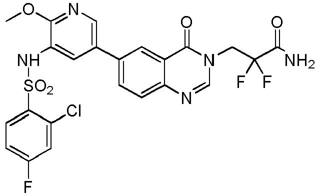

### Synthetic route:

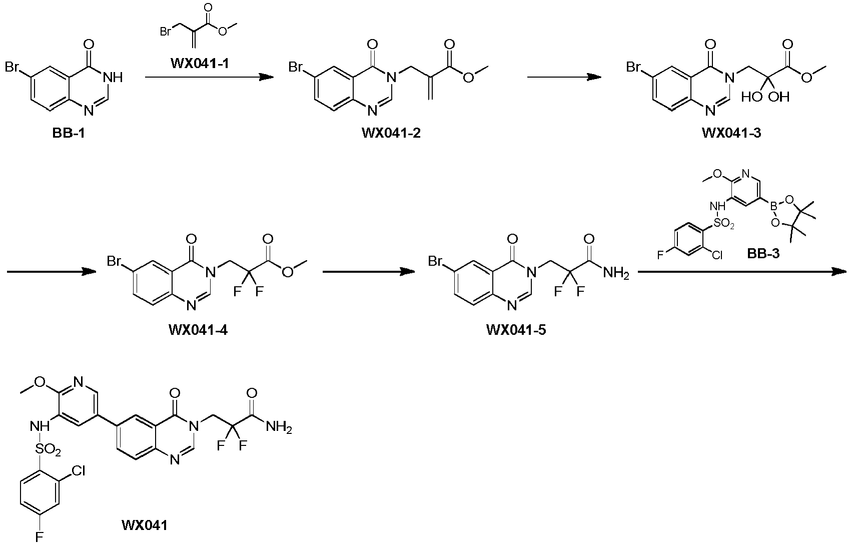

### Step 1: synthesis of compound WX041-2

In a 40 mL reaction flask, **WX041-1** (1.91 g, 10.67 mmol) was added to a solution of **BB-1** (2.00 g, 8.89 mmol) and potassium carbonate (2.46 g, 17.78 mmol) in *N*,*N*-dimethylformamide (20.00 mL). After the addition was completed, the reaction solution was stirred at 25 °C for 12 hours under nitrogen atmosphere. After the reaction was completed, water (15 mL) was added to the reaction solution to quench the reaction, and then extracted with dichloromethane (20 mL × 3). The organic phases were combined, dried over anhydrous sodium sulfate, concentrated, and purified by column chromatography (petroleum ether: ethyl acetate = 1:1) to obtain **WX041-2.**

### Step 2: synthesis of compound WX041-3

In a 250 mL three-necked flask, ozone was introduced slowly to a solution of **WX041-2** (1 g, 3.09 mmol) in dichloromethane (50 mL) at -78 °C until the solution turned blue. Nitrogen was immediately bubbled thereto until the solution turned colorless, follwed by addition of dimethyl sulfide (961.41 mg, 15.47 mmol, 1.14 mL) was added. After the addition was completed, the reaction solution was naturally heated to 25 °C and stirred for 1 hour. The reaction solution was directly rotary-evaporated and purified by preparative TLC (petroleum ether: ethyl acetate = 0:1) to obtain **WX041-3.** ¹H NMR (400MHz, CHLOROFORM-d) δ = 8.41 (d, *J* = 2.0 Hz, 1H), 7.97-7.83 (m, 2H), 7.63 (d, *J* = 8.8 Hz, 1H), 5.24 (s, 2H), 3.97 (s, 3H), 2.77-2.73 (m, 1H).

### Step 3: synthesis of compound WX041-4

In a 40 mL reaction flask, diethylaminosulfur trifluoride (1.48 g, 9.18 mmol, 1.21 mL) was added to a solution of **WX041-3** (630 mg, 1.84 mmol) in dichloromethane (1.00 mL). After the addition was completed, the reaction solution was stirred at 25 °C for 12 hours under nitrogen atmosphere. After the reaction was completed, the reaction solution was slowly poured into an ice-water mixture (20 mL), and then extracted with dichloromethane (20 mL × 3). The organic phases were combined, dried over anhydrous sodium sulfate, concentrated, and purified by preparative TLC (petroleum ether: ethyl acetate = 1: 1) to obtain **WX041-4.** ¹H NMR (400MHz, CHLOROFORM-d) δ = 8.41 (d, *J* = 2.4 Hz, 1H), 8.04 (s, 1H), 7.87 (dd, *J* = 2.0, 8.7 Hz, 1H), 7.61 (d, *J* = 8.8 Hz, 1H), 4.62 (t, *J* = 13.0 Hz, 2H), 3.90 (s, 3H).

### Step 4: synthesis of compound WX041-5

In a 40 mL reaction flask, a solution of ammonia (7 M, 1.15 mL) in methanol was added to a solution of **WX041-4** (140 mg, 403.33 µmol) in methanol (1 mL). After the addition was completed, the reaction solution was stirred at 25 °C for 12 hours under nitrogen atmosphere. The reaction solution was directly concentrated to obtain a crude product. The crude product was washed once with methyl tert-butyl ether (5 mL) to obtain **WX041-5.** ¹H NMR (400MHz, CHLOROFORM-d) δ = 8.44 (d, *J* = 2.2 Hz, 1H), 8.08 (s, 1H), 7.89-7.85 (m, 1H), 7.61 (d, *J* = 8.4 Hz, 1H), 4.72 (t, *J* = 14.6 Hz, 2H).

### Step 5: synthesis of compound WX041

In a 40 mL reaction flask, sodium bicarbonate (34.16 mg, 406.60 µmol, 15.81 µL) and 1,1-bis(diphenylphosphine) ferrocene palladium chloride (9.92 mg, 13.55 µmol) were added to a solution of **BB-3** (60 mg, 135.53 µmol) and **WX041-5** (45.01 mg, 135.53 µmol) in 1,4-dioxane:water = 10:1 (1 mL). After the addition was completed, the reaction solution was stirred at 80 °C for 5 hours under nitrogen atmosphere. The reaction solution was cooled to room temperature, water (5 mL) was added to quench the reaction, and then extracted with dichloromethane (10 mL ^{∗} 3). The organic phases were combined, dried over anhydrous sodium sulfate, concentrated, purified by preparative TLC (dichloromethane: methanol = 15: 1), and further purified by preparative HPLC (HPLC_ET12919-102-P1A2, column: Luna C18 100 ^{∗} 30mm 5µm; mobile phase: [water (0.1% TFA) -ACN]; B%: 40% -60%, 10min) to obtain **WX041.** ¹H NMR (400MHz, METHANOL-*d₄*) δ = 8.45-8.21 (m, 3H), 8.15-7.98 (m, 3H), 7.82 (d, *J* = 8.2 Hz, 1H), 7.49 (br d, *J* = 8.2 Hz, 1H), 7.25 (br t, *J* = 7.8 Hz, 1H), 3.89 (d, *J* = 1.0 Hz, 3H).

### Example 28: WX042, WX043

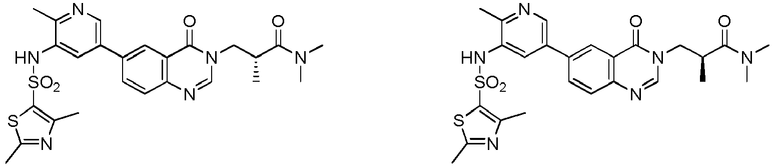

### Synthetic route:

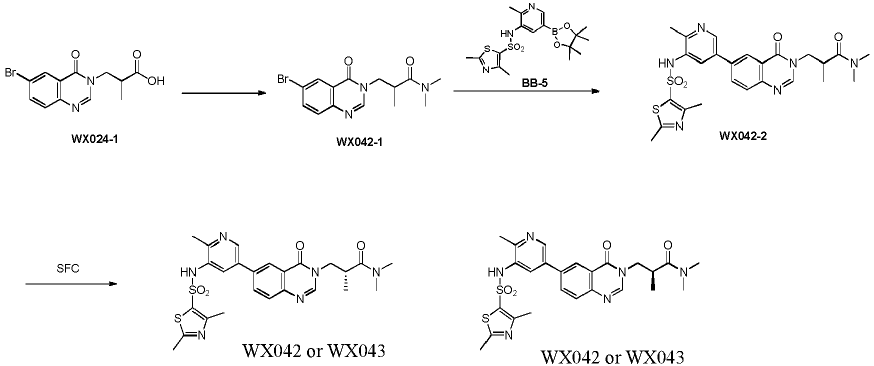

### Step 1: synthesis of compound WX042-1

Compound **WX024-1** (0.2 g, 610.68 µmol), dimethylamine alcohol solution (445.00 mg, 3.26 mmol, 0.5 mL), carbodiimide (0.12 g, 625.97 µmol), 2-hydroxypyridine-N-oxide (0.07 g, 630.07 µmol) and triethylamine (363.50 mg, 3.59 mmol, 0.5 mL) was dissolved in dichloromethane (6 mL) and stirred at 50 °C for 16 hours. The reaction solution was diluted with water (30 mL) and extracted with dichloromethane (30 mL × 2). The organic phase was rotary-evaporated to obtain the target compound **WX042-1.**

### Step 2: synthesis of compound WX042-2

Compound **WX042-1** (0.15 g, 337.52 µmol), compound **BB-5** (180.00 mg, 367.83 µmol), potassium acetate (132.50 mg, 1.35 mmol) and ferrocene palladium chloride (49.39 mg, 67.50 µmol) were dissolved in dioxane (10 mL) and water (2 mL), the reaction solution was stirred at 105 °C for 2 hours under nitrogen atmosphere. The reaction solution was diluted with water (30 mL), and then extracted with dichloromethane (30 mL × 2). The organic phase was rotary-evaporated, which was separated by preparative HPLC (Kromasil 150 ^{∗} 25mm ^{∗} 10µm; mobile phase: [water (0.05% hydroxide Ammonium v/v) -ACN]; B%: 13% -23%, 8min) to obtain target compound **WX042-2.**

### Step 3: synthesis of compound WX042 and WX043

Compound **WX042-2** was resolved by supercritical fluid chromatography (separation conditions [chromatographic column: OD-3 (100mm ^{∗} 4.6mm, 3µm); mobile phase: [0.1% NH₄HCO₃ EtOH]; B%: 40%-40%, 8min]) to obtain the enantiomers **WX042** and **WX043. WX042:** ¹H NMR (400 MHz, METHANOL-*d₄*) δ ppm 1.11 (d, *J* = 6.78 Hz, 3 H) 2.17-2.28 (m, 3 H) 2.33 (s, 3 H) 2.58 (s, 3 H) 2.78 (s, 3 H) 2.93 (s, 3 H) 3.48-3.57 (m, 1 H) 3.98-4.12 (m, 2 H) 7.66-7.80 (m, 2 H) 7.95 (dd, *J* = 8.53, 2.26 Hz, 1 H) 8.16-8.31 (m, 1 H) 8.16-8.31 (m, 1 H) 8.58 (d, *J* = 2.26 Hz, 1 H) **WX043**:¹H NMR (400 MHz, METHANOL- *d₄*) δ ppm 1.11 (d, *J* = 6.78 Hz, 3 H) 2.17-2.28 (m, 3 H) 2.33 (s, 3 H) 2.58 (s, 3 H) 2.78 (s, 3 H) 2.93 (s, 3 H) 3.48-3.57 (m, 1 H) 3.98-4.12 (m, 2 H) 7.66-7.80 (m, 2 H) 7.95 (dd, *J* = 8.53, 2.26 Hz, 1 H) 8.16-8.31 (m, 1 H) 8.16-8.31 (m, 1 H) 8.58 (d, *J* = 2.26 Hz, 1 H). The retention time is 4.001 min and 4.960 min respectively, and the ratio is 1:1.

### Example 29: WX044, WX045

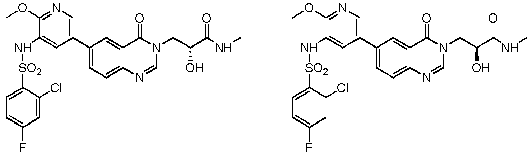

### Synthetic route:

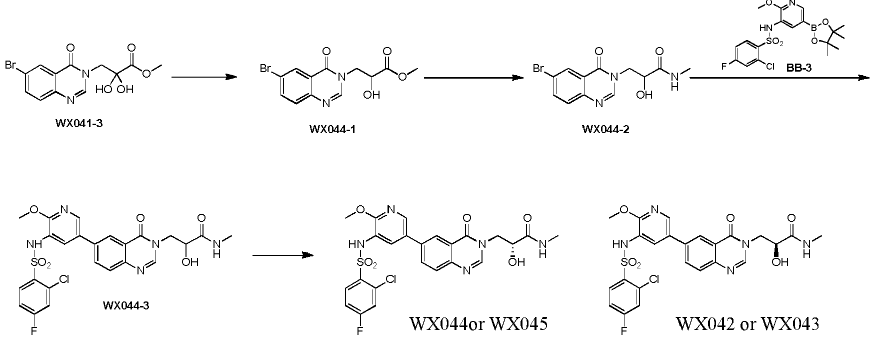

### Step 1: synthesis of compound WX044-1

In a reaction flask, sodium borohydride (1.67 g, 7.87 mmol) was added to a solution of **WX041-3** (1.35 g, 3.93 mmol) in dichloromethane (13.5 mL). After the addition was completed, the reaction solution was stirred at 25°C for 12 hours. Water (10 mL) was added to the reaction solution to quench the reaction, and then extracted with dichloromethane (10 mL × 3). The organic phases were combined, dried over anhydrous sodium sulfate, concentrated, and purified by preparative TLC (petroleum ether: ethyl acetate = 0: 1) to obtain **WX044-1.** ¹H NMR (400MHz, CHLOROFORM-d) δ = 8.40 (d, *J* = 2.2 Hz, 1H), 8.08-7.99 (m, 1H), 7.86-7.78 (m, 1H), 7.58 (d, *J* = 8.8 Hz, 1H), 7.38 (dd, *J* = 2.2, 8.6 Hz, 1H), 6.60 (d, *J* = 8.6 Hz, 1H), 4.86-4.69 (m, 1H), 4.65-4.53 (m, 1H), 4.51-4.38 (m, 1H), 4.19 (dd, *J* = 6.4, 13.8 Hz, 1H), 4.05-3.91 (m, 1H), 3.87 (s, 3H).

### Step 2: synthesis of compound WX044-2

In a reaction flask, a solution of methylamine (2 M, 9.17 mL) in tetrahydrofuran was added to a solution of **WX044-1** (300 mg, 917.07 µmol) in tetrahydrofuran (10 mL). After the addition was completed, the reaction solution was stirred under nitrogen for 12 hours at 25 °C. The reaction solution was directly concentrated to obtain **WX044-2,** which was directly used in the next reaction.

### Step 3: synthesis of compound WX044-3

In a reaction flask, sodium bicarbonate (113.86 mg, 1.36 mmol, 52.71 µL) and 1,1-bis(diphenylphosphine) ferrocene palladium chloride (33.06 mg, 45.18 µmol) were added to a solution of **BB-3** (200 mg, 451.78 µmol) and **WX044-2** (147.35 mg, 451.78 µmol) in 1,4-dioxane:water = 10:1 (3 mL). After the addition was completed, the reaction solution was stirred at 100 °C for 8 hours under nitrogen atmosphere. The reaction solution was cooled to room temperature, water (5 mL) was added to quench the reaction, and then extracted with dichloromethane (10 mL × 3). The organic phases were combined, dried over anhydrous sodium sulfate, concentrated, and purified by preparative TLC (dichloromethane: methanol = 15: 1) to obtain the target compound **WX044-3.**

### Step 3: synthesis of compound WX044 and WX045

**WX044-3** was purified by SFC (chromatographic column: AS (250mm ^{∗} 30mm, 5µm); mobile phase: [MeOH]; B%: 40% -40%), resolved to obtain enantiomers, which were then purified by prep-HPLC (min column: Luna C18 100 ^{∗} 30mm 5µm; mobile phase: [water (0.1% TFA) -ACN]; B%: 30% -60%, 10min) to obtain **WX044,** with a retention time of 1.22 minutes and **WX045** with a retention time of 2.61 minutes. **WX044:** ¹H NMR (400MHz, METHANOL-*d₄*) δ = 8.36-8.21 (m, 3H), 8.10 (dd, *J* = 5.7, 8.8 Hz, 1H), 8.04-7.97 (m, 2H), 7.77 (d, *J* = 8.4 Hz, 1H), 7.47 (dd, *J* = 2.6, 8.4 Hz, 1H), 7.26-7.20 (m, 1H), 4.68 (dd, *J* = 4.0, 13.7 Hz, 1H), 4.45 (dd, *J* = 3.9, 8.3 Hz, 1H), 3.98 (dd, *J* = 8.4, 13.7 Hz, 1H), 3.86 (s, 3H), 2.78 (s, 3H); **WX045**: ¹H NMR (400MHz, METHANOL- *d₄*) δ = 8.38-8.26 (m, 2H), 8.22 (d, *J =* 2.2 Hz, 1H), 8.10 (dd, *J* = 6.0, 8.8 Hz, 1H), 8.05-7.95 (m, 2H), 7.77 (d, *J* = 8.6 Hz, 1H), 7.46 (dd, *J* = 2.5, 8.5 Hz, 1H), 7.29-7.19 (m, 1H), 4.68 (dd, *J* = 3.7, 13.7 Hz, 1H), 4.45 (dd, *J* = 3.8, 8.4 Hz, 1H), 3.98 (dd, *J* = 8.4, 13.8 Hz, 1H), 3.86 (s, 3H), 2.78 (s, 3H).

### Example 30: WX046, WX047

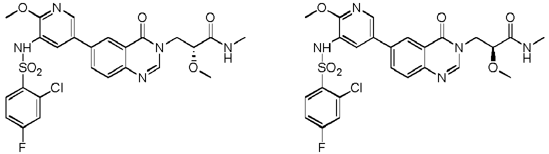

### Synthetic route:

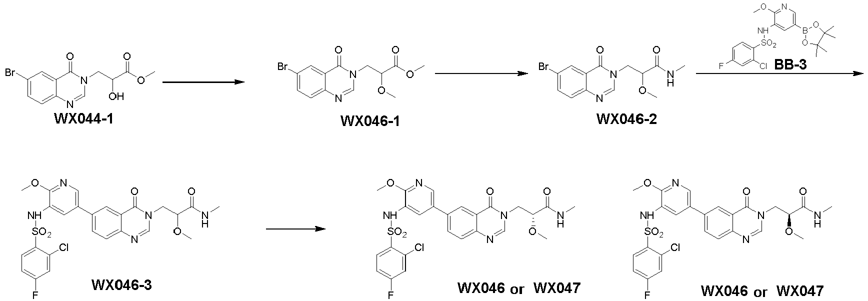

### Step 1: synthesis of compound WX046-1

In a 40 mL reaction flask, methyl iodide (1.30 g, 9.17 mmol, 570.91 µL) was added to a solution of **WX044-1** (300 mg, 917.07 µmol) and silver oxide (2.13 g, 9.17 mmol) in acetonitrile (10 mL). After the addition was completed, the reaction solution was stirred at 80 °C for 12 hours under nitrogen. The reaction solution was filtered to obtain a mother liquor. The mother liquor was diluted with dichloromethane (50 mL) and further washed once with water (10 mL). The organic phase was dried over anhydrous sodium sulfate, concentrated, and purified by preparative TLC (petroleum ether: ethyl acetate = 1: 2) to obtain **WX046-1.** ¹H NMR (400MHz, CHLOROFORM-d) δ = 8.42 (d, *J* = 2.0 Hz, 1H), 8.05 (s, 1H), 7.83 (dd, *J* = 2.2, 8.6 Hz, 1H), 7.58 (d, *J* = 8.8 Hz, 1H), 4.55 (dd, *J* = 3.4, 13.8 Hz, 1H), 4.16 (dd, *J* = 3.4, 8.2 Hz, 1H), 3.99 (dd, *J* = 8.0, 13.8 Hz, 1H), 3.81 (s, 3H), 3.39 (s, 3H).

### Step 2: synthesis of compound WX046-2

In a 40 mL reaction flask, a solution of methylamine (2 M, 2.93 mL) in methanol (7 M, 1.15 mL) was added to a solution of **WX046-1** (100 mg, 293.12 µmol) in tetrahydrofuran (10 mL). After the addition was completed, the reaction solution was stirred under nitrogen atmosphere for 12 hours at 25 °C. The reaction solution was directly concentrated to obtain **WX046-2**, which was directly used in the next reaction. ¹H NMR (400MHz, CHLOROFORM-d) δ = 8.43 (d, *J* = 2.0 Hz, 1H), 8.06 (s, 1H), 7.83 (dd, *J* = 2.2, 8.6 Hz, 1H), 7.58 (d, *J* = 8.6 Hz, 1H), 6.60 (br s, 1H), 4.81-4.62 (m, 1H), 4.10-3.97 (m, 2H), 3.46 (s, 3H), 2.88-2.81 (m, 2H), 2.89-2.80 (m, 1H).

### Step 3: synthesis of compound WX046-3

In a reaction flask, 1,1-bis (diphenylphosphine) ferrocene palladium chloride (20.66 mg, 28.24 µmol) and sodium bicarbonate (47.44 mg, 564.72 µmol, 21.96 µL) were added to a solution of **WX046-2** (125 mg, 282.36 µmol) and **BB-3** (96.05 mg, 282.36 µmol) in 1,4-dioxane:water = 10:1 (1 mL). After the addition was completed, the reaction solution was stirred at 100 °C for 8 hours under nitrogen atmosphere. The reaction solution was cooled to room temperature, water (5 mL) was added to quench the reaction, and then extracted with dichloromethane (10 mL × 3). The organic phases were combined, dried over anhydrous sodium sulfate, concentrated, and purified by preparative TLC (dichloromethane: methanol = 15: 1) to obtain the target compound **WX046-3.**

### Step 4: synthesis of compound WX046 and WX047

**WX046-3** was purified by SFC (chromatographic column: AS (250mm ^{∗} 30mm, 5µm); mobile phase: [0.1% NH₄HCO₃ MeOH]; B%: 30%-30%), and resolved to obtain the enantiomers, which were then purified by prep-HPLC (column: Luna C18 100 ^{∗} 30mm 5µm; mobile phase: [water (0.1% TFA)-ACN]; B%: 35% -60%, 10min) to obtain **WX046** with a retention time of 3.31 minutes and **WX047** with a retention time of 3.65 minutes. **WX046:** ¹H NMR (400MHz, METHANOL-*d₄*) δ = 8.38-8.19 (m, 3H), 8.10 (dd, *J* = 5.8, 8.9 Hz, 1H), 8.04-7.94 (m, 2H), 7.76 (d, *J* = 8.6 Hz, 1H), 7.46 (dd, *J* = 2.4, 8.6 Hz, 1H), 7.28-7.20 (m, 1H), 4.55 (dd, *J* = 4.2, 13.9 Hz, 1H), 4.20-4.13 (m, 1H), 4.11-4.04 (m, 1H), 3.86 (s, 3H), 3.39 (s, 3H), 2.77 (s, 3H); **WX047**:¹H NMR (400MHz, METHANOL- *d₄*) δ = 8.35-8.19 (m, 3H), 8.10 (dd, *J=* 5.8, 8.9 Hz, 1H), 8.04-7.96 (m, 2H), 7.77 (d, *J* = 8.4 Hz, 1H), 7.47 (dd, *J* = 2.5, 8.5 Hz, 1H), 7.28-7.17 (m, 1H), 4.55 (dd, *J* = 4.2, 13.7 Hz, 1H), 4.20-4.12 (m, 1H), 4.11-4.04 (m, 1H), 3.87 (s, 3H), 3.39 (s, 3H), 2.77 (s, 3H).

### Example 31: WX048, WX049

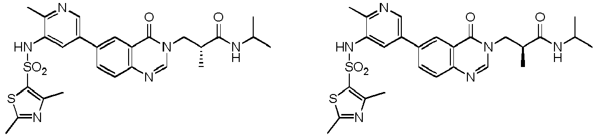

### Synthetic route:

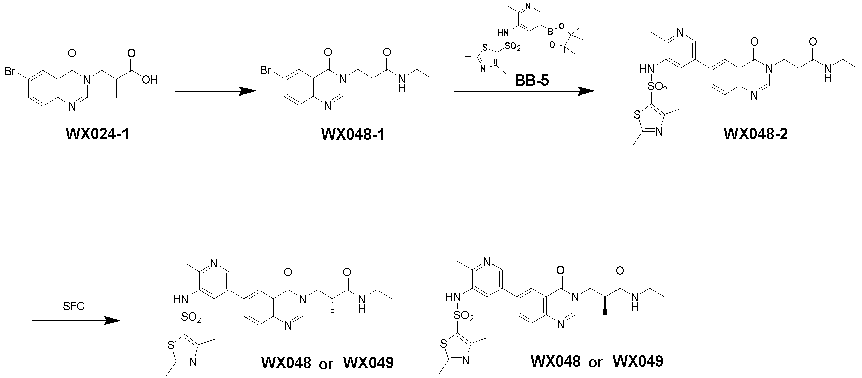

### Step 1: synthesis of compound WX048-1

Compound **WX024-1** (0.5 g, 1.53 mmol), isopropylamine (859.99 mg, 14.55 mmol, 1.25 mL), carbodiimide (300.00 mg, 1.56 mmol), 2-hydroxypyridine *N*-oxide (175.00 mg, 1.58 mmol) and triethylamine (908.75 mg, 8.98 mmol, 1.25 mL) were dissolved in dichloromethane (20.00 mL) and stirred at 50 °C for 16 hours. The reaction solution was diluted with water (30 mL) and extracted with dichloromethane (30 mL × 2), and the organic phase was rotary-evaporated to obtain the target compound **WX048-1.**

### Step 2: synthesis of compound WX048-2

Compound **WX048-1** (0.5 g, 996.80 µmol), compound **BB-5** (0.52 g, 1.06 mmol), potassium acetate (0.4 g, 4.08 mmol) and ferrocene palladium chloride (0.15 g, 205.00 µmol) were dissolved in dioxane (10.00 mL) and water (2 mL), and the reaction solution was stirred at 105 °C for 2 hours under nitrogen atmosphere. The reaction solution was diluted with water (30mL), and then extracted with dichloromethane (30mL × 2). The organic phase was rotary-evaporated, and separated by preparative HPLC (Phenomenex Gemini C18 250 ^{∗} 50mm 10µm; mobile phase: [water (0.05% ammonium hydroxide v / v) -ACN]; B%: 22% -32%, 8min) to obtain the target compound **WX048-2.**

### Step 3: synthesis of compound WX048 and WX049

Compound **WX048-2** was resolved by supercritical fluid chromatography (separation conditions OD-3 (100mm ^{∗} 4.6mm, 3µm); mobile phase: [0.1% NH₄HCO₃ EtOH]; B%: 40% -40%, 8min) to obtain the enantiomers **WX048** and **WX049. WX048:** ¹H NMR (400 MHz, METHANOL-*d₄*) δ ppm 0.75 (d, *J* = 6.53 Hz, 3 H) 0.94 (d, *J* = 6.53 Hz, 3 H) 1.06-1.16 (m, 1 H) 1.12 (br d, *J* = 6.78 Hz, 2 H) 2.14-2.39 (m, 6 H) 2.48-2.70 (m, 3 H) 2.74-2.93 (m, 1 H) 3.71-3.94 (m, 2 H) 4.15 (br dd, *J* = 13.18, 4.64 Hz, 1 H) 7.64-7.78 (m, 2 H) 7.83 (br d, *J* = 7.53 Hz, 1 H) 7.94 (br dd, *J* = 8.41, 1.88 Hz, 1 H) 8.08 (s, 1 H) 8.26 (d, *J* = 1.76 Hz, 1 H) 8.47-8.68 (m, 1 H); **WX049**:¹H NMR (400 MHz, METHANOL- *d₄*) δ ppm 0.73 (d, *J* = 6.53 Hz, 3 H) 0.91 (d, *J* = 6.78 Hz, 3 H) 1.10 (d, *J* = 6.78 Hz, 3 H) 2.19 (s, 3 H) 2.30 (s, 3 H) 2.56 (s, 3 H) 2.71-2.90 (m, 1 H) 3.66-3.91 (m, 2 H) 4.12 (br dd, *J* = 13.30, 4.77 Hz, 1 H) 7.55-7.71 (m, 1 H) 7.55-7.71 (m, 1 H) 7.76-7.84 (m, 1 H) 7.89 (dd, *J* = 8.28, 2.01 Hz, 1 H) 8.05 (s, 1 H) 8.20 (d, *J* = 1.76 Hz, 1 H) 8.54 (d, *J* = 1.76 Hz, 1 H). The retention time is 3.471 min and 3.593 min respectively, and the ratio is 1:1.

### Example 32: WX050, WX051

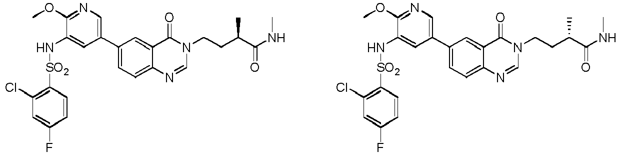

### Synthetic route:

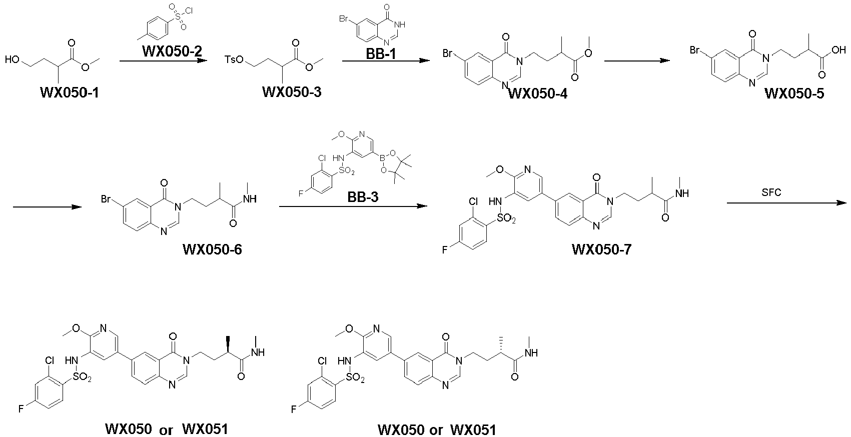

### Step 1: synthesis of compound WX050-3

**WX050-1** (1.6 g, 12.11 mmol) was dissolved in dichloromethane (20.00 mL), and then triethylamine (2.45 g, 24.21 mmol, 3.37 mL) and **WX050-2** (2.77 g, 14.53 mmol) were added. The mixed solution was stirred at 25 °C for 10 hours, and TLC (petroleum ether: ethyl acetate = 1: 1) showed that the reaction was completed. The reaction solution was rotary-evaporated and separated by silica gel column (petroleum ether: ethyl acetate = 5:1 ∼ 3:1) to obtain the target compound **WX050-3.** ¹H NMR (400MHz, CHLOROFORM-d) δ: 7.77(d, *J* = 8.4Hz, 2H), 7.33 (d, *J* = 8Hz, 2H), 4.01-4.08 (m, 1H), 3.60 (s, 3H), 2.41-2.44(m, 3H), 2.40-2.41 (m, 1H), 1.88-1.93 (m, 2H), 1.18 (d, *J* = 7.2Hz, 2H).

### Step 2: synthesis of compound WX050-4

**BB-1** (0.1 g, 444.36 µmol) was dissolved in *N*,*N'*-dimethylformamide (10.00 mL), and then cesium carbonate (217.17 mg, 666.54 µmol) and **WX050-3** (190.86 mg, 666.54 µmol, 208.66 µL) were added. The reaction solution was stirred at 60 °C for 3 hours. After the reaction was completed, water (10.00 mL) was added to the reaction solution, extracted three times with ethyl acetate (10.00 mL). The organic phase was washed three times with water (10.00 mL), washed with saturated brine (10.00 mL), dried over anhydrous sodium sulfate, and rotary-evaporated to obtain the target compound **WX050-4.**

### Step 3: synthesis of compound WX050-5

**WX050-4** (0.11 g, 324.31 µmol) was dissolved in methanol (3.00 mL) and water (1.00 mL), followed by addition of lithium hydroxide monohydrate (27.22 mg, 648.61 µmol). The reaction solution was stirred at 25 °C for 5 hours. After the reaction was completed, the reaction solution was rotary-evaporated, water (10.00 mL) was added thereto, and washed with ethyl acetate (5.00 mL). The aqueous phase was adjusted to pH=5 with hydrochloric acid (1 M), and extracted three times with ethyl acetate (5.00 mL). The organic phase was washed with saturated brine (10.00 mL), dried over anhydrous sodium sulfate, and rotary-evaporated to obtain the target compound **WX050-5.** ¹H NMR (400 MHz, CHLOROFORM-*d*) δ = 8.42 (d, *J* = 2.3 Hz, 1 H), 8.22 (s, 1 H), 7.80 (dd, *J* = 8.7, 2.1 Hz, 1 H), 7.54 (d, *J* = 8.5 Hz, 1 H), 4.12 (br t, *J* = 7.0 Hz, 2 H), 2.53-2.73 (m, 2 H), 1.66-1.82 (m, 1 H), 1.24 (br d, *J* = 7.0 Hz, 3 H).

### Step 4: synthesis of compound WX050-6

**WX050-5** (0.1 g, 307.54 µmol) was dissolved in *N*,*N'*-dimethylformamide (3.00 mL), followed by addition of methylamine hydrochloride (31.15 mg, 461.32 µmol), tetramethylurea hexafluorophosphate (175.41 mg, 461.32 µmol) and diisopropylethylamine (158.99 mg, 1.23 mmol, 214.27 µL). The reaction solution was stirred at 25 °C for 5 hours under nitrogen atmosphere. After the reaction was completed, water was added to the reaction solution (10.00 mL), extracted three times with ethyl acetate (10.00 mL). The organic phase was washed three times with water (10.00 mL), washed with saturated brine (10.00 mL), dried over anhydrous sodium sulfate, and rotary-evaporated to obtain the target compound **WX050-6.**

### Step 5: synthesis of compound WX050-7

**WX050-6** (0.09 g, 266.12 µmol) was dissolved in dioxane (5.00 mL), followed by addition of [1,1'-bis(diphenylphosphino) ferrocene] palladium dichloride (38.94 mg , 53.22 µmol), BB-3 (141.37 mg, 319.34 µmol) and potassium acetate (104.47 mg, 1.06 mmol). The reaction solution was stirred at 100 °C for 3 hours under nitrogen atmosphere. After the reaction was completed, the reaction solution was rotary-evaporated and separated by preparative HPLC (formic acid system) to obtain the target compound **WX050-7.**

### Step 6: synthesis of compound WX050 and WX051

**WX050-7** was resolved and purified by SFC (chromatographic column: AD (250mm ^{∗} 30mm, 10µm), elution condition: 0.1% NH₄HCO₃ EtOH, B%: 55% -55%; flow rate: 80mL/min) to obtain the target compound **WX050** (Rt = 0.740 min) and **WX051** (Rt = 1.656 min). **WX050:** ¹H NMR (400 MHz, CHLOROFORM-*d*) δ = 8.35 (d, *J* = 2.0 Hz, 1 H), 8.15 (d, *J* = 2.3 Hz, 1 H), 8.10-8.14 (m, 1 H), 8.08 (s, 1 H), 7.99 (d, *J* = 2.0 Hz, 1 H), 7.84-7.88 (m, 1 H), 7.78-7.82 (m, 1 H), 7.54 (s, 1 H), 7.28 (br d, *J* = 2.5 Hz, 1 H), 7.08-7.15 (m, 1 H), 6.11 (br s, 1 H), 4.12-4.32 (m, 1 H), 3.98-4.00 (m, 3 H), 3.95 (s, 1 H), 2.86 (d, *J* = 4.8 Hz, 3 H), 2.28-2.39 (m, 1 H), 2.13-2.26 (m, 1 H), 1.89 (br dd, *J* = 13.7, 3.9 Hz, 1 H), 1.21 (d, *J* = 6.8 Hz, 3 H). **WX051**:¹H NMR (400 MHz, CHLOROFORM-*d*) δ = 8.35 (d, *J* = 1.8 Hz, 1 H), 8.15 (d, *J* = 2.3 Hz, 1 H), 8.10-8.14 (m, 1 H), 8.08 (s, 1 H), 7.99 (d, *J* = 2.0 Hz, 1 H), 7.84-7.89 (m, 1 H), 7.77-7.83 (m, 1 H), 7.54 (s, 1 H), 7.28 (d, *J* = 2.5 Hz, 1 H), 7.07-7.18 (m, 1 H), 6.11 (br s, 1 H), 4.16-4.28 (m, 1 H), 3.98-4.00 (m, 3 H), 3.95 (s, 1 H), 2.86 (d, *J* = 4.8 Hz, 3 H), 2.28-2.38 (m, 1 H), 2.11-2.26 (m, 1 H), 1.89 (br dd, *J* = 13.6, 4.5 Hz, 1 H), 1.21 (d, *J* = 7.0 Hz, 3 H).

### Example 33: WX052, WX053

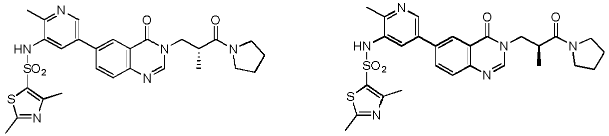

### Synthetic route:

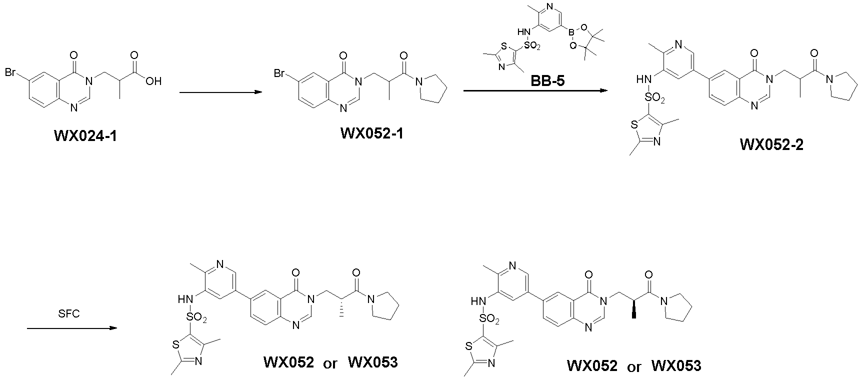

### Step 1: synthesis of compound WX052-1

Compound **WX024-1** (0.5 g, 1.53 mmol), tetrahydropyrrole (127.80 mg, 1.80 mmol, 150.00 µL), tetramethylurea hexafluorophosphate (600.00 mg, 1.58 mmol) and triethylamine (727.00 mg, 7.18 mmol, 1.00 mL) were dissolved in dichloromethane (20 mL) and stirred at 50 °C for 16 hours. After the reaction was completed, the reaction solution was diluted with water (30 mL) and extracted with dichloromethane (30 mL × 2). The organic phase was rotary-evaporated to obtain the target compound **WX052-1.**

### Step 2: synthesis of compound WX052-2

Compound **WX052-1** (0.9 g, 1.29 mmol), compound **BB-5** (702.13 mg, 1.37 mmol), potassium acetate (0.51 g, 5.19 mmol) and ferrocene palladium chloride (0.19 g, 259.46 µmol) were dissolved in dioxane (25 mL) and water (5 mL), and the reaction solution was stirred at 105 °C for 2 hours under nitrogen atmosphere. After the reaction was completed, the reaction solution was diluted with water (30 mL), and then extracted with dichloromethane (30 mL × 2). The organic phase was rotary-evaporated and separated by preparative HPLC (Phenomenex Gemini C18 250 ^{∗} 50 10u; 0.05% ammonium hydroxide v/v) -ACN]; B%: 17% -27%, 8min) to obtain the target compound **WX052-2**.

### Step 3: synthesis of compound WX052 and WX053

Compound **WX052-2** was resolved by supercritical fluid chromatography (separation conditions column: Phenomenex Gemini C18 250 ^{∗} 50mm10µ; mobile phase: [water (0.05% ammonium hydroxide v / v) -ACN]; B%: 17% -27%, 8min) to obtain the enantiomers **WX052** and **WX053,** the retention time of which is 4.252 min, 4.909 min, respectively, and the ratio is 1:1. **WX052:** ¹H NMR (400 MHz, METHANOL-d4) δ : 1.13 (d, *J* = 7.03 Hz, 3 H) 1.56-1.84 (m, 4 H) 2.21 (s, 3 H) 2.32 (s, 3 H) 2.58 (s, 3 H) 3.21-3.44 (m, 5 H) 3.95-4.13 (m, 2 H) 7.61-7.74 (m, 2 H) 7.93 (br d, *J* = 8.53 Hz, 1 H) 8.11-8.24 (m, 2 H) 8.57 (s, 1 H); **WX053**:¹H NMR (400 MHz, METHANOL-d4) δ : 1.12 (d, *J* = 6.78 Hz, 3 H) 1.58-1.83 (m, 4 H) 2.22 (s, 3 H) 2.32 (s, 3 H) 2.58 (s, 3 H) 3.22-3.43 (m, 5 H) 3.94-4.14 (m, 2 H) 7.62-7.76 (m, 2 H) 7.92 (dd, *J* = 8.53, 1.76 Hz, 1 H) 8.13-8.25 (m, 2 H) 8.56 (s, 1 H).

### Example 34: WX054, WX055

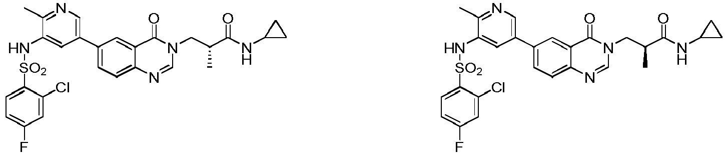

### Synthetic route:

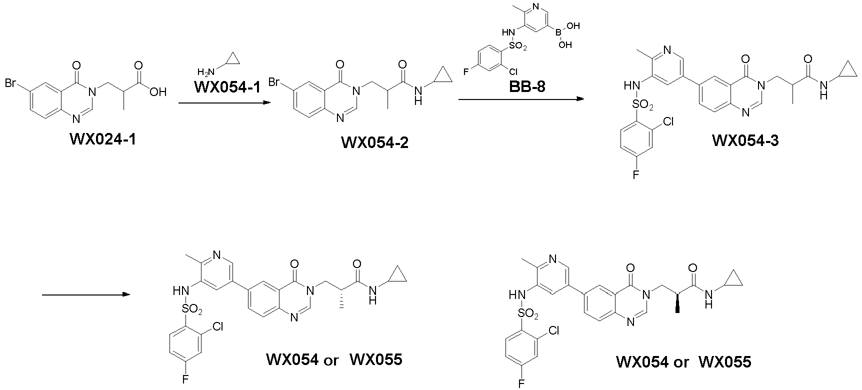

### Step 1: synthesis of compound WX054-2

Compound **WX024-1** (0.2 g, 642.82 µmol), **WX054-1** (82.40 mg, 1.44 mmol, 0.1 mL), carbodiimide (0.123 g, 641.62 µmol), 2-hydroxypyridine *N*-oxide (0.084 g, 756.08 µmol) and triethylamine (260.27 mg, 2.57 mmol, 0.358 mL) were dissolved in dichloromethane (10.00 mL) and stirred at 50 °C for 16 hours. After the reaction was completed, the reaction solution was rotary-evaporated, diluted with water (50 mL), and extracted with dichloromethane (50 mL). The organic phase was rotary-evaporated to obtain the target compound **WX054-2.**

### Step 2: synthesis of compound WX054-3

Compound **WX054-2** (0.2 g, 547.04 µmol), compound **BB-8** (0.188 g, 545.63 µmol), potassium acetate (0.215 g, 2.19 mmol), ferrocene palladium chloride (0.04 g, 54.67 µmol) were dissolved in dioxane (5.00 mL) and water (1 mL), and the reaction solution was stirred at 100 °C for 1 hour under nitrogen atmosphere. After the reaction was completed, the reaction solution was rotary-evaporated, diluted with water (50 mL), extracted with dichloromethane (50 mL), and the organic phase was rotary-evaporated. TLC (dichloromethane: methanol = 10:1) showed that a new spot was formed. The residue was subjected to column chromatography (dichloromethane: methanol = 1: 0-10: 1) to obtain the target compound **WX054-3.**

### Step 3: synthesis of compound WX054 and WX055

Compound **WX054-3** was resolved by supercritical fluid chromatography (separation conditions [chromatographic column: OD (250mm ^{∗} 30mm, 5µm); mobile phase: [0.1% NH₄HCO₃ ETOH]; B%: 35%-35%]) to obtain the enantiomers **WX054** and **WX055,** the retention time of which is 4.179 min and 4.465 min, respectively, and the ratio is 1:1. **WX054:** ¹H NMR (400 MHz, METHANOL-*d₄*) δ ppm 8.36 (d, *J* = 2.0 Hz, 1 H), 8.10 (d, *J* = 2.0 Hz, 1 H), 7.98 (s, 1 H), 7.75-7.87 (m, 2 H), 7.67 (d, *J* = 2.0 Hz, 1 H), 7.56 (d, *J* = 8.0 Hz, 1 H), 7.31 (dd, *J* = 8.0, 2.5 Hz, 1 H), 6.97-7.09 (m, 1 H), 4.02 (dd, *J* = 13.3, 4.8 Hz, 1 H), 3.80 (dd, *J* = 13.6, 10.0 Hz, 1 H), 2.64-2.79 (m, 1 H), 2.33 (tt, *J* = 7.3, 3.7 Hz, 1 H), 2.26 (s, 3 H), 1.01 (d, *J* = 7.0 Hz, 3 H), 0.35-0.44 (m, 2 H), -0.03-0.09 (m, 2 H); **WX055:** ¹H NMR (400 MHz, METHANOL-*d₄*) δ ppm 8.37 (d, *J* = 2.0 Hz, 1 H), 8.10 (d, *J* = 2.5 Hz, 1 H), 7.98 (s, 1 H), 7.76-7.88 (m, 2 H), 7.67 (d, *J* = 2.0 Hz, 1 H), 7.56 (d, *J* = 8.5 Hz, 1 H), 7.31 (dd, *J* = 8.5, 2.5 Hz, 1 H), 6.98-7.07 (m, 1 H), 4.02 (dd, *J* = 13.6, 4.5 Hz, 1 H), 3.80 (dd, *J* = 13.6, 10.0 Hz, 1 H), 2.68-2.78 (m, 1 H), 2.29-2.42 (m, 1 H), 2.26 (s, 3 H), 1.01 (d, *J* = 7.0 Hz, 3 H), 0.39 (dd, *J* = 7.5, 2.0 Hz, 2 H), -0.04-0.10 (m, 2 H).

### Example 35: WX056, WX057

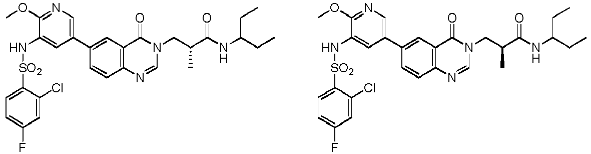

### Synthetic route:

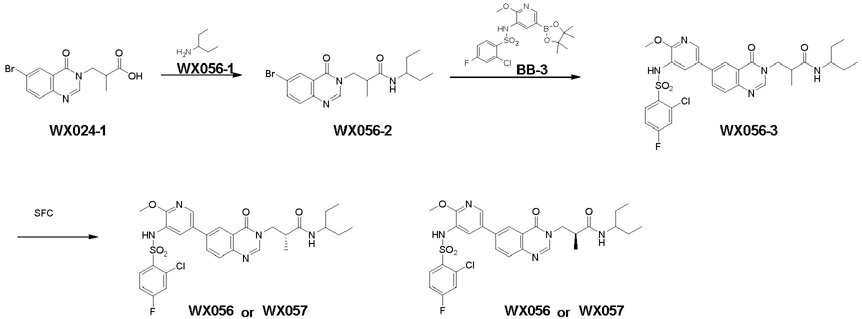

### Step 1: synthesis of compound WX056-2

In a pre-dried 40 mL flask, **WX056-1** (168.09 mg, 1.93 mmol, 224.72 µL) and **WX024-1** (200 mg, 642.82 µmol) were dissolved with DCM (2 mL), followed by addition of *p*-(7-azobenzotriazole)-*N*,*N*,*N*,*N*-tetramethylurea hexafluorophosphate (366.63 mg, 964.22 µmol) and diisopropylethylamine (166.16 mg, 1.29 mmol, 223.93 µL). The reaction solution was stirred at 20 °C for 16 hours. After the reaction was completed, 10 mL of water and 10 mL of ethyl acetate were added to the reaction system for dilution. The organic phase was collected after liquid separation, and the aqueous phase was extracted with ethyl acetate (5 mL × 3). The organic phases were combined, washed with saturated brine (20 mL), dried over anhydrous sodium sulfate, concentrated under reduced pressure, and then purified by thin layer chromatography silica gel plate (dichloromethane: methanol = 20: 1) to obtain **WX056-2.**

### Step 2: synthesis of compound WX056-3

In a pre-dried 40mL reaction flask, **WX056-2** (209.54 mg, 473.34 µmol) and **BB-3** (180 mg, 473.34 µmol) were added, followed by addition of the solvents 1,4-dioxane (3 mL) and H₂O (0.3 mL) for dissolution. Potassium acetate (139.36 mg, 1.42 mmol) was then added thereto, and the mixture was replaced with nitrogen. [1,1'-bis(diphenylphosphino) ferrocene] palladium dichloride (34.63 mg, 47.33 µmol) was added thereto, and the mixture was replaced with nitrogen. The reaction solution was stirred at 90 °C for 16 hours. After the reaction was completed, 10 mL of water and 10 mL of ethyl acetate were added to the reaction system for dilution. The organic phase was collected after liquid separation, and the aqueous phase was extracted with ethyl acetate (5 mL × 3). The organic phases were combined, washed with saturated brine (20 mL), dried over anhydrous sodium sulfate, concentrated under reduced pressure, and purified by preparative HPLC (chromatographic column: Luna C18 100 ^{∗} 30mm 5µm; mobile phase: [water (0.1% TFA) -ACN]; B%: 40% -65%, 10min) to obtain the target compound **WX056-3.**

### Step 3: synthesis of compound WX056 and WX057

**WX056-3** was purified by supercritical fluid chromatography (neutral) (chromatographic column: Chiralpak AD-H 250 ^{∗} 30mm id 5µm; mobile phase: A: CO₂, B: IPA(0.1% NH₄HCO₃); gradient: B% = 45 %; Flow rate: 70 g / min; wavelength: 220 nm; column temperature: 40 °C; back pressure: 100 bar) to obtain the enantiomers **WX056** and **WX057. WX056:** ¹H NMR (400MHz, CHLOROFORM-d) δ = 8.32 (s, 1H), 8.21-8.08 (m, 3H), 7.98 (d, *J* = 2.0 Hz, 1H), 7.91-7.80 (m, 1H), 7.80-7.73 (m, 1H), 7.51 (br s, 1H), 7.18-7.06 (m, 1H), 5.17 (br d, *J* = 9.3 Hz, 1H), 4.20 (dd, *J* = 4.7, 13.1 Hz, 1H), 4.08-3.99 (m, 1H), 3.98 (s, 3H), 3.72 (br d, *J* = 8.6 Hz, 1H), 3.02-2.91 (m, 1H), 1.49 (br dd, *J* = 6.2, 13.7 Hz, 1H), 1.38-1.24 (m, 5H), 1.15 (tt, *J* = 7.4, 14.5 Hz, 1H), 0.85 (t, *J* = 7.4 Hz, 3H), 0.51 (t, *J* = 7.4 Hz, 3H), m/z = 616.2 [M+1], the retention time of which is 3.05min. **WX057:** ¹H NMR (400MHz, CHLOROFORM-d) δ = 8.32 (s, 1H), 8.21-8.08 (m, 3H), 7.98 (d, *J=* 2.0 Hz, 1H), 7.91-7.80 (m, 1H), 7.80-7.73 (m, 1H), 7.51 (br s, 1H), 7.18-7.06 (m, 1H), 5.17 (br d, *J* = 9.3 Hz, 1H), 4.20 (dd, *J* = 4.7, 13.1 Hz, 1H), 4.08-3.99 (m, 1H), 3.98 (s, 3H), 3.72 (br d, *J* = 8.6 Hz, 1H), 3.02-2.91 (m, 1H), 1.49 (br dd, *J* = 6.2, 13.7 Hz, 1H), 1.38-1.24 (m, 5H), 1.15 (tt, *J* = 7.4, 14.5 Hz, 1H), 0.85 (t, *J* = 7.4 Hz, 3H), 0.51 (t, *J* = 7.4 Hz, 3H), m/z = 616.2 [M+l], the retention time of which is 3.71min. The ratio of **WX056** and **WX057** is about 1:1.

### Example 36: WX058, WX059

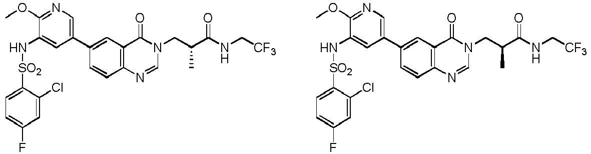

### Synthetic route:

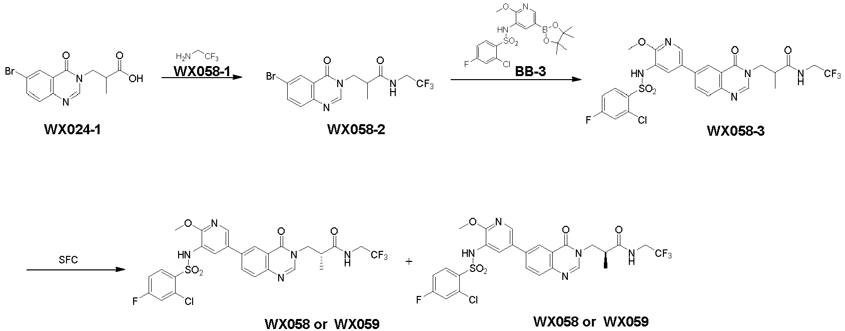

### Step 1: synthesis of compound WX058-2

**WX024-1** (0.25 g, 803.52 µmol) and **WX058-1** (238.78 mg, 2.41 mmol, 189.51 µL) were added to a pre-dried 40 mL flask, and dissolved with dichloromethane (3 mL), followed by addition of 2-(7-azobenzotriazole)-*N*,*N*,*N*,*N*-tetramethylurea hexafluorophosphate (458.28 mg, 1.21 mmol) and diisopropylethylamine (207.69 mg, 1.61 mmol, 279.91 µL). The reaction solution was stirred at 20 °C for 16 hours. After the reaction was completed, 10 mL of water and 10 mL of ethyl acetate were added to the reaction system for dilution. The organic phase was collected after liquid separation, and the aqueous phase was extracted with ethyl acetate (5 mL × 3). The organic phases were combined, washed with saturated brine (20 mL), dried over anhydrous sodium sulfate, and concentrated under reduced pressure to obtain a residue. The residue was purified by thin layer chromatography silica gel plate (dichloromethane: methanol = 20:1) to obtain the target compound **WX058-2,** which was directly used in the next step.

### Step 2: synthesis of compound WX058-3

**WX058-2** (259.63 mg, 586.48 µmol) and **BB-3** (230 mg, 586.48 µmol) were added into a pre-dried 40mL reaction flask, and dissolved with the solvent 1,4-dioxane (3 mL) and water (0.3 mL). Potassium acetate (172.67 mg, 1.76 mmol) was then added thereto and the mixture was replaced with the nitrogen. [1,1'-bis(diphenylphosphino) ferrocene] palladium dichloride (42.91 mg, 58.65 µmol) was then added thereto and the mixture was replaced with the nitrogen. The reaction solution was stirred at 90 °C for 16 hours. After the reaction was completed, 10 mL of water and 10 mL of ethyl acetate were added to the reaction system for dilution. The organic phase was collected after liquid separation, and the aqueous phase was extracted with ethyl acetate (5 mL × 3). The organic phases were combined, washed with saturated brine (20 mL), dried over anhydrous sodium sulfate, concentrated under reduced pressure, and then purified by preparative HPLC (method: chromatographic column: Luna C18 100 ^{∗} 30mm 5µm; mobile phase: [water (0.1% TFA) -ACN]; B%: 45% -65%, 10 min) to obtain the target compound **WX058-3**.

### Step 3: synthesis of compound WX058 and WX059

After mechanical separation, **WX058-3** was resolved by supercritical fluid chromatography (neutral) (method: chromatographic column: AD (250mm ^{∗} 30mm, 5µm); mobile phase: [0.1% NH₄HCO₃ EtOH]; B%: 35%-35%) to obtain the enantiomers **WX058** (retention time 2.99min) and **WX059** (retention time 3.27min). **WX058:** ¹H NMR (400MHz, CHLOROFORM-d) δ = 8.31 (s, 1H), 8.17-8.10 (m, 2H), 8.08 (s, 1H), 7.98 (d, *J* = 1.8 Hz, 1H), 7.89-7.82 (m, 1H), 7.81-7.75 (m, 1H), 7.53 (br s, 1H), 7.16-7.07 (m, 1H), 5.92 (br t, *J* = 6.5 Hz, 1H), 4.23-4.13 (m, 1H), 4.11-4.03 (m, 1H), 3.98 (s, 3H), 3.91 (br dd, *J* = 8.5, 15.5 Hz, 1H), 3.85-3.73 (m, 1H), 3.15-3.03 (m, 1H), 1.33 (d, *J* = 6.8 Hz, 3H).MS, m/z = 628.1 [M+1]. **WX059:** ¹H NMR (400MHz, CHLOROFORM-d) δ = 8.31 (s, 1H), 8.17-8.10 (m, 2H), 8.08 (s, 1H), 7.98 (d, *J* = 1.8 Hz, 1H), 7.89-7.82 (m, 1H), 7.81-7.75 (m, 1H), 7.53 (br s, 1H), 7.16-7.07 (m, 1H), 5.92 (br t, *J* = 6.5 Hz, 1H), 4.23-4.13 (m, 1H), 4.11-4.03 (m, 1H), 3.98 (s, 3H), 3.91 (br dd, *J* = 8.5, 15.5 Hz, 1H), 3.85-3.73 (m, 1H), 3.15-3.03 (m, 1H), 1.33 (d, *J* = 6.8 Hz, 3H). MS, m/z = 628.1 [M+1].

### Example 37: WX060, WX061

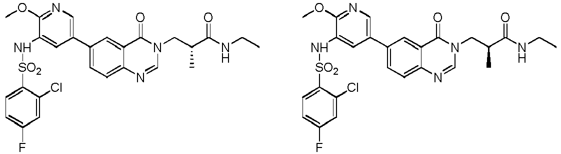

### Synthetic route:

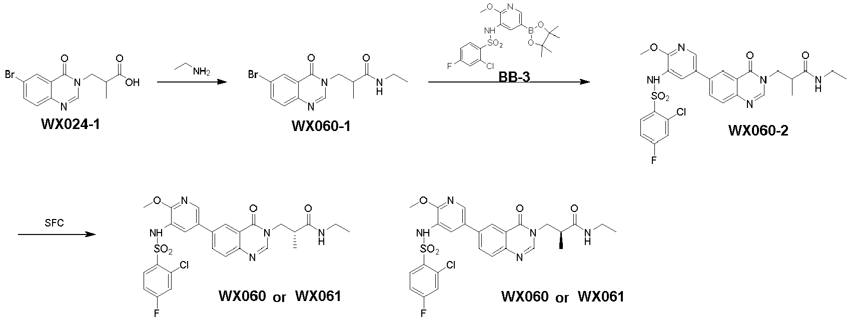

### Step 1: synthesis of compound WX060-1

Raw materials **WX024-1** (250 mg, 803.52 µmol) and 2-(7-azobenzotriazole)-*N*,*N*,*N*,*N*-tetramethylurea hexafluorophosphate (458.28 mg, 1.21 mmol) were added into a pre-dried 40 mL reaction flask and dissolved with the solvent dichloromethane (3 mL) The raw material ethylamine (108.68 mg, 2.41 mmol, 157.73 µL) was then added, followed by addition of *N*,*N*-diisopropylethylamine (207.70 mg, 1.61 mmol, 279.92 µL), and stirred at 80 °C for 12 hours. TLC detection (dichloromethane: methanol = 10:1) showed that the raw materials disappeared and new products were formed. The reaction was quenched with 10 mL of water, extracted with dichloromethane (100 mL × 3). The organic phases were combined, dried over anhydrous sodium sulfate, concentrated under reduced pressure using a water pump at 50 °C and purified by preparation TLC (dichloromethane: methanol = 15: 1) to obtain the target compound **WX060-1.** ¹H NMR (400MHz, CHLOROFORM-d) δ = 8.41 (d, *J* = 2.3 Hz, 1H), 8.10 (s, 1H), 7.84 (dd, *J* = 2.3, 8.6 Hz, 1H), 7.59 (d, *J* = 8.7 Hz, 1H), 4.16 (dd, *J* = 4.9, 13.2 Hz, 1H), 3.99 (dd, *J* = 9.5, 13.2 Hz, 1H), 3.30-3.26 (m, 1H), 3.22-3.17 (m, 2H), 3.01 (s, 3H), 2.81 (s, 4H).

### Step 2: synthesis of compound WX060-2

Raw materials **WX060-1** (250 mg, 739.21 µmol), **BB-3** (327.25 mg, 739.21 µmol) and potassium acetate (217.64 mg, 2.22 mmol) were added to a pre-dried 40 mL reaction flask, and dissolved with the solvent 1,4-dioxane (3 mL) and water (0.3 mL), and replaced with nitrogen. 1,1 -bis(diphenylphosphine) ferrocene palladium chloride (54.09 mg, 73.92 µmol) was then added thereto, and the mixture was replaced with nitrogen, and further stirred at 80 °C for 3 hours. After the reaction was completed, the reaction was quenched with 10 mL of water, and extracted with dichloromethane (10 mL × 3). The organic phases were combined, dried over anhydrous sodium sulfate, and concentrated under reduced pressure using a water pump at 50 °C to obtain the target compound **WX060-2.**

### Step 3: synthesis of compound WX060 and WX061

**WX060-2** was resolved by SFC (chromatographic column: AD (250mm ^{∗} 30mm, 5µµ); mobile phase: [A is CO₂, B is IPA (0.1% NH₄HCO₃)]; B%: 45%-45% flow rate: 70 g/min: wavelength: 220 nm; column temperature: 40 °C; system back pressure: 100 bar) to obtain the enantiomers **WX060** (retention time is 3.08 min) and **WX061** (retention time is 3.31min). **WX060:** ¹H NMR (400MHz, METHANOL-*d₄*) δ = 7.78 (s, 1H), 7.64 (d, *J* = 2.0 Hz, 1H), 7.62 (s, 1H), 7.54 (dd, *J* = 6.0, 8.8 Hz, 1H), 7.47-7.41 (m, 2H), 7.20 (d, *J* = 8.4 Hz, 1H), 6.90 (dd, *J* = 2.3, 8.5 Hz, 1H), 6.68 (br t, *J* = 8.5 Hz, 1H), 3.69 (dd, *J* = 4.9, 13.5 Hz, 1H), 3.45 (dd, *J* = 9.9, 13.2 Hz, 1H), 3.31 (s, 3H), 2.60-2.47 (m, 2H), 2.42 (s, 1H), 0.68 (d, *J* = 7.1 Hz, 3H), 0.37 (t, *J* = 7.3 Hz, 3H); **WX061:** ¹H NMR (400MHz, METHANOL-*d₄*) δ = 8.33 (s, 1H), 8.21 (s, 1H), 8.18 (s, 1H), 8.10 (dd, *J* = 6.0, 8.8 Hz, 1H), 8.03-7.98 (m, 2H), 7.76 (d, *J* = 8.4 Hz, 1H), 7.46 (dd, *J* = 2.4, 8.4 Hz, 1H), 7.26-7.21 (m, 1H), 4.29-4.23 (m, 1H), 4.01 (dd, *J* = 10.0, 13.3 Hz, 1H), 3.87 (s, 3H), 3.16-3.03 (m, 3H), 2.98 (s, 2H), 1.24 (d, *J* = 6.8 Hz, 3H), 0.93 (t, *J* = 7.3 Hz, 3H).

### Example 38: WX062, WX063

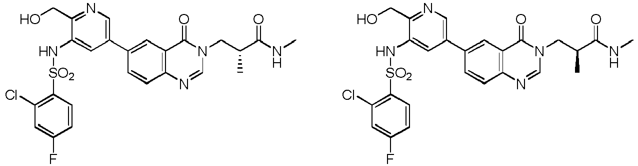

### Synthetic route:

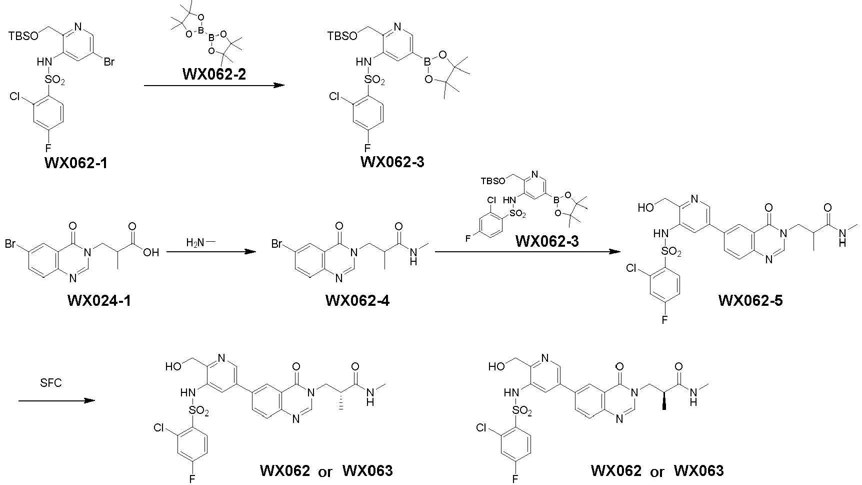

### Step 1: synthesis of compound WX062-4

Raw materials **WX024-1** (5 g, 16.07 mmol) and methylamine (598.92 mg, 19.28 mmol) and the solvent dichloromethane (50 mL) were added to a pre-dried 500 mL single-necked flask, followed by addition of 2,4,6-tripropyl-1,3,5,2,4,6-trioxytriphosphate-2,4,6-trioxide (12.27 g, 19.28 mmol, 11.47 mL, 50% purity) and *N*,*N*-diisopropylethylamine (6.23 g, 48.21 mmol, 8.40 mL), and further stirred at 25 °C for 12 hours. TLC detection (dichloromethane: methanol = 10:1) showed the completion of the reaction. The reaction was quenched by adding water (50 mL) to the reaction system, followed by extraction with dichloromethane (50 mL × 3). The organic phases were combined, dried over with anhydrous sodium sulfate, rotary-evaporated by a water pump at 45 °C, dried under reduced pressure, and separated by column chromatography (petroleum ether: ethyl acetate = 1: 0 to 0: 1) to obtain target compound **WX062-4.** ¹H NMR (400 MHz, CHLOROFORM-d) δ = 8.40 (br s, 1H), 8.11 (s, 1H), 7.84 (br d, *J* = 8.8 Hz, 1H), 7.80-7.76 (m, 1H), 7.80-7.76 (m, 1H), 7.60 (d, *J* = 8.5 Hz, 1H), 4.20-4.10 (m, 2H), 4.02 (dd, *J* = 9.5, 13.2 Hz, 2H), 2.95-2.89 (m, 1H), 2.74 (d, *J* = 4.8 Hz, 4H), 2.73-2.72 (m, 1H), 1.28 (d, *J* = 7.0 Hz, 4H).

### Step 2: synthesis of compound WX062-3

Raw materials **WX062-1** (525 mg, 1.03 mmol) and **WX062-2** (261.46 mg, 1.03 mmol) were added into a pre-dried 15mL reaction flask, and dissolved with the solvent 1,4-dioxane (5 mL). Potassium acetate (202.10 mg, 2.06 mmol) was then added thereto and the mixture was replaced with nitrogen. 1,1-bis(diphenylphosphine) ferrocene palladium chloride (75.34 mg, 102.96 µmol) was then added thereto and the mixture was replaced with nitrogen, and further stirred at 110 °C for 3 hours. After the reaction was completed, the target compound **WX062-3** was obtained without posttreatment, which was directly used in the next reaction.

### Step 3: synthesis of compound WX062-5

Raw materials **WX062-4** (300 mg, 925.43 µmol) and **WX062-3** (566.97 mg, 1.02 mmol) were added into a pre-dried 15mL reaction flask, and dissolved with the solvent 1,4-dioxane (3 mL) and water (0.3 mL). Potassium acetate (181.65 mg, 1.85 mmol) was then added thereto and the mixture was replaced with nitrogen. 1,1-bis(diphenylphosphine) ferrocene palladium chloride (67.71 mg, 92.54 µmol) was then added thereto and the mixture was replaced with nitrogen, and further stirred at 90 °C for 12 hours. TLC detection (dichloromethane: methanol = 10:1) showed the completion of the reaction. Water (10 mL) was added to the reaction system, followed by extraction with dichloromethane (10 mL × 3). The resulting organic phase was dried over anhydrous sodium sulfate, rotary-evaporated under reduced pressure by a water pump, and then purified by preparative TLC (dichloromethane: methanol = 15:1) to obtain target compound **WX062-5.**

### Step 3: synthesis of compound WX062 and WX063

**WX062-5** was resolved by SFC (chromatographic column: ChiralcelOD-H 250 ^{∗} 30mm id 5µm; mobile phase: A: CO₂, B: MeOH (0.1% NH₄HCO₃); gradient: B% = 45%; flow rate: 70 g / min; wavelength: 220 nm; column temperature: 40 °C; back pressure: 100 bar) to obtain the enantiomers **WX062** (retention time is 1.460 min) and **WX063** (retention time is 1.453 min). **WX062:** ¹H NMR (400MHz, CHLOROFORM-d) δ = 8.44 (s, 1H), 8.25 (s, 1H), 8.19-8.14 (m, 2H), 7.98 (s, 1H), 7.82-7.74 (m, 2H), 7.30 (dd, *J* = 2.3, 8.0 Hz, 1H), 7.21-7.14 (m, 1H), 5.80 (br d, *J* = 4.4 Hz, 1H), 4.91 (br s, 2H), 4.20 (dd, *J* = 4.6, 13.2 Hz, 1H), 4.05-4.05 (m, 1H), 4.06 (dd, *J* = 9.6, 13.4 Hz, 1H), 2.98 (br d, *J* = 9.3 Hz, 1H), 2.75 (d, *J* = 4.8 Hz, 3H), 1.65 (br s, 1H), 1.31 (d, *J* = 6.9 Hz, 3H); **WX063:** ¹H NMR (400MHz, CHLOROFORM-d) δ = 8.44 (br s, 1H), 8.26 (br s, 1H), 8.20-8.13 (m, 2H), 7.98 (s, 1H), 7.83-7.74 (m, 2H), 7.30 (br d, *J* = 8.3 Hz, 1H), 7.17 (brt, *J* = 7.2 Hz, 1H), 5.79-5.79 (m, 1H), 4.91 (br s, 2H), 4.23-4.15 (m, 1H), 4.11-4.10 (m, 1H), 4.06-4.06 (m, 1H), 4.11-3.99 (m, 1H), 2.98 (br d, *J* = 8.8 Hz, 1H), 2.75 (br d, *J* = 4.5 Hz, 3H), 1.31 (br d, *J* = 6.8 Hz, 3H).

### Example 39: WX064, WX065

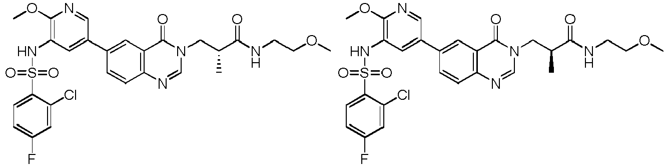

### Synthetic route:

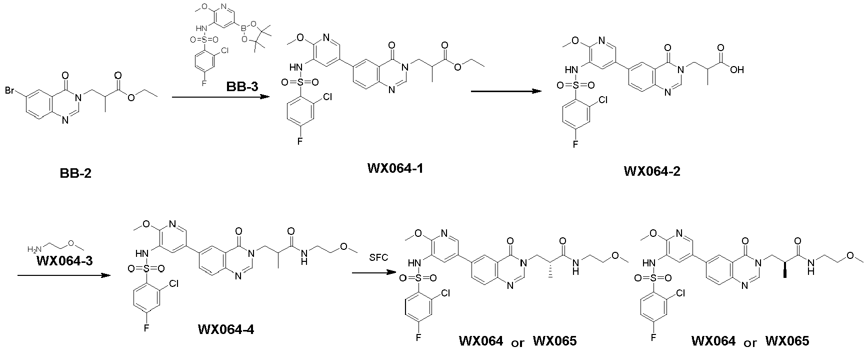

### Step 1: synthesis of compound WX064-1

**BB-2** (1.5 g, 4.42 mmol), **BB-3** (2.35 g, 5.31 mmol) and potassium carbonate (1.30 g, 13.27 mmol) were added into a pre-dried 100 mL single-necked flask, followed by addition of 1,4-dioxane (15 mL) and water (2 mL) for dissolution, and the mixture was replaced with nitrogen. [1,1'-bis(diphenylphosphino) ferrocene] palladium dichloride (323.59 mg, 442.24 µmol) was finally added thereto and the mixture was replaced with nitrogen. The reaction solution was stirred at 90 °C for 16 hours. After the reaction was completed, 10 mL of water and 10 mL of ethyl acetate were added to the reaction system for dilution. The organic phase was collected after liquid separation, and the aqueous phase was extracted with ethyl acetate (5 mL × 3). The organic phases were combined, washed with saturated brine (20 mL), dried over anhydrous sodium sulfate, concentrated under reduced pressure, and separated and purified by column chromatography (petroleum ether: ethyl acetate = 30: 1, 1: 1) to obtain **WX064-1.** ¹H NMR (400MHz, CHLOROFORM-d) δ = 8.26 (d, *J* = 2.0 Hz, 1H), 8.11-8.01 (m, 3H), 7.90 (d, *J* = 2.3 Hz, 1H), 7.81-7.74 (m, 1H), 7.73-7.67 (m, 1H), 7.47 (s, 1H), 7.20-7.15 (m, 1H), 7.10-7.00 (m, 1H), 4.17-3.95 (m, 4H), 3.91 (s, 3H), 3.19-3.03 (m, 1H), 1.25 (d, *J* = 7.3 Hz, 3H), 1.13 (t, *J* = 7.2 Hz, 3H).

### Step 2: synthesis of compound WX064-2

**WX064-1** (1.30 g, 2.26 mmol) was added into a pre-dried 40mL reaction flask, followed by addition of tetrahydrofuran (10 mL) for dissolution, and addition of a solution of lithium monohydrate monohydrate (271.30 mg, 6.78 mmol) in water (10 mL). The reaction solution was stirred at 0 °C for 1 hour. After the reaction was completed, 10 mL of water and 10 mL of ethyl acetate were added to the reaction system for dilution. The aqueous phase was collected after liquid separation. The aqueous phase was adjusted to pH = 3 with IN hydrochloric acid and extracted with ethyl acetate (5 mL × 3). The organic phases were combined, washed with saturated brine (20 mL), dried over anhydrous sodium sulfate, and concentrated under reduced pressure to obtain WX064-2. ¹H NMR (400MHz, METHANOL-d4) δ = 8.31 (s, 2H), 8.21 (d, *J =* 2.0 Hz, 1H), 8.08 (dd, *J* = 6.0, 8.8 Hz, 1H), 8.02-7.96 (m, 2H), 7.75 (d, *J* = 8.6 Hz, 1H), 7.44 (br d, *J* = 8.6 Hz, 1H), 7.21 (br t, *J* = 8.6 Hz, 1H), 4.21-4.15 (m, 2H), 3.85 (s, 3H), 3.11 (br d, *J* = 6.4 Hz, 1H), 1.27 (d, *J* = 7.3 Hz, 3H).

### Step 3: synthesis of compound WX064-4

Raw materials **WX064-2** (146 mg, 266.93 µmol) and **WX064-3** (24.06 mg, 320.32 µmol, 27.85 µL) and the solvent dichloromethane (2 mL) were added into a pre-dried reaction flask, followed by addtion of 2,4,6-tripropyl-1,3,5,2,4,6-trioxytriphosphate-2,4,6-trioxide (203.84 mg, 320.32 µmol, 190.50 µL, 50% purity). The system was cooled to 0 °C, and N,N-diisopropylethylamine (103.50 mg, 800.80 µmol, 139.48 µL) was slowly added thereto. The reaction solution was stirred at 25 °C for 12 hours. TLC detection (dichloromethane: methanol = 10:1) showed that the reaction was completed. Water (10 mL) was added to the reaction solution, followed by extraction with dichloromethane (10 mL × 3). The obtained organic phases were dried over anhydrous sodium sulfate, rotary-evaporated under reduced pressure by a water pump and purified by preparative TLC (dichloromethane: methanol = 15:1) to obtain the target compound **WX064-4.**

### Step 4: synthesis of compound WX064 and WX065

**WX064-4** was resolved by SFC (chromatographic column: Chiralcel OJ-H 250 ^{∗} 30mm id 5µm; mobile phase: A: CO₂, B: MeOH; gradient: B% = 30%; flow rate: 60 g / min; wavelength: 220 nm; column temperature: 40 °C; back pressure: 100 bar) to obtain the enantiomers **WX064** (retention time is 1.846 min) and **WX065** (retention time is 2.85 min). **WX064:** ¹H NMR (400MHz, CHLOROFORM-d) δ = 8.31 (s, 1H), 8.15-8.15 (m, 1H), 8.15-8.11 (m, 2H), 8.10 (s, 1H), 7.97 (d, *J* = 2.0 Hz, 1H), 7.85-7.79 (m, 1H), 7.79-7.74 (m, 1H), 7.25 (d, *J* = 2.2 Hz, 1H), 7.15-7.07 (m, 1H), 5.99 (br s, 1H), 4.27-4.15 (m, 1H), 4.04-3.99 (m, 1H), 3.97 (s, 3H), 3.46-3.37 (m, 1H), 3.37-3.27 (m, 2H), 3.26-3.19 (m, 1H), 3.12 (s, 3H), 3.02-2.93 (m, 1H), 1.33-1.33 (m, 1H), 1.29 (d, *J* = 7.1 Hz, 2H), 1.30-1.22 (m, 1H); **WX065:** ¹H NMR (400MHz, CHLOROFORM-d) δ = 8.34 (br s, 1H), 8.12 (br d, *J* = 13.2 Hz, 3H), 7.99 (s, 1H), 7.87-7.81 (m, 1H), 7.81-7.74 (m, 1H), 7.13 (br s, 1H), 5.87 (br s, 1H), 4.27-4.17 (m, 1H), 4.04 (s, 1H), 3.99 (s, 3H), 3.40 (br s, 1H), 3.35 (br s, 2H), 3.24 (br d, *J* = 9.3 Hz, 1H), 3.13 (s, 3H), 2.98 (br s, 1H), 1.30 (br d, *J* = 6.8 Hz, 3H), 1.27-1.23 (m, 1H), 1.15 (br s, 1H).

### Example 40: WX066, WX067

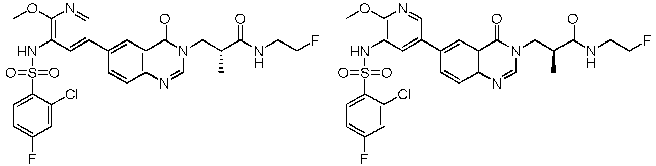

### Synthetic route:

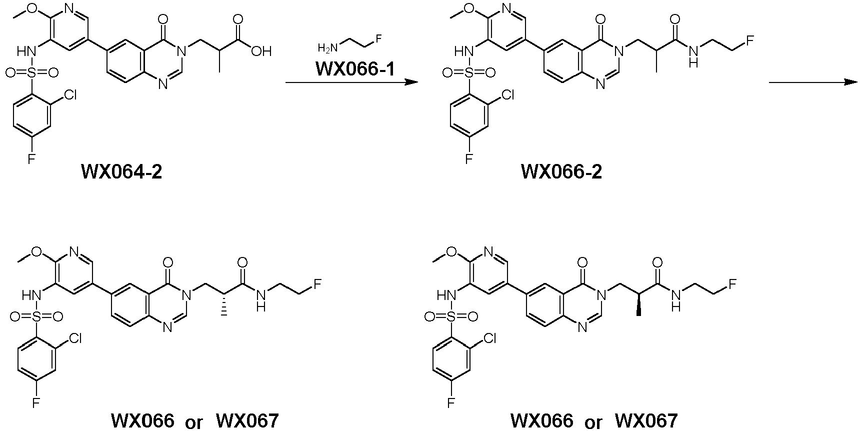

### Step 1: synthesis of compound WX066-2

Raw materials **WX064-2** (120 mg, 219.40 µmol) and **WX066-1** (26.21 mg, 263.28 µmol, HCl) and the solvent dichloromethane (2 mL) were added into a pre-dried reaction flask, followed by addition of 2,4,6-tripropyl-1,3,5,2,4,6-trioxytriphosphate-2,4,6-trioxide (167.54 mg, 263.28 µmol, 156.58 µL, 50% purity). The system was cooled to 0 °C, followed by slow addition of *N*,*N*-diisopropylethylamine (113.42 mg, 877.59 µmol, 152.86 µL), and the reaction solution was stirred at 25 °C for 12 hours. TLC detection (dichloromethane: methanol = 10:1) showed that the reaction was completed. Water (10 mL) was added to the reaction solution, followed by extraction with dichloromethane (10 mL ^{∗} 3). The organic phases were dried over anhydrous sodium sulfate, rotary-evaporated under reduced pressure by a water pump and purified (dichloromethane: methanol = 15: 1) to obtain the target compound **WX066-2.**

### Step 2: synthesis of compound WX066 and WX067

**WX066-2** was resolved by SFC (column: Chiralcel OJ-H 250 ^{∗} 30mm id 5µm; mobile phase: A: CO₂, B: MeOH; gradient: B% = 35%; flow rate: 60 g/min; wavelength: 220 nm; column temperature: 40 °C; back pressure: 100 bar) to obtain the enantiomers **WX066** (retention time is 1.907min) and **WX067** (retention time is 1.916 min). **WX066:** ¹H NMR (400MHz, CHLOROFORM-d) δ = 8.27 (d, *J* = 1.8 Hz, 1H), 8.16-8.12 (m, 1H), 8.11-8.09 (m, 2H), 7.96 (d, *J* = 2.2 Hz, 1H), 7.84-7.80 (m, 1H), 7.77-7.73 (m, 1H), 7.25 (d, *J* = 2.4 Hz, 1H), 7.15-7.07 (m, 1H), 6.13 (br t, *J* = 5.6 Hz, 1H), 4.50-4.38 (m, 1H), 4.37-4.26 (m, 1H), 4.21 (dd, *J* = 5.0, 13.3 Hz, 1H), 4.03 (dd, *J* = 9.5, 13.2 Hz, 1H), 3.98 (s, 3H), 3.54 (q, *J* = 5.1 Hz, 1H), 3.47 (q, *J* = 5.1 Hz, 1H), 3.08-2.99 (m, 1H), 1.31 (d, *J* = 7.1 Hz, 3H); **WX067:** ¹H NMR (400MHz, CHLOROFORM-d) δ = 8.31 (d, *J* = 1.8 Hz, 1H), 8.17-8.13 (m, 2H), 8.10 (s, 1H), 7.98 (d, *J* = 2.2 Hz, 1H), 7.86-7.82 (m, 1H), 7.80-7.76 (m, 1H), 7.54 (s, 1H), 7.28 (br s, 1H), 7.27-7.25 (m, 1H), 7.26 (d, *J* = 2.4 Hz, 1H), 7.16-7.10 (m, 1H), 5.96 (br s, 1H), 4.50-4.37 (m, 1H), 4.37-4.25 (m, 1H), 4.21 (dd, *J* = 5.1, 13.2 Hz, 1H), 4.04 (dd, *J* = 9.6, 13.1 Hz, 1H), 3.99 (s, 3H), 3.54 (q, *J* = 5.1 Hz, 1H), 3.50-3.44 (m, 1H), 3.06-2.96 (m, 1H), 1.32 (d, *J* = 7.1 Hz, 3H).

### Example 41: WX068, WX069

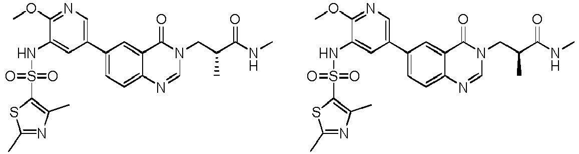

### Synthetic route:

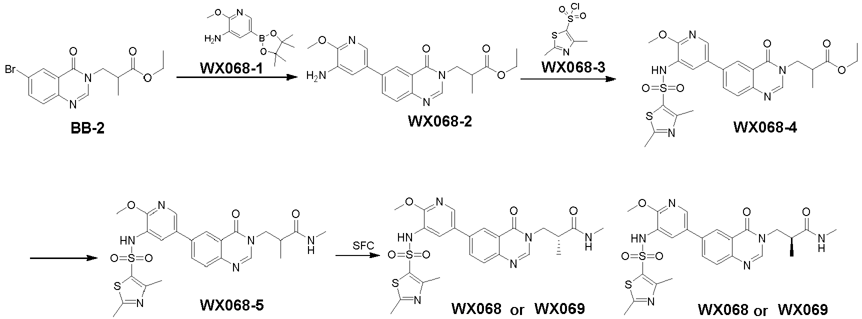

### Step 1: synthesis of compound WX068-2

**BB-2** (209.26 mg, 616.95 µmol) was dissolved in dioxane (5.00 mL) and water (1.00 mL), followed by addition of **WX068-1** (185.16 mg, 740.35 µmol) and [1,1'-bis(diphenylphosphino)ferrocene] palladium dichloride dichloromethane complex (100.77 mg, 123.39 µmol) and potassium acetate (242.19 mg, 2.47 mmol), and the reaction solution was stirred at 100 °C for 3 hours under nitrogen. After the reaction was completed, the reaction solution was rotary-evaporated and separated by preparative HPLC to obtain the target compound **WX068-2.**

### Step 2: synthesis of compound WX068-4

**WX068-2** (0.49 g, 1.28 mmol) was dissolved in pyridine (4.00 mL), then **WX068-3** (298.06 mg, 1.41 mmol) was added thereto, and the reaction solution was stirred at 25 °C for 10 hours. After the reaction was completed, and the reaction solution was rotary-evaporated to obtain the target compound **WX068-4.**

### Step 3: synthesis of compound WX068-5

**WX068-4** (0.5 g, 896.63 µmol) was dissolved in methylamine solution (2 M, 50 mL), and the reaction solution was stirred at 80 °C for 10 hours. After the reaction was completed, the reaction solution was rotary-evaporated, and the compound was separated by preparative HPLC to obtain target compound **WX068-5.**

### Step 4: synthesis of compound WX068 and WX069

**WX068-5** was separated and purified by SFC (separation conditions: chromatographic column: AD (250mm ^{∗} 30mm, 10µm), elution conditions: 0.1% NH₄HCO₃ EtOH, B%: 55% -55%, flow rate: 80mL/min) to obtain the enantiomers **WX068** (Rt = 0.732 min) and **WX069** (Rt = 2.220 min). **WX068:** ¹H NMR (400MHz, CHLOROFORM-d) δ = 8.40(d, J = 2Hz, 1H), 8.22 (d, J = 2Hz, 1H), 8.13 (s, 1H), 8.09 (d, J = 2.4Hz, 1H), 7.89(d, J = 2.4Hz, 1H), 7.80-7.82 (m, 1H), 7.14 (m, 1H), 5.56 (s, 1H), 4.17-4.21 (m, 1H), 4.03-4.08 (m, 1H), 3.97 (s, 3H), 2.93-2.99 (m, 1H), 2.76 (s, 3H), 2.64 (s, 3H), 1.29 (d, J = 6.8Hz, 1H). **WX069:** ¹H NMR (400 MHz, CHLOROFORM-*d*) δ = 8.32 (d, *J* = 2.0 Hz, 1 H), 8.14 (d, *J* = 2.3 Hz, 1 H), 8.05 (s, 1 H), 8.01 (d, *J* = 2.3 Hz, 1 H), 7.82 (dd, *J* = 8.4, 2.1 Hz, 1 H), 7.71-7.76 (m, 1 H), 7.19 (s, 1 H), 6.93-7.17 (m, 1 H), 5.54 (br d, *J* = 4.5 Hz, 1 H), 4.11 (dd, *J* = 13.2, 4.9 Hz, 1 H), 3.98 (dd, *J* = 13.2, 9.4 Hz, 1 H), 3.89 (s, 3 H), 2.84-2.94 (m, 1 H), 2.67 (d, *J* = 4.8 Hz, 3 H), 2.56 (s, 3 H), 2.49 (s, 3 H), 1.22 (d, *J* = 7.0 Hz, 3 H).

### Example 42: WX070, WX071

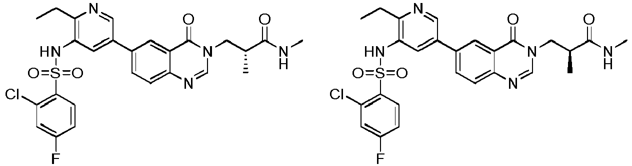

### Synthetic route:

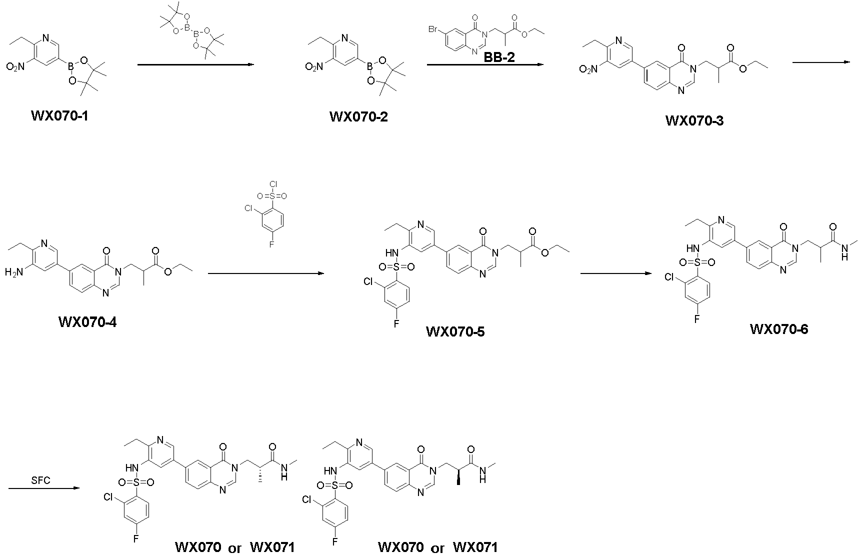

### Step 1: synthesis of compound WX070-2

Compound **WX070-1** (5.0 g, 21.64 mmol), bis(pinacolato)diboron (5.50 g, 21.64 mmol) and potassium acetate (4.25 g, 43.28 mmol) were dissolved in dioxane (10 mL), followed by addition of Pd(dppf)Cl₂ (353.45 mg, 432.81 µmol), and heated to 100 °C and stirred for 5 hours under nitrogen atmosphere. After the reaction was completed, the mixture was cooled to room temperature, and the organic solvent was rotary-evaporated, poured into water (50 mL), and extracted with dichloromethane (50 mL × 3). The organic phases obtained were combined and dried over anhydrous sodium sulfate. After the desiccant was removed by filtration, the solvent was removed under reduced pressure and separated by chromatographic column (eluent: methanol/dichloromethane = 0%-5%) to obtain the target compound **WX070-2**. ¹H NMR (400MHz, Methanol-d4) δ: 9.03-8.74 (m, 1H), 8.57-8.39 (m, 1H), 3.17-2.97 (m, 2H), 1.49-1.31 (m, 15H). MS-ESI m/z: 197.1[M+H]⁺.

### Step 2: synthesis of compound WX070-3

Compound **WX070-2** (0.5 g, 1.80 mmol), **BB-2** (610.53 mg, 1.80 mmol) and potassium acetate (706.61 mg, 7.20 mmol) were dissolved in dioxane (2 mL) and water (0.2 mL), followed by addition of Pd(dppf)Cl₂ (153.34 mg, 187.77 µmol), and heated to 100 °C and stirred for 2 hours under nitrogen atmosphere. After the reaction was completed, the mixture was cooled to room temperature. The organic solvent was rotary-evaporated, and poured into water (50 mL), and extracted with dichloromethane (50 mL × 3). The organic phases obtained were combined and dried over anhydrous sodium sulfate. After the desiccant was removed by filtration, the solvent was removed under reduced pressure and separated by preparative chromatographic plate (eluent: methanol/dichloromethane = 1:20) to obtain the target compound **WX070-3**. MS-ESI m/z: 411.0[M+H]⁺, 413.0[M+H+2]⁺.

### Step 3: synthesis of compound WX070-4

Compound **WX070-3** (0.5 g, 1.22 mmol) was dissolved in methanol (30 mL), followed by addition of ammonium chloride (651.66 mg, 12.18 mmol) and zinc powder (398.31 mg, 6.09 mmol), and stirred at 20 °C for 2 hours. After the reaction was completed, the mixture was filtered to collect the mother liquor. The organic solvent was rotary-evaporated, poured into water (50 mL), and extracted with dichloromethane (50 mL × 3). The organic phases obtained were combined and dried over anhydrous sodium sulfate. After the desiccant was removed by filtration, the solvent was removed under reduced pressure to obtain the target compound **WX070-4,** which was directly used in the next step. MS-ESI m/z:381.0[M+H]⁺, 383.0[M+H+2]⁺.

### Step 4: synthesis of compound WX070-5

Compound **WX070-4** (300 mg, 788.56 µmol) was dissolved in pyridine (3 mL), followed by addition of 2-chloro-4-fluorobenzenesulfonyl chloride (270.94 mg, 1.18 mmol) at 0 °C, and stirred at 20 °C for 2 hours. After the reaction was completed, the organic solvent was rotary-evaporated, poured into water (10 mL), and extracted with dichloromethane (10 mL × 3). The organic phases obtained were combined and dried over anhydrous sodium sulfate. After the desiccant was removed by filtration, the solvent was removed under reduced pressure, separated by preparative chromatographic plate (eluent: methanol/dichloromethane = 1:20), and further separated by high-performance liquid preparative column to obtain the target compound **WX070-5**. MS-ESI m/z: 573.1[M+H]⁺, 575.1[M+H+2]⁺.

### Step 5: synthesis of compound WX070-6

**WX070-5** (0.3 g, 523.53 µmol) was dissolved in methylamine alcohol solution (20 mL), heated to 80 °C and stirred for 3 hours. After the reaction was completed, the mixture was cooled to room temperature. The solvent was removed under reduced pressure, separated by preparative chromatographic plate (eluent: methanol/dichloromethane = 1:15), and further separated by high performance liquid preparative column (water Xbridge 150^{∗}25 5u; mobile phase: [water (10mM NH₄HCO₃)-ACN]; B%: 15%-45%, 8min) to obtain target compound **WX070-6.**

### Step 5: synthesis of compound WX070, WX071

Compound **WX070-6** was resolved by supercritical fluid chromatography (separation conditions: chromatographic column: C2 250mm ^{∗} 30mm, 10µm; mobile phase: [0.1% NH₄HCO₃ MeOH]; B%: 55% -55%) to obtain the enantiomers **WX070** and **WX071,** the retention time of which is 4.997min and 7.676min respectively, and the ratio is 1:1. **WX070:** ¹H NMR (400MHz, Methanol-*d₄*) δ: 8.56 (d, *J* = 2.0Hz, 1H), 8.23 (d, *J* = 2.0 Hz, 1H), 8.10 (s, 1H), 8.04-7.82 (m, 2H), 7.80-7.59 (m, 2H), 7.45 (dd, *J* = 2.4, 8.4 Hz, 1H), 7.15 (dt, *J* = 2.5, 8.4 Hz, 1H), 4.14 (dd, *J* = 4.8, 13.3 Hz, 1H), 3.93 (dd, *J* = 9.8, 13.3 Hz, 1H), 2.99-2.81 (m, 1H), 2.76 (q, *J* = 7.5 Hz, 2H), 2.64-2.42 (m, 3H), 1.14 (d, *J* = 7.0 Hz, 3H), 1.04 (t, *J* = 7.5 Hz, 3H). MS-ESI m/z:558.1[M+H]⁺,560.1[M+H+2]⁺. **WX071:** ¹H NMR (400MHz, Methanol-*d₄*) δ: 8.52 (d, *J* = 2.0 Hz, 1H), 8.23 (d, *J* = 2.0 Hz, 1H), 8.10 (s, 1H), 8.00-7.83 (m, 2H), 7.75 (d, *J* = 2.0 Hz, 1H), 7.68 (d, *J* = 8.5 Hz, 1H), 7.43 (dd, *J* = 2.5, 8.3 Hz, 1H), 7.15 (dt, *J* = 2.5, 8.4 Hz, 1H), 4.14 (dd, *J* = 4.9, 13.4 Hz, 1H), 3.93 (dd, *J* = 9.9, 13.4 Hz, 1H), 3.51 (q, *J* = 7.0 Hz, 2H), 2.93-2.84 (m, 1H), 2.96-2.82 (m, 1H), 2.76 (q, *J* = 7.5 Hz, 2H), 2.52 (s, 3H), 1.18-1.11 (m, 3H), 1.09-1.05 (m, 3H). MS-ESI m/z:558.1[M+H]⁺,560.1 [M+H+2]⁺.

### Example 43: WX072, WX073

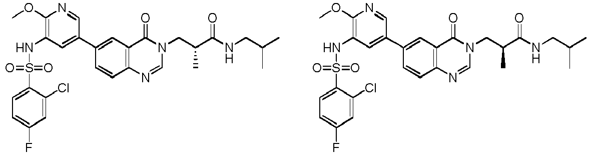

### Synthetic route:

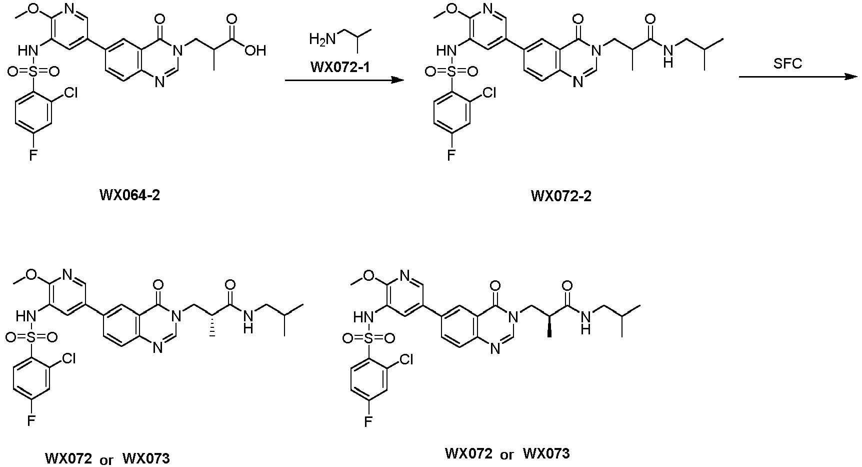

### Step 1: synthesis of compound WX072-2

**WX064-2** (150 mg, 274.25 µmol) was added to a pre-dried 40 mL reaction flask, and dissolved with dichloromethane (5 mL), followed by addition of **WX072-1** (40.11 mg, 548.49 µmol, 54.50 µL) and propylphosphonic anhydride (261.78 mg, 411.37 µmol, 244.65 µL, 50% purity) at 0 °C. Diisopropylethylamine was finally added dropwise (70.89 mg, 548.49 µmol, 95.54 µL) thereto. The reaction was stirred at 20 °C for 16 hours. After the reaction was completed, the mixture was rotary-evaporated directly and separated by preparative HPLC (chromatographic column: Luna C18 100 ^{∗} 30 5µ; mobile phase: [water (0.1% TFA) -ACN]; B%: 50% -80%, 10min) to obtain **WX072-2**.

### Step 2: synthesis of compound WX072, WX073

**WX072-2** was resolved by supercritical fluid chromatography (resolution method: chromatographic column: Chiralpak AD-H 250 ^{∗} 30mm id 5µm; mobile phase: A: CO₂, B: IPA (0.1% NH₄HCO₃); gradient: B% = 42%; flow rate: 70 g/min; wavelength: 220 nm; column temperature: 40 °C; back pressure: 100 bar) to obtain the enantiomers **WX072** (retention time is 2.97min) and **WX073** (retention time is 3.28min). **WX072:** ¹H NMR (400MHz, METHANOL-d4) δ = 8.21 (d, *J* = 2.1 Hz, 1H), 8.10 (d, *J* = 2.3 Hz, 1H), 8.07 (s, 1H), 8.00 (dd, *J* = 5.9, 8.9 Hz, 1H), 7.93-7.85 (m, 2H), 7.64 (d, *J* = 8.5 Hz, 1H), 7.37 (dd, *J* = 2.5, 8.5 Hz, 1H), 7.19-7.08 (m, 1H), 4.16 (dd, *J* = 4.8, 13.3 Hz, 1H), 3.97-3.84 (m, 1H), 3.05-2.94 (m, 1H), 2.89 (dd, *J* = 6.7, 13.2 Hz, 1H), 2.66 (dd, *J* = 7.2, 13.2 Hz, 1H), 1.46 (quind, *J* = 6.8, 13.6 Hz, 1H), 1.15 (d, *J* = 7.0 Hz, 3H), 0.59 (d, *J* = 6.7 Hz, 3H), 0.54 (d, *J* = 6.8 Hz, 3H). MS, m/z = 602.2 [M+1]; **WX073:** ¹H NMR (400MHz, METHANOL-d4) δ = 8.21 (d, *J* = 2.1 Hz, 1H), 8.10 (d, *J* = 2.3 Hz, 1H), 8.07 (s, 1H), 8.00 (dd, *J* = 5.9, 8.9 Hz, 1H), 7.93-7.85 (m, 2H), 7.64 (d, *J* = 8.5 Hz, 1H), 7.37 (dd, *J* = 2.5, 8.5 Hz, 1H), 7.19-7.08 (m, 1H), 4.16 (dd, *J* = 4.8, 13.3 Hz, 1H), 3.97-3.84 (m, 1H), 3.05-2.94 (m, 1H), 2.89 (dd, *J* = 6.7, 13.2 Hz, 1H), 2.66 (dd, *J* = 7.2, 13.2 Hz, 1H), 1.46 (quind, *J* = 6.8, 13.6 Hz, 1H), 1.15 (d, *J* = 7.0 Hz, 3H), 0.59 (d, *J* = 6.7 Hz, 3H), 0.54 (d, *J* = 6.8 Hz, 3H). MS, m/z = 602.2 [M+1]. The ratio of isomers **WX072** and **WX073** is about 1:1.

### Example 44: WX074, WX075

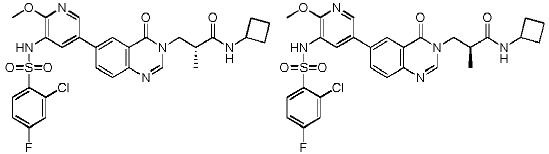

### Synthetic route:

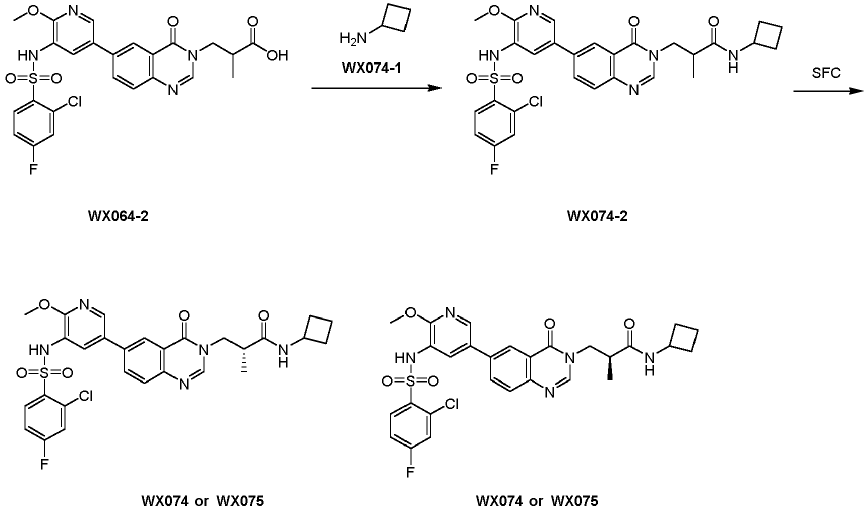

### Step 1: synthesis of compound WX074-2

**WX074-1** (39.01 mg, 548.50 µmol, 47.00 µL) was added into a pre-dried 40 mL reaction flask, and dissolved with dichloromethane (5 mL), followed by addition of **WX064-2** (150 mg, 274.25 µmol) and propylphosphonic anhydride (261.78 mg, 411.38 µmol, 244.65 µL, 50% purity). Diisopropylethylamine (70.89 mg, 548.50 µmol, 95.54 µL) was finally added dropwise thereto at 0°C, and the reaction solution was stirred at 20 °C for 2 hours. After the reaction was completed, the mixture was rotary-evaporated directly, separated and purified by preparative HPLC (method: chromatographic column: Luna C18 100 ^{∗} 30mm 5µm; mobile phase: [water (0.1% TFA) -ACN]; B%: 45% -65%, 10min) to obtain **WX074-2**.

### Step 2: synthesis of compound WX074, WX075

**WX074-2** was resolved by supercritical fluid chromatography (method: column: Chiralpak AD-H 250 ^{∗} 30mm id 5µm; mobile phase: A: CO₂, B: IPA(0.1% NH₄HCO₃); gradient: B% = 42%; flow rate: 70 g/min; wavelength: 220 nm; column temperature: 40 °C; back pressure: 100 bar) to obtain enantiomers **WX074** (retention time is 3.21min) and **WX075** (retention time is 3.46min). **WX074:** ¹H NMR (400MHz, CHLOROFORM-d) δ = 8.25 (d, *J* = 2.0 Hz, 1H), 8.12-8.03 (m, 2H), 8.01 (s, 1H), 7.91 (d, *J* = 2.3 Hz, 1H), 7.80-7.74 (m, 1H), 7.73-7.67 (m, 1H), 7.47 (s, 1H), 7.21 (d, *J* = 2.5 Hz, 1H), 7.09-6.98 (m, 1H), 5.58 (br d, *J* = 8.0 Hz, 1H), 4.32-4.18 (m, 1H), 4.10 (dd, *J* = 5.0, 13.2 Hz, 1H), 3.98-3.85 (m, 4H), 2.91-2.67 (m, 1H), 2.30-2.16 (m, 1H), 2.15-2.03 (m, 1H), 1.77-1.62 (m, 1H), 1.54-1.50 (m, 3H), 1.20 (d, *J* = 7.0 Hz, 3H). MS, m/z = 600.2 [M+1]; **WX075:** ¹H NMR (400MHz, CHLOROFORM-d) δ = 8.25 (d, *J* = 2.0 Hz, 1H), 8.12-8.03 (m, 2H), 8.01 (s, 1H), 7.91 (d, *J* = 2.3 Hz, 1H), 7.80-7.74 (m, 1H), 7.73-7.67 (m, 1H), 7.47 (s, 1H), 7.21 (d, *J* = 2.5 Hz, 1H), 7.09-6.98 (m, 1H), 5.58 (br d, *J* = 8.0 Hz, 1H), 4.32-4.18 (m, 1H), 4.10 (dd, *J* = 5.0, 13.2 Hz, 1H), 3.98-3.85 (m, 4H), 2.91-2.67 (m, 1H), 2.30-2.16 (m, 1H), 2.15-2.03 (m, 1H), 1.77-1.62 (m, 1H), 1.54-1.50 (m, 3H), 1.20 (d, *J* = 7.0 Hz, 3H). MS, m/z = 600.2 [M+1]. The ratio of isomers **WX074** and **WX075** is about 1:1.

### Example 45: WX076, WX077

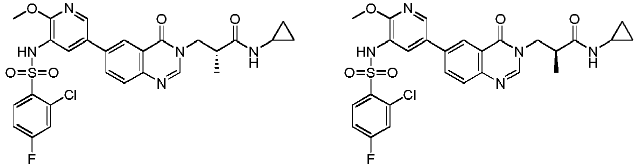

### Synthetic route:

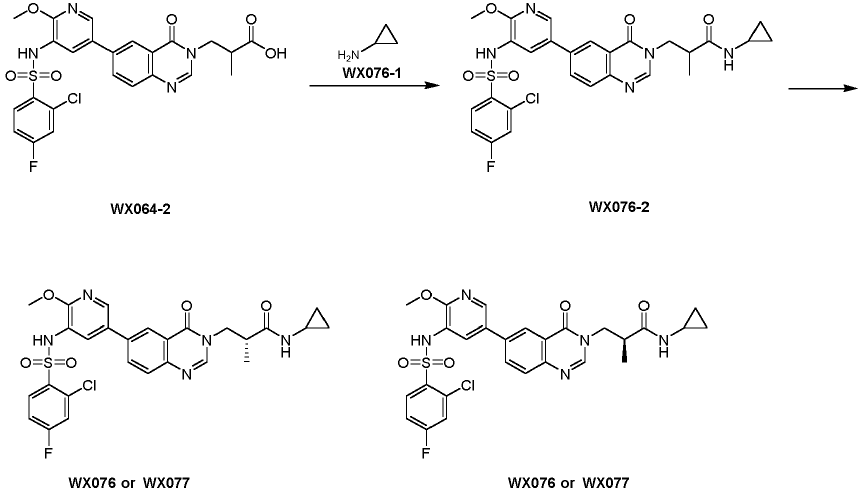

### Step 1: synthesis of compound WX076-2

**WX076-1** (31.32 mg, 548.50 µmol, 38.01 µL) was added into a pre-dried 40 mL reaction flask, dissolved with dichloromethane (5 mL), followed by addition of **WX064-2** (150 mg, 274.25 µmol) and propylphosphonic anhydride (261.78 mg, 411.38 µmol, 244.65 µL, 50% purity). Diisopropylethylamine (70.89 mg, 548.50 µmol, 95.54 µL) was finally added dropwise thereto at 0 °C. The reaction solution was stirred at 20 °C for 2 hours. After the reaction was completed, the mixture was rotary-evaporated directly, separated and purified by preparative HPLC (method: chromatographic column: Luna C18 100 ^{∗} 30mm 5µm; mobile phase: [water (0.1% TFA) -ACN]; B%: 40%-80%, 10min) to obtain **WX076-2.**

### Step 2: synthesis of compound WX076, WX077

Compound **WX076-2** (130mg) was resolved by supercritical fluid chromatography (chromatographic column: Chiralpak AD-H 250 ^{∗} 30mm id 5µm; mobile phase: A: CO₂, B: MeOH (0.1% NH₄HCO₃); gradient: B% = 42% Flow rate: 70 g/min; wavelength: 220 nm; column temperature: 40 °C; back pressure: 100 bar) to obtain the enantiomers **WX076** (retention time is 3.04min) and **WX077** (retention time is 5.63min). **WX076:** ¹H NMR (400MHz, METHANOL-d4) δ = 8.10 (d, J= 2.1 Hz, 1H), 7.99 (d, *J* = 2.1 Hz, 1H), 7.97 (s, 1H), 7.88 (dd, *J* = 5.8, 8.9 Hz, 1H), 7.83-7.76 (m, 2H), 7.55 (d, *J* = 8.4 Hz, 1H), 7.25 (dd, *J* = 2.6, 8.5 Hz, 1H), 7.02 (ddd, *J* = 2.6, 7.9, 8.8 Hz, 1H), 4.02 (dd, *J* = 4.8, 13.4 Hz, 1H), 3.79 (dd, *J* = 10.0, 13.4 Hz, 1H), 3.65 (s, 3H), 2.78-2.65 (m, 1H), 2.33 (tt, *J* = 3.8, 7.3 Hz, 1H), 1.01 (d, *J* = 6.9 Hz, 3H), 0.39 (dd, *J* = 1.7, 7.2 Hz, 2H), 0.08--0.02 (m, 2H). MS, m/z = 586.1 [M+1]; **WX077:** ¹H NMR (400MHz, METHANOL-d4) δ = 8.10 (d, *J* = 2.0 Hz, 1H), 7.97 (s, 2H), 7.89 (dd, *J* = 5.8, 8.8 Hz, 1H), 7.80 (dd, *J* = 2.1, 8.5 Hz, 1H), 7.75 (br s, 1H), 7.55 (d, *J* = 8.4 Hz, 1H), 7.24 (dd, *J* = 2.4, 8.5 Hz, 1H), 7.06-6.95 (m, 1H), 4.02 (dd, *J* = 4.8, 13.4 Hz, 1H), 3.79 (dd, *J* = 10.0, 13.3 Hz, 1H), 3.66 (s, 3H), 2.76-2.65 (m, 1H), 2.33 (tt, *J* = 3.8, 7.3 Hz, 1H), 1.01 (d, *J* = 6.9 Hz, 3H), 0.42-0.35 (m, 2H), 0.08-0.03 (m, 2H). MS, m/z = 586.1 [M+1]. The ratio of isomers **WX076** and **WX077** is about 1:1.

### Example 46: WX078, WX079

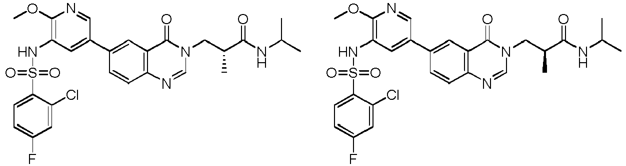

### Synthetic route:

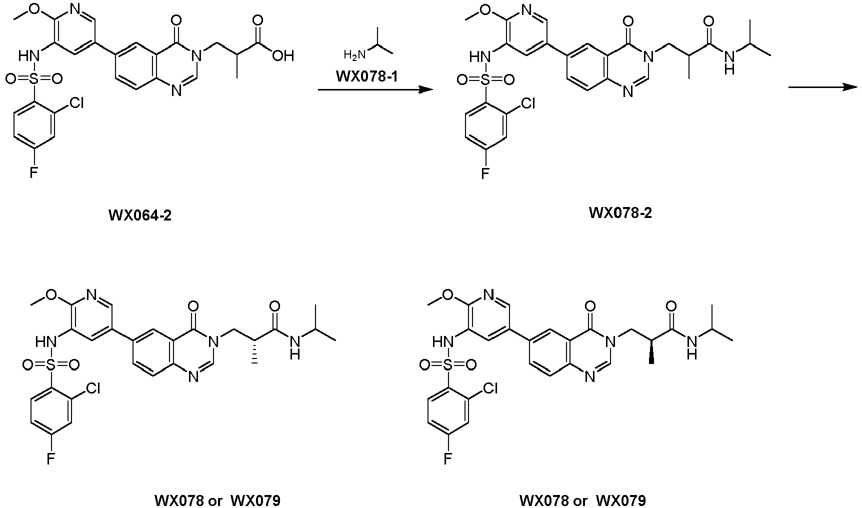

### Step 1: synthesis of compound WX078-2

Compound **WX064-2** (0.15 g, 274.25 µmol), compound **WX078-1** (32.42 mg, 548.49 µmol, 47.12 µL), propylphosphonic anhydride (209.42 mg, 329.09 µmol, 195.72 µL, 50% purity) and dichloromethane (2 mL) were added into a pre-dried reaction flask, and finally *N*,*N*-diethylpropylamine (70.89 mg, 548.49 µmol, 95.54 µL) was added. The mixture was replaced with nitrogen and stirred at 15 °C for 2 hours. After the reaction was completed, the reaction solution was evaporated under reduced pressure to remove the solvent, separated and purified by preparative HPLC (purification method: column: Agela Dµrashell C18 150 ^{∗} 25mm 5µm; mobile phase: [water (10mM NH₄HCO₃)-ACN]; B%: 24 %-54%, 10.5 min) to obtain compound **WX078-2.** ¹H NMR (400MHz, METHANOL-d4) δ = 8.34 (d, *J* = 2.1 Hz, 1H), 8.22 (d, *J* = 2.3 Hz, 1H), 8.17 (s, 1H), 8.10 (dd, *J* = 5.8, 8.9 Hz, 1H), 8.04-7.98 (m, 2H), 7.76 (d, *J* = 8.5 Hz, 1H), 7.47 (dd, *J* = 2.6, 8.5 Hz, 1H), 7.28-7.20 (m, 1H), 4.26 (dd, *J* = 4.8, 13.3 Hz, 1H), 3.99 (dd, *J* = 10.3, 13.2 Hz, 1H), 3.92-3.83 (m, 4H), 3.02-2.91 (m, 1H), 1.23 (d, *J* = 6.9 Hz, 3H), 1.04 (d, *J* = 6.5 Hz, 3H), 0.85 (d, *J* = 6.7 Hz, 3H).

### Step 2: synthesis of compound WX078 and WX079

Compound **WX078-2** was resolved by SFC (chromatographic column: Chiralpak AD-H 250 ^{∗} 30mm id 5µm; mobile phase: A: CO₂, B: EtOH (0.1% NH₄HCO₃); gradient: B% = 42%; flow rate: 70 g/min; wavelength: 220 nm; column temperature: 40 °C; back pressure: 100 bar) to obtain the enantiomers **WX078** (retention time is 3.40 min) and **WX079** (retention time is 3.96 min). **WX078:** ¹H NMR (400MHz, METHANOL-d4) δ = 8.34 (d, *J* = 2.1 Hz, 1H), 8.22 (d, *J* = 2.3 Hz, 1H), 8.17 (s, 1H), 8.10 (dd, *J* = 5.8, 8.9 Hz, 1H), 8.04-7.98 (m, 2H), 7.76 (d, *J* = 8.5 Hz, 1H), 7.47 (dd, *J* = 2.6, 8.5 Hz, 1H), 7.28-7.20 (m, 1H), 4.26 (dd, *J* = 4.8, 13.3 Hz, 1H), 3.99 (dd, *J* = 10.3, 13.2 Hz, 1H), 3.92-3.83 (m, 4H), 3.02-2.91 (m, 1H), 1.23 (d, *J* = 6.9 Hz, 3H), 1.04 (d, *J* = 6.5 Hz, 3H), 0.85 (d, *J* = 6.7 Hz, 3H); **WX079:** ¹H NMR (400MHz, METHANOL-d4) Shift = 8.34 (d, *J* = 2.1 Hz, 1H), 8.22 (d, *J* = 2.3 Hz, 1H), 8.17 (s, 1H), 8.10 (dd, *J* = 5.8, 8.9 Hz, 1H), 8.04-7.98 (m, 2H), 7.76 (d, *J* = 8.5 Hz, 1H), 7.47 (dd, *J* = 2.6, 8.5 Hz, 1H), 7.28-7.20 (m, 1H), 4.26 (dd, *J* = 4.8, 13.3 Hz, 1H), 3.99 (dd, *J* = 10.3, 13.2 Hz, 1H), 3.92-3.83 (m, 4H), 3.02-2.91 (m, 1H), 1.23 (d, *J* = 6.9 Hz, 3H), 1.04 (d, *J* = 6.5 Hz, 3H), 0.85 (d, *J* = 6.7 Hz, 3H).

### Example 47: WX080, WX081

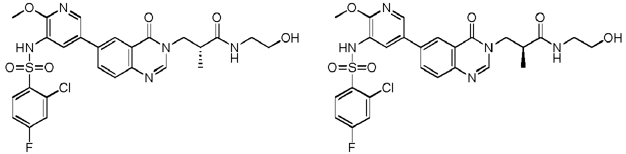

### Synthetic route:

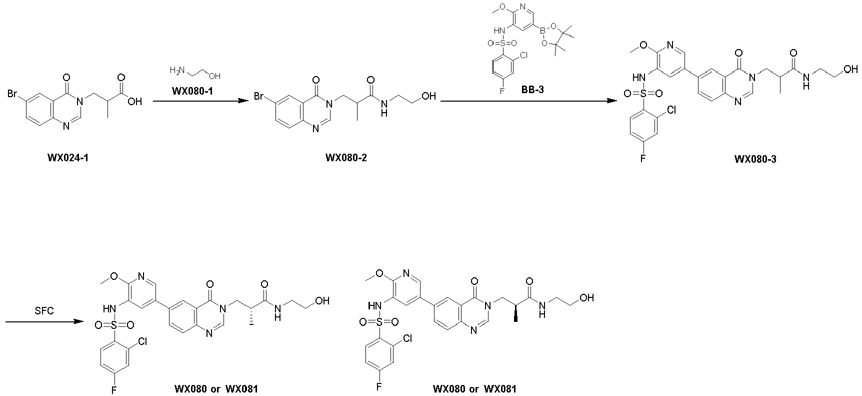

### Step 1: synthesis of compound WX080-2

Raw materials **WX024-1** (1.5 g, 4.82 mmol) and **WX080-1** (353.39 mg, 5.79 mmol, 349.89 µL) were added into a pre-dried 40mL reaction flask, dissolved with the solvent dichloromethane (15 mL), and replaced with nitrogen. The above system was placed at 0 °C, propylphosphonic anhydride (3.68 g, 5.79 mmol, 3.44 mL, 50% purity) and *N*,*N*-diisopropylethylamine (1.87 g, 14.46 mmol, 2.52 mL) were slowly added thereto, and stirred at 25 °C for 12 hours. TLC detection (dichloromethane: methanol = 10:1) showed that the reaction was completed. Water (10 mL) was added to the reaction solution, followed by extraction with dichloromethane (10 mL × 3). The resulting organic phase was dried over anhydrous sodium sulfate, and rotary-evaporated under reduced pressure by a water pump at 35 °C, which was then separated and purified by column chromatography (dichloromethane: methanol = 9:1) to obtain the target compound **WX080-2.** ¹H NMR (400MHz, CHLOROFORM-d) δ = 8.41-8.41 (m, 1H), 8.42-8.41 (m, 1H), 8.42-8.41 (m, 1H), 8.39 (s, 1H), 8.10-8.08 (m, 1H), 8.09 (s, 1H), 7.83 (dd, J = 2.3, 8.7 Hz, 1H), 7.59 (d, J = 8.6 Hz, 1H), 6.04 (br s, 1H), 4.18 (dd, J = 4.9, 13.2 Hz, 1H), 4.03-3.94 (m, 1H), 3.62-3.56 (m, 2H), 3.40-3.25 (m, 2H), 3.01-2.94 (m, 1H), 1.30-1.25 (m, 3H).

### Step 2: synthesis of compound WX080-3

Raw materials **WX080-2** (350 mg, 988.15 µmol) and **BB-3** (743.66 mg, 1.68 mmol), and the solvents 1,4-dioxane (1 mL) and water (0.1 mL) were added into a pre-dried 15mL reaction flask, followed by addition of potassium acetate (193.96 mg, 1.98 mmol). The mixture was replaced with nitrogen, followed by addition of 1,1-bis(diphenylphosphine) ferrocene palladium chloride (72.30 mg, 98.81 µmol). The mixture was replaced with nitrogen and further stirred at 90 °C for 12 hours. TLC detection (dichloromethane: methanol = 10:1) showed that the reaction was completed. Water (10 mL) was added to the system, followed by extraction with dichloromethane (10 mL × 3). The resulting organic phase was dried over anhydrous sodium sulfate, and then rotary-evaporated under reduced pressure, which was then purified by preparative TLC (dichloromethane: methanol = 15:1), and further separated and purified by preparative HPLC (chromatographic column: AD (250mm ^{∗} 30mm, 5µm); mobile phase: [0.1% NH₄HCO₃ EtOH]; B%: 45%-45%, 15min) to obtain the target compound **WX080-3.**

### Step 3: synthesis of compound WX080 and WX081

**WX080-3** was resolved by SFC (column: Agela Durashell C18 150 ^{∗} 25mm 5µm; mobile phase: [water (10mM NH₄HCO₃)-ACN]; B%: 14%-44%, 10.5min) to obtain the enantiomers **WX080** (retention time is 2.61min) and **WX081** (retention time is 1.668min). **WX080:** ¹H NMR (400MHz, CHLOROFORM-d) δ = 8.26 (d, *J* = 2.0 Hz, 1H), 8.15 (dd, *J* = 5.7, 8.8 Hz, 1H), 8.11-8.09 (m, 2H), 7.95 (d, *J* = 2.2 Hz, 1H), 7.82-7.78 (m, 1H), 7.76-7.72 (m, 1H), 7.48 (br s, 1H), 7.25 (d, *J* = 2.4 Hz, 1H), 7.17-7.11 (m, 1H), 6.24 (br t, *J* = 5.4 Hz, 1H), 4.22 (dd, *J* = 4.9, 13.5 Hz, 1H), 4.03 (dd, *J* = 9.5, 13.2 Hz, 1H), 3.98 (s, 3H), 3.63 (t, *J* = 5.0 Hz, 2H), 3.45-3.37 (m, 1H), 3.35-3.27 (m, 1H), 3.05-2.96 (m, 1H), 2.37 (br s, 1H), 1.31 (d, *J* = 7.1 Hz, 3H); **WX081:** ¹H NMR (400MHz, CHLOROFORM-d) δ = 8.24 (d, *J* = 1.8 Hz, 1H), 8.15 (dd, *J* = 5.7, 9.0 Hz, 1H), 8.12-8.07 (m, 2H), 7.93 (d, *J* = 2.2 Hz, 1H), 7.81-7.77 (m, 1H), 7.75-7.70 (m, 1H), 7.59 (br s, 1H), 7.25 (d, *J* = 2.4 Hz, 1H), 7.17-7.11 (m, 1H), 6.35 (br s, 1H), 4.22 (dd, *J* = 4.7, 13.3 Hz, 1H), 4.03 (dd, *J* = 9.6, 13.3 Hz, 1H), 3.97 (s, 3H), 3.63 (t, *J* = 5.0 Hz, 2H), 3.41 (dt, *J* = 5.1, 9.7 Hz, 1H), 3.35-3.26 (m, 1H), 3.05-2.97 (m, 1H), 2.53 (br s, 1H), 1.31 (d, *J* = 7.1 Hz, 3H).

### Example 48: WX082, WX083

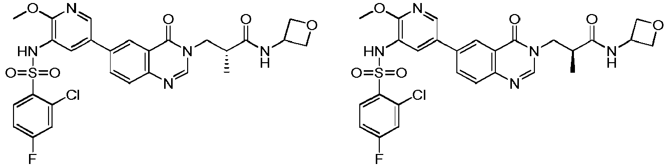

### Synthetic route:

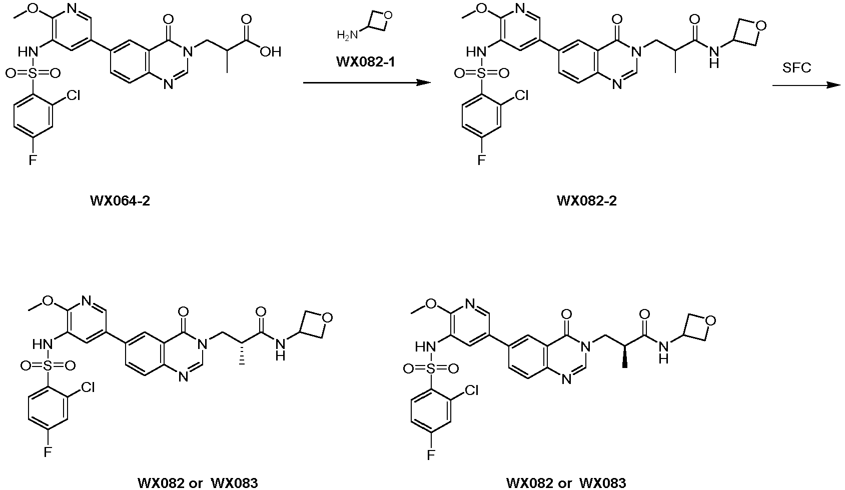

### Step 1: synthesis of compound WX082-2

**WX082-1** (40.09 mg, 548.50 µmol, 47.00 µL) was added into a pre-dried 40 mL reaction flask, dissolved with dichloromethane (5 mL), followed by addition of **WX064-2** (150 mg, 274.25 µmol) and propylphosphonic anhydride (261.78 mg, 411.38 µmol, 244.65 µL, 50% purity). Diisopropylethylamine (70.89 mg, 548.50 µmol), 95.54 µL) was finally added dropwise thereto. The reaction solution was stirred at 20 °C for 16 hours. TLC (dichloromethane: methanol = 20: 1) showed that the reaction was completed. 10 mL of water and 10 mL of ethyl acetate were added to the reaction system for dilution. The organic phase was collected after liquid separation, and the aqueous phase was extracted with ethyl acetate (5 mL × 3). The organic phases were combined, washed with saturated brine (20 mL), dried over anhydrous sodium sulfate, concentrated under reduced pressure, and purified by thin layer chromatography silica gel plate (dichloromethane: methanol = 20: 1) to obtain **WX082-2.**

### Step 2: synthesis of compound WX082, WX083

**WX082-2** (130mg) was resolved by supercritical fluid chromatography (chromatographic column: Chiralpak AD-H 250 ^{∗} 30mm id 5µ; mobile phase: A: CO₂, B: IPA(0.1% NH₄HCO₃); gradient: B% = 40%; flow rate: 60 g/min; wavelength: 220 nm; column temperature: 40 °C; back pressure: 100 bar) to obtain the enantiomers **WX082** (retention time is 3.52 min) and **WX083** (retention time is 3.87 min). **WX082:** ¹H NMR (400MHz, CHLOROFORM-d) δ = 8.28 (d, *J* = 1.8 Hz, 1H), 8.15-8.08 (m, 2H), 8.04 (s, 1H), 7.96 (d, *J* = 2.2 Hz, 1H), 7.85-7.80 (m, 1H), 7.77-7.72 (m, 1H), 7.53 (s, 1H), 7.26-7.24 (m, 1H), 7.14-7.07 (m, 1H), 6.19 (br d, *J* = 7.5 Hz, 1H), 5.03-4.90 (m, 1H), 4.88-4.83 (m, 1H), 4.79 (t, *J* = 7.1 Hz, 1H), 4.40 (t, *J* = 6.4 Hz, 1H), 4.21-4.13 (m, 2H), 4.00 (dd, *J* = 9.3, 13.5 Hz, 1H), 3.96 (s, 3H), 3.08-2.93 (m, 1H), 1.29 (d, *J* = 6.8 Hz, 3H). MS, m/z = 602.2 [M+1]. **WX083:** ¹H NMR (400MHz, CHLOROFORM-d) δ = 8.28 (d, *J* = 1.8 Hz, 1H), 8.15-8.08 (m, 2H), 8.04 (s, 1H), 7.96 (d, *J* = 2.2 Hz, 1H), 7.85-7.80 (m, 1H), 7.77-7.72 (m, 1H), 7.53 (s, 1H), 7.26-7.24 (m, 1H), 7.14-7.07 (m, 1H), 6.19 (br d, *J* = 7.5 Hz, 1H), 5.03-4.90 (m, 1H), 4.88-4.83 (m, 1H), 4.79 (t, *J* = 7.1 Hz, 1H), 4.40 (t, *J* = 6.4 Hz, 1H), 4.21-4.13 (m, 2H), 4.00 (dd, *J* = 9.3, 13.5 Hz, 1H), 3.96 (s, 3H), 3.08-2.93 (m, 1H), 1.29 (d, *J* = 6.8 Hz, 3H). MS, m/z = 602.2 [M+1].

### Example 49: WX084

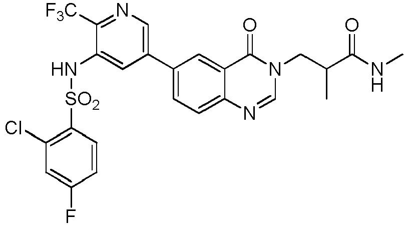

### Synthetic route:

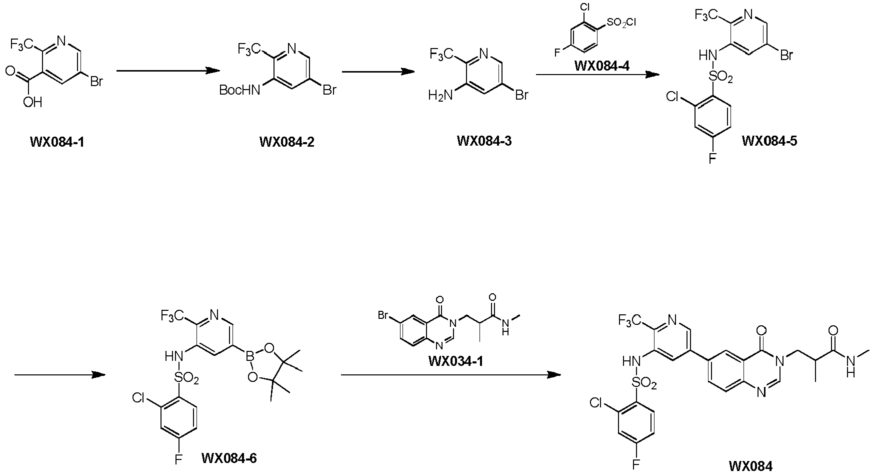

### Step 1: synthesis of compound WX084-2

**WX084-1** (500 mg, 1.85 mmol) was added into a pre-dried 40mL flask and dissolved with *tert*-butanol (10 mL), followed by addition of diphenyl azidephosphate (560.59 mg, 2.04 mmol, 441.41 µL) and triethylamine (224.86 mg, 2.22 mmol, 309.30 µL), and replaced with nitrogen. The reaction solution was stirred at 80 °C for 2 hours. After the reaction was completed, 10 mL of saturated sodium bicarbonate and 10 mL of ethyl acetate were added to the reaction system for dilution. The organic phase was collected after liquid separation, and the aqueous phase was extracted with ethyl acetate (5 mL × 3). The organic phases were combined, washed with saturated brine (20 mL), dried over anhydrous sodium sulfate, concentrated under reduced pressure, and purified by thin layer chromatography silica gel plate (petroleum ether: ethyl acetate = 10: 1) to obtain **WX084-2.** ¹H NMR (400MHz, CHLOROFORM-d) δ = 8.85 (s, 1H), 8.31 (d, *J=* 1.8 Hz, 1H), 6.85 (br s, 1H), 1.54-1.39 (m, 9H).

### Step 2: synthesis of compound WX084-3

**WX084-2** (350 mg, 1.03 mmol) was added into a pre-dried 40 mL reaction flask, and dissolved with hydrochloric acid and ethyl acetate (3 mL). The reaction solution was stirred at 20 °C for 16 hours. TLC (petroleum ether: ethyl acetate = 3:1) showed that the reaction was completed. 30 mL of saturated sodium bicarbonate and 10 mL of ethyl acetate were added to the reaction system for dilution. The organic phase was collected after liquid separation, and the aqueous phase was extracted with ethyl acetate (5 mL × 3). The organic phases were combined, washed with saturated brine (20 mL), dried over anhydrous sodium sulfate, and concentrated under reduced pressure to obtain **WX084-3.** ¹H NMR (400MHz, METHANOL-d4) δ = 7.89 (d, *J* = 1.8 Hz, 1H), 7.49 (d, *J* = 1.3 Hz, 1H).

### Step 3: synthesis of compound WX084-5

**WX084-3** (120 mg, 497.91 µmol) was added into a pre-dried 40 mL flask, and dissolved with tetrahydrofuran (6 mL), then sodium hydrogen (99.58 mg, 2.49 mmol) was added thereto. The reaction was performed at 0 °C for 30 minutes, followed by addition of a solution of **WX084-4** (148.26 mg, 647.28 µmol, 94.44 µL) in tetrahydrofuran (2 mL) at 20 °C. The reaction solution was stirred at 20 °C for 1 hour, followed by addition of sodium hydrogen (99.58 mg, 2.49 mmol, 60% purity). The reaction solution was stirred at 20 °C for 1 hour, followed by further addition of sodium hydrogen (99.58 mg, 2.49 mmol, 60% purity). The reaction solution was stirred at 20 °C for 1 hour, followed by further addition of sodium hydrogen (99.58 mg, 2.49 mmol, 60% purity). The reaction solution was stirred at 20 °C for 1 hour. After the reaction was completed, 10 mL of saturated ammonium chloride and 10 mL of ethyl acetate were added to the reaction system for dilution. The organic phase was collected after liquid separation, and the aqueous phase was extracted with ethyl acetate (5 mL × 3). The organic phases were combined, washed with saturated brine (20 mL), dried over anhydrous sodium sulfate, concentrated under reduced pressure, and purified by thin-layer chromatography silica gel plate (dichloromethane: methanol = 20:1) to obtain **WX084-5.**

### Step 4: synthesis of compound WX084-6

**WX084-5** (60 mg, 138.38 µmol) and bis(pinacolato)diboron (38.65 mg, 152.21 µmol) were added to a pre-dried 40 mL reaction flask and dissolved with 1,4-dioxane (3 mL), followed by addition of potassium acetate (40.74 mg, 415.13 µmol), and replaced with nitrogen. [1,1'-bis(diphenylphosphino) ferrocene] palladium dichloride (10.13 mg, 13.84 µmol) was finally added thereto. The reaction was stirred at 90 °C for 3 hours. After the reaction was completed, the reaction solution was directly filtered and rotary-evaporated to obtain **WX084-6,** which was directly used in the next step.

### Step 5: synthesis of compound WX084

**WX084-6** (60 mg, 124.83 µmol), **WX034-1** (60.70 mg, 187.24 µmol) and potassium carbonate (36.75 mg, 374.48 µmol) were added into a pre-dried 40mL reaction flask and dissolved in 1,4-dioxane (1 mL) and water (0.1 mL), followed by addition of [1,1'-bis(diphenylphosphino) ferrocene] palladium dichloride (9.13 mg, 12.48 µmol), and replaced with nitrogen. The reaction solution was stirred at 90 °C for 16 hours. After the reaction was completed, 10 mL of water and 10 mL of ethyl acetate were added to the reaction system for dilution. The organic phase was collected after liquid separation, and the aqueous phase was extracted with ethyl acetate (5 mL × 3). The organic phases were combined, washed with saturated brine (20 mL), dried over anhydrous sodium sulfate, concentrated under reduced pressure, purified by thin layer chromatography silica gel plate (dichloromethane: methanol = 20: 1), and further separated by preparative HPLC (method: chromatographic column: Agela Durashell C18 150 ^{∗} 25mm 5µm; mobile phase: [water (10mM NH₄HCO₃) -ACN]; B%: 15% -45%, 10.5min) to obtain **WX084.** ¹H NMR (400MHz, METHANOL-d4) δ = 8.77 (s, 1H), 8.45 (d, *J* = 2.2 Hz, 1H), 8.13-8.05 (m, 2H), 7.81 (d, *J* = 8.6 Hz, 1H), 7.45 (dd, *J* = 2.5, 8.5 Hz, 1H), 7.25 (ddd, *J* = 2.6, 7.9, 8.9 Hz, 1H), 4.23 (dd, *J* = 4.9, 13.5 Hz, 1H), 4.02 (dd, *J* = 9.7, 13.5 Hz, 1H), 3.04-2.91 (m, 1H), 2.62-2.55 (m, 3H), 1.22 (d, *J* = 7.1 Hz, 3H). MS, m/z = 598.1 [M+1].

### Example 50: WX085, WX086

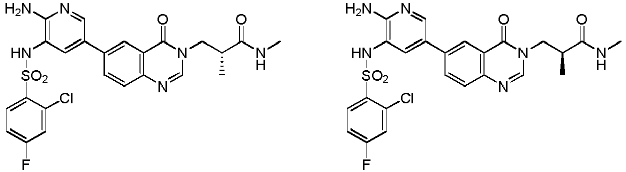

### Synthetic route:

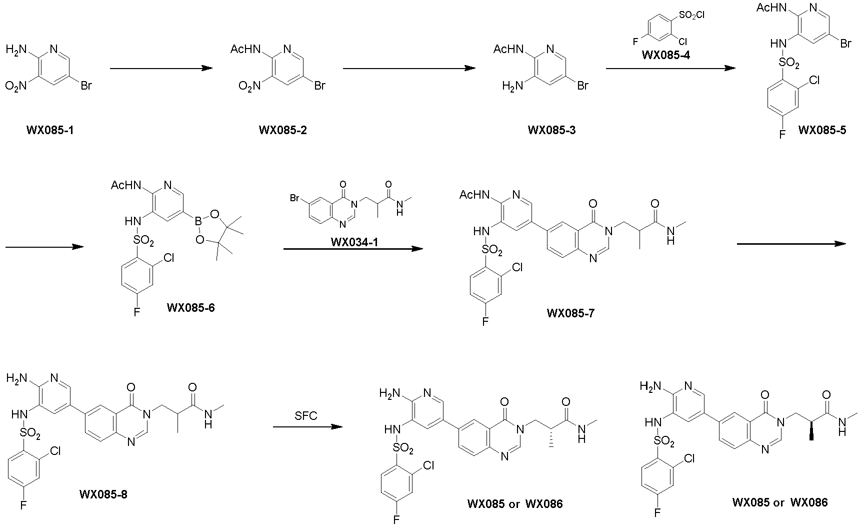

### Step 1: synthesis of compound WX085-2

Compound **WX085-1** (5 g, 22.93 mmol) and acetic acid (10 mL) were sequentially added into a pre-dried single-necked flask (100 mL), followed by addition of acetic anhydride (10.95 g, 107.22 mmol, 10.04 mL) and concentrated sulfuric acid (1.12 g, 11.38 mmol, 606.69 µL). The mixture was replaced with nitrogen, heated to 60 °C and stirred for 2 hours. After the reaction was completed, the reaction solution was slowly added to ice water (40 mL), stirred for ten minutes, and filtered. After the filter cake was washed with water (20 mL × 2), the filter cake was lyophilized to remove residual moisture to obtain target compound **WX085-2.** ¹H NMR (400MHz, CHLOROFORM-d) δ = 9.82 (br s, 1H), 8.70 (d, *J* = 2.3 Hz, 1H), 8.63 (d, *J* = 2.3 Hz, 1H), 2.46 (s, 3H).

### Step 2: synthesis of compound WX085-3

Compound **WX085-2** (3.6 g, 13.84 mmol), ammonium chloride (8.89 g, 166.13 mmol, 5.81 mL), methanol (180 mL) and water (90 mL) were sequentially added into a pre-dried three-neck flask, followed by slow addition of zinc powder (6.34 g, 96.91 mmol). The mixture was replaced with nitrogen and stirred at 20 °C for 2 hours. TLC (petroleum ether/ethyl acetate = 1/1) showed that the reaction was completed. The mixture was filtered and washed with methanol (100 mL × 3). The filtrate was combined, rotary-evaporated under reduced pressure, and extracted with saturated sodium bicarbonate (100 mL) and ethyl acetate (300 mL × 4). The organic phases was combined, washed with saturated chlorine sodium sulfate (200 mL), dried over anhydrous sodium sulfate, concentrated under reduced pressure, and purified by column chromatography (petroleum ether: ethyl acetate = 5:1 to 1:1) to obtain compound **WX085-3.** ¹H NMR (400MHz, METHANOL-d4) δ = 7.74 (d, *J* = 2.1 Hz, 1H), 7.37 (d, *J* = 2.1 Hz, 1H), 2.16 (s, 3H).

### Step 3: synthesis of compound WX085-5

Compound **WX085-3** (1.1 g, 4.78 mmol) and pyridine (10 mL) were added into a pre-dried reaction flask, and compound **WX085-4** (1.20 g, 5.26 mmol, 767.33 µL) was finally added thereto. The mixture was replaced with nitrogen, and stirred at 50 °C for 10 hours. TLC (ethyl acetate) showed the reaction was completed. After the solvent was rotary-evaporated under reduced pressure, the residue was separated and purified by column chromatography (petroleum ether: ethyl acetate = 5: 1 to 0: 1) to obtain compound **WX085-5.** ¹H NMR (400MHz, CHLOROFORM-d) δ = 9.40 (br s, 1H), 8.18 (d, *J* = 2.0 Hz, 1H), 8.02-7.95 (m, 2H), 7.71 (br s, 1H), 7.26 (d, *J* = 2.6 Hz, 1H), 7.06 (ddd, *J* = 2.6, 7.4, 8.9 Hz, 1H), 2.28 (s, 3H).

### Step 4: synthesis of compound WX085-6

Compound **WX085-5** (0.5 g, 1.18 mmol), bis(pinacolato)diboron (330.45 mg, 1.30 mmol), potassium acetate (232.20 mg, 2.37 mmol) and 1,4-dioxane (5 mL) were sequentially added to the pre-dried reaction flask (10 mL), and replaced with nitrogen. 1,1-bis(diphenylphosphine) ferrocene palladium chloride (86.56 mg, 118.30 µmol) was added thereto finally. The mixture was replaced with nitrogen, heated to 110 °C and stirred for 10 hours. After the reaction was completed, the reaction solution was cooled down and the solvent was evaporated under reduced pressure to obtain compound **WX085-6,** which was used directly in the next step.

### Step 5: synthesis of compound WX085-7

Compound **WX034-1** (414.08 mg, 1.28 mmol), compound **WX085-6** (0.6 g, 1.28 mmol), potassium acetate (376.08 mg, 3.83 mmol), the solvents 1,4-dioxane (5 mL) and water (0.5 mL) were added to a pre-dried reaction flask, and replaced with nitrogen. 1,1-bis(diphenylphosphine) ferrocene palladium chloride (93.47 mg, 127.74 µmol) was added thereto, and replaced with nitrogen again. The mixture was heated to 110 °C and stirred for 10 hours. TLC (dichloromethane/methanol = 10/1) showed that the reaction was completed. After the reaction solution was cooled down, the solvent was evaporated under reduced pressure, and separated by column chromatography (petroleum ether: ethyl acetate = 5:1 to 1:1) to obtain compound **WX085-7.** ¹H NMR (400MHz, METHANOL-d4) δ = 8.55 (s, 1H), 8.43 (d, *J* = 2.0 Hz, 1H), 8.27-8.15 (m, 3H), 8.09 (dd, *J* = 2.1, 8.5 Hz, 1H), 7.92 (dd, *J* = 5.8, 8.8 Hz, 1H), 7.80 (d, *J* = 8.5 Hz, 1H), 7.62-7.47 (m, 6H), 7.18 (dt, *J* = 2.4, 8.4 Hz, 1H), 4.34-4.17 (m, 2H), 4.09-3.95 (m, 2H), 3.60 (s, 1H), 3.05-2.92 (m, 2H), 2.63-2.61 (m, 3H), 2.22 (s, 3H), 1.24 (d, *J* = 7.0 Hz, 3H).

### Step 6: synthesis of compound WX085-8

Compound **WX085-7** (0.28 g, 476.98 µmol), methanol (10 mL), potassium carbonate (197.77 mg, 1.43 mmol) were sequentially added to a pre-dried reaction flask. The mixture was replaced with nitrogen, heated to 80 °C and stirred for 10 hours. After the reaction was completed, the reaction solution was cooled down, concentrated under reduced pressure, and purified by preparative HPLC (purification method: column: Agela Durashell C18 150 ^{∗} 25mm 5µm; mobile phase: [water (10mM NH₄HCO₃) -ACN]; B%: 12% -42%, 10.5min) to obtain compound **WX085-8.** ¹H NMR (400MHz, METHANOL-d4) δ = 8.17 (d, *J* = 2.0 Hz, 1H), 8.15 (s, 1H), 8.11 (dd, *J* = 3.3, 9.3 Hz, 1H), 8.01-7.91 (m, 1H), 7.87 (dd, *J* = 2.1, 8.5 Hz, 1H), 7.72-7.66 (m, 1H), 7.54-7.44 (m, 2H), 7.28-7.20 (m, 1H), 4.23 (dd, *J* = 5.0, 13.4 Hz, 1H), 4.01 (dd, *J* = 9.9, 13.4 Hz, 1H), 2.99 (br dd, *J* = 9.7, 11.9 Hz, 1H), 2.61 (s, 3H), 1.23 (d, *J* = 6.9 Hz, 3H).

### Step 7: synthesis of compound WX085 and WX086

Compound **WX085-8** (0.1 g, 183.49 µmol) was resolved by preparative SFC (resolution method: column: OD (250mm ^{∗} 30mm, 5µm); mobile phase: [0.1% NH₄HCO₃ MeOH]; B%: 40% -40%, 13min) to obtain the enantiomers **WX085** (retention time is 3.28min) and **WX086** (retention time is 3.90min). **WX085:** ¹H NMR (400MHz, METHANOL-d4) δ = 8.18-8.13 (m, 2H), 8.10 (dd, *J* = 5.9, 8.9 Hz, 1H), 8.01 (br s, 1H), 7.85 (dd, *J* = 2.1, 8.5 Hz, 1H), 7.69 (d, *J* = 8.5 Hz, 1H), 7.54-7.47 (m, 2H), 7.29-7.21 (m, 1H), 4.23 (dd, *J* = 4.9, 13.3 Hz, 1H), 4.01 (dd, *J* = 9.8, 13.4 Hz, 1H), 3.05-2.92 (m, 1H), 2.65-2.58 (m, 3H), 1.23 (d, *J* = 7.0 Hz, 3H). **WX086**:¹H NMR (400MHz, METHANOL-d4) δ = 8.19-8.14 (m, 2H), 8.13-8.06 (m, 1H), 8.01 (br s, 1H), 7.86 (dd, *J* = 2.2, 8.6 Hz, 1H), 7.72-7.67 (m, 1H), 7.53-7.44 (m, 2H), 7.28-7.20 (m, 1H), 4.23 (dd, *J* = 5.0, 13.3 Hz, 1H), 4.02 (dd, *J* = 9.7, 13.5 Hz, 1H), 3.04-2.93 (m, 1H), 2.62 (s, 3H), 1.23 (d, *J* = 6.8 Hz, 3H).

### Example 51: WX087, WX088

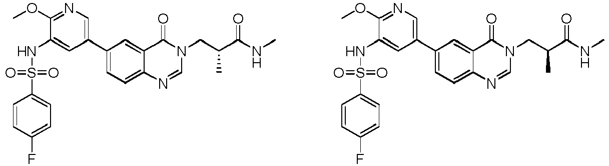

### Synthetic route:

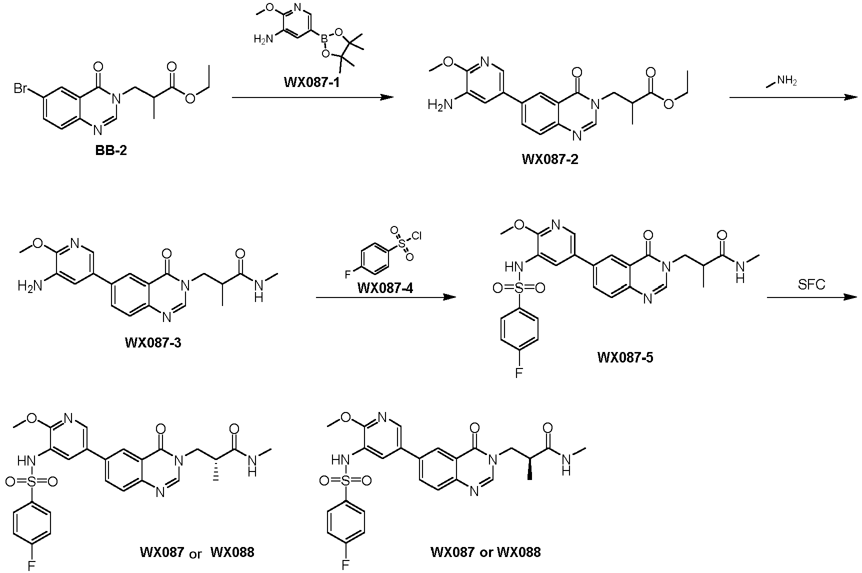

### Step 1: synthesis of compound WX087-2

Compound **BB-2** (2 g, 5.90 mmol) was dissolved in dioxane (20 mL) and water (4 mL), followed by addition of compound **WX087-1** (1.77 g, 7.08 mmol), Pd(dppf)Cl₂ (963.06 mg, 1.18 mmol) and potassium acetate (2.31 g, 23.59 mmol). The reaction solution was stirred at 100 °C for 3 hours under nitrogen atmosphere. After the reaction was completed, the reaction solution was rotary-evaporated. The obtained residue was separated by chromatography column (eluent: methanol / dichloromethane = 5 ∼ 10%) to obtain the target compound **WX087-2.** MS-ESI *m*/*z*: 383.1 [M+H]⁺.

### Step 2: synthesis of compound WX087-3

Compound **WX087-2** (2.3 g, 6.01 mmol) was dissolved in methylamine ethanol solution (2 M, 50 mL), and the reaction solution was stirred at 80 °C for 10 hours. After the reaction was completed, the reaction solution was rotary-evaporated to obtain the target compound **WX087-3.** MS-ESI *m*/*z*: 368.1 [M+H]⁺.

### Step 3: synthesis of compound WX087-5

Compound **WX087-3** (0.3 g, 816.55 µmol) was dissolved in pyridine (5 mL). Compound **WX087-4** (144.46 mg, 742.32 µmol) was added thereto, and the reaction solution was stirred at 25 °C for 10 hours. After the reaction was completed, the reaction solution was rotary-evaporated and separated by preparative HPLC (TFA) to obtain the target compound **WX087-5.** MS-ESI *m*/*z*: 526.1 [M+H]⁺.

### Step 4: synthesis of compound WX087 and WX088

Compound **WX087-5** was resolved by supercritical fluid chromatography (separation conditions: column: AD (250mm * 30mm, 10µm), elution conditions: 0.1% NH₄HCO₃ EtOH, EtOH; B%: 55% -55%, flow rate (mL/min): 80mL/min) to obtain the enantiomers **WX087** (retention time is 0.863 min) and **WX088** (retention time is 2.485min). **WX087:** ¹H NMR (400 MHz, DMSO-*d*₆) δ = 8.32 (d, *J* = 1.8 Hz, 1 H), 8.24 (d, *J* = 2.0 Hz, 1H), 8.19 (s, 1H), 8.06 (dd, *J* = 8.5, 2.3 Hz, 1 H), 7.89 (d, *J* = 2.5 Hz, 2 H), 7.79-7.84 (m, 2 H), 7.76 (d, *J =* 8.5 Hz, 1 H), 7.40 (t, *J =* 8.9 Hz, 2 H), 4.03-4.11 (m, 1 H), 3.97 (dd, *J* = 13.3, 9.0 Hz, 1 H), 3.69 (s, 3 H), 2.87 (br dd, *J* = 14.8, 7.0 Hz, 1 H), 2.48 (br s, 3 H), 1.08 (d, *J =* 7.0 Hz, 3 H). MS-ESI *m*/*z*: 526.1 [M+H]⁺. **WX088:** ¹H NMR (400 MHz, DMSO-*d*₆) δ = 8.31-8.36 (m, 1 H), 8.25 (d, *J =* 2.0 Hz, 1 H), 8.19 (s, 1 H), 8.07 (dd, *J =* 8.5, 2.0 Hz, 1 H), 7.87-7.93 (m, 2 H), 7.82 (dd, *J =* 8.8, 5.3 Hz, 1 H), 7.79-7.84 (m, 1 H), 7.41 (t, *J =* 8.8 Hz, 2 H), 4.02-4.13 (m, 1 H), 3.97 (dd, *J* = 13.2, 9.2 Hz, 1 H), 3.69 (s, 3 H), 2.81-2.94 (m, 1 H), 2.48-2.49 (m, 3 H), 1.08 (d, *J* = 7.0 Hz, 3 H). MS-ESI *m*/*z:* 526.1 [M+H]⁺. The ratio of two isomers is 1:1.

### Example 52: WX089, WX090

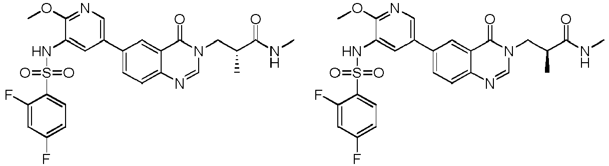

### Synthetic route:

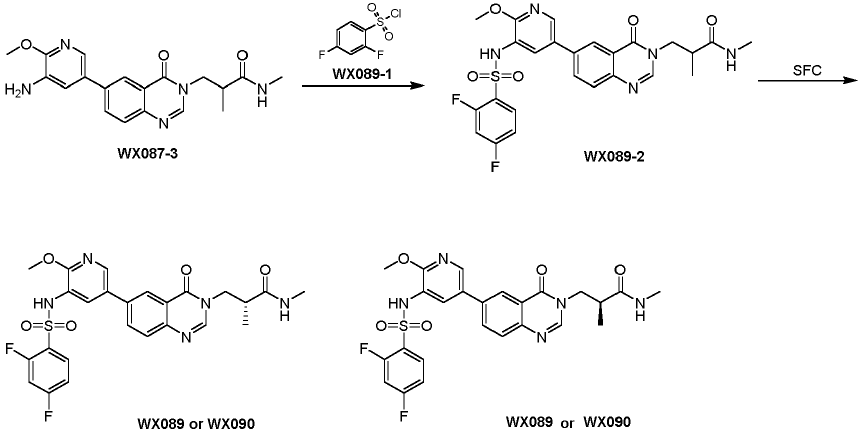

### Step 1: synthesis of compound WX089-2

Compound **WX087-3** (0.3 g, 816.55 µmol) was dissolved in pyridine (5 mL). Compound **WX089-1** (157.82 mg, 742.32 µmol, 99.88 µL) was added thereto, and the reaction solution was stirred at 25 °C for 10 hours. After the reaction was completed, the reaction solution was rotary-evaporated, and separated by preparative HPLC (TFA) to obtain target compound **WX089-2.** MS-ESI *m*/*z*: 544.1 [M+H]⁺.

### Step 2: synthesis of compound WX089 and WX090

Compound **WX089-2** was resolved by supercritical fluid chromatography (separation conditions: chromatographic column: AD (250mm * 30mm, 10µm); mobile phase: [0.1% NH₄HCO₃ EtOH]; B%: 55% -55%) to obtain enantiomers **WX089** (retention time is 0.711 min) and **WX090** (retention time is 2.308 min). **WX089:** ¹H NMR (400 MHz, DMSO-*d*₆) δ = 8.22-8.36 (m, 2 H), 8.18 (s, 1 H), 8.06 (dd, *J* = 8.4, 2.1 Hz, 1 H), 7.82-7.93 (m, 2 H), 7.72-7.81 (m, 2 H), 7.50 (br t, *J =* 9.2 Hz, 1 H), 7.16-7.22 (m, 1 H), 4.03-4.17 (m, 1 H), 3.87-4.02 (m, 1H), 3.70 (s, 3 H), 2.87 (dq, *J =* 14.5, 7.1 Hz, 1 H), 2.48 (br s, 3 H), 1.08 (d, *J* = 7.0 Hz, 3 H). MS-ESI m/z: 544.1 [M+H]⁺. **WX090:** ¹H NMR (400 MHz, DMSO-*d*₆) δ = 8.22-8.36 (m, 2 H), 8.18 (s, 1 H), 8.06 (dd, *J =* 8.4, 2.1 Hz, 1 H), 7.82-7.93 (m, 2 H), 7.72-7.81 (m, 2 H), 7.50 (br t, *J =* 9.2 Hz, 1 H), 7.16-7.22 (m, 1 H), 4.03-4.17 (m, 1 H), 3.87-4.02 (m, 1 H), 3.70 (s, 3 H), 2.87 (dq, *J* = 14.5, 7.1 Hz, 1 H), 2.48 (br s, 3 H), 1.08 (d, *J* = 7.0 Hz, 3 H). MS-ESI *m*/*z*: 544.1 [M+H]⁺. The ratio of two isomers is 1:1.

### Example 53: WX091, WX092

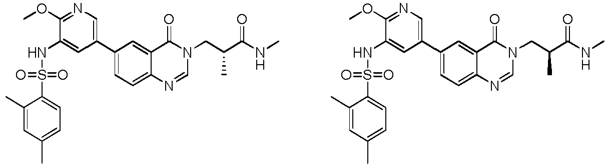

### Synthetic route:

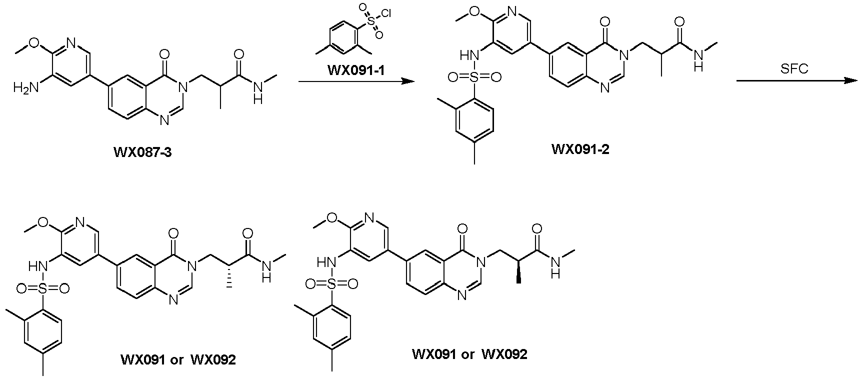

### Step 1: synthesis of compound WX091-2

Compound **WX087-3** (0.3 g, 816.55 µmol) was dissolved in pyridine (5 mL), compound **WX091-1** (151.93 mg, 742.32 µmol) was added thereto, and the reaction solution was stirred at 25 °C for 10 hours. After the reaction was completed, the reaction solution was rotary-evaporated, and separated by preparative HPLC (TFA) to obtain target compound **WX091-2.** MS-ESI *m*/*z:* 536.1 [M+H]⁺.

### Step 2: synthesis of compound WX091 and WX092

Compound **WX091-2** was resolved by supercritical fluid chromatography (separation conditions: chromatographic column: AD (250mm * 30mm, 10µm); mobile phase: [0.1% NH₄HCO₃ EtOH]; B%: 55% -55%, flow rate (mL/min) : 80mL/min) to obtain enantiomers **WX091** (retention time is 0.796 min) and **WX092** (retention time is 2.452 min). **WX091:** ¹HNMR (400 MHz, DMSO-*d*₆) δ = 8.20-8.30 (m, 1 H), 8.18 (br d, *J* = 2.5 Hz, 2 H), 7.94-8.04 (m, 1 H), 7.90 (br d, *J* = 4.5 Hz, 1 H), 7.77-7.82 (m, 1 H), 7.71-7.77 (m, 1 H), 7.64 (d, *J* = 7.8 Hz, 1 H), 7.19 (s, 1 H), 7.10 (br d, *J* = 8.3 Hz, 1 H), 4.04-4.13 (m, 1 H), 3.90-4.01 (m, 1 H), 3.73 (s, 3 H), 2.76-3.02 (m, 1 H), 2.60 (s, 3 H), 2.29 (s, 3 H), 1.08 (br d, *J* = 6.8 Hz, 3 H). MS-ESI *m*/*z:* 536.1 [M+H]⁺. **WX092:** ¹H NMR (400 MHz, DMSO-*d*₆) δ = 8.31 (d, *J* = 2.0 Hz, 1 H), 8.20 (d, *J* = 5.2 Hz, 2 H), 8.03 (dd, *J* = 8.5, 2.0 Hz, 1 H), 7.87-7.93 (m, 1 H), 7.84 (d, *J* = 2.0 Hz, 1 H), 7.76 (d, *J* = 8.5 Hz, 1 H), 7.63 (d, *J =* 8.0 Hz, 1 H), 7.23 (s, 1 H), 7.12 (br d, *J =* 8.0 Hz, 1 H), 4.03-4.13 (m, 1 H), 3.97 (br dd, *J =* 13.2, 9.2 Hz, 1 H), 3.74 (s, 3 H), 2.87 (br dd, *J* = 14.6, 7.0 Hz, 1 H), 2.62 (s, 3 H), 2.30 (s, 3 H), 1.09 (d, *J* = 7.0 Hz, 3 H). MS-ESI *m*/*z*: 536.1 [M+H]⁺. The ratio of two isomers is 1:1.

### Example 54: WX093, WX094

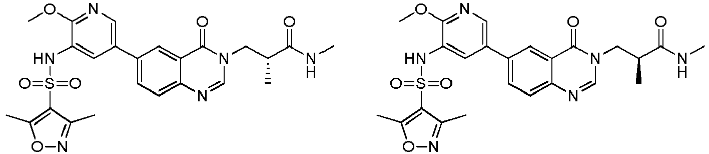

### Synthetic route:

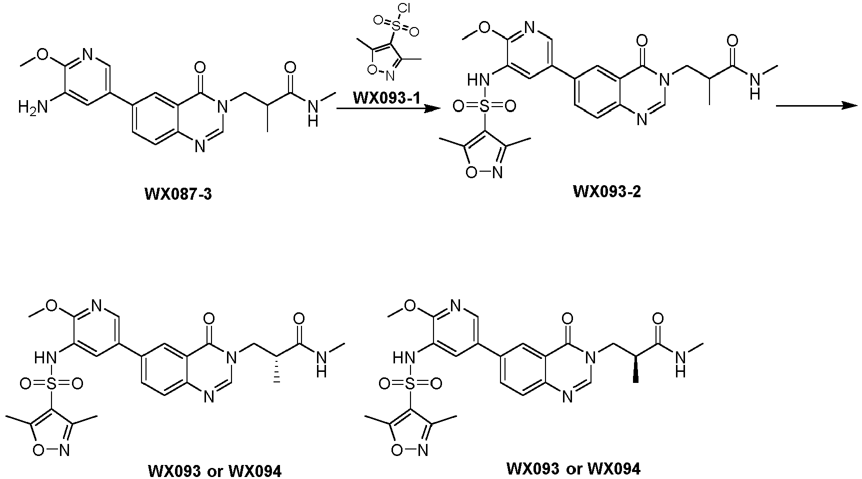

### Step 1: synthesis of compound WX093-2

Compound **WX087-3** (0.3 g, 816.55 µmol) was dissolved in pyridine (5 mL). Compound **WX093-1** (159.74 mg, 816.55 µmol) was added thereto, and the reaction solution was stirred at 25 °C for 10 hours. After the reaction was completed, the reaction solution was rotary-evaporated, and separaed by preparative HPLC (TFA) to obtain target compound **WX093-2.** MS-ESI *m*/*z*: 527.1 [M+H]⁺, 549.1 [M+Na]⁺.

### Step 2: synthesis of compound WX093 and WX094

Compound **WX093-2** was resolved by supercritical fluid chromatography (separation conditions: chromatography column: AD (250mm * 30mm, 10µm); mobile phase: [0.1% NH₄HCO₃ EtOH]; B%: 55% -55%, flow rate (mL/min) : 80mL/min) to obtain enantiomers **WX093** (retention time is 0.473 min) and **WX094** (retention time is 1.176 min). **WX093:** ¹H NMR (400 MHz, DMSO-*d*₆) δ = 8.33 (br s, 1 H), 8.26 (s, 1 H), 8.18 (s, 1 H), 8.09 (br d, *J=* 8.5 Hz, 1 H), 7.89 (br d, *J=* 4.3 Hz, 2 H), 7.73-7.79 (m, 1 H), 4.05-4.10 (m, 1 H), 3.93-4.00 (m, 1 H), 3.74 (s, 3 H), 2.86 (br dd, *J=* 15.1, 6.3 Hz, 1 H), 2.48 (br s, 3 H), 2.37 (s, 3 H), 2.26 (s, 3 H), 1.08 (d, *J* = 6.8 Hz, 3 H). MS-ESI *m*/*z*: 527.1 [M+H]⁺, 549.1 [M+Na]⁺. **WX094:** ¹H NMR (400 MHz, DMSO-*d*₆) δ = 8.36 (br s, 1 H), 8.26 (s, 1 H), 8.18 (s, 1 H), 8.10 (br d, *J* = 8.5 Hz, 1 H), 7.89 (br d, *J* = 4.5 Hz, 2 H), 7.76 (d, *J* = 8.5 Hz, 1 H), 4.04-4.12 (m, 1 H), 3.96 (dd, *J* = 13.1, 9.0 Hz, 1 H), 3.74 (s, 3 H), 2.81-2.92 (m, 1 H), 2.35 (s, 2 H), 2.26 (s, 3 H), 1.08 (d, *J* = 7.0 Hz, 3 H). MS-ESI *m*/*z*: 527.1 [M+H]⁺, 549.1 [M+Na]⁺. The ratio of two isomers is 1:1.

### Example 55: WX095, WX096

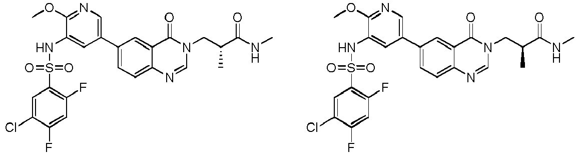

### Synthetic route:

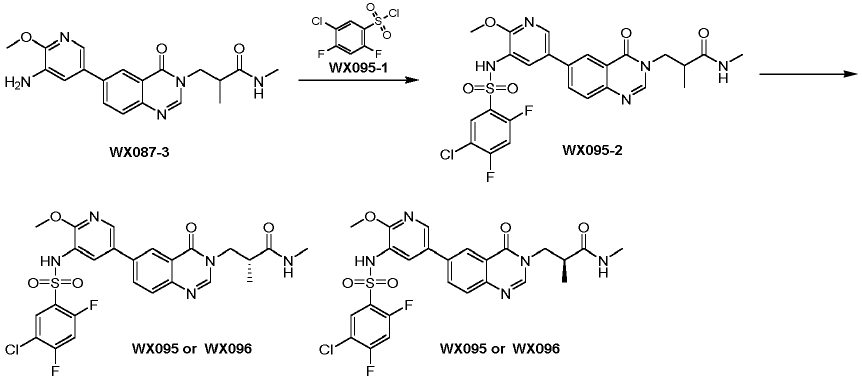

### Step 1: synthesis of compound WX095-2

Compound **WX087-3** (0.3 g, 816.55 µmol) was dissolved in pyridine (5 mL), compound **WX095-1** (201.73 mg, 816.55 µmol) was added thereto, and the reaction solution was stirred at 25 °C for 10 hours. After the reaction was completed, the reaction solution was rotary-evaporated, and separated by preparative HPLC (TFA) to obtain target compound **WX095-2.** MS-ESI *m*/*z:* 578.0 [M+H]⁺.

### Step 2: synthesis of compound WX095 and WX096

Compound **WX095-2** was resolved by supercritical fluid chromatography (separation conditions: chromatographic column: AD (250mm * 30mm, 5µm); mobile phase: [0.1% NH₄HCO₃ IPA]; B%: 40% -40%, flow rate (mL/min) : 80mL/min) to obtain enantiomers **WX095** (retention time is 3.939 min) and **WX096** (retention time is 3.580 min). **WX095:** ¹H NMR (400 MHz, DMSO-*d*₆) δ = 8.20-8.33 (m, 2 H), 8.17 (s, 1 H), 8.06 (dd, *J* = 8.4, 2.1 Hz, 1 H), 7.86-7.94 (m, 3 H), 7.75 (d, *J* = 8.5 Hz, 2 H), 4.02-4.11 (m, 1H), 3.96 (dd, *J* = 13.2, 9.2 Hz, 1H), 3.73 (s, 3 H), 2.86 (br dd, *J* = 14.7, 6.9 Hz, 1 H), 2.48 (br s, 3 H), 1.08 (d, *J* = 7.0 Hz, 3 H). MS-ESI *m*/*z:* 578.0 [M+H]⁺. **WX096:** ¹H NMR (400 MHz, DMSO-*d*₆) δ = 8.42 (d, *J =* 2.0 Hz, 1 H), 8.29 (d, *J =* 2.0 Hz, 1 H), 8.19 (s, 1 H), 8.10 (dd, *J* = 8.5, 2.3 Hz, 1 H), 7.98 (d, *J* = 2.3 Hz, 1 H), 7.90-7.94 (m, 1 H), 7.89 (s, 1 H), 7.83-7.87 (m, 1 H), 7.76 (d, *J* = 8.5 Hz, 1 H), 4.02-4.14 (m, 1 H), 3.97 (dd, *J* = 13.3, 9.0 Hz, 1 H), 3.70 (s, 3 H), 2.82-2.93 (m, 1 H), 2.48 (br s, 3 H), 1.08 (d, *J* = 7.0 Hz, 3 H). MS-ESI *m*/*z:* 578.0 [M+H]⁺. The ratio of two isomers is 1:1.

### Example 56: WX097, WX098

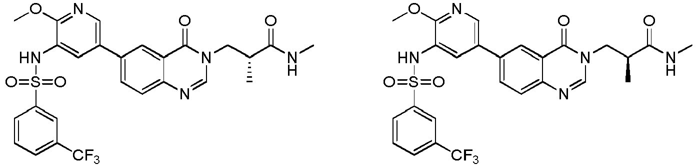

### Synthetic route:

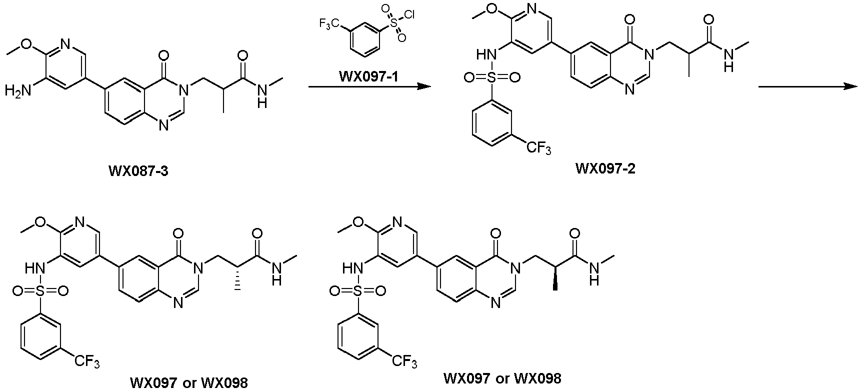

### Step 1: synthesis of compound WX097-2

Compound **WX087-3** (0.3 g, 816.55 µmol) was dissolved in pyridine (5 mL), compound **WX097-1** (199.74 mg, 816.55 µmol, 130.55 µL) was added thereto, and the reaction solution was stirred at 25 °C for 10 hours. After the reaction was completed, the reaction solution was rotary-evaporated, and separated by preparative HPLC (TFA) to obtain target compound **WX097-2.** MS-ESI *m*/*z*: 576.0 [M+H]⁺.

### Step 2: synthesis of compound WX097 and WX098

Compound **WX097-2** was resolved by supercritical fluid chromatography (separation conditions: column: AD (250mm * 30mm, 10µm); mobile phase: [0.1% NH₄HCO₃ EtOH]; B%: 55% -55%, flow rate (ml/min) : 80mL/min) to obtain enantiomers **WX097** (retention time is 0.567 min) and **WX098** (retention time is 1.348 min). **WX097:** ¹H NMR (400 MHz, DMSO-*d*₆) δ = 8.38 (s, 1 H), 8.28 (d, *J* = 2.3 Hz, 1H), 8.19 (s, 1 H), 8.01-8.12 (m, 3 H), 7.94-8.01 (m, 2 H), 7.89 (br d, *J =* 4.8 Hz, 1H), 7.80 (t, *J =* 7.5 Hz, 1 H), 7.76 (d, *J* = 8.5 Hz, 1 H), 4.02-4.12 (m, 1 H), 3.97 (dd, *J =* 13.2, 9.2 Hz, 1 H), 3.59 (s, 3 H), 2.87 (br dd, *J* = 15.1, 7.0 Hz, 1 H), 2.48 (br s, 3 H), 1.08 (d, *J* = 7.0 Hz, 3 H). MS-ESI *m*/*z*: 576.0 [M+H]⁺. **WX098:** ¹H NMR (400 MHz, DMSO-*d*₆) δ = 8.41 (d, *J =* 2.3 Hz, 1 H), 8.28 (d, *J* = 2.0 Hz, 1 H), 8.20 (s, 1 H), 8.08 (s, 3 H), 7.99 (br d, *J =* 8.0 Hz, 1 H), 7.95-7.97 (m, 1 H), 7.87-7.95 (m, 1 H), 7.78-7.84 (m, 1 H), 7.76 (d, *J* = 8.1 Hz, 1 H), 4.08 (dd, *J* = 13.2, 5.6 Hz, 1 H), 3.93-4.02 (m, 1H), 3.59 (s, 3 H), 2.82-2.91 (m, 1 H), 2.48 (br s, 3 H), 1.08 (d, *J* = 6.8 Hz, 3 H). MS-ESI *m*/*z:* 576.0 [M+H]⁺. The ratio of two isomers is 1:1.

### Example 57: WX099, WX100

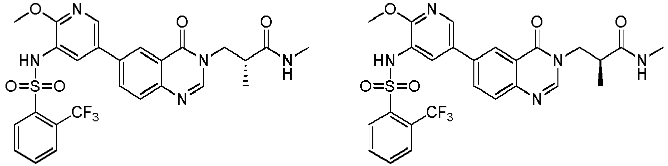

### Synthetic route:

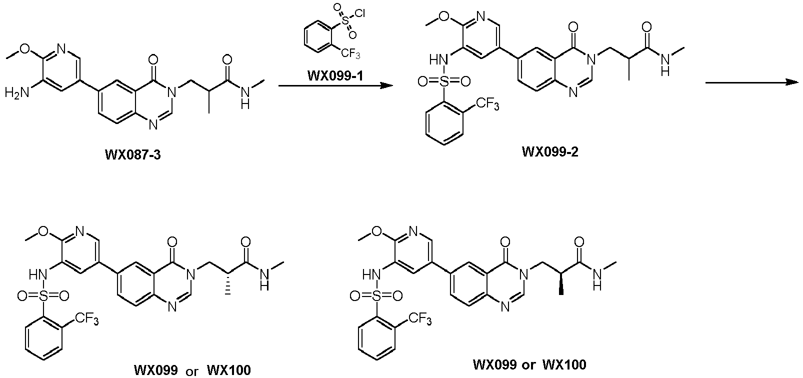

### Step 1: synthesis of compound WX099-2

Compound **WX087-3** (0.3 g, 816.55 µmol) was dissolved in pyridine (5 mL), compound **WX099-1** (199.74 mg, 816.55 µmol, 125.62 µL) was added thereto, and the reaction solution was stirred at 25 °C for 10 hours. After the reaction was completed, the reaction solution was rotary-evaporated, and separatedby preparative HPLC (TFA) to obtain target compound **WX099-2.** MS-ESI *m*/*z*: 576.0 [M+H]⁺.

### Step 2: synthesis of compound WX099 and WX100

Compound **WX099-2** was resolved by supercritical fluid chromatography (separation conditions: column: AD (250mm * 30mm, 10µm); mobile phase: [0.1% NH₄HCO₃ EtOH]; B%: 55% -55%, flow rate (mL/min): 80mL/min) to obtain enantiomers **WX099** (retention time is 0.583 min) and **WX100** (retention time is 1.365 min). **WX099:** ¹H NMR (400 MHz, DMSO-*d*₆) δ = 8.36 (br s, 1 H), 8.23 (s, 1 H), 8.18 (s, 1 H), 8.01-8.08 (m, 2 H), 7.99 (br d, *J =* 3.8 Hz, 1 H), 7.86-7.92 (m, 2 H), 7.81-7.86 (m, 2 H), 7.75 (d, *J =* 8.5 Hz, 1 H), 4.02-4.11 (m, 1H), 3.96 (dd, *J =* 13.3, 9.3 Hz, 1 H), 3.64 (s, 3 H), 2.81-2.91 (m, 1 H), 2.48 (br s, 3 H), 1.08 (d, *J =* 7.0 Hz, 3 H). MS-ESI m/z: 576.0 [M+H]⁺. **WX100:** ¹H NMR (400 MHz, DMSO-*d*₆) δ = 8.39 (d, *J* = 2.3 Hz, 1H), 8.24 (d, *J* = 2.0 Hz, 1H), 8.19 (s, 1 H), 7.99-8.09 (m, 3 H), 7.91 (br d, *J =* 2.3 Hz, 2 H), 7.82-7.87 (m, 2 H), 7.76 (d, *J* = 8.5 Hz, 1 H), 4.01-4.10 (m, 1 H), 3.96 (dd, *J* = 13.2, 8.9 Hz, 1 H), 3.63 (s, 3 H), 2.86 (br dd, *J* = 14.9, 6.4 Hz, 1H), 2.48 (br s, 3 H), 1.08 (d, *J* = 7.0 Hz, 3 H). The ratio of two isomers is 1:1.

### Example 58: WX101, WX102

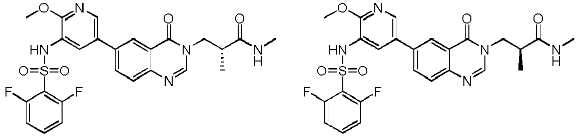

### Synthetic route:

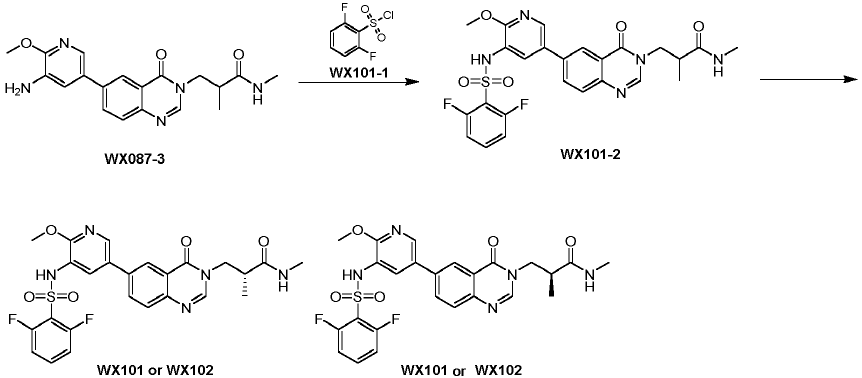

### Step 1: synthesis of compound WX101-2

Compound **WX087-3** (0.3 g, 816.55 µmol) was dissolved in pyridine (5 mL). Compound **WX101-1** (173.60 mg, 816.55 µmol, 110.57 µL) was added thereto, and the reaction solution was stirred at 25 °C for 10 hours. After the reaction was completed, the reaction solution was rotary-evaporated, and separated by preparative HPLC separation (TFA) to obtain target compound **WX101-2.** MS-ESI *m*/*z:* 544.0 [M+H]⁺.

### Step 2: synthesis of compound WX101 and WX102

Compound **WX101-2** was resolved by supercritical fluid chromatography (separation conditions: chromatographic column: AD (250mm * 30mm, 10µm); mobile phase: [0.1% NH₄HCO₃ ETOH]; B%: 55% -55%, flow rate (mL/min) : 80mL/min) to obtain enantiomers **WX101** (retention time is 0.841 min) and **WX102** (retention time is 2.518 min). **WX101:** ¹HNMR (400 MHz, DMSO-*d*₆) δ = 8.21-8.36 (m, 2 H), 8.18 (s, 1 H), 8.06 (br d, *J =* 7.0 Hz, 1 H), 7.90 (br d, *J =* 4.3 Hz, 2 H), 7.75 (d, *J =* 8.3 Hz, 1 H), 7.62-7.70 (m, 1 H), 7.24 (br t, *J* = 9.0 Hz, 2 H), 4.01-4.15 (m, 1 H), 3.96 (dd, *J* = 13.3, 9.3 Hz, 1 H), 3.67 (s, 3 H), 2.81-2.91 (m, 1 H), 2.48 (br s, 3 H), 1.08 (d, *J =* 7.0 Hz, 3 H). MS-ESI *m*/*z*: 544.0 [M+H]⁺. **WX102:** ¹H NMR (400 MHz, DMSO-*d*₆) δ = 8.25 (d, *J =* 2.0 Hz, 2 H), 8.18 (s, 1H), 8.07 (br d, *J =* 8.3 Hz, 1H), 7.86-7.95 (m, 2 H), 7.76 (d, *J* = 8.5 Hz, 1H), 7.64-7.73 (m, 1H), 7.25 (br t, *J* = 9.2 Hz, 2 H), 4.02-4.11 (m, 1 H), 3.96 (dd, *J* = 13.2, 9.2 Hz, 1 H), 3.66 (s, 3 H), 2.87 (br dd, *J* = 15.1, 7.0 Hz, 1 H), 2.48 (br s, 3 H), 1.08 (d, *J* = 7.0 Hz, 3 H). MS-ESI *m*/*z:* 544.0 [M+H]⁺. The ratio of two isomers is 1:1.

### Example 59: WX103, WX104

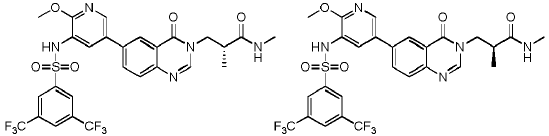

### Synthetic route:

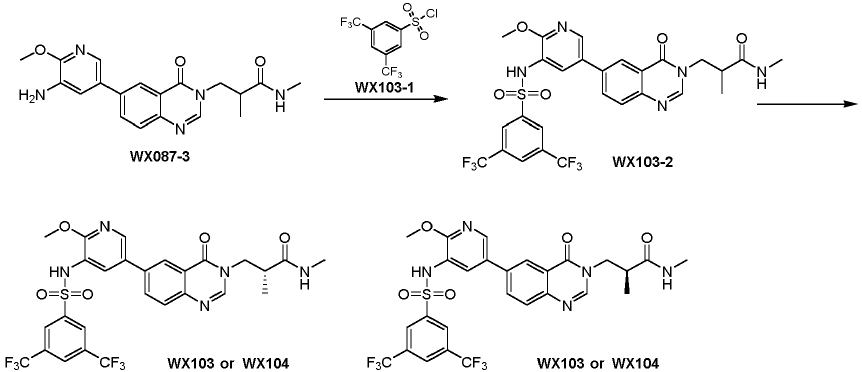

### Step 1: synthesis of compound WX103-2

Compound **WX087-3** (0.3 g, 816.55 µmol) was dissolved in pyridine (5 mL), compound **WX103-1** (255.27 mg, 816.55 µmol) was added thereto, and the reaction solution was stirred at 25 °C for 10 hours. After the reaction was completed, the reaction solution was rotary-evaporated, and separated by preparative HPLC separation (TFA) to obtain target compound **WX103-2.** MS-ESI *m*/*z*: 644.1 [M+H]⁺.

### Step 2: synthesis of compound WX103 and WX104

Compound **WX103-2** was resolved by supercritical fluid chromatography (separation conditions: column: C2 250mm * 30mm, 10µm; mobile phase: [0.1% NH₄HCO₃ MeOH]; B%: 40% -40%, flow rate (mL/min): 80mL/min) to obtain enantiomers **WX103** (retention time is 5.201 min) and **WX104** (retention time is 6.417min). **WX103:** ¹H NMR (400 MHz, DMSO-*d*₆) δ = 8.39 (br s, 1 H), 8.29 (s, 2 H), 8.22-8.28 (m, 2 H), 8.18 (s, 1 H), 8.04 (dd, *J* = 8.5, 2.0 Hz, 1 H), 7.87-7.94 (m, 2 H), 7.74 (d, *J* = 8.5 Hz, 1 H), 4.02-4.12 (m, 1 H), 3.96 (dd, *J* = 13.2, 9.2 Hz, 1 H), 3.62 (s, 3 H), 2.82-2.91 (m, 1 H), 2.48 (br s, 3 H), 1.08 (d, *J* = 6.8 Hz, 3 H). MS-ESI *m*/*z*: 644.1 [M+H]⁺. **WX104** : ¹H NMR (400 MHz, DMSO-*d*₆) δ = 8.47 (s, 1 H), 8.36 (br s, 1 H), 8.30 (s, 3 H), 8.19 (s, 1 H), 8.08 (br d, *J =* 8.5 Hz, 1 H), 7.98 (s, 1 H), 7.89 (br d, *J* = 4.5 Hz, 1 H), 7.75 (d, *J* = 8.5 Hz, 1 H), 4.03-4.17 (m, 1 H), 3.97 (dd, *J* = 13.2, 9.2 Hz, 1 H), 3.58 (s, 3 H), 2.82-2.91 (m, 1 H), 2.52 (br s, 3 H), 1.08 (d, *J* = 7.0 Hz, 3 H). MS-ESI *m*/*z:* 644.1 [M+H]⁺. The ratio of two isomers is 1:1.

### Example 60: WX105, WX106

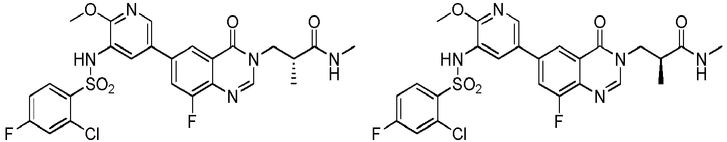

### Synthetic route:

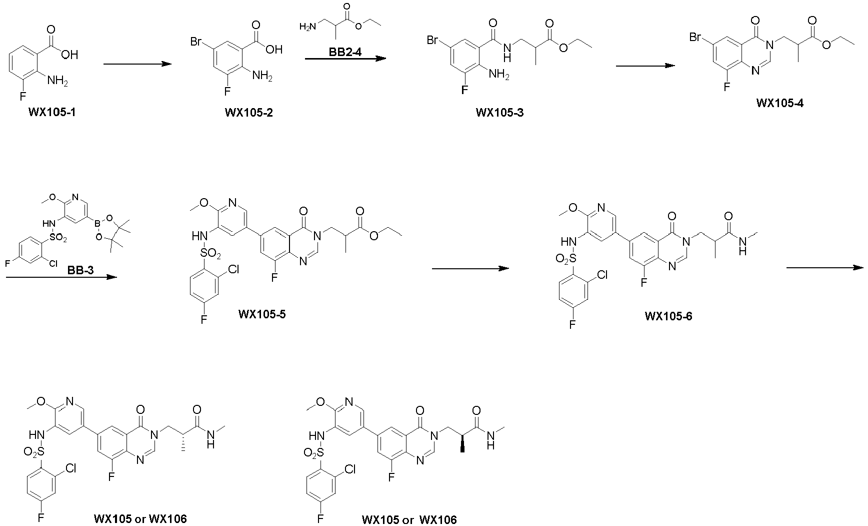

### Step 1: synthesis of compound WX105-2

Compound **WX105-1** (4 g, 25.79 mmol), NBS (4.59 g, 25.79 mmol) was added to DCM (50 mL), and stirred at 25 °C for 2 hours. After the reaction was completed. The reaction solution was filtered, the filter cake was washed with DCM (50 mL), and the filter cake was rotary-evaporated to obtain compound **WX105-2.** ¹H NMR (400 MHz, DMSO-*d*₆) δ ppm 7.63-7.66 (m, 2 H), 7.54 (d, *J =* 2.5 Hz, 1 H), 7.51 (d, *J* = 2.0 Hz, 1H).

### Step 2: synthesis of compound WX105-3

Compound **WX105-2** (1.00 g, 4.27 mmol), compound **BB2-4** (0.84 g, 6.40 mmol), EDCI (0.83 g, 4.33 mmol), TEA (1.73 g, 17.09 mmol) and HOPO (0.55 g, 4.95 mmol) were added to DCM (50 mL) and stirred at 50 °C for 16 hours. After the reaction was completed. The reaction solution was rotary-evaporated, the residue was diluted with water (100 mL), extracted with DCM (100 mL), the organic phase was rotary-evaporated, and the resultant was separated by a chromatography column (ethyl acetate: petroleum ether = 0% ∼ 10%) to obtain compound **WX105-3.** ¹H NMR (400 MHz, DMSO-*d*₆) δ ppm 8.59 (t, *J =* 5.5 Hz, 1 H), 7.54 (d, *J =* 1.5 Hz, 1 H), 7.44 (dd, *J* = 10.8, 2.3 Hz, 1 H), 6.44 (s, 2 H), 4.03-4.08 (m, 1 H), 4.06 (br s, 1 H), 3.38-3.47 (m, 1 H), 3.22-3.31 (m, 1 H), 2.69-2.77 (m, 1 H), 1.15-1.19 (m, 3 H), 1.09 (d, *J* = 7.0 Hz, 3 H).

### Step 3: synthesis of compound WX105-4

Compound **WX105-3** (0.6 g, 1.73 mmol) was added to formic acid (12.20 g, 265.07 mmol, 10 mL), and stirred at 100 °C for 16 hours. After the reaction was completed. The reaction solution was rotary-evaporated, diluted with water (50 mL), extracted with DCM (50 mL), and the organic phase was rotary-evaporated to obtain compound **WX105-4.** MS-ESI *m*/*z:* 359.0 [M+H]⁺.

### Step 4: synthesis of compound WX105-5

Compound **WX105-4** (0.6 g, 1.34 mmol), **BB-3** (0.63 g, 1.34 mmol), Pd(dppf)Cl₂ (0.098 g, 133.93 µmol) and KOAc (0.527 g, 5.37 mmol) were added to dioxane (8 mL) and water (1.6 mL), the system was replaced with nitrogen 3 times, and then stirred at 100 °C for 1 hour under nitrogen atmosphere. After the reaction was completed, the reaction solution was rotary-evaporated, diluted with water (50 mL), and extracted with DCM (50 mL). The organic phase was rotary-evaporated, and the resultant was separated by chromatography column (methanol: dichloromethane = 0% ∼ 3%) to obtain compound **WX105-5.** MS-ESI *m*/*z*: 593.0 [M+H]⁺.

### Step 5: synthesis of compound WX105-6

Compound **WX105-5** (0.4 g, 635.75 µmol) was added to a methylamine solution (78.98 mg, 635.75 µmol, 10 mL), and stirred at 80 °C for 16 hours. After the reaction was completed, the reaction solution was rotary-evaporated and purified by HPLC (Phenomenex Gemini C18 250 * 50mm * 10 µm; mobile phase: [water (0.05% ammonium hydroxide v/v) -ACN]; B%: 30%-40%, 8min) to obtain compound **WX105-6.**

### Step 6: synthesis of compound WX105 and WX106

**WX105-6** was resolved by SFC (colµmn: AD (250mm * 30mm, 10µm); mobile phase: [0.1% NH₄HCO₃ EtOH]; B%: 55% -55%) to obtain compound **WX105** (Rt = 5.372min) and **WX106** (Rt = 6.218min). **WX105:** ¹H NMR (400 MHz, METHANOL-*d*₄) δ ppm 8.13 (d, *J* = 2.0 Hz, 1 H), 8.10 (s, 1 H), 7.97-8.05 (m, 2 H), 7.88 (d, *J* = 2.5 Hz, 1H), 7.73 (br d, *J* = 11.5 Hz, 1H), 7.37 (dd, *J* = 8.5, 2.5 Hz, 1H), 7.14 (td, *J* = 8.3, 2.5 Hz, 1 H), 4.14 (dd, *J* = 13.6, 5.0 Hz, 1 H), 3.95 (dd, *J* = 13.3, 9.8 Hz, 1 H), 3.77 (s, 3 H), 2.85-2.95 (m, 1 H), 2.53 (s, 3 H), 1.14 (d, *J =* 7.0 Hz, 3 H). **WX106:** ¹H NMR (400 MHz, METHANOL-*d*₄) δ ppm 8.07-8.13 (m, 2 H), 7.95-8.03 (m, 2 H), 7.86 (d, *J* = 2.0 Hz, 1H), 7.70 (dd, *J* = 11.3, 1.8 Hz, 1H), 7.37 (dd, *J* = 8.5, 2.5 Hz, 1H), 7.14 (td, *J =* 8.3, 2.5 Hz, 1H), 4.14 (dd, *J =* 13.6, 5.0 Hz, 1 H), 3.90-4.02 (m, 1 H), 3.76 (s, 3 H), 2.82-2.97 (m, 1 H), 2.53 (s, 3 H), 1.14 (d, *J* =7.0 Hz, 3 H).

### Example 61: WX107, WX108

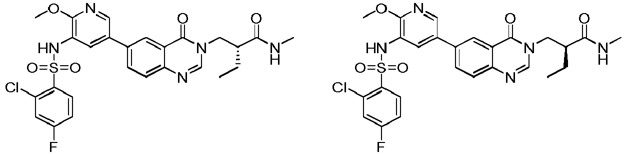

### Synthetic route:

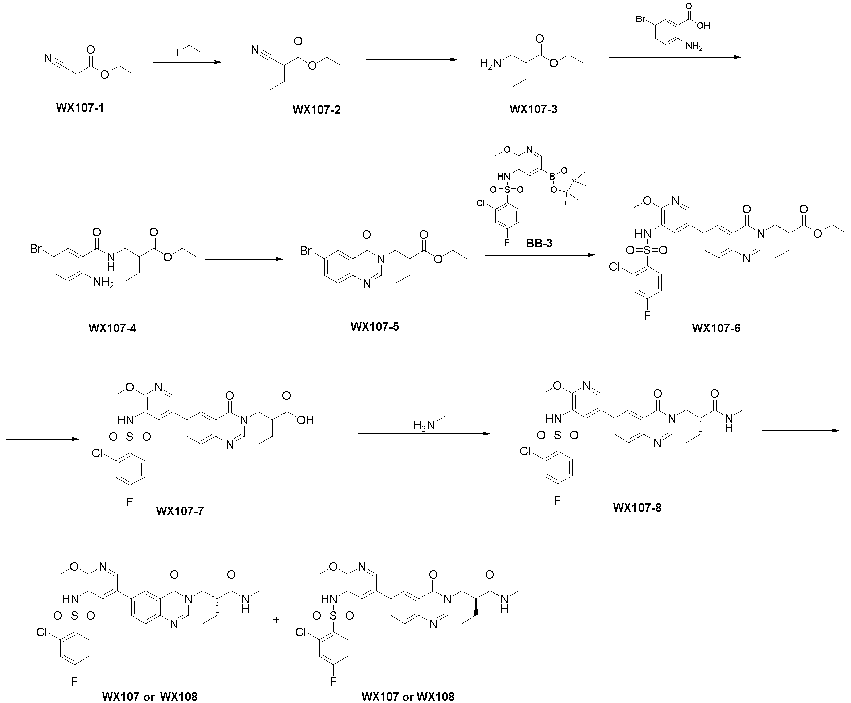

### Step 1: synthesis of compound WX107-2

Compound **WX107-1** (4 g, 31.46 mmol) and potassium carbonate (8.70 g, 62.92 mmol) were dissolved in DMF (5 mL), and ethyl iodide (4.91 g, 31.46 mmol) was added at 0 °C, and stirred at 25 °C overnight. After the reaction was completed, the mixture was poured into water (100 mL) and extracted with dichloromethane (100 mL × 3). The organic phases obtained were combined and dried over anhydrous sodium sulfate. After the desiccant was removed by filtration, the solvent was removed under reduced pressure, and purified by column chromatography (ethyl acetate / petroleum ether = 0-10%) to obtain compound **WX107-2.** ¹H NMR (400MHz, Methanol-*d₄*) δ: 4.36-4.17 (m, 2H), 4.12 (q, *J* = 7.1 Hz, 1H), 2.03-1.86 (m, 2H), 1.39-1.27 (m, 3H), 1.15-1.04 (m, 3H).

### Step 2: synthesis of compound WX107-3

Compound **WX107-2** (2.0 g, 14.17 mmol) and concentrated hydrochloric acid (1.08 mL) were dissolved in methanol (25 mL), followed by addition of Raney Ni (242.75 mg, 2.83 mmol) under nitrogen atmosphere, and introduction of hydrogen (50 psi) was added, and stirred at 30 °C overnight. After the reaction was completed, the solvent was removed under reduced pressure to obtain compound **WX107-3.** ¹H NMR (400MHz, Methanol-*d₄*) δ: 4.29-4.05 (m, 2H), 2.91-2.66 (m, 2H), 2.49-2.06 (m, 1H), 1.69-1.51 (m, 2H), 1.33-1.22 (m, 3H), 1.05-0.90 (m, 3H).

### Step 3: synthesis of compound WX107-4

2-Amino-5-bromobenzoic acid (0.5 g, 2.31 mmol) was dissolved in DMF (10 mL), followed by addition of DIEA (298.54 mg, 2.31 mmol), HATU (878.33 mg, 2.31 mmol) and **WX107-3** (419.63 mg, 2.31 mmol), and stirred at 25 °C for 2 hours. After the reaction was completed, the mixture was poured into water (10 mL) and extracted with ethyl acetate (10 mL × 3). The organic phases obtained were combined and dried over anhydrous sodium sulfate. After the desiccant was removed by filtration, the solvent was removed under reduced pressure, and subjected to column chromatography (eluent: ethyl acetate / petroleum ether = 0-20%) to obtain the target compound **WX107-4.** ¹HNMR (400MHz, Methanol-*d₄*) δ: 7.65-7.38 (m, 1H), 7.27 (dd, *J* = 2.3, 8.8 Hz, 1H), 6.69 (d, *J =* 8.8 Hz, 1H), 4.18 (q, *J =* 7.3 Hz, 2H), 3.54-3.40 (m, 2H), 2.83-2.54 (m, 1H), 1.80-1.45 (m, 2H), 1.36-1.21 (m, 3H), 1.05-0.91 (m, 3H). MS-ESI m/z: 344.9[M+H]⁺, 346.9[M+H+2]⁺.

### Step 4: synthesis of compound WX107-5

Compound **WX107-4** (0.4 g, 1.17 mmol) was dissolved in ethanol (80 mL), followed by addition of formamidine acetate (364.00 mg, 3.50 mmol), and stirred at 80 °C for 2 hours. After the reaction was completed, the mixture was rotary-evaporated to remove the organic solvent, poured into water (20 mL), and extracted with dichloromethane (20 mL × 3). The organic phases obtained were combined and dried over anhydrous sodium sulfate. After the desiccant was removed by filtration, the solvent was removed under reduced pressure to obtain the target compound **WX107-5,** which was directly used in the next step. MS-ESI m/z: 352.9[M+H]⁺, 354.9[M+H+2]⁺.

### Step 5: synthesis of compound WX107-6

Compound **WX107-5** (0.25 g, 707.79 µmol), **BB-2** (313.34 mg, 707.79 µmol) and potassium acetate (277.85 mg, 2.83 mmol) were dissolved in dioxane (2 mL) and water (0.2 mL), followed by addition of Pd(dppf)Cl₂ (10.36 mg, 14.16 µmol). The mixture was heated to 95 °C and stirred for 2 hours under nitrogen atmosphere. After the reaction was completed, the mixture was cooled to room temperature, rotary-evaporated to remove the organic solvent, poured into water (20 mL) and extracted with dichloromethane (20 mL × 3). The organic phases obtained were combined and dried over anhydrous sodium sulfate. After the desiccant was removed by filtration, the solvent was removed under reduced pressure, and separated by preparative chromatographic plate (methanol / dichloromethane = 1:30) to obtain target compound **WX107-6.** MS-ESI m/z: 589.1[M+H]⁺, 591.1[M+H+2]⁺.

### Step 6: synthesis of compound WX107-7

**WX107-6** (0.1 g, 178.26 µmol) was dissolved in methylamine alcohol solution (20 mL), heated to 80 °C and stirred overnight. After the reaction was completed, the mixture was cooled to room temperature. The solvent was removed under reduced pressure, and separated by a chromatographic plate (methanol/dichloromethane/triethylamine = 1:20:0.2) to obtain target compound **WX107-7.** MS-ESI m/z: 561.0[M+H]⁺, 563.0[M+H+2]⁺.

### Step 7: synthesis of compound WX107-8

Compound **WX107-7** (0.1 g, 178.26 µmol) was dissolved in DMF (2 mL), then TEA (36.08 mg, 356.52 µmol), HATU (67.78 mg, 178.26 µmol) and methylamine hydrochloride (12.04 mg, 178.26 µmol) were added, and stirred for 2 hours at 30 °C. After the reaction was completed, the solvent was removed under reduced pressure. The residue was poured into water (10 mL), and extracted with dichloromethane (10 mL × 3). The organic phases were combined and dried over anhydrous sodium sulfate. After the desiccant was removed by filtration, the solvent was removed under reduced pressure, separated by preparative chromatographic plate (eluent: methanol/dichloromethane = 1:20), and further separated by preparative high-performance liquid column (AD (250mm * 30mm, 10µm); mobile phase: [0.1% NH₄HCO₃ ETOH]; B%: 55% -55%, min) to obtain target compound **WX107-8.**

### Step 8: synthesis of compound WX107, WX108

Compound **WX107-8** was resolved by supercritical fluid chromatography (chromatographic column: AD (250mm * 30mm, 10µm); mobile phase: [0.1% NH₄HCO₃ EtOH]; B%: 55% -55%) to obtain enantiomers **WX107** and **WX108,** the retention time of which is 0.729min, 1.837min, respectively, and the ratio is 1:1. **WX107:** ¹H NMR (400MHz, CDCl₃) δ: 8.21 (d, *J* = 2.0 Hz, 1H), 8.11-8.02 (m, 2H), 8.01 (s, 1H), 7.90 (d, *J* = 2.3 Hz, 1H), 7.82-7.73 (m, 1H), 7.73-7.60 (m, 1H), 7.22-7.19 (m, 1H), 7.11-6.97 (m, 1H), 5.55 (br d, *J* = 4.5 Hz, 1H), 4.21 (dd, *J* = 4.5, 13.3 Hz, 1H), 4.00-3.87 (m, 4H), 2.67 (m, 4H), 1.76-1.63 (m, 1H), 1.63-1.47 (m, 3H), 0.94 (t, *J* = 7.4 Hz, 3H). MS-ESI m/z:574.1[M+H]⁺,576.1[M+H+2]⁺. **WX108:** ¹H NMR (400MHz, CDCl₃) δ: 8.20 (d, *J* = 1.8 Hz, 1H), 8.10-8.01 (m, 2H), 8.01 (s, 1H), 7.90 (d, *J* = 2.3 Hz, 1H), 7.79-7.70 (m, 1H), 7.70-7.65 (m, 1H), 7.22-7.20 (m, 1H), 7.10-7.00 (m, 1H), 5.58 (br d, *J* = 4.5 Hz, 1H), 4.21 (dd, *J* = 4.5, 13.1 Hz, 1H), 3.96-3.85 (m, 4H), 2.67 (d, *J* = 5.0 Hz, 3H), 2.84-2.55 (m, 1H), 1.76-1.63 (m, 1H), 1.75-1.48 (m, 1H), 1.63-1.47 (m, 2H), 0.94 (t, *J* = 7.4 Hz, 3H). MS-ESI m/z: 574.1[M+H]⁺, 576.1[M+H+2]⁺.

### Example 62: WX109, WX110, WX111, WX112

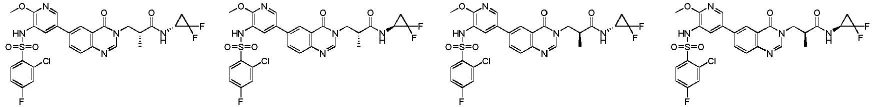

### Synthetic route:

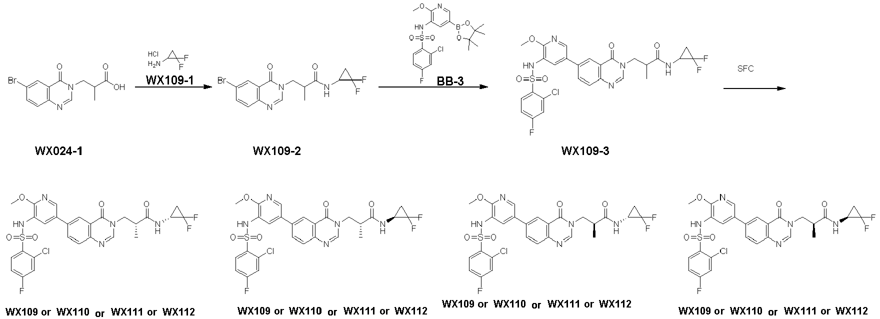

### Step 1: synthesis of compound WX109-2

**WX024-1** (380 mg, 1.22 mmol, 1 eq) and **WX109-1** (174.03 mg, 1.34 mmol, 1.1 eq, HCl) were added to a pre-dried 40 mL reaction flask, followed by addition of the solvent dichloromethane (10 mL) for dissolution. The reaction solution was cooled to 0 °C, followed by addition of propylphosphonic anhydride (1.17 g, 1.83 mmol, 1.09 mL, 50% purity, 1.5 eq) and diisopropylethylamine (473.54 mg, 3.66 mmol, 638.20 µL, 3 eq). The reaction solution was stirred at 20 °C for 12 hours. After the reaction was completed, water (10 mL) and ethyl acetate (10 mL) were added to the reaction system for dilution. The organic phase was collected after liquid separation, and the aqueous phase was extracted with ethyl acetate (5 mL × 3). The organic phases were combined, washed with saturated brine (20 mL), dried over anhydrous sodium sulfate, and concentrated under reduced pressure to obtain a residue. The crude product was purified by thin layer chromatography silica gel plate (dichloromethane: methanol = 20: 1) to obtain target compound **WX109-2.**

### Step 2: synthesis of compound WX109-3

**WX109-2** (220 mg, 569.67 µmol, 1 eq), **BB-3** (302.63 mg, 683.60 µmol, 1.2 eq) and potassium acetate (167.72 mg, 1.71 mmol, 3 eq) were added into a pre-dried 40 mL reaction flask, followed by addition of dioxane (6 mL) and water (0.5 mL). The mixture was replaced with nitrogen, followed by addition of [1,1'-bis(diphenylphosphino) ferrocene] palladium dichloride (41.68 mg, 56.97 µmol, 0.1 eq), and replaced with nitrogen again. The reaction was solution stirred at 90 °C for 12 hours. After the reaction was completed, water (10 mL) and ethyl acetate (10 mL) were added to the reaction system for dilution. The organic phase was collected after liquid separation, and the aqueous phase was extracted with ethyl acetate (5 mL × 3). The organic phases were combined, washed with saturated brine (20 mL), dried over anhydrous sodium sulfate, and concentrated under reduced pressure to obtain a residue. The crude product was subjected to thin layer chromatography silica gel plate (dichloromethane: methanol = 20: 1) to obtain target compound **WX109-3.**

### Step 3: synthesis of compound WX109, WX110, WX111, WX112

**WX109-3** was resolved by SFC (resolution column: Chiralpak IC-H 250 * 30mm 5µm; mobile phase: [MeOH]; B%: 45%-45%, 9min) to obtain target compounds **WX109** (retention time is 2.18 min) and **WX110** (retention time is 3.28 min). **WX109:** ¹H NMR (400MHz, CHLOROFORM-d) δ = 8.29 (s, 1H), 8.17-8.08 (m, 3H), 7.98 (d, *J =* 2.0 Hz, 1H), 7.87-7.83 (m, 1H), 7.82-7.75 (m, 1H), 7.58 (s, 1H), 7.29-7.25 (m, 1H), 7.17-7.08 (m, 1H), 6.00 (br s, 1H), 4.21 (br dd, *J =* 4.8, 13.2 Hz, 1H), 4.10-4.03 (m, 1H), 4.00 (s, 3H), 3.30 (br s, 1H), 3.12-3.01 (m, 1H), 1.83-1.74 (m, 1H), 1.34 (br d, *J=* 6.9 Hz, 3H), 1.23-1.13 (m, 1H), 1.17 (ddd, *J* = 4.8, 9.3, 14.0 Hz, 1H). **WX110:** ¹H NMR (400MHz, CHLOROFORM-d) δ = 8.32-8.24 (m, 1H), 8.15-8.07 (m, 3H), 7.96 (d, *J =* 2.2 Hz, 1H), 7.85-7.81 (m, 1H), 7.79-7.75 (m, 1H), 7.54 (s, 1H), 7.25-7.23 (m, 1H), 7.14-7.07 (m, 1H), 6.01 (br s, 1H), 4.16-4.10 (m, 1H), 4.09-4.01 (m, 1H), 3.96 (s, 3H), 3.32-3.19 (m, 1H), 3.09-2.98 (m, 1H), 1.91-1.69 (m, 1H), 1.31-1.24 (m, 3H), 1.30-1.24 (m, 1H). The rest was collected and resolved by SFC again (OJ (250mm * 30mm, 5µm); mobile phase: [0.1% NH₄HCO₃ MEOH]; B%: 35% -35%, 4min) to obtain **WX111** (retention time is 2.38 min) and **WX112** (retention time is 2.60 min). **WX111:** ¹H NMR (400MHz, CHLOROFORM-d) δ = 8.27 (d, *J* = 2.2 Hz, 1H), 8.15-8.08 (m, 2H), 8.06 (s, 1H), 7.95 (d, *J =* 2.4 Hz, 1H), 7.84-7.80 (m, 1H), 7.78-7.73 (m, 1H), 7.54 (s, 1H), 7.26-7.24 (m, 1H), 7.10 (ddd, *J* = 2.4, 7.5, 8.8 Hz, 1H), 5.94 (br s, 1H), 4.17 (dd, *J* = 5.1, 13.2 Hz, 1H), 4.09-3.99 (m, 1H), 3.98-3.96 (m, 3H), 3.33-3.20 (m, 1H), 3.08-2.96 (m, 1H), 1.73-1.67 (m, 1H), 1.30 (d, *J* = 6.8 Hz, 3H), 1.19-1.07 (m, 1H), 1.12 (dq, *J* = 5.2, 9.6 Hz, 1H). **WX112:** ¹H NMR (400MHz, CHLOROFORM-d) δ = 8.32-8.24 (m, 1H), 8.15-8.07 (m, 3H), 7.96 (d, *J* = 2.2Hz, 1H), 7.85-7.81 (m, 1H), 7.79-7.75 (m, 1H), 7.54 (s, 1H), 7.25-7.23 (m, 1H), 7.14-7.07 (m, 1H), 6.01 (br s, 1H), 4.16-4.10 (m, 1H), 4.09-4.01 (m, 1H), 3.96 (s, 3H), 3.32-3.19 (m, 1H), 3.09-2.98 (m, 1H), 1.91-1.69 (m, 1H), 1.31-1.24 (m, 3H), 1.30-1.24 (m, 1H).

### Example 63: WX113, WX114

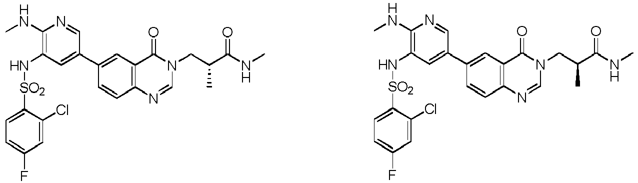

### Synthetic route:

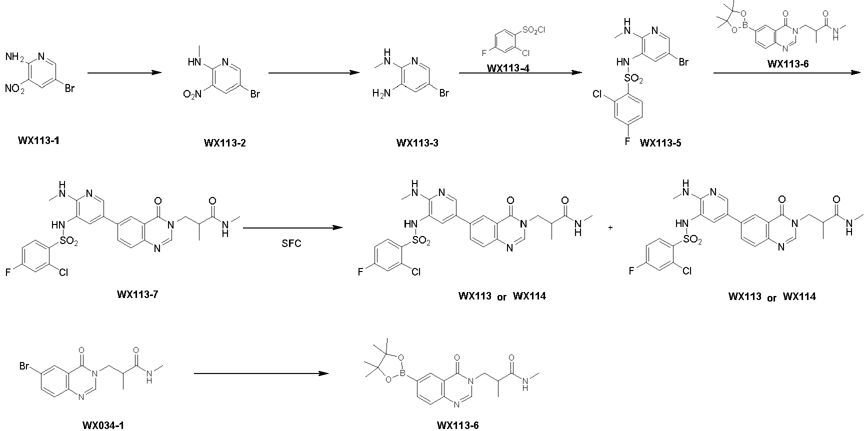

### Step 1: synthesis of compound WX113-2

Raw material **WX113-1** (5 g, 22.93 mmol) and solvent *N,N-*dimethylformamide (30 mL) were added to a pre-dried three-neck flask, and cooled to 0 °C, followed by addition of sodium hydrogen (605.42 mg, 25.23 mmol). The mixture was stirred for 30 minutes, followed by addition of methyl iodide (3.58 g, 25.23 mmol, 1.57 mL), and stirred for another 30 minutes. After thin-layer chromatography detection (petroleum ether: ethyl acetate = 5:1) showed the completion of the reaction, water (20mL) and dichloromethane (20mL * 3) were added to the reaction solution for extraction. The resulting organic phase was dried over anhydrous sodium sulfate and rotary-evaporated under reduced pressure by a water pump to obtain a crude product. The crude product was separated and purified by preparative thin layer chromatography (petroleum ether: ethyl acetate = 4: 1) to obtain target product **WX113-2.** ¹H NMR (400MHz, CHLOROFORM-d) δ = 8.55 (d, *J* = 2.2 Hz, 1H), 8.47 (d, *J* = 2.2 Hz, 1H), 3.17 (d, *J =* 4.9 Hz, 4H), 3.20-3.15 (m, 1H).

### Step 2: synthesis of compound WX113-3

Raw materials **WX113-2** (3.1 g, 13.36 mmol) and ammonium chloride (8.58 g, 160.32 mmol, 5.61 mL), and the solvents methanol (30 mL) and water (10 mL) were added into a pre-dried single-necked flask, followed by addition of zinc powder (6.12 g, 93.52 mmol), and stirred at 25 °C for 2 hours. TLC detection (petroleum ether: ethyl acetate = 1: 1) showed that the raw materials disappeared and a new product was formed. Water (10 mL) and dichloromethane (20 mL × 2) were added to the reaction solution for extraction. The resulting organic phase was dried over anhydrous sodium sulfate, and rotary-evaporated under reduced pressure by a water pump to obtain a crude product. The crude product was purified by prep-TLC (petroleum ether: ethyl acetate = 1:1) to obtain product **WX113-3.** ¹H NMR (400MHz, CHLOROFORM-d) δ = 7.81 (d, *J =* 2.0 Hz, 1H), 6.96 (d, *J =* 2.2 Hz, 1H), 3.24 (br s, 2H), 2.99 (s, 4H).

### Step 3: synthesis of compound WX113-5

Raw materials **WX113-3** (2.2 g, 10.89 mmol) and **WX113-4** (2.49 g, 10.89 mmol, 1.59 mL) and the solvent pyridine (15 mL) were added into a pre-dried reaction flask, and stirred at 25 °C for 12 hours. TLC detection (petroleum ether: ethyl acetate = 3:1) showed that the raw materials disappeared and a new product was formed. Water (10 mL) and dichloromethane (15 mL * 3) was added to the reaction solution, and the resulting organic phase was dried over anhydrous sodium sulfate, and rotary-evaporated under reduced pressure by a water pump to obtain a crude product. The crude product was purified by prep-TLC (petroleum ether: ethyl acetate = 3: 1) to obtain target product **WX113-5.** ¹H NMR (400MHz, CHLOROFORM-d) δ = 8.08 (d, *J* = 2.4 Hz, 1H), 7.97 (dd, *J =* 5.7, 9.0 Hz, 1H), 7.40 (dd, *J =* 2.4, 7.9 Hz, 1H), 7.13 (ddd, *J* = 2.4, 7.4, 8.9 Hz, 1H), 6.85 (d, *J* = 2.2 Hz, 1H), 6.39 (br s, 1H), 5.45 (br s, 1H), 2.98 (s, 4H), 3.00-2.96 (m, 1H), 3.00-2.96 (m, 1H), 3.00-2.96 (m, 1H).

### Step 4: synthesis of compound WX113-6

Raw material **WX034-1** (1.33 g, 4.10 mmol) and the solvent 1,4-dioxane (13 mL) were added into a pre-dried reaction flask, followed by addition of potassium acetate (805.31 mg, 8.21 mmol) and bis(pinacolato)diboron (1.15 g, 4.51 mmol). The mixture was replaced with nitrogen, followed by addition of 1,1-bis(diphenylphosphine) ferrocene palladium chloride (300.20 mg, 410.28 µmol), and replaced with nitrogen. The mixture was stirred at 100 °C for 3 hours. After the reaction was completed, the target product **WX113-6** was obtained without post-treatment, which was directly used the next reaction.

### Step 5: synthesis of compound WX113-7

**WX113-5** (1.5 g, 4.04 mmol), **WX113-6** (1.75 g, 4.44 mmol) and potassium acetate (396.54 mg, 4.04 mmol) were added into a pre-dried flask, and dissolved with water (2 mL) and 1,4-dioxane (20 mL). The mixture was replaced with nitrogen, followed by addition of 1,1-bis(diphenylphosphine) ferrocene palladium chloride (295.65 mg, 404.05 µmol), and replaced with nitrogen again. The reaction solution was stirred at 80 °C for 24 hours. TLC (petroleum ether: ethyl acetate = 1:1) showed that the raw materials did not disappear and a new spot was formed. The mixture was rotary-evaporated directly without other post-processing, which was separated and purified by column chromatography (petroleum ether: ethyl acetate = 1:0 to 1:1) to obtain a crude product. The crude product was purified by preparative HPLC to obtain the target product **WX113-7.**

### Step 6: synthesis of compound WX113 and WX114

**WX113-7** (0.15 g, 268.33 µmol) was resolved by SFC (column: Chiralpak AD-H 250 * 30mm id 5µm; mobile phase: A: CO₂, B: IPA(0.1% NH₄HCO₃); Gradient: B% = 35%; flow rate: 62 g/min; wavelength: 220 nm; column temperature: 40 °C) to obtain compounds **WX113** (Rt = 1.556 min) and **WX114** (Rt = 2.111 min). **WX113:** ¹H NMR (400MHz, METHANOL-d4) δ = 8.15-8.12 (m, 2H), 8.12-8.06 (m, 1H), 8.09 (dd, *J =* 5.8, 8.9 Hz, 1H), 7.98 (br s, 1H), 7.84 (dd, *J* = 2.1, 8.5 Hz, 1H), 7.69 (d, *J* = 8.6 Hz, 1H), 7.49 (dd, *J* = 2.4, 8.6 Hz, 1H), 7.36 (d, *J =* 2.0 Hz, 1H), 7.28-7.20 (m, 1H), 4.23 (dd, *J* = 5.0, 13.3 Hz, 1H), 4.01 (dd, *J* = 9.9, 13.5 Hz, 1H), 3.35-3.25 (m, 14H), 3.00 (s, 3H), 2.62 (s, 3H), 1.23 (d, *J* = 7.1 Hz, 3H). **WX114:** 1H NMR (400MHz, METHANOL-d4) δ = 8.14 (s, 1H), 8.11 (d, *J* = 2.2 Hz, 1H), 8.07 (dd, *J* = 5.6, 8.9 Hz, 1H), 7.98 (br d, *J =* 15.4 Hz, 1H), 7.81 (dd, *J* = 2.3, 8.5 Hz, 1H), 7.68 (d, *J =* 8.6 Hz, 1H), 7.51 (dd, *J* = 2.6, 8.4 Hz, 1H), 7.36 (d, *J* = 2.2 Hz, 1H), 7.24 (dt, *J* = 2.6, 8.4 Hz, 1H), 4.21 (dd, *J* = 4.9, 13.5 Hz, 1H), 4.00 (dd, *J* = 9.9, 13.5 Hz, 1H), 3.01 (s, 3H), 2.99-2.92 (m, 1H), 2.64-2.56 (m, 3H), 1.31-1.26 (m, 1H), 1.32-1.26 (m, 1H), 1.32-1.26 (m, 1H), 1.23-1.19 (m, 1H), 1.23-1.19 (m, 1H), 1.23-1.19 (m, 1H), 1.36-1.19 (m, 1H), 1.24-1. 17 (m, 1H), 1.24-1.17 (m, 1H).

### Example 64: WX115, WX116, WX117, WX118

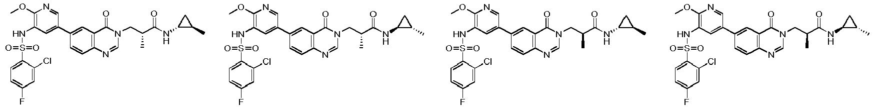

### Synthetic route:

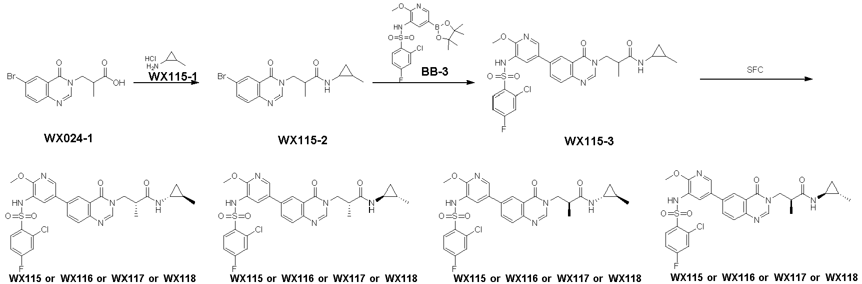

### Step 1: synthesis of compound WX115-2

Compound **WX024-1** (0.5 g, 1.61 mmol), compound **WX115-1** (2 M, 964.22 µL, hydrochloric acid) and dichloromethane (25 mL) were sequentially added into a pre-dried one-neck flask (100 mL), followed by addition of *N,N*-diisopropylethylamine (623.08 mg, 4.82 mmol, 839.73 µL) and a 50% solution of propylphosphonic anhydride in ethyl acetate (1.23 g, 1.93 mmol, 1.15 mL, 50% purity). The mixture was replaced with nitrogen, and stirred at 25 °C for 10 hours. After the reaction was completed, the reaction solution was rotary-evaporated under reduced pressure, and purified by column chromatography (petroleum ether: ethyl acetate = 5:1 to 0:1) to obtain compound **WX115-2.** ¹H NMR (400MHz, CHLOROFORM-d) δ = 8.46-8.30 (m, 1H), 8.16-8.07 (m, 1H), 7.82 (dd, *J* = 2.3, 8.6 Hz, 1H), 7.64-7.55 (m, 1H), 5.86-5.52 (m, 1H), 4.28-4.08 (m, 1H), 4.06-3.82 (m, 1H), 3.05-2.57 (m, 1H), 2.30 (quind, *J* = 3.4, 7.0 Hz, 1H), 1.34-1.21 (m, 3H), 1.03 (t, *J* = 6.0 Hz, 3H), 0.76-0.58 (m, 1H), 0.56-0.38 (m, 2H).

### Step 2: synthesis of compound WX115-3

Compound **WX115-2** (0.517 g, 1.42 mmol), compound **BB-3** (628.37 mg, 1.42 mmol), potassium acetate (417.90 mg, 4.26 mmol), and the solvents 1,4-dioxane (2 mL) and water (0.2 mL) were added into a pre-dried one-neck flask (100 mL). The mixture was replaced with nitrogen, followed by addition of 1,1-bis(diphenylphosphine) ferrocene palladium chloride (103.86 mg, 141.94 µmol), and replaced with nitrogen again. The mixture was heated to 90 °C and stirred for 5 hours. After the reaction was completed, the reaction solution was cooled down and filtered. The filtrate was evaporated under reduced pressure to remove the solvent, and then separated by column chromatography (petroleum ether: ethyl acetate = 5:1 to 0:1) to obtain compound **WX115-3.** ¹H NMR (400MHz, METHANOL-d4) δ = 8.32 (t, *J* = 1.8 Hz, 1H), 8.22 (d, *J* = 2.3 Hz, 1H), 8.18 (d, *J* = 2.4 Hz, 1H), 8.10 (dd, *J* = 5.8, 8.8 Hz, 1H), 8.05-7.98 (m, 2H), 7.77 (dd, *J* = 2.0, 8.5 Hz, 1H), 7.48 (dd, *J* = 2.5, 8.4 Hz, 1H), 7.28-7.19 (m, 1H), 4.29-4.18 (m, 1H), 4.00 (dd, *J* = 10.2, 13.3 Hz, 1H), 3.87 (s, 3H), 3.00-2.85 (m, 1H), 2.23 (td, *J* = 3.7, 7.2 Hz, 1H), 1.24-1.18 (m, 3H), 0.99 (t, *J* = 6.3 Hz, 3H), 0.71-0.52 (m, 1H), 0.49-0.33 (m, 2H).

### Step 3: synthesis of compound WX115, WX116, WX117, WX118

Compound **WX115-3** (0.8 g, 1.33 mmol) was resolved by SFC (1: column: OJ (250mm * 30mm, 5µm); mobile phase: [MeOH]; B%: 30% -30%, 7min; 2: column : OJ (250mm * 30mm, 5µm); mobile phase: [MeOH]; B%: 30% -30%, 5min; 3: column: OJ (250mm * 30mm, 5µm); mobile phase: [MeOH]; B %: 30% -30%, 5min; column: Chiralpak IC-H 250 * 30mm 5µm; mobile phase: [0.1% NH₄HCO₃ MeOH]; B%: 45%-45%, 13min) to obtain **WX115** (retention time is 2.852min), **WX116** (retention time is 2.43min), **WX117** (retention time is 3.96min) and **WX118** (retention time is 4.89min). **WX115:** ¹H NMR (400MHz, METHANOL-*d₄*) δ = 8.29 (d, J = 2.0 Hz, 1H), 8.19-8.14 (m, 2H), 8.11 (dd, *J* = 5.8, 8.9 Hz, 1H), 7.99 (dd, *J* = 2.1, 8.5 Hz, 1H), 7.95 (d, *J* = 2.2 Hz, 1H), 7.75 (d, *J* = 8.4 Hz, 1H), 7.45 (dd, *J* = 2.5, 8.5 Hz, 1H), 7.23 (dt, *J* = 2.5, 8.3 Hz, 1H), 4.23 (dd, *J* = 4.9, 13.5 Hz, 1H), 3.99 (dd, *J* = 10.0, 13.3 Hz, 1H), 3.87 (s, 3H), 3.00-2.84 (m, 1H), 2.23 (td, *J* = 3.6, 7.3 Hz, 1H), 1.21 (d, *J* = 7.1 Hz, 3H), 0.98 (d, *J* = 6.2 Hz, 3H), 0.57 (qt, *J* = 6.1, 9.2 Hz, 1H), 0.47-0.34 (m, 2H). **WX116:** ¹HNMR (400MHz, METHANOL-d4) δ = 8.29 (d, *J* = 2.0 Hz, 1H), 8.19-8.14 (m, 2H), 8.11 (dd, *J* = 5.8, 8.9 Hz, 1H), 7.99 (dd, *J* = 2.1, 8.5 Hz, 1H), 7.95 (d, *J* = 2.2 Hz, 1H), 7.75 (d, *J* = 8.4 Hz, 1H), 7.45 (dd, *J* = 2.5, 8.5 Hz, 1H), 7.23 (dt, *J* = 2.5, 8.3 Hz, 1H), 4.23 (dd, *J* = 4.9, 13.5 Hz, 1H), 3.99 (dd, *J* = 10.0, 13.3 Hz, 1H), 3.87 (s, 3H), 3.00-2.84 (m, 1H), 2.23 (td, *J* = 3.6, 7.3 Hz, 1H), 1.21 (d, *J* = 7.1 Hz, 3H), 0.98 (d, *J* = 6.2 Hz, 3H), 0.57 (qt, *J* = 6.1, 9.2 Hz, 1H), 0.47-0.34 (m, 2H). **WX117:** ¹HNMR (400MHz, METHANOL-d4) δ = 8.29 (d, *J* = 2.0 Hz, 1H), 8.19-8.14 (m, 2H), 8.11 (dd, *J* = 5.8, 8.9 Hz, 1H), 7.99 (dd, *J* = 2.1, 8.5 Hz, 1H), 7.95 (d, *J* = 2.2 Hz, 1H), 7.75 (d, *J* = 8.4 Hz, 1H), 7.45 (dd, *J* = 2.5, 8.5 Hz, 1H), 7.23 (dt, *J* = 2.5, 8.3 Hz, 1H), 4.23 (dd, *J* = 4.9, 13.5 Hz, 1H), 3.99 (dd, *J* = 10.0, 13.3 Hz, 1H), 3.87 (s, 3H), 3.00-2.84 (m, 1H), 2.23 (td, *J* = 3.6, 7.3 Hz, 1H), 1.21 (d, *J* = 7.1 Hz, 3H), 0.98 (d, *J* = 6.2 Hz, 3H), 0.57 (qt, *J* = 6.1, 9.2 Hz, 1H), 0.47-0.34 (m, 2H). **WX118:** ¹HNMR (400MHz, METHANOL-d4) δ = 8.29 (d, *J* = 2.0 Hz, 1H), 8.19-8.14 (m, 2H), 8.11 (dd, *J* = 5.8, 8.9 Hz, 1H), 7.99 (dd, *J* = 2.1, 8.5 Hz, 1H), 7.95 (d, *J* = 2.2 Hz, 1H), 7.75 (d, *J* = 8.4 Hz, 1H), 7.45 (dd, *J* = 2.5, 8.5 Hz, 1H), 7.23 (dt, *J* = 2.5, 8.3 Hz, 1H), 4.23 (dd, *J* = 4.9, 13.5 Hz, 1H), 3.99 (dd, *J* = 10.0, 13.3 Hz, 1H), 3.87 (s, 3H), 3.00-2.84 (m, 1H), 2.23 (td, *J* = 3.6, 7.3 Hz, 1H), 1.21 (d, *J* = 7.1 Hz, 3H), 0.98 (d, *J* = 6.2 Hz, 3H), 0.57 (qt, *J* = 6.1, 9.2 Hz, 1H), 0.47-0.34 (m, 2H).

### Example 65: WX119, WX120

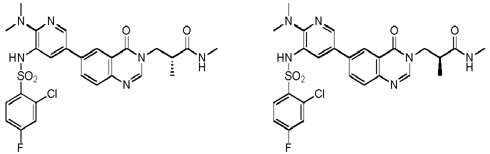

### Synthetic route:

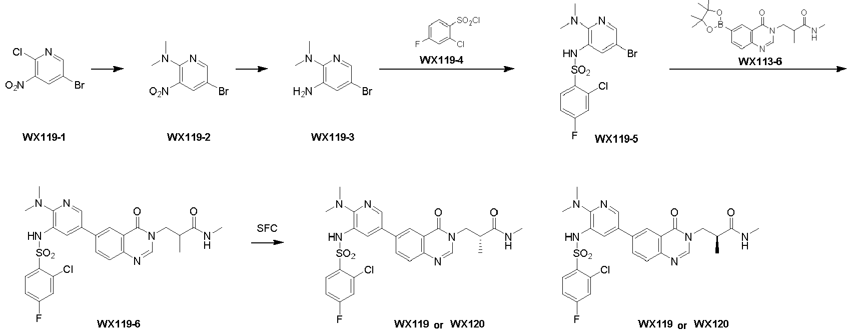

### Step 1: synthesis of compound WX119-2

Compound **WX119-1** (5 g, 21.06 mmol) and dimethylamine hydrochloride (3.43 g, 42.12 mmol, 1.28 mL, hydrochloric acid) were sequentially added into a pre-dried one-neck flask (100 mL). The mixture was replaced with nitrogen, and stirred at 25 °C for 10 hours. After the reaction was completed, the reaction solution was extracted with saturated sodium bicarbonate (100 mL) and dichloromethane (50 mL × 3). The organic phases were combined, washed with saturated sodium chloride, dried over anhydrous sodium sulfate, filtered, and finally dried under reduced pressure to obtain target compound **WX119-2,** which was directly used in the next step. ¹H NMR (400MHz, CHLOROFORM-d) δ = 8.33 (d, *J =* 2.3 Hz, 1H), 8.24 (d, *J =* 2.3 Hz, 1H), 3.05 (s, 6H).

### Step 2: synthesis of compound WX119-3

Compound **WX119-2** (4.5 g, 18.29 mmol), ammonium chloride (11.74 g, 219.46 mmol), methanol (180 mL) and water (90 mL) were sequentially added into a pre-dried three-neck flask (500mL), followed by addition of zinc powder (8.37 g, 128.02 mmol). The mixture was replaced with nitrogen, and stirred at 50 °C for 10 hours. After the reaction was completed, the reaction solution was filtered, washed with methanol (100 mL × 3). The filtrate was combined, dried under reduced pressure, extracted with saturated sodium bicarbonate (100 mL) and dichloromethane (100 mL × 4). The organic phases were combined, washed with saturated sodium chloride, dried over anhydrous sodium sulfate, filtered, and finally rotary-evaporated under reduced pressure, and separated by column chromatography (petroleum ether: ethyl acetate = 100:1 to 30:1) to obtain compound **WX119-3.** ¹H NMR (400MHz, CHLOROFORM-d) δ = 7.80 (d, *J* = 2.1 Hz, 1H), 7.04 (d, *J* = 2.1 Hz, 1H), 3.95-3.74 (m, 2H), 2.75 (s, 6H).

### Step 3: synthesis of compound WX119-5

Compound **WX119-3** (0.7 g, 3.24 mmol), compound **WX119-4** (742.04 mg, 3.24 mmol) were added into a pre-dried reaction flask (40 mL), followed by addition of pyridine (14 mL). The mixture was replaced with nitrogen, heated to 20 °C and stirred for 5 hours. After the reaction was completed, the reaction solution was rotary-evaporated under reduced pressure and purified by preparative HPLC (chromatographic column: Agela Durashell C18 150 * 25mm 5µm; mobile phase: [water (10mM NH₄HCO₃) -ACN]; B%: 25%-60%, 10.5min) to obtain compound **WX119-5.** ¹H NMR (400MHz, CHLOROFORM-d) δ = 8.20 (dd, *J* = 5.7, 8.8 Hz, 1H), 8.06 (d, *J* = 2.3 Hz, 1H), 7.72 (d, *J* = 2.1 Hz, 1H), 7.27-7.23 (m, 1H), 7.17 (ddd, *J* = 2.5, 7.4, 8.9 Hz, 1H), 2.75 (s, 1H), 2.68 (s, 6H).

### Step 4: synthesis of compound WX119-6

Compound **WX113-6** (687.75 mg, 1.85 mmol), compound **WX119-5** (393 mg, 961.65 µmol), water (0.7 mL), 1,4-dioxane (7 mL) and potassium acetate (283.13 mg, 2.88 mmol) were sequentially added into a pre-dried reaction flask (10 mL). The mixture was replaced with nitrogen, followed by addition of 1,1-bis(diphenylphosphine) ferrocene palladium chloride (70.36 mg, 96.16 µmol). The mixture was replaced with nitrogen again, heated to 90 °C and stirred for 10 hours. After the reaction was completed, the reaction solution was evaporated under reduced pressure to remove the solvent, separated by column chromatography (petroleum ether: ethyl acetate = 5:1 to 0:1), and then further purified by preparative HPLC (purification method: chromatographic column: Xtimate C18 150 * 25mm * 5µm; mobile phase: [water (10mM NH₄HCO₃) -ACN]; B%: 27%-47%, 10.5min) to obtain compound **WX119-6.** ¹H NMR (400MHz, METHANOL-d4) δ = 8.34 (d, *J* = 2.3 Hz, 1H), 8.19-8.14 (m, 2H), 8.06 (dd, *J* = 5.8, 8.9 Hz, 1H), 7.87 (dd, *J* = 2.3, 8.5 Hz, 1H), 7.71 (d, *J* = 8.5 Hz, 1H), 7.57-7.50 (m, 2H), 7.31-7.24 (m, 1H), 4.23 (dd, *J* = 5.0, 13.4 Hz, 1H), 4.02 (dd, *J* = 9.8, 13.4 Hz, 1H), 3.02-2.91 (m, 7H), 2.65-2.59 (m, 3H), 1.24 (d, *J* = 7.0 Hz, 3H).

### Step 5: synthesis of compound WX119, WX120

Compound **WX119-6** (0.22 g, 383.92 µmol, 1 eq) was resolved by SFC (instrument: Thar SFC80 preparative SFC; column: Chiralpak AD-H 250 * 30mm id 5µ; mobile phase: A: CO₂, B: IPA; gradient: B% = 30%; flow rate: 65 g/min; wavelength: 220 nm; column temperature: 40 °C; back pressure: 100 bar) to obtain compounds **WX119** (retention time is 2.19min) and **WX120** (retention time is 2.34min). **WX119:** ¹H NMR (400MHz, METHANOL-*d₄*) δ = 8.34 (d, *J* = 2.4 Hz, 1H), 8.20-8.15 (m, 2H), 8.06 (dd, *J* = 5.8, 9.0 Hz, 1H), 7.88 (dd, *J* = 2.3, 8.5 Hz, 1H), 7.72 (d, *J* = 8.7 Hz, 1H), 7.57-7.49 (m, 2H), 7.27 (dt, *J* = 2.6, 8.3 Hz, 1H), 4.24 (dd, *J* = 5.0, 13.3 Hz, 1H), 4.02 (dd, *J* = 9.9, 13.5 Hz, 1H), 2.95 (s, 7H), 2.62 (s, 3H), 1.24 (d, *J* = 6.9 Hz, 3H). **WX120:** ¹H NMR (400MHz, METHANOL-*d₄*) δ = 8.34 (d, *J* = 2.4 Hz, 1H), 8.20-8.15 (m, 2H), 8.06 (dd, *J* = 5.8, 9.0 Hz, 1H), 7.88 (dd, *J* = 2.3, 8.5 Hz, 1H), 7.72 (d, *J* = 8.7 Hz, 1H), 7.57-7.49 (m, 2H), 7.27 (dt, *J* = 2.6, 8.3 Hz, 1H), 4.24 (dd, *J* = 5.0, 13.3 Hz, 1H), 4.02 (dd, *J* = 9.9, 13.5 Hz, 1H), 2.95 (s, 7H), 2.62 (s, 3H), 1.24 (d, *J* = 6.9 Hz, 3H).

### Example 66: WX121, WX122

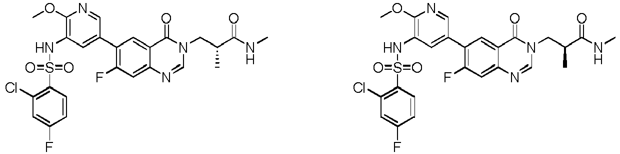

### Synthetic route:

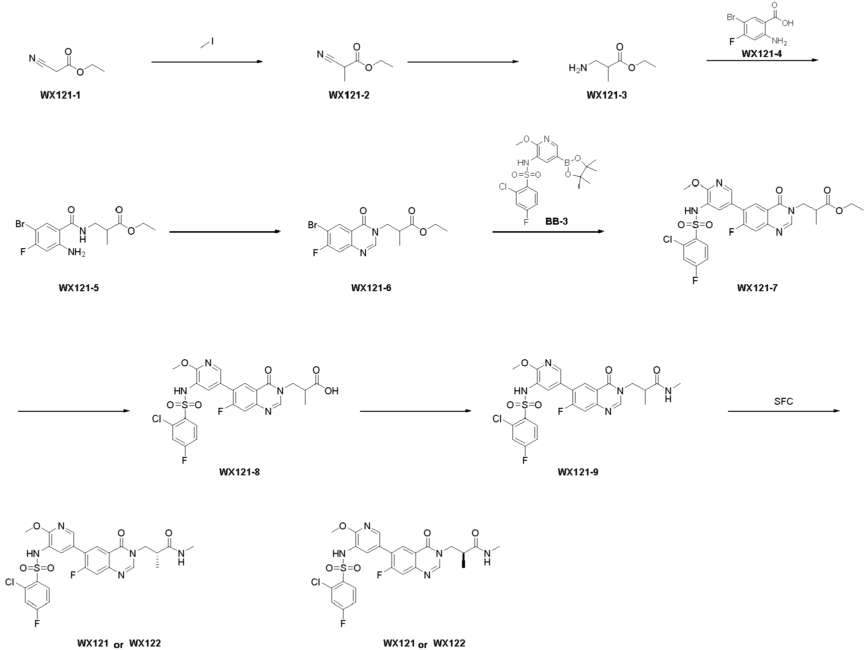

### Step 1: synthesis of compound WX121-2

Compound **WX121-1** (20.00 g, 176.82 mmol) and potassium carbonate (23.22 g, 167.98 mmol) were dissolved in DMF (500.00 mL), followed by addition of methyl iodide (23.84 g, 167.98 mmol) at 0 °C, and stirred at 25 °C overnight. After the reaction was completed, the mixture was poured into water (500.00 mL) and extracted three times with dichloromethane (500 mL). The organic phases obtained were combined and dried over anhydrous sodium sulfate. After the desiccant was removed by filtration, the solvent was removed under reduced pressure, and the crude residue was purified by column chromatography (ethyl acetate / petroleum ether = 0%-10%) to obtain compound **WX121-2.**

### Step 2: synthesis of compound WX121-3

Compound **WX121-2** (3.0 g, 23.60 mmol) was dissolved in methanol (25.00 mL), followed by addition of Raney Ni (404.29 mg, 4.72 mmol) under nitrogen atmosphere and introduction of hydrogen (50 psi). The mixture was stirred at 30 °C overnight. After the reaction was completed, the solvent was removed under reduced pressure to obtain compound **WX121-3.** MS-ESI m/z: 133.1[M+H]+.

### Step 3: synthesis of compound WX121-5

Compound 2-amino-4-fluoro-5-bromobenzoic acid (356.81 mg, 1.52 mmol) was dissolved in *N,N'*-dimethylformamide (3.00 mL), followed by addition of diisopropylethylamine (394.11 mg, 3.05 mmol), HATU (878.33 mg, 2.31 mmol) and **WX121-3** (419.63 mg, 2.31 mmol), and stirred at 25 °C for 16 hours. After the reaction was completed, the mixture was poured into water (10 mL) and extracted three times with dichloromethane (10 mL). The organic phases obtained were combined and dried over anhydrous sodium sulfate. After the desiccant was removed by filtration, the solvent was removed under reduced pressure, and the crude residue was separated by a preparative chromatographic plate (eluent: methanol/dichloromethane = 1:30) to obtain target compound **WX121-5.** MS-ESI m/z: 349.0[M+H]+, 351.0[M+H+2]+.

### Step 4: synthesis of compound WX121-6

Compound **WX121-5** (200 mg, 576.07 µmol) was dissolved in ethanol (10.00 mL), followed by addition of formamidine acetate (299.87 mg, 2.88 mmol 1), and stirred at 80 °C for 2 hours. After the reaction was completed, the mixture was rotary-evaporated to remove the organic solvent, poured into water (20 mL), and extracted three times with dichloromethane (20 mL). The organic phases obtained were combined and dried over anhydrous sodium sulfate. After removing the desiccant by filtration, the solvent was removed under reduced pressure, and the crude residue was separated by a chromatographic plate (eluent: methanol/dichloromethane/triethylamine = 1:20:0.02) to obtain the target compound **WX121-6.** MS-ESI m/z: 359.0[M+H]+, 361.0[M+H+2]+.

### Step 5: synthesis of compound WX121-7

Compound **WX121-6** (0.19 g, 531.95 µmol), **BB-2** (235.49 mg, 531.95 µmol), potassium acetate (208.82 mg, 2.13 mmol) were dissolved in dioxane (2.00 mL) and water (0.20 mL), followed by addition of [1,1'-bis(diphenylphosphino) ferrocene] palladium dichloride (7.78 mg, 10.64 µmol), heated to 95 °C and stirred for 2 hours under nitrogen atmosphere. After the reaction was completed, the mixture was cooled to room temperature, rotary-evaporated to remove the organic solvent, poured into water (20.00 mL), and extracted three times with dichloromethane (20.00 mL). The organic phases obtained were combined and dried over anhydrous sodium sulfate. After the desiccant was removed by filtration, the solvent was removed under reduced pressure, and the residue was separated by preparative chromatographic plate (eluent: methanol/dichloromethane = 1:30) to obtain target compound **WX121-7.** MS-ESI m/z: 593.0[M+H]+, 595.0[M+H+2]+.

### Step 6: synthesis of compound WX121-8

**WX121-7** (0.1 g, 178.26 µmol) was dissolved in tetrahydrofuran (5.00 mL) and water (5.00 mL), then lithium hydroxide monohydrate (56.61 mg, 1.35 mmol) was added thereto, and the reaction solution was stirred at 25 °C for 1 hour. After the reaction was completed, the reaction solution was rotary-evaporated, followed by addition of water (10 mL). The mixture was washed three times with dichloromethane (10 mL), and concentrated hydrochloric acid (0.20 mL) was added dropwise to the aqueous phase, which was then extracted three times dichloromethane (5 mL). The organic phase was washed with saturated brine (10.00 mL), dried over anhydrous sodium sulfate, and rotary-evaporated to obtain target compound **WX121-8.** MS-ESI m/z: 565.0[M+H]+, 567.0[M+H+2]+.

### Step 7: synthesis of compound WX121-9

Compound **WX121-8** (150 mg, 265.51 µmol) was dissolved in DMF (3.00 mL), followed by addition of triethylamine (53.73 mg, 531.02 µmol), HATU (100.96 mg, 265.51 µmol) and methylamine hydrochloride (17.93 mg, 265.51 µmol), and stirred at 30 °C for 2 hours. After the reaction was completed, the solvent was removed under reduced pressure, and the residue was poured into water (5 mL), and extracted three times with dichloromethane (10 mL). The organic phases obtained were combined and dried over anhydrous sodium sulfate. After the desiccant was removed by filtration, the solvent was removed under reduced pressure, and the crude residue was separated by preparative chromatographic plate (eluent: methanol/dichloromethane = 1:15), and further separated by preparative high-performance liquid column (water Xbridge 150 * 25 5u; Mobile phase: [Water (10mM NH₄HCO₃) -ACN]; B%: 26% - 56%, 7min) to obtain target compound **WX121-9.** MS-ESI m/z: 578.0[M+H]⁺, 580.0[M+H+2]⁺.

### Step 8: synthesis of compound WX121, WX122

Compound **WX121-9** was resolved by supercritical fluid chromatography (separation condition column: AD (250mm * 30mm, 10µm); mobile phase: [0.1% NH₄HCO₃ EtOH]; B%: 55% -55%) to obtain enantiomers **WX121** and **WX122,** the retention time of which is 0.626min and 1.531min, respectively, and the ratio is 1:1. **WX121:** ¹H NMR (400MHz, CDCl₃) δ : 8.14 (d, *J =* 8.3 Hz, 1H), 8.10-8.01 (m, 2H), 7.97 (s, 1H), 7.85 (br s, 1H), 7.36 (d, *J =* 11.3 Hz, 1H), 7.18 (br s, 1H), 7.06 (br t, *J* = 7.3 Hz, 1H), 5.58 (br s, 1H), 4.18-4.04 (m, 1H), 4.02-3.83 (m, 4H), 2.97-2.79 (m, 1H), 2.67 (d, *J* = 4.8 Hz, 3H), 1.27-1.20 (m, 3H). **WX122:** ¹HNMR (400MHz, CDCl₃) δ : 8.14 (d, *J =* 8.3 Hz, 1H), 8.12-8.01 (m, 2H), 7.96 (s, 1H), 7.85 (br s, 1H), 7.37 (d, *J* = 11.0 Hz, 1H), 7.17 (br s, 1H), 7.06 (br t, *J* = 7.0 Hz, 1H), 5.55 (br s, 1H), 4.18-4.03 (m, 1H), 4.01-3.81 (m, 4H), 2.96-2.79 (m, 1H), 2.67 (d, *J =* 4.8 Hz, 3H), 1.21 (br d, *J =* 7.0 Hz, 3H).

### Example 67: WX123, WX124

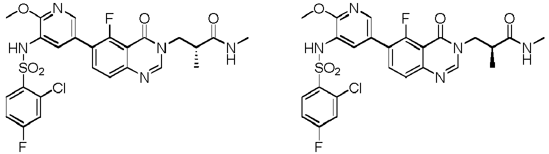

### Synthetic route:

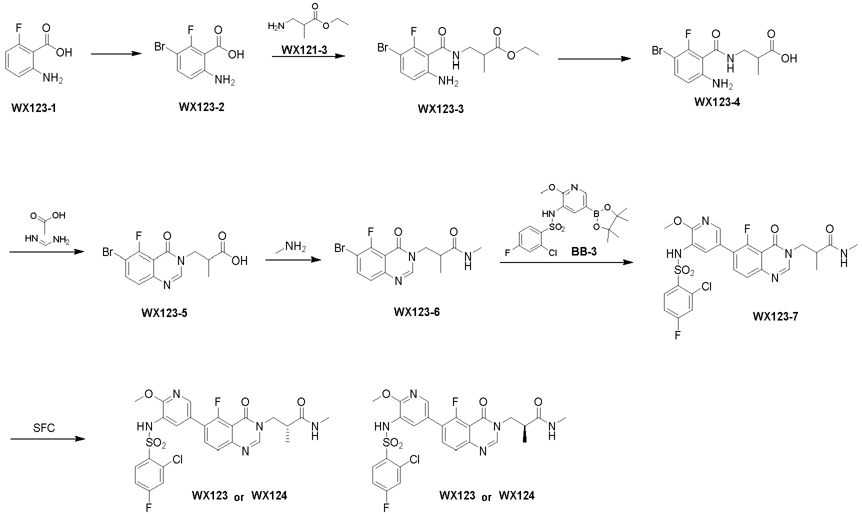

### Step 1: synthesis of compound WX123-2

**WX123-1** (3 g, 19.34 mmol) and NBS (3.44 g, 19.34 mmol) were added to dichloromethane (60 mL) and reacted at 25 °C for 2 hours. After the reaction was completed, the reaction solution was filtered. The filter cake was washed with dichloromethane (100 mL), and rotary-evaporated to obtain target compound **WX123-2,** which was directly used in the next reaction. ¹H NMR (400MHz, DMSO-d6) δ = 6.54-6.58 (m, 1H), 7.37-7.42 (m, 1H).

### Step 2: synthesis of compound WX123-3

Compound **WX123-2** (2 g, 8.55 mmol), **WX121-3** (1.68 g, 12.82 mmol), EDCI (1.67 g, 8.72 mmol), TEA (3.46 g, 34.18 mmol, 4.76 mL), and 1-oxidopyridin-1-iµm-2-ol (1.11 g, 10.00 mmol) were added to DCM (50 mL) and reacted at 50 °C for 16 hours. After the reaction was completed, the reaction solution was rotary-evaporated, diluted with water (100 mL), extracted with DCM (100 mL). The organic phase was rotary-evaporated, separated and purified by column chromatography (ethyl acetate: petroleum ether = 0%-20%) to obtain target compound **WX123-3.** MS-ESI *m*/*z*: 346.9[M+H]⁺, 348.9[M+H+2]⁺.

### Step 3: synthesis of compound WX123-4

**WX123-3** (0.72 g, 1.59 mmol) and lithium hydroxide monohydrate (0.668 g, 15.92 mmol) were added to EtOH (10 mL) and H₂O (10 mL), and reacted at 25 °C for 16 hours. After the reaction was completed, the reaction solution was rotary-evaporated, extracted with DCM (50 mL), and the aqueous phase was rotary-evaporated to obtain target compound **WX123-4,** which was directly used in the next reaction. MS-ESI *m*/*z*: 318.9[M+H]⁺, 320.9[M+H+2]⁺.

### Step 4: synthesis of compound WX123-5

**WX123-4** (0.9 g, 2.82 mmol) and formamidine acetate (585.00 mg, 5.62 mmol) were added to EtOH (80 mL) and reacted at 80 °C for 48 hours. After the reaction was completed, the reaction solution was rotary-evaporated, diluted with water (100 mL), extracted with DCM (100 mL × 3), and the organic phase was rotary-evaporated to obtain target compound **WX123-5.** MS-ESI *m*/*z*: 328.8[M+H]⁺, 330.8[M+H+2]⁺.

### Step 5: synthesis of compound WX123-6

**WX123-5** (0.4 g, 418.93 µmol), methylamine (0.084 g, 1.24 mmol, HCl), HATU (0.26 g, 683.80 µmol) and DIEA(163.24 mg, 1.26 mmol, 0.22 mL) were added to DMF (5 mL) and reacted at 20 °C for 16 hours. After the reaction was completed, the reaction solution was rotary-evaporated, diluted with water (50 mL), extracted with DCM (50 mL), and the organic phase was rotary-evaporated, separated and purified by column chromatography (EA: PE = 0% ∼ 80%) to obtain target compound **WX123-6.** MS-ESI *m*/*z*: 341.9[M+H]⁺, 343.9[M+H+2]⁺.

### Step 6: synthesis of compound WX123-7

**WX123-6** (0.18 g, 305.07 µmol), **BB-3** (0.143 g, 304.19 µmol), Pd(dppf)Cl₂ (0.022 g, 30.07 µmol) and KOAc (0.12 g, 1.22 mmol) were added to dioxane (5 mL) and water (1 mL). The system was replaced with N₂ three times, and then reacted at 105 °C for 1 hour under N₂ atmosphere. After the reaction was completed, the reaction solution was rotary-evaporated, diluted with water (50 mL), extracted with DCM (50 mL), and the organic phase was rotary-evaporated, separated and purified by preparative HPLC (chromatographic column: Xtimate C18 150 * 25mm * 5µm; mobile phase: [water (0.225 % FA) -ACN]; B%: 40%-50%, 9.5 min) to obtain target compound **WX123-7.**

### Step 7: synthesis of compound WX123 and WX124

**WX123-7** was resolved by SFC (column: OJ (250mm * 30mm, 5µm); mobile phase: [0.1% NH₄HCO₃ EtOH]; B%: 40%-%) to obtain enantiomers **WX123** (rt = 4.531min) and **WX124** (rt = 5.318min). **WX123:** ¹H NMR (400 MHz, DMSO-*d*₆) δ ppm 10.25 (br s, 1 H), 8.14-8.25 (m, 2 H), 7.85-8.00 (m, 3 H), 7.69-7.82 (m, 2 H), 7.56 (br d, *J* = 8.5 Hz, 1 H), 7.36 (brt, *J* = 7.3 Hz, 1 H), 3.86-4.09 (m, 2 H), 3.71 (s, 3 H), 2.85 (br d, *J*= 7.5 Hz, 1 H), 2.47-2.49 (m, 3 H), 1.08 (br d, *J* = 7.0 Hz, 3 H), MS-ESI *m*/*z*: 578.0[M+H]⁺. **WX124:** ¹HNMR (400 MHz, DMSO-*d*₆) δ ppm 10.14-10.41 (m, 1 H), 8.19 (s, 2 H), 7.86-7.97 (m, 3 H), 7.78 (s, 1 H), 7.73 (dd, *J* = 8.5, 2.5 Hz, 1H), 7.57 (d, *J =* 8.5 Hz, 1H), 7.36 (td, *J =* 8.4, 2.8 Hz, 1H), 3.87-4.09 (m, 2 H), 3.71 (s, 3 H), 2.84 (br dd, *J* = 15.1, 6.5 Hz, 1H), 2.53 (d, *J =* 2.0 Hz, 3 H), 1.08 (d, *J =* 7.0 Hz, 3 H), MS-ESI *m*/*z*: 578.0[M+H]⁺.

### Example 68: WX125, WX126

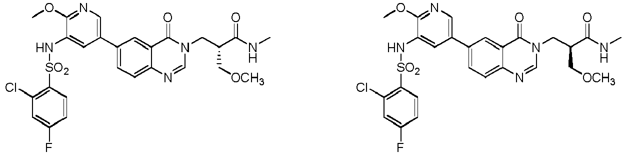

### Synthetic route:

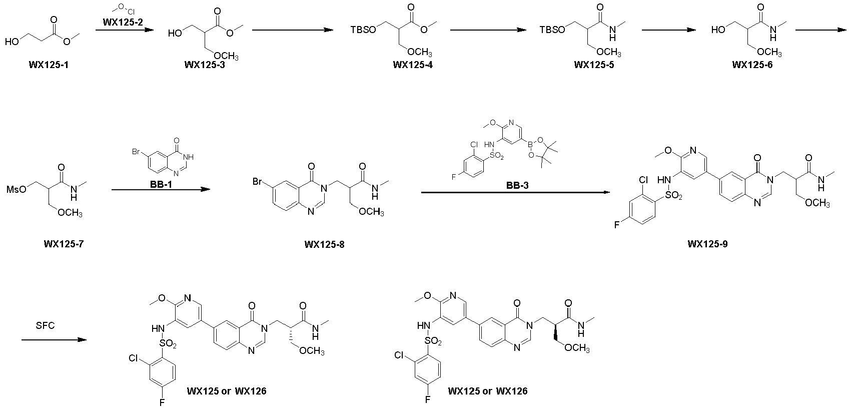

### Step 1: synthesis of compound WX125-3

Raw material lithium diisopropylamide (2 M, 76.85 mL, 2 eq, 77 mL) was added into a pre-dried three-necked bottle. The mixture was cooled to -78 °C, followed by addition of the solution of **WX125-1** (10 g, 96.06 mmol) in tetrahydrofuran (20 mL), and stirred at -78 °C for 1 hour. **WX125-2** (15.47 g, 192.12 mmol, 14.59 mL) was added thereto at -78 °C, and then the reaction system was warmed to -40 °C and further stirred at -40 °C for 3 hours. After the reaction was completed, saturated ammonium chloride solution was added to quench the reaction, followed by extraction with dichloromethane (10 mL × 3). The organic phase obtained was dried over anhydrous sodium sulfate, rotary-evaporated under reduced pressure by a water pump, and separated by flash column chromatography (petroleum ether: ethyl acetate = 1:0) to obtain target product **WX125-3.** ¹H NMR (400MHz, CHLOROFORM-d) δ = 3.96 (br d, *J* = 6.0 Hz, 1H), 3.98-3.92 (m, 1H), 3.98-3.92 (m, 1H), 3.91 (br s, 1H), 3.94-3.85 (m, 1H), 3.94-3.85 (m, 1H), 3.77-3.65 (m, 5H), 3.41-3.34 (m, 3H), 2.87 (quin, *J* = 5.8 Hz, 1H), 2.64-2.55 (m, 1H), 1.45-1.44 (m, 1H), 1.45-1.44 (m, 1H), 1.46 (d, *J* = 6.4 Hz, 2H).

### Step 2: synthesis of compound WX125-4

Raw material **WX125-3** (4.07 g, 27.47 mmol, 1 eq) and the solvent dichloromethane (40 mL) were added into a pre-dried single-necked flask, followed by addition of triethylamine (6.95 g, 68.68 mmol, 9.56 mL). The mixture was cooled to 0 °C, followed by slow addition of *tert*-butyldiphenylchlorosilane (4.97 g, 32.97 mmol, 4.04 mL), and further stirred at 25 °C for 12 hours. Thin layer chromatography detection (petroleum ether: ethyl acetate = 10: 1) showed that the reaction was completed, and water (20 mL) and dichloromethane (20 mL × 3) was added to the system for extraction. The resulting organic phase was dried over anhydrous sodium sulfate, rotary-evaporated under reduced pressure by a water pump to obtain a crude product. The crude product was separated and purified by column chromatography (petroleum ether: ethyl acetate = 1:0 to 30:1) to obtain target product **WX125-4.** ¹H NMR (400MHz, CHLOROFORM-d) δ = 3.86-3.82 (m, 2H), 3.71 (s, 3H), 3.66-3.58 (m, 2H), 3.35 (s, 3H), 2.86-2.81 (m, 1H), 0.92 (s, 14H), 0.88 (s, 11H), 0.13-0.12 (m, 1H), 0.12-0.09 (m, 8H), 0.05 (s, 5H).

### Step 3: synthesis of compound WX125-5

Raw material **WX125-4** (1.38 g, 5.26 mmol) was added to a pre-dried reaction flask, followed by addition of nitromethane (3.27 g, 105.18 mmol), and further stirred at 50 °C for 12 hours. TLC (petroleum ether / ethyl acetate = 3/1) showed that all raw materials were consumed and a new spot was formed. The reaction solution was rotary-evaporated and purified by column chromatography (petroleum ether: ethyl acetate = 5:1 to 1:1) to obtain target compound **WX125-5.** ¹H NMR (400MHz, CHLOROFORM-d) δ = 6.48 (br s, 1H), 3.83-3.69 (m, 2H), 3.61-3.45 (m, 2H), 3.28 (s, 3H), 2.73 (d, *J* = 4.8 Hz, 3H), 2.54 (quin, *J* = 6.2 Hz, 1H), 0.86-0.78 (m, 9H), 0.00 (s, 6H).

### Step 4: synthesis of compound WX125-6

Compound **WX125-5** (0.5 g, 1.91 mmol), tetrahydrofuran (5 mL) and tetrabutylammonium fluoride (1 M, 1.91 mL) were sequentially added into a pre-dried reaction flask (40 mL). The mixture was replaced with nitrogen, and stirred at 25 °C for 3 hours. TLC (petroleum ether/ethyl acetate = 3/1) showed that the reaction was completed. The reaction solution was rotary-evaporated under reduced pressure to obtain compound **WX125-6,** which was directly used in the next reaction.

### Step 5: synthesis of compound WX125-7

Compounds **WX125-6** (0.7 g, 4.72 mmol), triethylamine (717.14 mg, 7.09 mmol, 986.44 µL) and dichloromethane (7 mL) were sequentially added into a pre-dried reaction flask (8mL), and methanesulfonyl chloride (649.46 mg, 5.67 mmol, 438.83 µL) was finally added thereto at 0 °C. The mixture was replaced with nitrogen, slowly warmed to 5 °C and stirred for 3 hours. After the reaction was completed, the reaction solution was rotary-evaporated under reduced pressure, and then purified by preparative TLC (ethyl acetate) to obtain target product **WX125-7.** ¹H NMR (400MHz, CHLOROFORM-d) δ = 6.37 (br s, 1H), 4.57-4.38 (m, 2H), 3.69-3.55 (m, 2H), 3.46-3.33 (m, 3H), 3.05 (s, 3H), 2.93-2.76 (m, 4H).

### Step 6: synthesis of compound WX125-8

Compound **BB-1** (0.28 g, 1.24 mmol), compound **WX125-7** (280.27 mg, 1.24 mmol), potassium iodide (20.65 mg, 124.42 µmol) and potassium carbonate (343.92 mg, 2.49 mmol) were sequentially added into a pre-dried reaction flask (8mL), and finally *N,N*-dimethylformamide (3 mL) was added. The mixture was replaced with nitrogen, heated to 70 °C and stirred for 24 hours. After the reaction was completed, the reaction solution was cooled down, rotary-evaporated under reduced pressure, and purified by preparative HPLC to obtain target compound **WX125-8.** ¹H NMR (400MHz, CHLOROFORM-d) δ = 8.41 (d, *J* = 2.2 Hz, 1H), 8.17 (s, 1H), 7.84 (dd, *J=* 2.3, 8.7 Hz, 1H), 7.60 (d, *J* = 8.6 Hz, 1H), 6.23 (br s, 1H), 4.28-4.13 (m, 2H), 3.68-3.54 (m, 2H), 3.38 (s, 3H), 3.12-3.02 (m, 1H), 2.78 (d, *J* = 4.9 Hz, 3H).

### Step 7: synthesis of compound WX125-9

Compound **WX125-8** (88.50 mg, 249.87 µmol), compound **BB-3** (110.62 mg, 249.87 µmol), KOAc (73.57 mg, 749.62 µmol) and the solvents 1,4-dioxane (2 mL) and water (0.2 mL) were sequentially added into a pre-dried reaction flask (10mL). The mixture was replaced with nitrogen, followed by addition of 1,1-bis(diphenylphosphine) ferrocene palladium chloride (18.28 mg, 24.99 µmol), and then replaced with nitrogen again. The mixture was heated to 90 °C and stirred for 5 hours. After the reaction was completed, the reaction solution was cooled and filtered. The filtrate was evaporated under reduced pressure to remove the solvent, purified by preparative TLC (ethyl acetate), and further purified by preparative HPLC to obtain target compound **WX125-9.** ¹H NMR (400MHz, METHANOL-d4) δ = 8.33 (d, *J* = 2.2 Hz, 1H), 8.22 (d, *J* = 2.2 Hz, 1H), 8.19 (s, 1H), 8.10 (dd, *J =* 5.8, 8.9 Hz, 1H), 8.04-7.99 (m, 2H), 7.77 (d, *J*= 8.6 Hz, 1H), 7.47 (dd, *J* = 2.5, 8.5 Hz, 1H), 7.27-7.20 (m, 1H), 4.39 (dd, *J =* 5.2, 13.6 Hz, 1H), 4.11 (dd, *J* = 9.2, 13.6 Hz, 1H), 3.87 (s, 3H), 3.66-3.56 (m, 2H), 3.36 (s, 3H), 3.25-3.18 (m, 1H), 2.64 (s, 3H).

### Step 8: synthesis of compound WX125 and WX126

**WX125-9** was resolved by SFC (chromatographic column: OD (250mm * 30mm, 10µm); mobile phase: [ETOH]; B%: 45% -45%, 6min) to obtain enantiomers **WX125** (retention time 2.698 min) and **WX126** (retention time is 2.693 min). **WX125:** ¹H NMR (400MHz, CHLOROFORM-d) δ = 8.32 (d, *J =* 2.0 Hz, 1H), 8.18 (s, 1H), 8.15-8.11 (m, 2H), 7.98 (d, *J* = 2.2 Hz, 1H), 7.87-7.83 (m, 1H), 7.80-7.77 (m, 1H), 7.54 (br s, 1H), 7.60-7.48 (m, 1H), 7.29-7.27 (m, 1H), 7.29-7.27 (m, 1H), 7.15-7.09 (m, 1H), 6.25 (br d, *J* = 4.4 Hz, 1H), 4.30-4.18 (m, 2H), 3.99 (s, 3H), 3.69-3.57 (m, 2H), 3.40 (s, 3H), 3.15-3.08 (m, 1H), 2.80 (d, *J =* 4.8 Hz, 3H). **WX126:** ¹H NMR (400MHz, CHLOROFORM-d) δ = 8.32 (d, *J =* 1.7 Hz, 1H), 8.18 (s, 1H), 8.16-8.10 (m, 2H), 7.98 (s, 1H), 7.88-7.82 (m, 1H), 7.81-7.75 (m, 1H), 7.53 (br s, 1H), 7.29-7.27 (m, 1H), 7.12 (br t, *J =* 7.0 Hz, 1H), 6.24 (br s, 1H), 4.29-4.18 (m, 2H), 3.99 (s, 3H), 3.70-3.56 (m, 2H), 3.40 (s, 3H), 3.16-3.08 (m, 1H), 2.80 (d, *J* = 4.8 Hz, 3H).

### Example 69: WX127

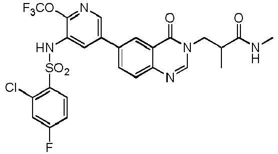

### Synthetic route:

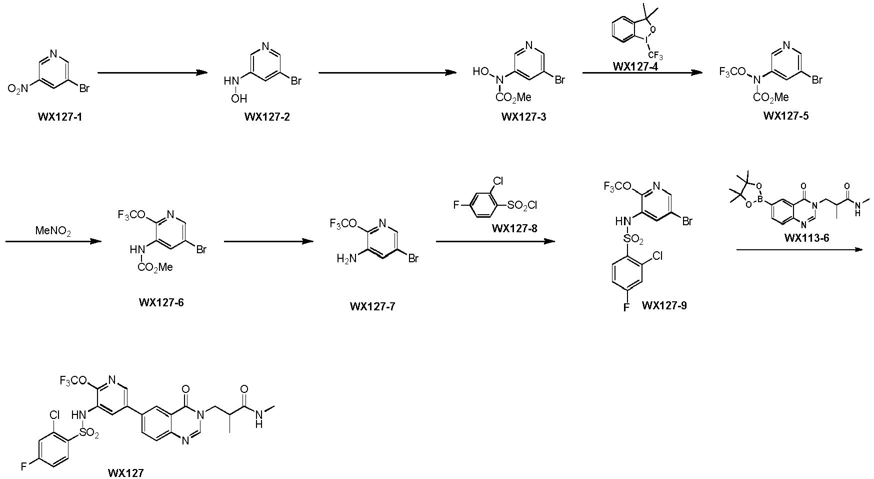

### Step 1: synthesis of compound WX127-2

Compound **WX127-1** (13.7 g, 67.49 mmol), hydrazine hydrate (4.14 g, 80.99 mmol, 4.02 mL, 98% purity) and tetrahydrofuran (140 mL) were added into a pre-dried single-necked flask, and finally palladium on carbon (1.71 g, 8.15 mmol) was added. The mixture was replaced with nitrogen, and stirred at 25 °C for 3 hours. After the reaction was completed, the reaction solution was cooled and filtered. The filter cake was washed with methanol (100 mL × 2) and then dried under reduced pressure to obtain crude compound **WX127-2.** ¹H NMR (400 MHz, METHANOL-*d₄*) δ = 8.09 (d, *J =* 2.3 Hz, 1H), 8.03 (d, *J =* 1.9 Hz, 1H), 7.52 (t, *J =* 2.1 Hz, 1H).

### Step 2: synthesis of compound WX127-3

Compound **WX127-2** (5 g, 26.45 mmol), sodium bicarbonate (2.67 g, 31.74 mmol, 1.23 mL), 4-dimethylaminopyridine (3.23 g, 26.45 mmol) and tetrahydrofuran (50 mL) were sequentially added into a pre-dried one-neck flask (100 mL), and finally methyl chloroformate (3.00 g, 31.74 mmol, 2.46 mL) was added. The mixture was replaced with nitrogen, and stirred at 20 °C for 5 hours. After the reaction was completed, the reaction solution was rotary-evaporated under reduced pressure, separated and purified by column chromatography (dichloromethane: methanol = 200: 1 to 20: 1) to obtain target product **WX127-3.** ¹H NMR (400MHz, CHLOROFORM-d) δ = 8.72 (d, *J=* 2.0 Hz, 1H), 8.33 (d, *J=* 1.8 Hz, 1H), 8.29 (s, 1H), 3.94 (s, 3H).

### Step 3: synthesis of compound WX127-5

Compound **WX127-3** (3 g, 12.14 mmol), compound **WX127-4** (4.01 g, 12.14 mmol) and dichloromethane (121 mL) were sequentially added into a pre-dried reaction flask. The mixture was replaced with nitrogen, and stirred at 20 °C for 10 hours. After the reaction was completed, the solvent was evaporated under reduced pressure. The residue was separated and purified by flash column chromatography (petroleum ether: ethyl acetate = 200: 1 to 30: 1), and further purified by preparative HPLC to obtain target compound **WX127-5.** ¹H NMR (400MHz, CHLOROFORM-*d*) δ = 8.66 (d, *J =* 1.9 Hz, 1H), 8.64 (d, *J =* 2.3 Hz, 1H), 7.92 (t, *J =* 2.1 Hz, 1H), 3.93 (s, 3H).

### Step 4: synthesis of compound WX127-6

Compound **WX127-5** (0.35 g, 1.11 mmol) and nitromethane (11 mL) were sequentially added to a pre-dried microwave tube, replaced with nitrogen, and stirred in the microwave reactor at 160 °C (17 bar) for 1 hour. After the reaction was completed, the solvent was evaporated under reduced pressure. The residue was purified by TLC (petroleum ether: ethyl acetate = 5: 1) to obtain target product **WX192-6.** 1H NMR (400MHz, CHLOROFORM-d) δ = 8.75 (br s, 1H), 7.99 (d, *J* = 2.3 Hz, 1H), 6.96-6.86 (m, 1H), 3.85 (s, 3H).

### Step 5: synthesis of compound WX127-7

Compound **WX127-6** (0.16 g, 507.87 µmol), methanol (2 mL) and water (0.4 mL) were sequentially added to a pre-dried reaction flask, and finally lithium hydroxide monohydrate (85.24 mg, 2.03 mmol) was added. The mixture was replaced with nitrogen, heated to 50 °C and stirred for 24 hours. After the reaction was completed, the reaction solution was cooled down, evaporated under reduced pressure to remove the solvent, and then extracted with dichloromethane (10 mL × 3). The organic phases were combined, washed with saturated sodium chloride (10 mL), and dried over anhydrous sodium sulfate, filtered, and finally rotary-evaporated under reduced pressure to obtain target compound **WX127-7.** ¹H NMR (400MHz, CHLOROFORM-d) δ = 7.70 (d, *J =* 2.2 Hz, 1H), 7.21 (d, *J =* 2.2 Hz, 1H), 3.96 (s, 2H).

### Step 6: synthesis of compound WX127-9

Compound **WX127-7** (110 mg, 428.00 µmol) and tetrahydrofuran (2 mL) were sequentially added into a pre-dried reaction flask, then sodium hydrogen (34.24 mg, 856.01 µmol, 60% purity) was slowly added, and finally compound **WX127-8** (147.05 mg, 642.00 µmol, 93.67 µL) was added dropwise. The mixture was replaced with nitrogen, and stirred at 25 °C for 5 hours. After the reaction was completed, the reaction solution was rotary-evaporated under reduced pressure, and purified by preparative TLC (petroleum ether/ethyl acetate = 5/1) to obtain compound **WX127-9.** ¹H NMR (400MHz, CHLOROFORM-d) δ = 8.11-8.06 (m, 2H), 8.04 (d, *J* = 2.2 Hz, 1H), 7.31 (dd, *J =* 2.5, 8.0 Hz, 1H), 7.17-7.11 (m, 1H).

### Step 7: synthesis of compound WX127

Compounds **WX127-9** (0.1 g, 269.37 µmol), **WX113-6** (53 mg, 117.88 µmol), water (0.2 mL), 1,4-dioxane (2 mL) and potassium acetate (79.31 mg, 808.11 µmol) were sequentially added into a pre-dried reaction flask. The mixture was replaced with nitrogen, followed by addition of 1,1-bis(diphenylphosphine) ferrocene palladium chloride (19.71 mg, 26.94 µmol). The mixture was replaced with nitrogen, heated to 110 °C and stirred for 3 hours. After the reaction was completed, the solvent was evaporated under reduced pressure. The residue was purified by preparative TLC (dichloromethane/methanol = 10/1), and further purified by preparative HPLC to obtain target product **WX127.** ¹H NMR (400MHz, METHANOL-d4) δ = 8.34 (d, *J* = 2.3 Hz, 1H), 8.19-8.14 (m, 2H), 8.06 (dd, *J* = 5.8, 8.9 Hz, 1H), 7.87 (dd, *J* = 2.3, 8.5 Hz, 1H), 7.71 (d, *J =* 8.5 Hz, 1H), 7.57-7.50 (m, 2H), 7.31-7.24 (m, 1H), 4.23 (dd, *J =* 5.0, 13.4 Hz, 1H), 4.02 (dd, *J* = 9.8, 13.4 Hz, 1H), 3.02-2.91 (m, 7H), 2.65-2.59 (m, 3H), 1.24 (d, *J =* 7.0 Hz, 3H).

### Example 70: WX128, WX129

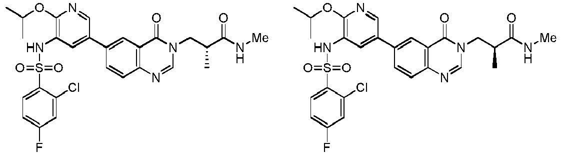

### Synthetic route:

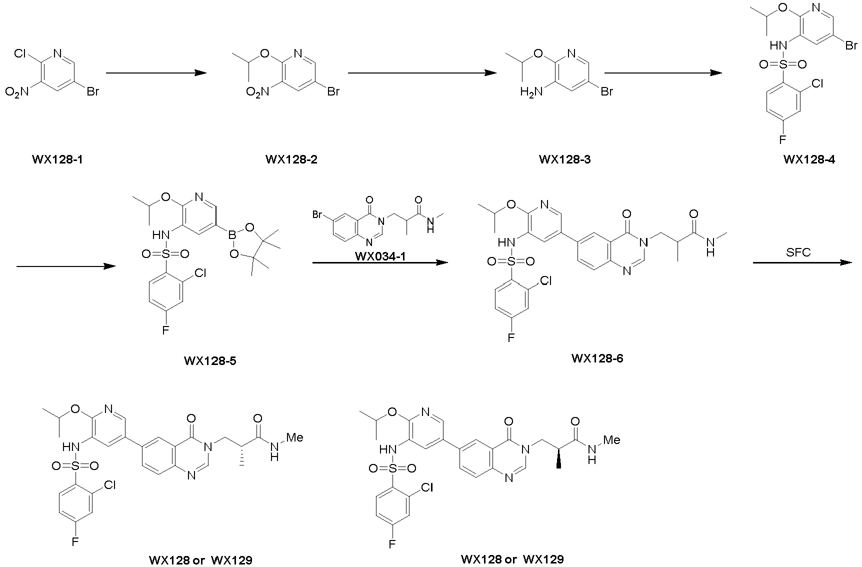

### Step 1: synthesis of compound WX128-2

Raw materials **WX128-1** (5 g, 21.06 mmol) and 1,8-diazabicycloundec-7-ene (14.43 g, 94.76 mmol, 14.28 mL) and the solvent isopropanol (40 mL) were added into a pre-dried single-necked flask and stirred at 25 °C for 12 hours. After the reaction was completed, water (20 mL) was added to the reaction solution, and extracted with dichloromethane (30 mL * 3). The resulting organic phase was dried over anhydrous sodium sulfate, rotary-evaporated under reduced pressure, and purified by preparative thin-layer chromatographic plate (petroleum ether: ethyl acetate = 50: 1) to obtain target compound **WX128-2.** ¹H NMR (400MHz, CHLOROFORM-d) δ = 8.41 (d, *J =* 2.2 Hz, 1H), 8.33 (d, *J =* 2.4 Hz, 1H), 5.49-5.45 (m, 1H).

### Step 2: synthesis of compound WX128-3

Raw material **WX128-2** (1.9 g, 7.28 mmol) and the solvent acetic acid (25 mL) were added into a pre-dried reaction flask, followed by addition of iron powder (4.06 g, 72.78 mmol), and further stirred at 25 °C for 2 hours. After the reaction was completed, the reaction solution was diluted with a small amount of ethanol, filtered, and the filtrate was added with water (15mL) and extracted with dichloromethane (15mL × 3). The organic phase was dried over anhydrous sodium sulfate, rotary-evaporated under reduced pressure and purified by flash column chromatography (petroleum ether: ethyl acetate as mobile phase = 1:0 to 10:1), to obtain target compound **WX128-3.** 1H NMR (400MHz, CHLOROFORM-d) δ = 7.56 (d, *J* = 2.2 Hz, 1H), 7.27 (s, 1H), 6.97-6.96 (m, 1H), 6.97 (d, *J* = 2.2 Hz, 1H), 5.28 (spt, *J* = 6.2 Hz, 1H), 1.36 (d, *J* = 6.2 Hz, 7H).

### Step 3: synthesis of compound WX128-4

Raw materials **WX128-3** (1.42 g, 6.14 mmol) and 2-chloro-4-fluorobenzenesulfonyl chloride (1.83 g, 7.99 mmol, 1.17 mL) and the solvent pyridine (15 mL) were added into a pre-dried single-necked flask, and stirred at 25 °C for 12 hours. After the reaction was completed, water (10 mL) was added to the reaction solution, and extracted with dichloromethane (10 mL × 3). The resulting organic phase was dried over anhydrous sodium sulfate, rotary-evaporated under reduced pressure, separated and purified by flash column chromatography (petroleum ether: ethyl acetate) ester = 1: 0 to 10: 1), to obtain target compound **WX128-4.** ¹H NMR (400MHz, CHLOROFORM-d) δ = 8.34 (dd, *J =* 5.7, 9.0 Hz, 1H), 8.26 (d, *J =* 2.3 Hz, 1H), 8.08 (dd, *J* = 5.7, 8.8 Hz, 1H), 8.01 (d, *J* = 2.4 Hz, 1H), 7.86-7.86 (m, 1H), 7.87-7.84 (m, 1H), 7.49 (s, 1H), 7.25-7.23 (m, 1H), 7.10 (ddd, *J =* 2.5, 7.5, 8.8 Hz, 1H), 5.28-5.23 (m, 1H), 1.29 (d, *J* = 6.1 Hz, 6H), 1.06 (d, *J* = 6.3 Hz, 1H).

### Step 4: synthesis of compound WX128-5

Raw materials **WX128-4** (300 mg, 708.07 µmol) and bis(pinacolato)diboron (179.81 mg, 708.07 µmol) and the solvent 1,4-dioxane (3 mL) were added into a pre-dried reaction flask, followed by addition of potassium acetate (138.98 mg, 1.42 mmol). The mixture was replaced with nitrogen, followed by addition of 1,1-bis(diphenylphosphine) ferrocene palladium chloride (51.81 mg, 70.81 µmol). The mixture was further replaced with nitrogen, and stirred at 90 °C for 3 hours. After the reaction was completed, the target compound **WX128-5** was obtained, which was directly used in the next reaction.

### Step 5: synthesis of compound WX128-6

Raw materials **WX128-5** (210.00 mg, 647.80 µmol) and **WX034-1** (320.20 mg, 680.19 µmol), and the solvents 1,4-dioxane (2 mL) and water (0.6 mL) were added into a pre-dried reaction flask, followed by addition of potassium acetate (127.15 mg, 1.30 mmol). The mixture was replaced with nitrogen, followed by addition of 1,1-bis(diphenylphosphine) ferrocene palladium chloride (47.40 mg, 64.78 µmol). The mixture was further replaced with nitrogen, and stirred at 80 °C for 12 hours. After the reaction was completed, the mixture was rotary-evaporated directly and separated by flash column chromatography (petroleum ether: ethyl acetate = 1:0 to 0:1, then dichloromethane: methanol = 100: 1) to obtain target compound **WX128-6.**

### Step 6: synthesis of compound WX128, WX129

**WX128-6** was resolved by SFC (instrument: Thar SFC80 preparative SFC; column: Chiralpak AS-H 250 * 30mm id 5µ; mobile phase: A: CO₂, B: MeOH (0.1% NH₄HCO₃); Gradient: B% = 42%; flow rate: 70g/min; wavelength: 220 nm; column temperature: 40 °C; back pressure: 100 bar) to obtain the enantiomers **WX128** (retention time is 3.143 min) and **WX129** (retention time is 3.134 min). **WX128:** ¹H NMR (400MHz, CHLOROFORM-d) δ = 8.32 (d, *J* = 2.2 Hz, 1H), 8.15-8.08 (m, 3H), 8.01 (d, *J =* 2.2 Hz, 1H), 7.87-7.82 (m, 1H), 7.80-7.76 (m, 1H), 7.58 (s, 1H), 7.28 (s, 1H), 7.14-7.09 (m, 1H), 5.60 (br d, *J* = 4.8 Hz, 1H), 5.37 (quin, *J* = 6.1 Hz, 1H), 4.22-4.14 (m, 1H), 4.09-4.01 (m, 1H), 3.03-2.90 (m, 1H), 2.75 (d, *J =* 4.8 Hz, 3H), 1.34 (d, *J* = 6.1 Hz, 6H), 1.29 (d, *J* = 7.0 Hz, 3H). **WX129:** ¹H NMR (400MHz, CHLOROFORM-d) δ = 8.31 (d, *J* = 1.8 Hz, 1H), 8.14-8.08 (m, 3H), 8.01 (d, *J=* 2.2 Hz, 1H), 7.87-7.82 (m, 1H), 7.80-7.75 (m, 1H), 7.58 (s, 1H), 7.28 (s, 1H), 7.14-7.09 (m, 1H), 5.61 (br d, *J* = 4.4 Hz, 1H), 5.37 (td, *J* = 6.1, 12.3 Hz, 1H), 4.22-4.14 (m, 1H), 4.09-4.00 (m, 1H), 3.02-2.89 (m, 1H), 2.75 (d, *J* = 4.8 Hz, 3H), 1.34 (d, *J* = 6.1 Hz, 6H), 1.29 (d, *J=* 7.0 Hz, 3H).

### Example 71: WX130, WX131

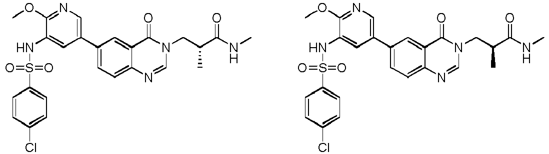

### Synthetic route:

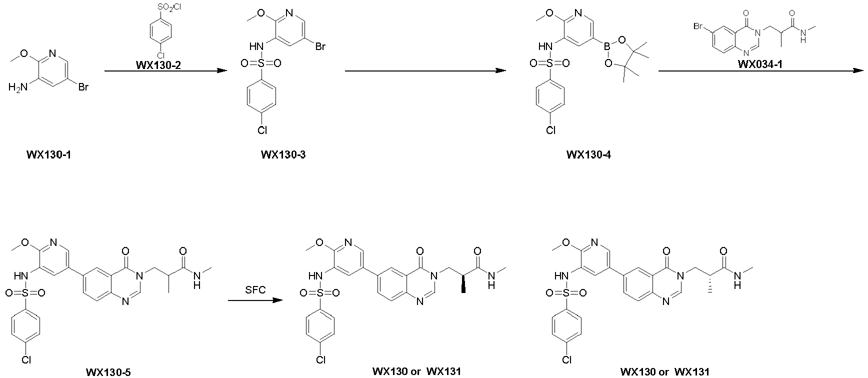

### Step 1: synthesis of compound WX130-3

Compound **WX130-1** (1g, 4.93 mmol) and pyridine (6 mL) were sequentially added into a pre-dried reaction flask, followed by addition of compound **WX130-2** (1.04 g, 4.93 mmol). The mixture was replaced with nitrogen and stirred at 25 °C for 5 hours to complete the reaction. The solvent was evaporated under reduced pressure, and separated by flash column chromatography (petroleum ether: ethyl acetate as mobile phase= 10:1 to 5: 1) to obtain target compound **WX130-3.** ¹H NMR (400MHz, CHLOROFORM-d) δ = 7.94 - 7.87 (m, 2H), 7.79 - 7.71 (m, 2H), 7.50 - 7.43 (m, 2H), 6.92 (s, 1H), 3.83 (s, 3H).

### Step 2: synthesis of compound WX130-4

Compound **WX130-3** (0.15 g, 397.20 µmol), bis(pinacolato)diboron (110.95 mg, 436.92 µmol), potassium acetate (77.96 mg, 794.41 µmol) and 1,4-dioxane (3 mL) were added into a pre-dried reaction flask. The mixture was replaced with nitrogen, and 1,1-bis (diphenylphosphine) ferrocene palladium chloride (29.06 mg, 39.72 µmol) was added thereto finally. The mixture was replaced with nitrogen, heated to 110 °C and stirred for 3 hours to complete the reaction. The reaction solution was cooled down, and the solvent was evaporated under reduced pressure to obtain compound **WX130-4,** which was used directly in the next step.

### Step 3: synthesis of compound WX130-5

Compound **WX130-4** (0.2 g, 470.91 µmol), compound **WX034-1** (0.15 g, 462.72 µmol), 1,4-dioxane (2 mL), water (0.2 mL) and potassium acetate (136.23 mg, 1.39 mmol) were added into a pre-dried reaction flask. The mixture was replaced with nitrogen, and 1,1-bis(diphenylphosphine) ferrocene palladium chloride (33.86 mg, 46.27 µmol) was added thereto finally. The mixture was replaced with nitrogen again, heated to 110 °C and stirred for 3 hours to complete the reaction. The reaction solution was cooled down, and the solvent was evaporated under reduced pressure, purified by preparative TLC (dichloromethane: methanol = 10:1) to obtain target compound **WX130-5.** ¹HNMR (400MHz, METHANOL-*d₄*) δ = 8.40 (d, *J* = 2.2 Hz, 1H), 8.25 (d, *J* = 2.4 Hz, 1H), 8.19 (s, 1H), 8.09 (d, *J* = 2.4 Hz, 1H), 8.05 (dd, *J* = 2.2, 8.6 Hz, 1H), 7.78 (d, *J* = 8.6 Hz, 3H), 7.56-7.51 (m, 2H), 4.25 (dd, *J* = 4.9, 13.5 Hz, 1H), 4.03 (dd, *J* = 9.9, 13.5 Hz, 1H), 3.77 (s, 3H), 3.07-2.96 (m, 1H), 2.62 (s, 3H), 1.25 (s, 3H).

### Step 4: synthesis of compound WX130 and WX131

Compound **WX130-5** (0.12 g, 221.40 µmol) was resolved by SFC (separation conditions: chromatographic column: AS (250mm * 30mm, 5µm); mobile phase: [0.1% NH₄HCO₃, MeOH]; B%: 50%-50%, 15min) to obtain enantiomers **WX130** (Rt = 3.267min) and **WX131** (Rt = 3.750min). **WX130:** ¹H NMR (400MHz, METHANOL-d4) δ = 8.38 (d, *J* = 1.8 Hz, 1H), 8.22-8.17 (m, 2H), 8.04 (td, *J* = 2.6, 5.8 Hz, 2H), 7.82-7.76 (m, 3H), 7.53 (d, *J* = 8.3 Hz, 2H), 4.25 (dd, *J* = 4.8, 13.2 Hz, 1H), 4.03 (dd, *J* = 9.9, 13.4 Hz, 1H), 3.79 (s, 3H), 3.02 (br d, *J* = 10.1 Hz, 1H), 2.62 (s, 3H), 1.24 (d, *J* = 7.0 Hz, 3H). **WX131:** ¹H NMR (400MHz, METHANOL-d4) δ = 8.36 (d, *J =* 2.2 Hz, 1H), 8.20-8.15 (m, 2H), 8.05-8.00 (m, 2H), 7.82-7.74 (m, 3H), 7.52 (d, *J* = 8.3 Hz, 2H), 4.24 (dd, *J* = 4.8, 13.6 Hz, 1H), 4.03 (dd, *J* = 9.9, 13.4 Hz, 1H), 3.79 (s, 3H), 3.07-2.94 (m, 1H), 2.62 (s, 3H), 1.24 (d, *J =* 7.0 Hz, 3H).

### Example 72: WX132, WX133

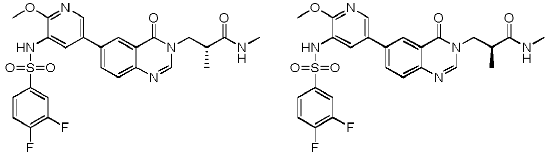

### Synthetic route:

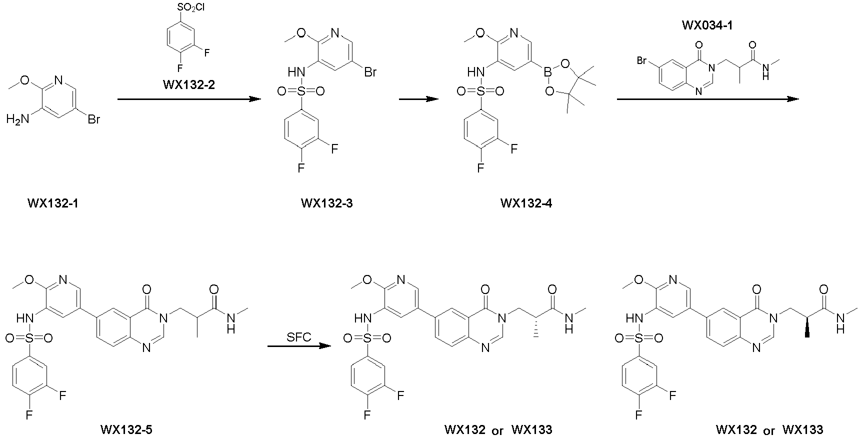

### Step 1: synthesis of compound WX132-3

Compound **WX132-1** (1 g, 4.93 mmol) and pyridine (6 mL) were sequentailly added into a pre-dried reaction flask, and compound **WX132-2** (1.05 g, 4.93 mmol, 659.20 µL) was added there to finally. The mixture was then replaced with nitrogen, and the reaction was completed after stirring at 25 °C for 5 hours. The reaction solution was evaporated to remove the solvent under reduced pressure, and separated by column chromatography (petroleum ether: ethyl acetate = 10: 1 to 5: 1) to obtain compound **WX132-3.** ¹H NMR (400MHz, CHLOROFORM-*d*) δ = 7.94 (d, *J* = 2.3 Hz, 1H), 7.90 (d, *J* = 2.1 Hz, 1H), 7.68 (ddd, *J* = 2.3, 7.1, 9.3 Hz, 1H), 7.63-7.57 (m, 1H), 7.33-7.27 (m, 1H), 6.90 (s, 1H), 3.93-3.79 (m, 3H).

### Step 2: synthesis of compound WX132-4

Compound **WX132-3** (0.15 g, 395.59 µmol), bis(pinacolato)diboron (110.50 mg, 435.15 µmol), potassium acetate (77.65 mg, 791.19 µmol) and 1,4-dioxane (3 mL) were added into a pre-dried reaction flask. The mixture was replaced with nitrogen, and 1,1-bis(diphenylphosphine) ferrocene palladium chloride (28.95 mg, 39.56 µmol) was added thereto finally. The mixture was then replaced with nitrogen, and the reaction was completed after heating to 110 °C and stirring for 3 hours. The reaction solution was cooled down, and evaporated under reduced pressure to remove the solvent to obtain target compound **WX132-4,** which was directly used in the next step.

### Step 3: synthesis of compound WX132-5

Compound **WX132-4** (0.2 g, 470.91 µmol), compound **WX034-1** (0.15 g, 462.72 µmol), 1,4-dioxane (2 mL), water (0.2 mL) and potassium acetate (136.23 mg, 1.39 mmol) were sequentially added into a pre-dried reaction flask. The mixture was replaced with replaced with nitrogen, and 1,1-bis(diphenylphosphine) ferrocene palladium chloride (33.86 mg, 46.27 µmol) was added thereto finally. The mixture was then replaced with nitrogen, heated to 110 °C and stirred for 3 hours to complete the reaction. The reaction solution was cooled down, evaporated under reduced pressure to remove the solvent, and purified by preparative TLC (dichloromethane: methanol = 10:1) to obtain compound **WX132-5.** ¹H NMR (400MHz, METHANOL-*d₄*) δ = 8.41 (d, *J* = 2.0 Hz, 1H), 8.28 (d, *J* = 2.2Hz, 1H), 8.19 (s, 1H), 8.10 (d, *J* = 2.2 Hz, 1H), 8.07 (dd, *J* = 2.2, 8.4 Hz, 1H), 7.83-7.74 (m, 2H), 7.66-7.60 (m, 1H), 7.48-7.38 (m, 1H), 4.24 (dd, *J* = 5.0, 13.3 Hz, 1H), 4.03 (dd, *J* = 9.8, 13.3 Hz, 1H), 3.80 (s, 3H), 3.05-2.97 (m, 1H), 2.64-2.61 (m, 3H), 1.25 (s, 3H).

### Step 4: synthesis of compound WX132, WX133

Compound **WX132-5** was resolved by SFC (separation conditions: chromatographic column: AS (250mm ^{∗} 30mm, 5µm); mobile phase: [0.1% NH₄HCO₃, MEOH]; B%: 50%-50%, 15min) to obtain enantiomers **WX132** (Rt = 2.859min) and **WX133** (Rt = 3.124min). **WX132:** ¹H NMR (400MHz, METHANOL-*d₄*) δ = 8.39 (d, *J* = 1.8 Hz, 1H), 8.27 (d, *J* = 2.2 Hz, 1H), 8.19 (s, 1H), 8.10-8.04 (m, 2H), 7.83-7.74 (m, 2H), 7.63 (br d, *J* = 8.8 Hz, 1H), 7.48-7.38 (m, 1H), 4.24 (dd, *J* = 4.8, 13.2 Hz, 1H), 4.03 (dd, *J* = 10.1, 13.6 Hz, 1H), 3.80 (s, 3H), 3.06-2.96 (m, 1H), 2.62 (s, 3H), 1.24 (d, *J* = 7.0 Hz, 3H). **WX133:** ¹H NMR (400MHz, METHANOL-*d₄*) δ = 8.39 (d, *J* = 1.8 Hz, 1H), 8.27 (d, *J* = 2.2 Hz, 1H), 8.19 (s, 1H), 8.10-8.04 (m, 2H), 7.83-7.74 (m, 2H), 7.63 (br d, *J=* 8.8 Hz, 1H), 7.48-7.38 (m, 1H), 4.24 (dd, *J* = 4.8, 13.2 Hz, 1H), 4.03 (dd, *J* = 10.1, 13.6 Hz, 1H), 3.80 (s, 3H), 3.06-2.96 (m, 1H), 2.62 (s, 3H), 1.24 (d, *J* = 7.0 Hz, 3H).

### Example 73: WX134, WX135

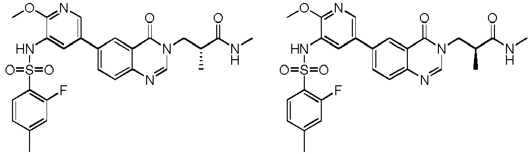

### Synthetic route:

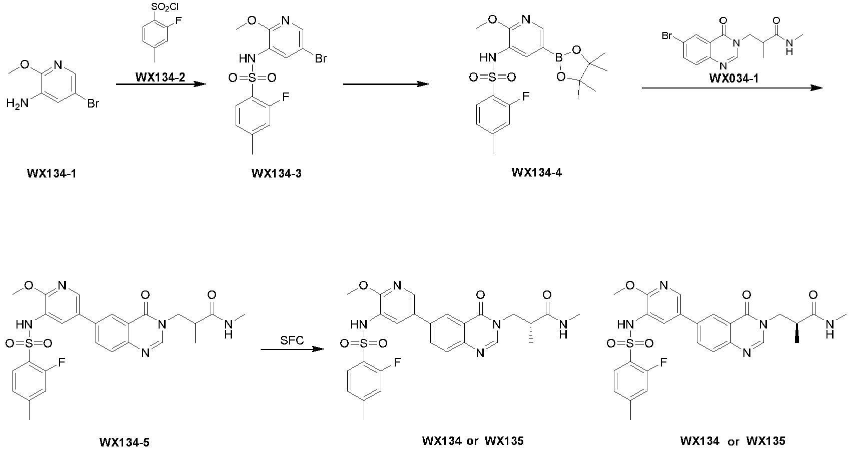

### Step 1: synthesis of compound WX134-3

Compound **WX134-1** (1 g, 4.93 mmol) and pyridine (6 mL) were sequentially added into a pre-dried reaction flask, followed by addition of compound **WX134-2** (1.03 g, 4.93 mmol, 659.20 µL). The mixture was replaced with nitrogen and the reaction was completed after stirring at 25 °C for 5 hours. The reaction solution was evaporated under reduced pressure to remove the solvent, and separated by flash column chromatography (petroleum ether: ethyl acetate = 10:1 to 5:1) to obtain target compound **WX134-3.** ¹H NMR (400MHz, CHLOROFORM-d) δ = 7.86 (d, *J* = 2.2 Hz, 1H), 7.82 (d, *J* = 2.1 Hz, 1H), 7.76 (t, *J* = 7.8 Hz, 1H), 7.22 (s, 1H), 7.05 (d, *J* = 8.1 Hz, 1H), 6.99 (d, *J* = 11.1 Hz, 1H), 3.90 (s, 3H), 2.40 (s, 3H).

### Step 2: synthesis of compound WX134-4

Compound **WX134-3** (0.15 g, 399.77 µmol), bis(pinacolato)diboron (101.52 mg, 399.77 µmol), potassium acetate (78.47 mg, 799.55 µmol) and 1,4-dioxane (3 mL) were added into a pre-dried reaction flask. The mixture was replaced with nitrogen, followed by addition of 1,1-bis(diphenylphosphine) ferrocene palladium chloride (29.25 mg, 39.98 µmol). The mixture was then replaced with nitrogen, heated to 110 °C and stirred for 3 hours to complete the reaction. The reaction solution was cooled down, evaporated under reduced pressure to remove the solvent to obtain target compound **WX134-4,** which was directly used in the next step.

### Step 3: synthesis of compound WX134-5

Compound **WX134-4** (0.2 g, 473.62 µmol), compound **WX034-1** (0.13 g, 401.02 µmol), 1,4-dioxane (2 mL), water (0.2 mL) and potassium acetate (118.07 mg, 1.20 mmol) were sequentially added into a pre-dried reaction flask. The mixture was then replaced with nitrogen, and 1,1-bis(diphenylphosphine) ferrocene palladium chloride (29.34 mg, 40.10 µmol) was added thereto finally. The mixture was then replaced with nitrogen, heated to 110 °C and stirred for 3 hours to complete the reaction. The reaction solution was cooled down, evaporated under reduced pressure to remove the solvent, and purified by preparative TLC (dichloromethane: methanol = 10:1) to obtain compound **WX134-5.** ¹H NMR (400MHz, METHANOL-*d₄*) δ = 8.35 (d, *J* = 2.0 Hz, 1H), 8.22 (d, *J* = 2.4 Hz, 1H), 8.18 (s, 1H), 8.05-8.00 (m, 2H), 7.77 (d, *J* = 8.4 Hz, 1H), 7.70 (t, *J* = 7.8 Hz, 1H), 7.16-7.08 (m, 2H), 4.24 (dd, *J* = 4.9, 13.5 Hz, 1H), 4.03 (dd, *J* = 10.0, 13.3 Hz, 1H), 3.85 (s, 3H), 3.05-2.95 (m, 1H), 2.64-2.61 (m, 3H), 2.39 (s, 3H), 1.25 (s, 3H).

### Step 4: synthesis of compound WX134, WX135

Compound **WX134-5** (0.12 g, 222.40 µmol) was resolved by SFC (column: Chiralpak AS-H 250 ^{∗} 30mm id 5µ; mobile phase: A: CO₂, B: MeOH (0.1% NH₄HCO₃); gradient: B% = 50% ; Flow rate: 80 g/min; wavelength: 220 nm; column temperature: 40 °C; back pressure: 100 bar) to obtain the enantiomers **WX134** (Rt = 2.976min) and **WX135** (Rt = 3.335min). **WX134:** ¹H NMR (400MHz, METHANOL-*d₄*) δ = 8.32 (d, *J=* 2.2 Hz, 1H), 8.21-8.16 (m, 2H), 8.02-7.98 (m, 2H), 7.76-7.67 (m, 2H), 7.16-7.07 (m, 2H), 4.24 (dd, *J* = 4.8, 13.6 Hz, 1H), 4.02 (dd, *J* = 9.9, 13.4 Hz, 1H), 3.84 (s, 3H), 3.06-2.96 (m, 1H), 2.64-2.60 (m, 3H), 2.39 (s, 3H), 1.24 (d, *J* = 7.0 Hz, 3H). **WX135:** ¹H NMR (400MHz, METHANOL-*d₄*) δ = 8.32 (d, *J* = 1.8 Hz, 1H), 8.19-8.17 (m, 2H), 8.00-7.97 (m, 2H), 7.75-7.68 (m, 2H), 7.15-7.10 (m, 2H), 4.24 (dd, *J* = 4.8, 13.6 Hz, 1H), 4.02 (dd, *J* = 9.9, 13.4 Hz, 1H), 3.84 (s, 3H), 3.00 (ddd, *J* = 4.8, 7.0, 9.6 Hz, 1H), 2.63-2.60 (m, 3H), 2.39 (s, 3H), 1.24 (d, *J=* 7.0 Hz, 3H).

### Example 74: WX136, WX137

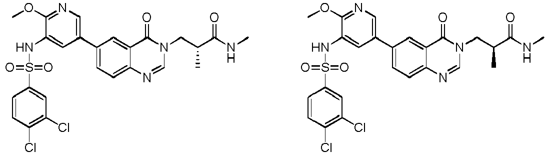

### Synthetic route:

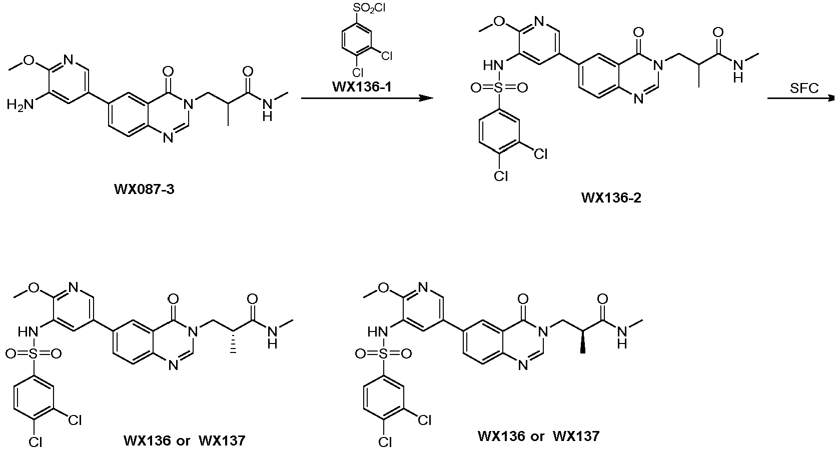

### Step 1: synthesis of compound WX136-2

Compound **WX087-3** (0.2 g, 544.36 µmol) and pyridine (6 mL) were sequentially added into a pre-dried reaction flask, followed by addition of compound **WX136-1** (133.65 mg, 544.36 µmol). The mixture was replaced with nitrogen and the reaction was completed after stirring at 80 °C for 5 hours, which was purified by preparative TLC (dichloromethane: methanol = 10: 1) to obtain target compound **WX136-2.** ¹H NMR (400MHz, METHANOL-d4) δ = 8.42 (d, *J* = 1.8 Hz, 1H), 8.30 (d, *J* = 2.2 Hz, 1H), 8.19 (s, 1H), 8.10 (d, *J* = 2.6 Hz, 1H), 8.07 (dd, *J* = 2.2, 8.8 Hz, 1H), 7.97 (d, *J* = 1.3 Hz, 1H), 7.79 (d, *J* = 8.8 Hz, 1H), 7.71-7.64 (m, 2H), 4.25 (dd, *J* = 4.8, 13.6 Hz, 1H), 4.03 (dd, *J* = 9.6, 13.6 Hz, 1H), 3.78 (s, 3H), 3.04-2.95 (m, 1H), 2.62 (s, 3H), 1.24 (d, *J* = 7.0 Hz, 3H).

### Step 2: synthesis of compound WX136, WX137

Compound **WX136-2** (0.12 g, 208.17 µmol) was resolved by SFC (separation condition column: Chiralpak AS-H 250 ^{∗} 30mm id 5µm; mobile phase: A: CO₂, B: MEOH (0.1% NH₄HCO₃); gradient: B% = 50%; flow rate: 80 g / min; wavelength: 220 nm; column temperature: 40 °C; back pressure: 100 bar) to obtain the enantiomers **WX136** (Rt = 3.337min) and **WX137** (Rt = 3.726 min). **WX136:** ¹H NMR(400MHz, METHANOL-*d₄*) δ = 8.41 (d, *J* = 1.8 Hz, 1H), 8.29 (d, *J* = 2.2 Hz, 1H), 8.19 (s, 1H), 8.11-8.04 (m, 2H), 7.97 (s, 1H), 7.78 (d, *J* = 8.3 Hz, 1H), 7.71-7.64 (m, 2H), 4.24 (dd, *J* = 4.8, 13.6 Hz, 1H), 4.03 (dd, *J* = 9.9, 13.4 Hz, 1H), 3.77 (s, 3H), 2.99 (br d, *J* = 9.6 Hz, 1H), 2.62 (s, 3H), 1.24 (d, *J* = 6.6 Hz, 3H). **WX137:** 1H NMR (400MHz, METHANOL-*d₄*) δ = 8.42 (d, *J* = 1.8 Hz, 1H), 8.30 (d, *J* = 2.2 Hz, 1H), 8.19 (s, 1H), 8.11-8.05 (m, 2H), 7.97 (d, *J* = 1.3 Hz, 1H), 7.78 (d, *J* = 8.8 Hz, 1H), 7.70-7.64 (m, 2H), 4.24 (dd, *J=* 4.8, 13.2 Hz, 1H), 4.03 (dd, *J=* 9.9, 13.4 Hz, 1H), 3.78 (s, 3H), 3.05-2.96 (m, 1H), 2.62 (s, 3H), 1.24 (d, *J=* 7.0 Hz, 3H).

### Example 75: WX138, WX139

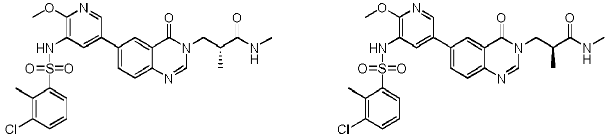

### Synthetic route:

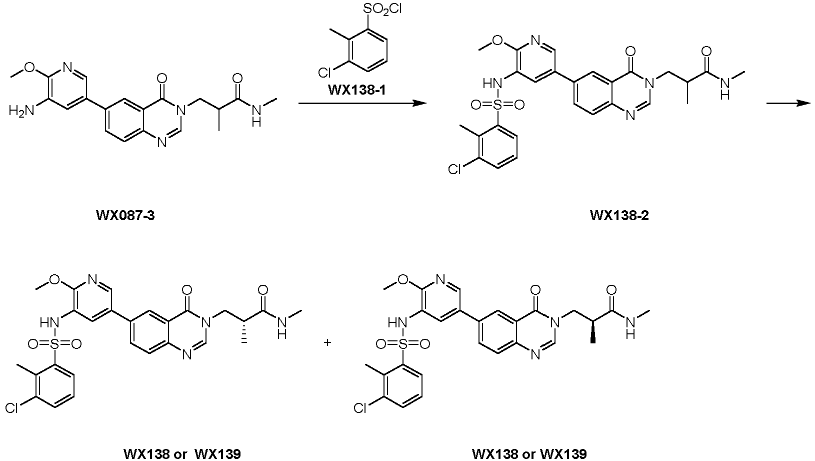

### Step 1: synthesis of compound WX138-2

**WX087-3** (150 mg, 408.27 µmol) and **WX138-1** (551.39 mg, 2.45 mmol, 5.94 µL) were added into a pre-dried 40 mL reaction flask, followed by addition of pyridine (6 mL). The reaction solution was stirred at 20 °C for 12 hours. After the reaction was completed, 10 mL of water and 10 mL of ethyl acetate were added to the reaction system for dilution. The organic phase was collected after liquid separation, and the aqueous phase was extracted with ethyl acetate (5 mL × 3). The organic phases were combined, washed with saturated brine (20 mL), dried over anhydrous sodium sulfate, concentrated under reduced pressure, and separated by preparative HPLC (method: column: Luna C18 100 * 30 5µ; mobile phase: [water (0.1% TFA) -ACN]; B%: 25% - 55%, 10min) to obtain target compound **WX138-2.**

### Step 2: synthesis of compound WX138 and WX139

**WX138-2** was resolved and purified by SFC (resolution method: chromatographic column: AS (250mm * 30mm, 5µm); mobile phase: [MeOH]; B%: 45% -45%, 6min) to obtain target compounds **WX138** (Rt = 3.240 min) and **WX139** (Rt = 3.611 min). **WX138:** ¹H NMR (400MHz, METHANOL-d4) δ = 8.32 (d, *J* = 2.0 Hz, 1H), 8.23 (s, 1H), 8.19 (d, *J=* 2.2 Hz, 1H), 8.00 (dd, *J=* 2.2, 8.6 Hz, 1H), 7.98 (d, *J* = 2.2 Hz, 1H), 7.85 (dd, *J* = 1.0, 8.0 Hz, 1H), 7.75 (d, *J* = 8.4 Hz, 1H), 7.61 (dd, *J* = 1.0, 8.0 Hz, 1H), 7.28 (t, *J=* 7.9 Hz, 1H), 4.24 (dd, *J=* 4.9, 13.5 Hz, 1H), 4.03 (dd, *J* = 9.9, 13.5 Hz, 1H), 3.83 (s, 3H), 3.06-2.95 (m, 1H), 2.77 (s, 3H), 2.61 (s, 3H), 1.23 (d, *J* = 7.1 Hz, 3H). **WX139:** ¹H NMR (400MHz, METHANOL-d4) 6 = 8.31 (d, *J* = 2.0 Hz, 1H), 8.22-8.17 (m, 2H), 8.01-7.96 (m, 2H), 7.85 (dd, *J* = 1.0, 8.0 Hz, 1H), 7.75 (d, *J* = 8.6 Hz, 1H), 7.61 (dd, *J* = 0.9, 8.2 Hz, 1H), 7.28 (t, J = 8.3 Hz, 1H), 4.23 (dd, *J* = 4.9, 13.5 Hz, 1H), 4.02 (dd, *J* = 9.8, 13.3 Hz, 1H), 3.82 (s, 3H), 3.04-2.94 (m, 1H), 2.77 (s, 3H), 2.61 (s, 3H), 1.23 (d, *J=* 7.1 Hz, 3H).

### Example 76: WX140, WX141

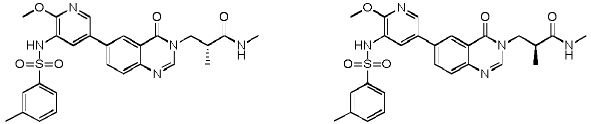

### Synthetic route:

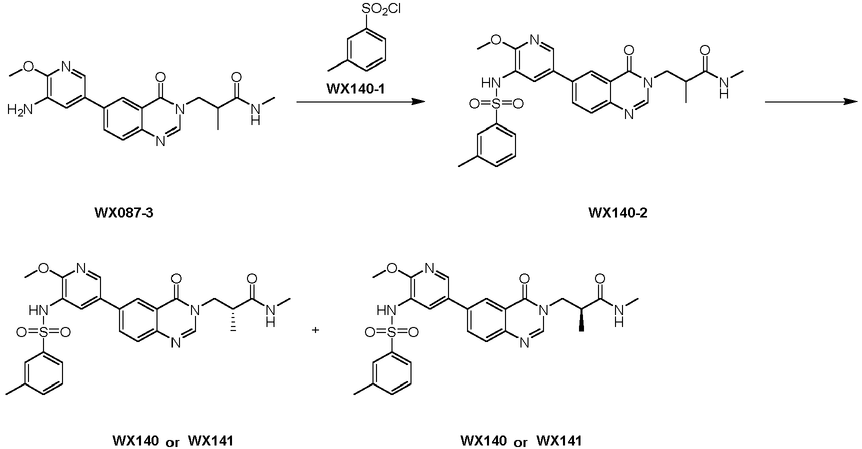

### Step 1: synthesis of compound WX140-2

**WX087-3** (150 mg, 408.27 µmol) and **WX140-1** (233.51 mg, 1.22 mmol, 178.25 µL) were added into a pre-dried 40 mL reaction flask, followed by addition of pyridine (5 mL). The reaction solution was stirred at 20 °C for 12 hours. After the reaction was completed, 10 mL of water and 10 mL of ethyl acetate were added to the reaction system for dilution. The organic phase was collected after liquid separation, and the aqueous phase was extracted with ethyl acetate (5 mL × 3). The organic phases were combined, washed with saturated brine (20 mL), dried over anhydrous sodium sulfate, concentrated under reduced pressure, and separated by preparative HPLC (method: column: Phenomenex Synergi C18 100 ^{∗} 30mm ^{∗} 4µm; mobile phase: [water (0.1 % TFA) -MeOH]; B%: 20% -45%, 10min) to obtain target compound **WX140-2.**

### Step 2: synthesis of compound WX140 and WX141

**WX140-2** was resolved and purified by SFC (resolution method: chromatography column: AS (250mm ^{∗} 30mm, 5µm); mobile phase: [MeOH]; B%: 45% -45%, 8.5min) to obtain the enantiomer **WX140** (Rt = 3.144 min) and **WX141** (Rt = 3.504 min). **WX140:** ¹H NMR (400MHz, METHANOL-d4) δ = 8.37 (d, J = 2.0 Hz, 1H), 8.20 (d, *J=* 2.2 Hz, 1H), 8.18 (s, 1H), 8.05-8.01 (m, 2H), 7.77 (d, *J* = 8.6 Hz, 1H), 7.65 (s, 1H), 7.60 (d, *J* = 7.1 Hz, 1H), 7.46-7.33 (m, 2H), 4.24 (dd, *J=* 4.9, 13.5 Hz, 1H), 4.02 (dd, *J=* 9.9, 13.5 Hz, 1H), 3.79 (s, 3H), 3.05-2.94 (m, 1H), 2.61 (s, 3H), 2.37 (s, 3H), 1.24-1.21 (m, 1H), 1.23 (d, *J* = 6.8 Hz, 2H). **WX141:** ¹H NMR (400MHz, METHANOL-d4) δ = 8.37 (d, *J* = 2.0 Hz, 1H), 8.20 (d, *J* = 2.2 Hz, 1H), 8.18 (s, 1H), 8.06-8.01 (m, 2H), 7.76 (d, *J=* 8.6 Hz, 1H), 7.65 (s, 1H), 7.60 (d, *J=* 7.3 Hz, 1H), 7.46-7.33 (m, 2H), 4.24 (dd, *J* = 4.9, 13.5 Hz, 1H), 4.02 (dd, *J* = 9.9, 13.5 Hz, 1H), 3.78 (s, 3H), 3.04-2.95 (m, 1H), 2.61 (s, 3H), 2.37 (s, 3H), 1.23 (d, *J=* 7.1 Hz, 3H).

### Example 77: WX142, WX143

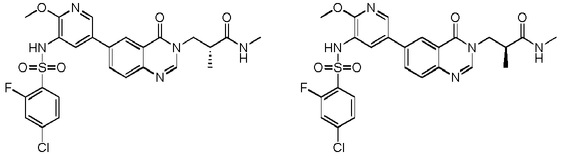

### Synthetic route:

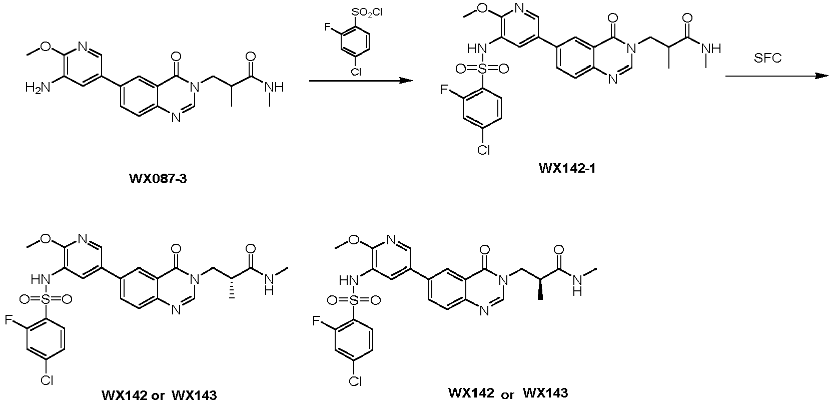

### Step 1: synthesis of compound WX142-1

**WX087-3** (748.14 mg, 3.27 mmol), 2-fluoro-4-chlorobenzenesulfonyl chloride (200 mg, 544.36 µmol) and the solvent pyridine (10 mL) were added into a pre-dried reaction flask, and stirred at 25 °C for 12 hours. After the reaction was completed, water (5 mL) was added to the reaction solution, followed by extraction with dichloromethane (10 mL × 3). The obtained organic phase was dried over anhydrous sodium sulfate, and then rotary-evaporated under reduced pressure to obtain a crude product, which was separated and purified by preparative HPLC to obtain target compound **WX142-1.**

### Step 2: synthesis of compound WX142, WX143

**WX142-1** was resolved by SFC (chromatographic column: AS (250mm ^{∗} 30mm, 5µm); mobile phase: [MeOH]; B%: 45% -45%, 6min) to obtain the enantiomers **WX142** (retention time is 2.750min) and **WX143** (retention time is 2.765min). **WX142:** ¹H NMR (400MHz, METHANOL-d4) δ = 8.38 (s, 1H), 8.27 (s, 1H), 8.21 (s, 1H), 8.07-8.01 (m, 2H), 7.82-7.74 (m, 2H), 7.46 (d, *J* = 9.9 Hz, 1H), 7.34 (d, *J* = 8.4 Hz, 1H), 4.24 (dd, *J* = 4.7, 13.4 Hz, 1H), 4.03 (dd, *J* = 10.0, 13.4 Hz, 1H), 3.82 (s, 3H), 3.31 (s, 25H), 3.04-2.93 (m, 1H), 2.62 (s, 3H), 1.24 (d, *J* = 7.0 Hz, 3H). **WX143:** ¹H NMR (400MHz, METHANOL-*d₄*) δ = 8.39 (s, 1H), 8.28 (s, 1H), 8.22 (s, 1H), 8.09-8.01 (m, 2H), 7.83-7.74 (m, 2H), 7.47 (d, *J* = 9.7 Hz, 1H), 7.34 (d, *J* = 8.3 Hz, 1H), 4.24 (dd, *J* = 5.0, 13.3 Hz, 1H), 4.10-3.98 (m, 1H), 3.82 (s, 3H), 3.15-2.95 (m, 1H), 2.62 (s, 3H), 1.31-1.20 (m, 4H).

### Example 78: WX144, WX145

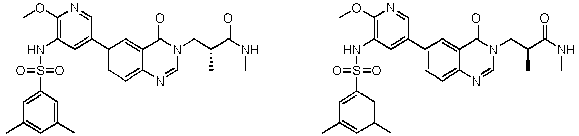

### Synthetic route:

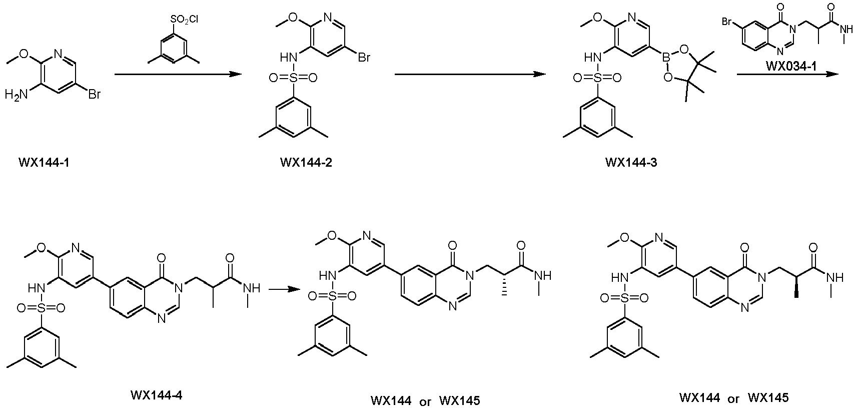

### Step 1: synthesis of compound WX144-2

Raw materials **WX144-1** (1 g, 4.93 mmol) and 3,5-dimethylbenzenesulfonyl chloride (1.51 g, 7.39 mmol) and the solvent pyridine (10 mL) were added into a pre-dried reaction flask and stirred at 0 °C for 12 hours. After the reaction was completed, water (5 mL) was added to the reaction solution, followed by extraction with dichloromethane (10 mL × 3). The organic phase obtained was dried over anhydrous sodium sulfate, rotary-evaporated under reduced pressure, separated and purified by flash column chromatography (petroleum ether: ethyl acetate ester = 1:0 to 10:1) to obtain the target compound **WX144-2.** ¹H NMR (400MHz, METHANOL-d4) δ = 7.90 (d, *J=* 2.2 Hz, 1H), 7.83 (d, *J=* 2.4 Hz, 1H), 7.39 (s, 2H), 7.24 (s, 1H), 7.25-7.23 (m, 1H), 3.74 (s, 4H), 2.33 (s, 8H).

### Step 2: synthesis of compound WX144-3

**WX144-2** (280 mg, 754.21 µmol), bis(pinacolato)diboron (191.52 mg, 754.21 µmol) and the solvent 1,4-dioxane (3 mL) were added into a pre-dried reaction flask, and then potassium acetate (148.04 mg, 1.51 mmol) was added thereto. The mixture was replaced with nitrogen, followed by addition of 1,1-bis(diphenylphosphine) ferrocene palladium chloride (55.19 mg, 75.42 µmol). The mixture was replaced with nitrogen, and further stirred at 90 °C for 12 hours. After the reaction was completed, the target compound **WX144-3** was obtained, which was directly used in the next reaction.

### Step 3: synthesis of compound WX144-4

Raw materials **WX144-3** (210.00 mg, 647.80 µmol) and **WX034-1** (298.08 mg, 712.58 µmol), and the solvents water (0.5 mL) and 1,4-dioxane (2 mL) were added into a pre-dried reaction flask, then potassium acetate (127.15 mg, 1.30 mmol) was added thereto. The mixture was replaced with nitrogen, followed by addition of 1,1-bis(diphenylphosphine) ferrocene palladium chloride (47.40 mg, 64.78 µmol) . The mixture was replaced with nitrogen, and further stirred at 80 °C for 12 hours. After the reaction was completed, water (2 mL) was added to the reaction solution, followed by extraction with dichloromethane (5 mL × 3). The obtained organic phase was dried over anhydrous sodium sulfate, separated and purified by preparative thin layer chromatography (dichloromethane: methanol = 15:1), and further purified by preparative HPLC to obtain target compound **WX144-4.**

### Step 4: synthesis of compound WX144, WX145

**WX144-4** was resolved by SFC (chromatographic column: AS (250mm ^{∗} 30mm, 5µm); mobile phase: [MeOH]; B%: 45%-45%, 7 min) to obtain a pair of enantiomers **WX144** (retention time is 2.725 min) and **WX145** (retention time is 2.727 min). **WX144:** ¹H NMR (400MHz, CHLOROFORM-d) δ = 8.35 (d, *J* = 1.8 Hz, 1H), 8.14-8.10 (m, 2H), 8.03 (d, *J* = 2.2 Hz, 1H), 7.88-7.83 (m, 1H), 7.80-7.76 (m, 1H), 7.46 (s, 2H), 7.18 (s, 1H), 7.04 (s, 1H), 5.75 (br s, 1H), 4.22-4.13 (m, 1H), 4.10-4.00 (m, 1H), 3.91 (s, 3H), 3.02-2.91 (m, 1H), 2.74 (d, *J=* 4.9 Hz, 3H), 2.34 (s, 6H), 1.73 (br s, 6H), 1.29 (d, *J* = 6.8 Hz, 3H). **WX145:** ¹H NMR (400MHz, CHLOROFORM-d) δ = 8.35 (d, *J* = 1.8 Hz, 1H), 8.12 (s, 2H), 8.03 (d, *J* = 2.2 Hz, 1H), 7.90-7.83 (m, 1H), 7.82-7.72 (m, 1H), 7.46 (s, 2H), 7.18 (s, 1H), 7.05 (s, 1H), 5.77 (br d, *J=* 4.6 Hz, 1H), 4.23-4.11 (m, 1H), 4.09-3.97 (m, 1H), 3.91 (s, 3H), 3.03-2.89 (m, 1H), 2.74 (d, *J=* 4.9 Hz, 3H), 2.34 (s, 6H), 1.82-1.66 (m, 6H), 1.29 (d, *J=* 6.8 Hz, 3H).

### Example 79: WX146, WX147

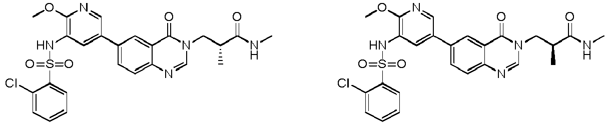

### Synthetic route:

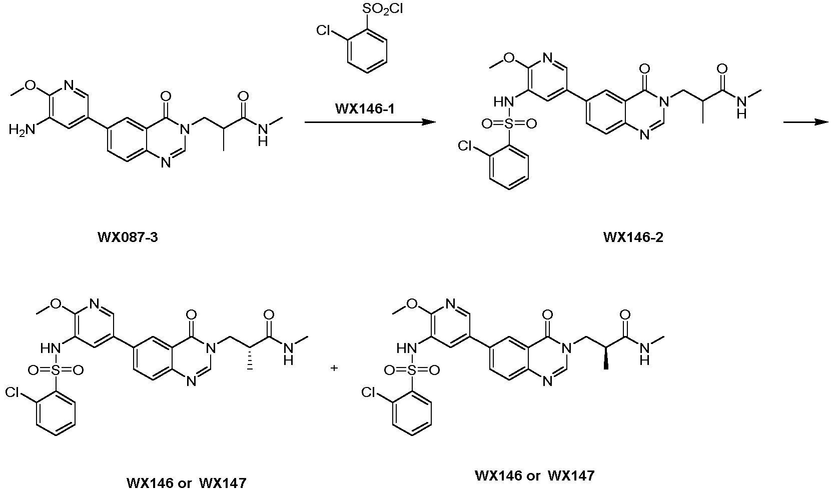

### Step 1: synthesis of compound WX146-2

**WX087-3** (150 mg, 408.27 µmol) and **WX146-1** (517.03 mg, 2.45 mmol, 333.57 µL) were added into a pre-dried reaction flask, followed by addition of pyridine (1 mL). The reaction solution was stirred at 20 °C for 12 hours. After the reaction was completed, 10 mL of water and 10 mL of ethyl acetate were added to the reaction system for dilution. The organic phase was collected after liquid separation, and the aqueous phase was extracted with ethyl acetate (5 mL × 3). The organic phases were combined, washed with saturated brine (20 mL), dried over anhydrous sodium sulfate, concentrated under reduced pressure, and separated by preparative HPLC (method: column: Agela Durashell C18 150 ^{∗} 25mm 5 µm; mobile phase: [water (10mM NH4HCO3) -ACN]; B%: 33%-63%, 10 min) to obtain target compound **WX146-2.**

### Step 2: synthesis of compound WX146 and WX147

**WX146-2** was resolved by SFC (resolution method: chromatographic column: AS (250mm ^{∗} 30mm, 5µm); mobile phase: [MeOH]; B%: 45% -45%, 8min) to obtain a pair of enantiomers **WX146** (Rt = 1.289 min) and **WX147** (Rt = 1.601 min). **WX146:** ¹H NMR (400MHz, METHANOL-d4) δ = 8.31 (d, *J* = 2.2 Hz, 1H), 8.21-8.15 (m, 2H), 8.05 (dd, *J* = 1.3, 7.9 Hz, 1H), 8.01-7.96 (m, 2H), 7.75 (d, *J* = 8.4 Hz, 1H), 7.65-7.53 (m, 2H), 7.49-7.42 (m, 1H), 4.23 (dd, *J* = 4.9, 13.5 Hz, 1H), 4.02 (dd, *J* = 9.9, 13.5 Hz, 1H), 3.85 (s, 3H), 3.05-2.91 (m, 1H), 2.61 (s, 3H), 1.23 (d, *J* = 7.1 Hz, 3H). **WX147:** ¹H NMR (400MHz, METHANOL-d4) δ = 8.30 (d, *J* = 2.0 Hz, 1H), 8.20-8.15 (m, 2H), 8.05 (dd, *J* = 1.3, 7.9 Hz, 1H), 8.01-7.95 (m, 2H), 7.75 (d, *J* = 8.4 Hz, 1H), 7.62-7.52 (m, 2H), 7.46 (ddd, *J* = 1.8, 7.1, 7.9 Hz, 1H), 4.23 (dd, *J* = 4.9, 13.5 Hz, 1H), 4.02 (dd, *J* = 9.9, 13.5 Hz, 1H), 3.05-2.94 (m, 1H), 1.23 (d, *J* = 7.1 Hz, 3H).

### Example 80: WX148, WX149

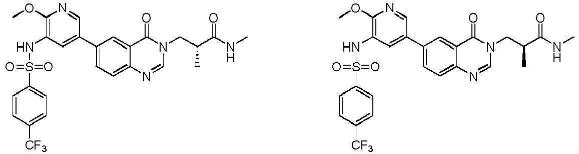

### Synthetic route:

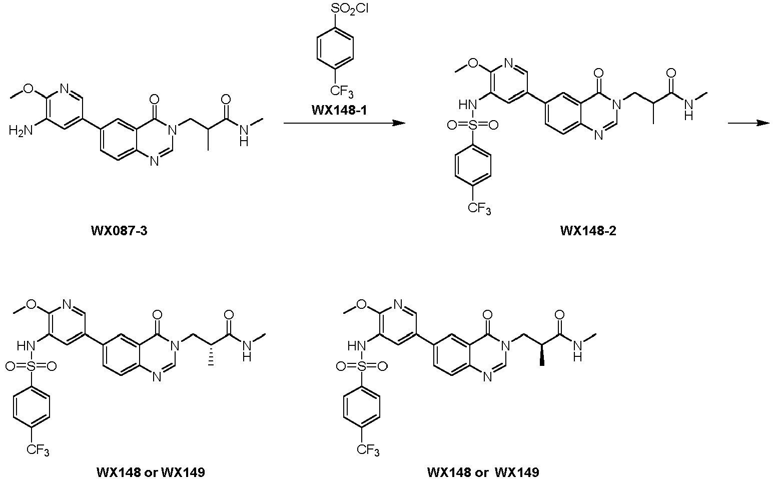

### Step 1: synthesis of compound WX148-2

**WX087-3** (200 mg, 544.36 µmol) and **WX148-1** (266.32 mg, 1.09 mmol, 5.56 µL) were added into a pre-dried reaction flask, followed by addition of pyridine (5 mL). The reaction solution was stirred at 20 °C for 12 hours. After the reaction was completed, 10 mL of water and 10 mL of ethyl acetate were added to the reaction system for dilution. The organic phase was collected after liquid separation, and the aqueous phase was extracted with ethyl acetate (5 mL × 3). The organic phases were combined, washed with saturated brine (20 mL), dried over anhydrous sodium sulfate, concentrated under reduced pressure, and separated by preparative HPLC (method: column: Agela Durashell C18 150 ^{∗} 25mm 5µm; mobile phase: [water (10mM NH4HCO3) -ACN]; B%: 33%-63%, 10 min) to obtain target compound **WX148-2.**

### Step 2: synthesis of compound WX148 and WX149

**WX148-2** was resolved and purified by SFC (purification method: column: AS (250mm ^{∗} 30mm, 5µm); mobile phase: [MeOH]; B%: 45% -45%, 7min) to obtain a pair of enantiomers **WX148** (Rt = 0.995 min) and **WX149** (Rt = 1.162 min). **WX148:** ¹H NMR (400MHz, METHANOL-d₄) δ = 8.40 (d, *J* = 2.2 Hz, 1H), 8.26 (d, *J* = 2.2 Hz, 1H), 8.18 (s, 1H), 8.10 (d, *J* = 2.4 Hz, 1H), 8.05 (dd, *J* = 2.2, 8.6 Hz, 1H), 7.97 (d, *J* = 8.4 Hz, 2H), 7.84 (d, *J* = 8.4 Hz, 2H), 7.77 (d, *J* = 8.6 Hz, 1H), 4.24 (dd, *J* = 5.0, 13.3 Hz, 1H), 4.03 (dd, *J* = 9.8, 13.3 Hz, 1H), 3.70 (s, 3H), 3.07-2.92 (m, 1H), 2.61 (s, 3H), 1.23 (d, *J* = 7.1 Hz, 3H). **WX149:** ¹H NMR (400MHz, METHANOL-d₄) δ = 8.41 (d, *J* = 2.0 Hz, 1H), 8.28 (d, *J* = 2.4 Hz, 1H), 8.19 (s, 1H), 8.11 (d, *J* = 2.4 Hz, 1H), 8.06 (dd, *J* = 2.2, 8.4 Hz, 1H), 7.97 (d, *J* = 8.2 Hz, 2H), 7.84 (d, *J* = 8.4 Hz, 2H), 7.78 (d, *J* = 8.6 Hz, 1H), 4.24 (dd, *J* = 4.9, 13.5 Hz, 1H), 4.03 (dd, *J* = 9.9, 13.5 Hz, 1H), 3.70 (s, 3H), 3.05-2.94 (m, 1H), 1.23 (d, *J* = 7.1 Hz, 3H).

### Example 81: WX150, WX151

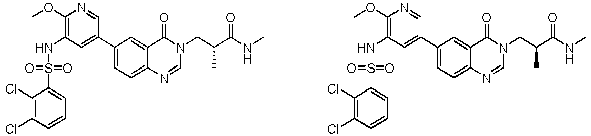

### Synthetic route:

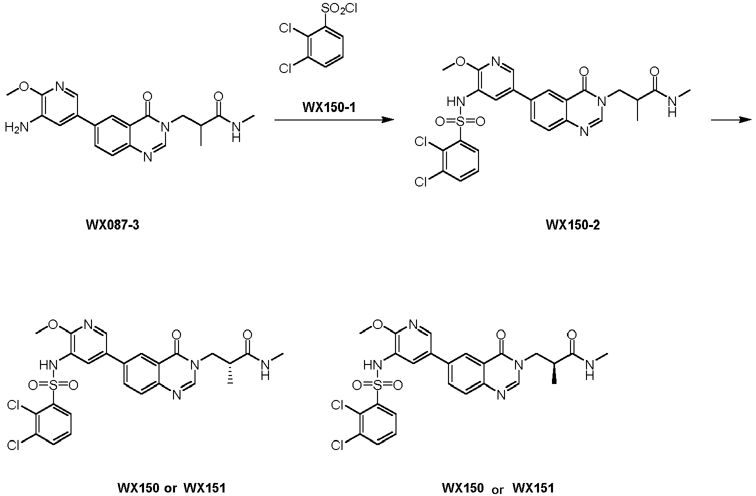

### Step 1: synthesis of compound WX150-2

**WX087-3** (150 mg, 408.27 µmol) and **WX150-1** (601.41 mg, 2.45 mmol, 5.94 µL) were added into a pre-dried reaction flask, followed by addition of pyridine (5 mL). The reaction solution was stirred at 20 °C for 12 hours. After the reaction was completed, 10 mL of water and 10 mL of ethyl acetate were added to the reaction system for dilution. The organic phase was collected after liquid separation, and the aqueous phase was extracted with ethyl acetate (5 mL × 3). The organic phases were combined, washed with saturated brine (20 mL), dried over anhydrous sodium sulfate, concentrated under reduced pressure, and separated by preparative HPLC (method: column: column: Agela Durashell C18 150 ^{∗} 25mm 5µm; mobile phase: [water (10mM NH4HCO3) -ACN]; B%: 33% -63%) to obtain target compound **WX150-2.**

### Step 2: synthesis of compound WX150 and WX151

**WX150-2** was resolved and purified by SFC (resolution method: chromatography column: AS (250mm ^{∗} 30mm, 5µm); mobile phase: [MeOH]; B%: 45% -45%, 10min) to obtain a pair of enantiomers **WX150** (Rt = 1.406 min) and **WX151** (Rt = 1.749 min). **WX150:** ¹H NMR (400MHz, METHANOL-d₄) δ = 8.31 (d, *J* = 2.0 Hz, 1H), 8.20 (d, *J*= 2.2 Hz, 1H), 8.17 (s, 1H), 8.02-7.95 (m, 3H), 7.80-7.71 (m, 2H), 7.42 (t, *J* = 8.0 Hz, 1H), 4.23 (dd, *J* = 4.9, 13.5 Hz, 1H), 4.02 (dd, *J* = 9.7, 13.5 Hz, 1H), 3.82 (s, 3H), 3.05-2.92 (m, 1H), 2.61 (s, 3H), 1.23 (d, *J* = 7.1 Hz, 3H). **WX151:** ¹H NMR (400MHz, METHANOL-d₄) δ = 8.34 (d, *J* = 2.0 Hz, 1H), 8.27 (s, 1H), 8.23 (d, *J* = 2.2 Hz, 1H), 8.06-7.94 (m, 3H), 7.83-7.72 (m, 2H), 7.42 (t, *J* = 8.0 Hz, 1H), 4.24 (dd, *J=* 4.9, 13.5 Hz, 1H), 4.11-3.95 (m, 1H), 3.82 (s, 3H), 3.06-2.93 (m, 1H), 2.61 (s, 3H), 1.23 (d, *J*= 7.1 Hz, 3H).

### Example 82: WX152, WX153

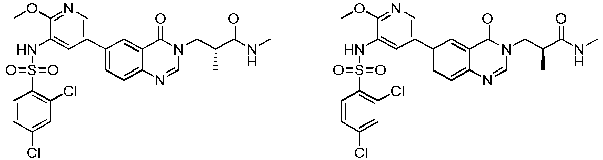

### Synthetic route:

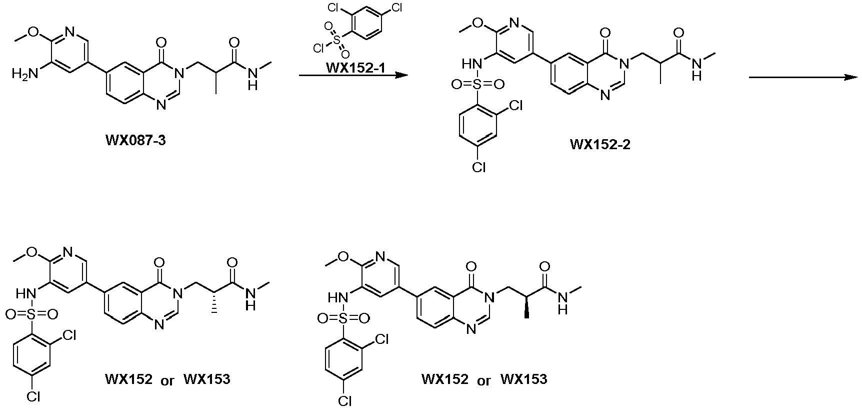

### Step 1: synthesis of compound WX152-2

**WX087-3** (0.15 g, 269.58 µmol) and **WX152-1** (0.1 g, 407.31 µmol) were added to pyridine (1 mL) and reacted at 25 °C for 16 hours. After the reaction was completed, the reaction solution was rotary-evaporated, diluted with water (50 mL), extracted with DCM (50 mL). The organic phase was rotary-evaporated, and the residue was purified by preparative TLC (petroleum ether: ethyl acetate = 0:1) to obtain target compound **WX152-2.**

### Step 2: synthesis of compound WX152 and WX153

**WX152-2** was resolved by SFC (column: OJ (250mm ^{∗} 30mm, 5µm); mobile phase: [0.1% NH₄HCO₃ EtOH]; B%: 30%-30%) to obtain enantiomers **WX152** (rt = 3.450 min) and **WX153** (rt = 3.827min). **WX152:** ¹H NMR (400 MHz, DMSO-*d₆*) δ ppm 10.33 (br s, 1 H), 8.38 (s, 1 H), 8.26 (d, *J=* 2.0 Hz, 1 H), 8.19 (s, 1 H), 8.08 (dd, *J* = 8.5, 2.5 Hz, 1 H), 7.86-7.94 (m, 4 H), 7.76 (d, *J=* 8.5 Hz, 1 H), 7.58 (dd, *J=* 8.5, 2.0 Hz, 1 H), 4.04-4.12 (m, 1 H), 3.92-4.02 (m, 1 H), 3.69 (s, 3 H), 2.87 (br dd, *J* = 14.8, 6.8 Hz, 1 H), 2.49 (br s, 3 H), 1.09 (d, *J* = 7.0 Hz, 3 H). **WX153:** ¹H NMR (400 MHz, DMSO-*d₆*) δ ppm 10.33 (br s, 1 H), 8.37 (s, 1 H), 8.26 (d, *J=* 2.0 Hz, 1 H), 8.19 (s, 1 H), 8.07 (dd, *J* = 8.5, 2.5 Hz, 1 H), 7.86-7.95 (m, 4 H), 7.76 (d, *J* = 8.5 Hz, 1 H), 7.58 (dd, *J=* 8.5, 2.0 Hz, 1 H), 4.04-4.13 (m, 1 H), 3.90-4.02 (m, 1 H), 3.69 (s, 3 H), 2.87 (br dd, *J* = 15.1, 6.5 Hz, 1 H), 2.49 (br s, 3 H), 1.09 (d, *J* = 7.0 Hz, 3 H).

### Example 83: WX154, WX155

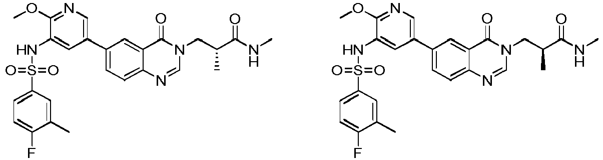

### Synthetic route:

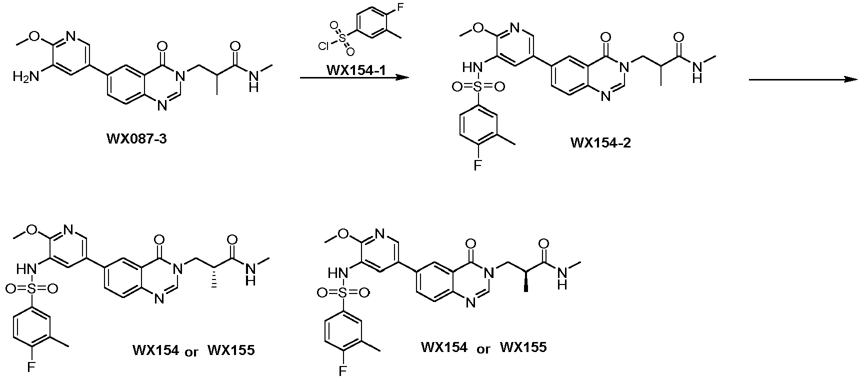

### Step 1: synthesis of compound WX154-2

**WX087-3** (0.15 g, 269.58 µmol) and **WX154-1** (0.084 g, 402.61 µmol) were added to pyridine (1 mL) and reacted at 25 °C for 16 hours. After the reaction was completed, the reaction solution was rotary-evaporated, diluted with water (50 mL), extracted with DCM (50 mL). The organic phase was rotary-evaporated, and the residue was purified by preparative TLC (petroleum ether: ethyl acetate = 0:1) to obtain target compound **WX154-2.**

### Step 2: synthesis of compound WX154 and WX155

**WX154-2** was resolved by SFC (chromatographic column: AD (250mm ^{∗} 30mm, 5µm); mobile phase: [0.1% NH₄HCO₃ EtOH]; B%: 55% -55%) to obtain enantiomers **WX154** (rt = 2.854 min) and **WX155** (rt = 2.999min). WX154: ¹H NMR (400 MHz, DMSO-*d₆*) δ ppm 10.11 (br s, 1 H), 8.40 (d, *J* = 2.0 Hz, 1 H), 8.21-8.33 (m, 2 H), 8.12 (dd, *J=* 8.5, 2.0 Hz, 1 H), 7.95 (d, *J=* 2.0 Hz, 2 H), 7.77-7.86 (m, 2 H), 7.65-7.73 (m, 1 H), 7.40 (t, *J=* 9.0 Hz, 1 H), 3.97-4.21 (m, 2 H), 3.77 (s, 3 H), 2.92 (br dd, *J* = 14.8, 6.8 Hz, 1 H), 2.54 (br s, 3 H), 2.34 (s, 3 H), 1.14 (d, *J* = 6.5 Hz, 3 H). WX155: ¹H NMR (400 MHz, DMSO-*d₆*) δ ppm 10.12 (br s, 1 H), 8.41 (d, *J* = 2.0 Hz, 1 H), 8.29 (d, *J* = 2.0 Hz, 1 H), 8.24 (s, 1 H), 8.12 (dd, *J* = 8.5, 2.0 Hz, 1 H), 7.95 (d, *J* = 2.0 Hz, 2 H), 7.77-7.84 (m, 2 H), 7.65-7.73 (m, 1 H), 7.39 (t, *J=* 9.0 Hz, 1 H), 3.95-4.19 (m, 2 H), 3.76 (s, 3 H), 2.83-2.98 (m, 1 H), 2.53 (br s, 3 H), 2.33 (s, 3 H), 1.13 (d, *J* = 6.5 Hz, 3 H).

### Example 84: WX156, WX157

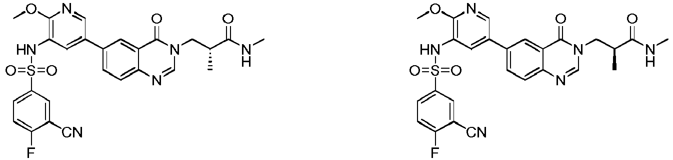

### Synthetic route:

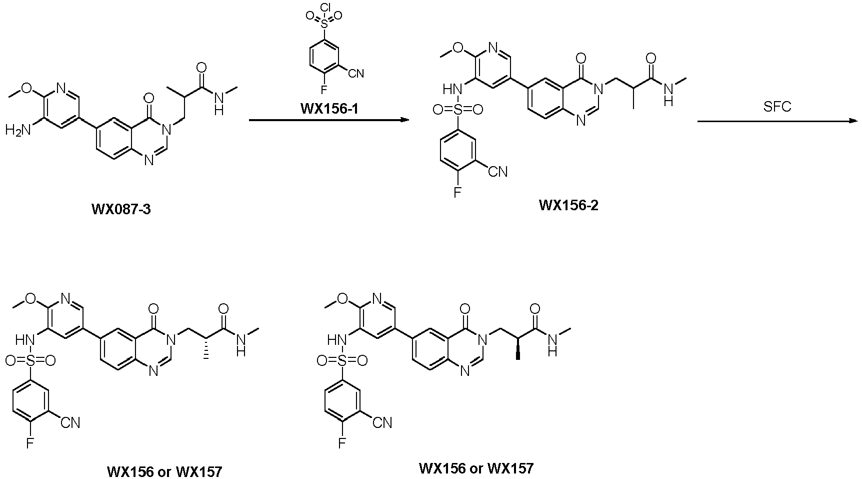

### Step 1: synthesis of compound WX156-2

**WX087-3** (0.15 g, 408.27 µmol) was dissolved in pyridine (3.0 mL), and **WX156-1** (116.56 mg, 530.75 µmol) was added dropwise at 25 °C, and the reaction solution was stirred at 30 °C for 16 hours to complete the reaction. The reaction solution was rotary-evaporated, followed by addition of water (10.00 mL) was added, washed three times with dichloromethane (10.00 mL). The organic phases were combined, dried over anhydrous sodium sulfate, and rotary-evaporated to obtain target compound **WX156-2.** MS-ESI m/z: 573.1[M+Na]⁺,575.1[M+Na+2]⁺.

### Step 2: synthesis of compound WX156, WX157

**WX156-2** was resolved and purified by SFC (chromatographic column: OD (250mm ^{∗} 30mm, 5µm); mobile phase: [0.1% NH₄HCO₃ MEOH]; B%: 45% -45%) to obtain a pair of enantiomers **WX156** (Rt = 1.632 min) and **WX157** (Rt = 1.892 min). **WX156:** ¹H NMR (400MHz, CDCl₃) δ : 8.31 (s, 1H), 8.16 (d, *J* = 2.0 Hz, 1H), 8.11-8.02 (m, 2H), 7.98 (br d, *J* = 2.3 Hz, 2H), 7.88-7.80 (m, 1H), 7.79-7.68 (m, 1H), 7.28 (t, *J=* 8.5 Hz, 1H), 5.54 (br s, 1H), 4.23-4.04 (m, 1H), 4.04-3.90 (m, 1H), 3.85 (s, 3H), 2.89 (brs, 1H), 2.67 (d, *J* = 4.8 Hz, 3H). **WX157**:¹H NMR (400MHz, CDCl₃) δ : 8.31 8.36-8.29 (m, 1H), 8.37-8.15 (m, 1H), 8.21-8.14 (m, 1H), 8.10-8.07 (m, 1H), 8.06 (s, 1H), 8.02-7.92 (m, 2H), 7.90-7.80 (m, 1H), 7.80-7.68 (m, 1H), 7.28 (t, *J=* 8.5 Hz, 1H), 6.95 (s, 1H), 5.54 (br s, 1H), 4.03-3.92 (m, 1H), 3.85 (s, 3H), 2.96-2.83 (m, 1H), 2.67 (d, *J* = 4.8 Hz, 3H).

### Example 85: WX158, WX159

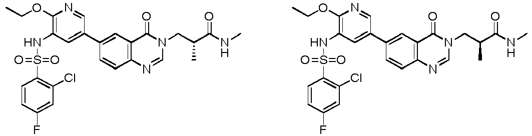

### Synthetic route:

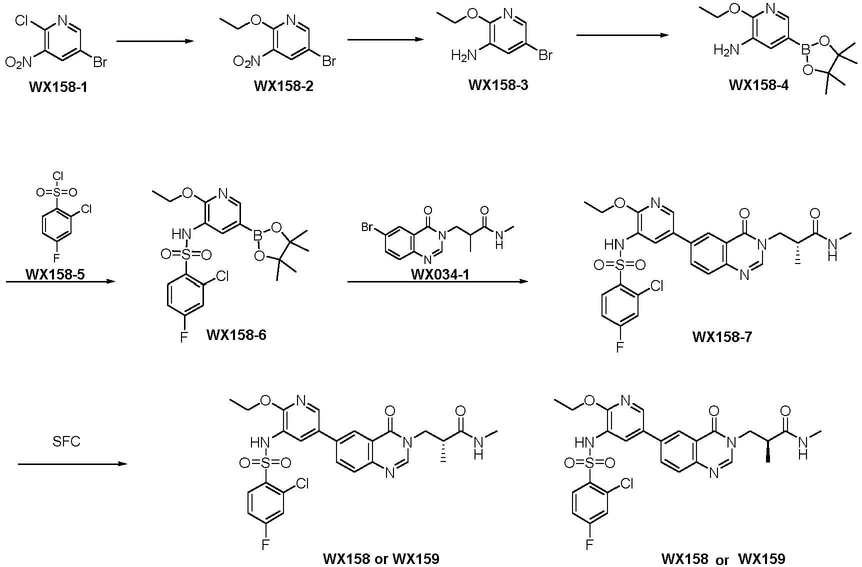

### Step 1: synthesis of compound WX158-1

**WX158-1** (2 g, 8.42 mmol) and sodium ethoxide (5 g, 73.48 mmol) were added to ethanol (100 mL), and reacted at 75 °C for 16 hours. After the reaction was completed, the reaction solution was rotary-evaporated to dryness and diluted with water (100 mL) and extracted with EA (50 mL × 3). The organic phases were collected and rotary-evaporated to obtain target compound **WX158-2.**

### Step 2: synthesis of compound WX158-3

**WX158-2** was dissolved in methanol (100 mL) and dichloromethane (10 mL), then zinc powder (1.90 g, 29.03 mmol) and ammonium chloride (1.43 g, 26.73 mmol) were added and stirred at 4 °C for 4 hours. After the reaction was completed, the reaction solution was filtered. The filtrate was collected, rotary-evaporated, diluted with water (50 mL), extracted with ethyl acetate (50 mL × 3). The organic phase was rotary-evaporated to obtain target compound **WX158-3,** which was used directly in the next step. MS-ESI *m*/*z:* 216.9 [M+H]⁺, 218.9[M+H+2]⁺.

### Step 3: synthesis of compound WX158-4

**WX158-3** (1 g, 4.61 mmol), bis(pinacolato)diboron (1.20 g, 4.73 mmol), Pd(dppf)Cl₂ (400.00 mg, 546.67 µmol) and KOAc (1.40 g, 14.27 mmol) were added to dioxane (50 mL), and was replaced with nitrogen three times. The reaction was carried out at 105 °C for 16 hours under nitrogen atmosphere. After the reaction was completed, the reaction solution was rotary-evaporated, diluted with water (50 mL), and then extracted with ethyl acetate (50 mL × 3). The organic phase was rotary-evaporated, separated and purified by column chromatography (PE: EA = 0%-10%) to obtain the target compound **WX158-4.** MS-ESI *m*/*z*: 265.1 [M+H]⁺.

### Step 4: synthesis of compound WX158-6

**WX158-4** (1.2 g, 4.54 mmol) was dissolved in pyridine (50 mL), and **WX158-5** (1.10 g, 4.80 mmol, 0.7 mL) were dropwise added thereto, and stirred at 30 °C for 16 hours. After the reaction was completed, the reaction solution was rotary-evaporated, diluted with water (50 mL), extracted with dichloromethane (50 × 3 mL). The organic phase was rotary-evaporated to obtain target compound **WX158-6.** MS-ESI *m*/*z*: 375.0 [M+H]⁺.

### Step 5: synthesis of compound WX158-7

**WX034-1** (0.17 g, 396.61 µmol), **WX158-6** (0.23 g, 503.69 µmol), Pd(dppf)Cl₂ (0.03 g, 41.00 µmol, 1.03e-1 eq) and KOAc (0.12 g, 1.22 mmol) were dissolved in dioxane (10 mL) and water (2 mL), replaced with nitrogen three times, and reacted at 105 °C for 2 hours. After the reaction was completed, the reaction solution was rotary-evaporated, diluted with water (50 mL), and then extracted with ethyl acetate (50 mL × 3). The organic phase was collected, rotary-evaporated, separated and purified by column chromatography (petroleum ether: ethyl acetate = 0%-50%) to obtain target compound **WX158-7.**

### Step 6: synthesis of compound WX158 and WX159

**WX158-7** was resolved by SFC (column: Chiralpak AS-H 250 ^{∗} 30mm 5µm; mobile phase: [0.1% NH₄HCO₃ EtOH]; B%: 35% -35%) to obtain the enantiomers **WX158** (RT = 3.525min) and **WX159** (RT = 3.996min). **WX158:** ¹H NMR (400 MHz, CHLOROFORM-d) δ ppm 1.21 (br d, *J* = 7.03 Hz, 3 H) 1.33 (t, *J=* 7.03 Hz, 3 H) 2.67 (d, *J=* 4.77 Hz, 3 H) 2.84-2.94 (m, 1 H) 3.89-4.02 (m, 1 H) 4.04-4.16 (m, 1 H) 4.25-4.43 (m, 2 H) 5.68 (br s, 1 H) 6.91-7.11 (m, 1 H) 7.11-7.25 (m, 2 H) 7.60-7.83 (m, 2 H) 7.91 (s, 1 H) 7.98-8.09 (m, 3 H) 8.20 (s, 1 H), MS-ESI *m*/*z*: 574.1 [M+H]⁺. **WX159:** ¹H NMR (400 MHz, CHLOROFORM-d) δ ppm 1.15-1.22 (m, 3 H) 1.33 (t, *J=* 7.15 Hz, 3 H) 2.67 (d, *J=* 4.77 Hz, 3 H) 2.80-2.98 (m, 1 H) 3.91-4.03 (m, 1 H) 4.04-4.16 (m, 1 H) 4.34 (q, *J=* 7.03 Hz, 2 H) 5.71 (br s, 1 H) 6.97-7.08 (m, 1 H) 7.13-7.28 (m, 2 H) 7.62-7.80 (m, 2 H) 7.91 (d, *J* = 1.76 Hz, 1 H) 7.97-8.10 (m, 3 H) 8.20 (s, 1 H), MS-ESI *m*/*z*: 574.1 [M+H]⁺.

### Example 86: WX160, WX161

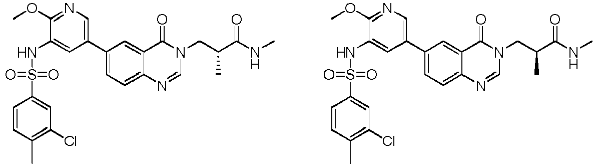

### Synthetic route:

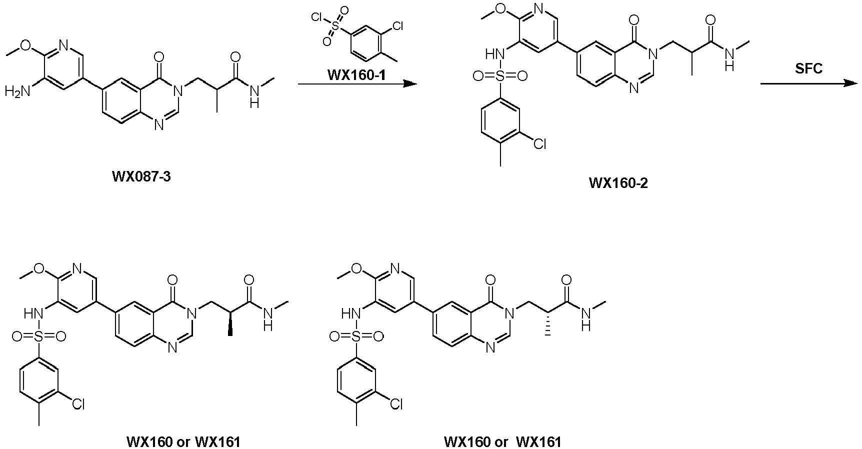

### Step 1: synthesis of compound WX160-2

Compound **WX087-3** (0.15 g, 408.27 µmol) was dissolved in pyridine (3 mL). Compound **WX160-1** (110.28 mg, 489.93 µmol) was added thereto, and the reaction solution was stirred at 25 °C for 10 hours. TLC (ethyl acetate: methanol = 10: 1) showed that the reaction of the raw materials was complete. The reaction solution was rotary-evaporated, and the residue was separated by preparative silica gel plate (ethyl acetate: methanol = 10: 1) to obtain the target compound **WX160-2.**

### Step 2: synthesis of compound WX160 and WX161

Compound **WX160-2** was resolved by supercritical fluid chromatography (separation conditions: column: Chiralpak AS-H 250 ^{∗} 30mm 5µm; mobile phase: [0.1% NH₄HCO₃ ETOH]; B%: 45% -45%) to obtain the enantiomers **WX160** and **WX161,** the retention time of which is 4.085 min, 4.702 min respectively, and the ratio is 1:1. **WX160:** ¹HNMR (400 MHz, DMSO-*d₆*) δ = 8.29 (br s, 1 H), 8.22-8.27 (m, 1 H), 8.17 (s, 1 H), 8.05 (dd, *J* = 8.4, 2.1 Hz, 1 H), 7.89 (br d, *J* = 2.5 Hz, 2 H), 7.75 (d, *J* = 8.5 Hz, 1 H), 7.68 (d, *J* = 4.8 Hz, 1 H), 7.00 (d, *J* = 5.0 Hz, 1 H), 4.02-4.13 (m, 1 H), 3.96 (dd, *J=* 13.2, 9.2 Hz, 1 H), 3.75 (s, 3 H), 3.33 (br s, 3 H), 2.82-2.91 (m, 1 H), 2.33 (s, 3 H), 1.08 (d, *J* = 7.0 Hz, 3 H). MS-ESI *m*/*z*: 556.1 [M+H]⁺. **WX161:** ¹H NMR (400 MHz, DMSO-*d₆*) δ ppm 8.18-8.26 (m, 2 H), 8.17 (s, 1 H), 8.03 (dd, *J* = 8.4, 1.9 Hz, 1 H), 7.90 (br d, *J* = 4.5 Hz, 1 H), 7.83 (s, 1 H), 7.72-7.79 (m, 2 H), 7.55-7.61 (m, 1 H), 7.48-7.53 (m, 1 H), 4.04-4.11 (m, 1 H), 3.91-4.00 (m, 1 H), 3.71 (s, 3 H), 2.86 (br dd, *J* = 14.7, 6.9 Hz, 1 H), 2.48 (br s, 3 H), 2.36 (s, 3 H), 1.08 (d, *J* = 6.8 Hz, 3 H). MS-ESI *m*/*z*: 556.1 [M+H]⁺.

### Example 87: WX162, WX163

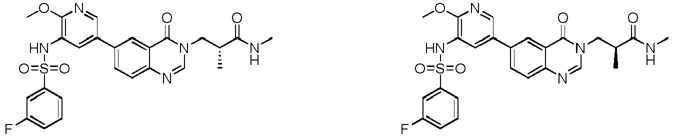

### Synthetic route:

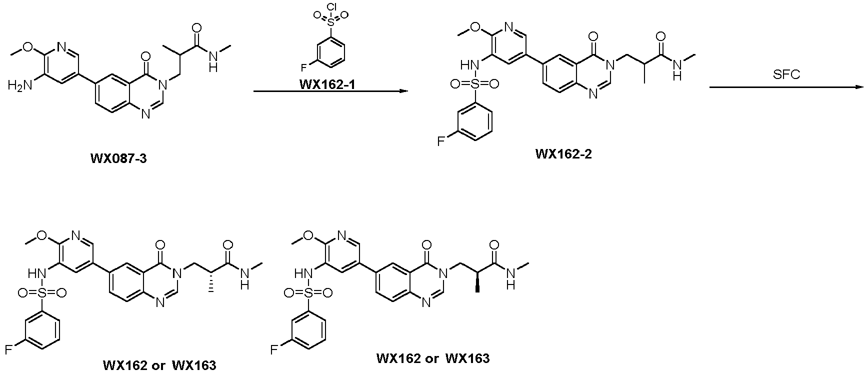

### Step 1: synthesis of compound WX162-2

**WX087-3** (0.15 g, 408.27 µmol) was dissolved in pyridine (3.0 mL), and **WX162-1** (103.29 mg, 530.76 µmol) was added dropwise at 25 °C. The reaction solution was stirred at 30 °C for 16 hours. After the reaction was completed, the reaction solution was rotary-evaporated, followed by addition of water (10.00 mL), and washed three times with dichloromethane (10.00 mL). The organic phases were combined, dried over anhydrous sodium sulfate, and rotary-evaporated to obtain target compound **WX162-2.** MS-ESI m/z: 526.2[M+H]⁺,528.2[M+H+2]⁺.

### Step 2: synthesis of compound WX162, WX163

**WX162-2** was resolved and purified by SFC (chromatographic column: OD (250mm ^{∗} 30mm, 5µm); mobile phase: [0.1% NH₄HCO₃ MEOH]; B%: 45% -45%) to obtain a pair of enantiomers **WX162** (Rt = 1.972 min) and **WX163** (Rt = 0.763 min). **WX162**:¹H NMR (400MHz, CDCl₃) δ : 8.31 (d, *J=* 2.0 Hz, 1H), 8.11 (d, *J* = 2.3 Hz, 1H), 8.05 (s, 1H), 8.00 (d, *J=* 2.0 Hz, 1H), 7.85-7.77 (m, 1H), 7.76-7.65 (m, 1H), 7.54 (d, *J* = 7.8 Hz, 1H), 7.47 (br d, *J* = 8.0 Hz, 1H), 7.40 (dt, *J* = 5.3, 8.0 Hz, 1H), 7.24-7.20 (m, 1H), 6.93 (br s, 1H), 5.51 (br d, *J=* 4.3 Hz, 1H), 4.11 (dd, *J* = 5.1, 13.2 Hz, 1H), 4.03-3.90 (m, 1H), 3.82 (s, 3H), 2.99-2.75 (m, 1H), 2.67 (d, *J=* 4.8 Hz, 3H), 1.22 (d, *J* = 7.0 Hz, 3H). **WX163**:¹H NMR (400MHz, Methanol-d4) δ: 8.41 (d, J = 2.0 Hz, 1H), 8.30-8.14 (m, 2H), 8.06 (dt, *J* = 2.3, 4.3 Hz, 2H), 7.79 (d, *J* = 8.5 Hz, 1H), 7.64-7.47 (m, 3H), 7.40-7.22 (m, 1H), 4.26 (dd, *J=* 4.8, 13.3 Hz, 1H), 4.04 (dd, *J* = 9.8, 13.3 Hz, 1H), 3.81 (s, 3H), 3.08-2.92 (m, 1H), 2.64 (s, 3H), 1.26 (d, *J=* 7.0 Hz, 3H).

### Example 88: WX164, WX165

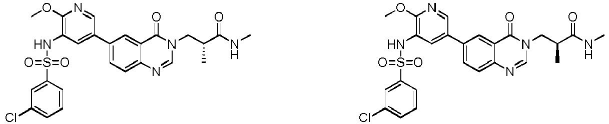

### Synthetic route:

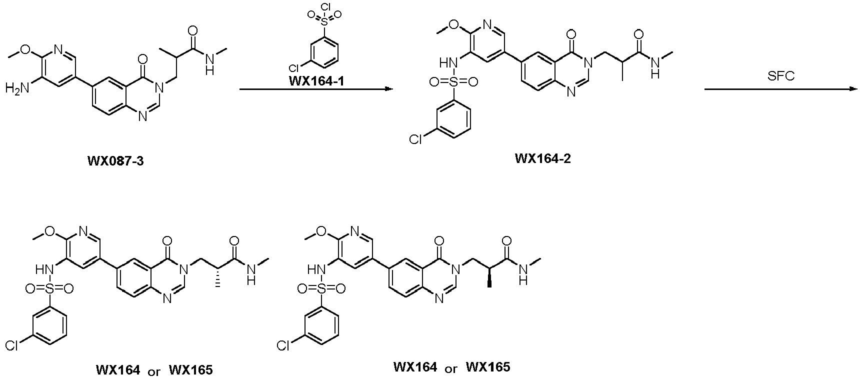

### Step 1: synthesis of compound WX164-2

**WX087-3** (0.15 g, 408.27 µmol) was dissolved in pyridine (3.0 mL), and **WX164-1** (129.26 mg, 612.41 µmol) was added dropwise at 25 °C. The reaction solution was stirred at 30 °C for 1 hour. The reaction solution was rotary-evaporated, followed by addition of water (10.00 mL), and washed three times with dichloromethane (10.00 mL). The organic phases were combined, dried over anhydrous sodium sulfate, and rotary-evaporated to obtain the target compound **WX164-2.** MS-ESI m/z: 542.0[M+H]⁺,544.0[M+H+2]⁺.

### Step 2: synthesis of compound WX164, WX165

**WX164-2** was resolved and purified by SFC (chromatographic column: OD (250mm ^{∗} 30mm, 5µm); mobile phase: [0.1% NH₄HCO₃ MEOH]; B%: 45% -45%) to obtain target compounds **WX164** (Rt = 6.203 min) and **WX165** (Rt = 5.777 min). **WX164:** ¹H NMR (400MHz, Methanol-d4) δ: 8.42 (s, 1H), 8.28 (d, *J* = 2.0 Hz, 1H), 8.21 (s, 1H), 8.15-7.99 (m, 2H), 7.85 (s, 1H), 7.80 (d, *J=* 8.5 Hz, 1H), 7.71 (d, *J=* 8.0 Hz, 1H), 7.63 (d, *J* = 9.0 Hz, 1H), 7.59-7.34 (m, 1H), 4.26 (dd, *J* = 5.0, 13.3 Hz, 1H), 4.16-3.93 (m, 1H), 3.80 (s, 3H), 3.10-2.92 (m, 1H), 2.64 (s, 3H), 1.26 (d, *J* = 7.0 Hz, 3H). **WX165:** ¹H NMR (400MHz, Methanol-d4) δ: 8.29 (d, *J* = 2.0 Hz, 1H), 8.15 (d, *J* = 2.3 Hz, 1H), 8.08 (s, 1H), 8.00-7.91 (m, 2H), 7.76-7.71 (m, 1H), 7.67 (d, *J* = 8.5 Hz, 1H), 7.60 (d, *J=* 7.8 Hz, 1H), 7.51 (d, *J* = 9.0 Hz, 1H), 7.45-7.30 (m, 1H), 4.14 (dd, *J=* 4.9, 13.4 Hz, 1H), 4.00-3.84 (m, 1H), 3.68 (s, 3H), 2.95-2.80 (m, 1H), 2.52 (s, 3H).

### Example 89: WX166, WX167

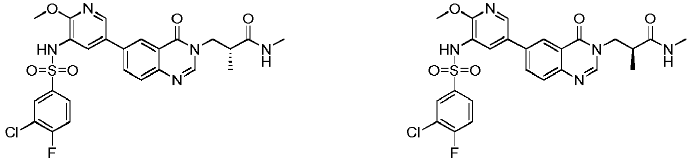

### Synthetic route:

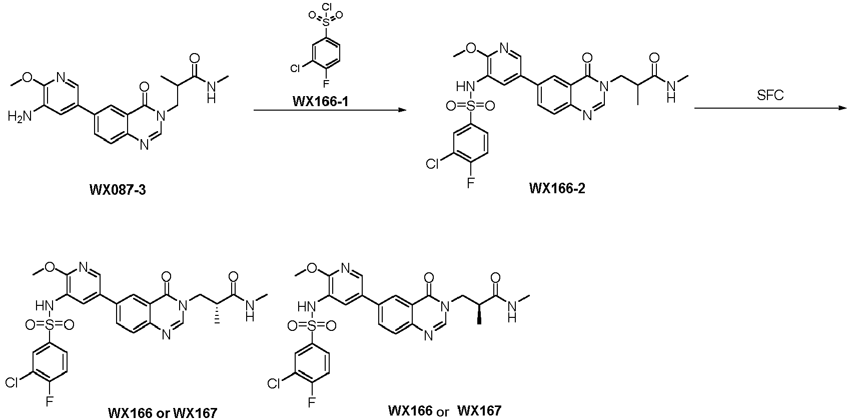

### Step 1: synthesis of compound WX166-2

**WX087-3** (0.15 g, 408.27 µmol) was dissolved in pyridine (3.0 mL), and **WX166-1** (121.57 mg, 530.75 µmol) was added dropwise at 25 °C. The reaction solution was stirred at 30 °C for 16 hours to complete the reaction. The reaction solution was rotary-evaporated, followed by addition of water (10.00 mL), and washed three times with dichloromethane (10.00 mL). The organic phases were combined, dried over anhydrous sodium sulfate, and rotary-evaporated to obtain target compound **WX166-2.** MS-ESI m/z: 560.1[M+H]⁺, 562.1[M+H+2]⁺.

### Step 2: synthesis of compound WX166, WX167

**WX166-2** was resolved and purified by SFC (chromatographic column: AD (250mm ^{∗} 30mm, 5µm); mobile phase: [0.1% NH₄HCO₃ IPA]; B%: 35% -35%) to obtain target compounds **WX166** (Rt = 0.913 min) and **WX167** (Rt = 2.422 min). **WX166:** ¹H NMR (400MHz, Methanol-d4) δ: 8.29 (d, *J=* 1.8 Hz, 1H), 8.17 (d, *J=* 2.0 Hz, 1H), 8.09 (s, 1H), 8.03-7.92 (m, 2H), 7.87 (dd, *J* = 2.0, 6.8 Hz, 1H), 7.74-7.60 (m, 2H), 7.29 (t, *J=* 8.7 Hz, 1H), 4.14 (dd, *J* = 4.8, 13.3 Hz, 1H), 4.03-3.81 (m, 1H), 3.69 (s, 3H), 3.00-2.81 (m, 1H), 2.52 (s, 3H), 1.14 (d, *J* = 7.0 Hz, 3H). **WX167:** ¹H NMR (400MHz, Methanol-d4) δ: 8.30 (d, *J=* 2.0 Hz, 1H), 8.15-8.05 (m, 2H), 8.01-7.91 (m, 2H), 7.88 (dd, *J* = 2.3, 6.8 Hz, 1H), 7.75-7.49 (m, 2H), 7.28 (t, *J* = 8.8 Hz, 1H), 4.14 (dd, *J* = 4.9, 13.4 Hz, 1H), 3.93 (dd, *J* = 9.8, 13.6 Hz, 1H), 3.71 (s, 3H), 2.95-2.79 (m, 1H), 2.52 (s, 3H), 1.14 (d, *J* = 6.8 Hz, 3H).

### Example 90: WX168, WX169

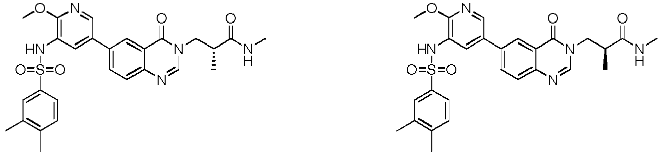

### Synthetic route:

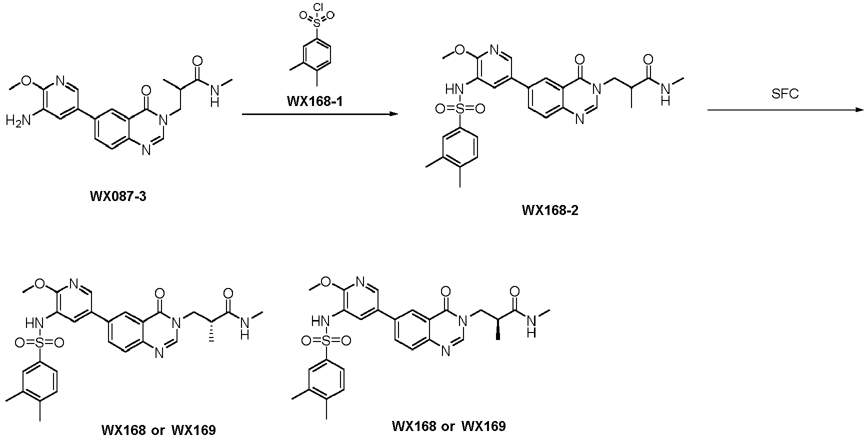

### Step 1: synthesis of compound WX168-2

**WX087-3** (0.15 g, 408.27 µmol) was dissolved in pyridine (3.0 mL), and **WX168-1** (108.63 mg, 530.75 µmol) was added dropwise at 25 °C. The reaction solution was stirred at 30 °C for 16 hours. After the reaction was completed, the reaction solution was rotary-evaporated, followed by addition of water (10.00 mL), and washed three times with dichloromethane (10.00 mL). The organic phases were combined, dried over anhydrous sodium sulfate, and rotary-evaporated to obtain target compound **WX168-2.** MS-ESI m/z: 536.1[M+H]⁺, 536.1[M+H+2]⁺.

### Step 2: synthesis of compound WX168, WX169

**WX168-2** was resolved and purified by SFC (chromatographic column: AD (250mm ^{∗} 30mm, 5µm); mobile phase: [0.1% NH₄HCO₃ ETOH]; B%: 55% -55%) to obtain target compounds **WX168** (Rt = 0.783min) and **WX169** (Rt = 1.910 min). **WX168**:¹H NMR (400MHz, Methanol-d4) δ: 8.26 (d, *J* = 2.0 Hz, 1H), 8.08 (s, 2H), 8.01-7.83 (m, 2H), 7.67 (d, *J* = 8.5 Hz, 1H), 7.51 (s, 1H), 7.46-7.37 (m, 1H), 7.16 (d, *J* = 8.0 Hz, 1H), 4.14 (dd, *J* = 4.9, 13.4 Hz, 1H), 3.93 (dd, *J* = 9.9, 13.4 Hz, 1H), 3.71 (s, 3H), 2.99-2.82 (m, 1H), 2.52 (s, 3H), 2.20 (s, 6H), 1.14 (d, *J* = 7.0 Hz, 3H). **WX169:¹H** NMR (400MHz, Methanol-d4) δδ: 8.24 (d, *J* = 2.0 Hz, 1H), 8.10-8.02 (m, 2H), 7.98-7.81 (m, 2H), 7.64 (d, *J=* 8.5 Hz, 1H), 7.51 (s, 1H), 7.43 (br d, *J=* 8.0 Hz, 1H), 7.16 (d, *J* = 8.0 Hz, 1H), 4.14 (dd, *J* = 4.9, 13.4 Hz, 1H), 3.92 (dd, *J* = 9.8, 13.3 Hz, 1H), 3.79-3.63 (m, 3H), 3.02-2.80 (m, 1H), 2.52 (s, 3H), 2.26-2.10 (m, 6H), 1.14 (d, *J=* 7.0 Hz, 3H).

### Example 91: WX170, WX171

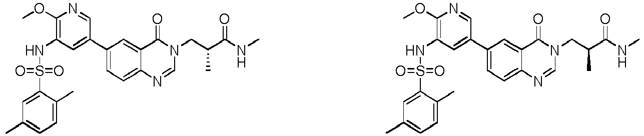

### Synthetic route:

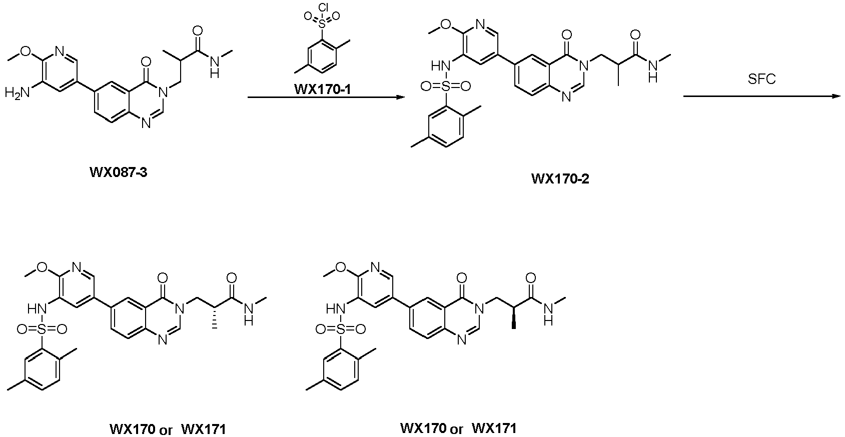

### Step 1: synthesis of compound WX170-2

**WX087-3** (0.15 g, 408.27 µmol) was dissolved in pyridine (3.0 mL), and **WX170-1** (125.34 mg, 612.40 µmol) was added dropwise at 25 °C. The reaction solution was stirred at 30 °C for 1 hour, and the reaction was completed. The reaction solution was rotary-evaporated, followed by addition of water (10 mL), and washed three times with dichloromethane (10 mL). The organic phases were combined, dried over anhydrous sodium sulfate, and rotary-evaporated to obtain the target compound **WX170-2.** MS-ESI m/z: 536.1[M+H]⁺,536.1[M+H+2]⁺.

### Step 2: synthesis of compound WX170, WX171

**WX170-2** was resolved and purified by SFC (chromatographic column: AD (250mm ^{∗} 30mm, 5µm); mobile phase: [0.1% NH₄HCO₃ ETOH]; B%: 55% -55%) to obtain target compounds **WX170** (Rt = 0.835min) and **WX171** (Rt = 1.735 min). **WX170:** ¹H NMR (400MHz, CDCl₃) δ : 8.22 (d, *J=* 1.3 Hz, 1H), 8.04 (s, 1H), 8.01 (d, *J* = 2.3 Hz, 1H), 7.82 (d, *J* = 2.3 Hz, 1H), 7.77 (s, 1H), 7.72-7.61 (m, 2H), 7.19-7.15 (m, 1H), 7.12-7.06 (m, 1H), 5.68 (br d, *J=* 4.8 Hz, 1H), 4.10 (dd, *J* = 5.0, 13.3 Hz, 1H), 3.97 (dd, *J=* 9.3, 13.3 Hz, 1H), 3.92-3.84 (m, 3H), 3.02-2.83 (m, 1H), 2.67 (d, *J=* 5.0 Hz, 3H), 2.58 (s, 3H), 2.28 (s, 3H), 1.21 (d, *J* = 6.8 Hz, 3H). **WX170:** ¹H NMR (400MHz, CDCl₃) δ : 8.21 (s, 1H), 8.13-7.95 (m, 2H), 7.88-7.74 (m, 2H), 7.74-7.53 (m, 2H), 7.38-7.24 (m, 1H), 7.20-7.15 (m, 1H), 7.13-7.02 (m, 1H), 5.72 (br s, 1H), 4.17-4.04 (m, 1H), 3.97 (dd, *J=* 9.4, 13.2 Hz, 1H), 3.90 (s, 3H), 2.94-2.79 (m, 1H), 2.66 (d, *J=* 4.8 Hz, 3H), 2.58 (s, 3H), 2.27 (s, 3H), 1.21 (d, *J=* 7.0 Hz, 3H).

### Example 92: WX172, WX173

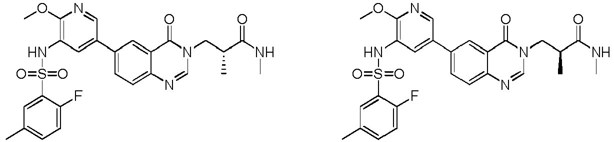

### Synthetic route:

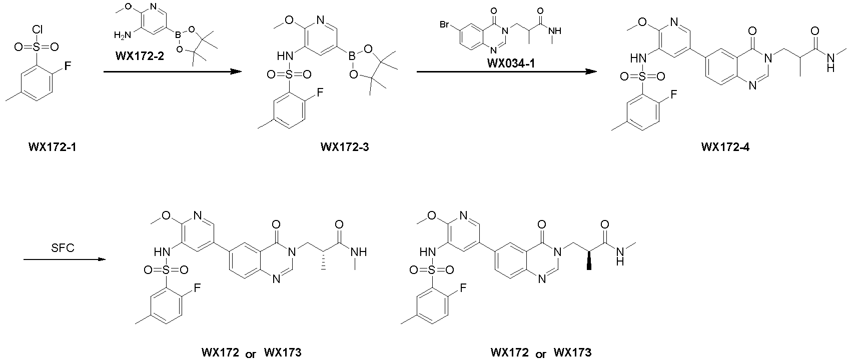

### Step 1: synthesis of compound WX172-3

**WX172-1** (2.50 g, 12.00 mmol), **WX172-2** (250 mg, 999.60 µmol) and the solvent pyridine (5 mL) were added into a pre-dried reaction flask and stirred continually at 25 °C for 12 hours. After the reaction was completed, water (5 mL) was added to the reaction solution, followed by extraction with dichloromethane (5 mL × 3). The organic phase was dried over anhydrous sodium sulfate, separated and purified by a preparative thin layer chromatographic plate (petroleum ether: ethyl acetate = 1: 1) to obtain target compound **WX172-3.**

### Step 2: synthesis of compound WX172-4

Raw materials **WX172-3** (160.00 mg, 493.56 µmol) and **WX034-1** (208.42 mg, 493.56 µmol), and the solvents 1,4-dioxane (3 mL) and water (0.5 mL) were added into a pre-dried reaction flask, followed by addition of potassium acetate (96.88 mg, 987.13 µmol). The mixture was replaced with nitrogen, followed by addition of 1,1-bis(diphenylphosphine) ferrocene palladium chloride (36.11 mg, 49.36 µmol). The mixture was then replaced with nitrogen, and further stirred at 80 °C for 12 hours. After the reaction was completed, water (5 mL) was added to the reaction solution, and extracted with dichloromethane (5 mL × 3). The obtained organic phase was dried over anhydrous sodium sulfate, separated and purified by preparative thin layer chromatography (dichloromethane: methanol = 15:1), and further separated by preparative HPLC to obtain target compound **WX172-4.**

### Step 3: synthesis of compound WX172, WX173

**WX172-4** was resolved by SFC (chromatographic column: AS (250mm ^{∗} 30mm, 5µm); mobile phase: [MeOH]; B%: 45% -45%, 10min) to obtain a pair of enantiomers **WX172** (with a retention time of 2.586 min) and **WX173** (with a retention time of 2.684 min). **WX172:** ¹H NMR (400MHz, METHANOL-d4) δ = 8.33 (d, *J* = 2.1 Hz, 1H), 8.28 (s, 1H), 8.20 (d, *J* = 2.3 Hz, 1H), 8.01 (dt, *J* = 2.3, 4.2 Hz, 2H), 7.75 (d, *J* = 8.4 Hz, 1H), 7.65 (dd, *J* = 2.0, 6.7 Hz, 1H), 7.46-7.40 (m, 1H), 7.16 (dd, *J* = 8.6, 10.1 Hz, 1H), 4.24 (dd, *J* = 4.9, 13.4 Hz, 1H), 4.04 (dd, *J* = 9.8, 13.4 Hz, 1H), 3.84 (s, 3H), 3.06-2.95 (m, 1H), 2.62 (s, 3H), 2.33 (s, 2H), 2.36-2.29 (m, 1H), 2.36-2.29 (m, 1H), 1.24 (d, *J* = 7.0 Hz, 3H). **WX173:** ¹H NMR (400MHz, CHLOROFORM-d) δ = 8.34 (d, *J* = 2.0 Hz, 1H), 8.15-8.09 (m, 2H), 8.02 (d, *J* = 1.8 Hz, 1H), 7.87-7.82 (m, 1H), 7.79-7.75 (m, 1H), 7.71-7.66 (m, 1H), 7.34 (br s, 1H), 7.07 (t, *J* = 9.2 Hz, 1H), 5.65 (br s, 1H), 4.22-4.14 (m, 1H), 4.05 (dd, *J* = 9.5, 13.2 Hz, 1H), 3.96 (s, 3H), 3.01-2.91 (m, 1H), 2.74 (d, *J* = 4.6 Hz, 3H), 2.35 (s, 3H), 1.29 (d, *J* = 7.1 Hz, 3H).

### Example 93: WX174, WX175

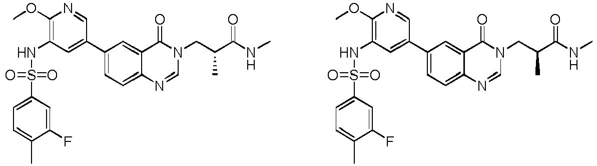

### Synthetic route:

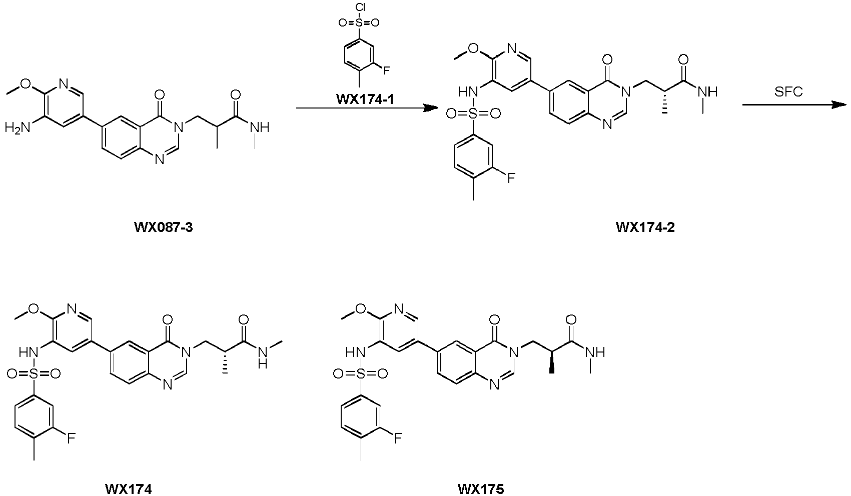

### Step 1: synthesis of compound WX174-2

Raw materials **WX087-3** (200 mg, 544.36 µmol) and **WX174-1** (170.36 mg, 816.55 µmol) and the solvent pyridine (4 mL) were added into a pre-dried reaction flask, and stirred at 25 °C for 12 hours. After reaction was completed, water (5 mL) was added to the reaction solution, followed by extraction with dichloromethane (5 mL × 3). The obtained organic phase was dried over anhydrous sodium sulfate, purified and separated by preparative HPLC to obtain target compound **WX174-2.**

### Step 2: synthesis of compound WX174, WX175

**WX174-2** was resolved and purified by SFC (chromatographic column: AS (250mm ^{∗} 30mm, 5µm); mobile phase: [MeOH]; B%: 45% -45%, 6.5min) to obtain a pair of enantiomers **WX174** (retention time is 2.704min) and **WX175** (retention time is 2.714min). **WX174:** ¹H NMR (400MHz, METHANOL-d4) δ = 8.39 (d, *J* = 1.8 Hz, 1H), 8.25-8.23 (m, 1H), 8.19 (s, 1H), 8.08 (d, *J* = 2.3 Hz, 1H), 8.04 (dd, *J* = 2.2, 8.6 Hz, 1H), 7.78 (d, *J* = 8.6 Hz, 1H), 7.50 (d, *J* = 8.3 Hz, 2H), 7.41-7.36 (m, 1H), 4.24 (dd, *J* = 4.9, 13.4 Hz, 1H), 4.09-3.97 (m, 1H), 3.80 (s, 3H), 3.06-2.93 (m, 1H), 2.65-2.60 (m, 3H), 2.31 (d, *J* = 1.8 Hz, 3H), 2.28-2.26 (m, 1H), 1.24 (d, *J* = 7.0 Hz, 3H). **WX175:** ¹H NMR (400MHz, METHANOL-d4) δ = 8.37 (d, *J* = 2.1 Hz, 1H), 8.23 (d, *J* = 2.3 Hz, 1H), 8.19 (s, 1H), 8.07 (d, *J* = 2.3 Hz, 1H), 8.03 (dd, *J* = 2.2, 8.6 Hz, 1H), 7.76 (d, *J* = 8.4 Hz, 1H), 7.49 (d, *J* = 8.3 Hz, 2H), 7.41-7.36 (m, 1H), 4.24 (dd, *J* = 5.0, 13.4 Hz, 1H), 4.03 (dd, *J* = 9.9, 13.4 Hz, 1H), 3.79 (s, 3H), 3.06-2.95 (m, 1H), 2.65-2.59 (m, 3H), 2.30 (d, *J* = 1.8 Hz, 3H), 1.24 (d, *J* = 7.0 Hz, 3H).

### Example 94: WX176, WX177

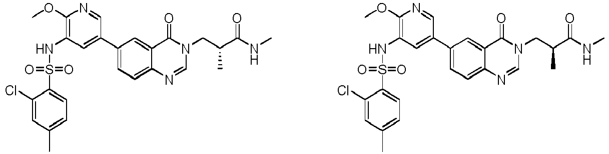

### Synthetic route:

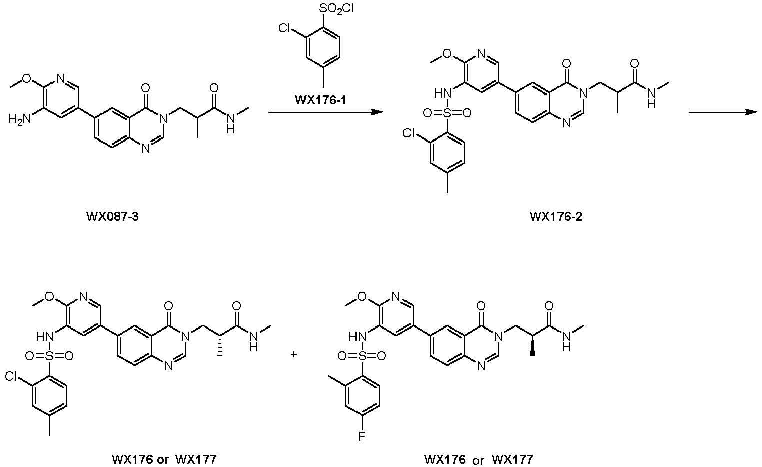

### Step 1: synthesis of compound WX176-2

Raw material **WX087-3** (200 mg, 544.36 µmol) was added into a pre-dried reaction flask, and then pyridine (5 mL) was added for dissolution, followed by addition of **WX176-1** (183.80 mg, 816.55 µmol). The reaction solution was stirred at 20 °C for 12 hours. After the reaction was completed, 10 mL of water and 10 mL of ethyl acetate were added to the reaction system for dilution. The organic phase was collected after liquid separation, and the aqueous phase was extracted with ethyl acetate (5 mL × 3). The organic phases were combined, washed with saturated brine (20 mL), dried over anhydrous sodium sulfate, concentrated under reduced pressure, and separated by preparative HPLC (method: Column: Nano-micro Kromasil C18 100 ^{∗} 30mm 5µm; mobile phase: [water (0.1% TFA) -ACN]; B%: 33%-53%, 10min) to obtain target compound **WX176-2.**

### Step 2: synthesis of compound WX176 and WX177

**WX176-2** was resolved and purified by SFC (resolution method: chromatography column: AS (250mm ^{∗} 30mm, 5µm); mobile phase: [MeOH]; B%: 45% -45%, 6.5min) to obtain a pair of enantiomers **WX176** (Rt = 3.140 min) and **WX177** (Rt = 3.480 min). **WX176:** ¹H NMR (400MHz, METHANOL-d4) δ = 8.31 (d, *J* = 2.2 Hz, 1H), 8.21 (s, 1H), 8.19 (d, *J=* 2.3 Hz, 1H), 8.03-7.91 (m, 3H), 7.77 (d, *J=* 8.4 Hz, 1H), 7.45 (s, 1H), 7.30 (d, *J=* 8.3 Hz, 1H), 4.26 (dd, *J=* 4.9, 13.4 Hz, 1H), 4.05 (dd, *J=* 9.9, 13.4 Hz, 1H), 3.90 (s, 3H), 3.08-2.93 (m, 1H), 2.64 (s, 3H), 2.39 (s, 3H), 1.26 (d, *J* = 7.0 Hz, 3H). **WX177:** ¹H NMR (400MHz, METHANOL-d4) δ = 8.28 (d, *J* = 2.1 Hz, 1H), 8.20 (s, 1H), 8.16 (d, *J* = 2.2 Hz, 1H), 7.98-7.91 (m, 3H), 7.74 (d, *J* = 8.6 Hz, 1H), 7.44 (d, *J=* 0.7 Hz, 1H), 7.29 (dd, *J=* 0.8, 8.1 Hz, 1H), 4.25 (dd, *J* = 4.9, 13.4 Hz, 1H), 4.04 (dd, *J=* 9.8, 13.4 Hz, 1H), 3.90 (s, 3H), 3.08-2.94 (m, 1H), 2.64 (s, 3H), 2.38 (s, 3H), 1.26 (d, *J* = 7.0 Hz, 3H).

### Example 95: WX178, WX179

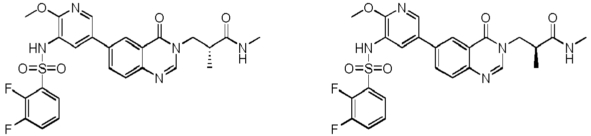

### Synthetic route:

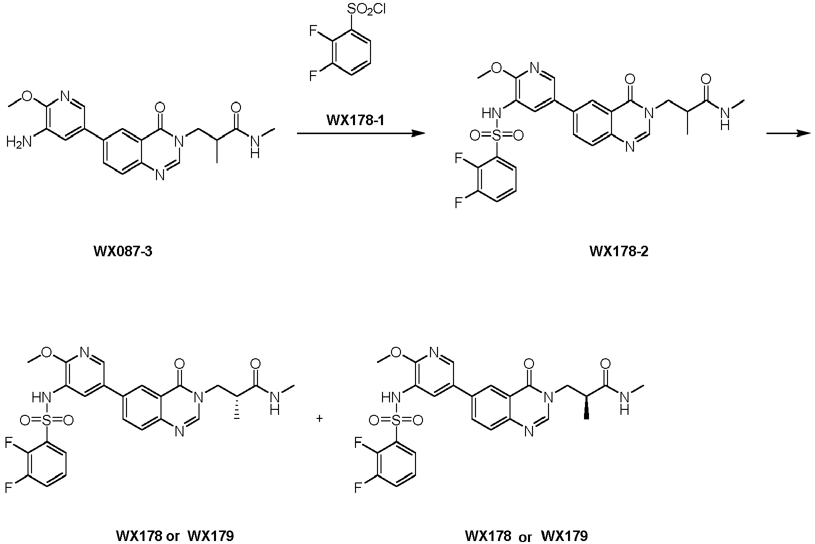

### Step 1: synthesis of compound WX178-2

Raw material **WX087-3** (150 mg, 408.27 µmol) was added into a pre-dried reaction flask, and then pyridine (3 mL) was added for dissolution, followed by addition of **WX178-1** (130.20 mg, 612.41 µmol). The reaction solution was stirred at 20 °C for 12 hours. After the reaction was completed, 10 mL of water and 10 mL of ethyl acetate were added to the reaction system for dilution. The organic phase was collected after liquid separation, and the aqueous phase was extracted with ethyl acetate (5 mL × 3). The organic phases were combined, washed with saturated brine (20 mL), dried over anhydrous sodium sulfate, concentrated under reduced pressure, and separated by preparative HPLC (method: column: Nano-micro Kromasil C18 100 ^{∗} 30mm 5µm; mobile phase: [water (0.1% TFA) -ACN]; B%: 30%-50%, 10min) to obtain target compound **WX178-2.**

### Step 2: synthesis of compound WX178 and WX179

**WX178-2** was resolved and purified by SFC (purification method: chromatography column: AS (250mm ^{∗} 30mm, 5µm); mobile phase: [MeOH]; B%: 45% -45%, 6.5min) to obtain a pair of enantiomers **WX178** (Rt = 2.864 min) and **WX179** (Rt = 3.136 min). **WX178:** ¹H NMR (400MHz, METHANOL-d4) δ = 8.38 (d, *J* = 2.1 Hz, 1H), 8.28 (d, *J=* 2.3 Hz, 1H), 8.20 (s, 1H), 8.09-8.00 (m, 2H), 7.78 (d, *J=* 8.4 Hz, 1H), 7.67-7.50 (m, 2H), 7.35-7.24 (m, 1H), 4.25 (dd, *J* = 4.9, 13.4 Hz, 1H), 4.05 (dd, *J=* 9.9, 13.3 Hz, 1H), 3.83 (s, 3H), 3.10-2.95 (m, 1H), 2.64 (s, 3H), 1.25 (d, *J* = 7.0 Hz, 3H). **WX179:** ¹H NMR (400MHz, METHANOL-d4) δ = 8.39 (d, *J* = 2.1 Hz, 1H), 8.29 (d, *J=* 2.3 Hz, 1H), 8.21 (s, 1H), 8.09-8.03 (m, 2H), 7.79 (d, *J=* 8.4 Hz, 1H), 7.68-7.49 (m, 2H), 7.30 (ddt, *J=* 1.6, 4.6, 8.2 Hz, 1H), 4.26 (dd, *J=* 4.9, 13.4 Hz, 1H), 4.05 (dd, *J* = 9.8, 13.4 Hz, 1H), 3.83 (s, 3H), 3.08-2.94 (m, 1H), 2.64 (s, 3H), 1.25 (d, *J=* 7.0 Hz, 3H).

### Example 96: WX180, WX181

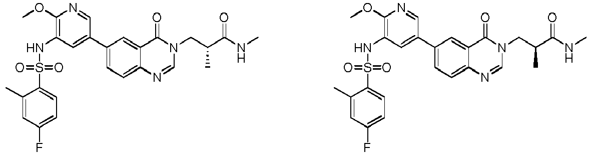

### Synthetic route:

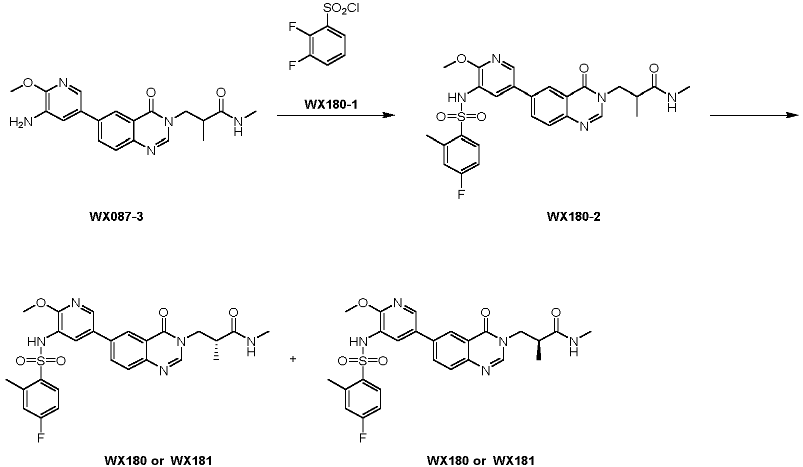

### Step 1: synthesis of compound WX180-2

**WX087-3** (200 mg, 544.36 µmol) was added in to a pre-dried reaction flask, and then pyridine (5 mL) was added for dissolution, followed by addition of **WX180-1** (170.36 mg, 816.55 µmol, 119.13 µL). The reaction solution was stirred at 20 °C for 12 hours. After the reaction was completed, 10 mL of water and 10 mL of ethyl acetate were added to the reaction system for dilution. The organic phase was collected after liquid separation, and the aqueous phase was extracted with ethyl acetate (5 mL × 3). The organic phases were combined, washed with saturated brine (20 mL), dried over anhydrous sodium sulfate, concentrated under reduced pressure, and separated by preparative HPLC (method: column: Nano-micro Kromasil C18 100 ^{∗} 30mm 5µm; mobile phase: [water (0.1% TFA) -ACN]; B%: 31% -51%, 10min) to obtain target compound **WX180-2.**

### Step 2: synthesis of compound WX180 and WX181

**WX180-2** was resolved and purified by SFC (purification method: chromatography column: AS (250mm ^{∗} 30mm, 5µm); mobile phase: [MeOH]; B%: 45% -45%, 6.5min) to obtain a pair of enantiomers **WX180** (Rt = 2.958 min) and **WX181** (Rt = 3.261 min). **WX180:** ¹H NMR (400MHz, METHANOL-d4) δ = 8.32 (d, *J* = 2.1 Hz, 1H), 8.23-8.15 (m, 2H), 8.04-7.98 (m, 2H), 7.95 (dd, *J* = 5.7, 8.8 Hz, 1H), 7.76 (d, *J* = 8.4 Hz, 1H), 7.16 (dd, *J* = 2.4, 9.5 Hz, 1H), 7.06 (dt, *J* = 2.6, 8.4 Hz, 1H), 4.26 (dd, *J* = 5.0, 13.4 Hz, 1H), 4.05 (dd, *J* = 9.8, 13.4 Hz, 1H), 3.87 (s, 3H), 3.08-2.96 (m, 1H), 2.73 (s, 3H), 2.64 (s, 3H), 1.26 (d, *J* = 7.0 Hz, 3H). **WX181:** ¹H NMR (400MHz, METHANOL-d4) δ = 8.32 (d, *J=* 2.1 Hz, 1H), 8.22-8.17 (m, 2H), 8.04-7.98 (m, 2H), 7.95 (dd, *J* = 5.7, 8.8 Hz, 1H), 7.76 (d, *J* = 8.6 Hz, 1H), 7.16 (dd, *J* = 2.4, 9.5 Hz, 1H), 7.06 (dt, *J* = 2.6, 8.4 Hz, 1H), 4.26 (dd, *J* = 4.9, 13.4 Hz, 1H), 4.05 (dd, *J* = 9.8, 13.4 Hz, 1H), 3.87 (s, 3H), 3.07-2.95 (m, 1H), 2.73 (s, 3H), 2.64 (s, 3H), 1.26 (d, *J=* 7.0 Hz, 3H).

### Example 97: WX182, WX183

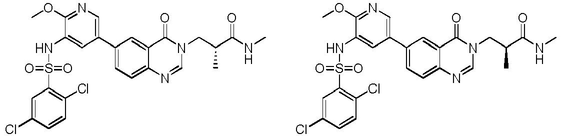

### Synthetic route:

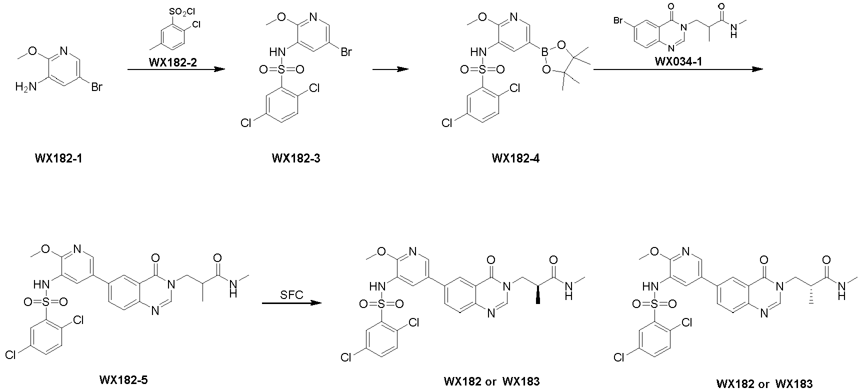

### Step 1: synthesis of compound WX182-3

Compound **WX182-1** (1 g, 4.93 mmol) and pyridine (6 mL) were sequentially added into a pre-dried reaction flask, followed by addition of compound **WX182-2** (1.21 g, 4.93 mmol, 659.20 µL). The mixture was replaced with nitrogen and the reaction was completed after stirring at 25 °C for 5 hours. The reaction solution was evaporated under reduced pressure to remove the solvent, and separated by flash column chromatography (petroleum ether: ethyl acetate = 10:1 to 5:1) to obtain target compound **WX182-3.** ¹H NMR (400MHz, CHLOROFORM-*d*) δ = 7.94 (d, *J* = 2.3 Hz, 1H), 7.90 (d, *J* = 2.1 Hz, 1H), 7.68 (ddd, *J* = 2.3, 7.1, 9.3 Hz, 1H), 7.63-7.57 (m, 1H), 7.33-7.27 (m, 1H), 6.90 (s, 1H), 3.93-3.79 (m, 3H).

### Step 2: synthesis of compound WX182-4

Compound **WX182-3** (0.15 g, 364.00 µmol), bis(pinacolato)diboron (101.68 mg, 400.40 µmol), potassium acetate (71.45 mg, 728.00 µmol) and 1,4-dioxane (3 mL) were added into a pre-dried reaction flask. The mixture was replaced with nitrogen, and 1,1-bis(diphenylphosphine) ferrocene palladium chloride (26.63 mg, 36.40 µmol) was added thereto finally. The mixture was replaced with nitrogen, heated to 110 °C and stirred for 3 hours to complete the reaction. The reaction solution was cooled down, evaporated under reduced pressure to remove the solvent to obtain target compound **WX182-4,** which was directly used in the next step.

### Step 3: synthesis of compound WX182-5

Compound **WX182-4** (0.2 g, 435.59 µmol), compound **WX034-1** (0.13 g, 401.02 µmol), 1,4-dioxane (2 mL), water (0.2 mL) and potassium acetate (118.07 mg, 1.20 mmol) were sequentially added into a pre-dried reaction flask. The mixture was then replaced with nitrogen, and 1,1-bis(diphenylphosphine) ferrocene palladium chloride (29.34 mg, 40.10 µmol) was added thereto finally. The mixture was replaced with nitrogen again, heated to 110 °C and stirred for 3 hours to complete the reaction. The reaction solution was cooled down, evaporated under reduced pressure to remove the solvent, and purified by preparative TLC (dichloromethane: methanol = 10: 1) and preparative HPLC (chromatographic column: water column Xbridge Prep OBD C18 150 ^{∗} 30mm 10µm; mobile phase: [water (0.04% NH₄HCO₃) -ACN]; B%: 5% -35%, 10min) to obtain target compound **WX182-5.** ¹H NMR (400MHz, METHANOL-*d₄*) δ = 8.40 (d, *J=* 2.2 Hz, 1H), 8.27 (s, 1H), 8.19 (s, 1H), 8.07-8.00 (m, 3H), 7.79 (d, *J* = 8.3 Hz, 1H), 7.61-7.57 (m, 2H), 4.29-4.23 (m, 1H), 4.03 (dd, *J=* 10.1, 13.2 Hz, 1H), 3.85 (s, 3H), 3.01 (s, 1H), 2.62 (s, 3H), 1.24 (d, *J* = 7.0 Hz, 3H).

### Step 4: synthesis of compound WX182, WX183

Compound **WX182-5** (0.07 g, 121.43 µmol) was resolved by SFC (separation conditions: chromatography column: AS (250mm ^{∗} 30mm, 5µm); mobile phase: [MEOH]; B%: 45% -45%, 8.5min) to obtain a pair of enantiomers **WX182** (Rt = 3.151min) and **WX183** (Rt = 3.458min). **WX182:** ¹H NMR (400MHz, METHANOL-*d₄*) δ = 8.39 (d, *J* = 1.8 Hz, 1H), 8.28 (d, *J* = 2.2 Hz, 1H), 8.19 (s, 1H), 8.05-7.98 (m, 3H), 7.77 (d, *J=* 8.8 Hz, 1H), 7.61-7.58 (m, 2H), 4.24 (dd, *J=* 4.8, 13.2 Hz, 1H), 4.03 (dd, *J=* 9.9, 13.4 Hz, 1H), 3.84 (s, 3H), 3.04-2.94 (m, 1H), 2.64-2.60 (m, 3H), 1.23 (d, *J* = 7.0 Hz, 3H). **WX183:** ¹H NMR (400MHz, METHANOL-*d₄*) δ = 8.38 (d, *J=* 2.2 Hz, 1H), 8.25 (d, *J* = 2.2 Hz, 1H), 8.18 (s, 1H), 8.04-7.98 (m, 3H), 7.77 (d, *J* = 8.3 Hz, 1H), 7.62-7.55 (m, 2H), 4.24 (dd, *J* = 5.0, 13.4 Hz, 1H), 4.02 (dd, *J* = 9.9, 13.4 Hz, 1H), 3.84 (s, 3H), 3.04-2.96 (m, 1H), 2.62 (s, 3H), 1.23 (d, *J* = 7.0 Hz, 3H).

### Example 98: WX184, WX185

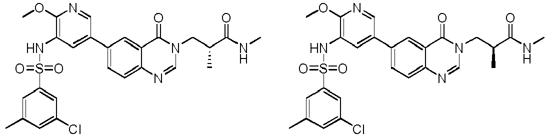

### Synthetic route:

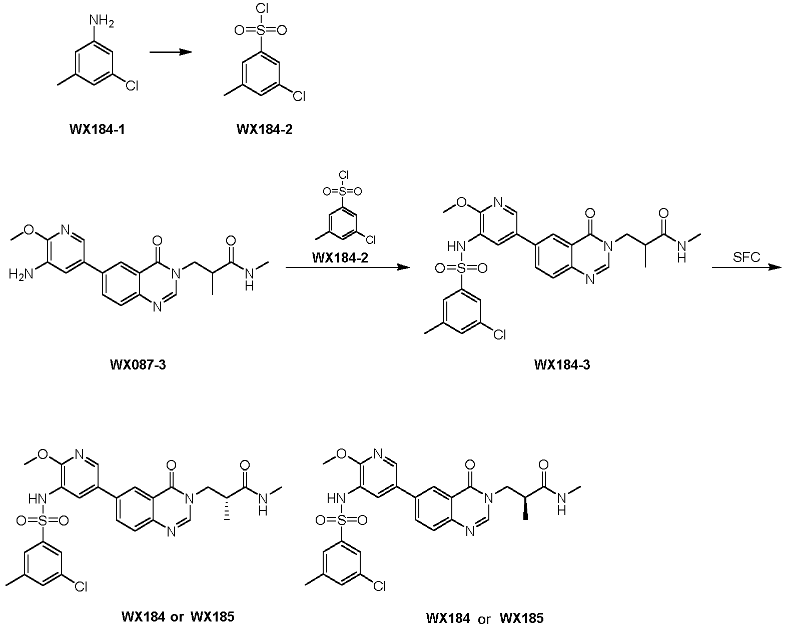

### Step 1: synthesis of compound WX184-2

A solution of sodium nitrite (3.65 g, 52.97 mmol) in water (10 mL) was slowly dropwise added into a three-necked flask (250 mL) containing compound **WX184-1** (5 g, 35.31 mmol) and hydrochloric acid (50 mL) at 0-5 °C with stirring. The mixture was replaced with nitrogen and further stirred for 1 hour. A solution of copper chloride (1.42 g, 10.59 mmol) in water (10 mL) and sulfur dioxide (5 M, 30 mL) (a solution of sulfur dioxide in acetic acid at 15 psi and 20 °C , 5M) were added. The mixture was replaced with nitrogen and further stirred for 2 hours to complete the reaction. The reaction solution was added to ice water (200 mL), stirred for 0.5 hours, filtered to obtain a filter cake, and the filter cake was rotary-evaporated under reduced pressure to obtain the target compound **WX184-2,** which was directly used in the next step. ¹H NMR (400MHz, CHLOROFORM-d) δ = 7.84 (s, 1H), 7.75 (s, 1H), 7.54 (s, 1H), 2.49 (s, 3H), 1.56 (s, 1H).

### Step 2: synthesis of compound WX184-3

Compound **WX087-3** (0.15 g, 408.27 µmol) and pyridine (6 mL) were sequentially added into a pre-dried reaction flask, and compound **WX184-2** (183.80 mg, 816.55 µmol) was added thereto finally. The mixture was replaced with nitrogen and the reaction was completed after stirring at 50 °C for 5 hours. The reaction solution was cooled down, evaporated under reduced pressure to remove the solvent, and purified by preparative TLC (dichloromethane: methanol = 10:1) to obtain compound **WX184-3.** ¹H NMR (400MHz, METHANOL-*d₄*) δ = 8.40 (d, *J* = 1.8 Hz, 1H), 8.26 (d, *J* = 2.2 Hz, 1H), 8.18 (s, 1H), 8.08-8.02 (m, 2H), 7.77 (d, *J* = 8.8 Hz, 1H), 7.61 (s, 1H), 7.53 (s, 1H), 7.45 (s, 1H), 4.24 (dd, *J* = 5.0, 13.4 Hz, 1H), 4.02 (dd, *J* = 10.1, 13.2 Hz, 1H), 3.79 (s, 3H), 2.99 (ddd, *J* = 4.8, 7.0, 9.6 Hz, 1H), 2.64-2.58 (m, 3H), 2.36 (s, 3H), 1.23 (d, *J* = 7.0 Hz, 3H).

### Step 3: synthesis of compound WX184, WX185

Compound **WX184-3** (0.13 g, 233.80 µmol) was resolved by SFC, (conditions: chromatographic column: AS (250mm ^{∗} 30mm, 5µm); mobile phase: [MeOH]; B%: 45% -45%, 8min) to obtain a pair of enantiomers **WX184** (RT = 2.70min) and compound **WX185** (RT = 2.97min). **WX184:** ¹H NMR (400 MHz, METHANOL-*d₄*) δ = 8.32 (d, *J* = 2.2 Hz, 1H), 8.21-8.16 (m, 2H), 8.02-7.98 (m, 2H), 7.76-7.67 (m, 2H), 7.16-7.07 (m, 2H), 4.24 (dd, *J* = 4.8, 13.6 Hz, 1H), 4.02 (dd, *J* = 9.9, 13.4 Hz, 1H), 3.84 (s, 3H), 3.06-2.96 (m, 1H), 2.64-2.60 (m, 3H), 2.39 (s, 3H), 1.24 (d, *J* = 7.0 Hz, 3H). **WX185:** ¹H NMR (400MHz, METHANOL-*d₄*) δ = 8.41 (d, *J* = 2.2 Hz, 1H), 8.28 (d, *J* = 2.2 Hz, 1H), 8.19 (s, 1H), 8.05 (d, *J* = 2.2 Hz, 1H), 8.07 (s, 1H), 7.79 (d, *J* = 8.8 Hz, 1H), 7.62 (s, 1H), 7.54 (s, 1H), 7.46 (s, 1H), 4.58 (s, 1H), 4.26 (d, *J* = 4.8 Hz, 1H), 4.23 (d, *J* = 4.8 Hz, 1H), 4.03 (d, *J* = 3.5 Hz, 1H), 4.00 (s, 1H), 4.06 (s, 1H), 3.80 (s, 3H), 2.99 (br s, 1H), 2.62 (s, 3H), 2.37 (s, 3H), 1.24 (d, *J=* 7.0 Hz, 3H).

### Example 99: WX186, WX187

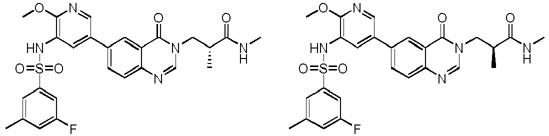

### Synthetic route:

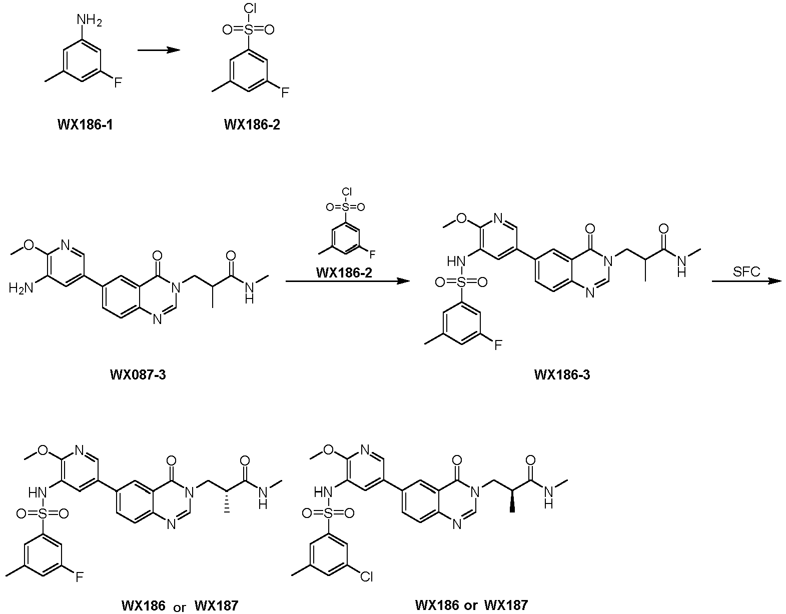

### Step 1: synthesis of compound WX186-2

A solution of sodium nitrite (4.14 g, 59.93 mmol) in water (10 mL) was slowly dropwise added into a three-necked flask (250 mL) containing compound WX186-1 (5 g, 39.95 mmol) and hydrochloric acid (50 mL) at 0-5 °C with stirring. A solution of copper chloride (1.61 g, 11.99 mmol) in water (10 mL) and sulfur dioxide (5 M, 30 mL) (a solution of sulfur dioxide in acetic acid at 15 psi and 20 °C, 5M) were added. The mixture was replaced with nitrogen and further stirred for 2 hours. After the reaction was completed, the reaction solution was added to ice water (200 mL), stirred for 0.5 hours, filtered to obtain a filter cake, and the filter cake was rotary-evaporated under reduced pressure to obtain target compound **WX186-2,** which was directly used in the next step. ¹H NMR (400MHz, CHLOROFORM-d) δ = 7.67 (s, 1H), 7.59-7.53 (m, 1H), 7.31-7.24 (m, 1H), 2.51 (s, 3H).

### Step 2: synthesis of compound WX186-3

Compound **WX087-3** (0.15 g, 408.27 µmol) and pyridine (6 mL) were sequentially added into a pre-dried reaction flask, and finally compound **WX186-2** (170.36 mg, 816.55 µmol) was added thereto finally. The mixture was replaced with nitrogen and the reaction was completed after stirring at 50 °C for 5 hours. After the reaction solution was cooled, evaporated under reduced pressure to remove the solvent, and purified by preparative TLC (dichloromethane: methanol = 10:1) to obtain compound **WX186-3.** ¹H NMR (400MHz, METHANOL-*d₄*) δ = 8.30 (d, *J* = 2.2 Hz, 1H), 8.19-8.14 (m, 2H), 8.00-7.91 (m, 3H), 7.75 (d, *J* = 8.8 Hz, 1H), 7.55 (d, *J* = 7.9 Hz, 1H), 7.39-7.32 (m, 1H), 4.23 (dd, *J* = 4.8, 13.6 Hz, 1H), 4.02 (dd, *J* = 9.9, 13.4 Hz, 1H), 3.87 (s, 3H), 3.00 (br d, *J=* 11.8 Hz, 1H), 2.61 (s, 3H), 2.45 (s, 3H), 1.23 (d, *J* = 7.0 Hz, 3H).

### Step 3: synthesis of compound WX186, WX187

Compound **WX186-3** (0.13 g, 240.93 µmol) was resolved by SFC (condition: chromatographic column: AS (250mm ^{∗} 30mm, 5µm); mobile phase: [MeOH]; B%: 45% -45%, 6.5min) to obtain a pair of enantiomers **WX186** (Rt = 2.55 min) and compound **WX187** (Rt = 3.00 min). **WX186:** ¹H NMR (400MHz, METHANOL-*d₄*) δ = 8.40 (d, *J* = 1.8 Hz, 1H), 8.25 (d, *J* = 2.2 Hz, 1H), 8.19 (s, 1H), 8.08-8.04 (m, 2H), 7.78 (d, *J* = 8.3 Hz, 1H), 7.46 (s, 1H), 7.35 (br d, *J* = 7.9 Hz, 1H), 7.18 (s, 1H), 4.27-4.22 (m, 1H), 4.03 (dd, *J=* 9.6, 13.2 Hz, 1H), 3.80 (s, 3H), 3.05-2.96 (m, 1H), 2.62 (s, 3H), 2.38 (s, 3H), 1.24 (d, *J* = 6.6 Hz, 3H). **WX187:** 1H NMR (400MHz, METHANOL-*d₄*) δ = 8.40 (d, *J=* 2.2 Hz, 1H), 8.25 (d, *J=* 2.2 Hz, 1H), 8.19 (s, 1H), 8.07 (s, 2H), 8.07-8.04 (m, 2H), 7.78 (d, *J=* 8.8 Hz, 1H), 7.46 (s, 1H), 7.35 (br d, *J* = 8.3 Hz, 1H), 7.19 (br d, *J* = 9.2 Hz, 1H), 4.24 (dd, *J* = 4.8, 13.2 Hz, 1H), 4.03 (dd, *J* = 10.1, 13.6 Hz, 1H), 3.81 (s, 3H), 3.01 (br dd, *J* = 7.2, 9.9 Hz, 1H), 2.62 (s, 3H), 2.38 (s, 3H), 1.24 (d, *J=* 7.0 Hz, 3H).

### Example 100: WX188, WX189

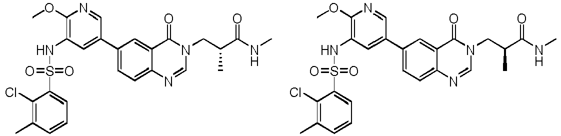

### Synthetic route:

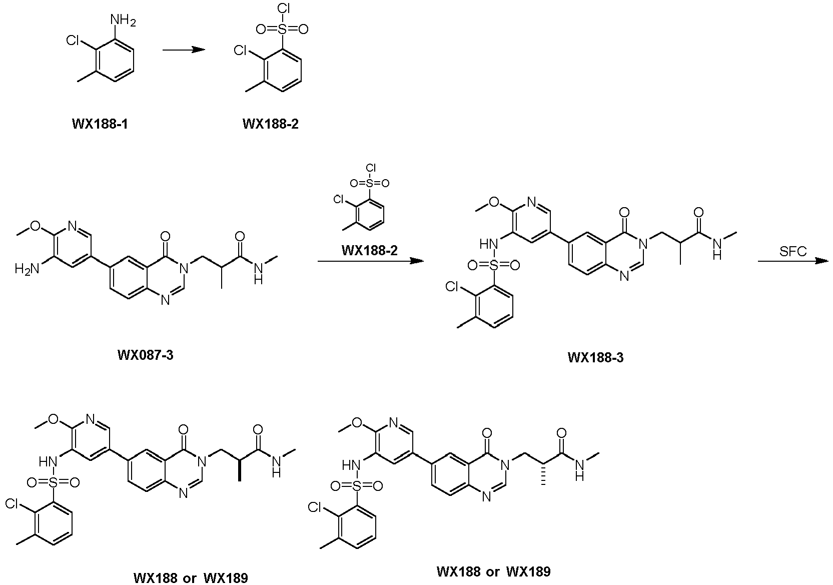

### Step 1: synthesis of compound WX188-2

A solution of sodium nitrite (1.46 g, 21.19 mmol) in water (4 mL) was slowly added dropwise into a three-necked flask (250 mL) containing compound **WX188-1** (2 g, 14.12 mmol) and hydrochloric acid (25 mL). A solution of copper chloride (569.71 mg, 4.24 mmol) in water (4 mL) and sulfur dioxide (5 M, 12 mL) (a solution of sulfur dioxide in acetic acid at 15 psi and 20 °C, 5M) were added. The mixture replaced with nitrogen and further stirred for 2 hours. After the reaction was completed, the reaction solution was added to ice water (200 mL), stirred for 0.5 hours, filtered to obtain a filter cake, and the filter cake was dried under reduced pressure to obtain the target compound **WX188-2,** which was directly used in the next step. ¹H NMR (400MHz, CHLOROFORM-*d*) δ = 8.06-8.00 (m, 1H), 7.62 (d, *J=* 7.0 Hz, 1H), 7.40 (t, *J=* 7.7 Hz, 1H), 2.53 (s, 3H).

### Step 2: synthesis of compound WX188-3

Compound **WX087-3** (0.15 g, 408.27 µmol) and pyridine (6 mL) were sequentially added into a pre-dried reaction flask, and compound **WX186-2** (183.80 mg, 816.55 µmol) was added thereto finally. The mixture was replaced with nitrogen, and stirred at 50 °C for 5 hours. After the reaction was completed, the reaction solution was cooled down and evaporated under reduced pressure to remove the solvent, and purified by preparative TLC (dichloromethane: methanol = 10: 1) to obtain target compound **WX188-3.** ¹H NMR (400MHz, METHANOL-*d₄*) δ = 8.30 (d, J = 2.2 Hz, 1H), 8.19-8.14 (m, 2H), 8.00-7.91 (m, 3H), 7.75 (d, *J* = 8.8 Hz, 1H), 7.55 (d, *J* = 7.9 Hz, 1H), 7.39-7.32 (m, 1H), 4.23 (dd, *J=* 4.8, 13.6 Hz, 1H), 4.28-4.18 (m, 1H), 4.02 (dd, *J* = 9.9, 13.4 Hz, 1H), 3.87 (s, 3H), 3.00 (br d, *J=* 11.8 Hz, 1H), 2.61 (s, 3H), 2.45 (s, 3H), 1.23 (d, *J* = 7.0 Hz, 3H).

### Step 3: synthesis of compound WX188, WX189

Compound **WX188-3** (0.13 g, 240.93 µmol) was resolved by SFC (chromatographic column: AS (250mm ^{∗} 30mm, 5µm); mobile phase: [MeOH]; B%: 45%-45%, 6.5min) to obtain a pair enantiomers **WX188** (Rt = 2.67min) and **WX189** (Rt = 3.01min). **WX188:** 1H NMR (400MHz, METHANOL-*d₄*) δ = 8.27 (d, *J* = 2.2 Hz, 1H), 8.17 (s, 1H), 8.15 (s, 1H), 7.97-7.93 (m, 3H), 7.73 (d, *J* = 8.8 Hz, 1H), 7.55 (d, *J* = 7.4 Hz, 1H), 7.36 (t, *J* = 7.6 Hz, 1H), 4.25 (d, *J* = 4.8 Hz, 1H), 4.22 (d, *J* = 4.8 Hz, 1H), 4.05 (s, 1H), 4.02 (d, *J=* 3.5 Hz, 1H), 4.00 (s, 1H), 3.87 (s, 3H), 3.00 (ddd, *J* = 4.8, 6.9, 9.8 Hz, 1H), 2.62 (s, 3H), 2.45 (s, 3H), 1.24 (d, *J* = 7.0 Hz, 3H). **WX189:** ¹H NMR (400MHz, METHANOL-*d₄*) δ = 8.30 (d, *J* = 2.2 Hz, 1H), 8.18-8.17 (m, 2H), 7.99-7.93 (m, 3H), 7.76 (s, 1H), 7.74 (s, 1H), 7.56 (d, *J* = 7.0 Hz, 1H), 7.36 (s, 1H), 7.38 (s, 1H), 7.34 (s, 1H), 4.58 (s, 1H), 4.26 (d, *J* = 4.8 Hz, 1H), 4.22 (d, *J* = 4.8 Hz, 1H), 4.03 (d, *J=* 3.5 Hz, 1H), 4.06 (s, 1H), 3.87 (s, 3H), 3.00 (dt, *J* = 2.2, 4.8 Hz, 1H), 2.62 (s, 3H), 2.45 (s, 3H), 1.24 (d, *J* = 7.0 Hz, 3H).

### Example 101: WX190, WX191

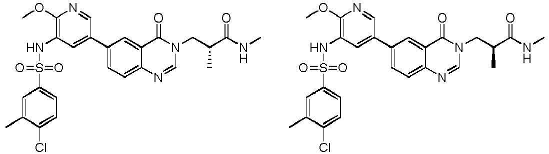

### Synthetic route:

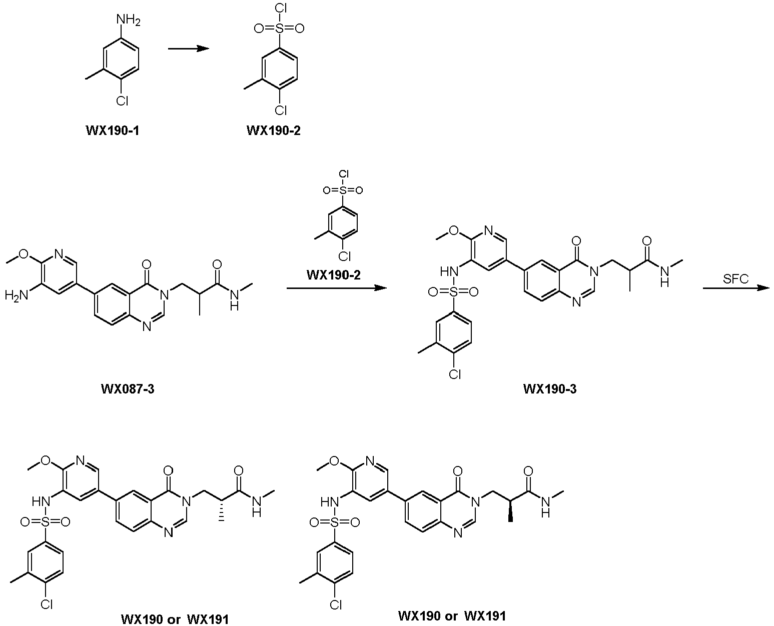

### Step 1: synthesis of compound WX190-2

A solution of sodium nitrite (3.65 g, 52.97 mmol) in water (10 mL) was slowly added dropwise into a three-necked flask (250 mL) containing compound **WX190-1** (5 g, 35.31 mmol) and hydrochloric acid (50 mL) at 0-5°C with stirring. A solution of copper chloride (1.42 g, 10.59 mmol) in water (10 mL) and sulfur dioxide (5 M, 30 mL) (a solution of sulfur dioxide in acetic acid at 15psi and 20 °C, 5M). The mixture was replaced with nitrogen and further stirred for 2 hours. After the reaction was completed, the reaction solution was added to ice water (200 mL), stirred for 0.5 hours, filtered to obtain a filter cake, and the filter cake was dried under reduced pressure to obtain target compound **WX190-2,** which was directly used in the next step. ¹H NMR (400MHz, CHLOROFORM-*d*) δ = 7.92 (d, *J* = 2.2 Hz, 1H), 7.82 (dd, *J=* 2.4, 8.6 Hz, 1H), 7.60 (d, *J=* 8.3 Hz, 1H), 2.51 (s, 3H).

### Step 2: synthesis of compound WX190-3

Compound **WX087-3** (0.15 g, 408.27 µmol) and pyridine (6 mL) were sequentially added into a pre-dried reaction flask, and compound **WX190-2** (183.80 mg, 816.55 µmol) was added thereto finally. The mixture was replaced with nitrogen, and stirred at 50 °C for 5 hours. After the reaction was completed, the reaction solution was cooled down and evaporated under reduced pressure to remove the solvent, and purified by preparative TLC (dichloromethane: methanol = 10:1) to obtain compound **WX190-3.** ¹H NMR (400MHz, METHANOL-*d₄*) δ = 8.40 (d, *J* = 2.2 Hz, 1H), 8.25 (d, *J=* 2.2 Hz, 1H), 8.19 (s, 1H), 8.10-8.04 (m, 2H), 7.81-7.73 (m, 2H), 7.63-7.58 (m, 1H), 7.63-7.58 (m, 1H), 7.53-7.48 (m, 1H), 4.25 (dd, *J=* 4.8, 13.6 Hz, 1H), 4.03 (dd, *J* = 9.9, 13.4 Hz, 1H), 3.79 (s, 3H), 3.00 (br dd, *J=* 9.9, 11.6 Hz, 1H), 2.62 (s, 3H), 2.40 (s, 3H), 1.24 (d, *J* = 7.0 Hz, 3H).

### Step 3: synthesis of compound WX190, WX191

Compound **WX190-3** (0.15 g, 269.77 µmol) was resolved by SFC (separation conditions: chromatography column: AS (250mm ^{∗} 30mm, 5µm); mobile phase: [MeOH]; B%: 45%-45%, 8min) to obtain a pair of enantiomers **WX190** (RT = 3.267min) and **WX191** (RT = 3.682min). **WX190:** ¹H NMR (400MHz, METHANOL-*d₄*) δ = 8.32 (d, *J* = 2.2 Hz, 1H), 8.18 (s, 1H), 8.17 (s, 1H), 8.04 (s, 1H), 7.98 (d, *J=* 8.6 Hz, 1H), 7.77-7.69 (m, 2H), 7.59 (dd, *J=* 2.0, 8.6 Hz, 1H), 7.48 (d, *J* = 8.3 Hz, 1H), 4.23 (dd, *J* = 4.8, 13.6 Hz, 1H), 4.02 (dd, *J* = 9.9, 13.4 Hz, 1H), 3.77 (s, 3H), 3.00 (ddd, *J=* 4.8, 7.0, 9.6 Hz, 1H), 2.62 (s, 3H), 2.38 (s, 3H), 1.24 (d, *J=* 7.0 Hz, 3H). **WX191:** 1H NMR (400MHz, METHANOL-*d₄*) δ = 8.39 (s, 1H), 8.24 (d, *J* = 2.2 Hz, 1H), 8.19 (s, 1H), 8.08-8.03 (m, 2H), 7.80-7.73 (m, 2H), 7.60 (dd, *J* = 2.0, 8.6 Hz, 1H), 7.50 (d, *J* = 8.8 Hz, 1H), 4.25 (dd, *J* = 4.8, 13.6 Hz, 1H), 4.03 (dd, *J* = 9.6, 13.2 Hz, 1H), 3.79 (s, 3H), 3.04-2.96 (m, 1H), 2.62 (s, 3H), 2.40 (s, 3H), 1.24 (d, *J* = 7.0 Hz, 3H).

### Example 102: WX192, WX193

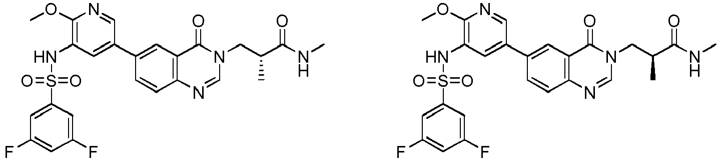

### Synthetic route:

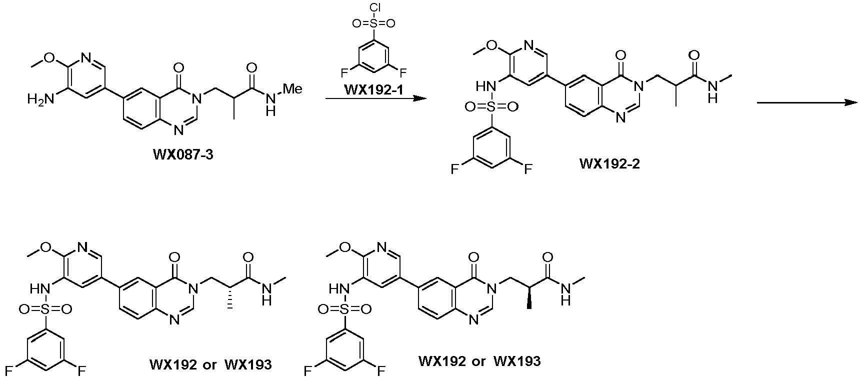

### Step 1: synthesis of compound WX192-2

**WX087-3** (150 mg, 269.58 µmol) and **WX192-1** (90 mg, 423.33 µmol) were dissolved in pyridine (2 mL). The reaction solution was stirred at 25 °C for 16 hours to complete the reaction, followed by addition of water (10 mL), and extracted with dichloromethane (5 mL) three times. The organic phase was washed with saturated brine (10 mL), dried over anhydrous sodium sulfate, filtered, and rotary-evaporated to dryness. The crude product was separated by preparative TLC (DCM: MeOH = 20: 1) to obtain target compound **WX192-2.**

### Step 2: synthesis of compound WX192 and WX193

**WX192-2** was resolved and purified by SFC (resolution method: chromatographic column: AD (250mm ^{∗} 30mm, 5µm); mobile phase: [0.1% NH₄HCO₃ ETOH]; B%: 55%-55%) to obtain target compounds **WX192** (Rt = 0.638 min) and **WX193** (Rt = 1.550 min). **WX192:** ¹H NMR (400 MHz, DMSO-*d₆*) δ ppm 1.14 (d, *J* = 7.03 Hz, 3 H) 2.53-2.55 (m, 3 H) 2.87-2.98 (m, 1 H) 3.77 (s, 3 H) 3.98-4.07 (m, 1 H) 4.10-4.19 (m, 1 H) 7.53 (br d, *J* = 4.52 Hz, 2 H) 7.65-7.76 (m, 1 H) 7.83 (d, *J* = 8.53 Hz, 1 H) 7.96 (br d, *J* = 4.52 Hz, 1 H) 8.00 (d, *J* = 2.01 Hz, 1 H) 8.16 (br d, *J* = 8.53 Hz, 1 H) 8.25 (s, 1 H) 8.34 (d, *J* = 1.51 Hz, 1 H) 8.47 (d, *J* = 2.01 Hz, 1 H). **WX193:** ¹H NMR (400 MHz, DMSO-*d₆*) δ ppm 1.14 (br d, *J* = 6.78 Hz, 3 H) 2.54 (br s, 3 H) 2.85-2.99 (m, 1 H) 3.76 (s, 3 H) 4.01-4.18 (m, 2 H) 7.52 (br d, *J* = 4.52 Hz, 2 H) 7.70 (br t, *J* = 9.03 Hz, 1 H) 7.82 (d, *J* = 8.53 Hz, 1 H) 7.95 (br d, *J* = 4.52 Hz, 1 H) 8.00 (d, *J* = 1.76 Hz, 1 H) 8.15 (dd, *J* = 8.53, 1.76 Hz, 1 H) 8.25 (s, 1 H) 8.33 (d, *J* = 1.51 Hz, 1 H) 8.46 (d, *J* = 1.76 Hz, 1H).

### Example 103: WX194, WX195

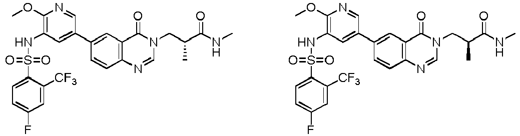

### Synthetic route:

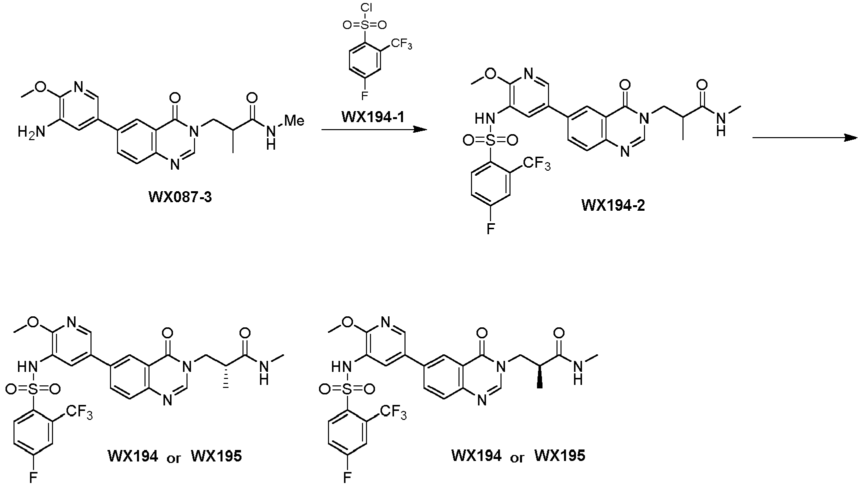

### Step 1: synthesis of compound WX194-2

**WX087-3** (150 mg, 269.58 µmol) and **WX194-1** (110 mg, 418.87 µmol) were dissolved in pyridine (2 mL). The reaction solution was stirred at 25 °C for 40 hours to complete the reaction, followed by addition of water (10 mL), and extracted with dichloromethane (5 mL) three times. The organic phase was washed with saturated brine (10 mL), dried over anhydrous sodium sulfate, filtered, and rotary-evaporated to dryness. The crude product was separated by preparative TLC plate (PE: EA = 0: 1) to obtain target compound **WX192-2.**

### Step 2: synthesis of compound WX194 and WX195

The enantiomer **WX194** (Rt = 0.450 min) and **WX195** (Rt = 0.947 min) were obtain by the resolution by SFC (resolution method: chromatographic column: AD (250mm ^{∗} 30mm, 5µm); mobile phase: [0.1% NH₄HCO₃ EtOH]; B%: 55% -55%). **WX194:** ¹H NMR (400 MHz, DMSO-*d₆*) δ ppm 1.08 (d, *J* = 7.03 Hz, 3 H) 2.48 (br s, 3 H) 2.81-2.92 (m, 1 H) 3.66 (s, 3 H) 3.92-4.12 (m, 2 H) 7.68-7.78 (m, 2 H) 7.85-7.95 (m, 3 H) 8.02-8.14 (m, 2 H) 8.18 (s, 1 H) 8.25 (d, *J* = 2.01 Hz, 1 H) 8.37 (s, 1 H). **WX195:** ¹H NMR (400 MHz, DMSO-*d₆*) δ ppm 1.15 (d, *J* = 7.03 Hz, 3 H) 2.55 (br s, 3 H) 2.86-2.99 (m, 1 H) 3.73 (s, 3 H) 3.98-4.07 (m, 1 H) 4.13 (td, *J* = 13.49, 6.15 Hz, 1 H) 7.76-7.86 (m, 2 H) 7.93-8.04 (m, 3 H) 8.12-8.20 (m, 2 H) 8.26 (s, 1 H) 8.33 (d, *J=* 2.01 Hz, 1 H) 8.47 (d, *J=* 2.01 Hz, 1 H).

### Example 104: WX196, WX197

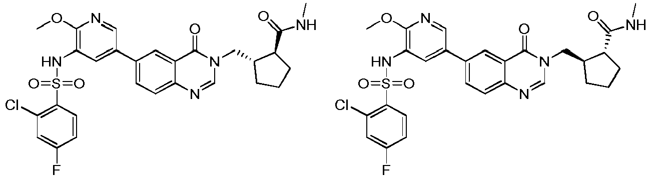

### Synthetic route:

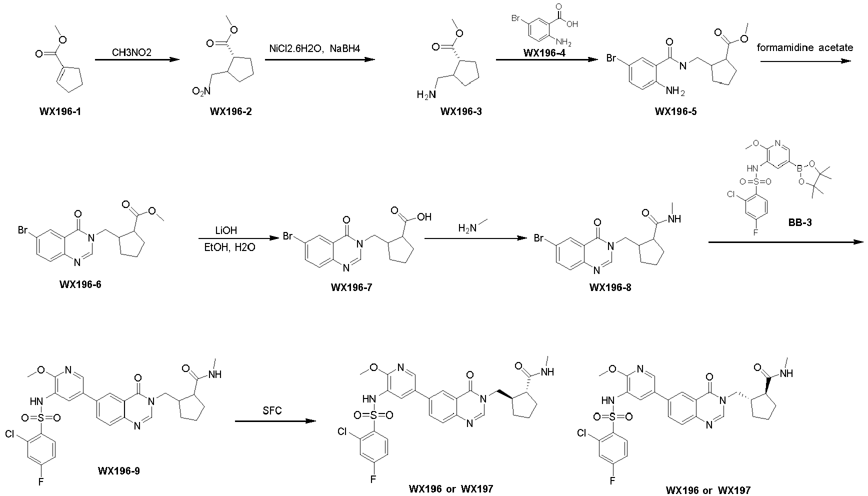

### Step 1: synthesis of compound WX196-2

Raw material **WX196-1** (3 g, 23.78 mmol) and the solvent tetrahydrofuran (100 mL) were added into a pre-dried single-necked flask, then nitromethane (4.35 g, 71.34 mmol, 3.85 mL) and tetrabutylammonium fluoride (12.69 g, 47.56 mmol, 98% purity) were added, and stirred at 25 °C for 12 hours. After the reaction was completed, water (50 mL) was added to the reaction solution, followed by extraction with dichloromethane (50 mL × 3). The resulting organic phase was dried over anhydrous sodium sulfate, rotary-evaporated under reduced pressure by a water pump, separated and purify by column chromatography (petroleum ether: ethyl acetate = 1:0 to 20:1) to obtain the target compound **WX196-2.** ¹H NMR (400MHz, CHLOROFORM-d) δ = 4.58-4.51 (m, 1H), 4.36 (dd, *J* = 7.9, 12.3 Hz, 1H), 3.72-3.67 (m, 3H), 2.97-2.84 (m, 1H), 2.55 (q, *J* = 8.6 Hz, 1H), 2.10-1.97 (m, 2H), 1.96-1.88 (m, 1H), 1.79-1.66 (m, 2H), 1.44-1.44 (m, 1H), 1.44 (qd, *J =* 8.3, 12.8 Hz, 1H).

### Step 2: synthesis of compound WX196-3

Raw material **WX196-2** (3.5 g, 18.70 mmol) and the solvent methanol (35 mL) were added into a pre-dried stock bottle, and then nickel chloride hexahydrate (6.67 g, 28.05 mmol) was added. The mixture was cooled to 0 °C, and sodium borohydride (2.12 g, 56.09 mmol) was slowly added thereto and further stirred at 0 °C for 2 hours. After the reaction was completed, the reaction solution was quenched with ammonium chloride, and rotary-evaporated under reduced pressure by a water pump, and adjusted to pH=3. A small amount of dichloromethane was added for extraction, allowed to stand for phase separation. The aqueous phase was adjusted to pH 10, and extract with dichloromethane: methanol = 10:1 (220mL × 2). The organic phase obtained was dried over anhydrous sodium sulfate, and rotary-evaporated under reduced pressure to obtain target compound **WX196-3,** which was directly put into the next reaction. ¹H NMR (400MHz, CHLOROFORM-d) δ = 3.66 (s, 6H), 2.75-2.63 (m, 5H), 2.44-2.37 (m, 2H), 2.22-2.13 (m, 2H), 1.93-1.78 (m, 6H), 1.80-1.78 (m, 1H), 1.72-1.55 (m, 5H), 1.39-1.26 (m, 1H), 1.29-1.20 (m, 6H).

### Step 3: synthesis of compound WX196-5

Raw materials **WX196-3** (2.9 g, 18.45 mmol) and **WX196-4** (3.99 g, 18.45 mmol) and the solvent dichloromethane (30 mL) were added into a pre-dried single-necked flask, and then 2,4,6-tripropyl-1,3,5,2,4,6-trioxytriphosphate-trioxide (14.09 g, 22.14 mmol, 13.16 mL, 50% purity) was added. The mixture was cooled to 0 °C, and *N*,*N-*diisopropylethylamine (7.15 g, 55.34 mmol, 9.64 mL) was slowly added thereto and further stirred at 25 °C for 10 hours. After the reaction was completed, water (20 mL) and dichloromethane (20 mL × 3) were added to the reaction solution for extraction. The resulting organic phase was dried over anhydrous sodium sulfate and rotary-evaporated under reduced pressure by a water pump, separated and purified by flash column chromatography (petroleum ether: ethyl acetate = 1:0 to 1:1) to obtain target compound **WX196-5.** ¹H NMR (400MHz, CHLOROFORM-d) δ = 7.45 (d, *J* = 2.4 Hz, 1H), 7.28 (d, *J* = 2.2 Hz, 1H), 6.57 (d, *J* = 8.6 Hz, 1H), 6.59-6.55 (m, 1H), 5.58 (br s, 2H), 3.74 (s, 3H), 3.57 (td, *J* = 5.1, 13.2 Hz, 1H), 3.31 (ddd, *J* = 5.2, 9.6, 13.4 Hz, 1H), 2.58-2.52 (m, 1H), 2.48-2.38 (m, 1H), 2.05-1.90 (m, 4H), 1.76-1.66 (m, 3H), 1.44-1.34 (m, 1H).

### Step 4: synthesis of compound WX196-6

Raw material **WX196-5** (956.3 mg, 2.69 mmol) and the solvent ethanol (10 mL) were added into a pre-dried stock bottle, then formamidine acetate (1.68 g, 16.15 mmol) was added, and further stirred at 80 °C for 12 hours. After the reaction was completed, water (5 mL) and dichloromethane (5 mL × 3) was added to the reaction solution to extract the organic phase. The resulting organic phase was dried over anhydrous sodium sulfate, filtered, rotary-evaporated under reduced pressure, and separated and purified by column chromatography (petroleum ether: ethyl acetate = 1:0 to 1:1) to obtain target compound **WX196-6.** ¹H NMR (400MHz, CHLOROFORM-d) δ = 8.42 (d, *J* = 2.4 Hz, 1H), 8.03 (s, 1H), 7.81 (dd, *J* = 2.3, 8.7 Hz, 1H), 7.57 (d, *J* = 8.8 Hz, 1H), 4.13-3.99 (m, 2H), 4.13-3.99 (m, 1H), 3.43 (s, 2H), 3.45-3.41 (m, 1H), 2.72-2.63 (m, 1H), 2.56 (q, *J* = 8.5 Hz, 1H), 2.04-1.95 (m, 2H), 1.92-1.82 (m, 3H), 1.75-1.66 (m, 2H), 1.46-1.37 (m, 1H), 1.24 (t, *J* = 7.2 Hz, 1H), 1.28-1.21 (m, 1H).

### Step 5: synthesis of compound WX196-7

Raw material **WX196-6** (336.5 mg, 921.36 µmol) and the solvent tetrahydrofuran (3 mL) and water (1.5 mL) were added into a pre-dried single-necked flask, then lithium hydroxide monohydrate (77.33 mg, 1.84 mmol) was added, and further stirred at 25 °C for 2 hours. After the reaction was completed, water (1 mL) and dichloromethane (2 mL) were added to the reaction solution for liquid separation. The aqueous phase was adjusted to pH=4 and dichloromethane (5 mL × 3) was added to the reaction solution for extraction. The organic phase obtained was dried over anhydrous sodium sulfate, filtered, and rotary-evaporated under reduced pressure to obtain target compound **WX196-7,** which was directly used in the next reaction. ¹H NMR (400MHz, CHLOROFORM-d) δ = 8.41 (d, *J* = 2.2 Hz, 1H), 8.09 (s, 1H), 7.67 (dd, *J* = 2.1, 8.7 Hz, 1H), 7.34 (d, *J* = 8.6 Hz, 1H), 4.19-4.02 (m, 3H), 2.75-2.63 (m, 1H), 2.60-2.52 (m, 1H), 2.08-1.87 (m, 5H), 1.79-1.68 (m, 3H), 1.47-1.40 (m, 1H).

### Step 6: synthesis of compound WX196-8

Raw materials **WX196-7** (304 mg, 865.62 µmol, 1 eq) and methylamine (2 M, 649.21 µL) and the solvent dichloromethane (3 mL) were added into a pre-dried single-necked flask, and then 6-tripropyl-1,3,5,2,4,6-trioxytriphosphate-trioxide (661.01 mg, 1.04 mmol, 617.77 µL, 50% purity) was added thereto. The mixture was cooled to 0 °C, followed by alow addition of *N,N*-diisopropylethylamine (335.62 mg, 2.60 mmol, 452.32 µL), and further stirred at 25 °C for 12 hours. After the reaction was completed, water (2 mL) and dichloromethane (5 mL × 3) were added to the reaction solution for extraction. The resulting organic phase was dried over anhydrous sodium sulfate, rotary-evaporated under reduced pressure, separated and purified by preparative thin-layer chromatographic plate (dichloromethane: methanol = 15: 1) to obtain target compound **WX196-8.** ¹H NMR (400MHz, CHLOROFORM-d) δ = 8.45 (d, *J* = 2.2 Hz, 1H), 8.04 (s, 1H), 7.86 (dd, *J* = 2.3, 8.7 Hz, 1H), 7.62 (d, *J* = 8.8 Hz, 1H), 4.74-4.73 (m, 1H), 4.20-4.13 (m, 1H), 4.12-4.06 (m, 1H), 2.60 (d, *J=* 4.9 Hz, 4H), 2.36 (q, *J* = 9.0 Hz, 1H), 1.92-1.92 (m, 1H), 1.99-1.87 (m, 4H), 1.79-1.68 (m, 3H), 1.48-1.36 (m, 1H), 1.48-1.36 (m, 1H), 1.48-1.36 (m, 1H), 0.08-0.08 (m, 1H).

### Step 7: synthesis of compound WX196-9

Raw materials **WX196-8** (196 mg, 538.11 µmol) and **BB-3** (262.04 mg, 591.92 µmol) and the solvents 1,4-dioxane (2 mL) and water (0.2 mL) were added into a pre-dried single-necked flask, and then potassium acetate (105.62 mg, 1.08 mmol) was added. The mixture was replaced with nitrogen, followed by addition of 1,1-bis(diphenylphosphine) ferrocene palladium chloride (39.37 mg, 53.81 µmol, 0.1 eq). The mixture was replaced with nitrogen, and further stirred at 90 °C for 12 hours. After the reaction was completed, water (5 mL) and dichloromethane (5 mL × 3) were added to the reaction solution for extraction. The resulting organic phase was dried over anhydrous sodium sulfate, rotary-evaporated under reduced pressure, separated and purified by a preparative thin-layer chromatographic plate (dichloromethane: methanol = 15:1) to obtain target compound **WX196-9.**

### Step 8: synthesis of compound WX196 and WX197

**WX196-9** was resolved and purified by SFC (chromatographic column: Chiralpak AD-H 250 * 30mm id 5µm; mobile phase: A: CO₂, B: MeOH (0.1% NH₄HCO₃); gradient: B% = 42%; flow rate: 70 g/min; wavelength: 220 nm; column temperature: 40 °C; back pressure: 100 bar) to obtain a pair of enantiomers **WX196** (retention time is 2.41 min) and **WX197** (retention time is 3.54 min). **WX196:** ¹H NMR (400MHz, METHANOL-d4) δ = 8.34 (d, J = 2.0 Hz, 1H), 8.28 (s, 1H), 8.23 (d, *J=* 2.4 Hz, 1H), 8.10 (dd, *J* = 5.8, 8.9 Hz, 1H), 8.02 (d, *J* = 2.2 Hz, 1H), 8.01-8.00 (m, 1H), 8.03-7.99 (m, 1H), 7.77 (d, *J* = 8.4 Hz, 1H), 7.47 (dd, *J* = 2.5, 8.5 Hz, 1H), 7.27-7.21 (m, 1H), 4.87 (s, 44H), 4.21-4.14 (m, 1H), 4.12-4.04 (m, 1H), 3.87 (s, 3H), 2.87-2.75 (m, 1H), 2.44 (q, *J* = 8.7 Hz, 1H), 2.32 (s, 3H), 2.05-1.93 (m, 2H), 1.77 (br dd, *J* = 6.2, 13.9 Hz, 2H), 1.53-1.42 (m, 1H). **WX197:** ¹H NMR (400MHz, METHANOL-d4) δ = 8.34 (s, 1H), 8.28 (s, 1H), 8.23 (s, 1H), 8.10 (dd, *J* = 5.8, 8.9 Hz, 1H), 8.03-7.98 (m, 2H), 7.77 (br d, *J* = 8.4 Hz, 1H), 7.47 (dd, *J* = 2.5, 8.5 Hz, 1H), 7.26-7.20 (m, 1H), 4.20-4.04 (m, 3H), 3.87 (s, 4H), 2.81 (br dd, *J* = 7.6, 16.0 Hz, 1H), 2.44 (q, *J* = 8.7 Hz, 1H), 2.33 (s, 4H), 2.34-2.31 (m, 1H), 2.34-2.31 (m, 1H), 2.34-2.31 (m, 1H), 2.02-1.94 (m, 3H), 1.77 (br dd, *J=* 5.8, 13.3 Hz, 3H), 1.51-1.42 (m, 1H).

### Example 105: WX198

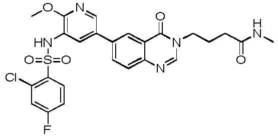

### Synthetic route:

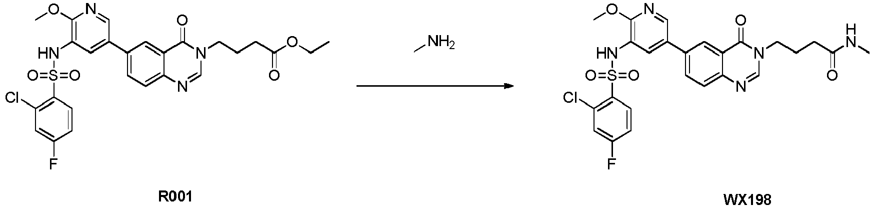

### Step 1: synthesis of compound WX198

**R001** (300.00 mg, 521.73 µmol) was dissolved in ethanol (20 mL), and methylamine alcohol solution (5 mL) was added, heated to 80 °C and stirred overnight. After the reaction was completed, the mixture was cooled to room temperature, and the organic solvent was rotary-evaporated, separated by preparative thin-layer chromatography (eluent: methanol dichloromethane/triethylamine = 1: 20: 0.2), and further separated by preparative high-performance liquid column to obtain target compound **WX198.** ¹H NMR (400MHz, CDCl₃) δ: 8.27 (d, *J* = 2.0 Hz, 1H), 8.13-7.99 (m, 3H), 7.91 (d, *J* = 2.3 Hz, 1H), 7.83-7.76 (m, 1H), 7.75-7.67 (m, 1H), 7.21 (br s, 1H), 7.07-6.98 (m, 1H), 5.80 (br s, 1H), 4.07 (t, *J* = 6.8 Hz, 2H), 3.91 (s, 3H), 2.76 (d, *J* = 4.8 Hz, 3H), 2.33-2.08 (m, 2H), 2.16-1.93 (m, 2H). MS-ESI m/z: 560.1[M+H]⁺, 562.1 [M+H+2]⁺.

### Example 106: WX199

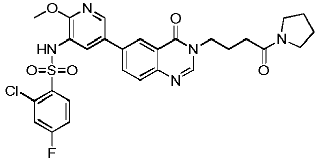

### Synthetic route:

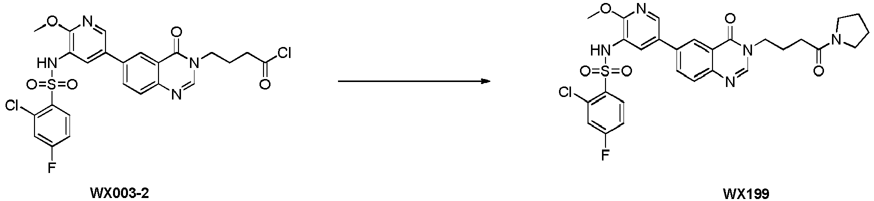

### Step 1: synthesis of compound WX199

Compound tetrahydropyrrole (15.09 mg, 212.24 µmol) and triethylamine (35.79 mg, 353.74 µmol) were dissolved in anhydrous dichloromethane (5 mL), and a solution of **WX003-2** (100.00 mg, 176.87µmol) in dichloromethane (1 mL) was added dropwise at 0 °C with stirring. The reaction solution was stirred at 0 °C for 1 hour. After the reaction was completed, the solvent was removed under reduced pressure. The residue was separated by preparative thin-layer chromatography (eluent: methanol / dichloromethane / triethylamine = 1: 15: 0.15), and further separated by preparative high-performance liquid column to obtain target compound **WX199.** ¹H NMR (400MHz, CDCl₃) δ : 8.36 (d, *J* = 2.0 Hz, 1H), 8.20-8.11 (m, 3H), 8.00 (d, *J* = 2.3 Hz, 1H), 7.92-7.76 (m, 2H), 7.55 (br s, 1H), 7.27 (br s, 1H), 7.19-7.07 (m, 1H), 4.20 (t, *J* = 7.2 Hz, 2H), 4.00 (s, 3H), 3.47 (t, *J* = 6.9 Hz, 2H), 3.41 (t, *J* = 6.8 Hz, 2H), 2.44-2.33 (m, 2H), 2.28-2.13 (m, 2H), 2.03-1.93 (m, 2H), 1.93-1.79 (m, 2H). MS-ESI m/z: 600.1[M+H]⁺, 602.1[M+H+2]⁺.

### Example 107: WX200, WX201

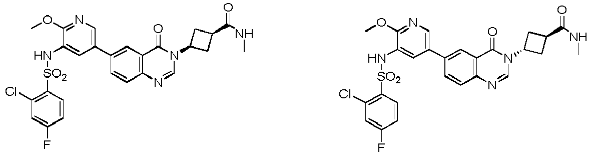

### Synthetic route:

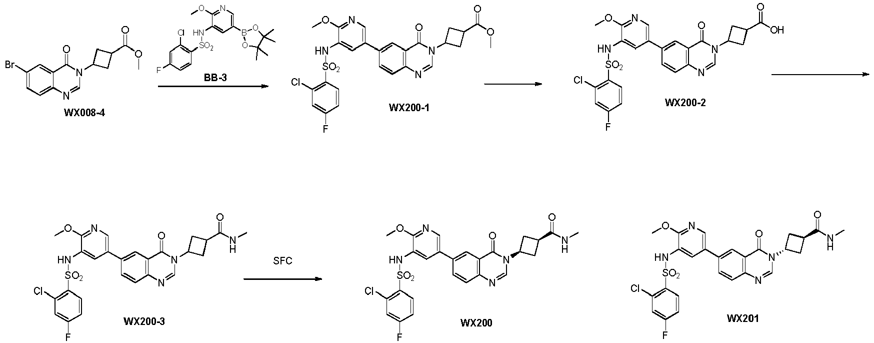

### Step 1: synthesis of compound WX200-1

A mixture of compound **WX008-4** (150.00 mg, 444.88 µmol), compound **BB-3** (236.34 mg, 533.86 µmol), potassium acetate (130.98 mg, 1.33 mmol), ferrocene palladium chloride (32.55 mg, 44.49 µmol), dioxane (3.00 mL) and water (300.00 µL) was stirred at 100 °C for 2 hours under nitrogen atmosphere. The reaction solution was rotary-evaporated, followed by addition of water (30 mL), and then extracted with (dichloromethane / methanol = 10:1) (30 mL × 3). The organic phases were combined, washed once with saturated brine (30 mL), dry over anhydrous sodium sulfate, filtered, concentrated to dryness under reduced pressure, separated and purified by preparative chromatographic plate (petroleum ether: ethyl acetate: dichloromethane = 1: 1: 0.5) to obtain target compound **WX200-1.** ¹H NMR (400MHz, CDCl₃) δ = 8.24 (s, 1H), 8.19 (s, 1H), 8.08-8.03 (m, 2H), 7.89 (s, 1H), 7.80-7.67 (m, 2H), 7.22-7.15 (m, 1H), 7.09-7.00 (m, 1H), 5.24-4.99 (m, 1H), 3.90 (s, 3H), 3.42 (s, 3H), 3.26-3.15 (m, 1H), 3.07-2.94 (m, 1H), 2.86-2.77 (m, 3H), 2.68-2.52 (m, 1H). MS-ESI m/z: 573.1 [M+H]⁺.

### Step 2: synthesis of compound WX200-2

A mixture of compound **WX200-1** (100.00 mg, 174.52 µmol), lithium hydroxide monohydrate (36.61 mg, 872.62 µmol), tetrahydrofuran (1.00 mL), water (1.00 mL) and methanol (1.00 mL) was stirred at 23 °C for 1 hour. The reaction solution was rotary-evaporated, followed by addition of water (5 mL), adjusted to pH = 3 with dilute hydrochloric acid (2 N). A light yellow solid was precipitated out, followed by filtration. The filter cake was washed with water (2 mL), dissolved with methanol (50 mL) and rotary-evaporated to obtain a crude product of the target compound **WX200-2.** MS-ESI m/z: 559 [M+H]⁺.

### Step 3: synthesis of compound WX200-3

A mixture of compound **WX200-2** (80.00 mg, 143.12 µmol), methylamine hydrochloride (14.50 mg, 214.68 µmol), 2-(7-benzotriazole)-*N,N,N,N*,-tetramethylurea hexafluorophosphate (81.63 mg, 214.68 µmol), triethylamine (43.45 mg, 429.36 µmol, 59.52 µL) and dichloromethane (3.00 mL) was stirred at 20 °C for 1 hour, and water was added to the reaction solution (30 mL), and then extracted with dichloromethane / methanol = 10: 1 (30 mL × 3). The organic phases were combined, washed once with saturated brine (30 mL), dried over anhydrous sodium sulfate and filtered. The filtrate was concentrated under reduced pressure to dryness, separated and purified by preparative HPLC to obtain the target compound **WX200-3.**

### Step 4: synthesis of compound WX200 and WX201

Compound **WX200-3** was resolved by supercritical fluid chromatography (separation condition column: OJ (250mm * 30mm, 10µm); mobile phase: [0.1% NH₄HCO₃ MEOH]; B%: 35% -35%) to obtain cis-trans isomers **WX200** (cis) and and **WX201** (trans) which were identified by NOE. **WX200**(cis): ¹H NMR (400MHz, CDCl₃) δ = 8.34-8.30 (m, 2H), 8.15-8.09 (m, 2H), 7.97 (d, *J* = 2.0 Hz, 1H), 7.86-7.77 (m, 2H), 7.56 (br s, 1H), 7.25 (d, *J* = 2.5 Hz, 1H), 7.11 (ddd, *J* = 2.5, 7.5, 8.8 Hz, 1H), 5.78 (br s, 1H), 5.16-5.03 (m, 1H), 3.97 (s, 3H), 2.88 (d, *J* = 5.0 Hz, 3H), 2.86-2.74 (m, 5H), MS-ESI m/z: 572.1 [M+H]⁺. **WX201** (trans), ¹H NMR (400MHz, CDCl₃) δ : 8.33 (d, *J* = 2.0 Hz, 1H), 8.16-8.07 (m, 3H), 7.98 (d, *J* = 2.3 Hz, 1H), 7.87-7.81 (m, 1H), 7.80-7.74 (m, 1H), 7.53 (br s, 1H), 7.25 (d, *J* = 2.3 Hz, 1H), 7.16-7.07 (m, 1H), 5.55 (br d, *J* = 4.0 Hz, 1H), 5.22 (quin, *J* = 8.4 Hz, 1H), 3.98 (s, 3H), 3.16-3.05 (m, 1H), 3.01-2.92 (m, 2H), 2.90 (d, *J* = 5.0 Hz, 3H), 2.89-2.82 (m, 2H), MS-ESI m/z: 572.1 [M+H]⁺. The retention time of **WX200** and **WX201** is 1.529min and 1.874min, respectively, and the ratio is 1:1.

### Example 108: WX202

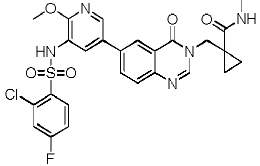

### Synthetic route:

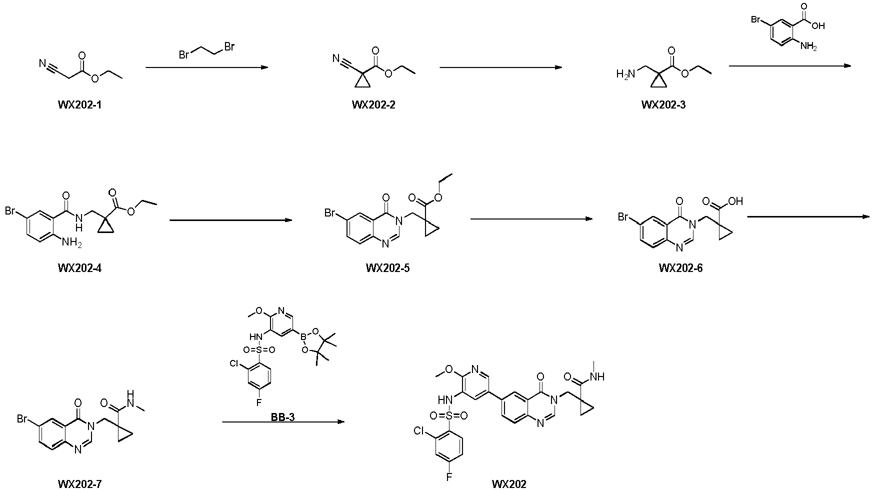

### Step 1: synthesis of compound WX202-2

Compound **WX202-1** (10 g, 88.41 mmol) and potassium carbonate (30.55 g, 221.02 mmol) were dissolved in DMF (20.00 mL), and 1,2-dibromoethane (15.78 g, 83.99 mmol) was added thereto at 0°C, and stirred at 25 °C overnight. After the reaction was completed, the mixture was poured into water (100 mL) and extracted three times with dichloromethane (100 mL). The organic phases obtained were combined and dried over anhydrous sodium sulfate. After the desiccant was removed by filtration, the solvent was removed under reduced pressure, and the residue was purified by column chromatography (ethyl acetate / petroleum ether = 0%-10%) to obtain target compound **WX202-2.** ¹H NMR (400MHz, CDCl₃) δ : 4.28 (q, *J* = 7.2 Hz, 2H), 1.76-1.57 (m, 4H), 1.35 (t, *J* = 7.2 Hz, 3H).

### Step 2: synthesis of compound WX202-3

Compound **WX202-2** (8 g, 57.49 mmol) was dissolved in methanol (25.00 mL), and Raney Ni (985.06 mg, 11.50 mmol) was added under nitrogen atmosphere, followed by introduction of hydrogen (50 psi). The mixture was stirred at 30 °C overnight. After the reaction was completed, the solvent was removed under reduced pressure to obtain target compound **WX202-3.** ¹H NMR (400MHz, CDCl₃) δ : 5.76 (br s, 1H), 4.29-4.00 (m, 2H), 1.57-0.95 (m, 7H). MS-ESI m/z: 144.1[M+H]+.

### Step 3: synthesis of compound WX202-4

The compound 2-amino-5-bromobenzoic acid (3.0 g, 13.89 mmol) was dissolved in *N,N'*-dimethylformamide (10.00 mL), and diisopropylethylamine (1.79 g, 13.89 mmol), HATU (5.28 g, 13.89 mmol) and **WX202-3** (2.49 g, 13.89 mmol) were added thereto and stirred at 25 °C for 2 hours. After the reaction was completed, the mixture was poured into water (100.00 mL) and extracted three times with dichloromethane (100.00 mL). The organic phases obtained were combined and dried over anhydrous sodium sulfate. After the desiccant was removed by filtration, the solvent was removed under reduced pressure and the residue was separated by chromatographic column (eluent: ethyl acetate / petroleum ether = 0%-20%) to obtain target compound **WX202-4.** MS-ESI m/z:525.1.0[M+H]⁺,327.1[M+H+2]⁺.

### Step 4: synthesis of compound WX202-5

Compound **WX202-4** (1.0 g, 2.93 mmol) was dissolved in ethanol (40.00 mL), and methylphenidate acetate (915.37 mg, 8.79 mmol) was added and stirred at 80 °C for 2 hours. After the reaction was completed, the mixture was rotary-evaporated to remove the organic solvent, poured into water (20.00 mL), and extracted three times with dichloromethane (20.00 mL). The organic phases obtained were combined and dried over anhydrous sodium sulfate. After the desiccant was removed by filtration, the solvent was removed under reduced pressure to obtain target compound **WX202-5.** MS-ESI m/z: 353.0[M+H]⁺, 355.0[M+H+2]⁺.

### Step 5: synthesis of compound WX202-6

**WX202-5** (0.8 g, 2.28 mmol l) was dissolved in tetrahydrofuran (10.00 mL) and water (10.00 mL), then lithium hydroxide monohydrate (382.36 mg, 9.11 mmol) was added thereto, and the reaction solution was stirred at 30 °C for 1 hour. After the reaction was completed, the reaction solution was rotary-evaporated, followed by addition of water (10 mL), and washed three times with dichloromethane (10 mL). The concentrated hydrochloric acid (1 mL) was added dropwise to the aqueous phase, extracted with dichloromethane (10 mL) three times and dried over anhydrous sodium sulfate. The organic phase was rotary-evaporated to obtain target compound **WX202-6.** MS-ESI m/z: 324.9[M+H]⁺, 326.9[M+H+2]⁺.

### Step 6: synthesis of compound WX202-7

Compound **WX202-6** (0.5 g, 1.55 mmol) was dissolved in *N,N'-*dimethylformamide (5.00 mL), followed by addition of diisopropylethylamine (199.97 mg, 1.55 mmol), HATU (588.33 mg, 1.55 mmol) and methylamine hydrochloride (104.47 mg, 1.55 mmol), and stirred at 30 °C for 2 hours. After the reaction was completed, the mixture was poured into water (20 mL) and extracted three times with dichloromethane (20 mL). The organic phases obtained were combined and dried over anhydrous sodium sulfate. After the desiccant was removed by filtration, the solvent was removed under reduced pressure to obtain target compound **WX202-7.** ¹H NMR (400MHz, Methanol-d4) δ: 8.48 (s, 1H), 8.45-8.26 (m, 1H), 7.99-7.83 (m, 1H), 7.71-7.46 (m, 1H), 3.87-3.56 (m, 2H), 2.88 (s, 3H), 1.56-1.38 (m, 9H). MS-ESI m/z:525.1.0[M+H]⁺,327.1[M+H+2]⁺.

### Step 7: synthesis of compound WX202

Compound **WX202-7** (0.15 g, 338.83 µmol), **BB-2** (0.15 g, 338.83 µmol), and potassium acetate (133.02 mg, 1.36 mmol) were dissolved in dioxane (2.00 mL) and water (0.20 mL), followed by addition of [1,1'-bis(diphenylphosphino) ferrocene] palladium dichloride (55.34 mg, 67.77 µmol). The mixture was heated to 95 °C and stirred for 2 hours under nitrogen atmosphere. After the reaction was completed, the mixture was cooled to room temperature, rotary-evaporated to remove the organic solvent, poured into water (100 mL), and extracted three times with dichloromethane (100 mL). The organic phases obtained were combined and dried over anhydrous sodium sulfate. After the desiccant was removed by filtration, the solvent was removed under reduced pressure, separated by preparative thin-layer chromatographic plate (eluent: methanol / dichloromethane = 1:20), and further separated by preparative high-performance liquid column to obtain target compound **WX202.** ¹H NMR (400MHz, Methanol-d4) δ: 8.48 (s, 1H), 8.34 (d, *J* = 1.8 Hz, 1H), 8.23 (d, *J* = 1.5 Hz, 1H), 8.12 (dd, *J* = 5.8, 8.8 Hz, 1H), 8.07-7.92 (m, 2H), 7.78 (d, *J* = 8.5 Hz, 1H), 7.48 (dd, *J* = 2.3, 8.5 Hz, 1H), 7.34-7.16 (m, 1H), 4.36 (s, 2H), 3.89 (s, 3H), 2.69 (s, 3H), 1.33-1.13 (m, 4H). MS-ESI m/z:572.0[M+H]⁺,574.0[M+H+2]⁺.

### Example 109: WX203, WX204

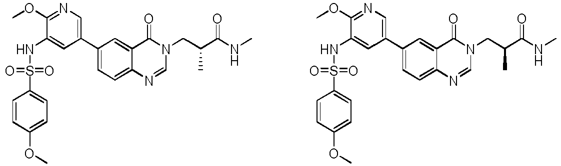

### Synthetic route:

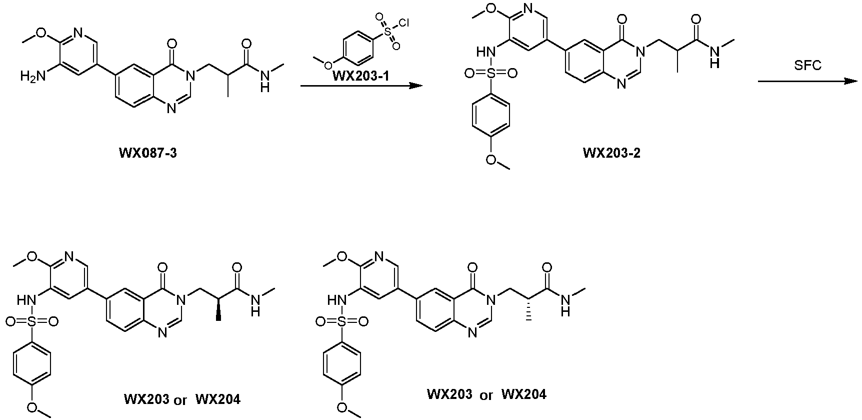

### Step 1: synthesis of compound WX203-2

Compound **WX087-3** (0.3 g, 816.55 µmol) was dissolved in pyridine (5 mL), followed by addition of **WX203-1** (157.82 mg, 742.32 µmol, 99.88 µL), and the reaction solution was stirred at 25 °C for 10 hours. After the reaction was completed, the reaction solution was rotary-evaporated and separated by preparative HPLC (TFA) to obtain target compound **WX203-2.** MS-ESI *m*/*z:* 538.2 [M+H]⁺.

### Step 2: synthesis of compound WX203 and WX204

Compound **WX203-2** was resolved by supercritical fluid chromatography (separation conditions: chromatographic column: AD (250mm * 30mm, 10µm); mobile phase: [0.1% NH₄HCO₃ ETOH]; B%: 55%-55%, flow rate (mL/min) : 80mL/min) to obtain a pair of enantiomers **WX203** (retention time is 1.089 min) and **WX204** (retention time is 3.422 min), and the ratio is 1:1. **WX203:** ¹H NMR (400 MHz, DMSO-*d*₆) δ = 8.25-8.31 (m, 1 H), 8.22 (d, *J* = 1.8 Hz, 1 H), 8.19 (s, 1 H), 8.05 (dd, *J* = 8.5, 2.0 Hz, 1 H), 7.83-7.92 (m, 2 H), 7.68-7.78 (m, 3 H), 7.08 (d, *J* = 8.8 Hz, 2 H), 4.01-4.17 (m, 1 H), 3.86-4.01 (m, 1 H), 3.81 (s, 3 H), 3.73 (s, 3 H), 2.76-3.01 (m, 1 H), 2.47-2.49 (m, 3 H), 1.08 (d, *J* = 7.0 Hz, 3 H). MS-ESI *m*/*z:* 538.2 [M+H]⁺. **WX204:** ¹H NMR (400 MHz, DMSO-*d*₆) δ = 8.26-8.31 (m, 1 H), 8.23 (s, 1 H), 8.18-8.22 (m, 1 H), 8.05 (dd, *J* = 8.5, 2.0 Hz, 1 H), 7.90 (br d, *J* = 4.8 Hz, 1 H), 7.85-7.88 (m, 1 H), 7.76 (d, *J* = 8.5 Hz, 1 H), 7.72 (d, *J* = 8.0 Hz, 2 H), 7.09 (d, *J* = 8.8 Hz, 2 H), 4.02-4.17 (m, 1 H), 3.85-4.02 (m, 1 H), 3.82 (s, 3 H), 3.74 (s, 3 H), 2.76-3.00 (m, 1 H), 2.49-2.50 (m, 3 H), 1.09 (d, *J* = 7.0 Hz, 3 H). MS-ESI *m*/*z:* 538.2 [M+H]⁺.

### Example 110: WX205, WX206

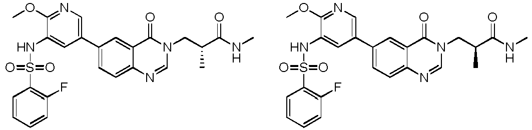

### Synthetic route:

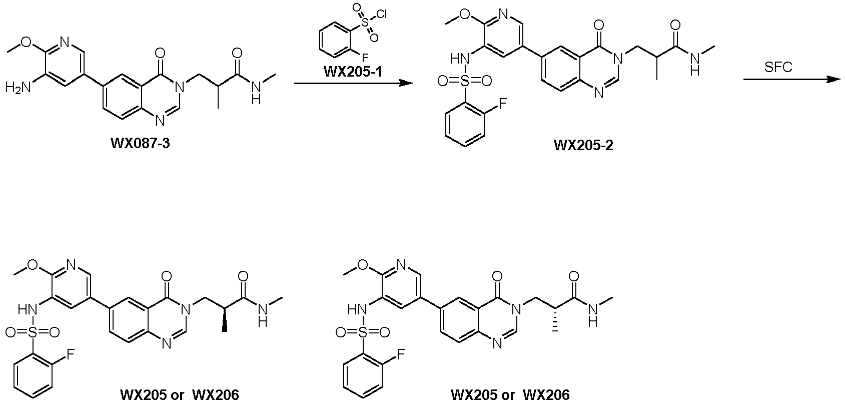

### Step 1: synthesis of compound WX205-2

Compound **WX087-3** (0.3 g, 816.55 µmol) was dissolved in pyridine (5 mL), followed by addition of compound **WX205-1** (158.91 mg, 816.55 µmol, 108.10 µL), and the reaction solution was stirred at 25 °C for 10 hours. After the reaction was completed, the reaction solution was rotary-evaporated and separated by preparative HPLC (TFA) to obtain target compound **WX205-2.** MS-ESI *m*/*z:* 526.1 [M+H]⁺.

### Step 2: synthesis of compound WX205 and WX206

Compound **WX205-2** was resolved by supercritical fluid chromatography (separation conditions: column: AD (250mm * 30mm, 10µm); mobile phase: [0.1% NH₄HCO₃ EtOH]; B%: 55%-55%, flow rate (ml/min) : 80 mL/min) to obtain a pair of enantiomers **WX205** (retention time is 0.845 min) and **WX206** (retention time is 2.551min), the ratio is 1:1. **WX205:** ¹H NMR (400 MHz, DMSO-*d*₆) δ = 8.33 (br s, 1 H), 8.24 (d, *J* = 2.0 Hz, 1 H), 8.18 (s, 1 H), 8.06 (dd, *J* = 8.5, 2.0 Hz, 1 H), 7.89 (br d, *J* = 2.5 Hz, 2 H), 7.65-7.77 (m, 3 H), 7.43 (t, *J* = 9.7 Hz, 1 H), 7.32 (t, *J* = 7.4 Hz, 1 H), 4.03-4.11 (m, 1 H), 3.92-4.01 (m, 1 H), 3.67 (s, 3 H), 3.31 (br s, 3 H), 2.86 (br dd, *J* = 14.6, 6.8 Hz, 1 H), 1.08 (d, *J* = 7.0 Hz, 3 H). MS-ESI *m*/*z:* 526.1 [M+H]⁺. **WX206:** ¹H NMR (400 MHz, DMSO-*d*₆) δ = 8.34 (br s, 1 H), 8.24 (d, *J* = 2.3 Hz, 1 H), 8.18 (s, 1 H), 8.07 (dd, *J* = 8.5, 2.3 Hz, 1 H), 7.85-7.93 (m, 2 H), 7.66-7.77 (m, 3 H), 7.44 (t, *J* = 9.3 Hz, 1 H), 7.33 (t, *J* = 7.7 Hz, 1 H), 4.02-4.11 (m, 1 H), 3.96 (dd, *J* = 13.3, 9.0 Hz, 1 H), 3.67 (s, 3 H), 2.86 (br dd, *J* = 14.6, 7.0 Hz, 1 H), 2.48 (br s, 3 H), 1.08 (d, *J* = 6.8 Hz, 3 H). MS-ESI *m*/*z:* 526.1 [M+H]⁺.

### Example 111: WX207, WX208

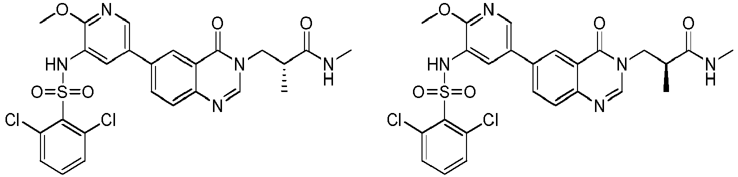

### Synthetic route:

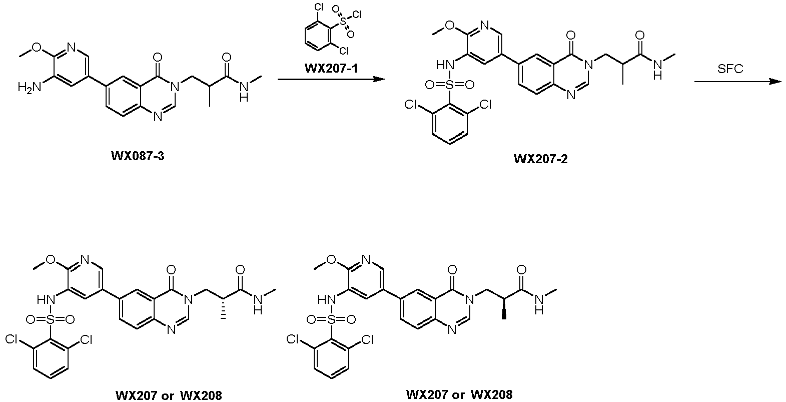

### Step 1: synthesis of compound WX207-2

Compound **WX087-3** (0.3 g, 816.55 µmol) was dissolved in pyridine (5 mL), followed by addition of compound **WX207-1** (200.47 mg, 816.55 µmol), and the reaction solution was stirred at 25 °C for 10 hours. After the reaction was completed, the reaction solution was rotary-evaporated and separated by preparative HPLC separation (TFA) to obtain target compound **WX207-2.** MS-ESI *m*/*z:* 576.1 [M+H]⁺.

### Step 2: synthesis of compound WX207 and WX208

Compound **WX207-2** was resolved by supercritical fluid chromatography (separation conditions: column: AD (250mm * 30mm, 10µm); mobile phase: [0.1% NH₄HCO₃ IPA]; B%: 45%-45%, flow rate (mL/min): 80mL/min) to obtain enantiomers **WX207** (retention time is 5.470 min) and **WX208** (retention time is 6.027 min). **WX207:** ¹H NMR (400 MHz, DMSO-*d*₆) δ = 8.26-8.33 (m, 1 H), 8.25 (d, *J* = 2.0 Hz, 1 H), 8.18 (s, 1 H), 8.05 (br *d, J* = 6.8 Hz, 1 H), 7.82-7.93 (m, 2 H), 7.75 (d, *J* = 8.5 Hz, 1 H), 7.58-7.64 (m, 2 H), 7.53 (br d, *J* = 8.0 Hz, 1 H), 4.02-4.11 (m, 1 H), 3.96 (dd, *J* = 13.3, 9.0 Hz, 1 H), 3.67 (s, 3 H), 3.31 (br s, 3 H), 2.86 (br dd, *J* = 14.6, 7.0 Hz, 1 H), 1.08 (d, *J* = 6.8 Hz, 3 H). MS-ESI *m*/*z*: 576.1 [M+H]⁺. **WX208:** ¹H NMR (400 MHz, DMSO-*d*₆) δ = 8.20-8.35 (m, 1 H), 8.17 (s, 1 H), 8.03 (br d, *J* = 8.8 Hz, 1H), 7.90 (br d, *J* = 4.5 Hz, 1H), 7.81 (br s, 1H), 7.73 (d, *J* = 8.5 Hz, 1H), 7.58 (br d, *J* = 7.8 Hz, 2H), 7.49 (br d, *J* = 8.3 Hz, 1H), 4.01-4.12 (m, 1H), 3.96 (dd, *J* = 13.3, 9.3 Hz, 1H), 3.68 (s, 3H), 3.31-3.33 (m, 3H), 2.81-2.91 (m, 1H), 1.08 (d, *J* = 7.0 Hz, 3H). MS-ESI *m*/*z:* 576.1 [M+H]⁺. The ratio of two isomers is 1:1.

### Example 112: WX209, WX2110

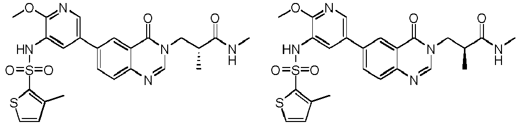

### Synthetic route:

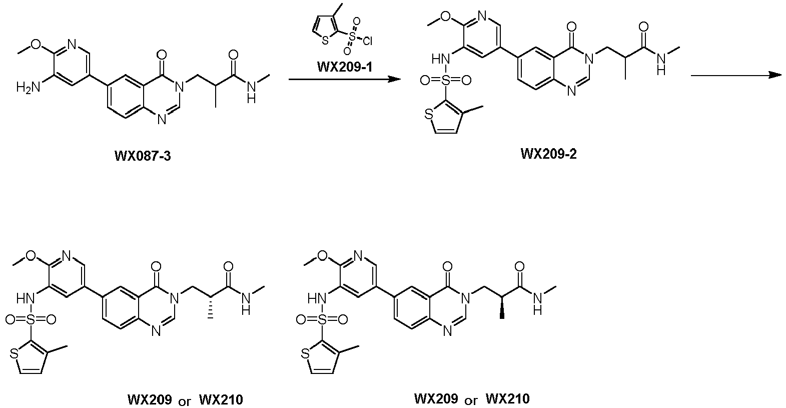

### Step 1: synthesis of compound WX209-2

Compound **WX087-3** (0.3 g, 816.55 µmol) was dissolved in pyridine (5 mL), followed by addition of compound **WX209-1** (0.350 g, 1.78 mmol), and the reaction solution was stirred at 25 °C for 10 hours. After the reaction was completed, the reaction solution was rotary-evaporated and separated by preparative HPLC (TFA) to obtain target compound **WX209-2.** MS-ESI *m*/*z:* 528.1 [M+H]⁺.

### Step 2: synthesis of compound WX209 and WX210

Compound **WX209-2** was resolved by supercritical fluid chromatography (separation conditions: chromatographic column: AD (250mm * 50mm, 10µm); mobile phase: [0.1% NH₄HCO₃ ETOH]; B%: 55%-55%) to obtain enantiomers **WX209** (retention time is 0.801 min) and **WX210** (retention time is 2.556min). **WX209:** ¹H NMR (400 MHz, DMSO-*d*₆) δ = 8.29 (br s, 1 H), 8.22-8.27 (m, 1 H), 8.17 (s, 1 H), 8.05 (dd, *J* = 8.4, 2.1 Hz, 1 H), 7.89 (br d, *J* = 2.5 Hz, 2 H), 7.75 (d, *J* = 8.5 Hz, 1 H), 7.68 (d, *J* = 4.8 Hz, 1 H), 7.00 (d, *J* = 5.0 Hz, 1 H), 4.02-4.13 (m, 1 H), 3.96 (dd, *J* = 13.2, 9.2 Hz, 1 H), 3.75 (s, 3 H), 3.33 (br s, 3 H), 2.82-2.91 (m, 1 H), 2.33 (s, 3 H), 1.08 (d, *J* = 7.0 Hz, 3 H). MS-ESI *m*/*z:* 528.1 [M+H]⁺. **WX210:** ¹H NMR (400 MHz, DMSO-*d*₆) δ = 8.26-8.35 (m, 1 H), 8.25 (d, *J* = 2.0 Hz, 1 H), 8.17 (s, 1 H), 8.05 (dd, *J* = 8.5, 2.3 Hz, 1 H), 7.89 (s, 2 H), 7.75 (d, *J* = 8.3 Hz, 1 H), 7.67 (br d, *J* = 5.0 Hz, 1 H), 6.99 (d, *J* = 5.0 Hz, 1 H), 4.04-4.11 (m, 1 H), 3.96 (dd, *J* = 13.3, 9.3 Hz, 1 H), 3.75 (s, 3 H), 3.33 (br s, 3 H), 2.82-2.92 (m, 1 H), 2.33 (s, 3 H), 1.08 (d, *J* = 6.8 Hz, 3 H). MS-ESI *m*/*z:* 528.1 [M+H]⁺. The ratio of two isomers is 1:1.

### Example 113: WX211, WX212

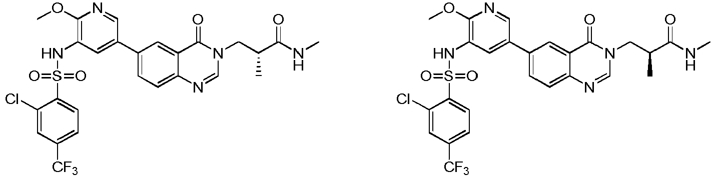

### Synthetic route:

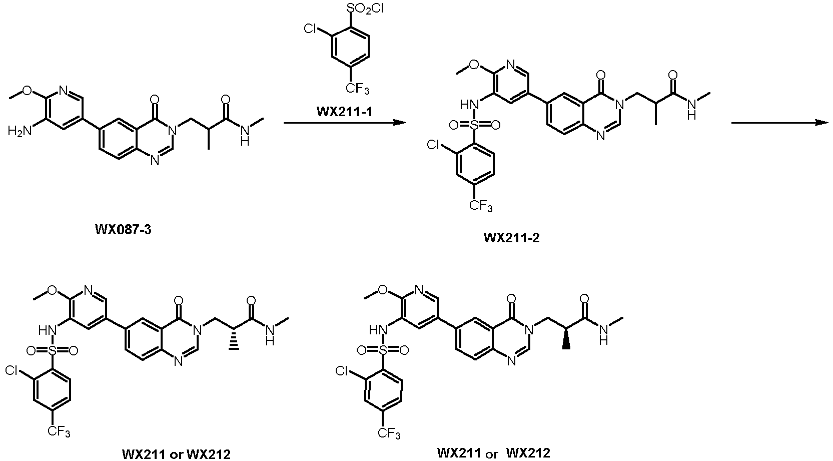

### Step 1: synthesis of compound WX211-2

**WX087-3** (200 mg, 544.36 µmol, 1 eq) and **WX211-1** (227.87 mg, 816.54 µmol, 142.42 µL) were added into a pre-dried 40 mL reaction flask, then pyridine (6 mL) was added. The reaction was stirred at 20 °C for 12 hours. After the reaction was completed, 10 mL of water and 10 mL of ethyl acetate were added to the reaction system for dilution. The organic phase was collected after liquid separation, and the aqueous phase was extracted with ethyl acetate (5 mL × 3). The organic phases were combined, washed with saturated brine (20 mL), dried over anhydrous sodium sulfate, concentrated under reduced pressure, and purified by preparative thin layer chromatography (DCM: MeOH = 20:1) to obtain target compound **WX211-2.**

### Step 2: synthesis of compound WX211 and WX212

**WX211-2** was resolved and purified by SFC (resolution method: chromatography column: AS (250mm * 30mm, 5µm); mobile phase: [MeOH]; B%: 20% -20%, 6.5min) to obtain **WX211** (Rt = 2.537 min) and **WX212** (Rt = 2.740 min). **WX211:** ¹H NMR (400MHz, METHANOL-d4) δ = 8.33 (s, 1H), 8.24 (d, *J* = 2.2 Hz, 1H), 8.22-8.14 (m, 2H), 8.02-7.96 (m, 2H), 7.96-7.95 (m, 1H), 7.79-7.70 (m, 2H), 4.23 (dd, *J* = 5.0, 13.3 Hz, 1H), 4.02 (dd, *J* = 9.8, 13.3 Hz, 1H), 3.78 (s, 3H), 3.06-2.92 (m, 1H), 2.62 (d, *J* = 4.6 Hz, 3H), 1.23 (d, J = 7.1 Hz, 3H). **WX212:** 1HNMR (400MHz, METHANOL-d4) δ = 8.35 (s, 1H), 8.26 (d, *J* = 2.2 Hz, 1H), 8.21-8.14 (m, 2H), 8.04-7.95 (m, 4H), 7.76 (d, *J* = 8.4 Hz, 2H), 4.23 (dd, *J* = 4.9, 13.5 Hz, 1H), 4.06-3.98 (m, 1H), 3.78 (s, 3H), 3.06-2.94 (m, 1H), 2.64-2.57 (m, 3H), 1.23 (d, *J* = 7.1 Hz, 3H).

### Example 114: WX213, WX214

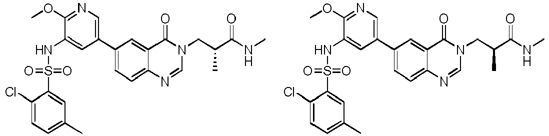

### Synthetic route:

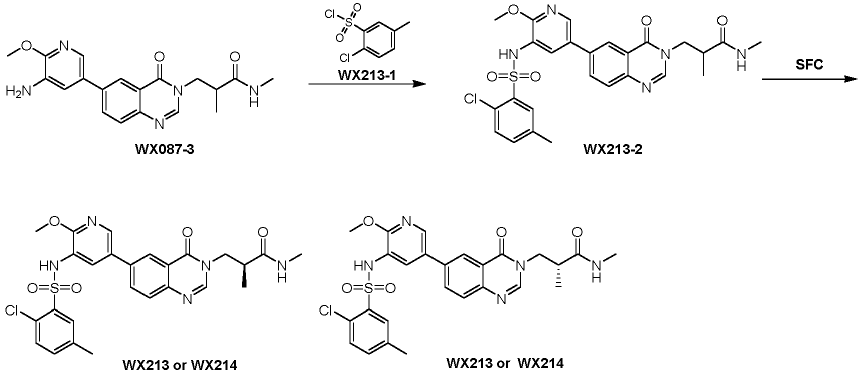

### Step 1: synthesis of compound WX213-2

Compound **WX087-3** (0.2 g, 544.36 µmol) was dissolved in pyridine (3 mL), followed by addition of compound **WX213-1** (147.04 mg, 653.24 µmol), and the reaction solution was stirred at 25 °C for 10 hours. After the reaction was completed, the reaction solution was rotary-evaporated and separated by prepariative thin-layer chromatographic plate (ethyl acetate: methanol = 10:1) to obtain the target compound **WX213-2.**

### Step 2: synthesis of compound WX213 and WX214

Compound **WX213-2** was resolved by supercritical fluid chromatography (separation conditions: column: Chiralpak AS-H 250 * 30 5µ; mobile phase: [0.1% NH₄HCO₃ EtOH]; B%: 35% -35%) to obtain pair of enantiomers **WX213** (retention time is 4.552 min) and **WX214** (retention time is 5.313 min), the ratio is 1:1. **WX213:** ¹HNMR (400 MHz, DMSO-*d*₆) δ = 8.32 (br s, 1 H), 8.25 (d, *J* = 1.8 Hz, 1 H), 8.18 (s, 1 H), 8.04 (br d, *J* = 7.5 Hz, 1 H), 7.85-7.91 (m, 2 H), 7.75 (d, *J* = 8.5 Hz, 1 H), 7.72 (d, *J* = 2.0 Hz, 1 H), 7.57 (br d, *J* = 6.3 Hz, 1 H), 7.43 (br d, *J* = 8.0 Hz, 1 H), 4.03-4.16 (m, 1 H), 3.92-4.00 (m, 1 H), 3.70 (s, 3 H), 2.80-2.93 (m, 1 H), 2.59 (s, 3 H), 2.48 (br s, 3 H), 1.08 (d, *J* = 6.8 Hz, 3 H). MS-ESI *m*/*z:* 556.1 [M+H]⁺. **WX214:** ¹HNMR (400 MHz, DMSO-*d*₆) δ = 8.32 (br s, 1 H), 8.25 (d, *J* = 1.8 Hz, 1 H), 8.18 (s, 1 H), 8.04 (br d, *J* = 7.5 Hz, 1 H), 7.85-7.91 (m, 2 H), 7.75 (d, *J* = 8.5 Hz, 1 H), 7.72 (d, *J* = 2.0 Hz, 1 H), 7.57 (br d, *J* = 6.3 Hz, 1 H), 7.43 (br d, *J* = 8.0 Hz, 1 H), 4.03-4.16 (m, 1 H), 3.92-4.00 (m, 1 H), 3.70 (s, 3 H), 2.80-2.93 (m, 1 H), 2.59 (s, 3 H), 2.48 (br s, 3 H), 1.08 (d, *J* = 6.8 Hz, 3 H). MS-ESI m/z: 556.1 [M+H]⁺.

### Example 115: WX215, WX216

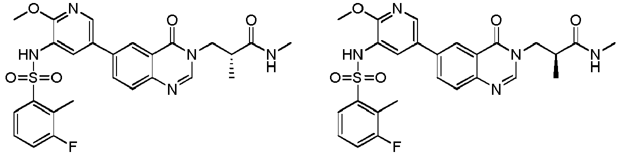

### Synthetic route:

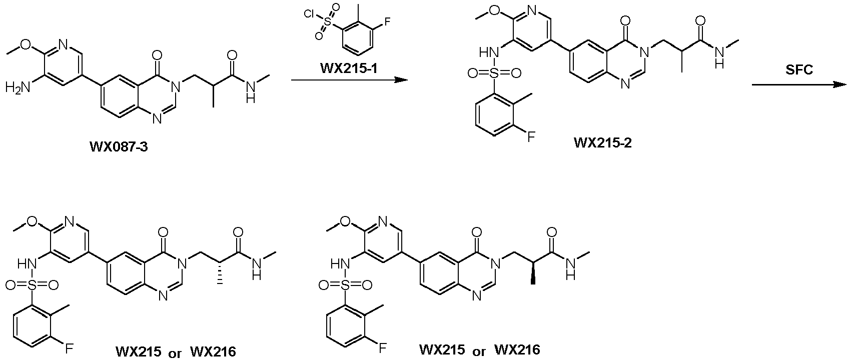

### Step 1: synthesis of compound WX215-2

Compound **WX087-3** (0.2 g, 544.36 µmol) was dissolved in pyridine (3 mL). Compound **WX215-1** (136.29 mg, 653.24 µmol) was added thereto, and the reaction solution was stirred at 25 °C for 10 hours. TLC (ethyl acetate: methanol = 10:1) showed that the reaction of the raw materials was completed. The reaction solution was rotary-evaporated and separated by preparative thin layer chromatographic plate (ethyl acetate: methanol = 10:1) to obtain target compound **WX215-2.**

### Step 2: synthesis of compound WX215 and WX216

Compound **WX215-2** was resolved by supercritical fluid chromatography (separation conditions: column: Chiralpak AS-H 250 * 30 5µ; mobile phase: [0.1% NH₄HCO₃ EtOH]; B%: 35% -35%) to obtain a pair of enantiomers **WX215** (retention time is 4.230 min) and **WX216** (retention time is 5.006 min), and the ratio is 1:1. **WX215:** ¹HNMR (400 MHz, DMSO-*d*₆) δ = 8.18-8.25 (m, 2 H), 8.17 (s, 1 H), 8.02 (dd, *J* = 8.5, 1.8 Hz, 1 H), 7.89 (br d, *J* = 4.8 Hz, 1 H), 7.81 (s, 1 H), 7.74 (d, *J* = 8.5 Hz, 1 H), 7.61 (d, *J* = 7.8 Hz, 1 H), 7.31-7.43 (m, 2 H), 4.03-4.15 (m, 1 H), 3.91-4.00 (m, 1 H), 3.71 (s, 3 H), 2.82-2.95 (m, 1 H), 2.54 (d, *J=* 1.8 Hz, 3 H), 2.48 (br s, 3 H), 1.08 (d, *J* = 7.0 Hz, 3 H). MS-ESI *m*/*z*: 540.1 [M+H]⁺. **WX216:** ¹HNMR (400 MHz, DMSO-*d*₆) δ = 8.23-8.31 (m, 1 H), 8.21 (s, 1 H), 8.18 (s, 1 H), 8.04 (dd, *J* = 8.4, 2.1 Hz, 1 H), 7.86-7.92 (m, 1 H), 7.84 (d, *J* = 2.0 Hz, 1 H), 7.75 (d, *J* = 8.5 Hz, 1 H), 7.59 (d, *J* = 7.8 Hz, 1 H), 7.43 (t, *J* = 9.0 Hz, 1 H), 7.31-7.38 (m, 1 H), 4.07 (dd, *J* = 13.3, 5.5 Hz, 1 H), 3.96 (dd, *J* = 13.2, 9.2 Hz, 1 H), 3.70 (s, 3 H), 2.86 (br dd, *J* = 14.8, 6.8 Hz, 1 H), 2.55 (d, *J* = 1.8 Hz, 3 H), 2.48 (br s, 3 H), 1.05-1.11 (m, 3 H). MS-ESI m/z: 540.1 [M+H]⁺.

### Example 116: WX217, WX218

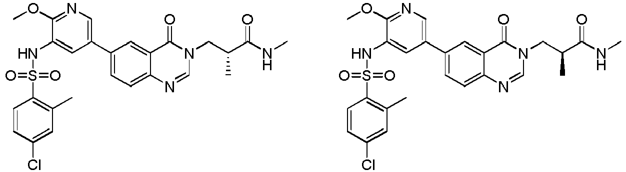

### Synthetic route:

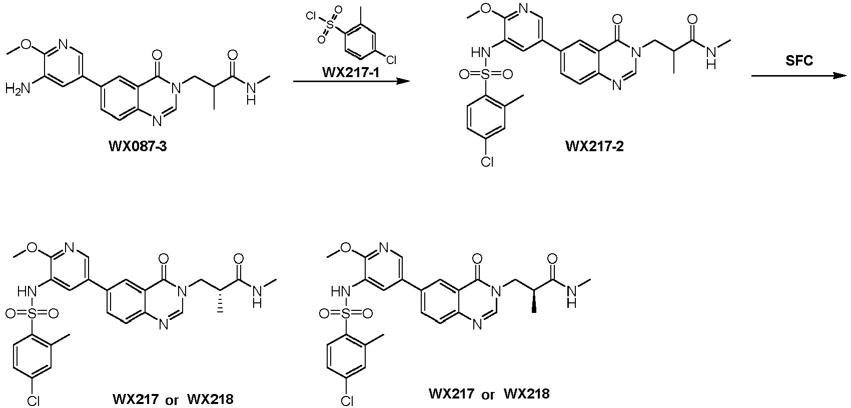

### Step 1: synthesis of compound WX217-2

Compound **WX087-3** (0.2 g, 544.36 µmol) was dissolved in pyridine (5 mL). Compound **WX217-1** (183.80 mg, 816.55 µmol, 439.49 µL) was added thereto, and the reaction solution was stirred at 25 °C for 10 hours. TLC (ethyl acetate: methanol = 10:1) showed that the reaction of the raw materials was completed. The reaction solution was rotary-evaporated and separated by preparative TLC plate (ethyl acetate: methanol = 10:1) to obtain target compound **WX217-2.**

### Step 2: synthesis of compound WX217 and WX218

Compound **WX217-2** was resolved by supercritical fluid chromatography (separation conditions: column: AD (250mm * 30mm, 10µm); mobile phase: [0.1% NH₄HCO₃ EtOH]; B%: 55% -55%) to obtain a pair of enantiomers **WX217** (retention time is 0.890 min) and **WX218** (retention time is 2.551 min), and the ratio is 1:1. **WX217:** ¹HNMR (400 MHz, DMSO-*d*₆) δ = 8.30 (br s, 1 H), 8.21-8.26 (m, 1 H), 8.15-8.21 (m, 1 H), 8.05 (br d, *J* = 8.0 Hz, 1 H), 7.82-7.94 (m, 2 H), 7.73-7.82 (m, 1 H), 7.69 (d, *J* = 8.5 Hz, 1 H), 7.49-7.58 (m, 1 H), 7.37 (br d, *J* = 8.8 Hz, 1 H), 4.01-4.14 (m, 1 H), 3.91-4.00 (m, 1 H), 3.66-3.74 (m, 3 H), 2.86 (br d, *J* = 7.3 Hz, 1 H), 2.58-2.68 (m, 3 H), 2.47-2.49 (m, 3 H), 1.08 (br d, *J* = 7.0 Hz, 3 H). MS-ESI *m*/*z:* 556.3 [M+H]⁺. **WX218:** ¹HNMR (400 MHz, DMSO-*d*₆) δ = 8.20-8.28 (m, 2 H), 8.18 (s, 1 H), 8.03 (dd, *J* = 8.5, 2.0 Hz, 1 H), 7.90 (br d, *J* = 4.8 Hz, 1 H), 7.78-7.87 (m, 1 H), 7.66-7.77 (m, 2 H), 7.51 (s, 1 H), 7.36 (br d, *J* = 8.5 Hz, 1 H), 4.04-4.12 (m, 1 H), 3.90-4.01 (m, 1 H), 3.60-3.79 (m, 4 H), 2.81-2.91 (m, 1 H), 2.64 (s, 3 H), 2.48 (br s, 3 H), 1.08 (d, *J* = 7.0 Hz, 3 H). MS-ESI *m*/*z:* 556.3 [M+H]⁺.

### Example 117: WX219, WX220

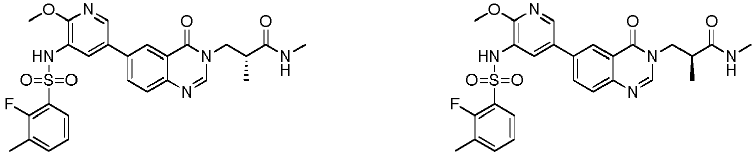

### Synthetic route:

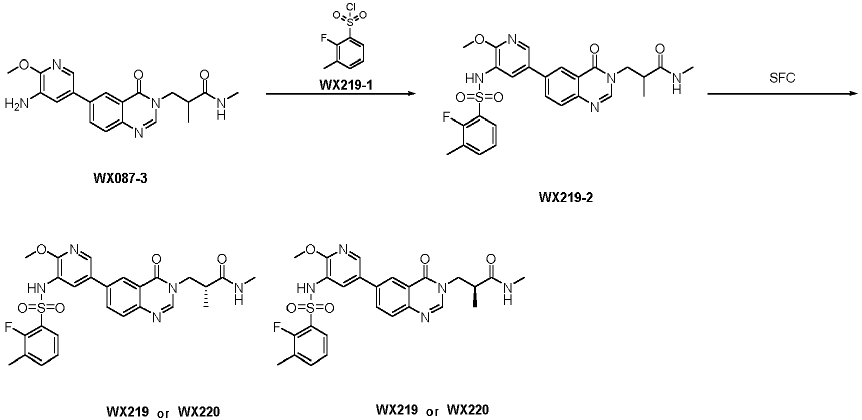

### Step 1: synthesis of compound WX219-2

**WX087-3** (0.1 g, 272.18 µmol) was dissolved in pyridine (2.0 mL), and **WX219-1** (56.79 mg, 272.18 µmol) was added dropwise at 25 °C. The reaction solution was stirred at 28 °C for 16 hours to complete the reaction. The reaction solution was rotary-evaporated, followed by addition of water (10.00 mL), and washed three times with dichloromethane (10.00 mL). The organic phases were combined, dried over anhydrous sodium sulfate, and rotary-evaporated to obtain target compound **WX219-2.** MS-ESI m/z: 540.1[M+H]⁺, 542.1[M+H+2]⁺.

### Step 2: synthesis of compound WX219, WX220

**WX219-2** was resolved and purified by SFC (chromatographic column: AD (250mm * 30mm, 10µm); mobile phase: [0.1% NH₄HCO₃ ETOH]; B%: 55% -55%) to obtain a pair of enantiomers **WX219** (Rt = 0.864 min) and **WX220** (Rt = 3.075 min). **WX222:** ¹H NMR (400MHz, CDCl₃) δ : 8.26 (d, *J* = 2.0 Hz, 1H), 8.10-8.00 (m, 2H), 7.93 (d, *J* = 2.0 Hz, 1H), 7.84-7.74 (m, 1H), 7.74-7.59 (m, 2H), 7.48-7.30 (m, 1H), 7.26 (s, 1H), 7.07 (t, *J* = 7.7 Hz, 1H), 5.51 (br d, *J* = 4.5 Hz, 1H), 4.17-4.04 (m, 1H), 3.97 (dd, *J* = 9.3, 13.3 Hz, 1H), 3.90 (s, 3H), 2.95-2.80 (m, 1H), 2.67 (d, *J* = 4.8 Hz, 3H), 2.25 (d, *J* = 1.8 Hz, 3H), 1.21 (d, *J* = 7.0 Hz, 3H). **WX223:** ¹H NMR(400MHz, CDCl₃) δ : 8.26 (d, *J* = 2.0 Hz, 1H), 8.06-8.00 (m, 2H), 7.93 (d, *J* = 2.3 Hz, 1H), 7.79-7.73 (m, 1H), 7.73-7.60 (m, 2H), 7.34 (t, *J* = 7.0 Hz, 1H), 7.26 (s, 1H), 7.07 (t, *J* = 7.7 Hz, 1H), 5.50 (br d, *J* = 4.5 Hz, 1H), 4.15-4.05 (m, 1H), 4.02-3.94 (m, 1H), 3.90 (s, 3H), 3.91-3.85 (m, 1H), 2.95-2.80 (m, 1H), 2.67 (d, *J* = 4.8 Hz, 3H), 2.25 (d, *J* = 1.5 Hz, 3H), 1.21 (d, *J* = 7.0 Hz, 3H).

### Example 118: WX221, WX222

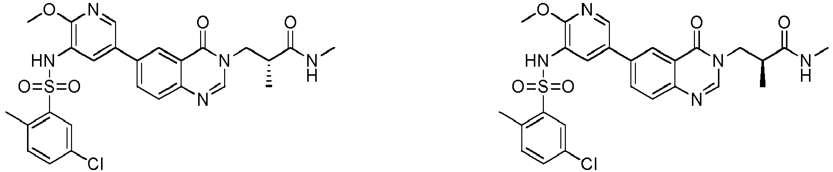

### Synthetic route:

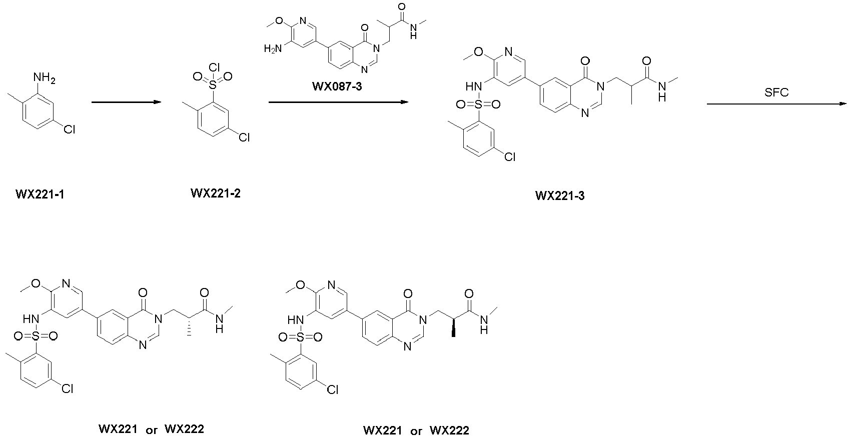

### Step 1: synthesis of compound WX221-2

WX221-1 (2 g, 14.12 mmol) was dissolved in a mixed solvent of concentrated hydrochloric acid (11.0 mL) and glacial acetic acid (3.0 mL), and a solution of sodium nitrite (1.06 g, 15.36 mmol) in water (1.8 mL) was added at 25 °C. The reaction solution was stirred at 0 °C for 1 hour, followed by addition of a solution of sulfur dioxide in glacial acetic acid (12.00 mL) and cuprous chloride (33.60 mg, 339.40 µmol). The reaction solution was stirred at 30 °C for 16 hours. The reaction solution was rotary-evaporated, followed by addition of ice water (500.0 mL), and washed three times with dichloromethane (100.0 mL). The organic phases were combined, dried over anhydrous sodium sulfate, and rotary-evaporated to obtain target compound **WX221-2** which was used directly in the next step.

### Step 2: synthesis of compound WX221-3

**WX087-3** (0.25 g, 680.46 µmol) was dissolved in pyridine (2.0 mL), and **WX221-2** (153.17 mg, 680.46 µmol) was added dropwise at 25 °C. The reaction solution was stirred at 28 °C for 16 hours to complete the reaction. The reaction solution was rotary-evaporated, followed by addition of water (10.00 mL), and washed three times with dichloromethane (10.00 mL). The organic phases were combined, dried over anhydrous sodium sulfate, and rotary-evaporated to obtain target compound **WX221-3.** MS-ESI m/z: 556.2[M+H]⁺, 558.2[M+H+2]⁺.

### Step 3: synthesis of compound WX221, WX222

**WX221-3** was resolved and purified by SFC (chromatographic column: AS (250mm * 30mm, 10µm); mobile phase: [0.1% NH₄HC0₃ EtOH]; B%: 40%-40%) to obtain a pair of enantiomers **WX221** (Rt = 3.891min) and **WX222** (Rt = 4.226 min). **WX221:** ¹H NMR (400MHz, 400 MHz, DMSO-*d*₆) δ: 10.28 (br s, 1H), 8.38 (d, *J* = 2.3 Hz, 1H), 8.25 (d, *J* = 2.3 Hz, 1H), 8.18 (s, 1H), 8.05 (dd, *J* = 2.3, 8.5 Hz, 1H), 7.95-7.83 (m, 2H), 7.75 (d, *J* = 8.3 Hz, 1H), 7.69 (d, *J* = 2.3 Hz, 1H), 7.59 (dd, *J* = 2.3, 8.3 Hz, 1H), 7.45 (d, *J* = 8.3 Hz, 1H), 4.15-4.02 (m, 1H), 4.14-3.87 (m, 1H), 3.69 (s, 3H), 3.32-3.28 (m, 3H), 2.85 (br dd, *J* = 6.4, 14.9 Hz, 1H), 2.59 (s, 3H), 1.07 (d, *J* = 7.0 Hz, 3H). **WX222:** ¹H NMR (400MHz, DMSO-*d*₆) δ: 10.33 (br s, 1H), 8.45 (d, *J* = 2.0 Hz, 1H), 8.33 (d, *J* = 2.3 Hz, 2H), 8.26 (s, 1H), 8.13 (dd, *J* = 2.0, 8.5 Hz, 2H), 8.05-7.92 (m, 1H), 7.83 (d, *J* = 8.5 Hz, 1H), 7.77 (d, *J* = 2.3 Hz, 1H), 7.67 (dd, *J* = 2.3, 8.3 Hz, 1H), 7.53 (d, *J* = 8.3 Hz, 1H), 4.21-4.07 (m, 2H), 3.76 (s, 3H), 3.58-3.44 (m, 3H), 2.93 (br dd, *J* = 6.7, 14.7 Hz, 1H), 2.66 (s, 3H), 1.15 (s, 3H).

### Example 119: WX223, WX224

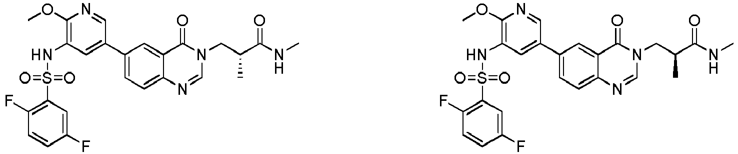

### Synthetic route:

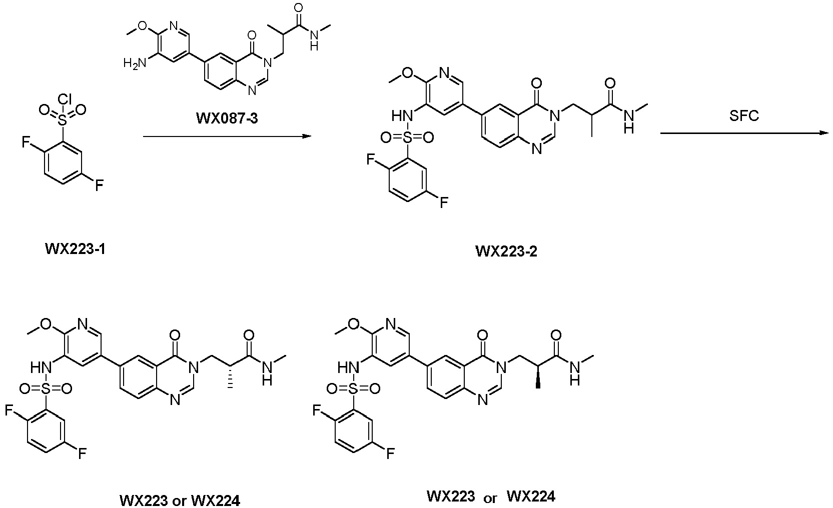

### Step 1: synthesis of compound WX223-2

**WX087-3** (0.15 g, 408.27 µmol) was dissolved in pyridine (2.0 mL), and **WX223-1** (86.80 mg, 408.27 µmol) was added dropwise at 25 °C. The reaction solution was stirred at 28 °C for 16 hours to complete the reaction. The reaction solution was rotary-evaporated, followed by addition of water (10 mL), and washed three times with dichloromethane (10 mL). The organic phases were combined, dried over anhydrous sodium sulfate, and rotary-evaporated to obtain target compound **WX223-2.** MS-ESI m/z: 544.1 [M+H]⁺, 546.1 [M+H+2]⁺.

### Step 2: synthesis of compound WX223, WX224

**WX223-2** was resolved and purified by SFC (chromatographic column: AS (250mm * 30mm, 10µm); mobile phase: [0.1% NH₄HCO₃ EtOH]; B%: 40% -40%) to obtain a pair of enantiomers **WX223** (Rt = 3.544 min) and **WX224** (Rt = 3.935 min). **WX223:** ¹H NMR (400MHz, CDCl₃) δ : 8.28 (s, 1H), 8.10 (d, *J* = 2.0 Hz, 1H), 8.05 (s, 1H), 7.97 (d, *J* = 2.3 Hz, 1H), 7.86-7.75 (m, 1H), 7.75-7.66 (m, 1H), 7.61-7.45 (m, 1H), 7.27 (br s, 1H), 7.19-7.06 (m, 2H), 5.61 (br s, 1H), 4.16-4.06 (m, 1H), 4.03-3.95 (m, 1H), 3.88 (s, 3H), 3.90-3.78 (m, 1H), 2.95-2.80 (m, 1H), 2.67 (d, *J* = 5.0 Hz, 3H), 1.22 (s, 3H). **WX224:** ¹H NMR (400MHz, CDCl₃) δ : 8.35-8.21 (m, 1H), 8.16-8.06 (m, 1H), 8.04 (s, 1H), 7.98 (d, *J* = 2.3 Hz, 1H), 7.85-7.75 (m, 1H), 7.73 (br d, *J* = 8.3 Hz, 1H), 7.56-7.47 (m, 1H), 7.19-7.03 (m, 2H), 5.49 (br s, 1H), 4.16-4.07 (m, 1H), 4.05-3.93 (m, 1H), 3.91-3.85 (m, 3H), 3.65 (q, *J* = 6.9 Hz, 2H), 2.94-2.82 (m, 1H), 2.67 (d, *J* = 4.8 Hz, 3H), 1.22 (s, 3H).

### Example 120: WX225, WX226

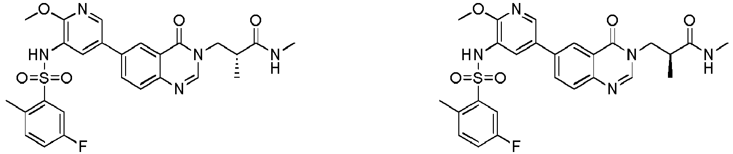

### Synthetic route:

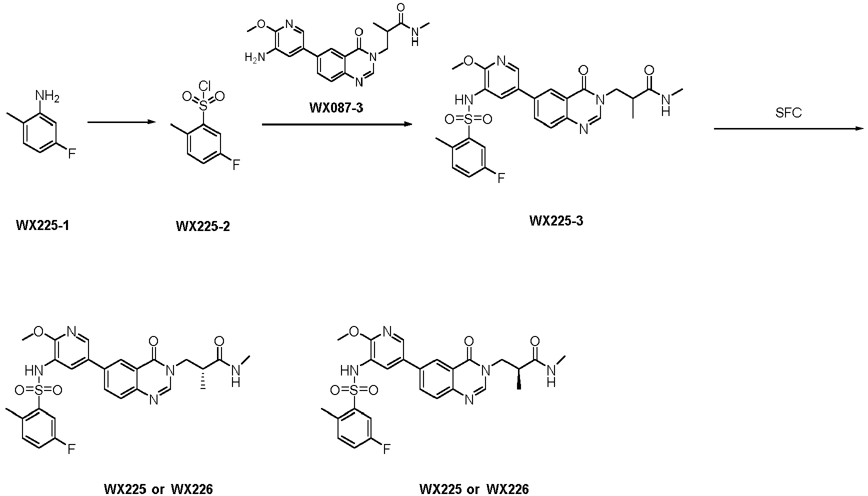

### Step 1: synthesis of compound WX225-2

**WX225-1** (1.0 g, 7.99 mmol) was dissolved in a mixed solvent of concentrated hydrochloric acid (5.5 mL) and glacial acetic acid (1.5 mL), and a solution of sodium nitrite (599.73 mg, 8.69 mmol) in water (1.8 mL) was added at 25 °C. The reaction solution was stirred at 0 °C for 1 hour, followed by addition of a solution of sulfur dioxide in glacial acetic acid (12 mL) and cuprous chloride (16.80 mg, 169.70 µmol), and the reaction solution was stirred at 30 °C for 16 hours. The reaction solution was rotary-evaporated, followed by addition of ice water (500 mL), and washed three times with dichloromethane (100 mL). The organic phases were combined, dried over anhydrous sodium sulfate, and rotary-evaporated to obtain target compound **WX225-2** which was used directly in the next step.

### Step 2: synthesis of compound WX225-3

**WX087-3** (0.15 g, 408.27 µmol) was dissolved in pyridine (2.0 mL), and **WX225-2** (85.18 mg, 408.27 µmol) was added dropwise at 25 °C. The reaction solution was stirred at 28 °C for 16 hours to complete the reaction. The reaction solution was rotary-evaporated, followed by addition of water (10 mL), and washed three times with dichloromethane (10 mL). The organic phases were combined, dried over anhydrous sodium sulfate, and rotary-evaporated to obtain target compound **WX225-3.** MS-ESI m/z: 540.1[M+H]⁺, 542.1[M+H+2]⁺.

### Step 3: synthesis of compound WX225, WX226

**WX225-3** was resolved and purified by SFC (chromatographic column: Chiralpak AS-H 250 * 30mm 5µm; mobile phase: [0.1% NH₄HCO₃ EtOH]; B%: 35% -35%) to obtain a pair of enantiomers **WX225** (Rt = 4.494 min) and **WX226** (Rt = 4.868 min). **WX225:** ¹H NMR (400MHz, CDCl₃) δ : 8.27 (d, *J* = 1.8 Hz, 1H), 8.16-7.99 (m, 2H), 7.88 (d, *J* = 2.3 Hz, 1H), 7.81-7.67 (m, 2H), 7.62 (dd, *J* = 2.8, 8.3 Hz, 1H), 7.23-7.20 (m, 1H), 7.12-6.94 (m, 2H), 5.55 (br d, *J* = 4.5 Hz, 1H), 4.20-4.06 (m, 1H), 3.97 (dd, *J* = 9.3, 13.3 Hz, 1H), 3.90 (s, 3H), 2.95-2.81 (m, 1H), 2.67 (d, *J* = 4.8 Hz, 3H), 2.60 (s, 3H), 1.21 (d, *J* = 7.0 Hz, 3H). **WX226:** ¹H NMR (400MHz, CDCl₃) δ : 8.27 (d, *J* = 1.8 Hz, 1H), 8.11-8.00 (m, 2H), 7.88 (d, *J* = 2.3 Hz, 1H), 7.80-7.66 (m, 2H), 7.62 (dd, *J* = 2.6, 8.4 Hz, 1H), 7.24-7.20 (m, 1H), 7.24-7.20 (m, 1H), 7.08 (dt, *J* = 2.6, 8.1 Hz, 1H), 5.57 (br d, *J* = 4.3 Hz, 1H), 4.17-4.04 (m, 1H), 4.03-3.91 (m, 1H), 3.93-3.91 (m, 1H), 3.90 (s, 2H), 3.65 (q, *J* = 7.0 Hz, 2H), 2.98-2.79 (m, 1H), 2.67 (d, *J* = 4.8 Hz, 3H), 2.60 (s, 3H), 1.21 (d, *J* = 7.0 Hz, 3H).

### Example 121: WX227, WX228

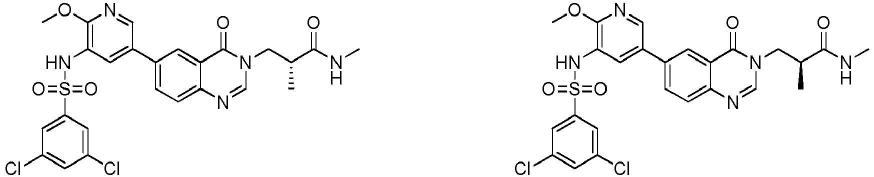

### Synthetic route:

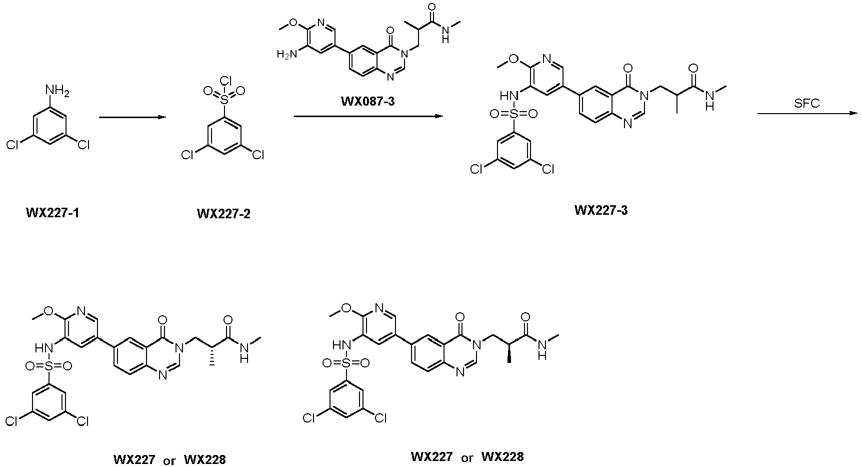

### Step 1: synthesis of compound WX227-2

**WX227-1** (1.00 g, 6.17 mmol) was dissolved in a mixed solvent of concentrated hydrochloric acid (5.5 mL) and glacial acetic acid (1.5 mL), and a solution of sodium nitrite (463.24 mg, 6.71 mmol) in water (1.8 mL) was added at 25 °C. The reaction solution was stirred at 0 °C for 1 hour, followed by addition of a solution of sulfur dioxide in glacial acetic acid (12.00 mL) and cuprous chloride (14.68 mg, 148.31 µmol), and the reaction solution was stirred at 30 °C for 16 hours. The reaction solution was rotary-evaporated, followed by addition of ice water (500.0 mL), and washed three times with dichloromethane (100.0 mL). The organic phases were combined, dried over anhydrous sodium sulfate, and rotary-evaporated to obtain target compound **WX227-2,** which was used directly in the next step.

### Step 2: synthesis of compound WX227-3

**WX087-3** (0.15 g, 408.27 µmol) was dissolved in pyridine (2.0 mL), and **WX227-2** (105.25 mg, 428.68 µmol) was added dropwise at 25 °C. The reaction solution was stirred at 28 °C for 16 hours to complete the reaction. The reaction solution was rotary-evaporated, followed by addition of water (10 mL), and washed three times with dichloromethane (10 mL). The organic phases were combined, dried over anhydrous sodium sulfate, and rotary-evaporated to obtain the target compound **WX227-3.** MS-ESI m/z: 576.0[M+H]⁺, 578.0[M+H+2]⁺, 580.0[M+H+4]⁺.

### Step 3: synthesis of compound WX227, WX228

**WX227-3** was resolved and purified by SFC (chromatographic column: Chiralpak AS-H 250 * 30mm 5µm; mobile phase: [0.1% NH₄HCO₃ EtOH]; B%: 35% -35%) to obtain a pair of enantiomers **WX227** (Rt = 4.446 min) and **WX228** (Rt = 5.228 min). **WX227**:¹H NMR (400MHz, CDCl₃) δ : 8.27 8.33 (s, 1H), 8.15 (br s, 1H), 8.05 (s, 1H), 7.98 (br s, 1H), 7.86-7.77 (m, 1H), 7.76-7.69 (m, 1H), 7.62 (d, *J* = 1.3 Hz, 2H), 7.46 (br s, 1H), 5.52 (br s, 1H), 4.15-4.05 (m, 1H), 4.05-3.92 (m, 1H), 3.85 (s, 3H), 2.94-2.80 (m, 1H), 2.67 (d, *J* = 5.0 Hz, 3H), 1.22 (d, *J* = 6.8 Hz, 3H). **WX228:** ¹H NMR (400MHz, CDCl₃) δ : 8.27 8.34 (d, *J* = 2.0 Hz, 1H), 8.16 (d, *J* = 2.3 Hz, 1H), 8.05 (s, 1H), 7.99 (d, *J* = 2.3 Hz, 1H), 7.86-7.77 (m, 1H), 7.77-7.70 (m, 1H), 7.62 (d, *J* = 1.8 Hz, 2H), 7.54-7.40 (m, 1H), 5.50 (br d, *J* = 4.5 Hz, 1H), 4.16-4.07 (m, 1H), 3.98 (dd, *J* = 9.3, 13.3 Hz, 1H), 3.85 (s, 3H), 2.96-2.82 (m, 1H), 2.67 (d, *J* = 4.8 Hz, 3H), 1.22 (d, *J* = 7.0 Hz, 3H).

### Example 122: WX229, WX230

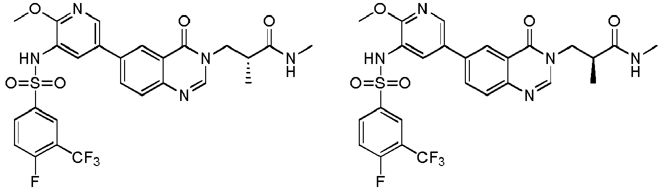

### Synthetic route:

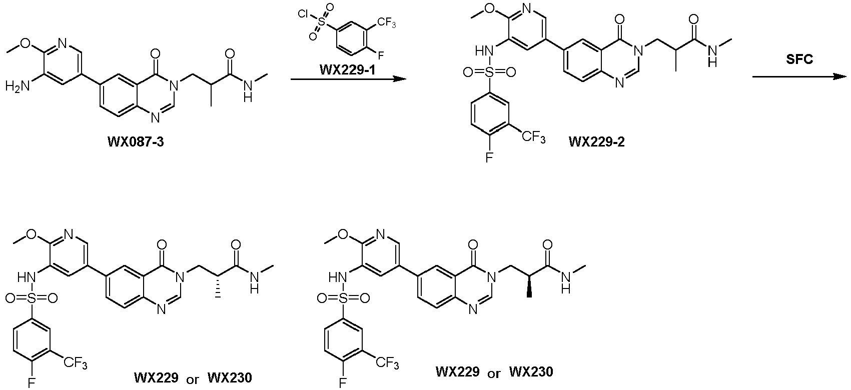

### Step 1: synthesis of compound WX229-2

Compound **WX087-3** (0.15 g, 408.27 µmol) was dissolved in pyridine (3 mL). Compound **WX229-1** (128.66 mg, 489.93 µmol) was added thereto, and the reaction solution was stirred at 25 °C for 10 hours. TLC (ethyl acetate: methanol = 10:1) showed that the reaction of the raw materials was complete. The reaction solution was rotary-evaporated and separated by preparative TLC plate (ethyl acetate: methanol = 10:1) to obtain the target compound **WX229-2.**

### Step 2: synthesis of compound WX229 and WX230

Compound **WX229-2** was resolved by supercritical fluid chromatography (resolution conditions: chromatographic column: AD (250mm * 30mm, 5µm); mobile phase: [0.1% NH₄HCO₃ EtOH]; B%: 55%-55%) to obtain a pair of enantiomers **WX229** (retention time 0.450 min) and **WX230** (retention time 0.908 min), the ratio is 1:1. **WX229:** ¹HNMR (400 MHz, DMSO-*d*₆) δ = 8.27 (br s, 2 H), 8.18 (s, 1 H), 8.08-8.15 (m, 1 H), 8.06 (br d, *J* = 8.3 Hz, 2 H), 7.86-7.93 (m, 2 H), 7.75 (d, *J* = 8.5 Hz, 1 H), 7.68 (t, *J* = 9.6 Hz, 1 H), 4.07 (br dd, *J* = 13.4, 5.6 Hz, 1 H), 3.91-4.01 (m, 1 H), 3.65 (s, 3 H), 2.78-2.92 (m, 1 H), 2.49-2.49 (m, 3 H), 1.08 (br d, *J* = 7.0 Hz, 3 H). MS-ESI *m*/*z:* 594.1 [M+H]⁺. **WX230:** ¹H NMR (400 MHz, CHLOROFORM-*d*) δ ppm 8.36 (s, 1 H), 8.28 (d, *J* = 1.8 Hz, 1 H), 8.19 (s, 1 H), 8.04-8.15 (m, 3 H), 7.86-7.96 (m, 2 H), 7.76 (d, *J* = 7.8 Hz, 1 H), 7.70 (t, *J* = 9.5 Hz, 1 H), 4.04-4.12 (m, 1 H), 3.97 (br dd, *J* = 13.2, 9.2 Hz, 1 H), 3.64 (s, 3 H), 2.82-2.91 (m, 1 H), 2.48-2.48 (m, 3 H), 1.08 (d, *J* = 6.8 Hz, 3 H). MS-ESI *m*/*z:* 594.1 [M+H]⁺.

### Example 123: WX231, WX232

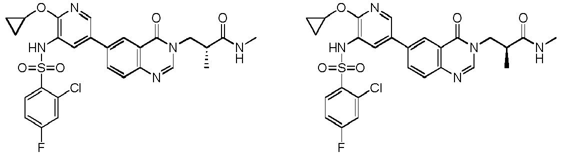

### Synthetic route:

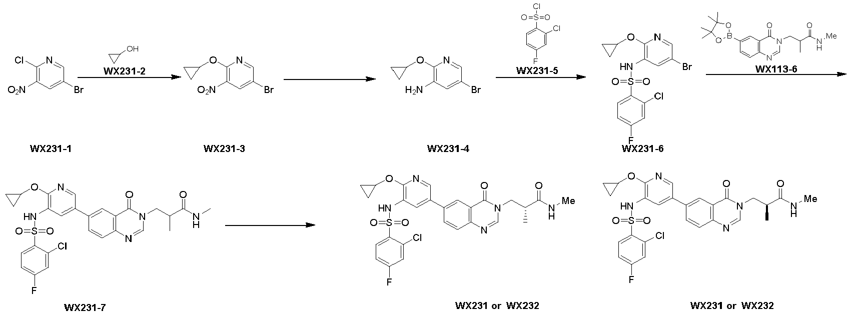

### Step 1: synthesis of compound WX231-3

Raw materials **WX231-1** (1.6 g, 6.74 mmol) and **WX231-2** (4 g, 68.87 mmol) and the solvent tetrahydrofuran (16 mL) were added ino a pre-dried stock bottle, and then 1,8-diazabicycloundec-7-ene (4.62 g, 30.32 mmol, 4.57 mL) was added thereto, and further stirred at 50 °C for 5 hours. After the reaction was completed, water (10 mL) and dichloromethane (10 mL × 3) were added to the reaction solution. The organic phase obtained was dried over anhydrous sodium sulfate, rotary-evaporated under reduced pressure by a water pump, separated and purified by column chromatography (petroleum ether: ethyl acetate = 1:0 to 30:1) to obtain target compound **WX231-3.** ¹H NMR (400MHz, CHLOROFORM-d) δ = 8.50 (d, *J* = 2.2 Hz, 1H), 8.36 (d, *J* = 2.4 Hz, 1H), 5.31 (s, 1H), 4.50-4.44 (m, 1H), 0.92-0.85 (m, 6H).

### Step 2: synthesis of compound WX231-4

Raw material **WX231-3** (1.3 g, 5.02 mmol) and solvent glacial acetic acid (15 mL) were added into a pre-dried stock bottle, and then iron powder (2.80 g, 50.18 mmol) was slowly added thereto, and further stirred at 25 °C for 2 hours. After the reaction was completed, ethanol (30 mL) was added to the reaction solution, followed by filtration. Water (10 mL) and dichloromethane (20 mL × 3) were added to the filtrate. The organic phase was dried over anhydrous sodium sulfate, rotary-evaporated under reduced pressure by a water pump, separated and purified by column chromatography (petroleum ether: ethyl acetate = 1: 0 to 15: 1) to obtain target compound **WX231-4.** ¹H NMR (400MHz, CHLOROFORM-d) δ = 7.65 (d, *J* = 2.2 Hz, 1H), 6.99 (d, *J* = 2.2 Hz, 1H), 4.32-4.23 (m, 1H), 3.84-3.74 (m, 1H), 3.78 (br s, 1H), 0.84-0.80 (m, 2H), 0.78-0.75 (m, 2H).

### Step 3: synthesis of compound WX231-6

Raw materials **WX231-4** (940 mg, 4.10 mmol) and **WX231-5** (1.03 g, 4.51 mmol, 658.55 µL) and the solvent pyridine (10 mL) were added to a pre-dried stock bottle, and further stirred at 25 °C for 12 hours. After the reaction was completed, water (10 mL) and dichloromethane (10 mL × 3) were added to the reaction solution for extraction. The resulting organic phase was dried over anhydrous sodium sulfate, rotary-evaporated under reduced pressure by a water pump, separated and purified by column chromatography (petroleum ether : ethyl acetate = 1:1 to 5:1) to obtain target compound **WX231-6.** ¹H NMR (400MHz, CHLOROFORM-d) δ = 8.06 (dd, *J* = 5.9, 9.0 Hz, 1H), 7.94 (d, *J* = 2.2 Hz, 1H), 7.84 (d, *J* = 2.2 Hz, 1H), 7.65 (d, *J* = 2.2 Hz, 1H), 7.65-7.64 (m, 1H), 7.32 (s, 1H), 7.28 (s, 1H), 7.26 (d, *J* = 2.2 Hz, 1H), 7.11 (ddd, *J* = 2.6, 7.5, 8.8 Hz, 1H), 6.99 (d, *J* = 2.2 Hz, 1H), 4.31-4.20 (m, 1H), 1.57 (s, 5H), 0.01-0.01 (m, 1H).

### Step 4: synthesis of compound WX231-7

Raw material **WX231-6** (640 mg, 1.52 mmol), raw material **WX113-6** (613.21 mg, 1.67 mmol), solvent 1,4-dioxane (10 mL) and water (1 mL) were added to a pre-dried stock bottle, then potassium acetate (297.92 mg, 3.04 mmol) was added, replaced with nitrogen, then 1,1-bis(diphenylphosphine) ferrocene palladium chloride (111.06 mg, 151.78 µmol) was added, replaced with nitrogen, and stirred continually at 70 °C for 10 hours. After the reaction was completed, water (2 mL) dichloromethane (5 mL × 3) was added to the reaction solution, and the resulting organic phase was dried over anhydrous sodium sulfate, rotary-evaporated under reduced pressure by a water pump and rotary-evaporated under reduced pressure, and separated and purified by preparative thin layer chromatography (dichloromethane: methanol = 20: 1), and then separated by preparative HPLC to obtain target compound **WX231-7.**

### Step 5: synthesis of compound WX231, WX232

**WX231-7** was resolved and purified by SFC: (resolution method: chromatography column: AD (250mm * 30mm, 5µm); mobile phase: [IPA]; B%: 40%-40%, 5.5min) to obtain a pair of enantiomers **WX231** (retention time is 2.868min) and **WX232** (retention time is 2.843min). **WX231:** ¹H NMR (400MHz, METHANOL-d4) δ = 8.36 (br s, 1H), 8.27 (d, J = 2.0 Hz, 1H), 8.19 (s, 1H), 8.06-7.99 (m, 3H), 7.77 (d, *J* = 8.6 Hz, 1H), 7.79-7.74 (m, 1H), 7.48 (dd, *J* = 2.4, 8.6 Hz, 1H), 7.21 (dt, *J* = 2.4, 8.4 Hz, 1H), 4.58 (br s, 1H), 4.29-4.17 (m, 2H), 4.03 (dd, *J* = 9.8, 13.4 Hz, 1H), 3.06-2.90 (m, 1H), 2.64-2.60 (m, 2H), 2.64-2.60 (m, 1H), 1.24 (d, *J=* 7.0 Hz, 3H), 0.75-0.69 (m, 2H), 0.61-0.54 (m, 2H), **WX232:** 1H NMR (400MHz, METHANOL-d4) δ = 8.36 (br s, 1H), 8.27 (d, *J* = 2.0 Hz, 1H), 8.19 (s, 1H), 8.06-7.99 (m, 3H), 7.77 (d, *J* = 8.6 Hz, 1H), 7.79-7.74 (m, 1H), 7.48 (dd, *J* = 2.4, 8.6 Hz, 1H), 7.21 (dt, *J* = 2.4, 8.4 Hz, 1H), 4.58 (br s, 1H), 4.29-4.17 (m, 2H), 4.03 (dd, *J* = 9.8, 13.4 Hz, 1H), 3.06-2.90 (m, 1H), 2.64-2.60 (m, 2H), 2.64-2.60 (m, 1H), 1.24 (d, *J* = 7.0 Hz, 3H), 0.75-0.69 (m, 2H), 0.61-0.54 (m, 2H).

### Example 124: WX233, WX234, WX235, WX236

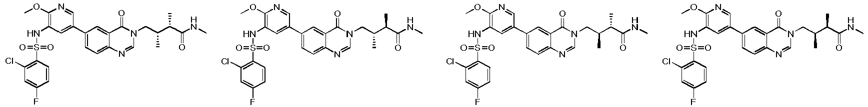

### Synthetic route:

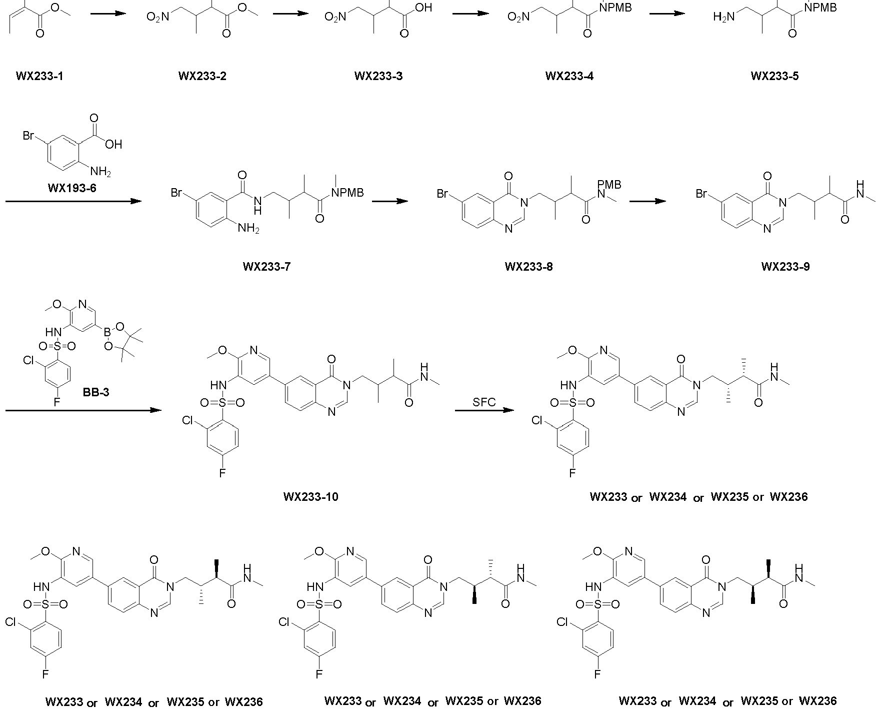

### Step 1: synthesis of compound WX233-2

Compound **WX233-1** (5 g, 43.81 mmol) and tetrahydrofuran (50 mL) were sequentially added into a pre-dried single-necked flask (250 mL), then nitromethane (8.02 g, 131.42 mmol, 7.10 mL) was added, and finally tetrabutylammonium fluoride trihydrate (27.64 g, 87.61 mmol) was added. The mixture was replaced with nitrogen, and stirred at 20 °C for 10 hours. After the reaction was completed, the reaction solution was evaporated under reduced pressure to remove the solvent, separated and purified by flash column chromatography (petroleum ether: ethyl acetate as mobile phase = 20: 1) to obtain compound **WX233-2.** ¹H NMR (400MHz, CHLOROFORM-d) δ = 4.59-4.41 (m, 1H), 4.35-4.25 (m, 1H), 3.73-3.67 (m, 3H), 2.76-2.51 (m, 2H), 1.21 (dd, *J=* 5.3, 7.1 Hz, 3H), 1.04 (dd, *J=* 1.3, 6.8 Hz, 3H).

### Step 2: synthesis of compound WX233-3

Compound **WX233-2** (5.1 g, 29.11 mmol), tetrahydrofuran (50 mL) and water (20 mL) were sequentially added into a pre-dried single-necked flask (100 mL), and lithium hydroxide monohydrate (2.44 g, 58.23 mmol) was added thereto finally. The mixture was replaced with nitrogen and stirred at 20 °C for 2 hours. After the reaction was completed, the reaction solution was extracted with water (30 mL) and ethyl acetate (50 mL). The organic phase was discarded, and the aqueous phase was adjusted to pH = 3 with IN hydrochloric acid, and then extracted with ethyl acetate (50 mL × 3). The organic phases were combined, washed with saturated sodium chloride (20 mL), dried over anhydrous sodium sulfate, and filtered. The filtrate was finally dried under reduced pressure to obtain target compound **WX233-3** which was used directly in the next step. ¹H NMR (400MHz, CHLOROFORM-d) δ = 4.65-4.44 (m, 1H), 4.41-4.30 (m, 1H), 2.82-2.52 (m, 2H), 1.27-1.23 (m, 3H), 1.13-1.06 (m, 3H).

### Step 3: synthesis of compound WX233-4

Compound WX233-3 (3 g, 18.62 mmol), dichloromethane (5 mL), triethylamine (2.83 g, 27.92 mmol, 3.89 mL) and *O*-(7-azabenzotriazole-1-yl)-*N,N,N,N-*tetramethylurea hexafluorophosphate (8.49 g, 22.34 mmol) were sequentially added into a pre-dried stock bottle (40mL), and 4-methoxy-*N*-methylbenzylamine (3.38 g, 22.34 mmol) was added thereto finally. The mixture was replaced with nitrogen, and stirred at 25 °C for 10 hours. After the reaction was completed, the reaction solution was evaporated under reduced pressure to remove the solvent to obtain a crude product. The crude product was separated and purified by flash column chromatography (petroleum ether: ethyl acetate as mobile phase = 5: 1 to 1: 1) to obtain compound **WX233-4.** ¹H NMR (400MHz, CHLOROFORM-d) δ = 7.21-7.05 (m, 2H), 6.96-6.82 (m, 2H), 4.71-4.27 (m, 4H), 3.84-3.77 (m, 3H), 3.02-2.92 (m, 3H), 2.88-2.53 (m, 2H), 1.23-1.13 (m, 3H), 1.13-1.00 (m, 3H).

### Step 4: synthesis of compound WX233-5

Platinum dioxide (308.59 mg, 1.36 mmol) was added to a dry hydrogenation flask (75 mL) pre-displaced with argon, then ethanol (40 mL) and compound **WX233-4** (4 g, 13.59 mmol) were added. The mixture was replaced with hydrogen and stirred at 20 °C for 10 hours under 50 psi hydrogen atmosphere. After the reaction was completed, the reaction solution was cooled down and filtered, and the filter cake was washed with methanol (100 mL × 2). The filtrates were combined and then dried under reduced pressure to obtain compound **WX233-5.** ¹H NMR (400MHz, METHANOL-d4) δ = 7.22-7.12 (m, 2H), 6.96-6.84 (m, 2H), 4.73-4.44 (m, 2H), 3.80-3.76 (m, 3H), 3.35 (s, 1H), 3.03-2.98 (m, 2H), 2.95-2.92 (m, 1H), 2.83-2.82 (m, 3H), 2.80-2.75 (m, 1H), 2.59-2.42 (m, 1H), 1.90-1.75 (m, 1H), 1.14-1.06 (m, 3H), 0.98-0.88 (m, 3H).

### Step 5: synthesis of compound WX233-7

Compound **WX233-6** (2.21 g, 10.21 mmol), compound **WX233-5** (2.7 g, 10.21 mmol), dichloromethane (50 mL) and *N,N*-diisopropylethylamine (3.96 g, 30.64 mmol, 5.34 mL) were sequentially added into a pre-dried stock bottle (8mL), and a 50% solution of propylphosphonic anhydride in ethyl acetate (7.80 g, 12.26 mmol, 7.29 mL, 50% purity) was added thereto finally. The mixture was replaced with nitrogen, and stirred at 20 °C for 10 hours. After the reaction was completed, the reaction solution was evaporated under reduced pressure to remove the solvent to obtain a crude product. The crude product was separated and purified by flash column chromatography (petroleum ether: ethyl acetate as mobile phase = 5:1 to 1:1) to obtain compound **WX233-7.** ¹H NMR (400MHz, CHLOROFORM-d) δ = 8.12-7.84 (m, 1H), 7.69-7.61 (m, 1H), 7.24 (dt, *J =* 2.8, 5.7 Hz, 1H), 7.20-7.03 (m, 2H), 6.93-6.79 (m, 2H), 6.56 (dd, *J* = 1.6, 8.7 Hz, 1H), 4.68-4.42 (m, 2H), 3.85-3.71 (m, 3H), 3.64-3.50 (m, 1H), 3.26-3.08 (m, 1H), 3.03-2.95 (m, 3H), 2.81-2.74 (m, 1H), 1.22-1.13 (m, 3H), 1.10-0.93 (m, 3H).

### Step 6: synthesis of compound WX233-8

Compound **WX233-7** (2.5 g, 5.41 mmol), formamidine acetate (3.38 g, 32.44 mmol, 6 eq) and ethanol (30 mL) were sequentially added into a pre-dried single-neck flask (100 mL). The mixture was replaced with nitrogen, and stirred at 80 °C for 10 hours. After the reaction was completed, the reaction solution was dried under reduced pressure to obtain a crude product. The crude product was separated and purified by flash column chromatography (petroleum ether: ethyl acetate as mobile phase = 5: 1 to 1: 3) to obtain compound **WX233-8.** ¹H NMR (400MHz, CHLOROFORM-d) δ = 8.46-8.41 (m, 1H), 8.07-8.00 (m, 1H), 7.91-7.80 (m, 1H), 7.66-7.57 (m, 1H), 7.22-7.00 (m, 2H), 6.91-6.80 (m, 2H), 4.61-4.40 (m, 2H), 4.23-3.87 (m, 2H), 3.80 (s, 3H), 3.00-2.87 (m, 3H), 2.83-2.77 (m, 1H), 2.47-2.30 (m, 1H), 1.32-1.21 (m, 3H), 1.03-0.89 (m, 3H).

### Step 7: synthesis of compound WX233-9

Compound **WX233-8** (1.3 g, 2.75 mmol), dichloromethane (13 mL) and TFA (6.5 mL) were added into a pre-dried stock bottle (40mL), followed by addition of trifluoromethanesulfonic anhydride (776.46 mg, 2.75 mmol, 454.07 µL). The mixture was replaced with nitrogen, and stirred at 20 °C for 10 hours. After the reaction was completed, the reaction solution was slowly quenched with saturated sodium bicarbonate (~ 50 mL) at 0 °C to pH = 9, and then extracted with dichloromethane (100 mL × 3). The organic phases were combined and washed with saturated sodium chloride, dried over anhydrous sodium sulfate, filtered, and finally dried under reduced pressure to obtain a crude product. The crude product was separated and purified by flash column chromatography (petroleum ether: ethyl acetate as mobile phase = 5:1 to 1:2) to obtain compound **WX193-9.** ¹H NMR (400MHz, CHLOROFORM-d) δ = 8.45 (d, *J* = 2.3 Hz, 1H), 8.15 (s, 1H), 7.90-7.82 (m, 1H), 7.62 (d, *J* = 8.7 Hz, 1H), 6.50 (br s, 1H), 4.34 (dd, J = 8.0, 13.7 Hz, 1H), 3.73 (dd, *J* = 6.7, 13.7 Hz, 1H), 2.85 (d, *J* = 4.6 Hz, 3H), 2.37-2.26 (m, 1H), 2.25-2.14 (m, 1H), 1.18 (d, *J* = 7.0 Hz, 3H), 1.01 (d, *J* = 6.9 Hz, 3H).

### Step 8: synthesis of compound WX233-10

Compound **WX233-9** (0.5 g, 1.42 mmol), compound **BB-3** (628.43 mg, 1.42 mmol), potassium acetate (417.94 mg, 4.26 mmol) and the solvents 1,4-dioxane (5 mL) and water (0.5 mL) were added to a pre-dried vial (10mL). The mixture was replaced with nitrogen, followed by addition of 1,1-bis(diphenylphosphine) ferrocene palladium chloride (103.87 mg, 141.95 µmol). The mixture was replaced with nitrogen again, heated to 100 °C and stirred for 5 hours. After the reaction was completed, the reaction solution was cooled down and filtered. The filtrate was evaporated under reduced pressure to remove the solvent to obtain a crude product. The crude product was separated and purified by flash column chromatography (petroleum ether: ethyl acetate as mobile phase = 5: 1 to 0: 1) to obtain compound **WX233-10.** ¹H NMR (400MHz, METHANOL-d4) δ = 8.36-8.31 (m, 2H), 8.27-8.22 (m, 1H), 8.10 (dd, *J* = 5.8, 8.9 Hz, 1H), 8.05-7.99 (m, 2H), 7.79 (d, *J* = 8.4 Hz, 1H), 7.47 (dd, *J* = 2.6, 8.5 Hz, 1H), 7.23 (dt, *J* = 2.6, 8.4 Hz, 1H), 4.29 (dd, *J* = 4.5, 13.5 Hz, 1H), 3.89-3.80 (m, 4H), 2.78-2.69 (m, 3H), 2.38-2.26 (m, 2H), 1.26-1.22 (m, 3H), 0.93 (d, *J* = 6.4 Hz, 3H).

### Step 9: synthesis of compound WX233, WX234, WX235, WX236

Compound **WX233-10** (0.8 g, 1.36 mmol) was resolved by SFC (instrument: Thar SFC80 preparative SFC; resolution column: Chiralpak AD-H 250 * 30mm id 5u; mobile phase: A for CO₂ and B for EtOH; Gradient: B% = 45%; Flow rate: 80 g/min; wavelength: 220 nm; column temperature 40 °C; system back pressure: 100 bar; time: 10 min) to obtain **WX233** (RT = 1.49min), **WX234** (RT = 2.00min), **WX235** (RT = 2.83min) and **WX236** (RT = 3.43min). **WX233:** ¹H NMR (400MHz, METHANOL-d4) δ = 8.34 (d, *J* = 2.2 Hz, 1H), 8.27 (s, 1H), 8.23 (d, *J* = 2.4 Hz, 1H), 8.11 (dd, *J* = 5.8, 8.9 Hz, 1H), 8.04-7.99 (m, 2H), 7.78 (d, *J* = 8.4 Hz, 1H), 7.47 (dd, *J* = 2.5, 8.5 Hz, 1H), 7.23 (dt, *J* = 2.5, 8.4 Hz, 1H), 4.10-3.93 (m, 2H), 3.87 (s, 3H), 2.59 (s, 3H), 2.40 (td, *J* = 7.3, 14.1 Hz, 1H), 2.33-2.25 (m, 1H), 1.19 (d, *J* = 7.1 Hz, 3H), 0.98 (d, *J* = 6.8 Hz, 3H). **WX234:** ¹H NMR (400MHz, METHANOL-d4) δ = 8.34 (d, *J* = 2.2 Hz, 1H), 8.27 (s, 1H), 8.24 (d, *J* = 2.4 Hz, 1H), 8.10 (dd, *J* = 6.0, 8.8 Hz, 1H), 8.04-7.99 (m, 2H), 7.91 (br s, 1H), 7.78 (d, *J* = 8.4 Hz, 1H), 7.48 (dd, *J* = 2.6, 8.6 Hz, 1H), 7.24 (dt, *J* = 2.6, 8.4 Hz, 1H), 4.09-3.93 (m, 2H), 3.87 (s, 3H), 2.59 (d, *J* = 4.6 Hz, 3H), 2.40 (td, *J* = 7.2, 14.2 Hz, 1H), 2.30 (quin, *J* = 7.1 Hz, 1H), 1.19 (d, *J* = 6.8 Hz, 3H), 0.98 (d, *J* = 6.8 Hz, 3H). **WX235:** ¹H NMR (400MHz, METHANOL-d4) δ = 8.37-8.30 (m, 2H), 8.24 (d, *J* = 2.0 Hz, 1H), 8.14-8.07 (m, 1H), 8.06-7.97 (m, 2H), 7.79 (d, *J* = 8.4 Hz, 1H), 7.48 (dd, *J* = 2.2, 8.8 Hz, 1H), 7.23 (t, *J* = 8.7 Hz, 1H), 4.30 (br dd, *J* = 4.6, 13.2 Hz, 1H), 3.90-3.79 (m, 4H), 2.72 (s, 3H), 2.36-2.27 (m, 2H), 1.25 (br d, *J* = 6.4 Hz, 3H), 0.93 (br d, *J* = 6.4 Hz, 3H). **WX236:** ¹H NMR (400MHz, METHANOL-d4) δ = 8.34 (s, 1H), 8.33 (s, 1H), 8.24 (d, *J* = 2.2 Hz, 1H), 8.10 (dd, *J* = 5.8, 8.9 Hz, 1H), 8.06-7.96 (m, 2H), 7.79 (d, *J* = 8.4 Hz, 1H), 7.48 (dd, *J* = 2.6, 8.6 Hz, 1H), 7.23 (ddd, *J* = 2.4, 7.9, 8.9 Hz, 1H), 4.60 (s, 1H), 4.30 (dd, *J* = 4.6, 13.2 Hz, 1H), 3.89-3.81 (m, 4H), 2.75-2.70 (m, 3H), 2.36-2.26 (m, 2H), 1.25 (d, *J* = 6.4 Hz, 3H), 0.96-0.88 (m, 3H).

### Example 125: WX237, WX238

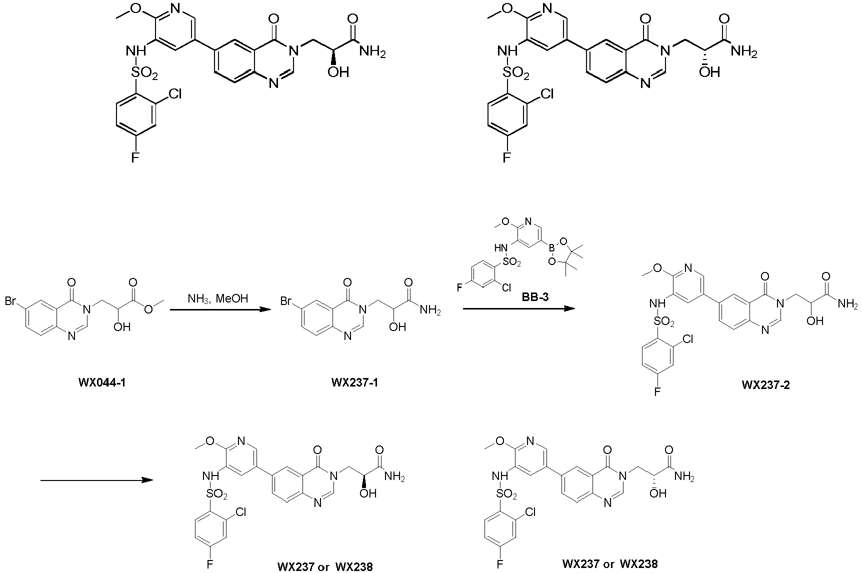

### Step 1: synthesis of compound WX237-1

In a 40 mL vial, a solution of NH₃ (7 M, 2.62 mL) in methanol (7 M, 1.15 mL) was added to a solution of **WX044-1** (300 mg, 917.07 µmol) in MeOH (3 mL). After the addition was completed, the reaction solution was stirred at 25 °C for 12 hours under nitrogen atmosphere. After the reaction was completed, the reaction solution was directly concentrated, and washed once with methyl tert-butyl ether (5 mL) to obtain target compound **WX237-1,** which was directly used in the next reaction. **MS,** m/z = 314.1 [M+1]⁺.

### Step 2: synthesis of compound WX237-2

In a 40 mL vial, NaHCO₃ (94.88 mg, 1.13 mmol, 43.93 uL) and Pd(dppf)Cl₂ (41.32 mg, 56.47 µmol, 0.1 eq) were added to a solution of **WX237-1** (250 mg, 564.72 µmol) and **BB-3** (176.26 mg, 564.72 µmol) in dioxane and water (dioxane:water=10:1, 3 mL). After the addition was completed, the reaction solution was stirred at 100 °C for 5 hours under nitrogen atmosphere. After the reaction was completed, the reaction solution was cooled to room temperature, followed by addition of H₂O (5 mL) to quench the reaction, and then extracted with dichloromethane (10 mL × 3). The organic phases were combined, dried over anhydrous sodium sulfate, concentrated, then isolated and purified by preparative thin layer chromatography (DCM: MeOH = 15:1) to obtain target compound **WX237-2.**

### Step 3: synthesis of compound WX237 and WX238

**WX237-2** was resolved by *prep*-SFC (resolution column: AS (250mm * 30mm, 5µm); mobile phase: [MeOH]; B%: 40% -40%) to obtain a pair of enantiomers, which were then purified by *prep*-HPLC (resolution column: Luna C18 100 * 30 5µm; mobile phase: [H₂O (0.1% TFA) -ACN]; B%: 25% -55%, 10min) to obtain **WX237** (Rt = 2.55 min) and **WX238** (Rt = 2.56 min). **WX237:** ¹H NMR (400MHz, CHLOROFORM-d) δ = 8.40-8.20 (m, 3H), 8.10 (dd, *J* = 6.0, 8.9 Hz, 1H), 8.04-7.96 (m, 2H), 7.78 (d, *J* = 8.4 Hz, 1H), 7.50 (dd, *J* = 2.5, 8.5 Hz, 1H), 7.30-7.17 (m, 1H), 4.69 (dd, *J* = 3.7, 13.7 Hz, 1H), 4.46 (dd, *J* = 3.7, 8.6 Hz, 1H), 4.00 (dd, *J* = 8.7, 13.8 Hz, 1H), 3.90 (s, 3H); **MS,** m/z = 548.1 [M+1]⁺. **WX238:** ¹H NMR (400MHz, METHANOL-d4) Shift = 8.40-8.20 (m, 3H), 8.10 (dd, *J* = 5.8, 8.9 Hz, 1H), 8.04-7.96 (m, 2H), 7.78 (d, *J* = 8.4 Hz, 1H), 7.47 (dd, *J* = 2.5, 8.5 Hz, 1H), 7.30-7.17 (m, 1H), 4.69 (dd, *J* = 3.7, 13.7 Hz, 1H), 4.46 (dd, *J* = 3.7, 8.6 Hz, 1H), 4.00 (dd, *J* = 8.7, 13.8 Hz, 1H), 3.86 (s, 3H); **MS,** m/z = 548.1 [M+1]⁺.

### Experimental Example 1: Evaluation in vitro

### 1. In vitro test of enzyme activity

The lipokinase reaction was carried out under the conditions of a suitable substrate and ATP, followed by two steps to detect the kinase activity with the ADP-Glo ™ kit. The first step was terminating the kinase reaction, wherein the remaining ATP was completely removed and only ADP was left; the second step was adding kinase detection reagents to convert ADP to ATP, accompanied by luciferin/luciferase reaction. Finally, the output value of fluorescence value was converted into kinase activity. The conditions for testing PI3K enzyme activity were shown in Table 1.

**Table 1 Conditions for testing PI3K enzyme activity**

| Subtype | Final enzyme concentration | ATP (µM) | PIP2:3PS (µM) | Reaction time (min) |
|---|---|---|---|---|
| PI3K alpha | 0.2 nM | 40 | 50 | 120 |
| PI3K beta | 0.6 nM | 40 | 50 | 120 |
| PI3K delta | 0.25 nM | 40 | 50 | 120 |
| PI3K gamma | 0.4 nM | 25 | 50 | 120 |

### Experimental materials and equipments:

| | | |
|---|---|---|
| 1. Enzyme: | PI3K alpha | Millipore #14-602-K |
| | PI3K beta | Promega #V1751 |
| | PI3K delta | Millipore #14-604-K |
| | PI3K gamma | Millipore #14-558-K |

### 2. Kit: ADP-Glo ™ lipokinase and PIP2: 3PS kit (Promega # V1792)

The kit contains: 1 mM PIP2: 3PS, 10 × lipid dilution buffer, 1 M magnesium chloride, 10 mM ATP, 10 mM ADP, ADP-Glo reagent, detection buffer and detection substrate.

### 3. Reaction plate: OptiPlate-384, white and transparent (PerkinElmer # 6007299).

### Reagent preparation:

1. 10 × reaction buffer: 500 mM HEPES, pH 7.5, 500 mM NaCl, 9 mM MgCl₂; BSA: 10% stock solution, homemade.
2. Final test system conditions: 1 × reaction system: 50 mM HEPES, 50 mM NaCl, 3mM MgCl₂, 0.01% BSA (freshly prepared on the day of the experiment), 1% DMSO (v/v) +/- compound
3. Reaction system: 3 µL mixture of the enzyme and the substrate (1:1) + 2 µL ATP/MgCl₂ mixture + 5 µL ADP-Glo reagent + 10 µL detection reagent.

The detailed experimental procedures are as follows:
1. Compound dilution: 50 nL 100 × compound/DMSO was transferred to the test well plate with Echo.
   - For PI3Kα, the compound was diluted three-fold from the highest concentration of 0.111 mM for a total of 10 concentrations.
   - For PI3Kβ/PI3Kδ/PI3Kγ, the compound was three-fold diluted from the highest concentration of 1.11 mM for a total of 10 concentrations.
2. Kinase reaction:
   (1) The compound to be tested was prepared and 50 nL of 100 plus compound solution or DMSO was added to the corresponding well plate.
   (2) 3.33 plate reaction buffer was prepared.
   (3) 3.33 solution PIP2:3PS was prepared, PIP2:3PS was thawed for at least 1 minute before use.
   (4) 2.5 mM containing 5.25 mM MgCl₂ was prepared.
   (5) 3.33 PI3Kα / PI3Kβ / PI3Kδ / PI3Kγ solution was prepared.
   (6) The lipokinase solution and the PIP2: 3PS solution were mixed in a volume ratio of 1:1.
   (7) 3.33: lipokinase buffer and PIP2:3PS solution were mixed in a volume ratio of 1:1.
   (8) 3 µL of buffer solution and PIP2:3PS were added to the first and the second columns of the well plate.
   (9) 3 µL of the mixed solution of the enzyme and PIP2:3PS was added to the wells except the first and the second columns, centrifuged for 10 seconds (1000 rpm) and incubated at 23 °C for 20 minutes.
   (10) 2 µL 2.5n1000 rpm₂ was added and well was shaken.
   (11) The well plate was covered and shaken for about 30 s, then the well plate was incubated at 23 °C for 2 hours.
   (12) 5 µL of ADP-Glo reagent containing 10 mM MgCl₂ was added.
   (13) The well plate was centrifuged at 1000 rpm for 10 seconds, and the well plate was then covered, shaken for about 30 seconds, and incubated at 23 °C for 60 minutes.
   (14) 10 µL kinase detection reagent was added.
   (15) The well plate was centrifuged at 1000 rpm for 10 seconds, and then incubated at 23 °C for 60 minutes.
   (16) The fluorescence value was measured on the Envision instrument.

### 2. In vitro test of cells activity

By the method of ELISA, the inhibition level of the test compound on the phosphorylation of PI3K downstream protein Akt in the signaling pathway was determined in MCF7 cells line to reflect the cells activity of the compound.

Cell culture medium: complete cell culture medium (RPMI 1640 + 10% serum + 1% L-glutamine + 1% double antibody)

Serum-free medium (without serum, RPMI 1640 + 1% L-glutamine + 1% double antibody)

The detailed procedures are as follows:
(1) MCF7 cells (ATCC® HTB-22 ™) were inoculated into a 96-well plate at 100 µL of cell complete medium per well (2.5* 10⁴ cells per well), and the cells were incubated at 37 °C and 5% CO₂ for 24 hours.
(2) The complete cell culture medium was replaced with 100 µL of serum-free medium, and incubated for starvation overnight.
(3) The compound was prepared (The initial concentration of the compound is 1 mM, which was subjected to a three-fold dilution in 10 concentrations, and the compound of each concentration was then diluted 100-fold with serum-free medium). 25 µL of the diluted compound was added to the cells orifice plate.
(4) The cells were incubated at 37 °C and 5% CO₂ for 2 hours.
(5) The cells in the well plate were stimulated with 10 µg/mL insulin (Sigma # I9278-5mL), incubated for 30 minutes, and then centrifuged at 1000 rpm for 5 minutes at room temperature.
(6) 250 µL of 1 × balanced salt solution (Invitrogen, # 14065-056, 4 °C, containing 1mM / L Na₃VO₄) was added to each well to wash the cells once.
(7) 100 µL of lysis buffer (trimethylolaminomethane hydrochloride, Invitrogen, # 15567-1000ml) was added to each well, shaken at 4 °C for 60 minutes, and then centrifuged at 4000 rpm for 4 minutes.
(8) The following procedures were carried out according to the instructions of ELISA kit (TGR BioSciences # EKT002).

The results were shown in Table 2.

**Table 2 Results of in vitro screening test of compounds of the present disclosure**

| **Compound** | **PI3Kα IC₅₀ (nM)** | **PI3Kβ IC₅₀ (nM)** | **PI3Kδ IC₅₀ (nM)** | **PI3Kγ IC₅₀ (nM)** | **MCF7 CellIC₅₀ (nM)** |
|---|---|---|---|---|---|
| **R001** | 3.77 | 100 | - | - | - |
| **R002** | 13.6 | 1056 | 122 | 20.4 | 628 |
| **R003** | 7.16 | 426 | 85.0 | 5.29 | 318 |
| **R004** | 3.21 | 46.4 | - | - | - |
| **R005** | 5.84 | 100 | - | - | - |
| **R006** | 5.73 | 159 | 8.57 | 58.5 | 161 |
| **R007** | - | - | - | - | 656 |
| **R008** | 12 | 121 | - | - | - |
| **R009** | 10.4 | 108 | - | - | - |
| **R010** | 34.4 | 345 | 45.8 | 421 | - |
| **R011 (A2)** | 74.5 | 168 | - | - | 230 |
| **R012 (A10)** | 13.9 | 67.9 | - | - | >1000 |
| **WX001** | 1.16 | 68.5 | 3.88 | 4.57 | 16.8 |
| **WX002** | 2.84 | 216 | 12.4 | 33.7 | - |
| **WX003** | 1.49 | 112 | 3.55 | 11.8 | 13.1 |
| **WX004** | 3.15 | 420 | 24.2 | 65.8 | - |
| **WX005** | 4.39 | 244 | 7.42 | 49.7 | - |
| **WX006** | 1.00 | 279 | 4.53 | 19.6 | 279 |
| **WX007** | 1.49 | 159 | 1.85 | 29.1 | - |
| **WX008** | 9.85 | 1051 | - | - | - |
| **WX009** | 3.69 | 746 | - | - | - |
| **WX010** | 4.93 | 809 | 41.4 | 70.6 | - |
| **WX011** | 1.13 | 377 | 10.54 | 20.74 | - |
| **WX012** | 4.95 | 239 | 8.12 | 60.8 | - |
| **WX013** | 1.22 | 95.9 | 5.25 | 15.2 | 209 |
| **WX014** | 1.18 | 238 | 1.23 | 35.5 | - |
| **WX015** | 0.69 | 124 | 1.53 | 9.9 | 180 |
| **WX016** | 2.81 | 325 | 1.35 | 70.2 | 116 |
| **WX017** | 0.42 | 161 | 1.27 | 8.27 | 152 |
| **WX018** | 2.02 | 168 | 10.2 | 14.9 | 17.1 |
| **WX019** | 2.75 | 116 | 9.22 | 8.18 | 23.9 |
| **WX020** | 10.2 | 413 | - | - | - |
| **WX021** | 6.89 | 133 | - | - | 52.3 |
| **WX022** | 25.5 | 832 | - | - | 125 |
| **WX023** | 5.71 | 82.4 | 2.02 | 20.6 | 39.9 |
| **WX024** | 0.29 | 19.9 | 0.47 | 2.03 | 71.5 |
| **WX025** | 0.41 | 13.2 | 0.47 | 1.00 | 23.9 |
| **WX026** | 5.4 | 928 | - | - | - |
| **WX027** | 9.01 | 582 | - | - | - |
| **WX028** | 3.09 | 596 | 34.5 | 37.9 | - |
| **WX029** | 4.88 | 454 | 28 | 21.6 | - |
| **WX030** | 2.34 | 89.7 | 9.95 | 11.5 | 59.3 |
| **WX031** | 1.8 | 271 | 13.9 | 13.8 | 32.9 |
| **WX032** | 0.61 | 139 | - | - | - |
| **WX033** | 1.09 | 126 | - | - | - |
| **WX034** | 0.71 | 554 | 11.6 | 29.9 | - |
| **WX035** | 1.57 | 158 | 7.30 | 19.6 | - |
| **WX036** | - | - | - | - | 223 |
| **WX037** | - | - | - | - | 712 |
| **WX038** | 3.00 | 1216 | 49.6 | 63.6 | 397 |
| **WX039** | 5.85 | 623 | 31.0 | 44.9 | - |
| **WX040** | 5.4 | 183 | 25.6 | 39.9 | 134 |
| **WX041** | 2.66 | 124 | 7.16 | 8.37 | - |
| **WX042** | 1.68 | 125 | 4.22 | 38 | - |
| **WX043** | 3.6 | 212 | 3.58 | 17.9 | - |
| **WX044** | 4.79 | 74.5 | 8.33 | 9.4 | - |
| **WX045** | 1.37 | 109 | 6.41 | 4.1 | - |
| **WX046** | 1.99 | 96.7 | 2.75 | 9.33 | 26.6 |
| **WX047** | 2.67 | 118 | 7.37 | 16.1 | 7.85 |
| **WX048** | 2.5 | 2050 | 18.9 | 50.0 | - |
| **WX049** | 6.07 | 715 | 15.9 | 26.5 | - |
| **WX050** | 4.15 | 189 | 6.85 | 15.8 | - |
| **WX051** | 11.8 | 265 | 12.0 | 29.4 | - |
| **WX052** | 4.31 | 344 | 9.09 | 63.8 | - |
| **WX053** | 4.30 | 337 | 2.40 | 8.84 | - |
| **WX054** | 7.08 | 3642 | 62.5 | 87.2 | - |
| **WX055** | 18.6 | 1317 | 52.2 | 59.7 | - |
| **WX056** | 19.6 | 5079 | 61.0 | 155 | - |
| **WX057** | 57.2 | 963 | 28.4 | 48.1 | - |
| **WX058** | 5.49 | 589 | 18.7 | 54.2 | 56.1 |
| **WX059** | 11.2 | 391 | 13.2 | 14.2 | - |
| **WX060** | 3.49 | 653 | 19.6 | 21.9 | 40.5 |
| **WX061** | 7.17 | 319 | 12.2 | 12.1 | - |
| **WX062** | 30.0 | >10000 | 538 | 374 | - |
| **WX063** | 71.4 | 7231 | 336 | 318 | - |
| **WX064** | 6.80 | 365 | 9.15 | 16.4 | - |
| **WX065** | 4.9 | 1121 | 29.7 | 45.2 | 72.0 |
| **WX066** | 5.26 | 181 | 7.78 | 9.25 | - |
| **WX067** | 2.47 | 399 | 18.8 | 18.3 | 67.4 |
| **WX068** | 0.47 | 54 | 0.98 | 5.49 | 10.9 |
| **WX069** | 0.6 | 26 | 0.21 | 4.04 | - |
| **WX070** | 5.95 | 1839 | 81.8 | 85.5 | - |
| **WX071** | 21.3 | 1731 | 61.1 | 66.3 | - |
| **WX072** | 8.02 | 3402 | 24.3 | 51.2 | 81.1 |
| **WX073** | 15.35 | 1069 | - | - | - |
| **WX074** | 8.38 | 1557 | 17.8 | 27.4 | 80.4 |
| **WX075** | 12.27 | 386 | - | - | - |
| **WX076** | 2.7 | 605 | 7.05 | 14.7 | 169.7 |
| **WX077** | 7.69 | 305 | - | - | - |
| **WX078** | 6.94 | 1705 | 15 | 29.5 | 61.5 |
| **WX079** | 11.1 | 447 | - | - | - |
| **WX080** | 1.72 | 260 | 11.7 | 10.9 | 338.6 |
| **WX081** | 3.55 | 150 | - | - | - |
| **WX082** | 4.57 | 192 | - | - | - |
| **WX083** | 2.09 | 548 | - | - | 189.7 |
| **WX084** | 0.56 | 110 | - | - | - |
| **WX085** | 19.3 | 5458 | - | - | - |
| **WX087** | 3.35 | 355 | 30.7 | 41.3 | 19.0 |
| **WX088** | 8.00 | 211 | - | - | - |
| **WX089** | 1.75 | 192 | 25.3 | 19.8 | 35.3 |
| **WX090** | 3.94 | 124 | - | - | - |
| **WX091** | 5.11 | 537 | 26.2 | 97.3 | 5.7 |
| **WX092** | 6.47 | 250 | - | - | - |
| **WX093** | 4.27 | 624 | 19.3 | 42.9 | 226 |
| **WX094** | 4.32 | 455 | - | - | - |
| **WX095** | 4.77 | 390 | 29.6 | 68.3 | - |
| **WX096** | 8.51 | 175 | - | - | - |
| **WX097** | 4.37 | 678 | 25.3 | 45.6 | 87.1 |
| **WX098** | 10.0 | 492 | - | - | - |
| **WX099** | 1.67 | 323 | 12.4 | 19.3 | 132 |
| **WX100** | 3.99 | 159 | - | - | - |
| **WX101** | 2.46 | 132 | 5.8 | 11.2 | - |
| **WX102** | 4.1 | 94.4 | - | - | - |
| **WX103** | 20.8 | 2276 | 403 | 360 | 13.4 |
| **WX104** | 38.5 | 1951 | - | - | - |
| **WX105** | 10.5 | 1154 | 41.1 | 29.2 | 192.6 |
| **WX106** | 19.5 | 736 | - | - | 460.2 |
| **WX107** | 18.1 | 1849 | - | - | 301.6 |
| **WX108** | 13.9 | 415 | - | - | 96.5 |
| **WX109** | 6.19 | 720 | - | - | - |
| **WX119** | 8.40 | 500 | - | - | - |
| **WX121** | 23.6 | >10000 | 294 | 428 | - |
| **WX122** | 54.6 | 2256 | - | - | - |
| **WX123** | 9.27 | 268 | - | - | - |
| **WX124** | 7.18 | 674 | - | - | - |
| **WX125** | 21.5 | 318 | - | - | 92.8 |
| **WX127** | 22.8 | 2163 | - | - | >1000 |
| **WX129** | - | - | - | - | 387 |
| **WX130** | 7.11 | 215 | - | - | - |
| **WX131** | 3.76 | 312 | - | - | - |
| **WX132** | 8.26 | 261 | - | - | - |
| **WX133** | 5.02 | 466 | - | - | - |
| **WX134** | 4.71 | 101 | - | - | - |
| **WX135** | 2.32 | 166 | - | - | - |
| **WX138** | 5.22 | 203 | - | - | - |
| **WX139** | 3.30 | 584 | - | - | 10.7 |
| **WX140** | 18.7 | 543 | - | - | - |
| **WX141** | 9.76 | 745 | - | - | - |
| **WX142** | 6.11 | 81.2 | - | - | - |
| **WX143** | 3.56 | 128 | - | - | - |
| **WX144** | 24 | 631 | - | - | - |
| **WX145** | 8.68 | 1057 | 75.0 | 140 | 9.06 |
| **WX146** | 2.70 | 95.4 | - | - | - |
| **WX147** | 1.60 | 222 | 8.70 | 15.8 | 19.1 |
| **WX148** | 7.69 | 381 | - | - | - |
| **WX149** | 4.78 | 559 | 19.8 | 64.1 | 26.7 |
| **WX150** | 1.67 | 81.0 | - | - | - |
| **WX151** | 1.07 | 187 | 12.4 | 9.49 | 0.45 |
| **WX152** | 2.84 | 159 | - | - | - |
| **WX153** | 2.12 | 278 | 11.4 | 23.0 | 13.4 |
| **WX154** | 7.22 | 620 | - | - | - |
| **WX155** | 13.0 | 411 | - | - | - |
| **WX156** | 3.56 | 498 | - | - | - |
| **WX157** | 5.81 | 287 | - | - | - |
| **WX158** | 23.7 | 1120 | - | - | 154 |
| **WX159** | 13.7 | 2097 | - | - | 116 |
| **WX160** | 11.2 | 2097 | - | - | - |
| **WX161** | 8.92 | 153 | - | - | - |
| **WX162** | 9.18 | 201 | - | - | - |
| **WX163** | 6.51 | 268 | - | - | - |
| **WX164** | 8.24 | 178 | - | - | - |
| **WX165** | 5.05 | 250 | - | - | - |
| **WX166** | 5.78 | 222 | - | - | - |
| **WX167** | 13.0 | 159 | - | - | - |
| **WX168** | 6.27 | 546 | - | - | - |
| **WX169** | 14.1 | 386 | - | - | - |
| **WX170** | 3.14 | 668 | 29.6 | 66.1 | 1.2 |
| **WX171** | 8.87 | 443 | - | - | - |
| **WX172** | 3.13 | 104 | - | - | - |
| **WX173** | 1.21 | 146 | 10.4 | 17.4 | 15.7 |
| **WX174** | 8.34 | 127 | - | - | - |
| **WX175** | 3.87 | 206 | - | - | - |
| **WX176** | 3.13 | 101 | - | - | - |
| **WX177** | 2.03 | 170 | - | - | - |
| **WX180** | 10.2 | 411 | - | - | - |
| **WX181** | 7.06 | 720 | 47.4 | 48.3 | 9.04 |
| **WX182** | 1.47 | 73.3 | - | - | - |
| **WX183** | 0.95 | 166 | 6.34 | 8.21 | 51.4 |
| **WX184** | 8.14 | 192 | - | - | - |
| **WX185** | 3.58 | 222 | - | - | - |
| **WX186** | 11.5 | 303 | - | - | - |
| **WX187** | 4.84 | 331 | - | - | - |
| **WX188** | 2.98 | 152 | - | - | - |
| **WX189** | 1.80 | 345 | 17.3 | 16.7 | 27.1 |
| **WX190** | 13.2 | 205 | - | - | - |
| **WX191** | 7.04 | 312 | - | - | - |
| **WX192** | 5.39 | 633 | 37.5 | 37.8 | 53.6 |
| **WX193** | 12.6 | 451 | - | - | - |
| **WX194** | 3.42 | 432 | 16.4 | 17.0 | 84.9 |
| **WX195** | 5.58 | 327 | - | - | - |
| **WX196** | 41.0 | 1771 | - | - | 395 |
| **WX197** | 38.5 | 153 | - | - | 233 |
| **WX198** | 1.10 | 33.0 | 2.10 | - | 9.43 |
| **WX199** | 4.86 | 199 | - | - | - |
| **WX200** | 2.44 | 42.1 | 1.44 | 7.93 | 24.4 |
| **WX201** | 1.04 | 36.4 | 0.94 | 3.06 | 10.8 |
| **WX202** | 6.71 | 88.5 | 4.36 | 44.1 | - |
| **WX203** | 12.8 | 595 | - | - | - |
| **WX204** | 22.5 | 527 | - | - | - |
| **WX205** | 3.70 | 97.3 | - | - | - |
| **WX206** | 5.12 | 133 | - | - | - |
| **WX207** | 0.85 | 20.5 | - | - | - |
| **WX208** | 2.49 | 119 | - | - | - |
| **WX209** | 4.08 | 176 | - | - | - |
| **WX210** | 6.77 | 182 | - | - | - |
| **WX211** | 3.07 | 192 | - | - | - |
| **WX212** | 1.76 | 253 | 9.22 | 27.7 | 28.0 |
| **WX213** | 4.52 | 201 | - | - | - |
| **WX214** | 2.22 | 278 | 18.8 | 31.4 | 17.2 |
| **WX215** | 4.11 | 168 | - | - | - |
| **WX216** | 2.58 | 440 | 38.6 | 34.6 | 48.5 |
| **WX217** | 3.47 | 269 | - | - | - |
| **WX218** | 5.04 | 216 | - | - | - |
| **WX219** | 5.36 | 276 | - | - | - |
| **WX220** | 10.1 | 139 | - | - | - |
| **WX221** | 4.61 | 162 | - | - | - |
| **WX222** | 1.80 | 361 | 20.7 | 29.7 | 12.6 |
| **WX223** | 5.37 | 184 | - | - | - |
| **WX224** | 3.08 | 326 | 22.0 | 23.7 | 43.5 |
| **WX225** | 5.28 | 199 | - | - | - |
| **WX226** | 2.52 | 283 | - | - | 44.3 |
| **WX227** | 6.68 | 326 | - | - | - |
| **WX228** | 2.70 | 393 | 70.5 | 58.2 | 6.14 |
| **WX229** | 11.5 | 1169 | 57.6 | 142 | 110 |
| **WX230** | 23.5 | 1306 | - | - | - |
| **WX232** | - | - | - | - | 55.3 |

| | | | | | |
|---|---|---|---|---|---|
| "-": means not determined | | | | | |

**Conclusion:** The compound of the present disclosure has a good inhibitory activity on PI3K kinase, and at the same time, it has a high subtype selectivity for PI3K β/γ/δ. In addition, it can also well inhibit the phosphorylation level of Akt which is the downstream of PI3K in cells.

### Experimental Example 2: In vivo study

### 1. In vivo DMPK study

Experimental objective: female Balb/c mice were used as the test animals, and the blood concentration of the compound was determined and the pharmacokinetic behavior was evaluated after a single administration.

Experimental operation: 12 healthy adult female Balb/c mice were selected, 6 for intravenous injection group and 6 for oral administration group. The compound to be tested was mixed with an appropriate amount of intravenous injection group vehicle (10% HP-betaCD: 10% solutol = 1:1, pH = 8), vortex-mixed and sonicated to prepare a 1.0 mg/mL clear solution, followed by filtration for subsequent use; the vehicle in the oral group was 0.5% MC/0.2% Tw80. After the test compound and the vehicle were mixed, it was vortex-mixed and sonicated to prepare a 1.0 mg/mL homogeneous suspension for subsequent use. After an intravenous administration at a dose of 1 mg/kg or an oral administration at the dose of 2 mg/kg and 10 mg/kg, whole blood of the mice was collected for a certain period of time to prepare plasma. The drug concentration was analyzed by LC-MS/MS method, and Phoenix WinNonlin software was used (Pharsight, USA) to calculate the pharmacokinetic parameters. The results were shown in Table 3.

**Table 3. Test results of the pharmacokinetic properties of the compounds of the present disclosure in mice**

| **Compound** | **Cₘₐₓ (nM)** | **F%** | **Oral DNAUC (nM** | **V_{d} (L/kg)** | **Cl (mL/min/ kg)** | **Intravenous injection T_{1/2} (h)** |
|---|---|---|---|---|---|---|
| **WX018** | 4453 (10 mpk) | 50.3 | 1599 | 0.529 | 9.80 | 0.918 |
| **WX019** | 3797 (10 mpk) | - | 1386 | - | - | - |
| **WX031** | 22167 (10 mpk) | 59.4 | 4228 | 0.179 | 4.16 | 0.966 |
| **WX046** | 14867 (10 mpk) | - | 1504 | - | - | - |
| **WX047** | 14100 (10 mpk) | - | 1540 | - | - | - |
| **WX060** | 9133 (10 mpk) | 30.7 | 695 | 0.222 | 12.8 | 0.278 |
| **WX067** | 3390 (2 mpk) | - | 1047 | - | - | - |
| **WX068** | 10623 (10 mpk) | 18.4 | 1021 | 0.229 | 5.49 | 2.30 |
| **WX078** | 2923 (2 mpk) | - | 787 | - | - | - |
| **WX087** | 5000 (10 mpk) | - | 1372 | - | - | - |
| **WX089** | 20267 (10 mpk) | - | 3477 | - | - | - |
| **WX097** | 24700 (10 mpk) | - | 2018 | - | - | - |
| **WX099** | 6667 (10 mpk) | 36.4 | 791 | 0.280 | 13.2 | 0.305 |
| **WX103** | 18300 (10 mpk) | 58.4 | 12262 | 0.324 | 1.29 | 3.55 |
| **WX139** | 10133 (10 mpk) | - | 1118 | - | - | - |
| **WX147** | 19900 (10 mpk) | - | 1947 | - | - | - |
| **WX151** | 24033 (10 mpk) | - | 5704 | - | - | - |
| **WX153** | 37000 (10 mpk) | - | 6560 | - | - | - |
| **WX170** | 10900 (10 mpk) | - | 885.0 | - | - | - |
| **WX173** | 26300 (10 mpk) | - | 4703 | - | - | - |
| **WX181** | 8340 (10 mpk) | - | 882.0 | - | - | - |
| **WX183** | 25867 (10 mpk) | - | 4867 | - | - | - |
| **WX189** | 8520 (10 mpk) | - | 1070 | - | - | - |
| **WX198** | - | - | - | 0.257 | 4.16 | 0.890 |
| **WX200** | 1736 (10 mpk) | - | 3687 | - | - | - |
| **WX201** | 5118 (10 mpk) | - | 18467 | - | - | - |
| **WX214** | 8413 (10 mpk) | - | 734 | - | - | - |
| **WX222** | 6877 (10 mpk) | - | 1300 | - | - | - |

| | | | | | | |
|---|---|---|---|---|---|---|
| "-": means not determined. | | | | | | |

Cₘₐₓ: the highest concentration of the drug in the body; F%: oral bioavailability; Oral DNAUC: area under the dose normalization curve; V_{d}: apparent volume of distribution; Cl: clearance rate; T_{1/2}: half-life.

**Conclusion:** The compound of the present disclosure exhibits high exposure, low clearance, and relatively good oral bioavailability in mice.

### 2. In vivo drug-efficacy study

### (1) BALB/c nude mouse subcutaneous xenograft tumor model of human breast cancer BT-474 cells

### Cell culture

Human breast cancer BT-474 cells (ATCC, Manassas, Virginia, batch number: HTB-20) were subjected to *in vitro* monolayer culture, and the culture conditions were: Hybri-Care medium with 10% fetal bovine serum and 1% double antibody, 37 °C and 5% CO₂ incubator. The cells were passaged by digestion with trypsin-EDTA twice a week. When the cells saturation reached 80-90%, and the quantity met the requirement, the cells was collected, counted and inoculated.

### Tumor cell inoculation (tumor inoculation)

The subcutaneous implantation of the estrogen tablets (Innovative Research, Cat # SE-121, 0.36 mg/60-day release) was carried out one day before cell inoculation. 0.2 mL (10.2 m) of BT-474 cells (with matrigel, volume 1:1) was subcutaneously inoculated into the right back of each mouse. When the average tumor volume reached 188 mm³, the administration was started in groups.

### Preparation of test substance

Vehicle group: 2.5 g of methylcellulose was weighed in a beaker, 400 mL of ultrapure water was added and stirred overnight. After completely dissolved, the mixture was transferred to a 500 mL volumetric flask and the volume was brought to 500 mL, followed by addition of 1 mL of Tween 80 and even mixing.

Test compound group: a certain amount of test compound was weighed in a brown dispensing bottle, and a corresponding volume of vehicle was added and vortex-mixed to obtain a uniform suspension or a clear solution.

### Tumor measurement and experimental index

The experimental index was to evaluate whether the tumor growth was inhibited, delayed or cured. The diameter of the tumor was measured with a vernier caliper twice a week. The calculation formula of tumor volume was: V = 0.5a × b², wherein a and b represent the long and short diameter of the tumor, respectively.

The antitumor efficacy of the compound was evaluated by TGI (%) or relative tumor proliferation rate T/C (%). The tumor growth inhibition rate was reflected by TGI (%). Calculation of TGI (%): TGI (%) = [(1- ((average tumor volume at the end of one treatment group) - (average tumor volume at the beginning of this treatment group))] / ((average tumor volume at the end of treatment in the vehicle control group)-(average tumor volume at the beginning of treatment in the vehicle control group))] × 100%.

**Table 4. The anti-tumor effect of the compound of the present disclosure on murine breast cancer model of BT-474 mice**

| Compound number | TGI% |
|---|---|
| **WX031**@30mpk | 82.3% |
| **WX058**@30mpk | 60.0% |
| **WX089**@30mpk | 77.6% |

### (2) BALB/c nude mouse subcutaneous xenograft tumor model of human ovarian cancer SK-OV-3 cells

### Cell culture

Human ovarian cancer SK-OV-3 cells (ECACC-91091004) were subjected to *in vitro* monolayer culture , and the culture conditions were: McCoy's 5a medium (Gibco, 16600-082) with 10% fetal bovine serum and 100 U/mL penicillin and 100 µg/mL streptomycin, 37 °C and 5% CO₂ incubator. The cells were passaged by digestion with trypsin-EDTA twice a week. When the cells saturation reached 80%-90%, the cells were collected, counted, and inoculated.

### Tumor cell inoculation

0.2 mL of 10 × 10⁶ SK-OV-3 cells were subcutaneously inoculated into the right back of each nude mouse (PBS: Matrigel = 1:1). When the average tumor volume reached 200 mm³, 48 tumor-bearing mice were divided into 8 groups by stratified random method with 6 mice in each group, which were administered on the day of grouping.

### Preparation of test substance

Vehicle group: 2.0 g of methyl cellulose was weighed in a 500 mL glass bottle, followed by addition of 399.2 mL of ddH₂O and 0.8 mL of Tween 80.

Test compound group: a certain amount of test compound was weighed in a brown dispensing bottle, and a corresponding volume of vehicle was added and rotary-evaporated to obtain a uniform suspension or clear solution.

### Tumor measurement and experimental index

The experimental index was to evaluate whether the tumor growth was inhibited, delayed or cured. The diameter of the tumor was measured with a vernier caliper twice a week. The calculation formula of tumor volume was: V = 0.5a × b², wherein a and b represented the long and short diameters of the tumor, respectively.

The antitumor efficacy of the compound was evaluated by TGI (%) or relative tumor proliferation rate T/C (%). The tumor growth inhibition rate was reflected by TGI (%). Calculation of TGI (%): TGI (%) = [(1- ((average tumor volume at the end of administration in one treatment group) - (average tumor volume at the beginning of administration in this treatment group))] / ((average tumor volume at the end of treatment in the solvent control group) - (average tumor volume at the beginning of treatment in the solvent control group))] × 100%. The results were shown in Table 5.

**Table 5. The anti-tumor effect of the compound of the present disclosure on SK-OV-3 mice of murine breast cancer model**

| Compound number | **WX031**@30mpk | **WX198**@30mpk |
|---|---|---|
| TGI% | 86.0% | 62.6% |

**Conclusion:** The compound of the present disclosure can significantly inhibit tumor growth *in vivo.*

## Claims

1. A compound of formula (I), an isomer thereof or a pharmaceutically acceptable salt thereof, 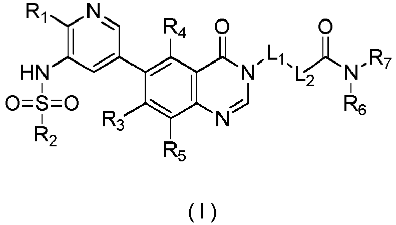 wherein,
R₁ is selected from H, F, Cl, Br, I, OH, NH₂, CN, C₁₋₆ alkyl, C₁₋₆ heteroalkyl or C₃₋₆ cycloalkyl-O-, each of the NH₂, C₁₋₆ alkyl, C₁₋₆ heteroalkyl and C₃₋₆ cycloalkyl-O- is optionally substituted by one, two or three R;
R₂ is selected from phenyl or 5-6 membered heteroaryl, each of the phenyl and 5-6 membered heteroaryl is optionally substituted by one, two or three R;
each of R₃, R₄ and R₅ is independently selected from H, F, Cl, Br, I, OH or NH₂;
R₆ is H, C₁₋₆ alkyl, C₁₋₆ heteroalkyl, C₃₋₇ cycloalkyl or 3-6 membered heterocycloalkyl, each of the C₁₋₆ alkyl, C₁₋₆ heteroalkyl, C₃₋₇ cycloalkyl and 3-6 membered heterocycloalkyl is optionally substituted by one, two or three R;
R₇ is H or C₁₋₆ alkyl which is optionally substituted by one, two or three R;
or, R₆ and R₇ are connected to form a 3-7-membered ring, which is optionally substituted by one, two or three R;
L₁ is a single bond or -C₁₋₆ alkyl- which is optionally substituted by one, two or three R;
L₂ is a single bond or -C₃₋₇ cycloalkyl- which is optionally substituted by one, two or three R;
each R is independently selected from H, F, Cl, Br, I, OH, NH₂, CN, C₁₋₆ alkyl or C₁₋₆ heteroalkyl; each of the C₁₋₆ alkyl and C₁₋₆ heteroalkyl is optionally substituted by one, two or three R';
each R' is independently selected from F, Cl, Br, I, OH, NH₂, CN, Me or Et;
the 3-6 membered heterocycloalkyl and the 5-6 membered heteroaryl contain 1-4 heteroatoms independently selected from N, O or S;
each heteroatom or heteroatomic group in the C₁₋₆ heteroalkyl is independently selected from N, -O-, -S-, -NH-, -C(=O)NH-, -C(=O)- or -C(=O)O-, and the number of the heteroatom or the heteroatomic group is one, two ,three or four.

2. The compound, the isomer thereof or the pharmaceutically acceptable salt thereof as defined in claim 1, wherein, R is selected from H, F, Cl, Br, I, OH, NH₂, CN, C₁₋₃ alkyl or C₁₋₃ alkoxy, each of the C₁₋₃ alkyl and C₁₋₃ alkoxy is optionally substituted by one, two or three R'.

3. The compound, the isomer thereof or the pharmaceutically acceptable salt thereof as defined in claim 2, wherein, R is selected from H, F, Cl, Br, I, OH, NH₂, CN, Me, Et or 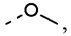 each of the Me, Et and 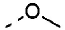 is optionally substituted by one, two or three R'.

4. The compound, the isomer thereof or the pharmaceutically acceptable salt thereof as defined in claim 3, wherein, R is selected from H, F, Cl, Br, I, OH, NH₂, CN, Me, CF₃, Et,

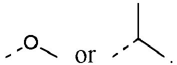

5. The compound, the isomer thereof or the pharmaceutically acceptable salt thereof as defined in any one of claims 1 to 4, wherein, R₁ is selected from H, F, Cl, Br, I, OH, NH₂, CN, C₁₋₃ alkyl, C₁₋₃ alkoxy, C₁₋₃ alkylamino or cyclopropyl-O-, each of the C₁₋₃ alkyl, C₁₋₃ alkoxy, C₁₋₃ alkylamino and cyclopropyl-O- is optionally substituted by one, two or three R.

6. The compound, the isomer thereof or the pharmaceutically acceptable salt thereof as defined in any one of claims 1 to 4, wherein, R₁ is selected from H, F, Cl, Br, I, OH, NH₂, CN, Me, Et, 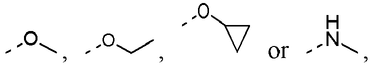 each of the Me, Et, 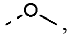 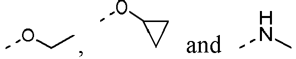 is optionally substituted by one, two or three R.

7. The compound, the isomer thereof or the pharmaceutically acceptable salt thereof as defined in claim 6, wherein, R₁ is selected from H, F, Cl, Br, I, OH, NH₂, CN, Me, Et, CF₃,

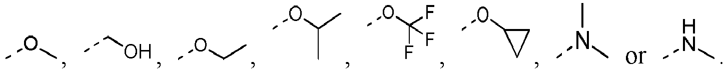

8. The compound, the isomer thereof or the pharmaceutically acceptable salt thereof as defined in any one of claims 1 to 4, wherein, R₂ is selected from phenyl, thiazolyl, furyl, oxazolyl, isoxazolyl, pyrrolyl or thienyl, each of the phenyl, thiazolyl, furyl, oxazolyl, isoxazolyl, pyrrolyl and thienyl is optionally substituted by one, two or three R.

9. The compound, the isomer thereof or the pharmaceutically acceptable salt thereof as defined in claim 8, wherein, R₂ is selected from 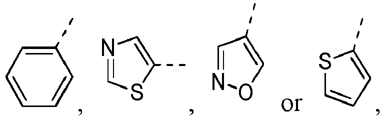 each of the 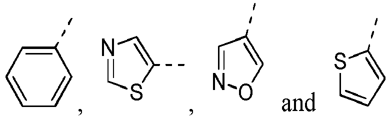 is optionally substituted by one, two or three R.

10. The compound, the isomer thereof or the pharmaceutically acceptable salt thereof as defined in claim 1 or 9, wherein, R₂ is selected from

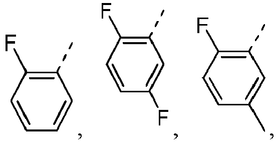

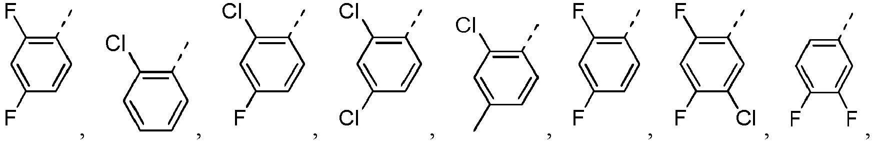

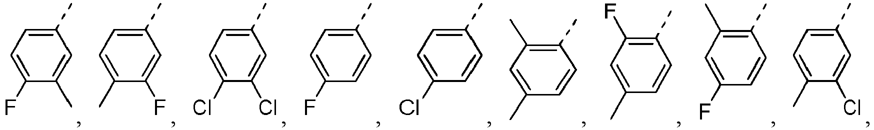

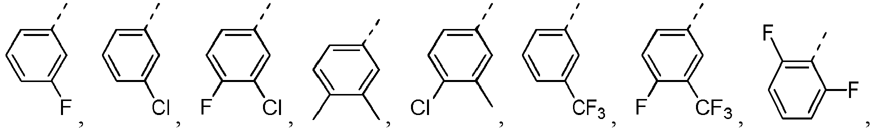

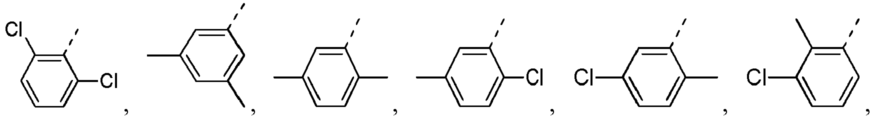

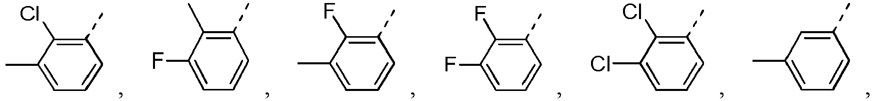

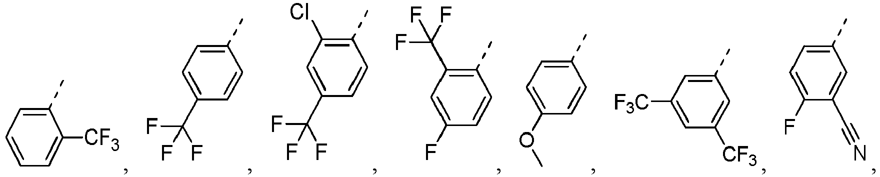

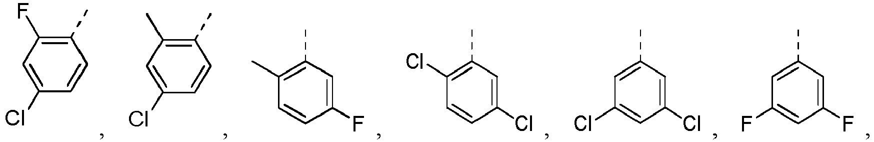

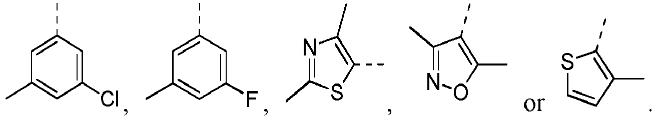

11. The compound, the isomer thereof or the pharmaceutically acceptable salt thereof as defined in any one of claims 1 to 4, wherein, R₆ is H, C₁₋₃ alkyl, C₁₋₃ heteroalkyl, cyclopropyl, cyclobutyl, oxbutyl or tetrahydrofuranyl, each of the C₁₋₃ alkyl, C₁₋₃ heteroalkyl, cyclopropyl, cyclobutyl, oxbutyl and tetrahydrofuranyl is optionally substituted by one, two or three R.

12. The compound, the isomer thereof or the pharmaceutically acceptable salt thereof as defined in claim 11, wherein, R₆ is H, Me, Et, 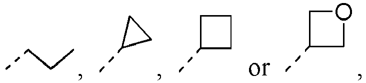 each of the Me, Et, 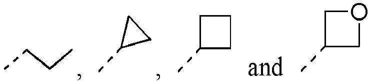 is optionally substituted by one, two or three R.

13. The compound, the isomer thereof or the pharmaceutically acceptable salt thereof as defined in claim 1 or 12, wherein, R₆ is selected from H, Me, Et, CF₃,

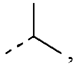

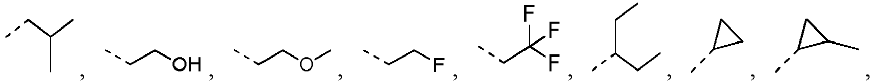

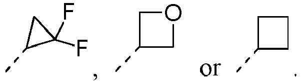

14. The compound, the isomer thereof or the pharmaceutically acceptable salt thereof as defined in any one of claims 1 to 4, wherein, R₇ is selected from H, Me or Et.

15. The compound, the isomer thereof or the pharmaceutically acceptable salt thereof as defined in any one of claims 1 to 4, wherein, structural unit 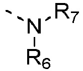 is selected from 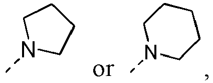 each of the 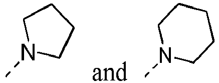 is optionally substituted by one, two or three R.

16. The compound, the isomer thereof or the pharmaceutically acceptable salt thereof as defined in claim 15, wherein, the structural unit 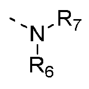 is

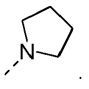

17. The compound, the isomer thereof or the pharmaceutically acceptable salt thereof as defined in any one of claims 1 to 4, wherein, L₁ is a single bond, -CH₂-, -CH₂-CH₂-, 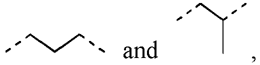 each of the -CH₂-, -CH₂-CH₂-, 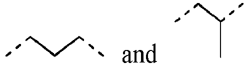 is optionally substituted by one, two or three R.

18. The compound, the isomer thereof or the pharmaceutically acceptable salt thereof as defined in claim 17, wherein, L₁ is selected from a single bond, -CH₂-, -CH₂-CH₂-,

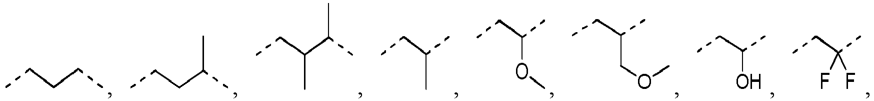

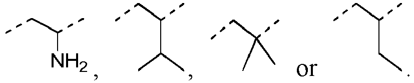

19. The compound, the isomer thereof or the pharmaceutically acceptable salt thereof as defined in any one of claims 1 to 4, wherein, L₂ is single bond, -cyclopropyl-, - cyclobutyl- or -cyclopentyl-, each of the -cyclopropyl-, -cyclobutyl- and -cyclopentyl- is optionally substituted by one, two or three R.

20. The compound, the isomer thereof or the pharmaceutically acceptable salt thereof as defined in claim 19, wherein, L₂ is selected from a single bond, 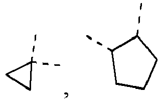 or

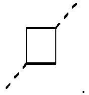

21. The compound, the isomer thereof or the pharmaceutically acceptable salt thereof as defined in claim 18 or 20, wherein, the structural unit 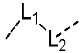 is selected from - CH₂-, -CH₂-CH₂-, 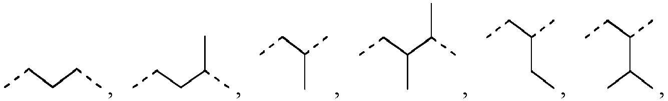 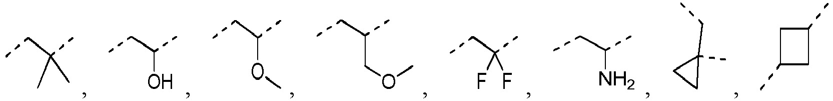 or

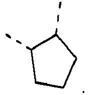

22. The compound, the isomer thereof or the pharmaceutically acceptable salt thereof as defined in any one of claims 1 to 7 or 11 to 16, wherein, the compound is selected from 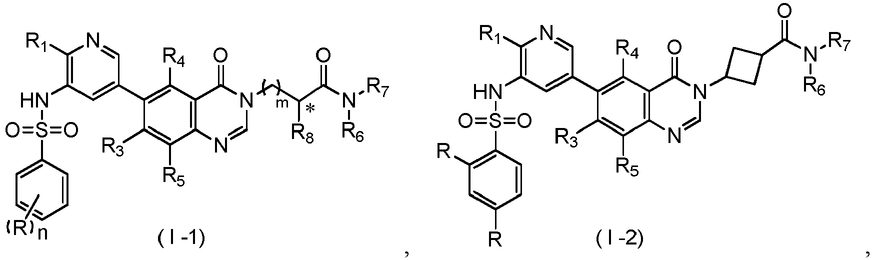 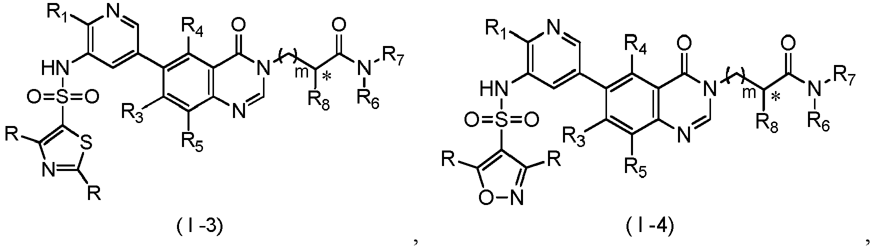 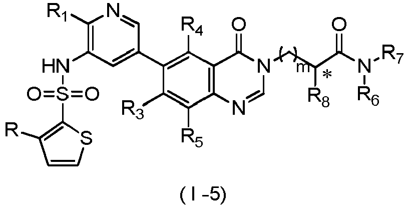 or 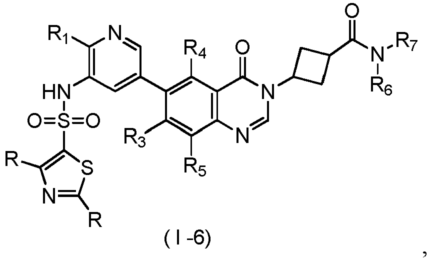 wherein,
n is 1, 2 or 3;
each m is independently 1, 2 or 3;
each R₈ is independently selected from H, F, Cl, Br, I, OH, NH₂, C₁₋₃ alkyl or C₁₋₃ alkoxy, each of the C₁₋₃ alkyl and C₁₋₃ alkoxy is optionally substituted by one, two or three R;
R, R₁ and R₃ to R₇ are as defined in claim 1; and
when R₈ is not H, then the carbon atom with "*" is a chiral carbon atom, which exists in the form of a single enantiomer or enrich in one enantiomer of (*R*) or (*S*).

23. The compound, the isomer thereof or the pharmaceutically acceptable salt thereof as defined in claim 22, wherein, each R₈ is independently selected from H, F, Cl, Br, I, OH, NH₂, Me, Et,

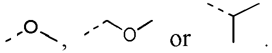

24. The compound, the isomer thereof or the pharmaceutically acceptable salt thereof as defined in claim 22, wherein, the compound is 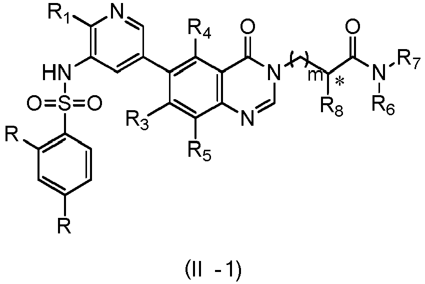 wherein,
m, R, R₁ and R₃ to R₈ are as defined in claim 1.

25. The compound, the isomer thereof or the pharmaceutically acceptable salt thereof as defined in any one of claims 1 to 24, the compound is selected from

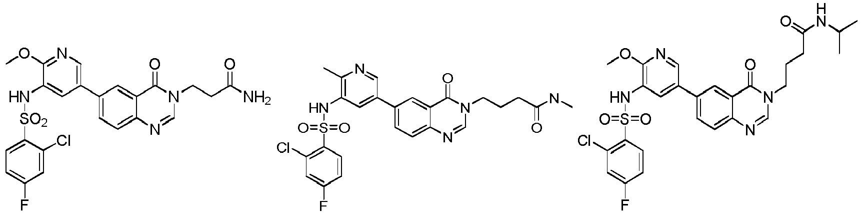

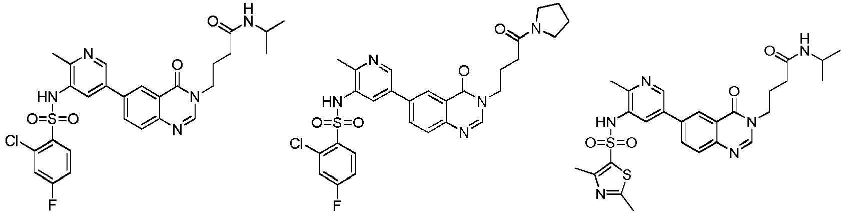

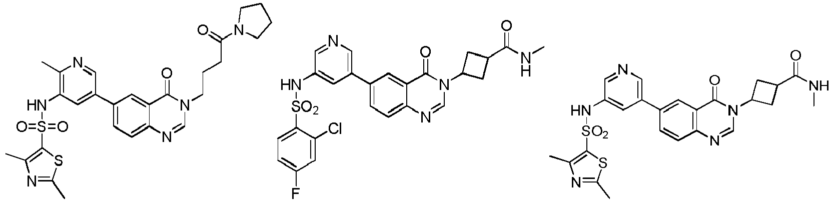

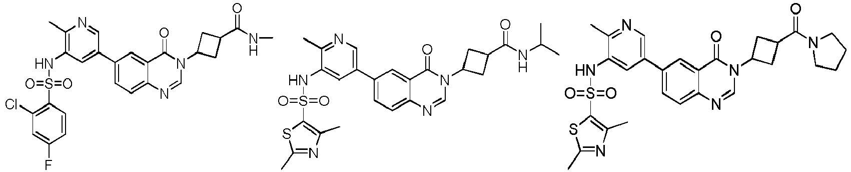

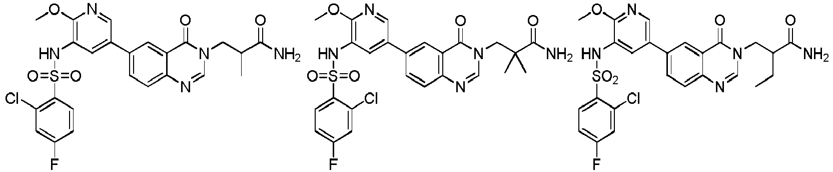

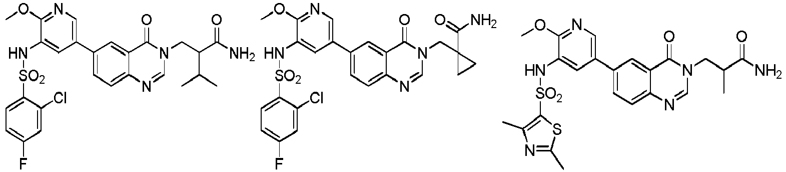

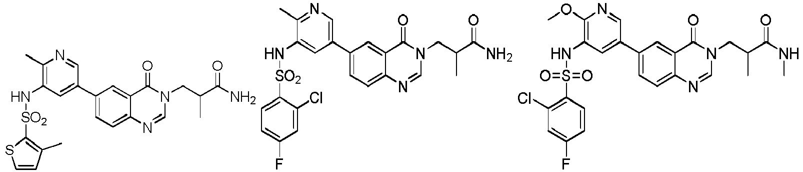

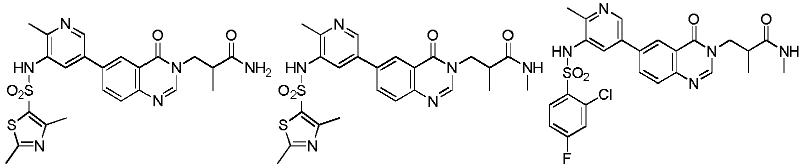

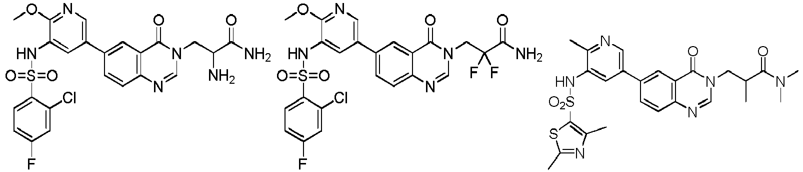

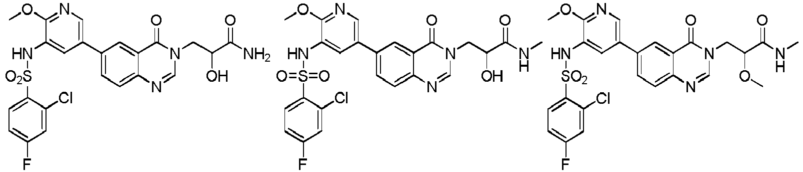

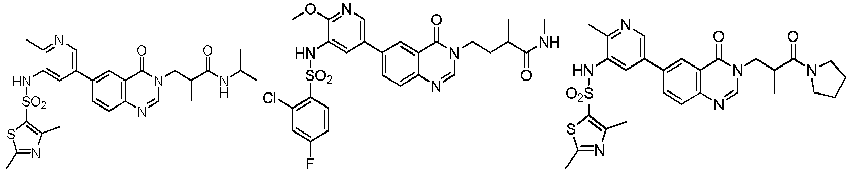

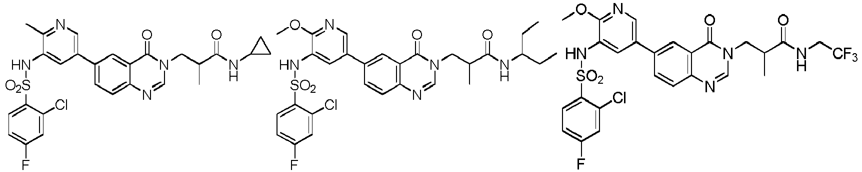

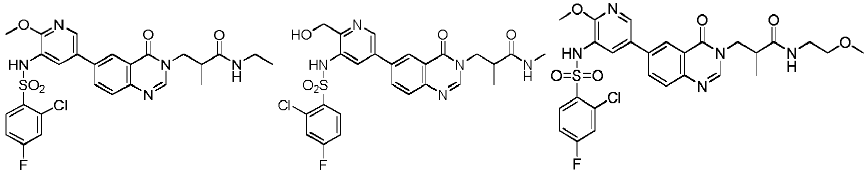

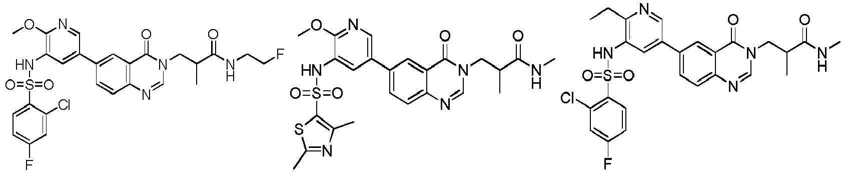

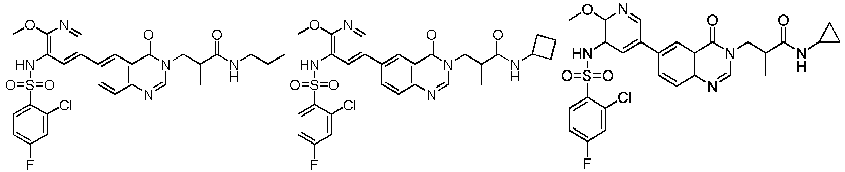

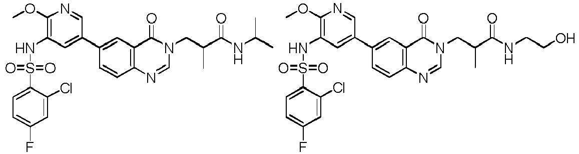

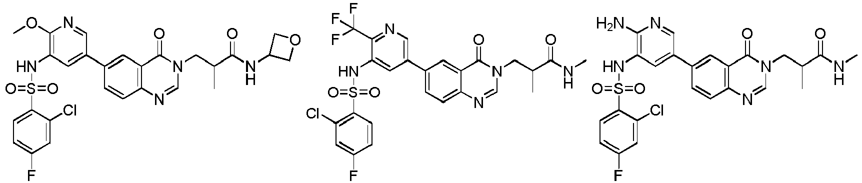

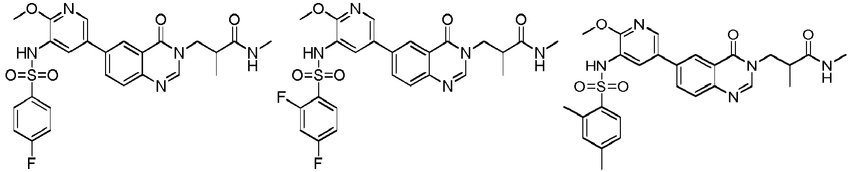

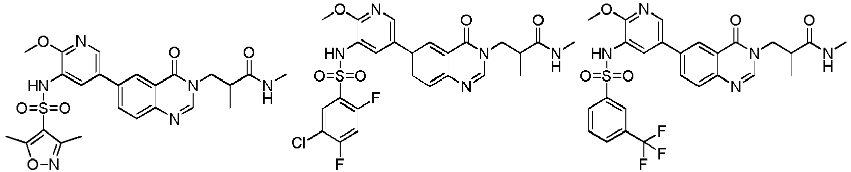

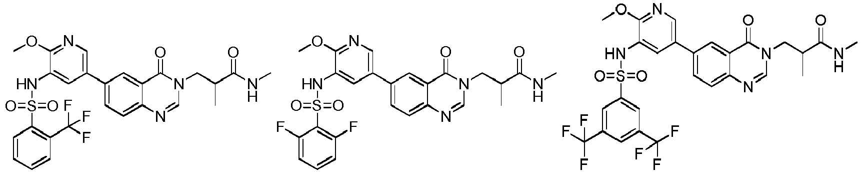

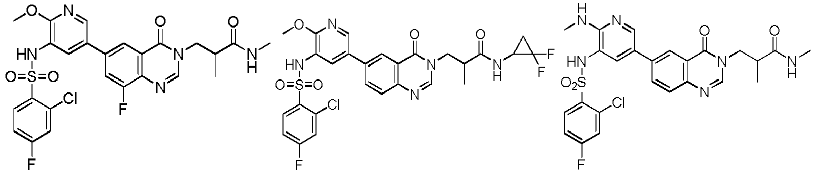

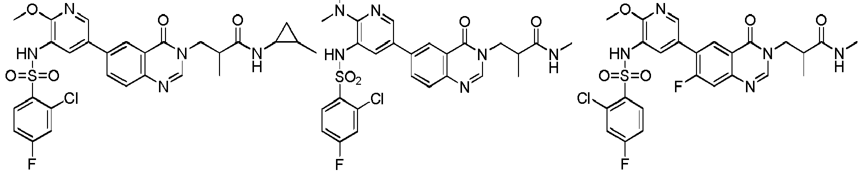

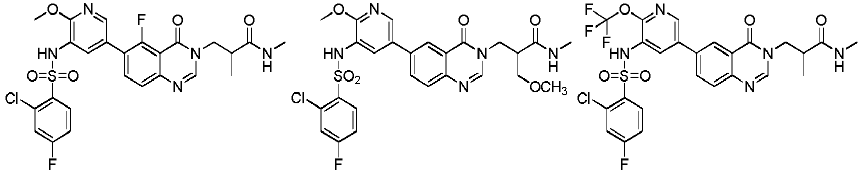

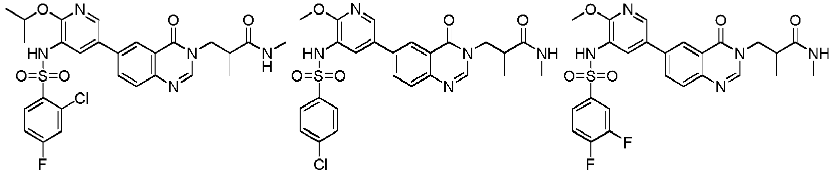

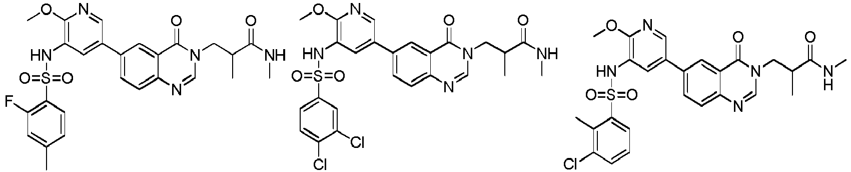

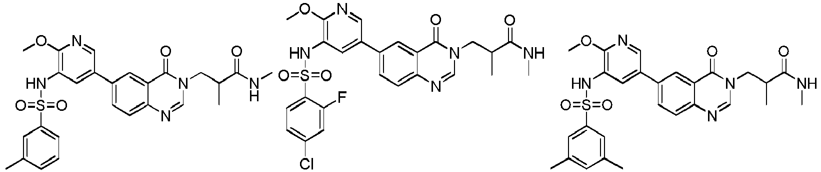

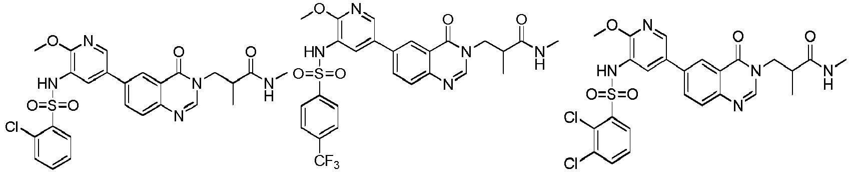

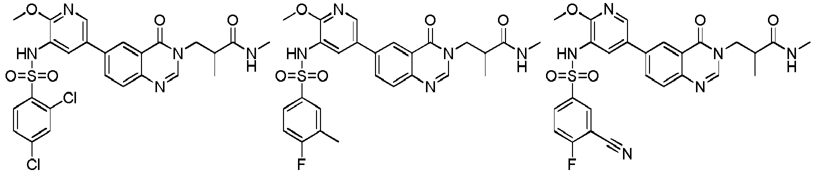

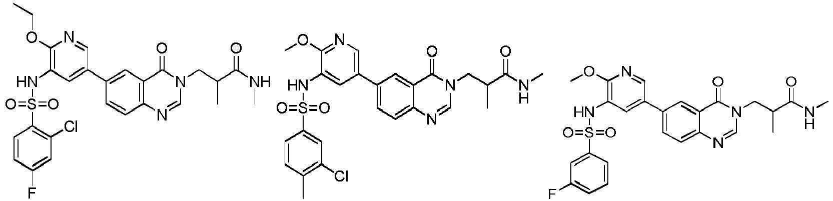

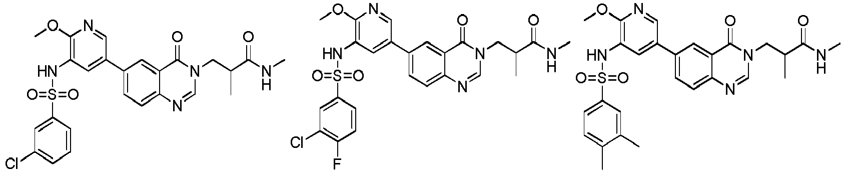

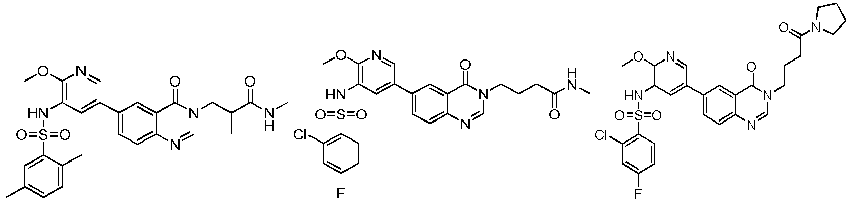

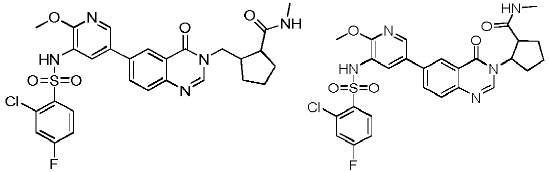

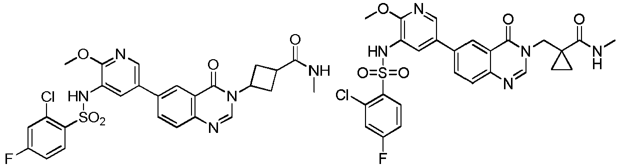

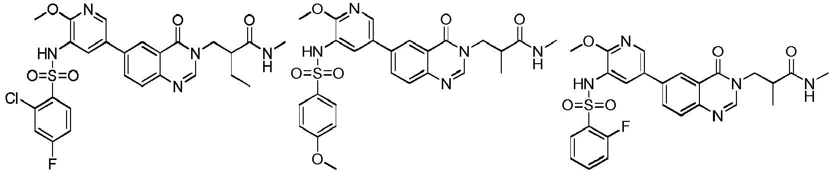

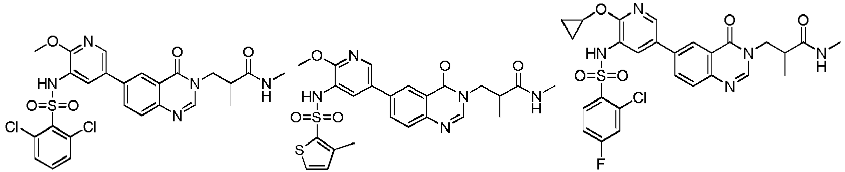

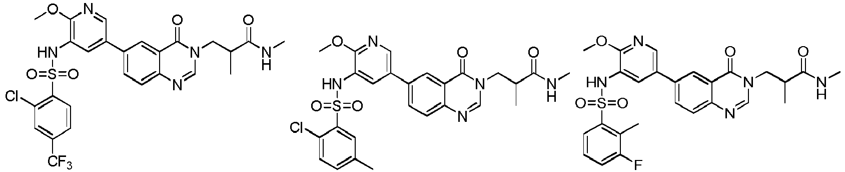

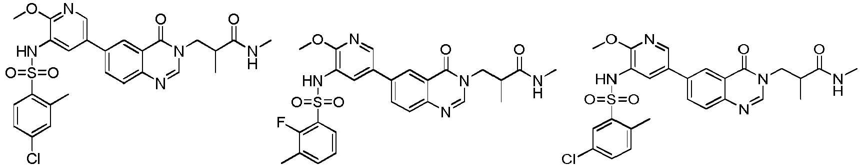

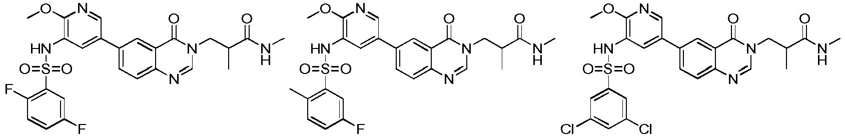

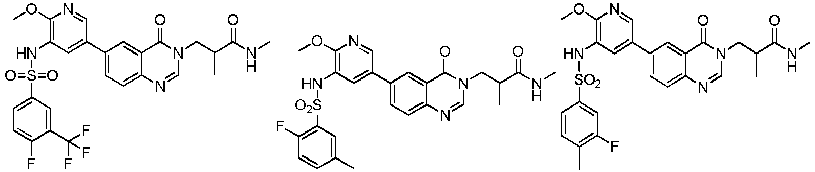

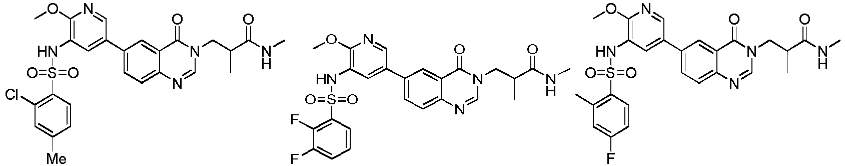

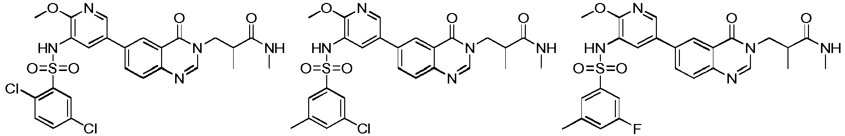

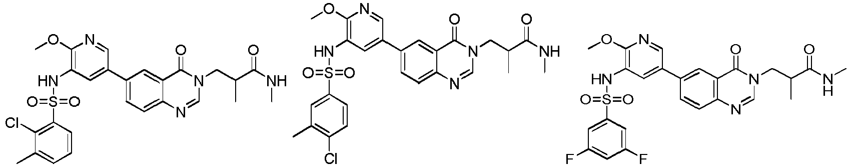

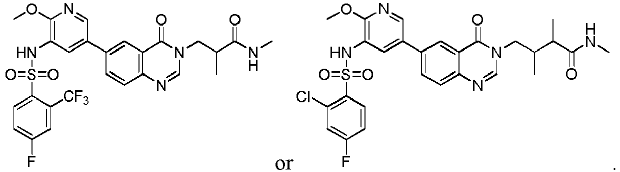

26. The compound, the isomer thereof or the pharmaceutically acceptable salt thereof as defined in any one of claims 1 to 24, the compound is selected from:

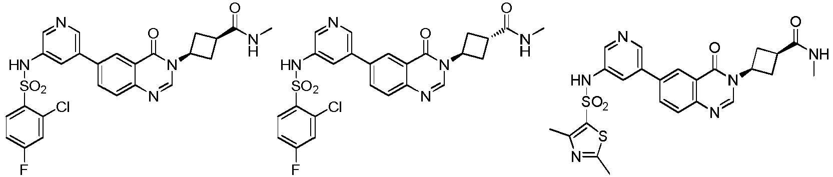

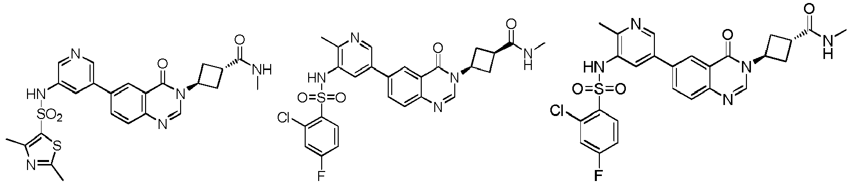

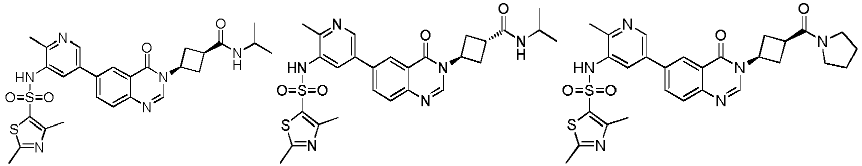

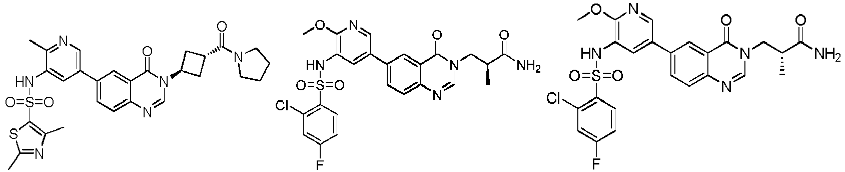

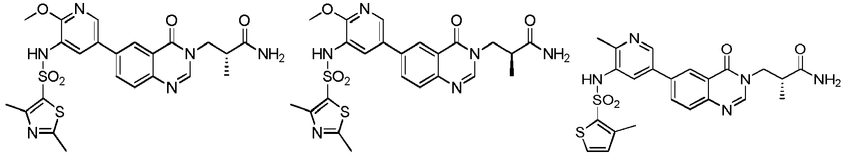

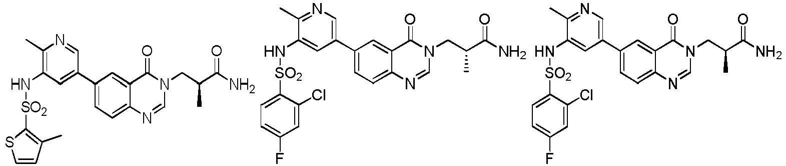

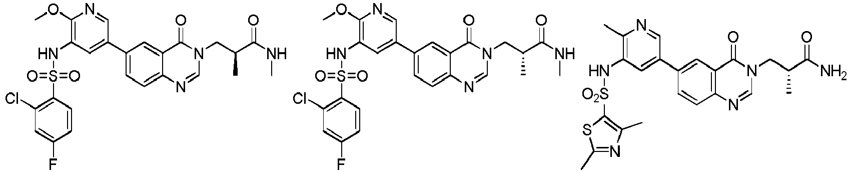

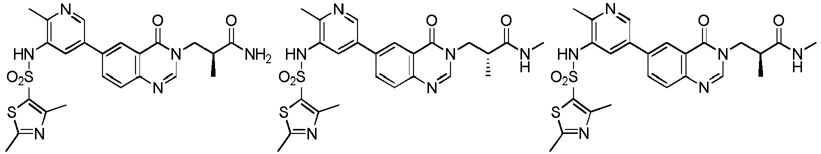

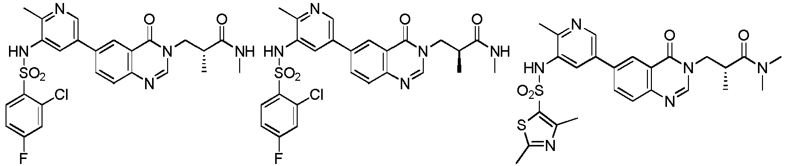

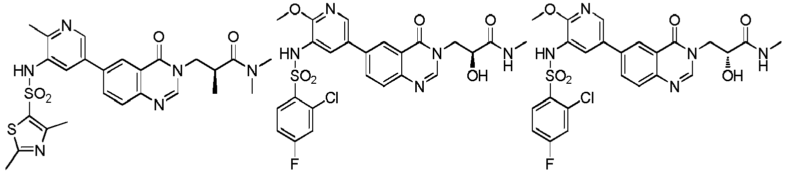

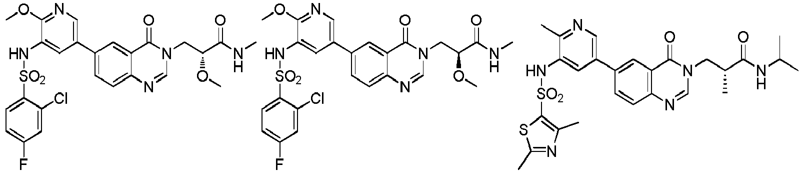

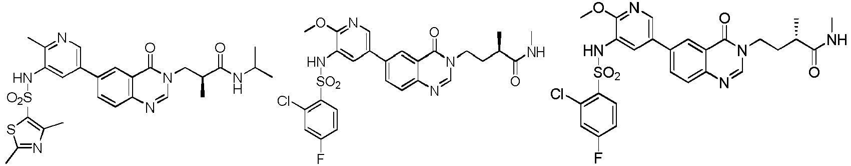

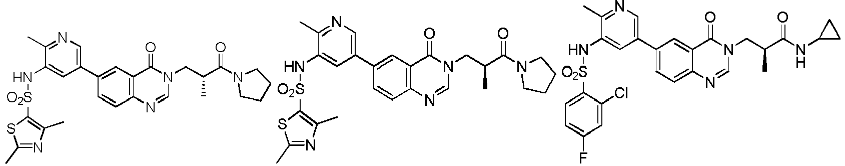

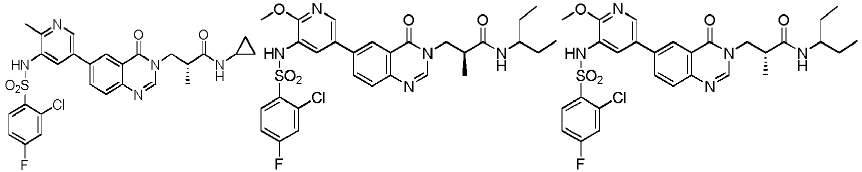

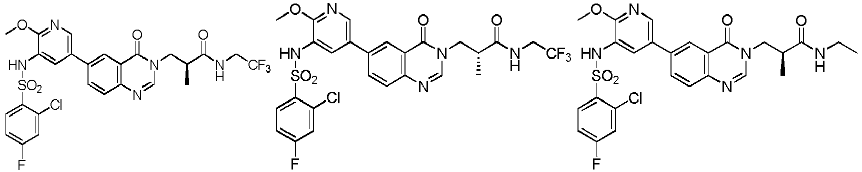

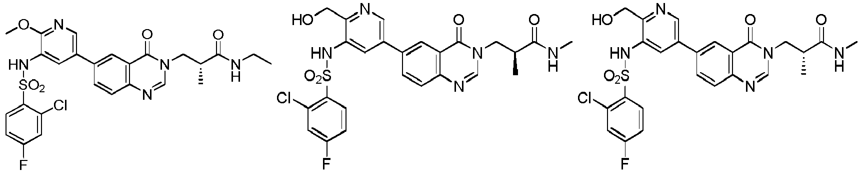

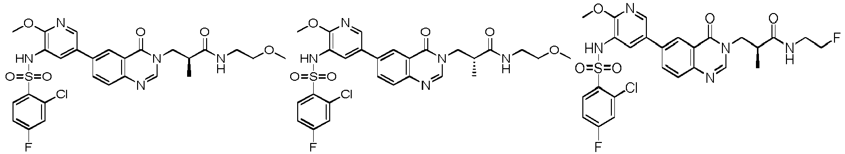

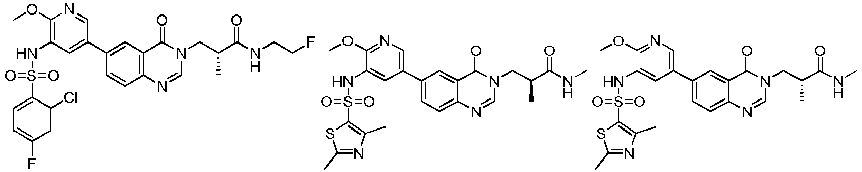

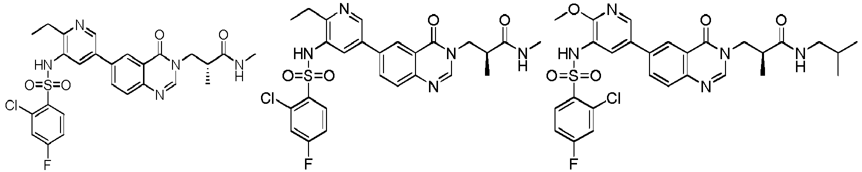

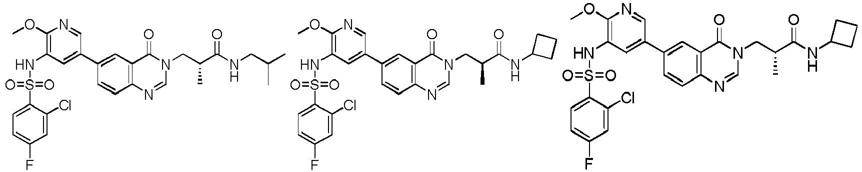

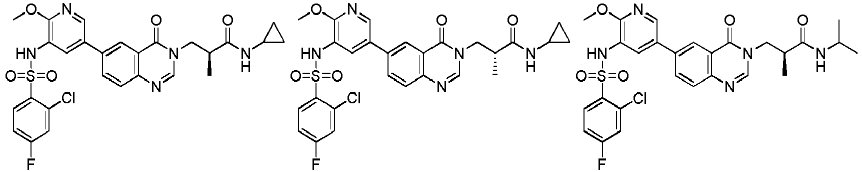

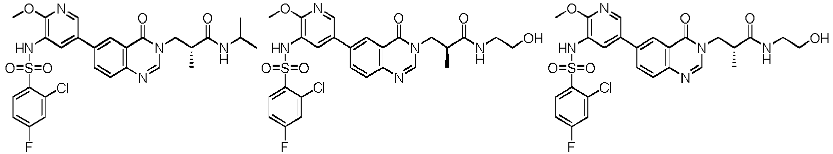

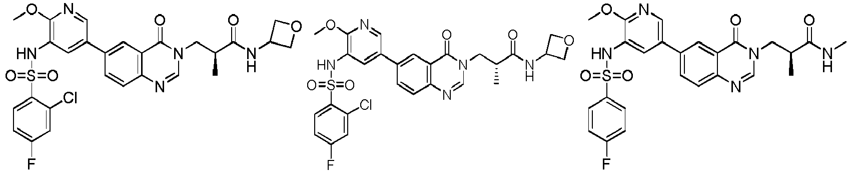

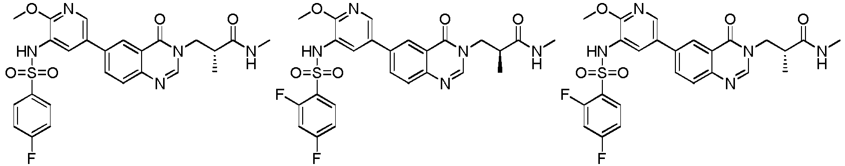

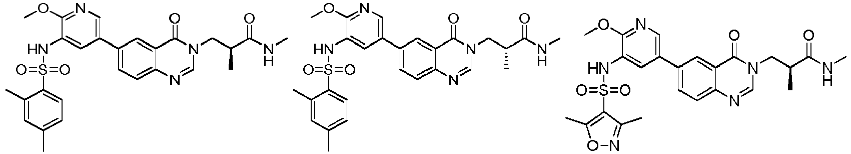

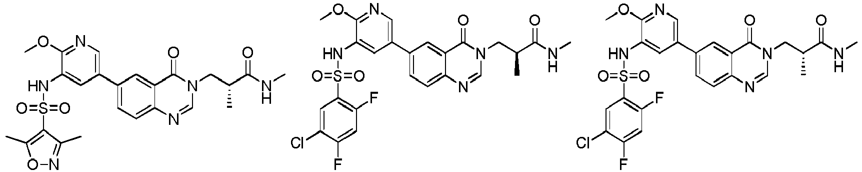

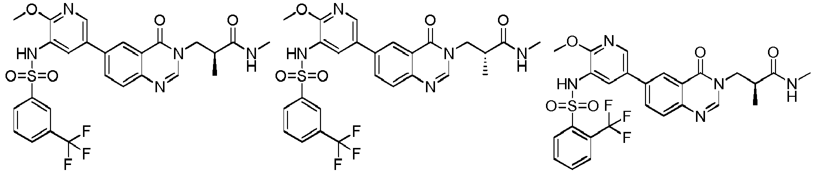

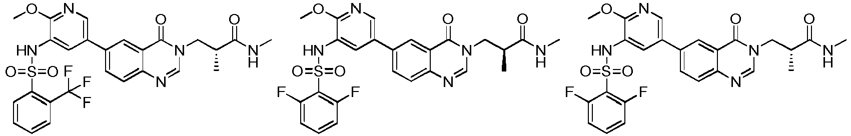

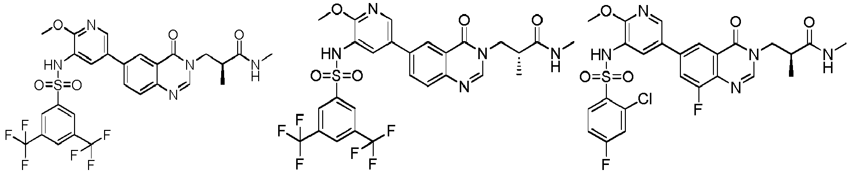

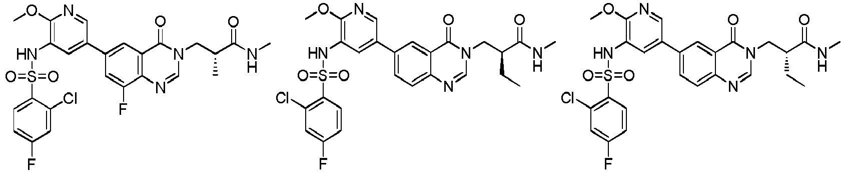

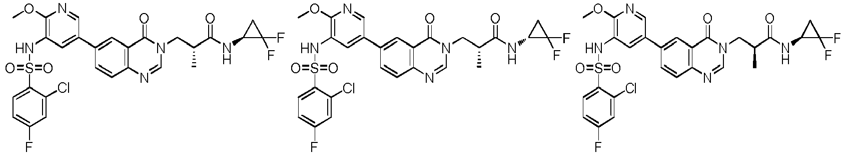

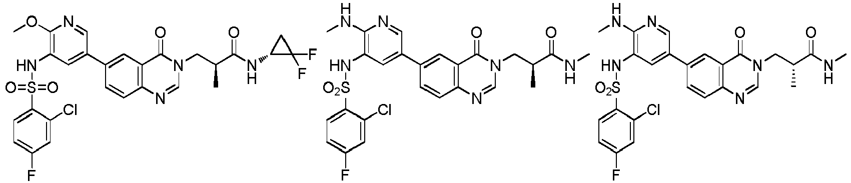

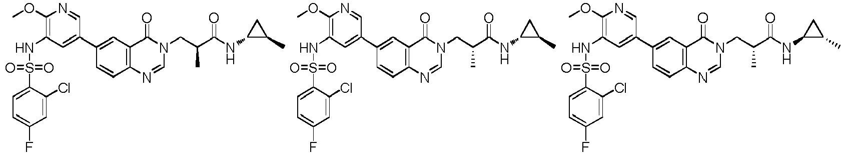

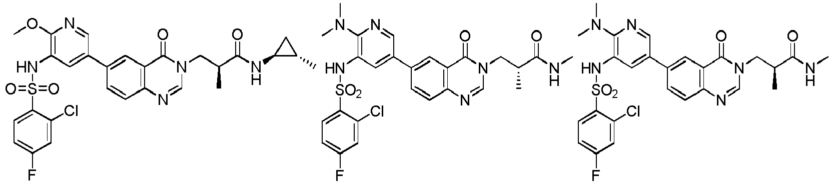

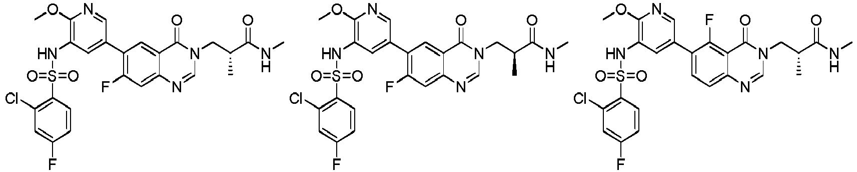

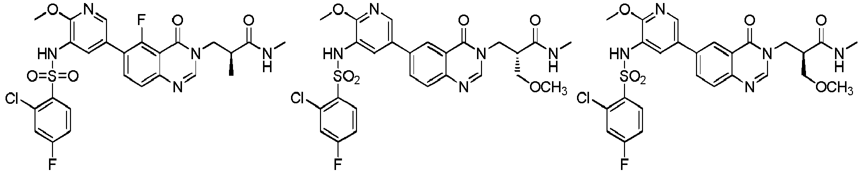

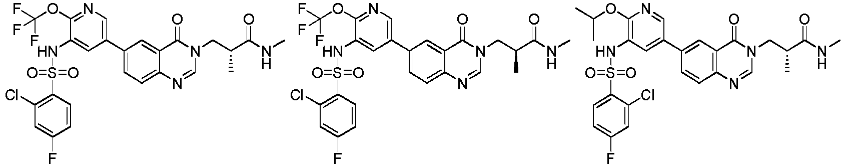

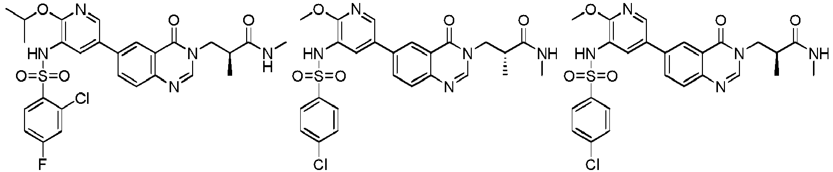

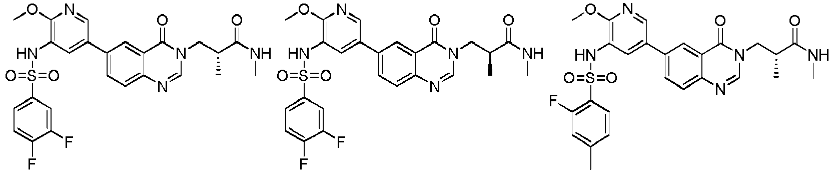

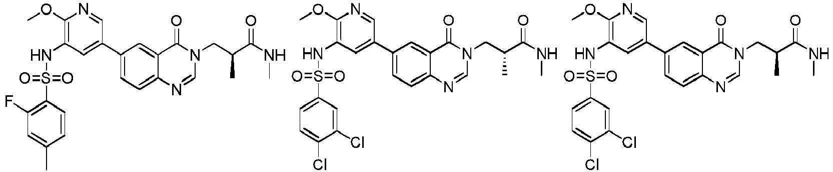

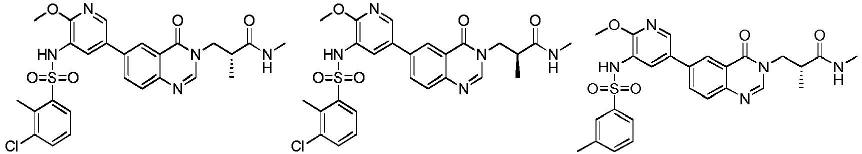

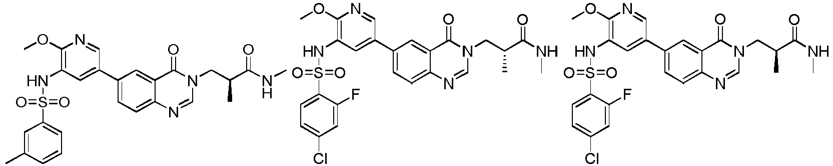

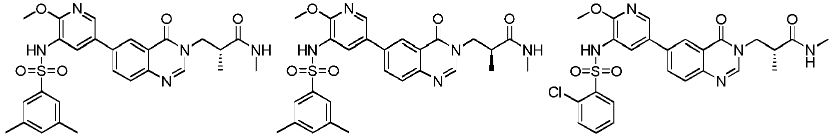

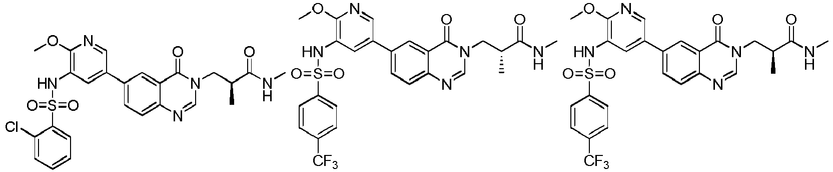

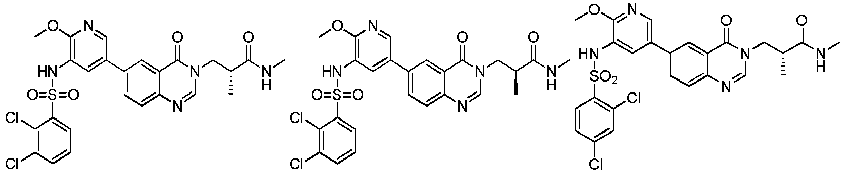

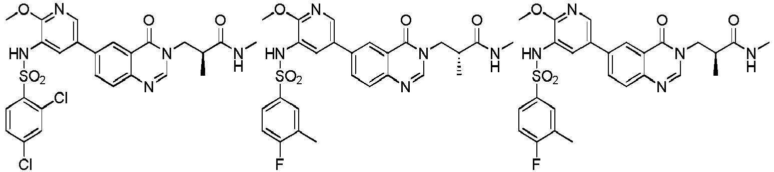

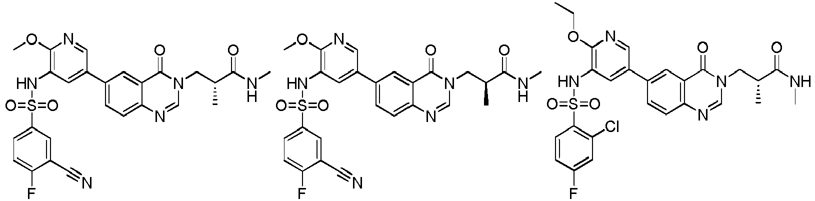

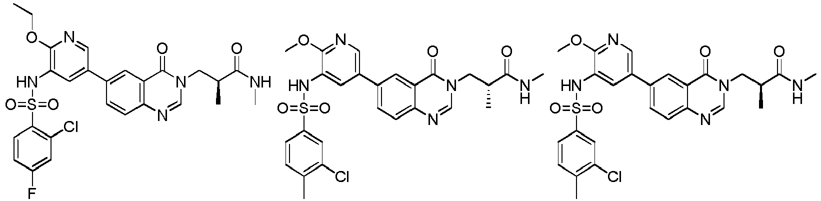

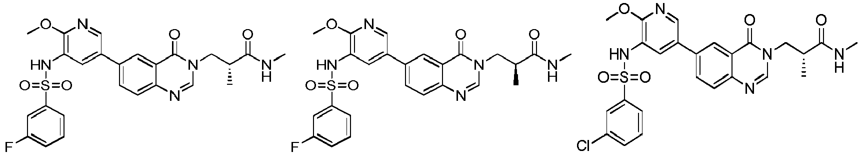

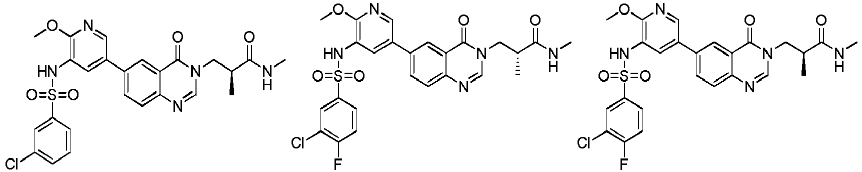

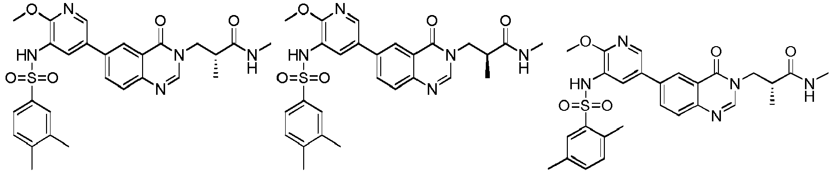

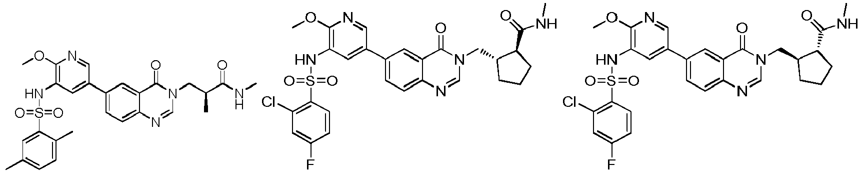

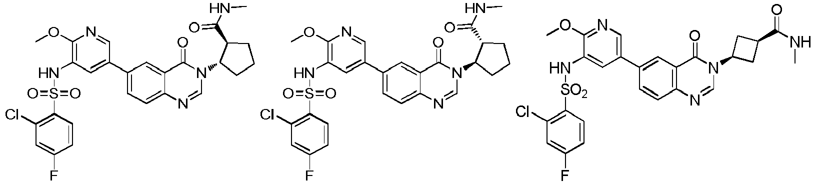

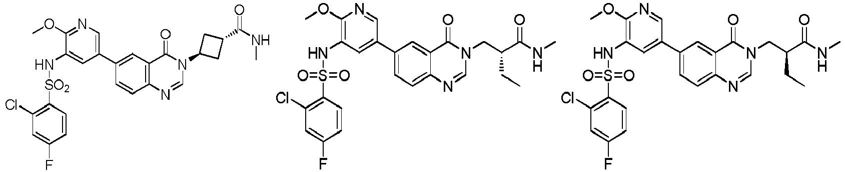

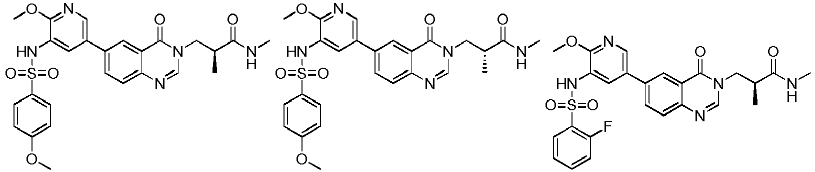

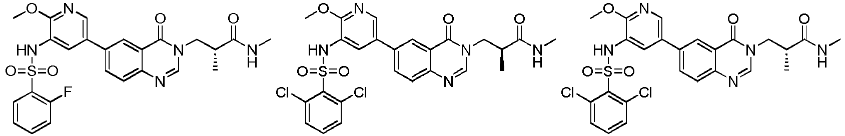

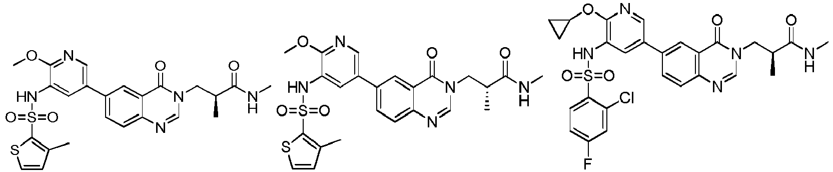

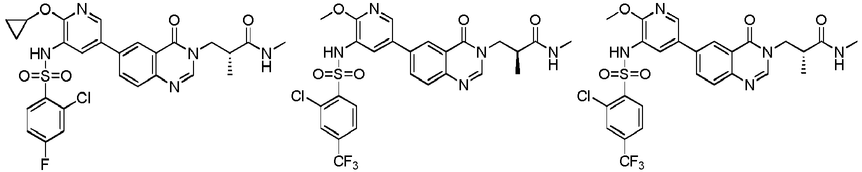

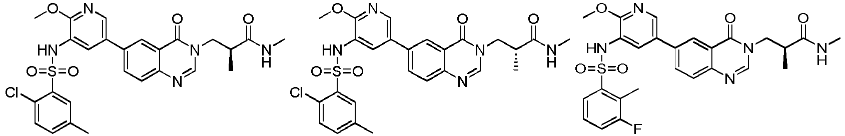

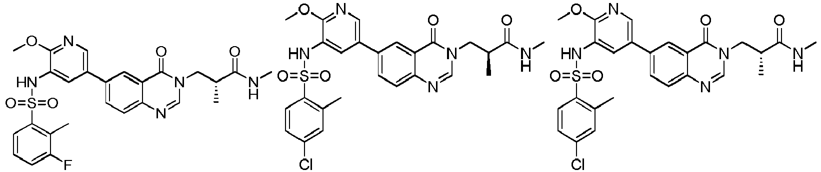

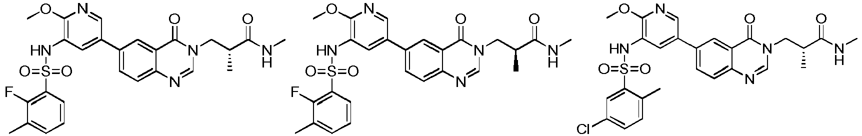

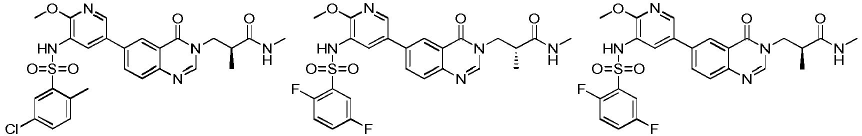

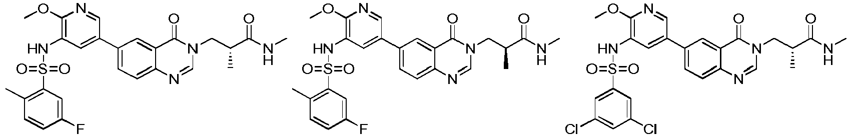

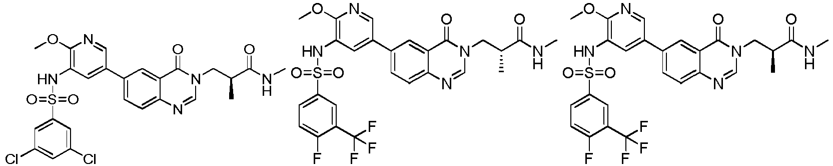

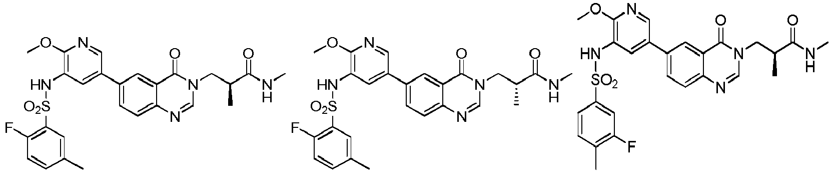

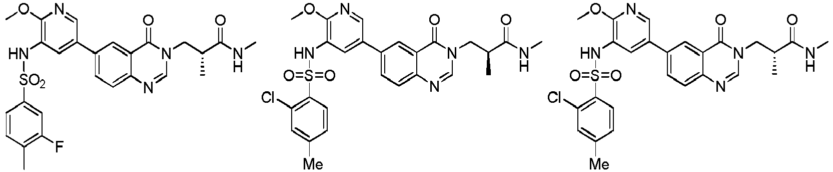

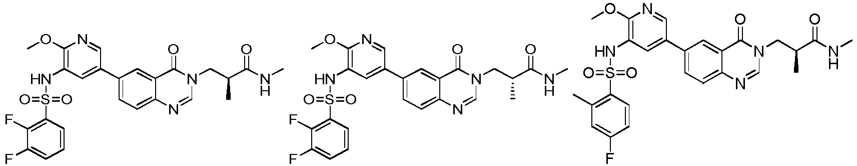

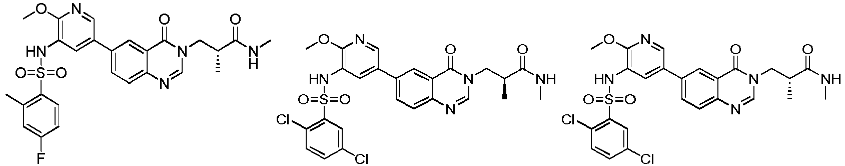

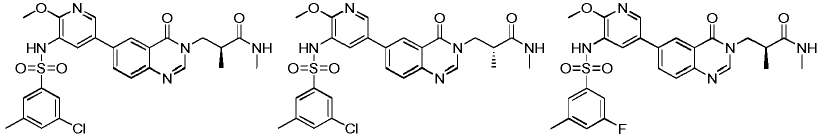

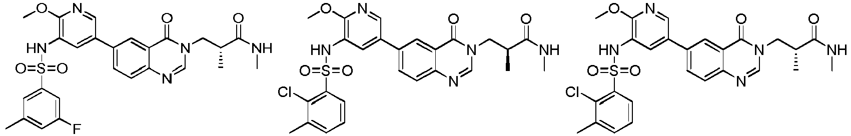

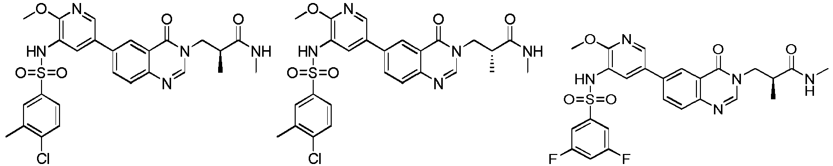

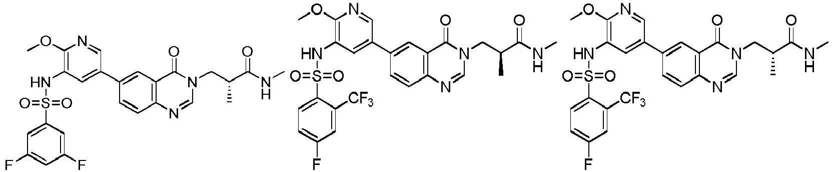

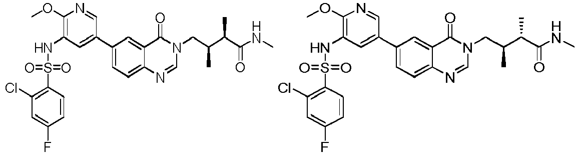

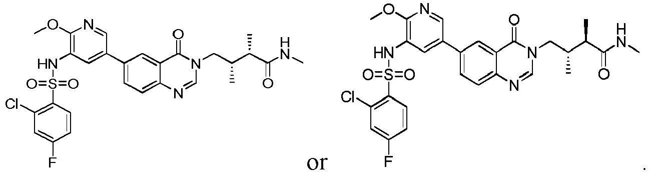

27. A pharmaceutical composition, comprising a therapeutically effective amount of the compound, the pharmaceutically acceptable salt thereof or the isomer thereof as defined in any one of claims 1 to 26 as an active ingredient, and a pharmaceutically acceptable carrier.

28. A use of the compound, the pharmaceutically acceptable salt thereof or the isomer thereof as defined in any one of claims 1 to 26 or the pharmaceutical composition as defined in claim 27 in manufacturing a PI3Kα inhibitor related medicament.

29. The use as defined in claim 28, wherein, the PI3Kα inhibitor related medicament is a medicament for use in treating solid tumors.
